# EUROPÄISCHE PATENTANMELDUNG

(11) **EP 1 475 435 A1**
(43) Veröffentlichungstag der Anmeldung: **10.11.2004**
(21) Anmeldenummer: 04010704.7
(22) Anmeldetag: 05.05.2004
(51) Int. Cl.: C12N 9/14, C12N 9/64, G01N 33/00

(54) **Kristallstruktur einer Prohormon/Proprotein-Proteinase und ihre Verwendung**

(30) Priorität: 09.05.2003 DE 10320879; 24.05.2003 DE 10323513
(71) Anmelder: Max-Planck-Gesellschaft zur Förderung der Wissenschaften e.V., 80539 München (DE); Proteros Biostructures GmbH, 82152 Planegg-Martinsried (DE)
(72) Erfinder: Henrich, Stefan, 81375 München (DE); Than, Manuel, Dr., 81475 München (DE); Bode, Wolfram, Prof. Dr., 82131 Gauting (DE); Kiefersauer, Rainer, Dr., 80634 München (DE); Huber, Robert, Prof. Dr., 82110 Germering (DE)
(74) Vertreter: Graf von Stosch, Andreas, Dr. rer. nat.

(57) **Zusammenfassung**

Die vorliegende Erfindung betrifft Raumformen eines Polypeptids einer Prohormon/Proprotein-Proteinase, Verfahren zur Strukturaufklärung von derartigen Polypeptiden, Speichermedien, enthaltend Strukturkoordinaten eines solchen Polypeptids; Kristalle mit derartigen Raumformen, Verfahren zur Herstellung derartiger Kristalle, Verbindungen mit der Fähigkeit, hochaffin an durch derartige Raumformen vorgegebene Strukturbereiche zu binden, Verwendungen derartiger Verbindungen zur Herstellung eines Arzneimittels sowie für Arzneimittel für bestimmte medizinische Indikationen, Verfahren zur Ermittlung derartiger Verbindungen und Verwendungen der mittels vorgenannter Verfahren erhältlichen Verbindungen.

## Beschreibung

Die vorliegende Erfindung betrifft Raumformen eines Polypeptids einer Prohormon/Proprotein-Proteinase, Verfahren zur Strukturau&lärung von derartigen Polypeptiden, Speichermedien, enthaltend Struktutkoordinaten eines solchen Polypeptids; Kristalle mit derartigen Raumformen, Verfahren zur Herstellung derartiger Kristalle, Verbindungen mit der Fähigkeit, hochaffin an durch derartige Raumformen vorgegebene Strukturbereiche zu binden, Verwendungen derartiger Verbindungen zur Herstellung eines Arzneimittels sowie für Arzneimittel für bestimmte medizinische Indikationen, Verfahren zur Ermittlung derartiger Verbindungen und Verwendungen der mittels vorgenannter Verfahren erhältlichen Verbindungen.

Aus dem Stand der Technik ist bekannt, daß es zahlreiche humane oder aus dem Säugetier stammende Calcium-abhängige Endoproteinasen gibt, sogenannte Prohormon/Proprotein Convertasen, häufig auch PCs abgekürzt. Als homologe Proteine zum aus dem Stand der Technik bekannten Hefeprotein kex 2/kexin (Endoproteinase (Fuller, Science, 246, 482 - 486, 1989)) gibt es weitere Mitglieder dieser Familie, die die Kurzbezeichnungen PC1/PC3, PC2, PACE4, PC4, PC5/PC6 und PC7/PC8/LPC/SPC7 tragen (Siezen et aL, Protein Science 6, 501 - 523, 1997). Bekannt ist weiterhin die Endoproteinase Furin (EC3.4.21.75, MEROPS clan SB, family S8), die auch SPC1/PACE genannt wird. Hierbei handelt es sich um ein 95 kDa Typ-I-Transmembranglykoprotein, das als 794 Aminosäuren umfassendes Präproenzym in allen bislang untersuchten Geweben oder Zellen zunächst translatiert wird. Die N-terminalen 83 Aminosäuren des Propeptids, das insbesondere bei der Faltung eine wichtige Rolle spielt, werden autokatalytisch aus dem im übrigen inaktiven Zymogen herausgeschnitten, wobei es mit der aktiven Domäne assoziiert bleibt. Hierbei kommt ihm die Rolle eines wirkungsvollen Autoinhibitors zu. Während der Wanderung zu den stärker sauren Abschnitten des Trans-Golgi-Netzwerks (TGN) dissoziiert das Prosegment nach einer zweiten inneren autokatalytischen Spaltung, wodurch das katalytisch aktive Furin entsteht (Anderson et al. EMBO Y. 16, 1508 - 1518, 1997 und Anderson et aL, J. BioL Chem. 277, 12879 - 12890, 2002). Hierbei bewegt sich Furin zwischen dem TEN, der Zelloberfläche und den endosomalen Kompartimenten (Anderson et aL, J. BioL Chem. 277,12897 -12890, 2002) und Thomas, Nature Rev. Mol. Cellbiol. 3, 753 - 776, 2002). Dabei prozessiert Furin zahlreiche membrangebundene und lösliche Precursor von Wachstumsfaktoren, Rezeptoren, Plasmoproteinen, Sekretasen und extrazellulären Matrixproteinasen. Furin wird auch für die Aktivierung von zahlreichen bakteriellen Toxinprecursom benötigt, wie auch für die Prozessierung von viralen Oberflächenglykoproteinen ("Envelope-Glykoproteins"), wie zum Beispiel HIV-1 gp160 oder den "Avian Influenza Virus Hämagglutinin". Neben den membrangebundenen Formen von Furin treten auch natürlich verkürzte "shed" Furinformen auf und wurden als funktional aktiv nachgewiesen. Trotz der Bedeutung von Furin und anderen PCs für wichtige physiologische Prozesse und seiner Bedeutung für zahlreiche ernst zu nehmende Erkrankungen wurde bislang eine Struktur eines Mitglieds der Familie der Prohormon/Proprotein-Convertasen (PCs) nicht gelöst. Es liegt im Stand der Technik ausschließlich die Struktur der Prodomäne PC1/PC3 in Lösung, aufgeklärt durch NMR Spektroskopie vor (Tankrea et aL, J. Mol. BioL 320, 801 - 812, 2002), vor. Daneben gibt es im Stand der Technik Strukturvorschläge der katalytischen Domäne (Siezen et aL, Eur. J. Biochem. 222, 255 - 266, 1994) und der P-Domäne (Lipkind, Proc. Natl. Acad. Sci. USA, 95, 7310 - 7215, 1998). Diese Strukturvorschläge wurden auf der Basis von bakteriellen Subtilisinen und Sekundärstrukturvorhersagen erstellt.

Insbesondere für das Verständnis der katalytischen Funktion von Prohormon/Proproteinconvertasen ist es jedoch erforderlich, eine Darstellung der gesamten dreidimensionalen Struktur dieser Proteinfamilie zu bestimmen. Eine derartige Proteinstruktur erlaubt es auch, Verständnis für die auch medizinisch relevanten Effekte der PCs zu erlangen und weitere, auch in pharmazeutisch/chemischer Hinsicht bedeutsame Untersuchungen an dieser Proteinfamilie vorzunehmen.

Aufgabe der vorliegenden Erfindung ist es daher, auf der Basis von biophysikalischen Methoden Erkenntnisse über die strukturellen Grundlagen von PCs zu gewinnen, und diese Erkenntnisse beispielsweise für die Modellierung von Liganden einzusetzen, die paßgenau die biologische Funktion von PCs modulieren können.

Erfindungsgemäß werden daher Kristallstrukturen von Polypeptiden der Klasse der Prohormon/Proprotein-Convertasen (PCs) offenbart. Erfindungsgemäße KristallstrukLuren umfassen die Strukturkoordinaten von Proteinen der Klasse der PCs, insbesondere von Furin, oder aber von Abschnitten (bspw. Domänen) von Proteinen der Klasse der PCs, bspw. insbesondere der katalytischen Domäne oder der P-Domäne von Furin. Offenbart werden daher Raumformen der vorgenannten Proteine oder Abschnitte, insbesondere Domänen, derselben, die ihren Ausdruck als "Raumform", also als Verkörperung dieser Strukturkoordinaten, finden.

Gemäß der vorliegenden Erfindung wird eine Raumform eines Polypeptids beansprucht, wobei das Polypeptid in der Raumform mindestens eine mindestens 50 Aminosäuren (AS) umfassende Aminosäuresequenz eines Proteins aus der Familie der PCs oder ein Derivat davon, das zu mindestens 80%, vorzugsweise mindestens 90%, mehr bevorzugt mindestens 95%, noch mehr bevorzugt mindestens 97% mit der mindestens 50 AS umfassenden Aminosäuresequenz (Primärsequenz) identisch ist. Vorzugsweise handelt es sich um ein mindestens 80 AS, mehr bevorzugt mindestens 100 AS, stärker bevorzugt mindestens 120 AS, noch stärker bevorzugt mindestens 150 AS umfassendes Fragment eines Proteins aus der Familie der PCs, das in der erfindungsgemäßen Raumform auftritt. Bei rekombinanten Polypeptiden in der erfindungsgemäßen Raumform kann es sich um solche handeln, die zwei oder mehr der vorgenannten mindestens 50 AS umfassenden Sequenzen von Proteinen aus der Familie der PCs enthalten, die in dem in der Raumform auftretenden Polypeptid eingebettet bzw. verbunden durch andere ("Linker")-Aminosäuresequenzen in beliebiger Reihenfolge vorliegen.

Vorzugsweise wird das Polypeptid in der erfindungsgemäßen Raumform mindestens eine einer Domäne eines Proteins aus der Familie der PCs entsprechende Aminosäuresequenz oder ein Derivat (gemäß obiger Definition) einer solchen Aminosäuresequenz enthalten.

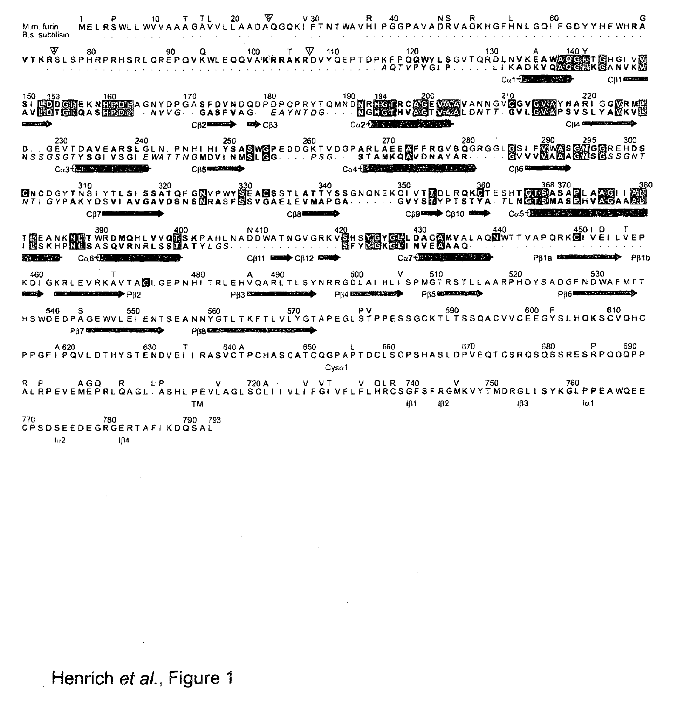

Bspw. kann das Polypeptid in der erfindungsgemäßen Raumform die Aminosäuresequenz der P-Domäne oder der katalytischen Domäne eines Proteins aus der Familie der PCs, insbesondere eukaryotischen Ursprungs, ganz besonders von Proteinen aus der Familie der PCs von Säugern, vor allem humanen Ursprungs, enthalten. Im Falle eines Furins als Beispielsfall eines Proteins aus der Familie der PCs handelt es sich bspw. um einen wie vorstehend definierten Sequenzabschnitt von mindestens 50 oder - mehr bevorzugt - mehr AS der in Fig. 1 (obere Sequenz) gezeigten Aminosäuresequenz, insbesondere des humanen oder murinen Furins, z. B. um Sequenzen der Postionen 1 oder 76 oder 108 oder 110 bis 445 oder 573 oder 793, vorzugsweise um Sequenzen der Positionen 110 bis 573, besonders bevorzugt um Sequenzen der Postionen 110 bis 445 und 446 bis 573.

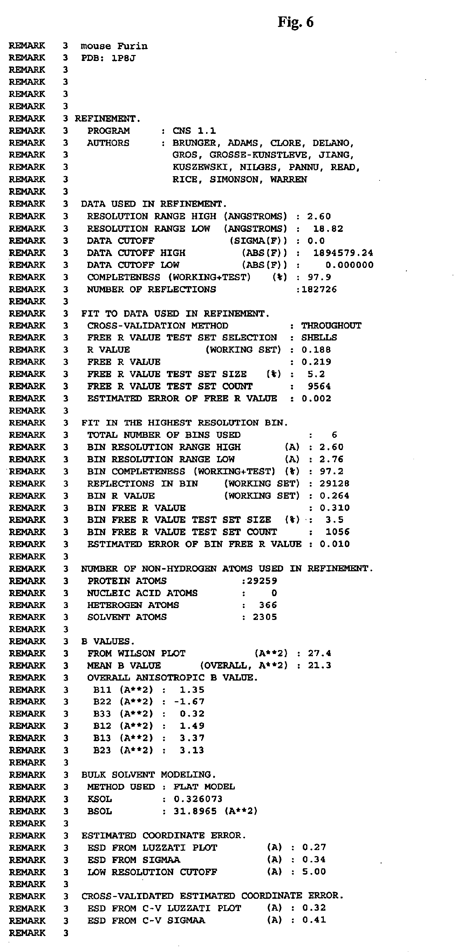

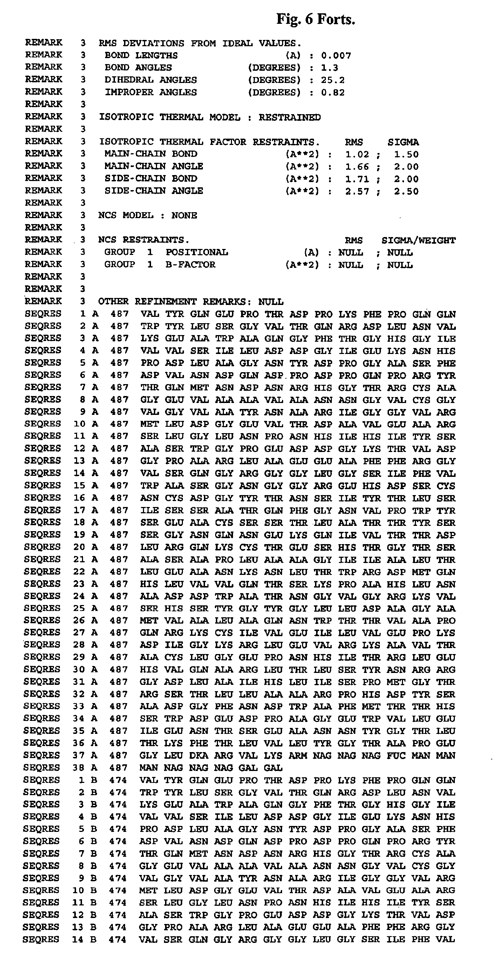

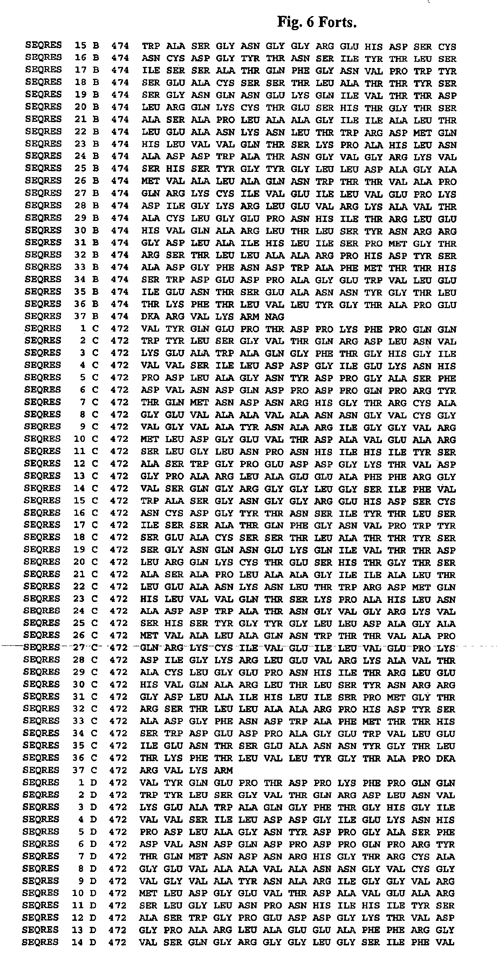

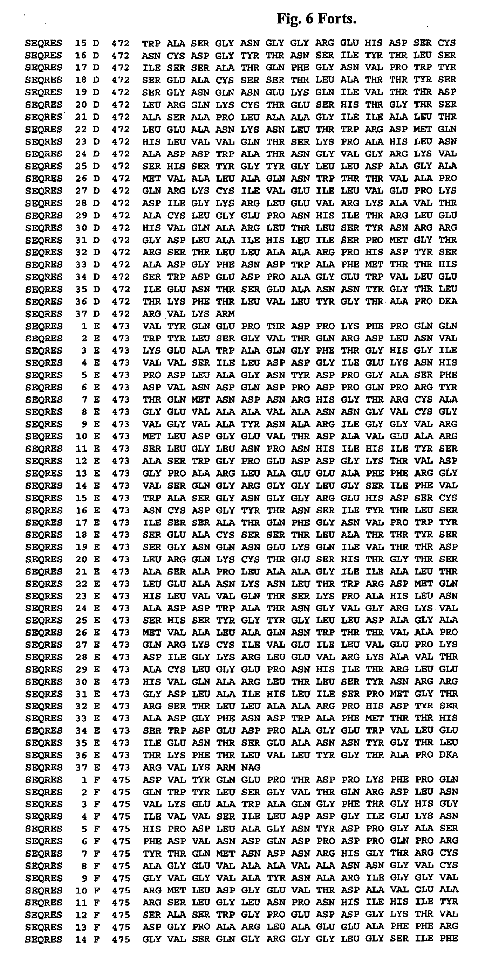

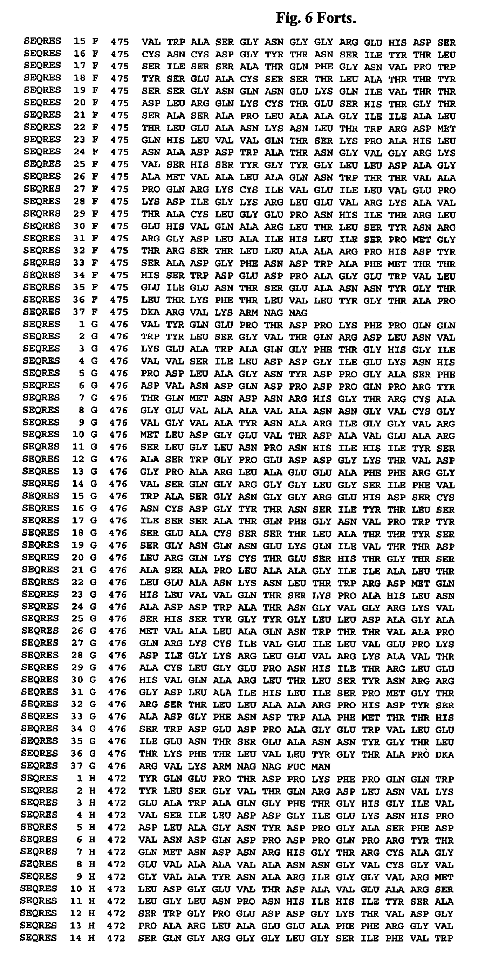

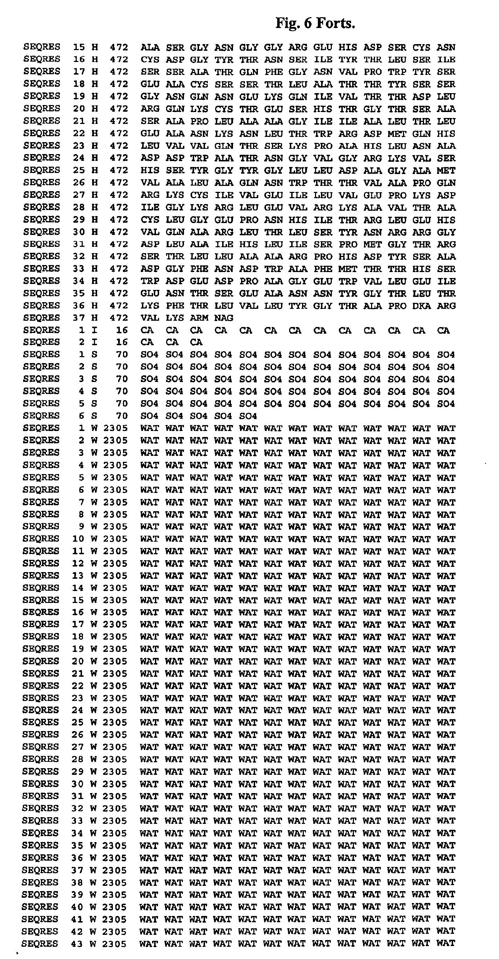

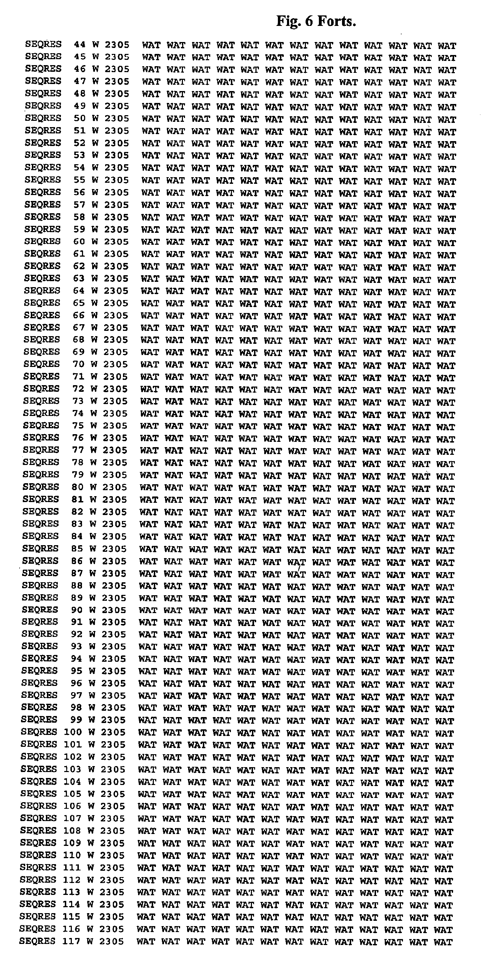

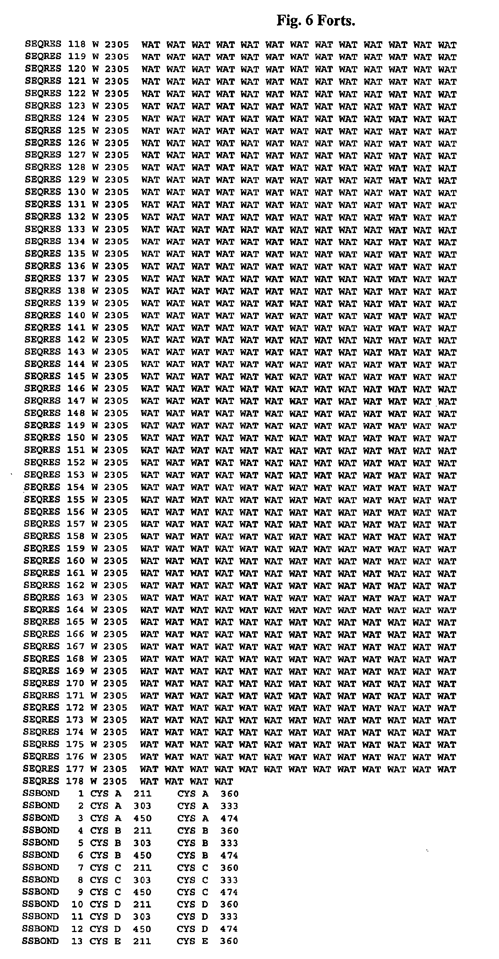

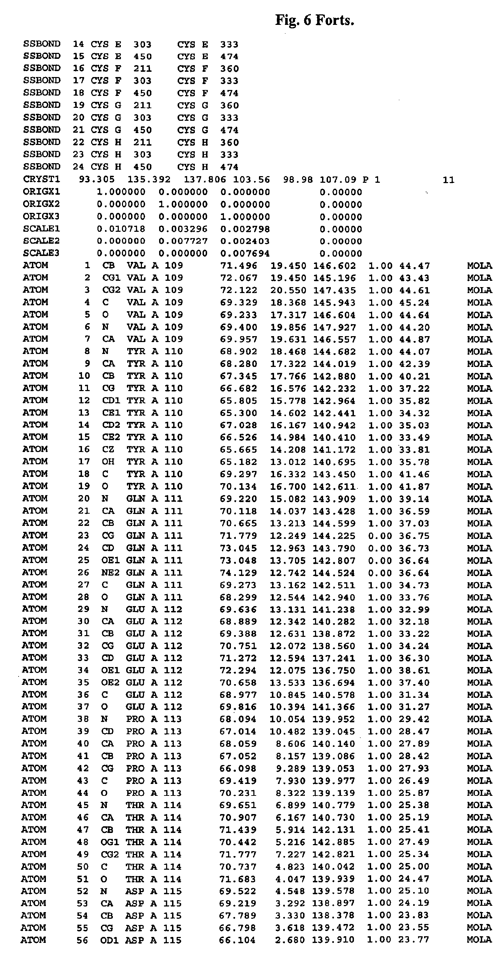

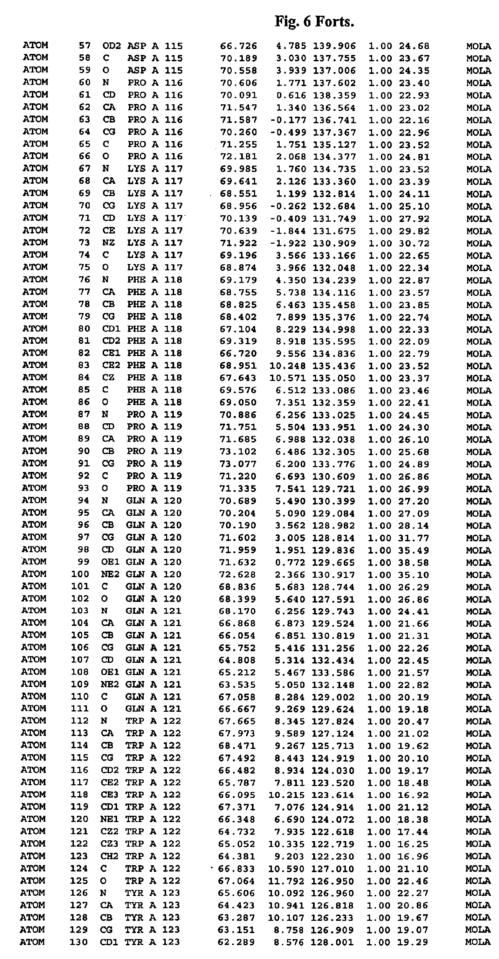

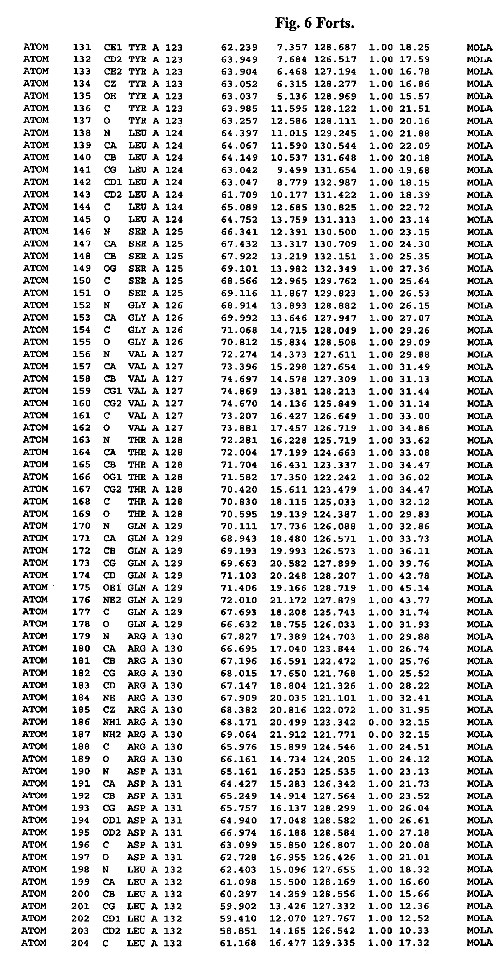

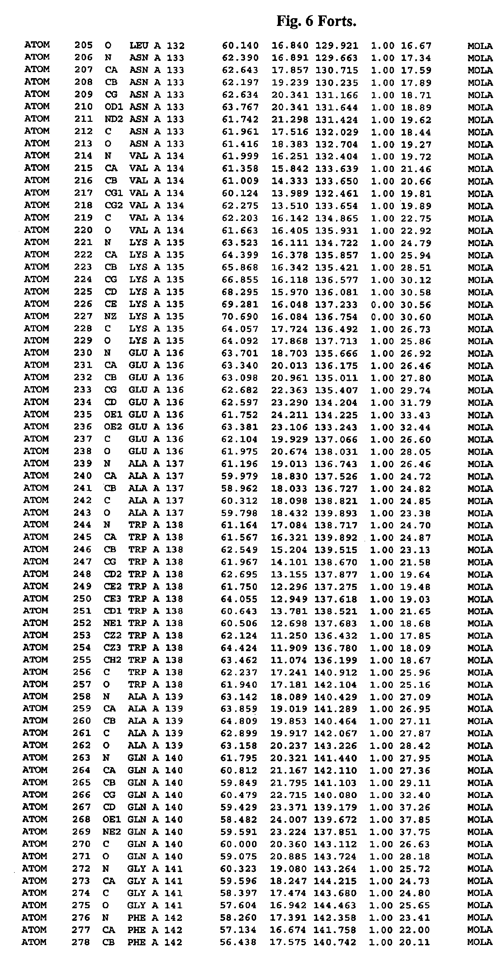

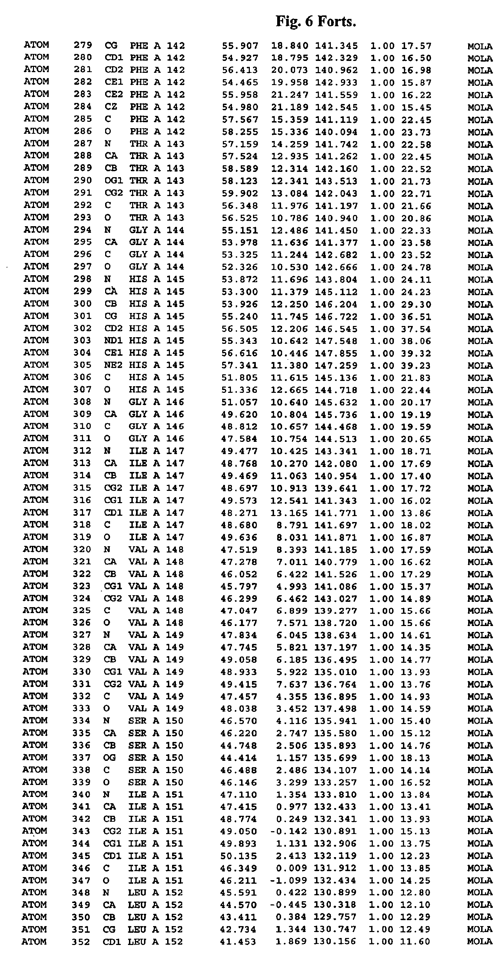

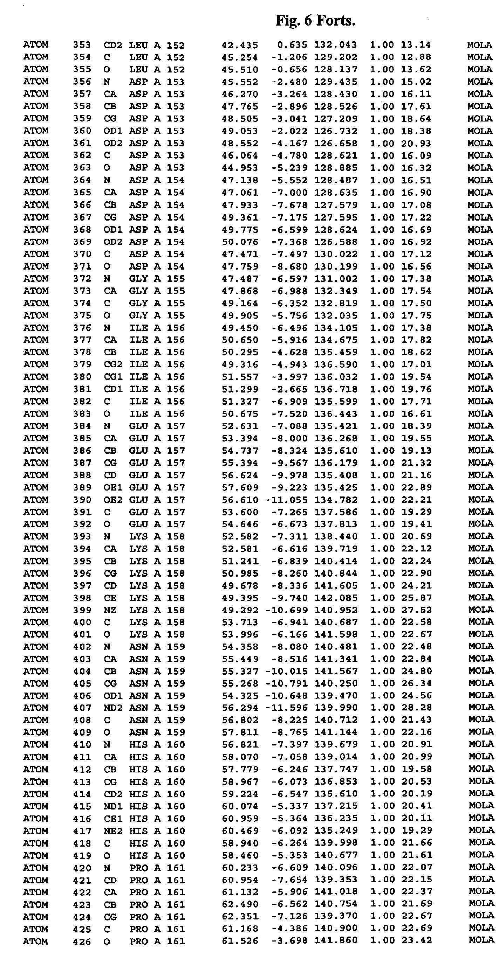

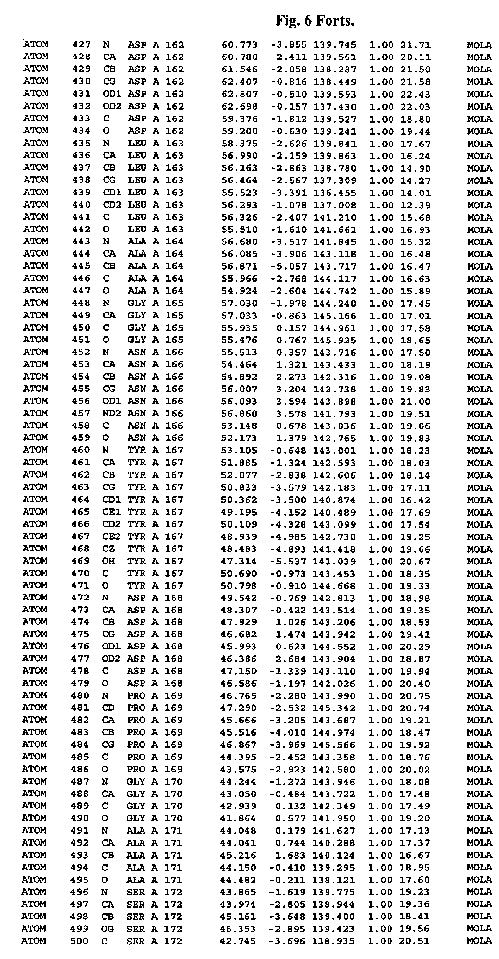

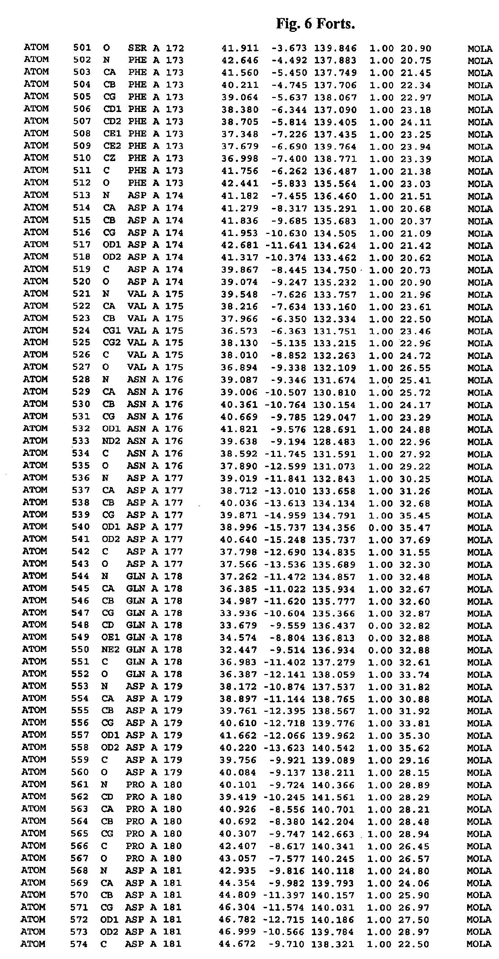

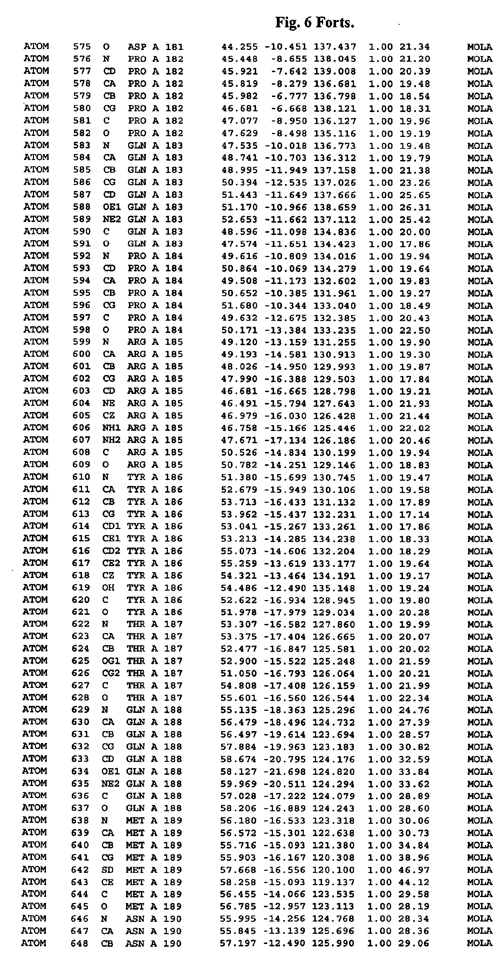

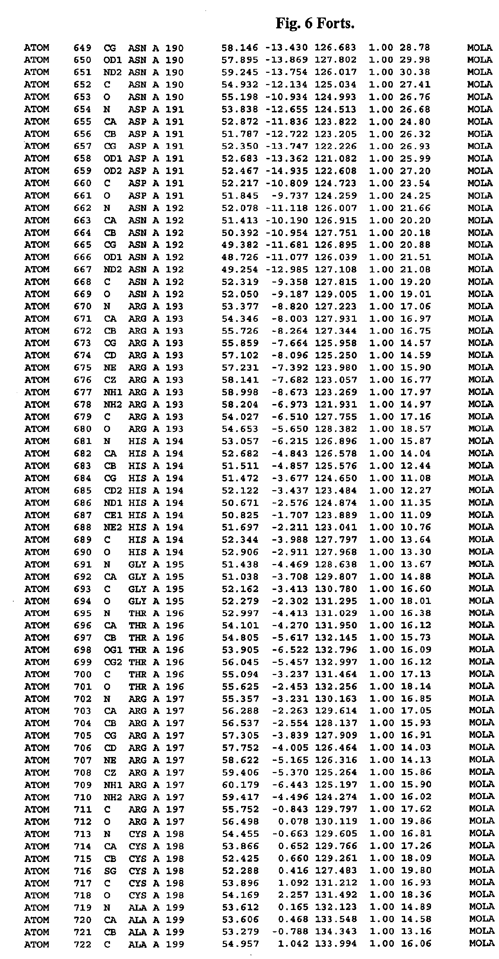

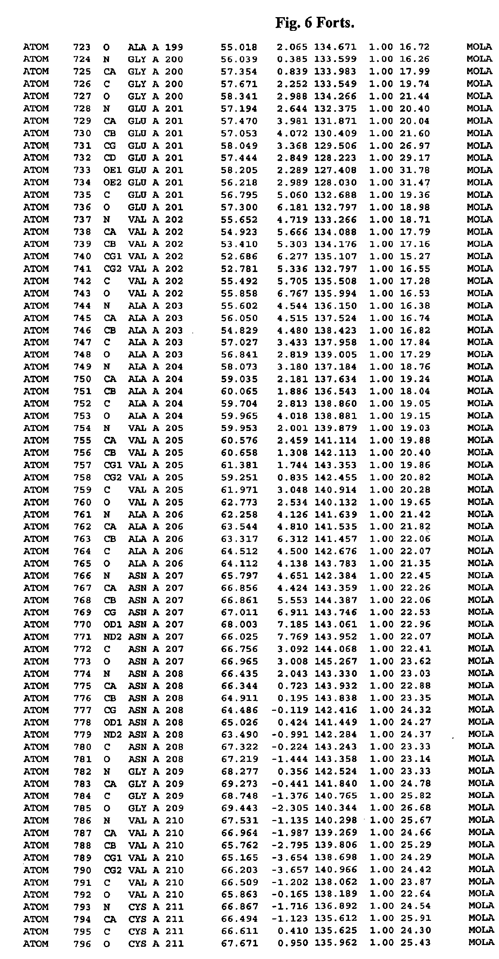

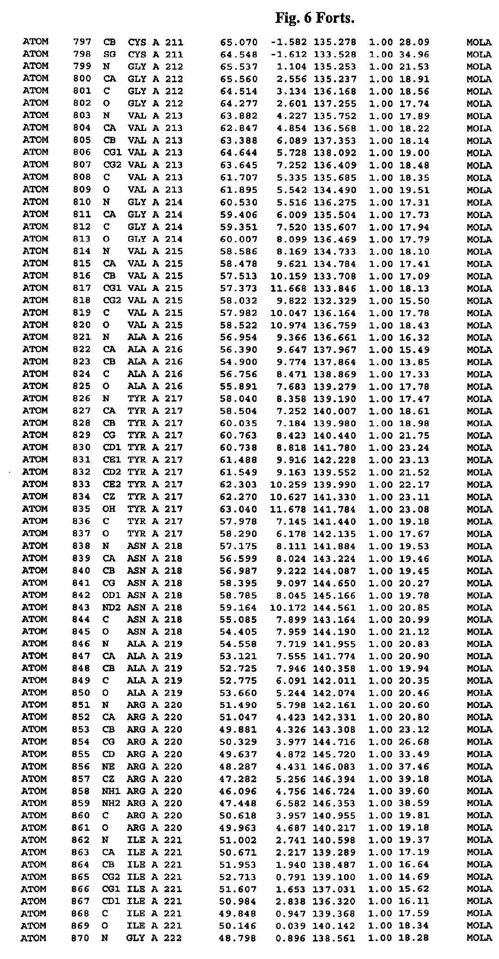

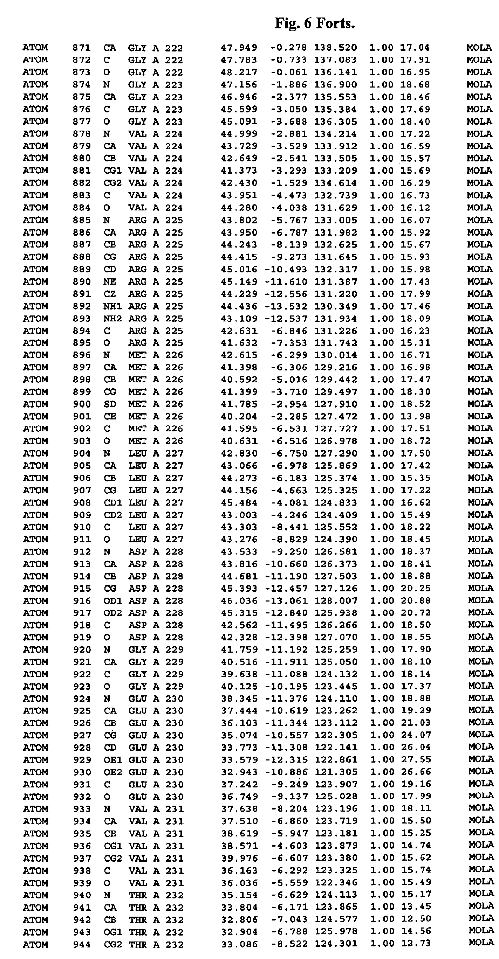

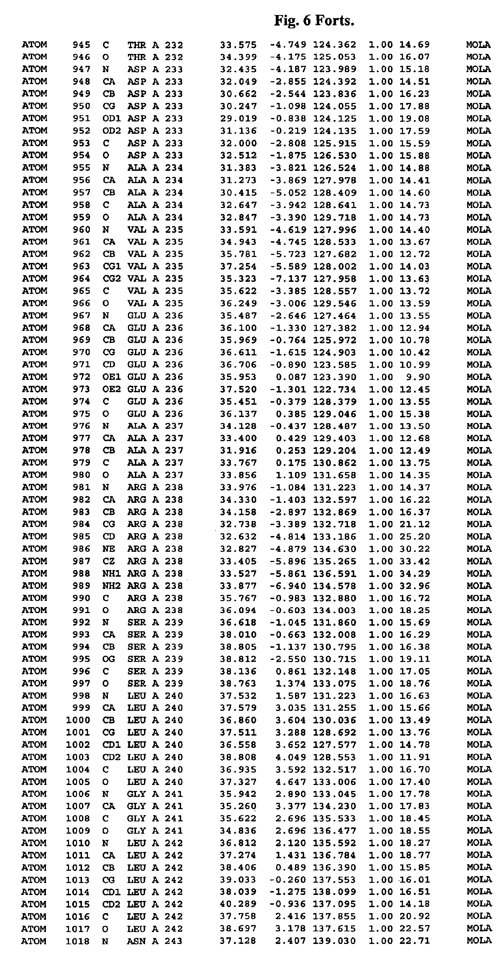

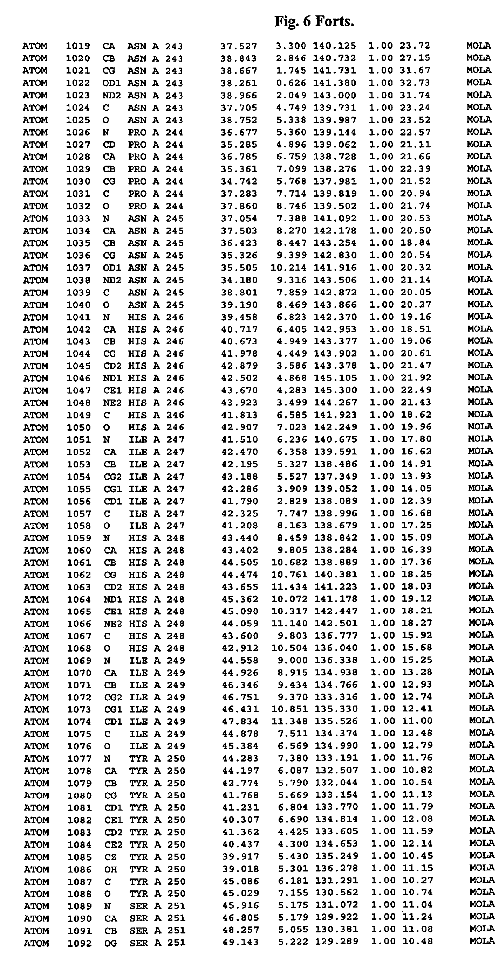

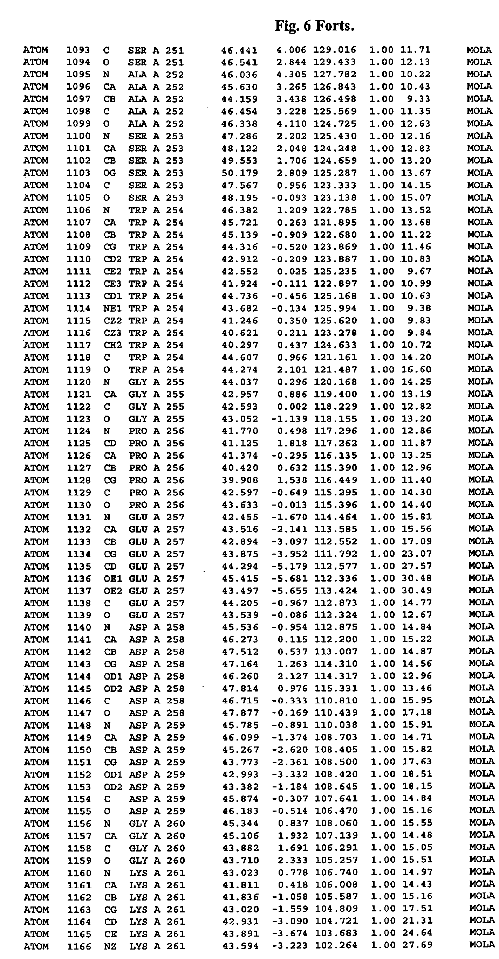

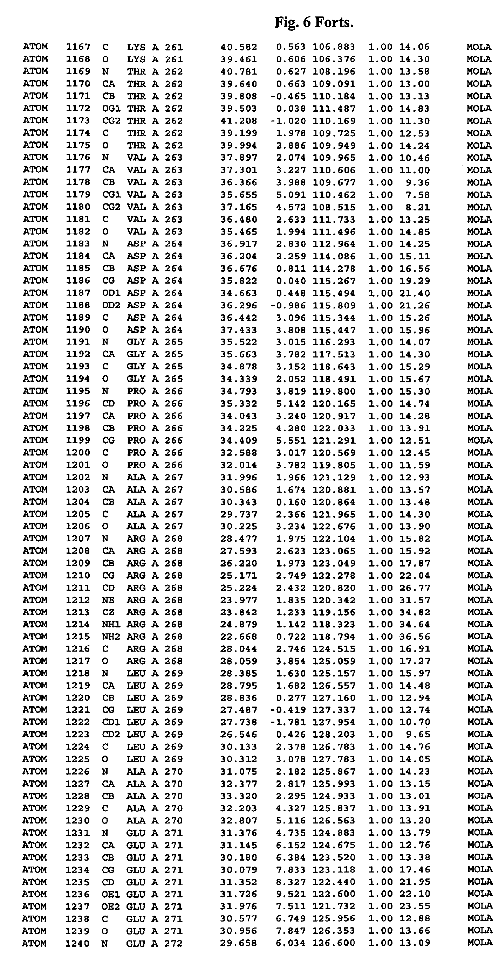

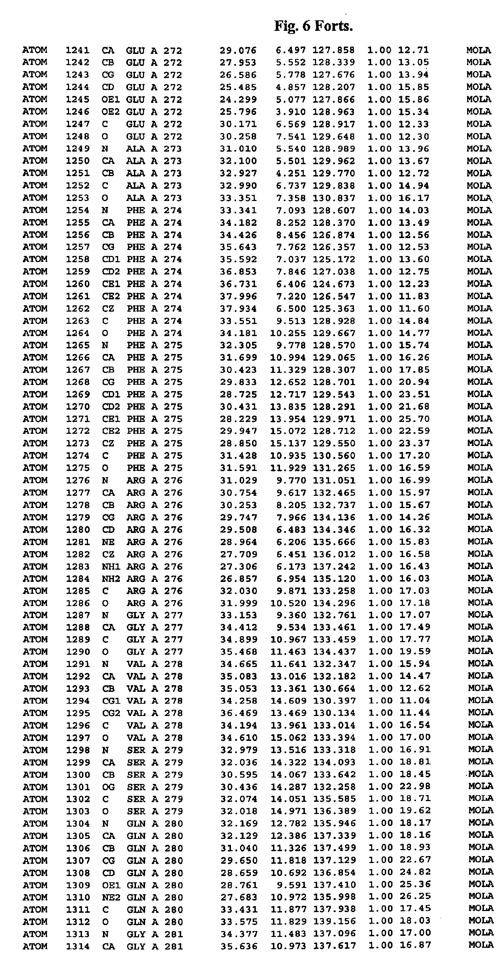

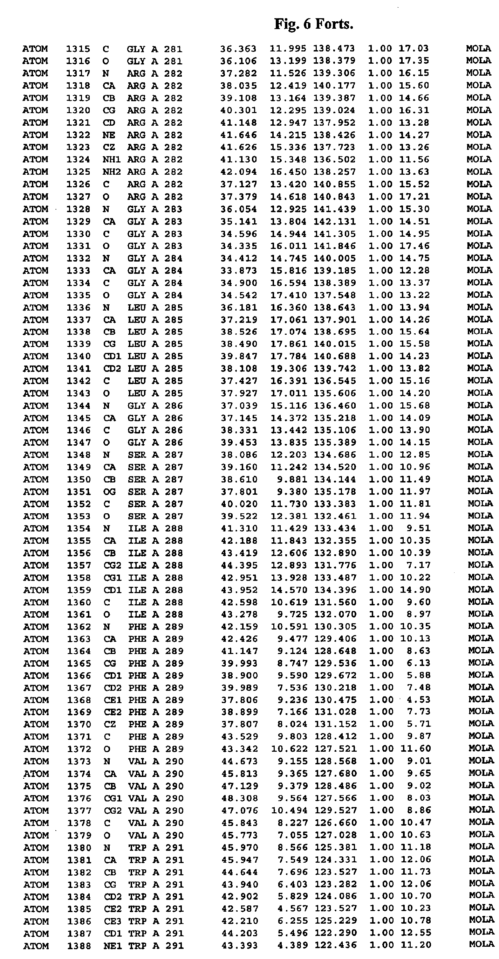

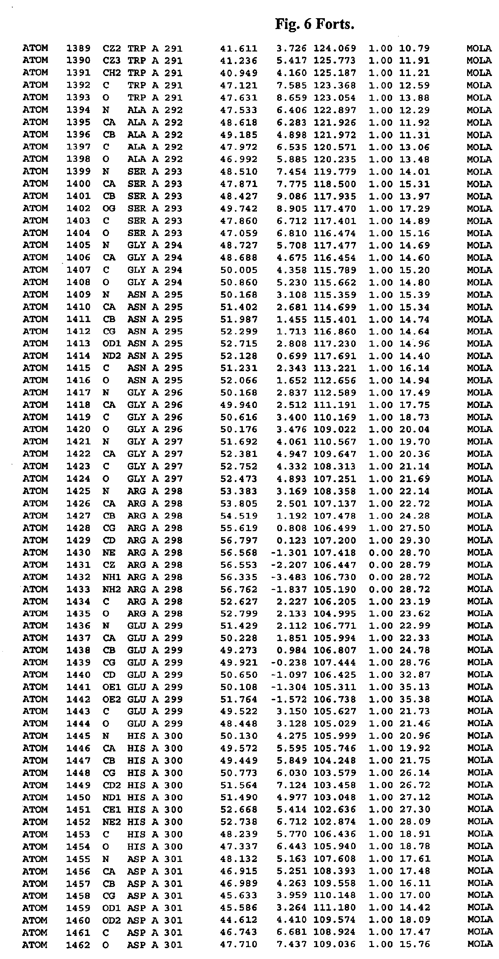

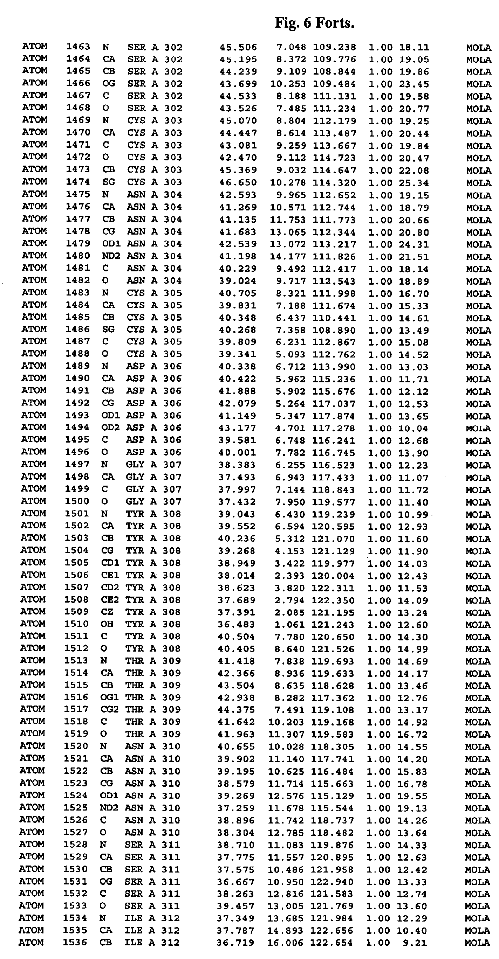

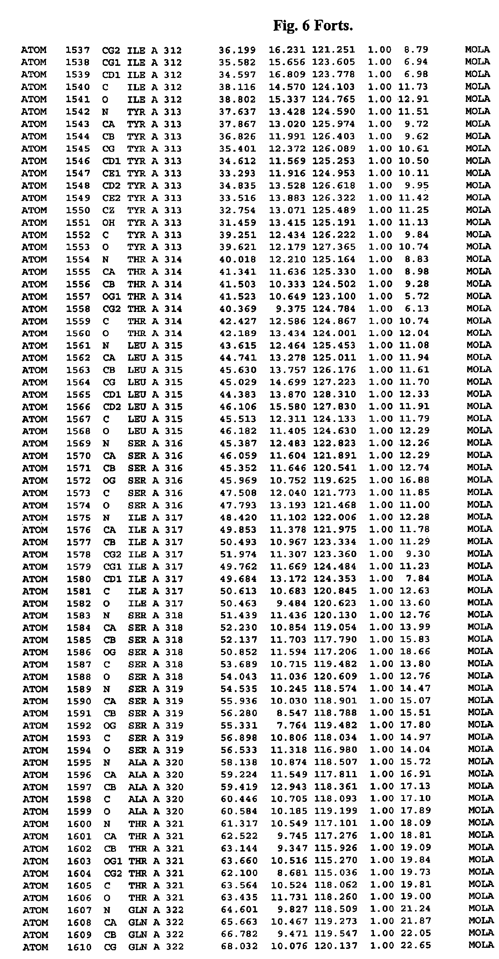

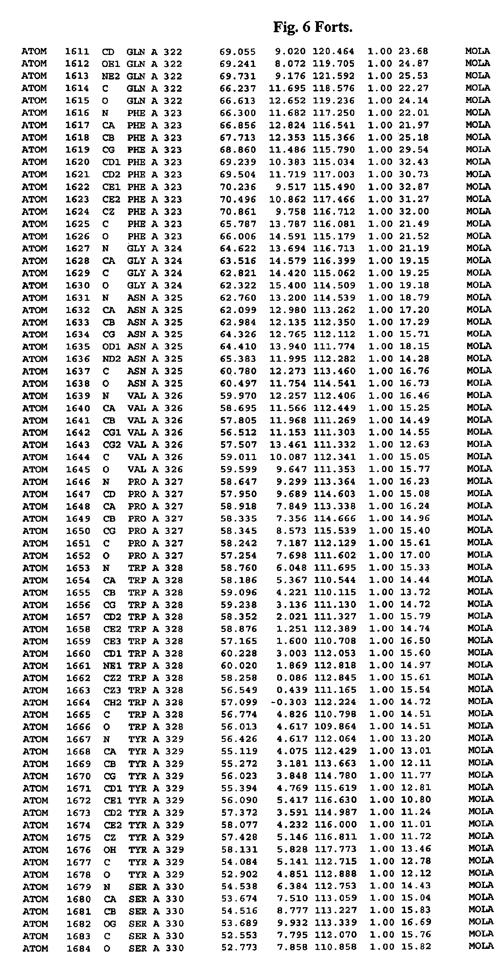

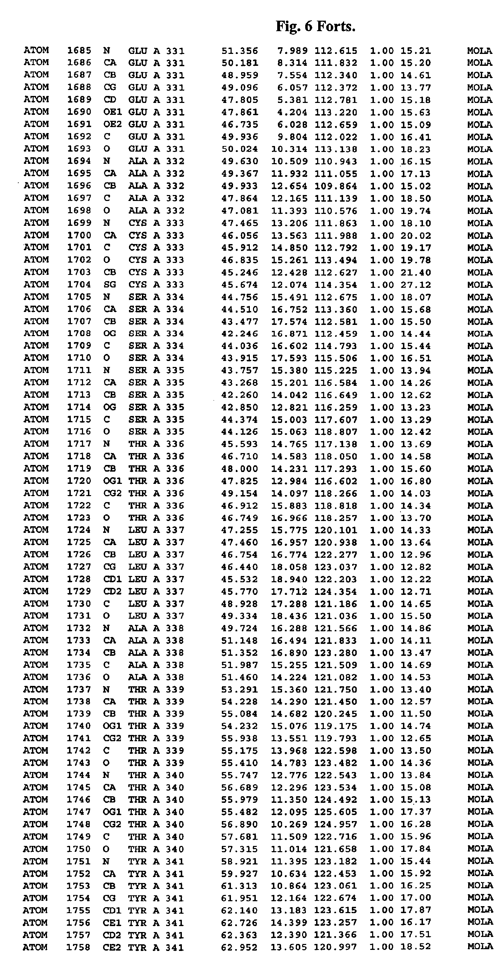

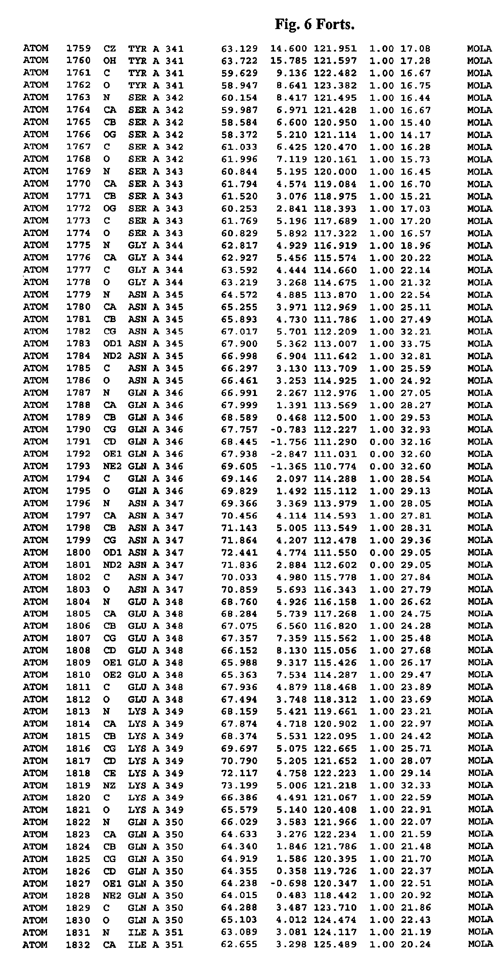

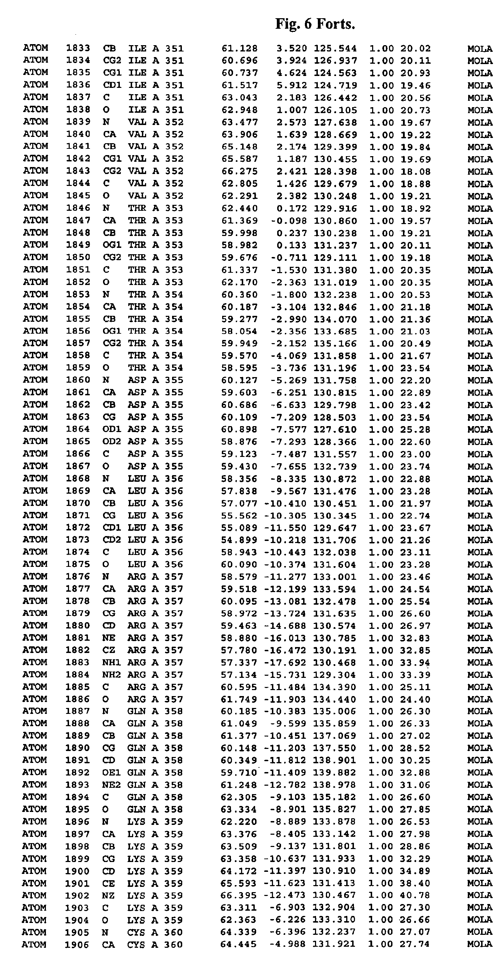

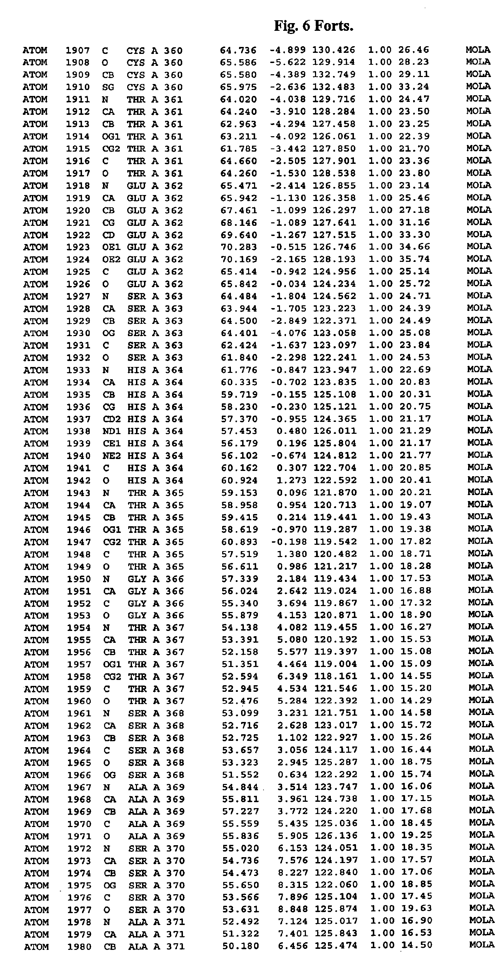

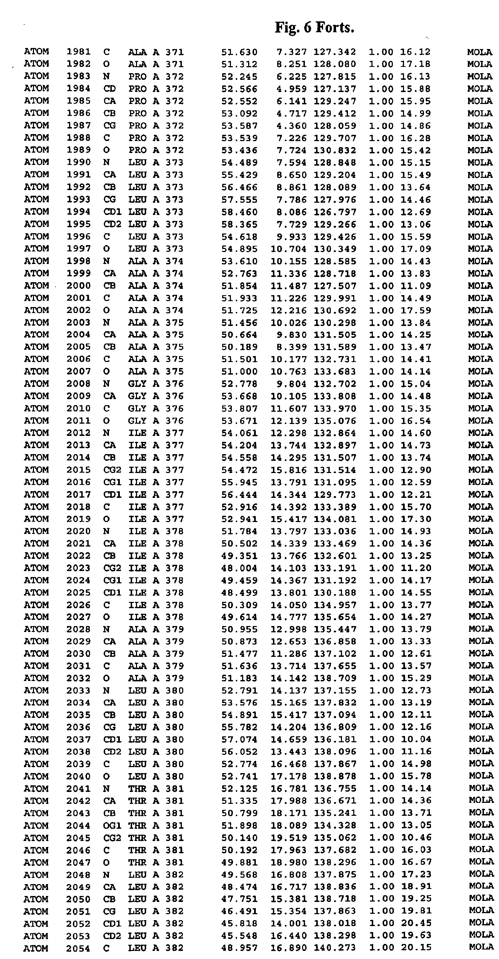

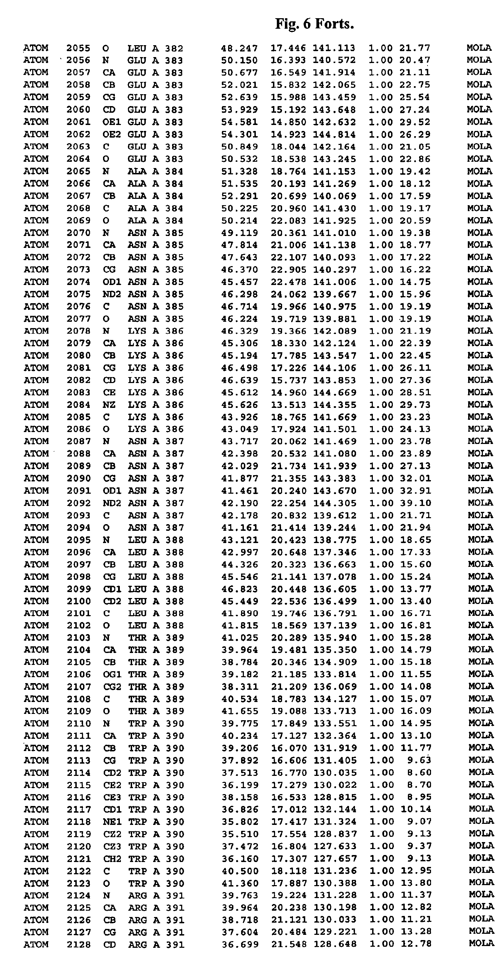

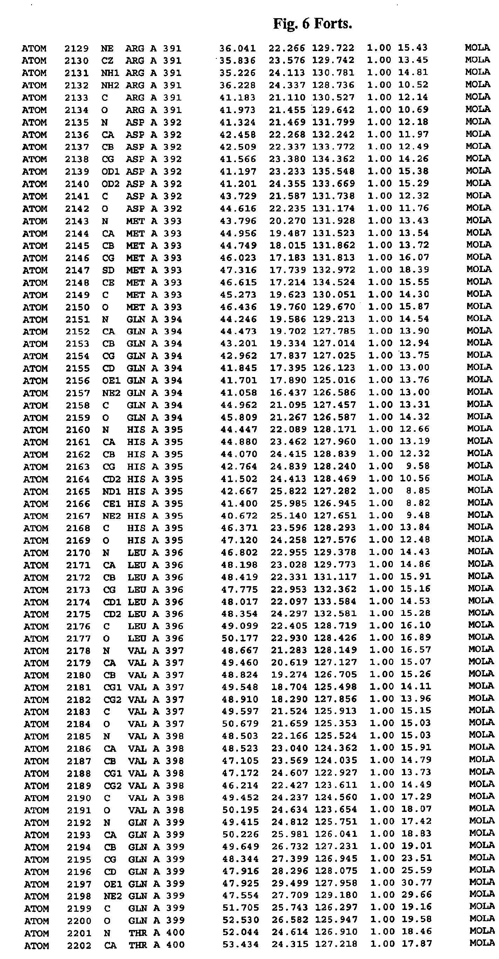

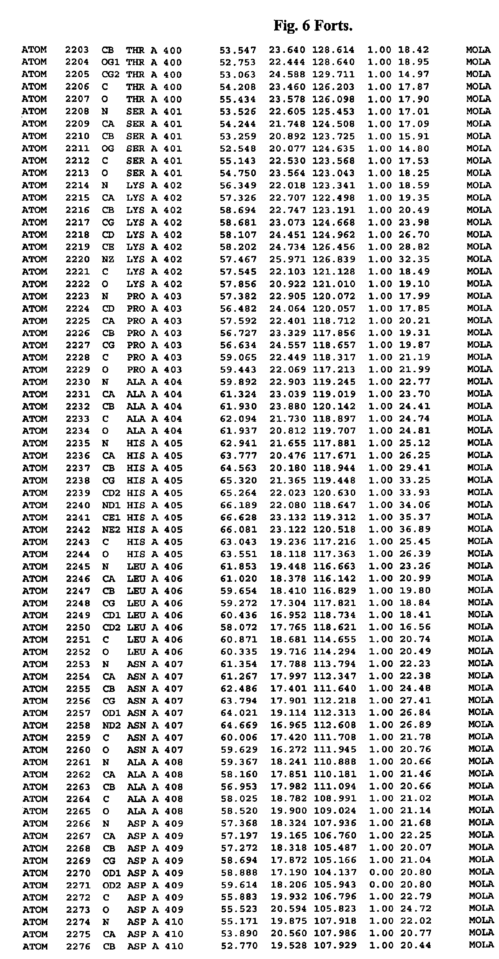

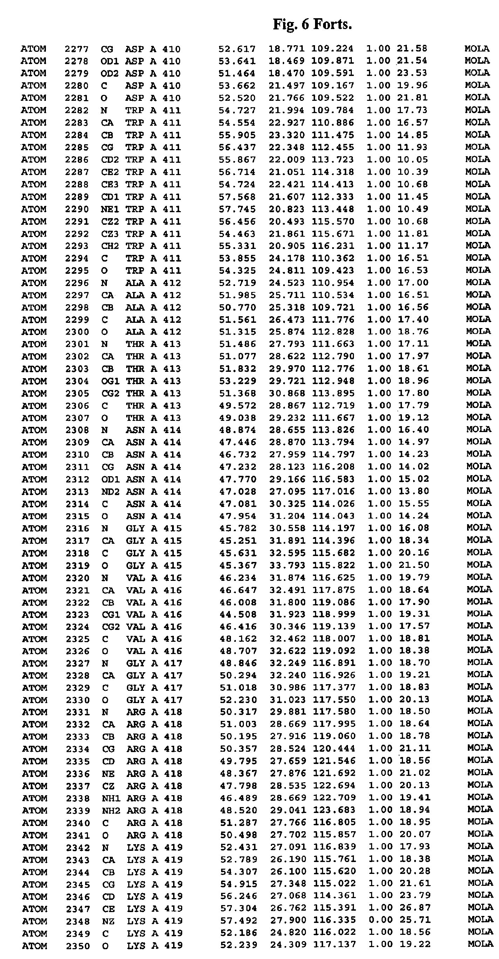

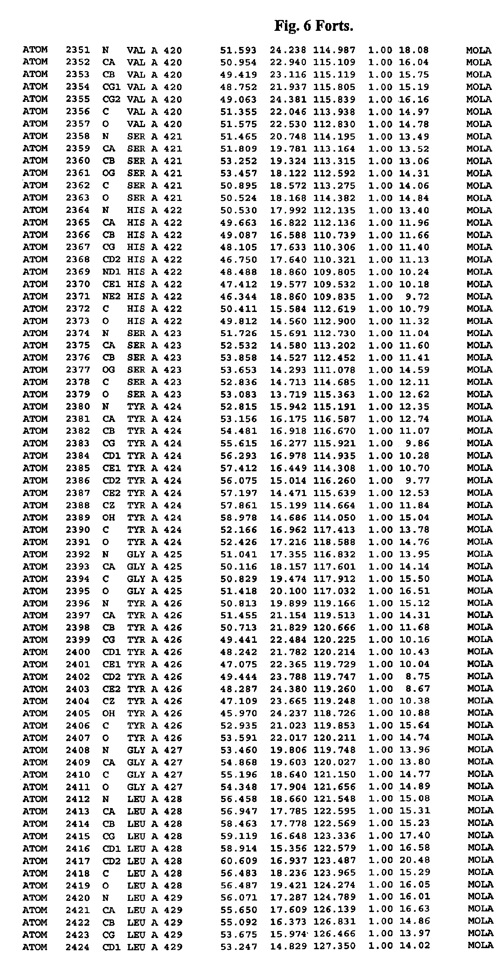

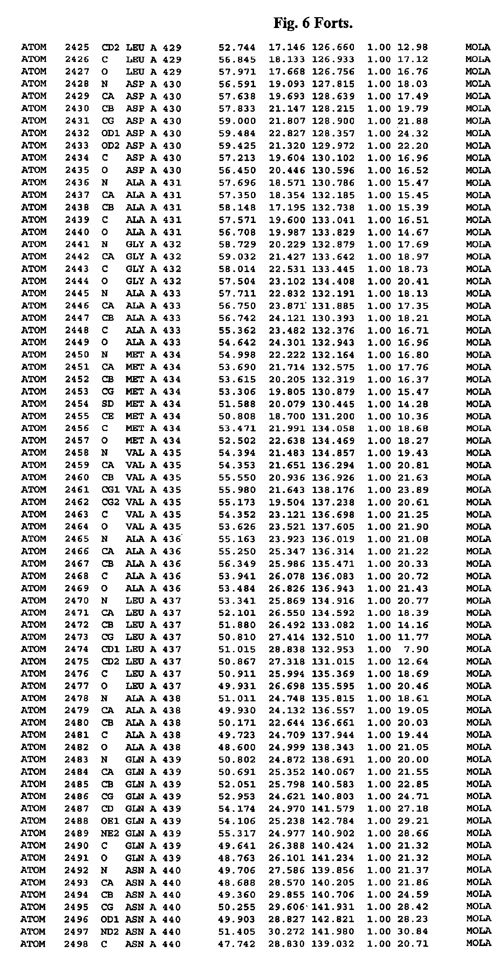

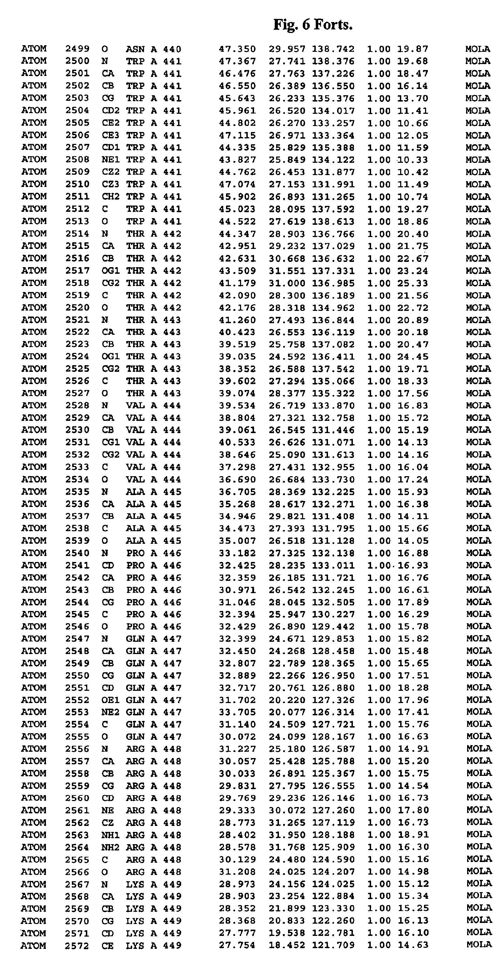

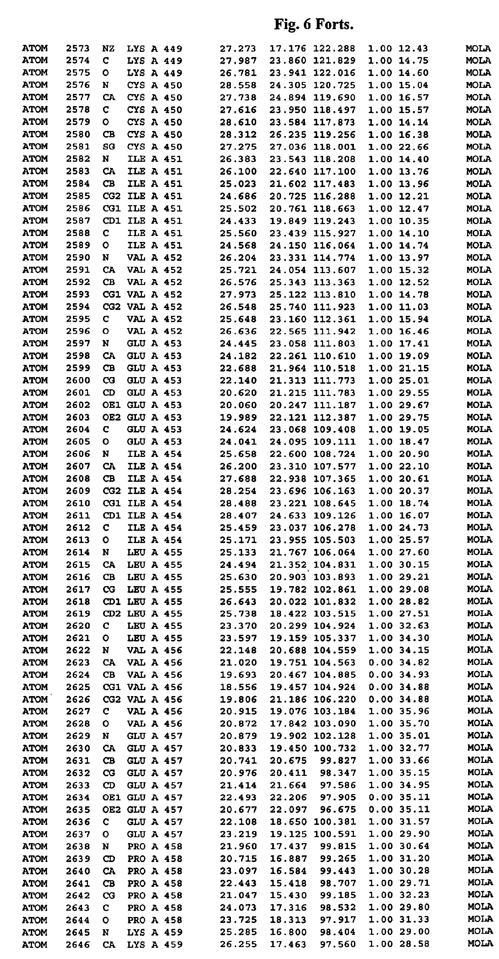

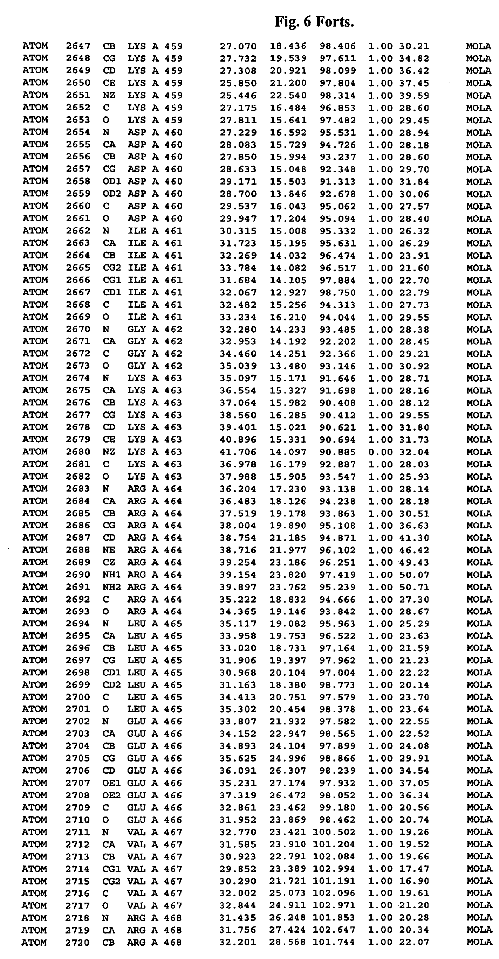

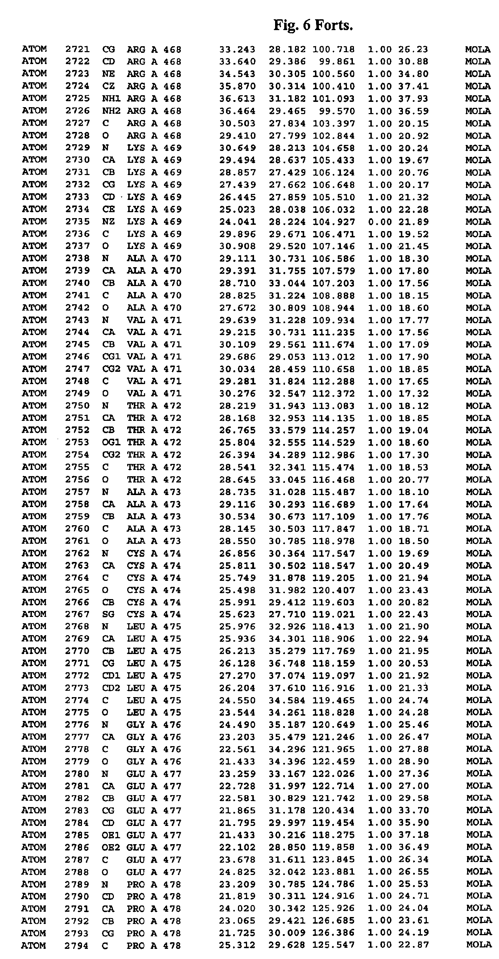

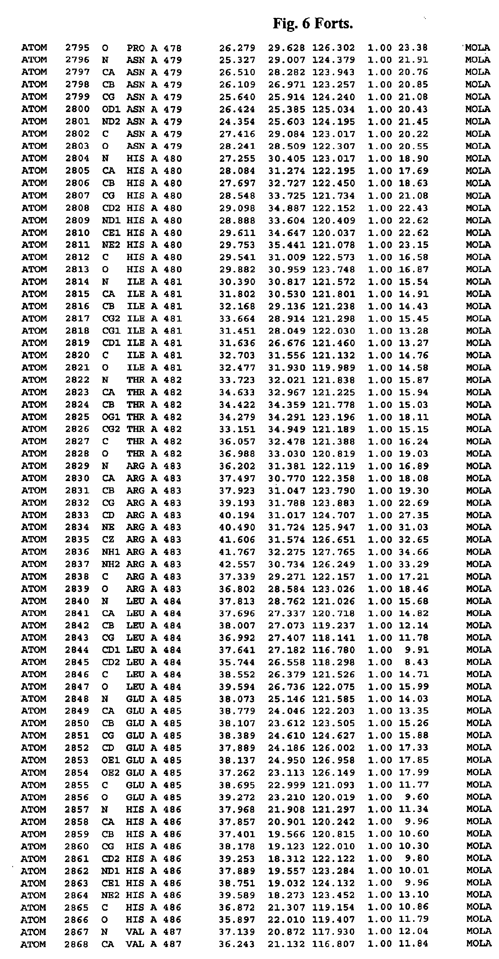

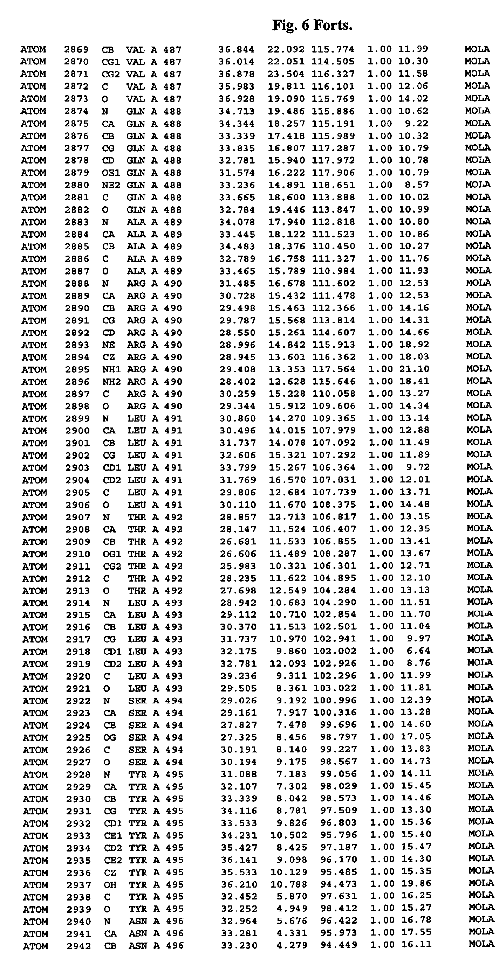

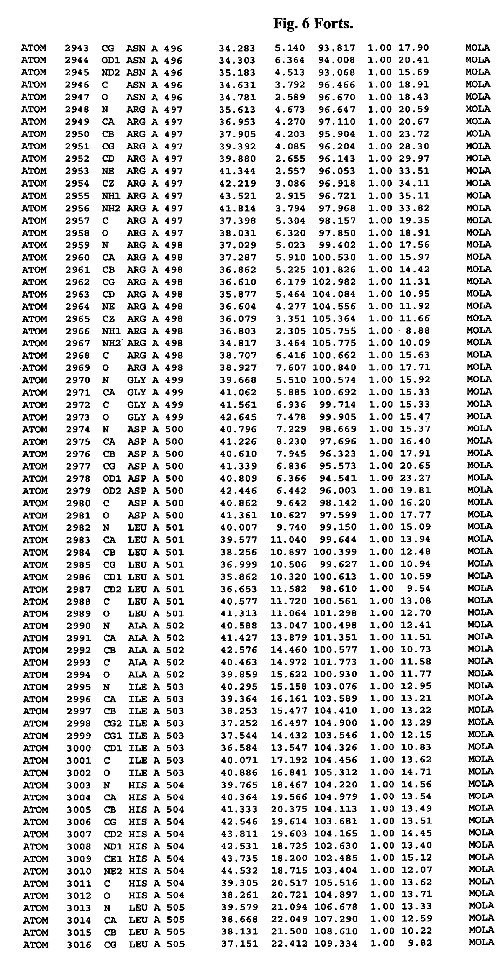

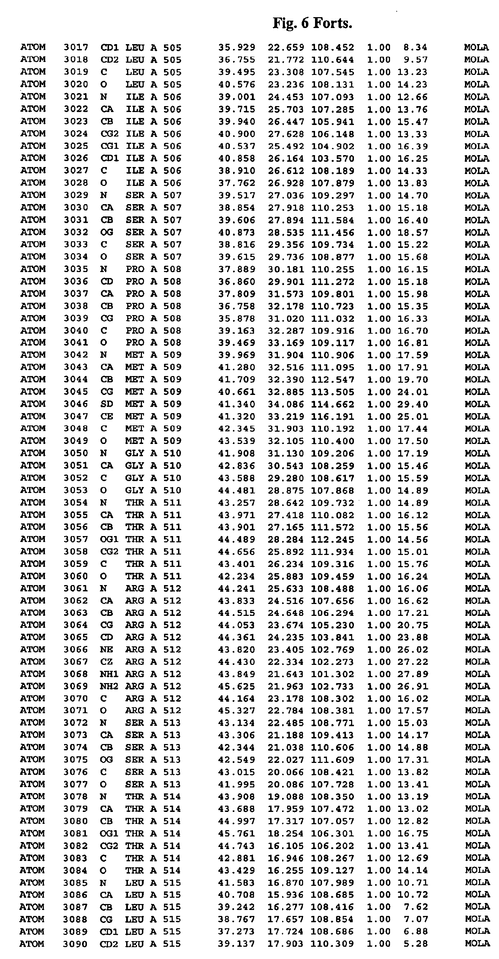

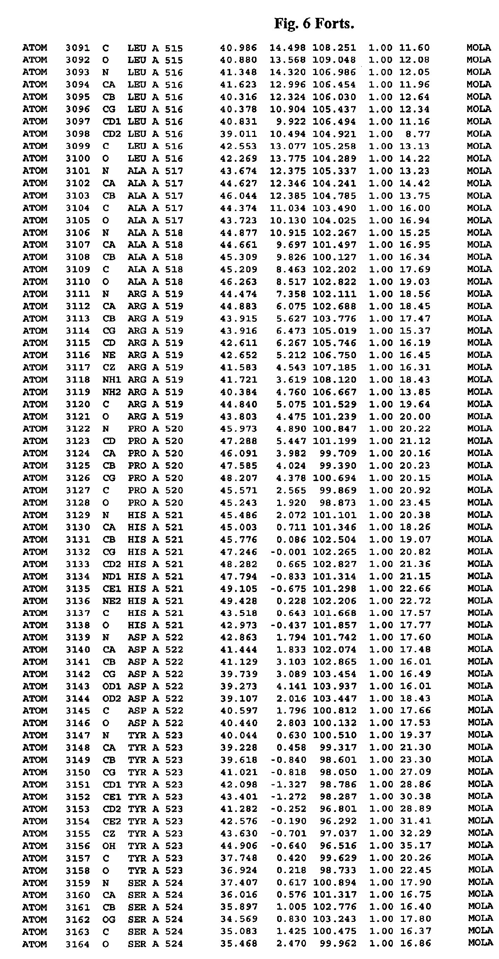

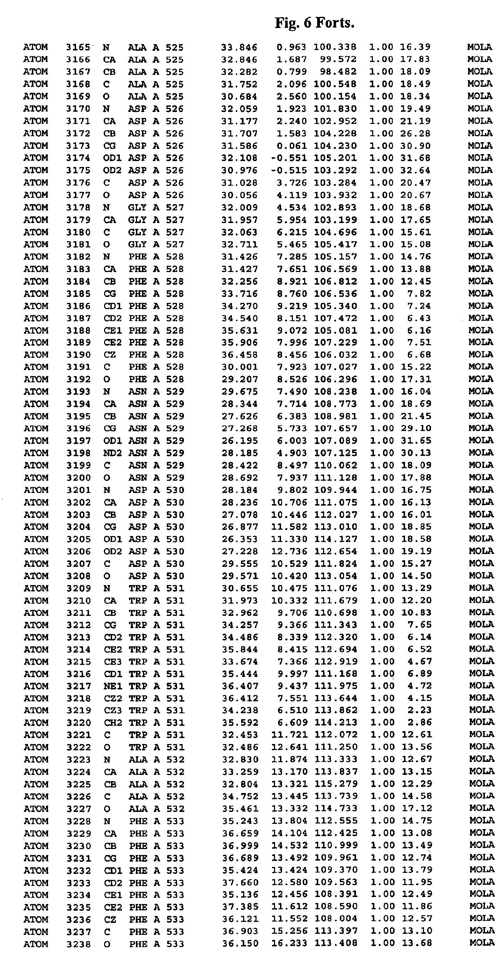

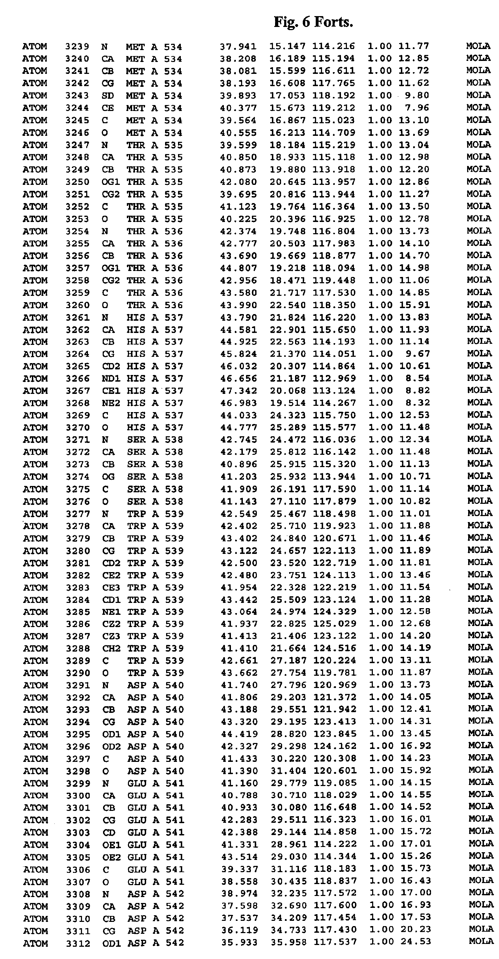

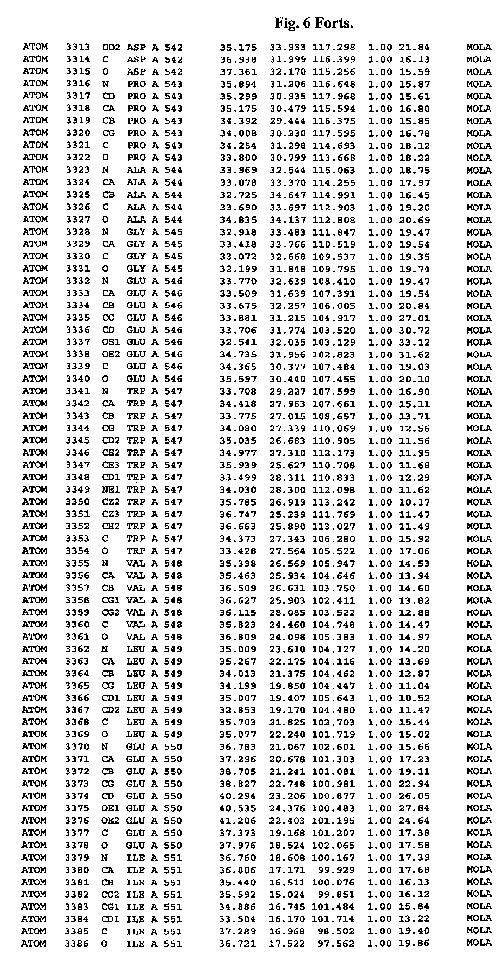

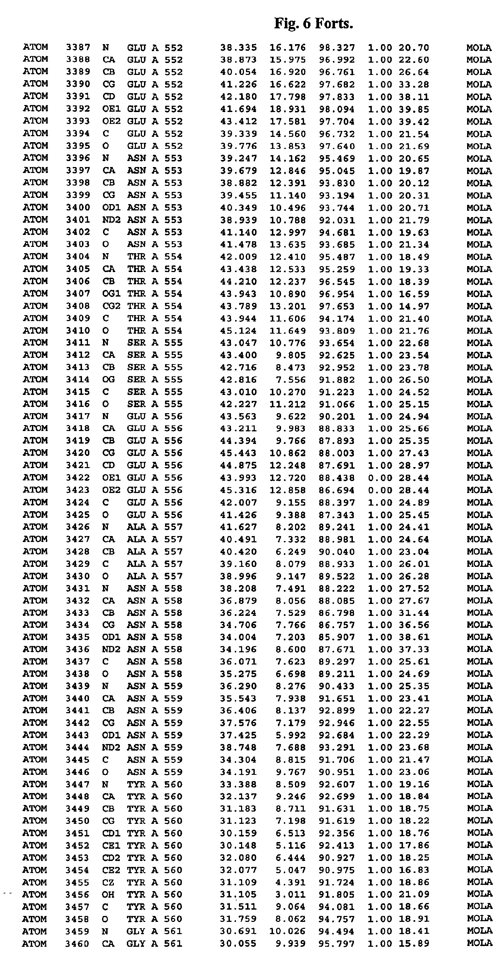

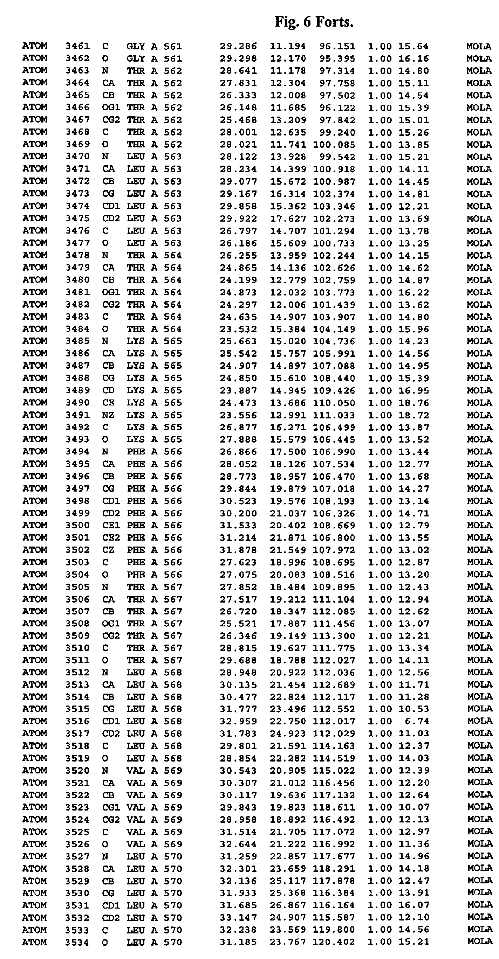

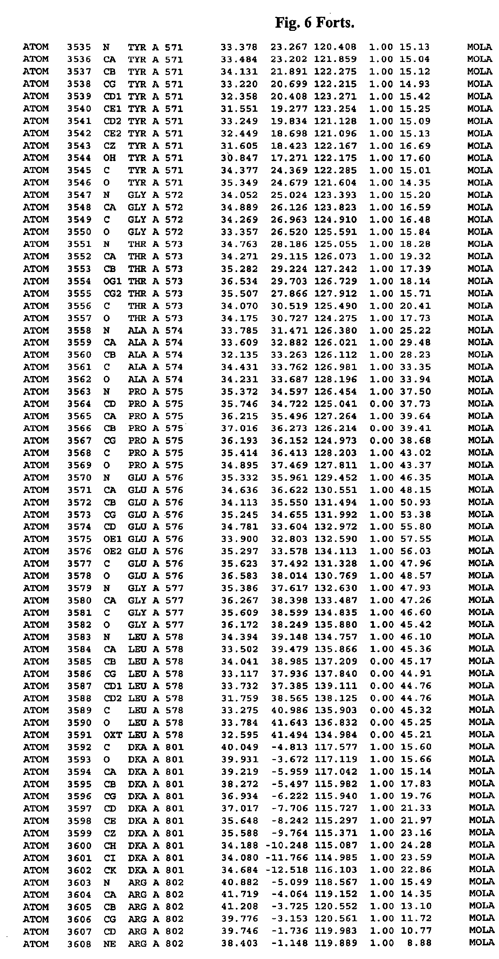

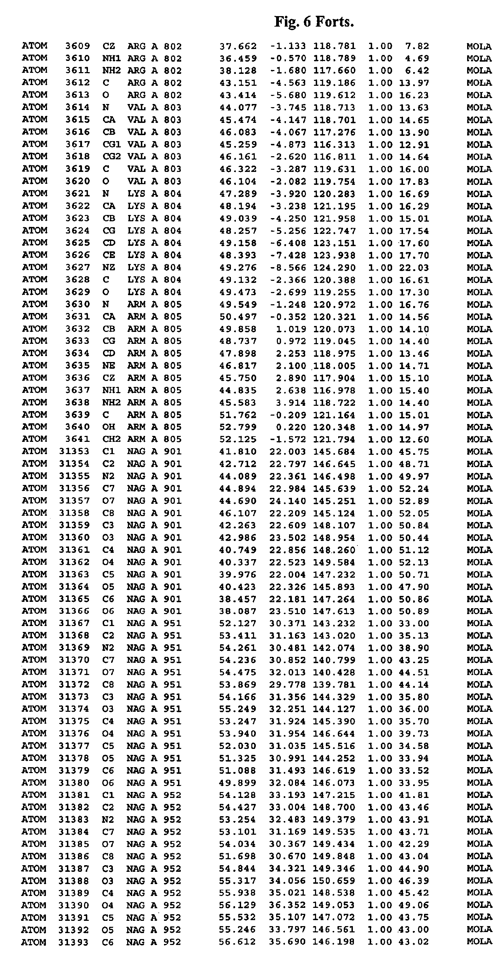

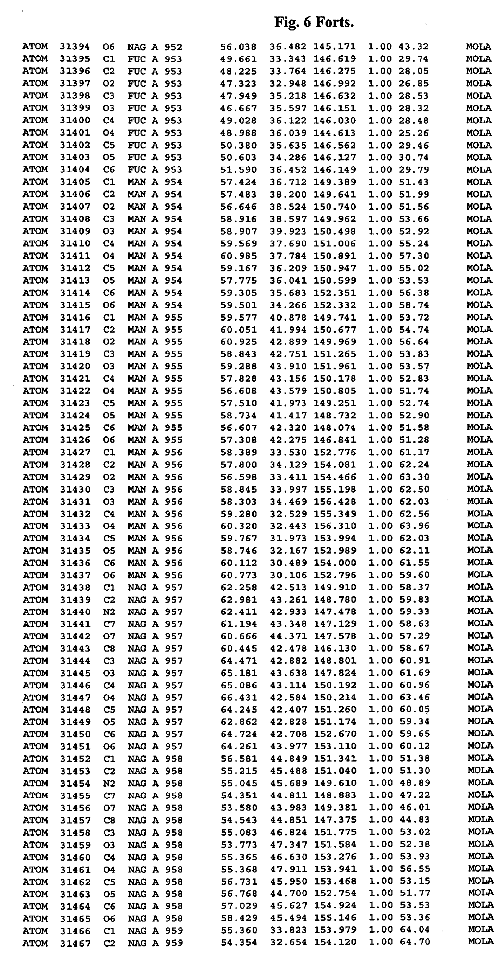

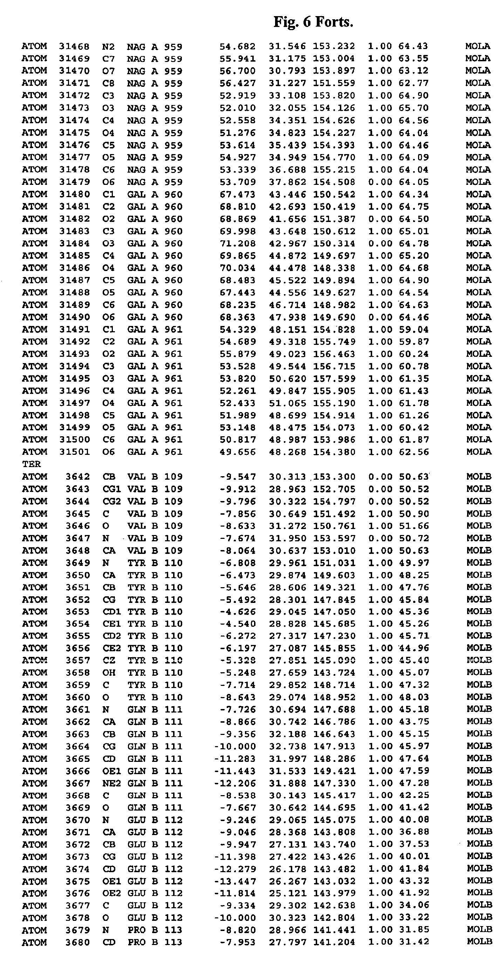

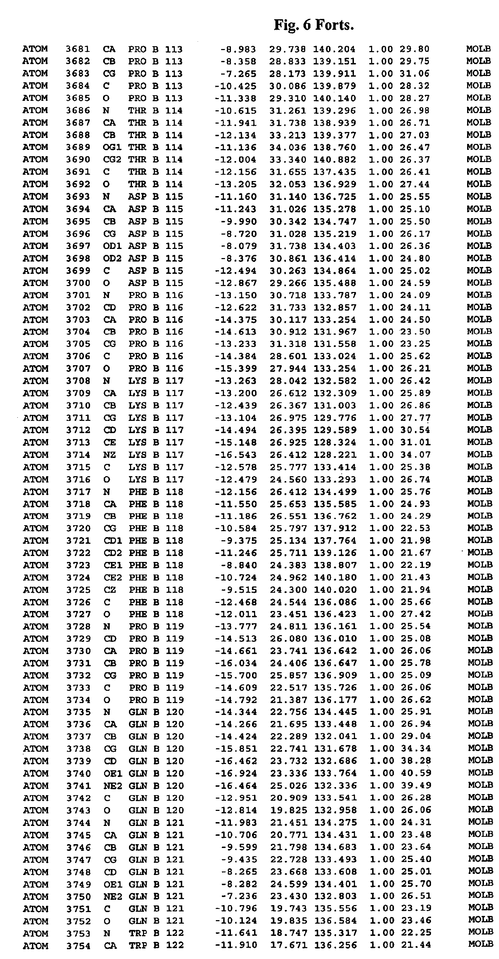

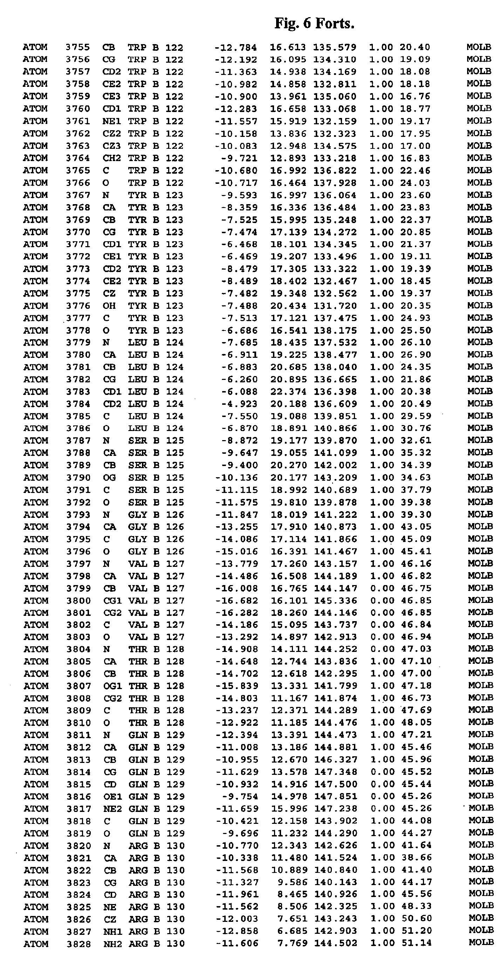

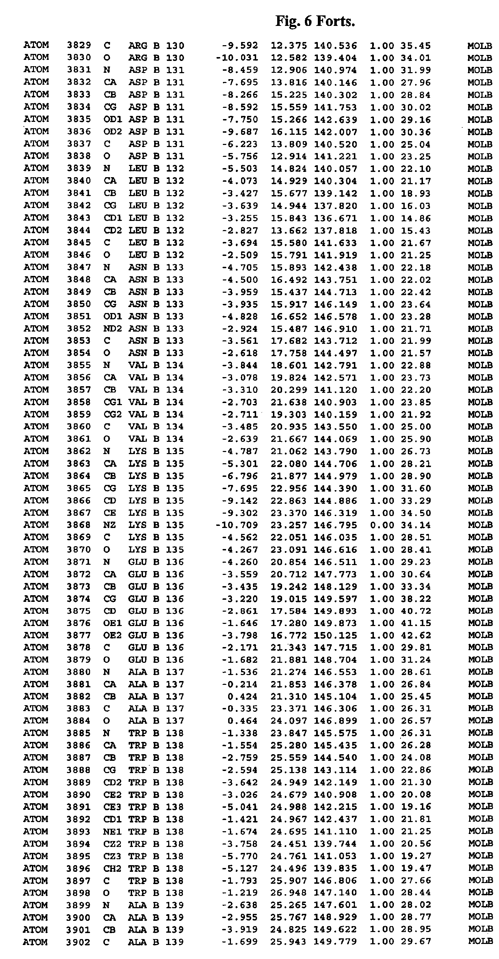

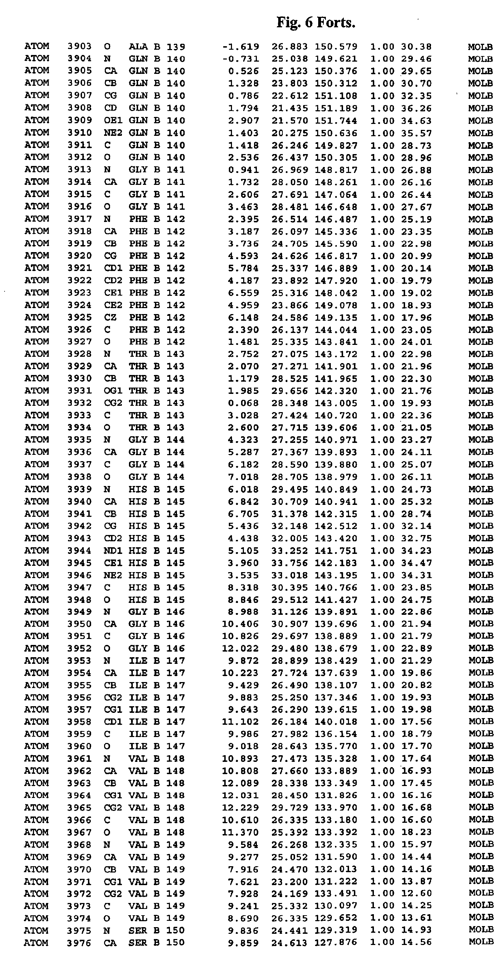

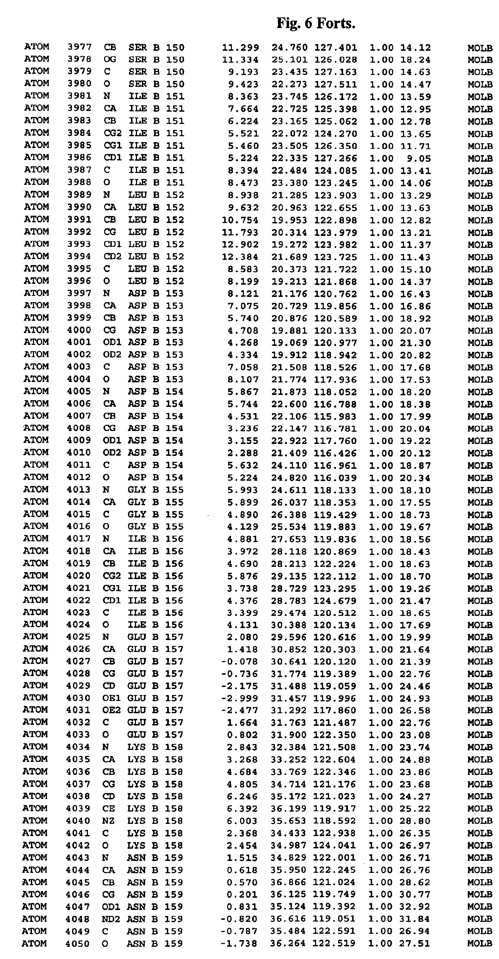

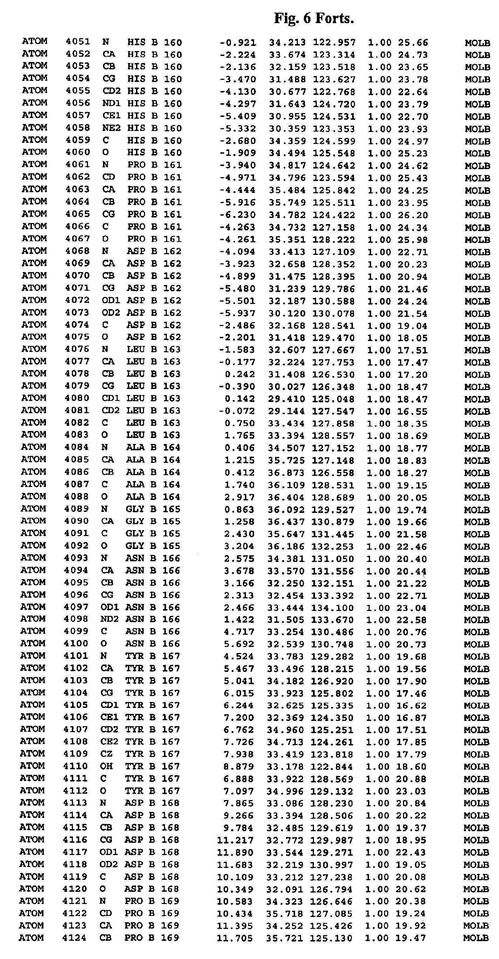

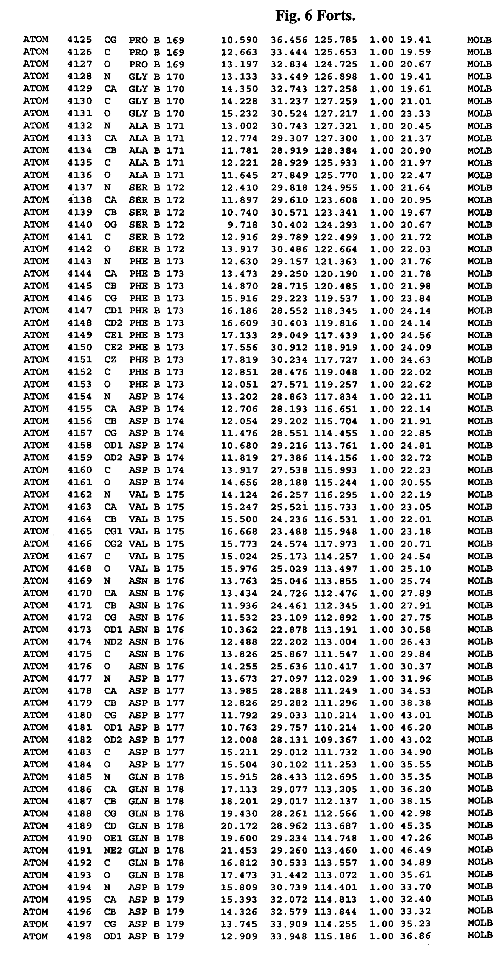

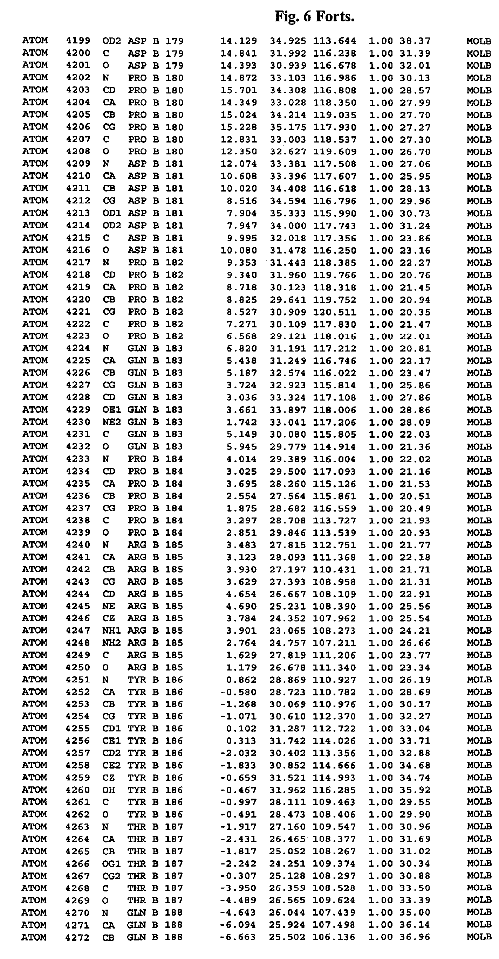

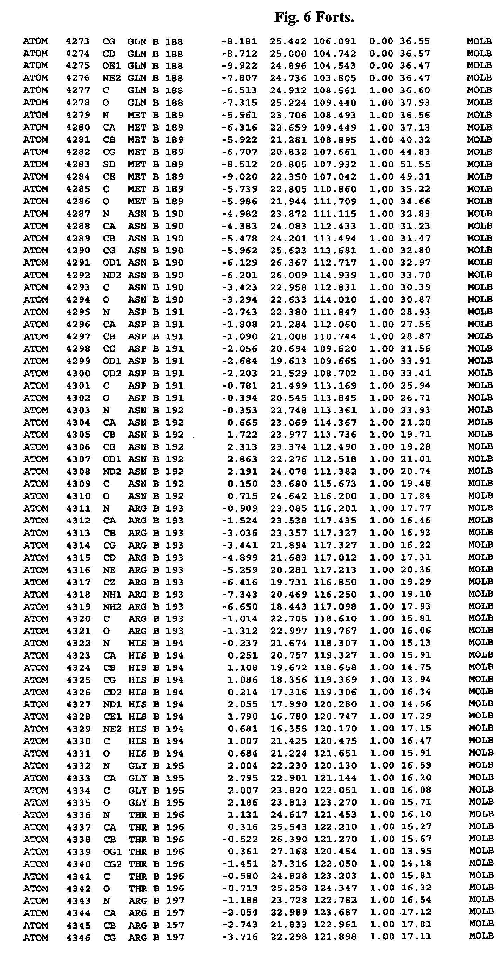

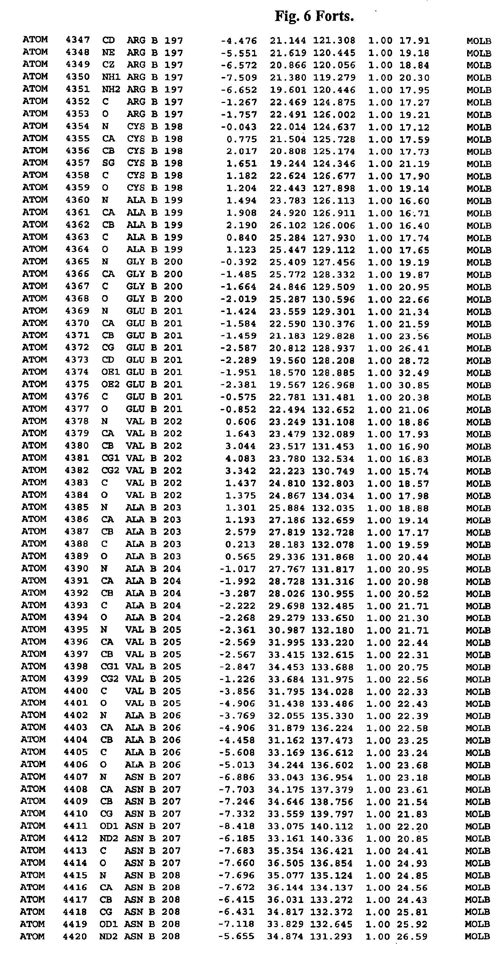

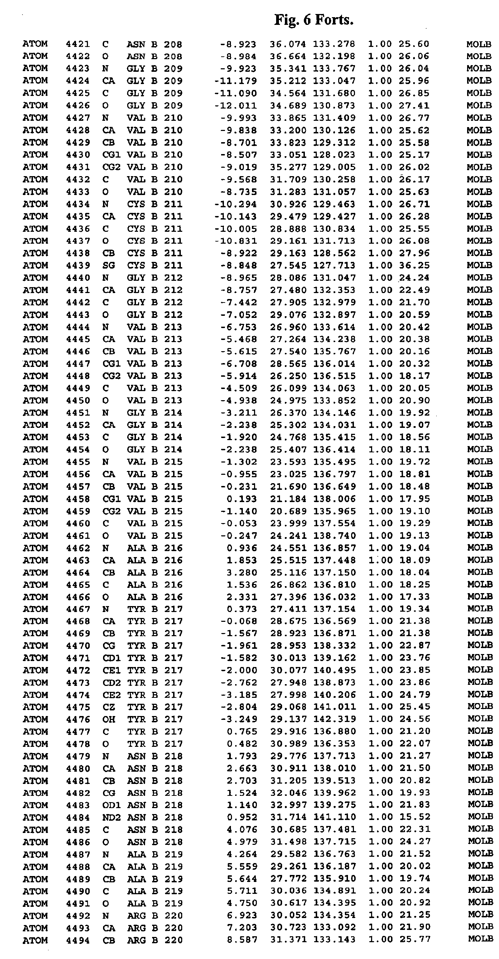

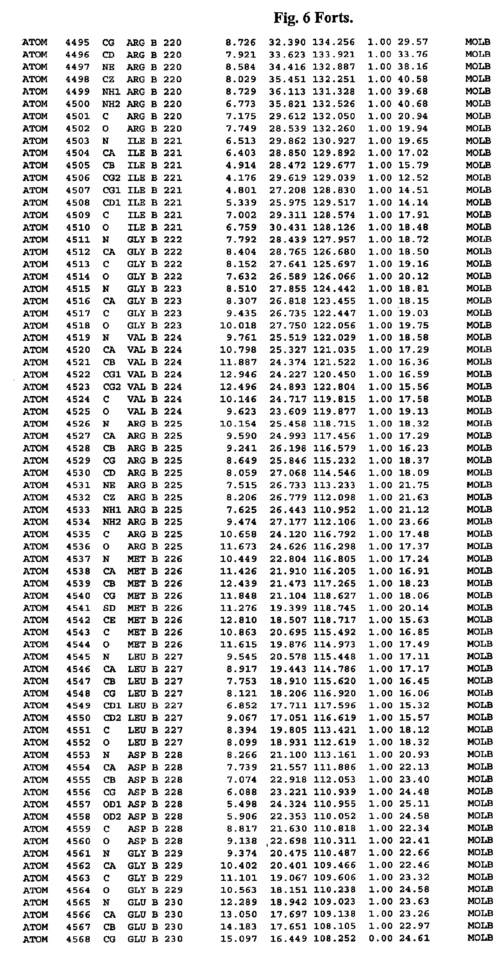

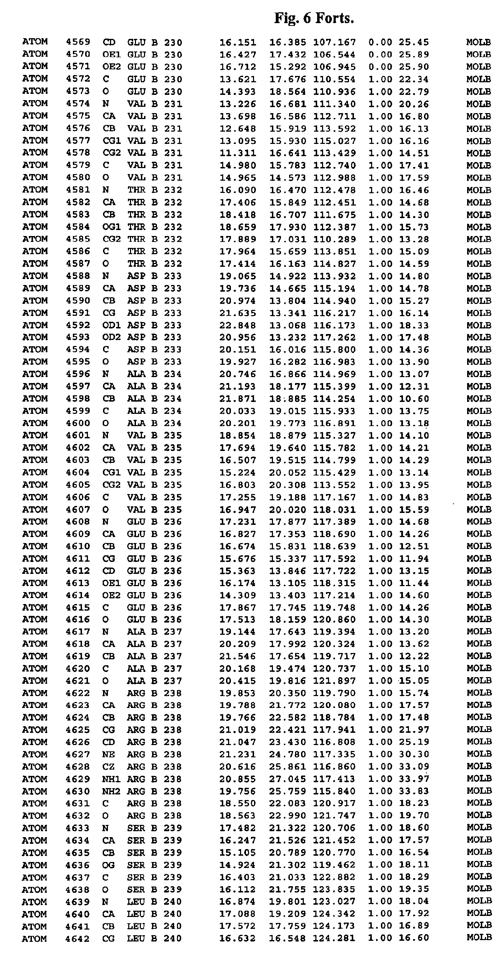

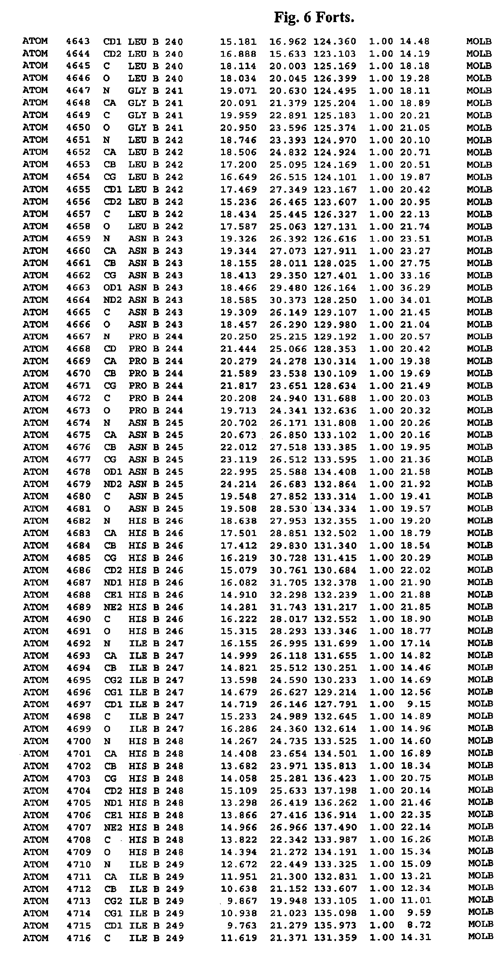

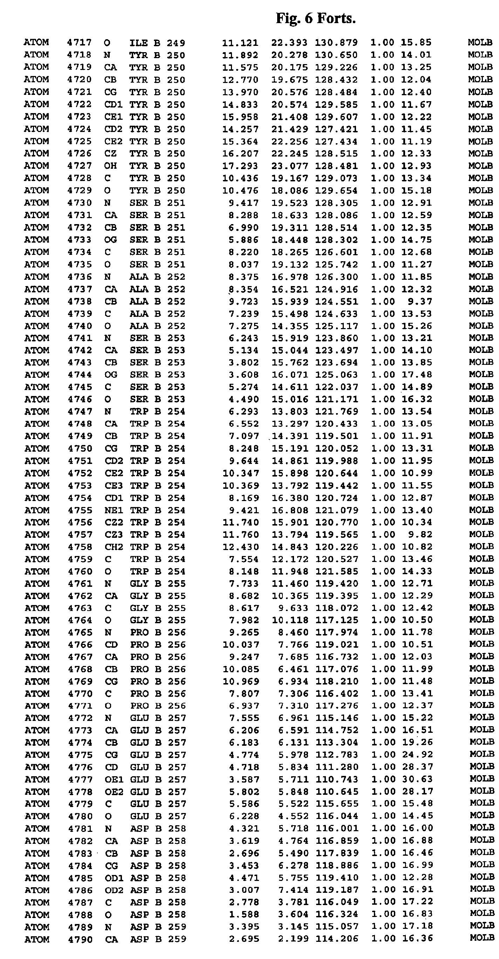

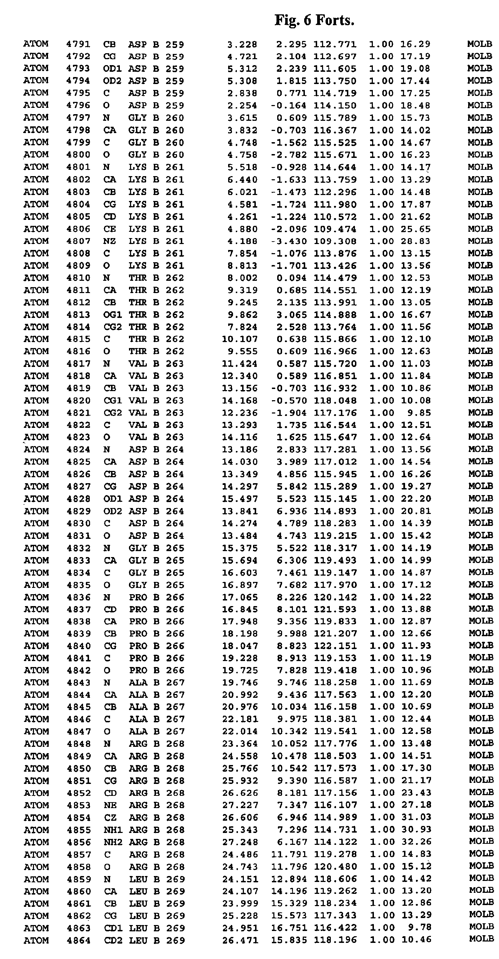

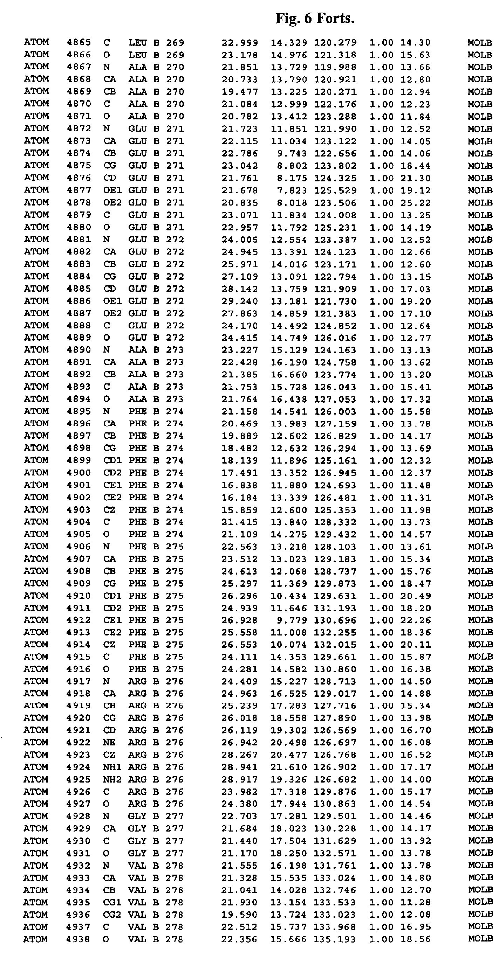

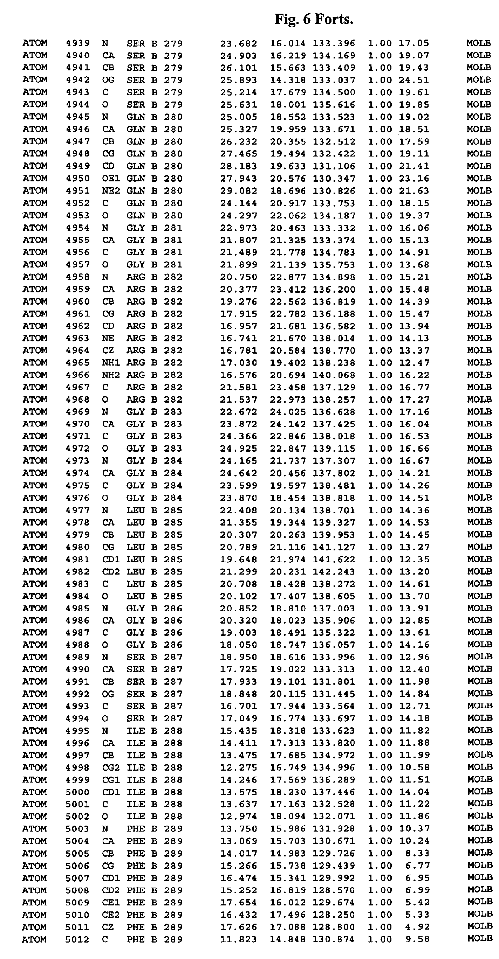

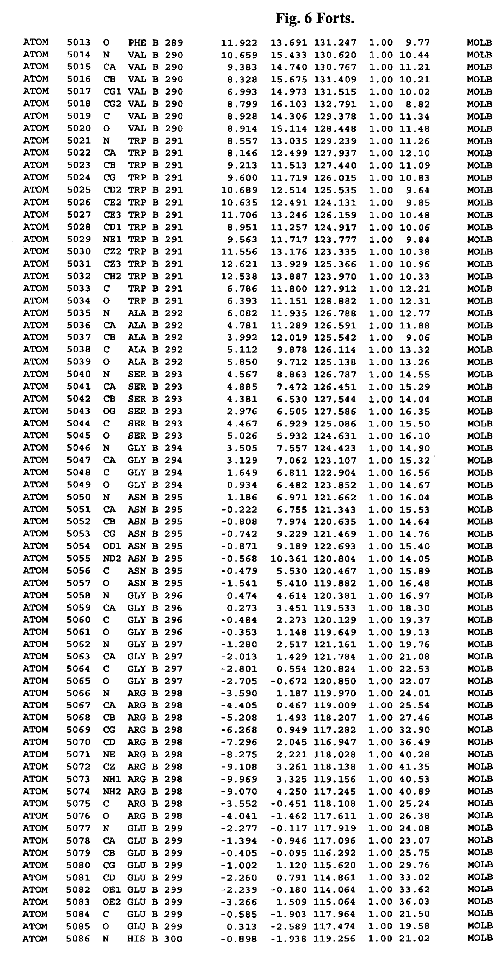

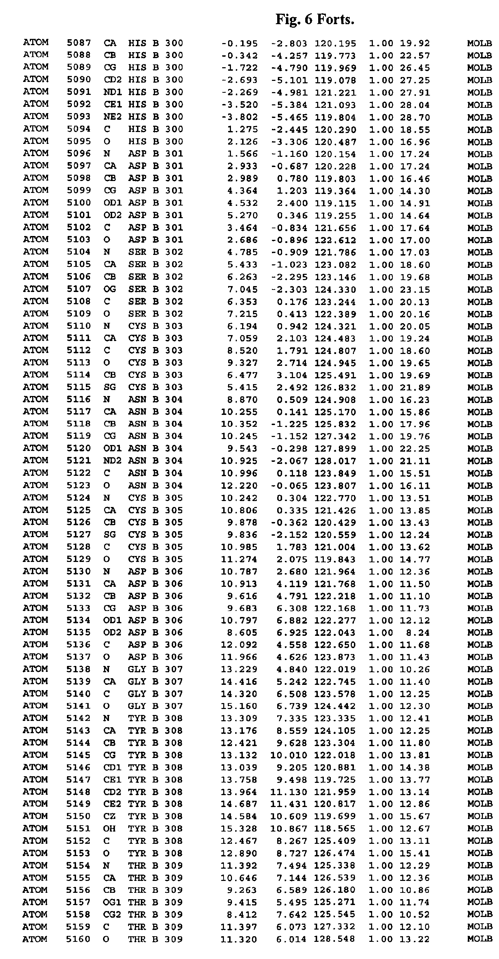

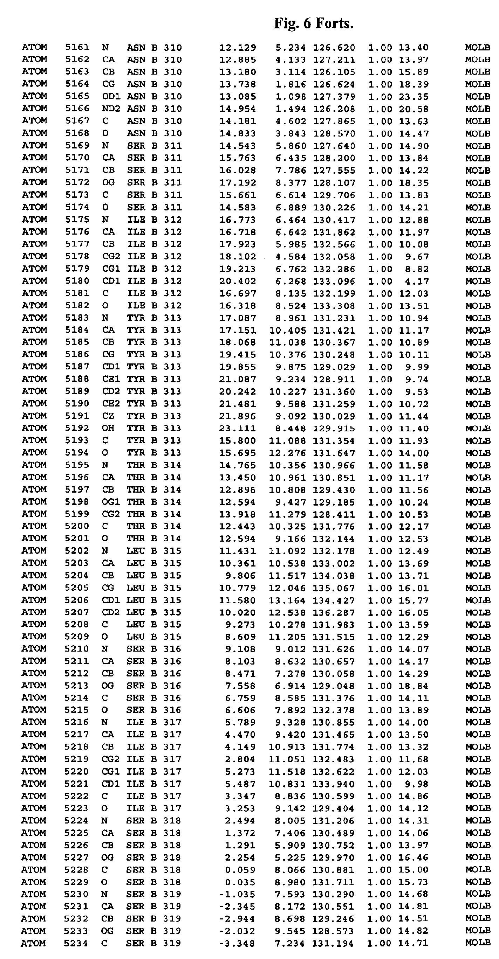

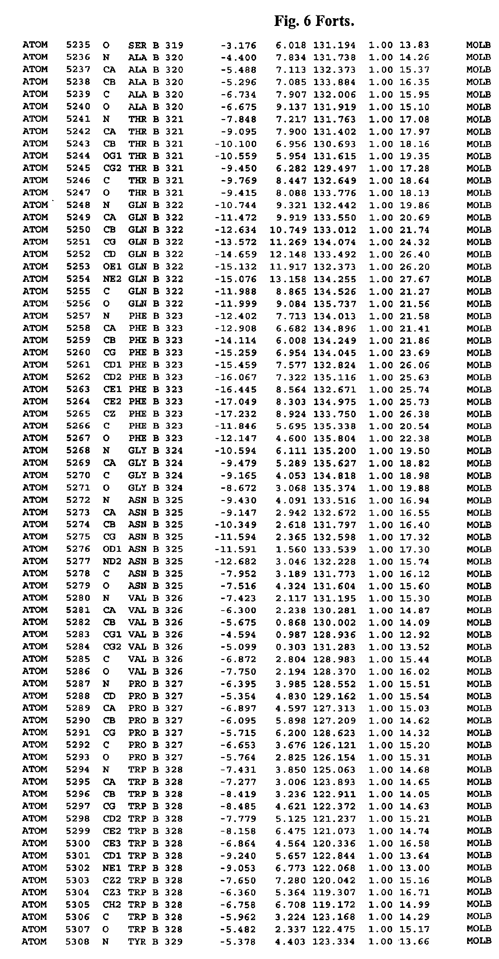

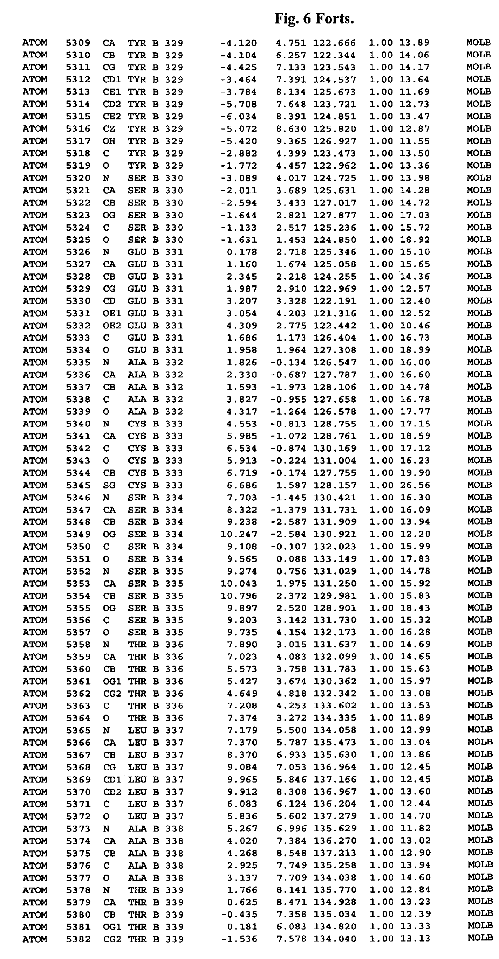

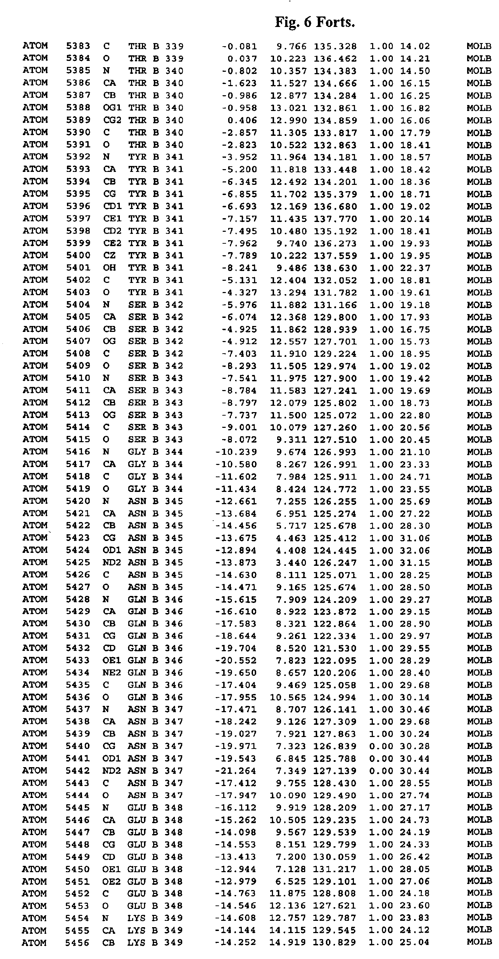

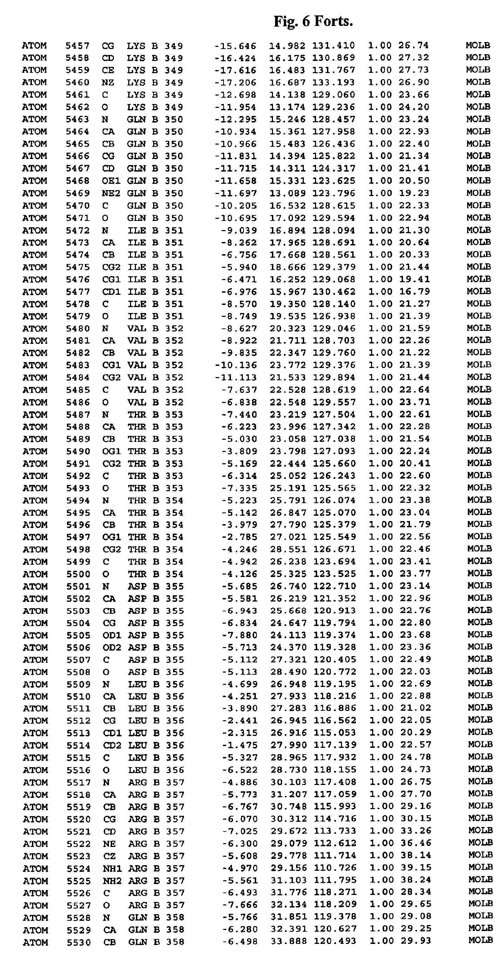

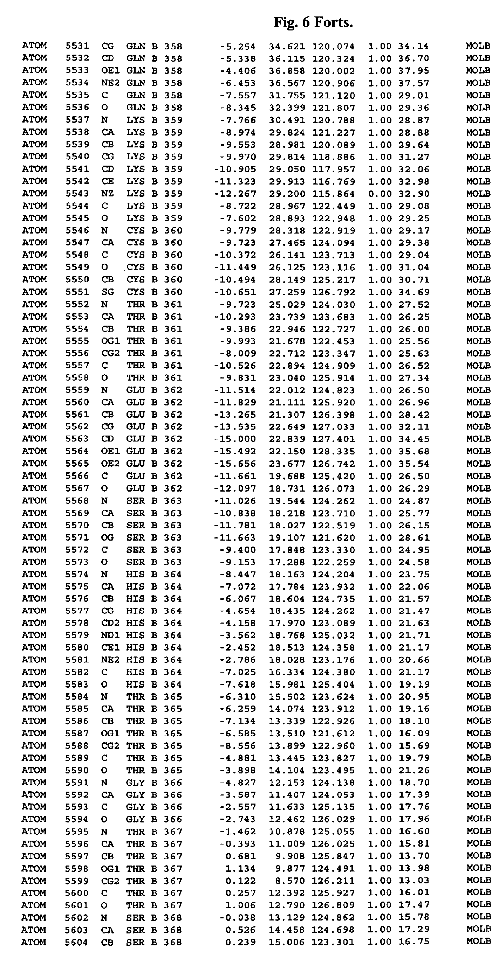

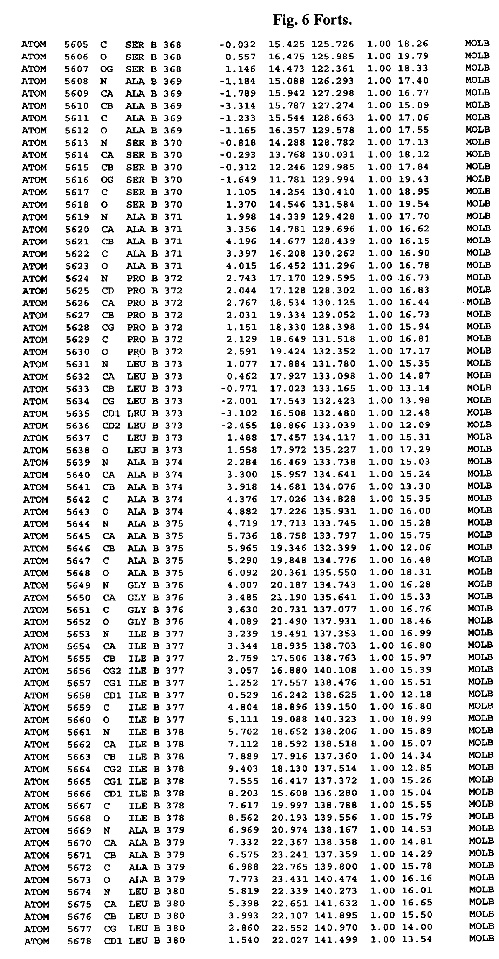

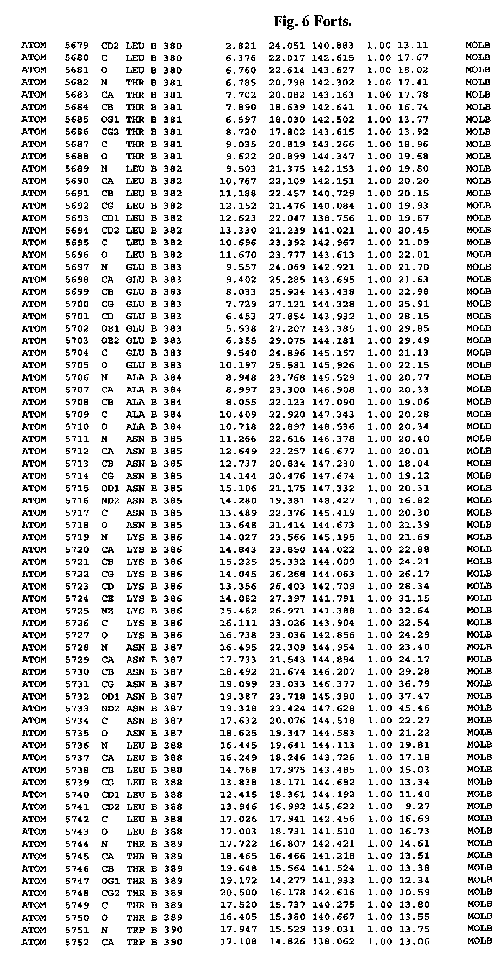

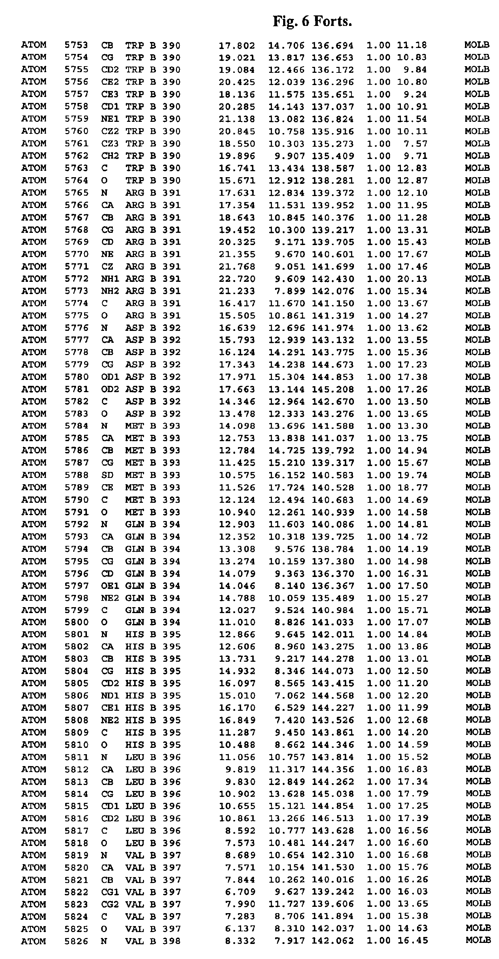

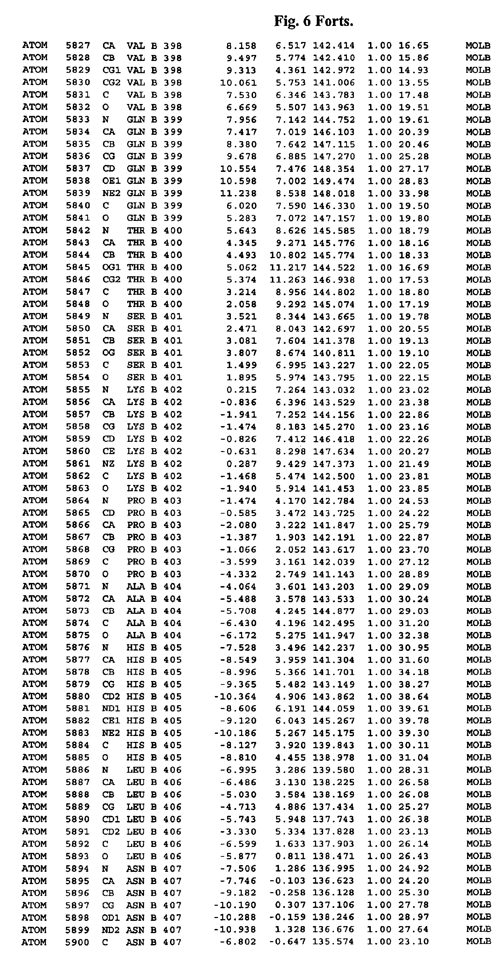

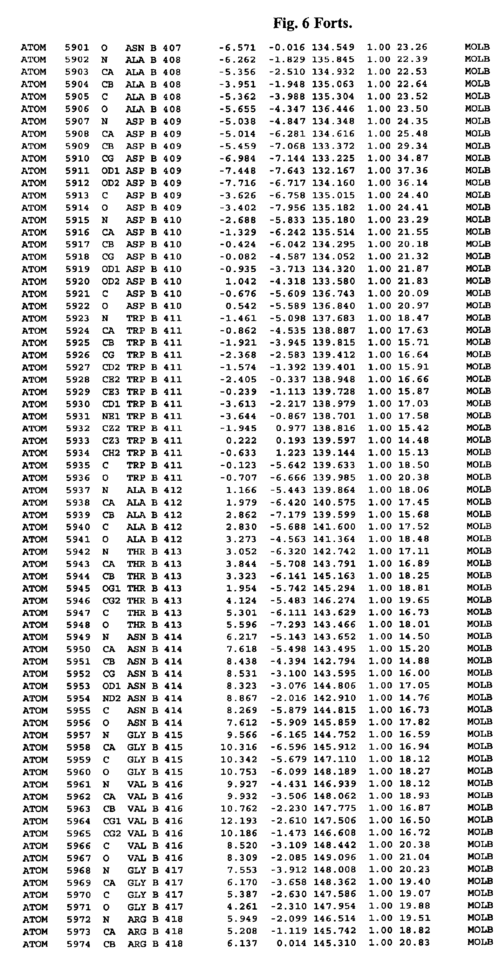

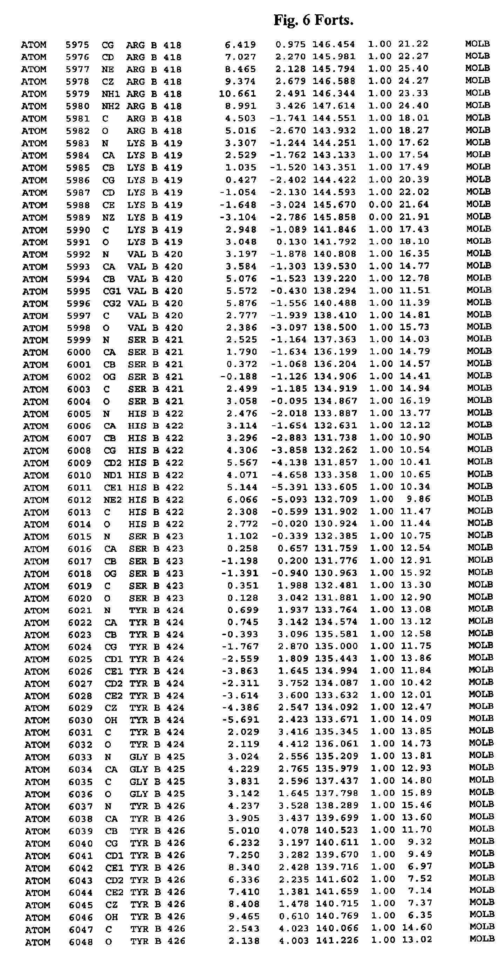

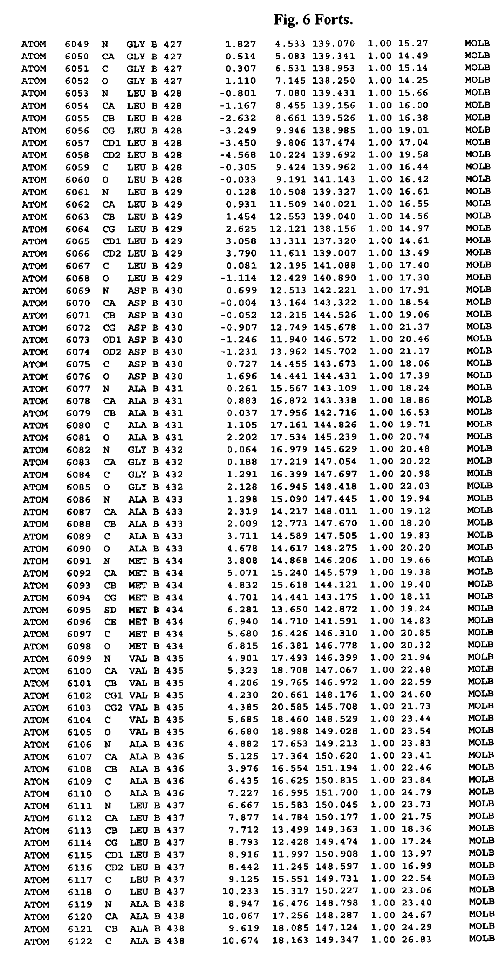

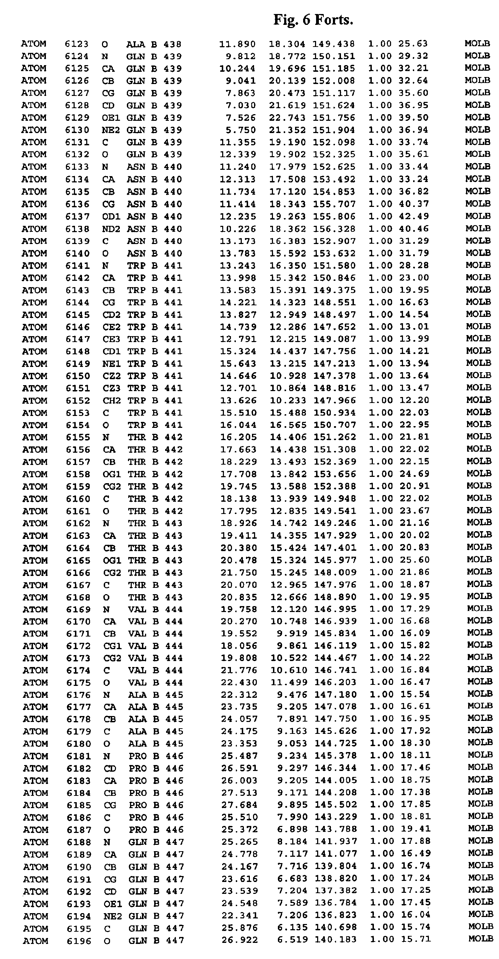

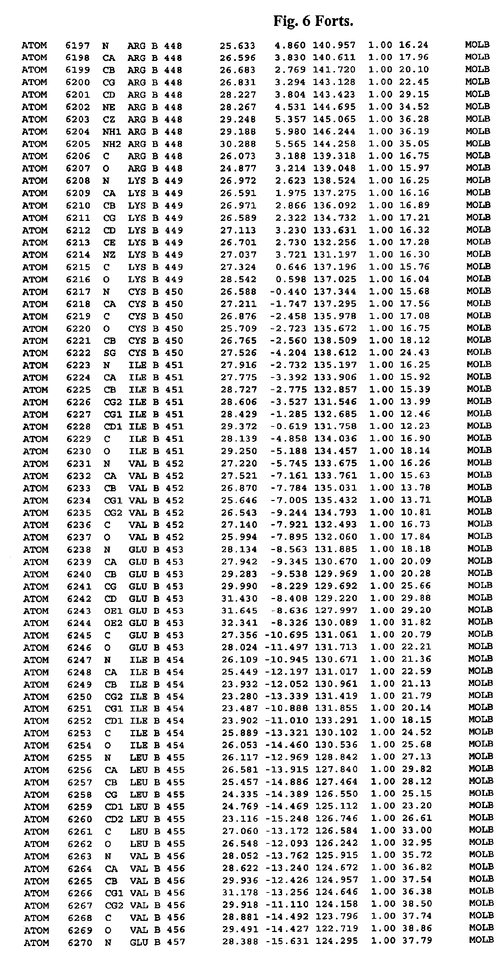

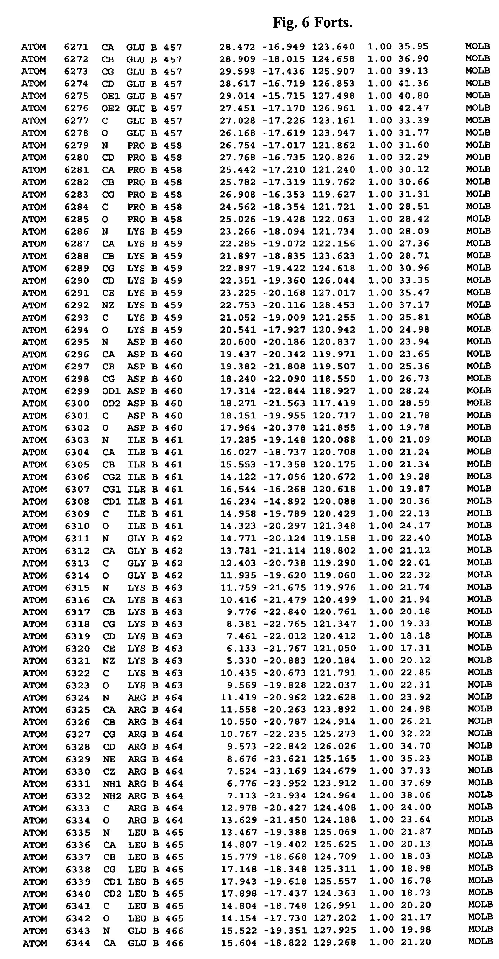

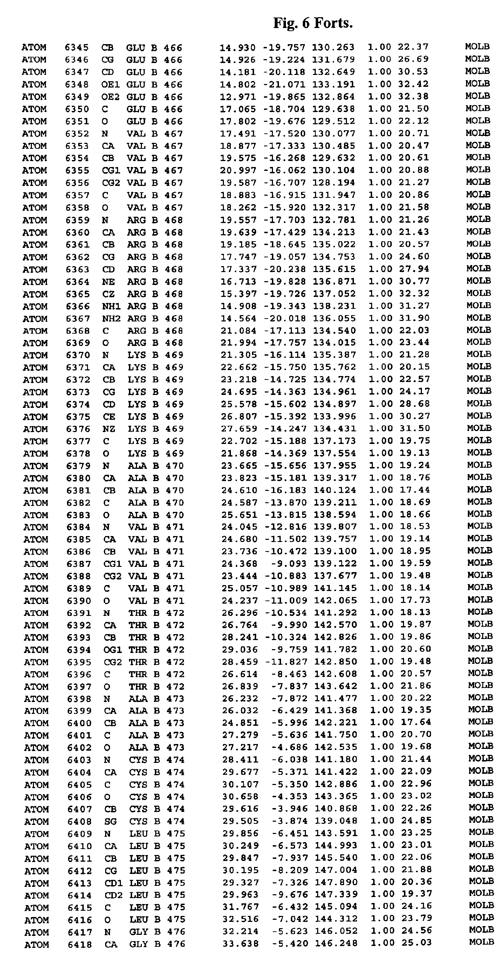

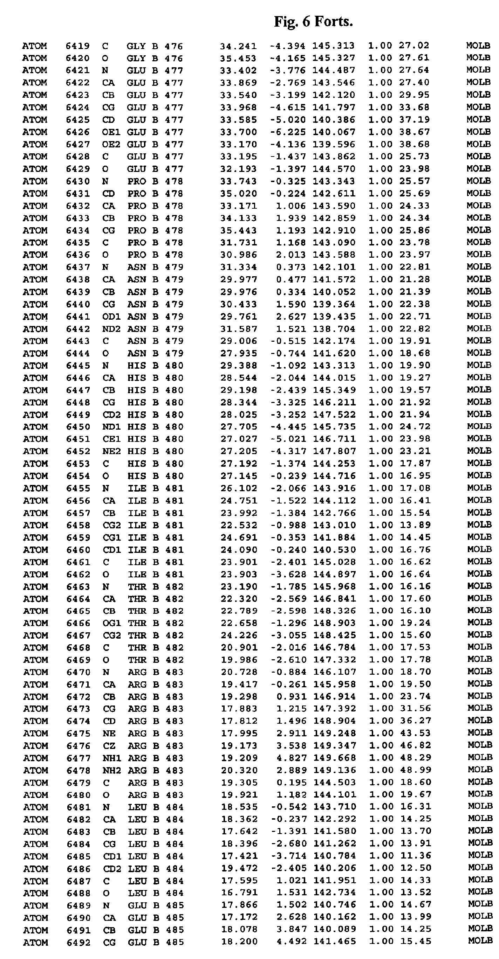

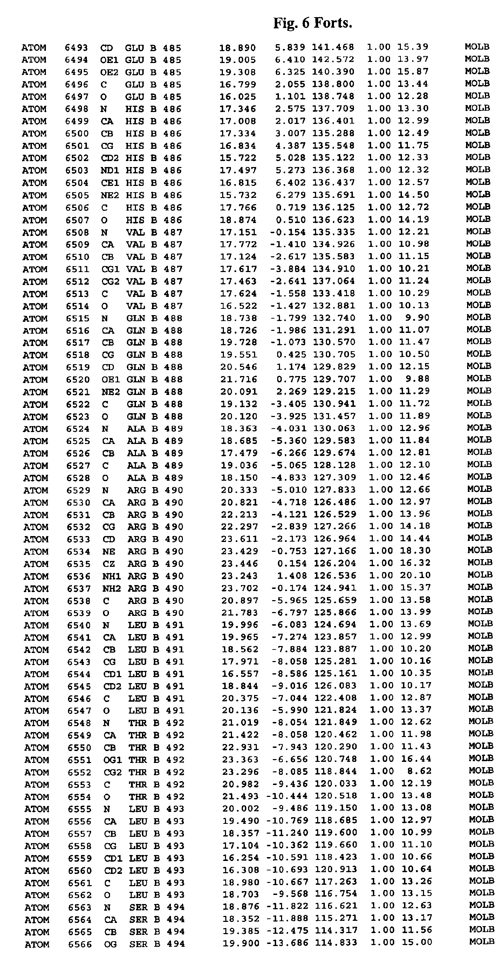

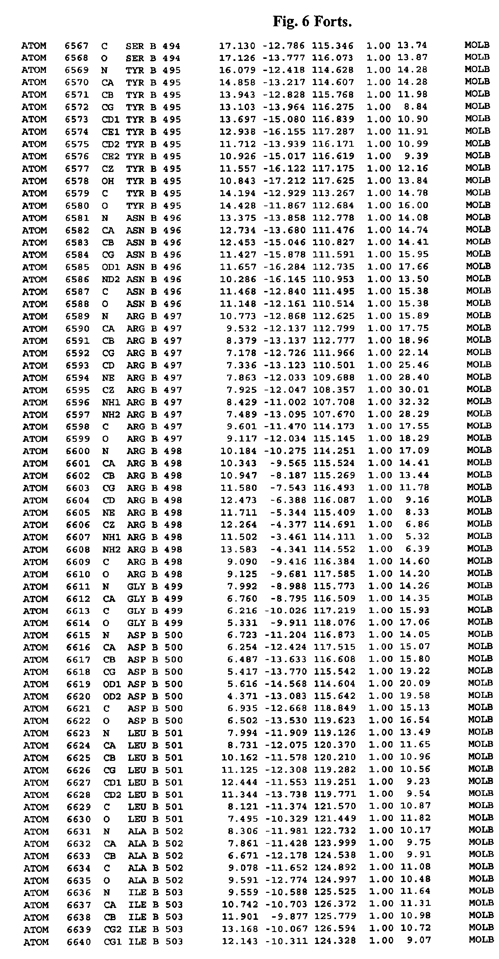

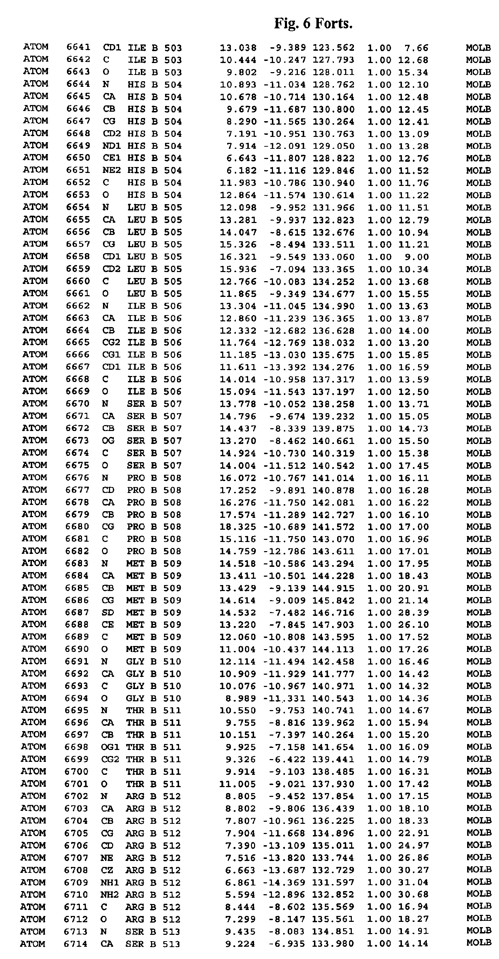

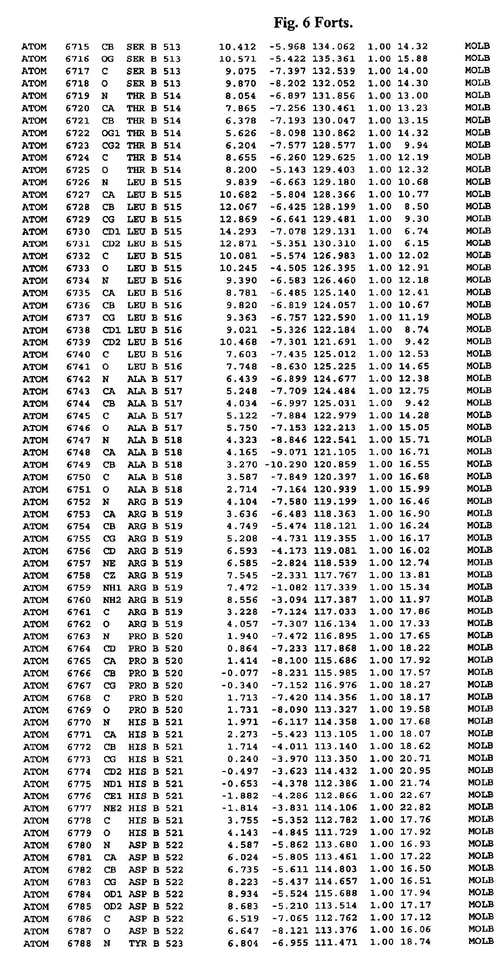

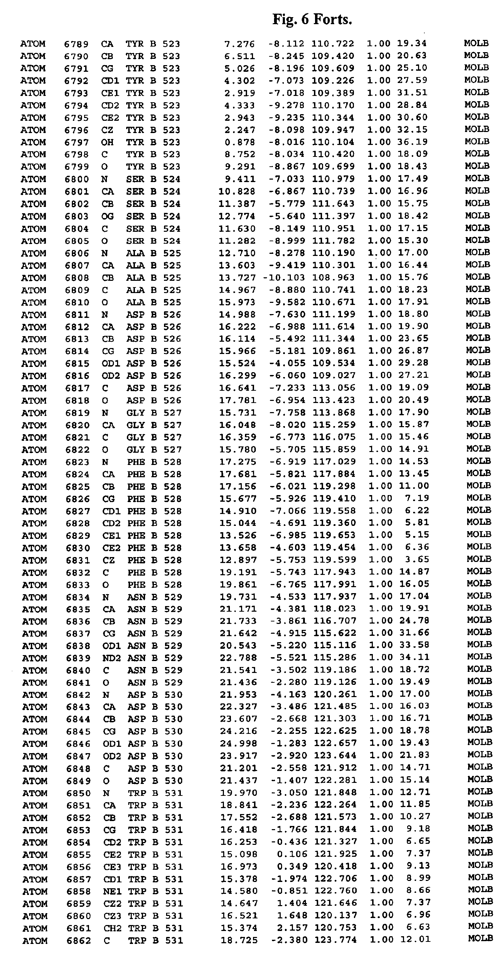

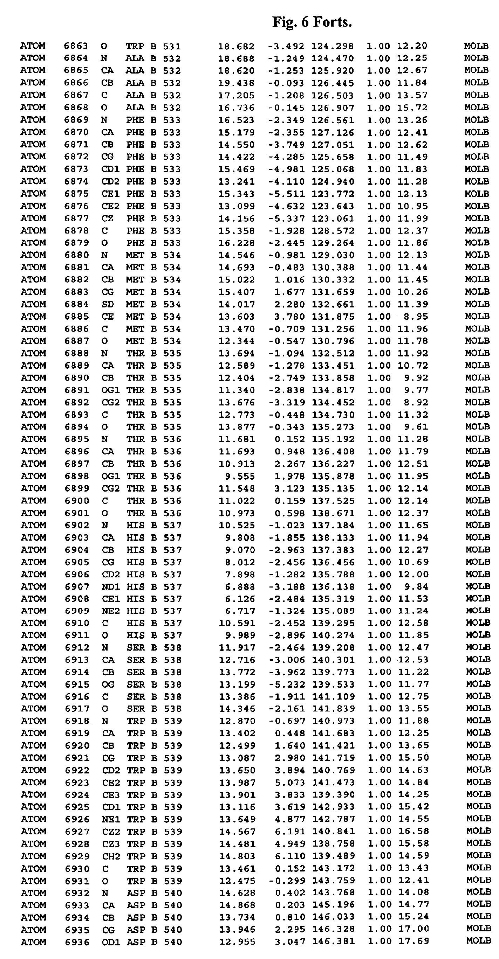

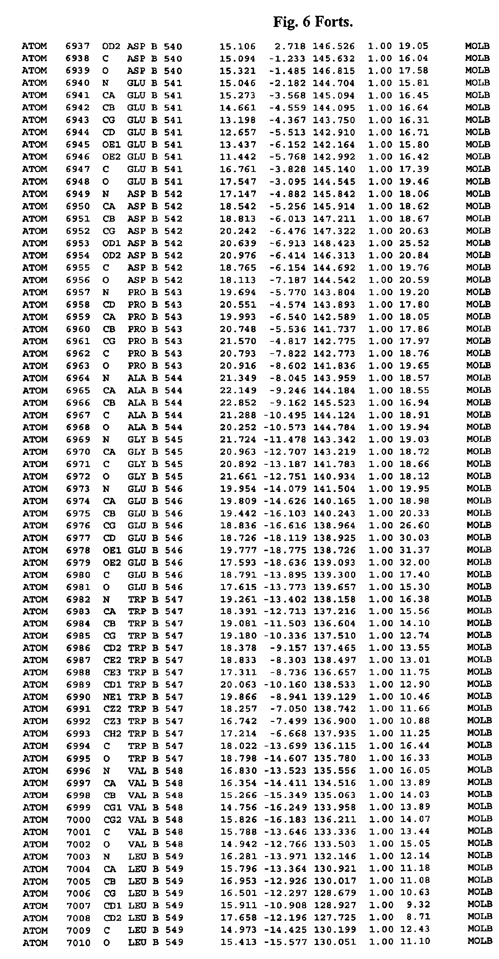

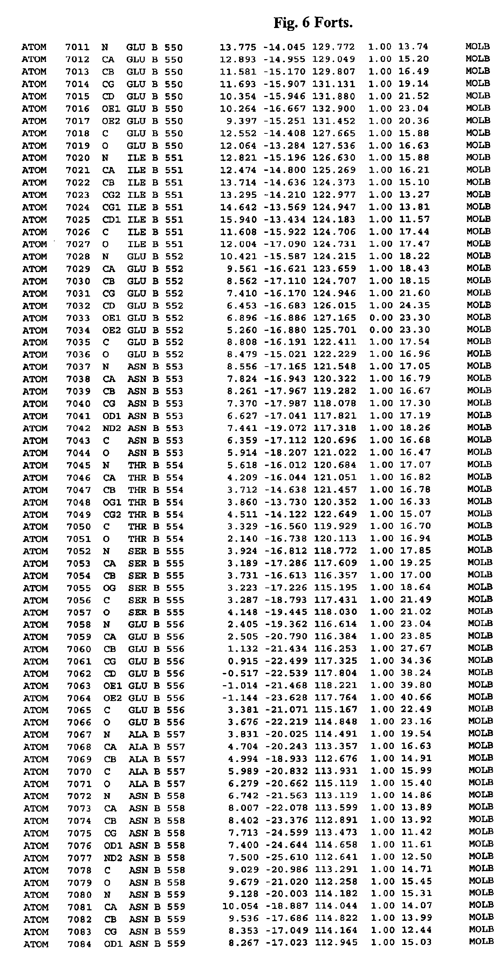

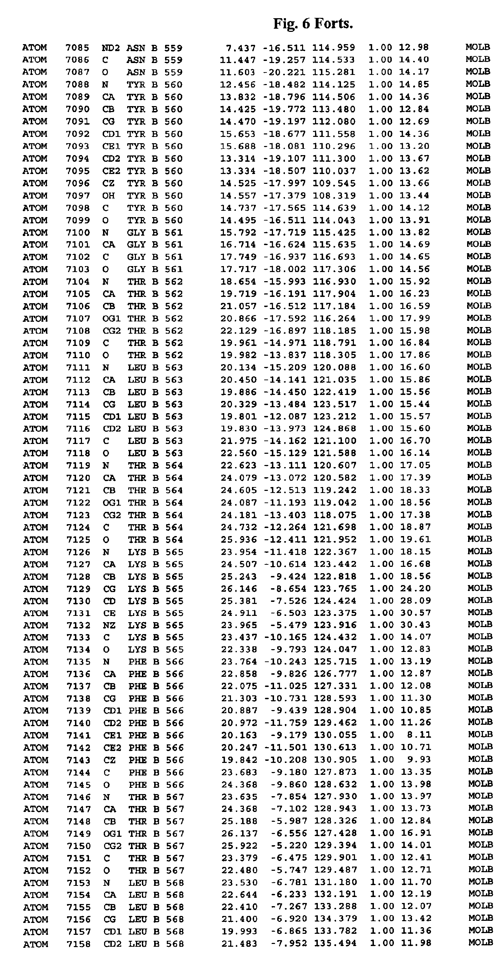

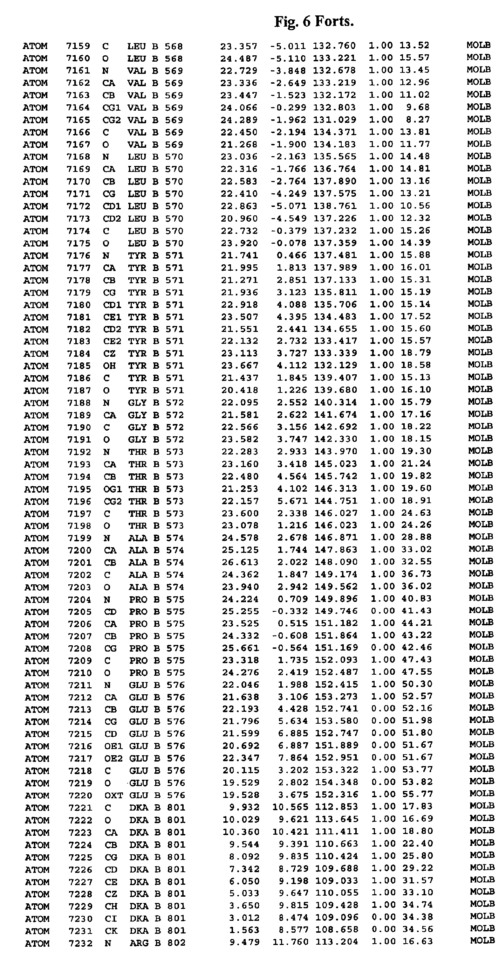

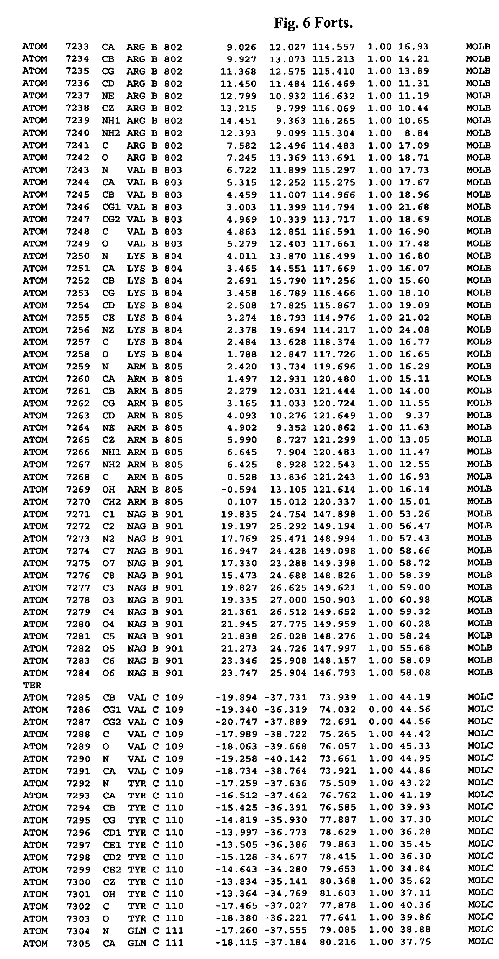

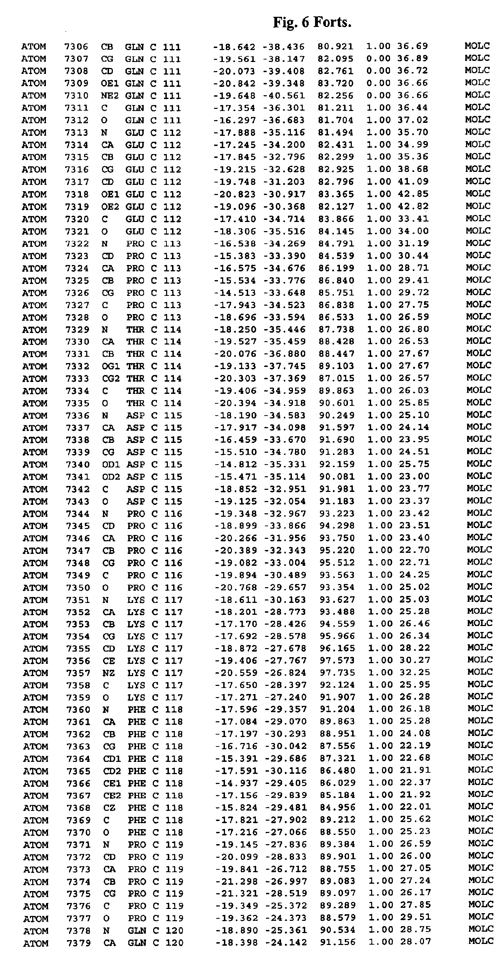

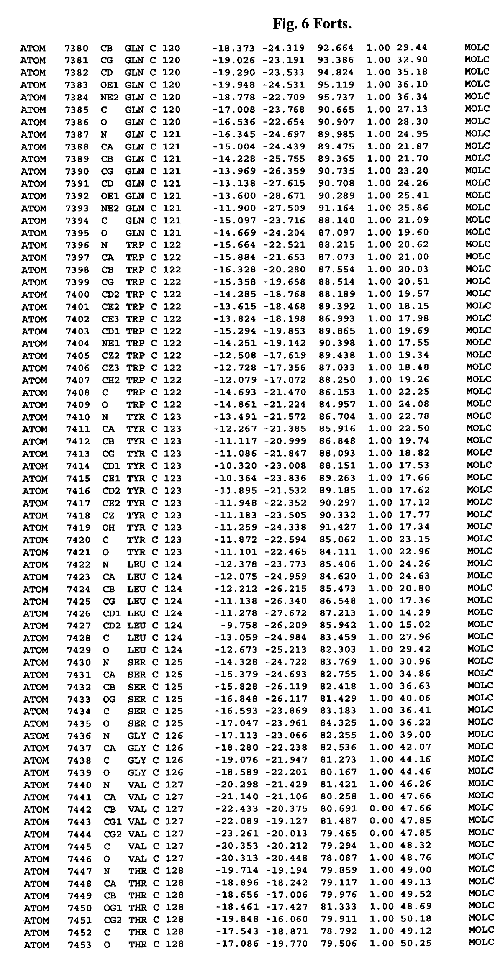

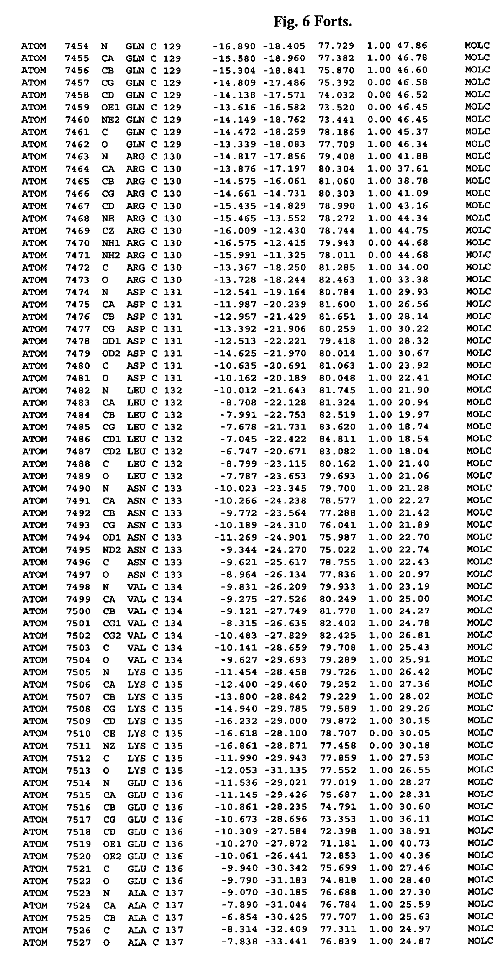

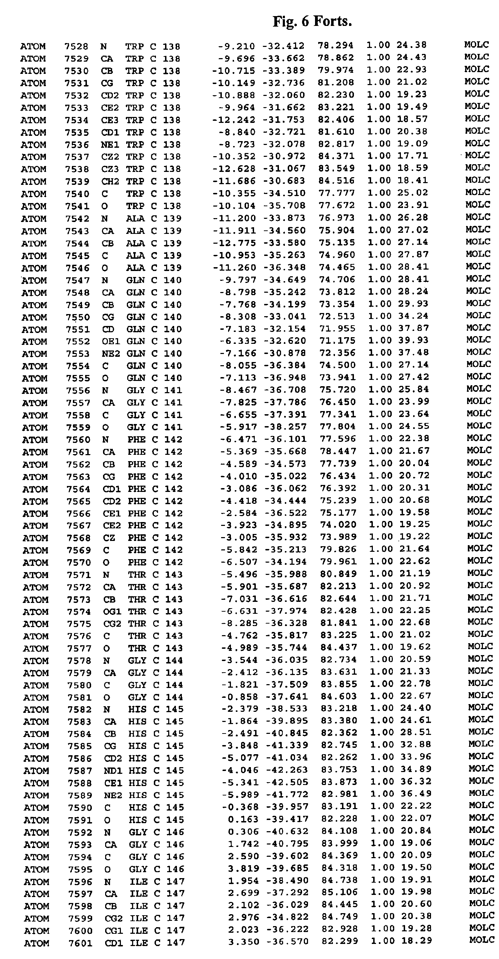

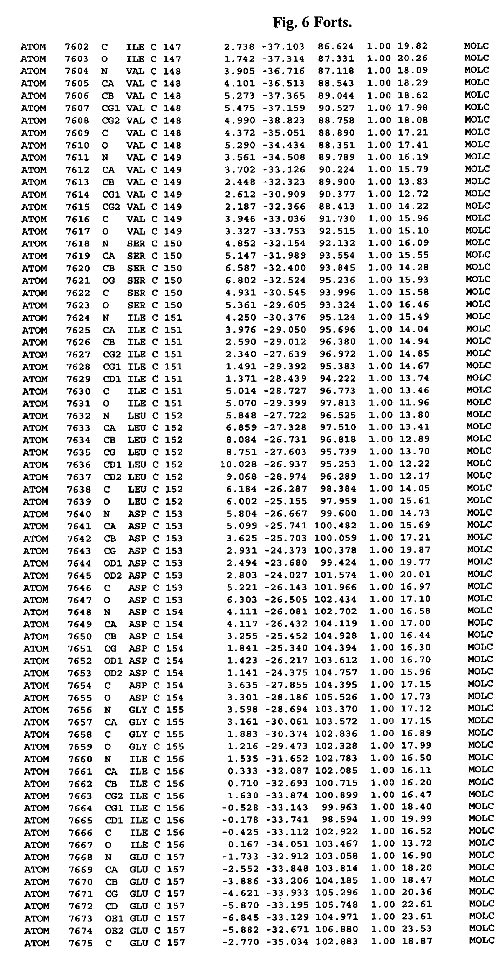

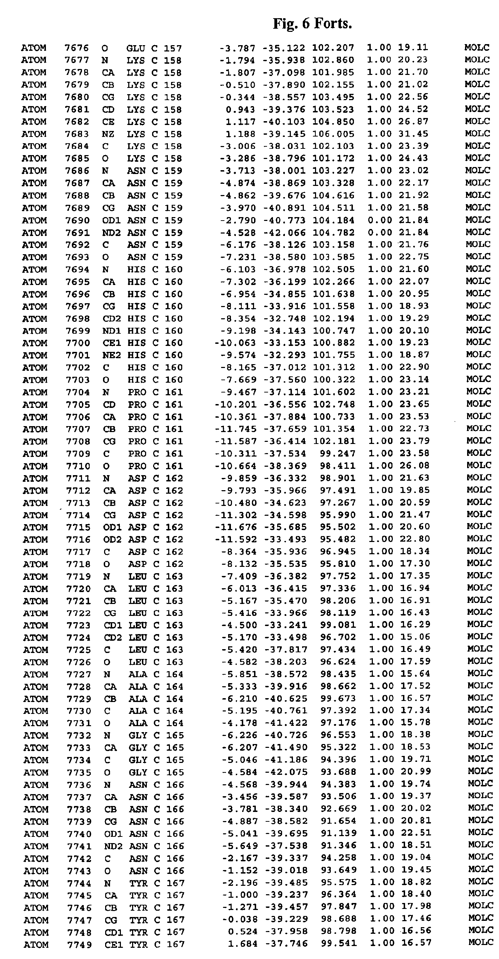

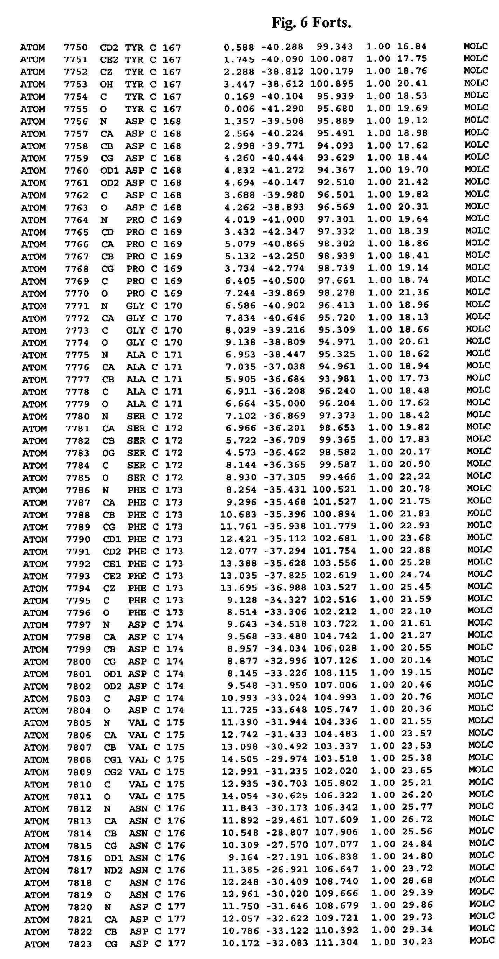

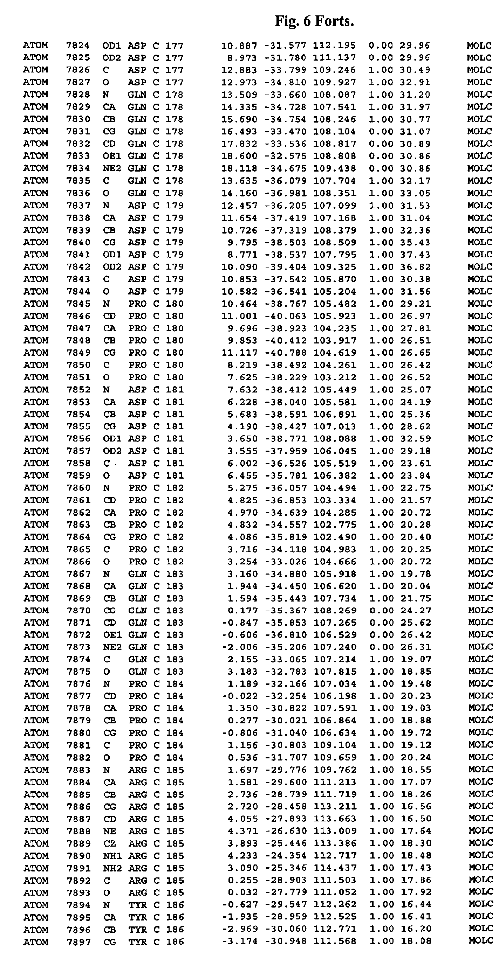

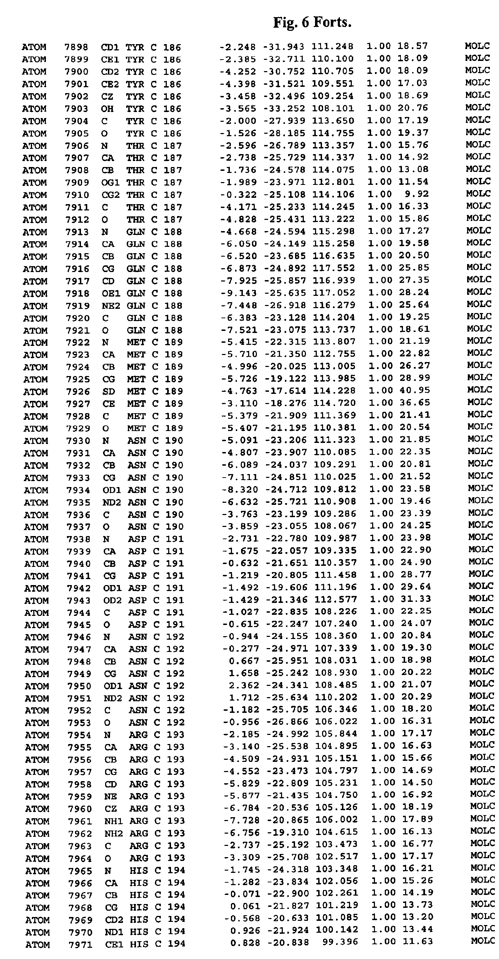

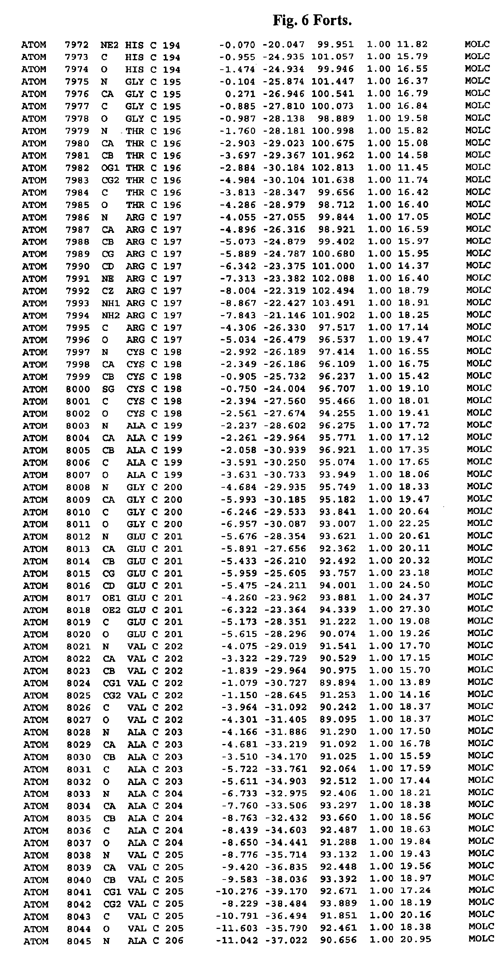

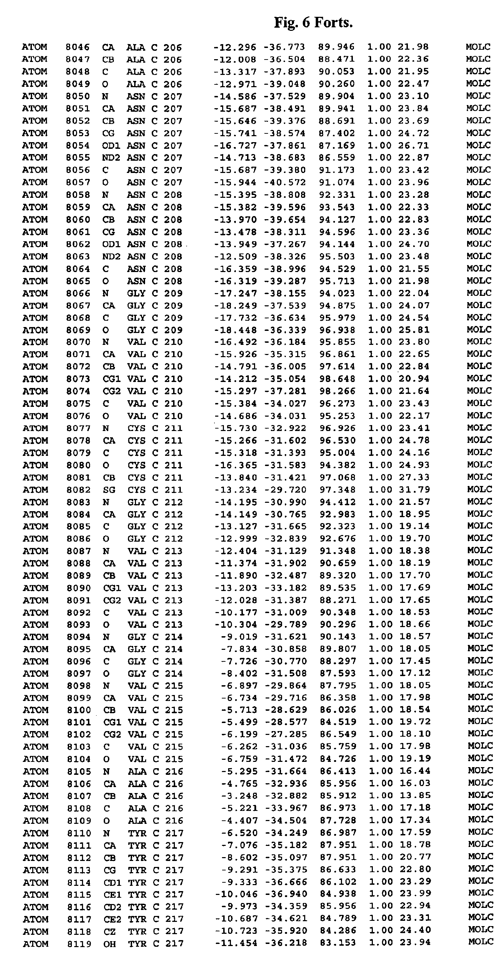

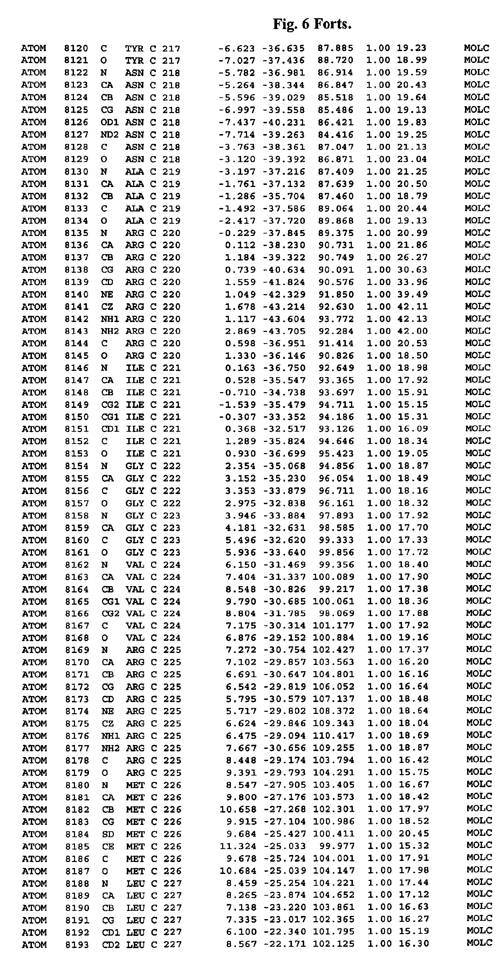

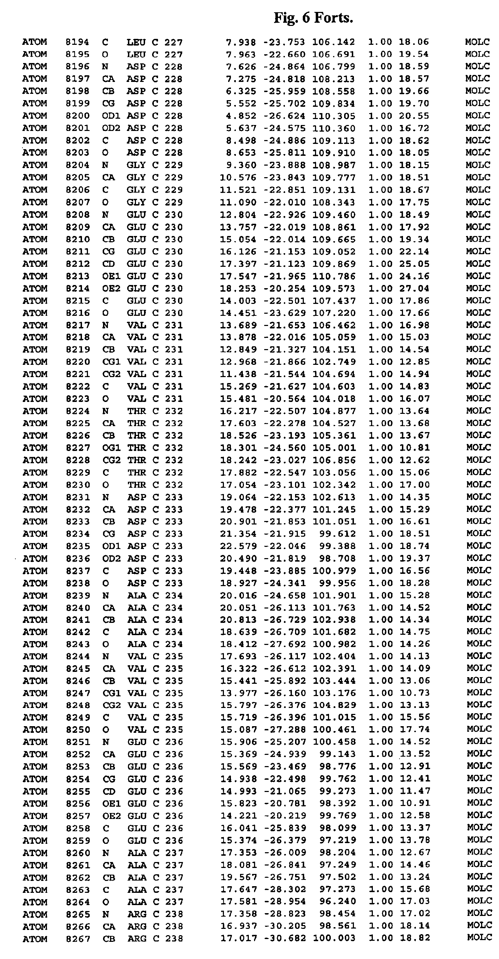

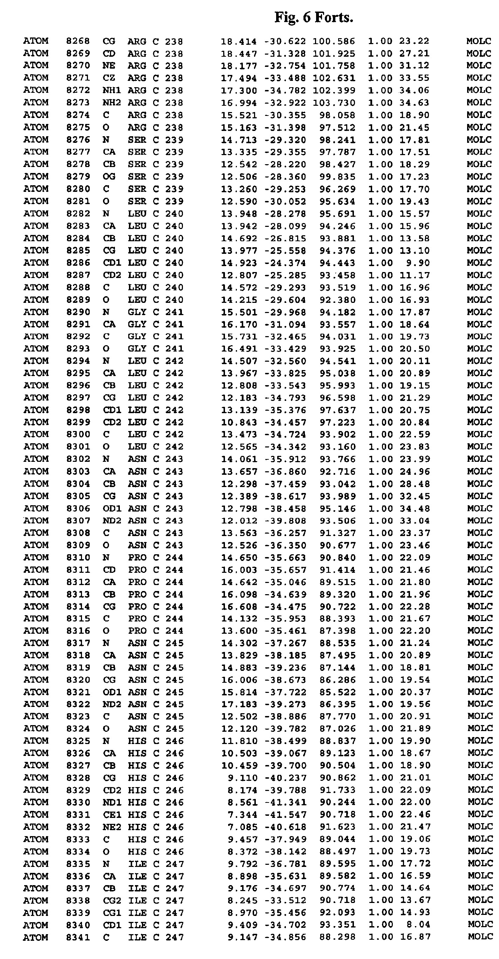

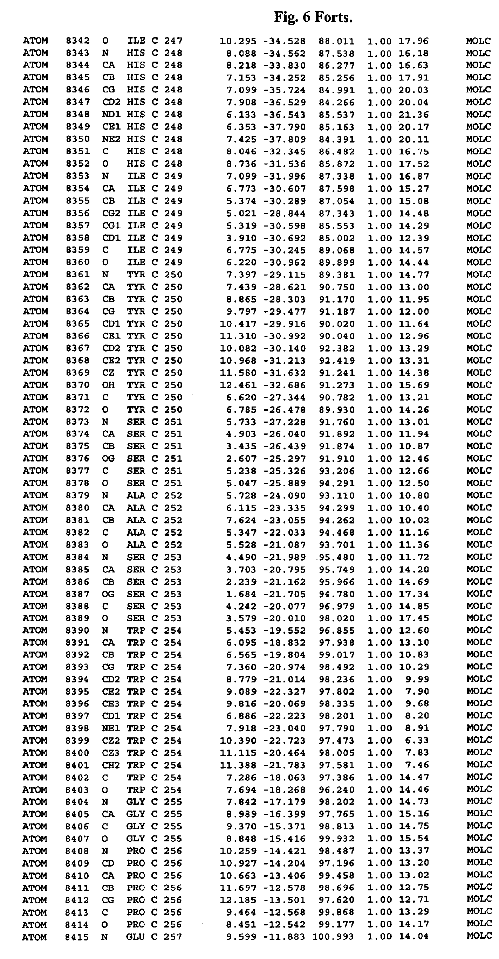

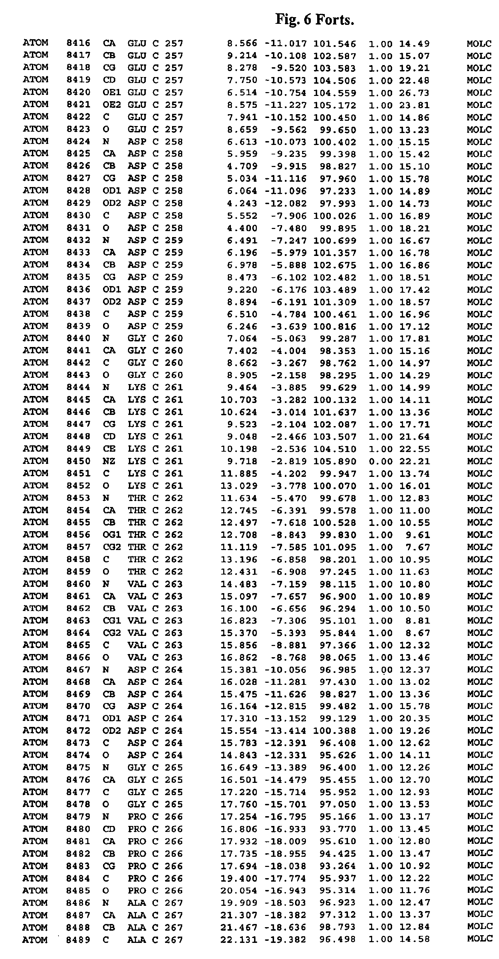

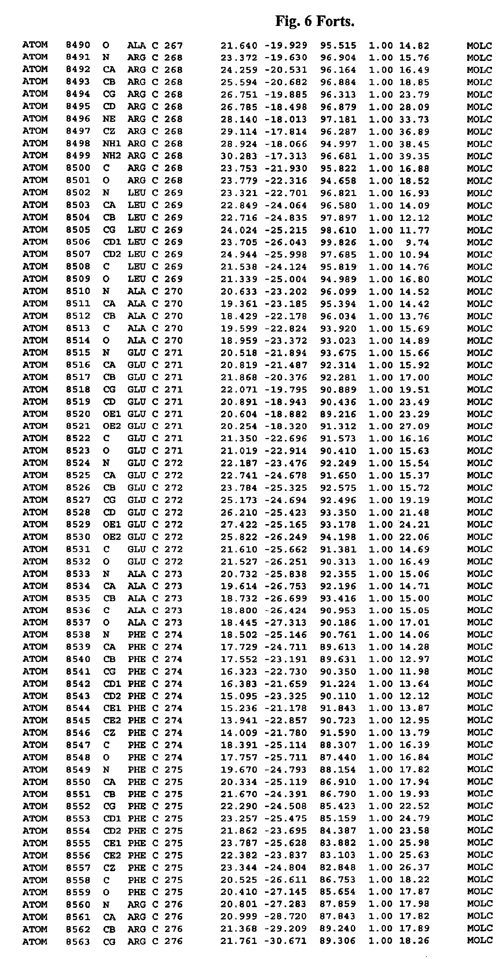

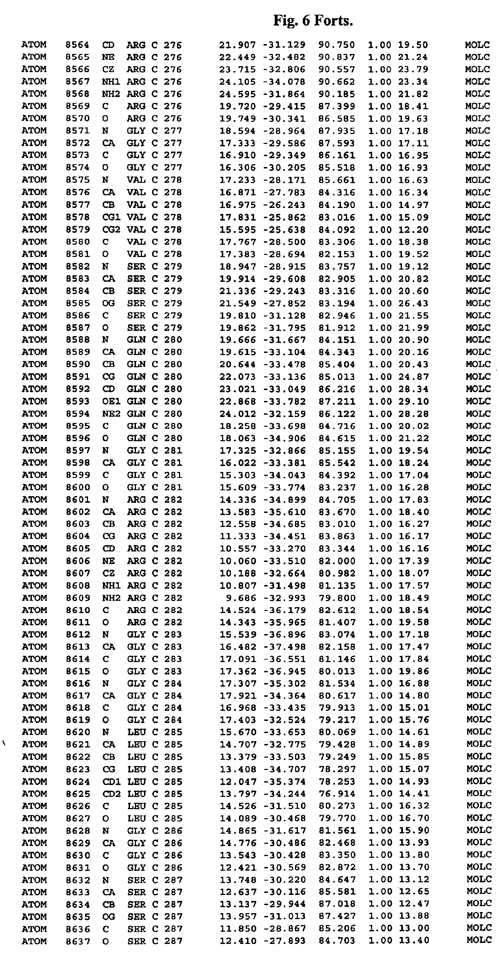

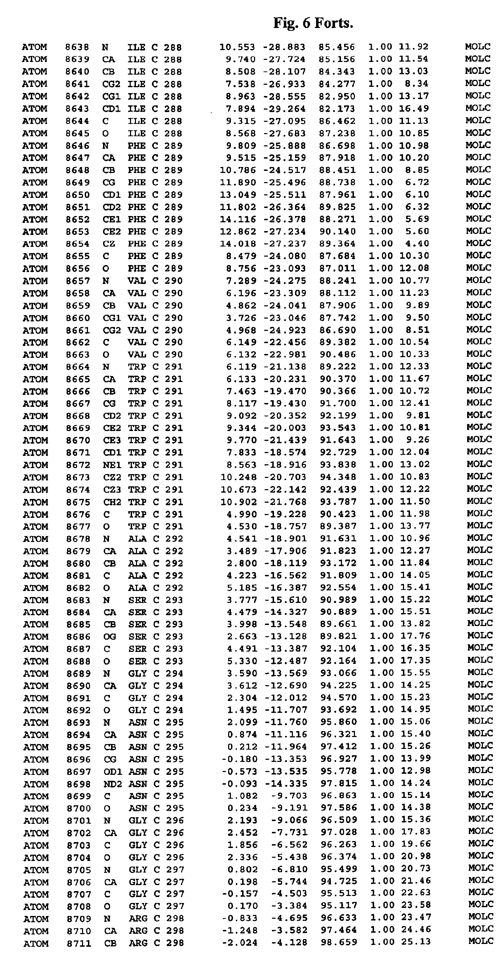

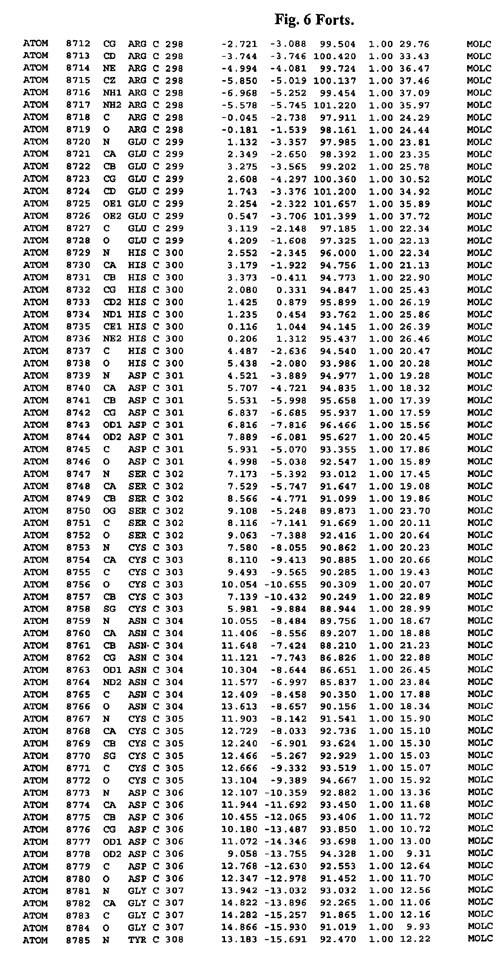

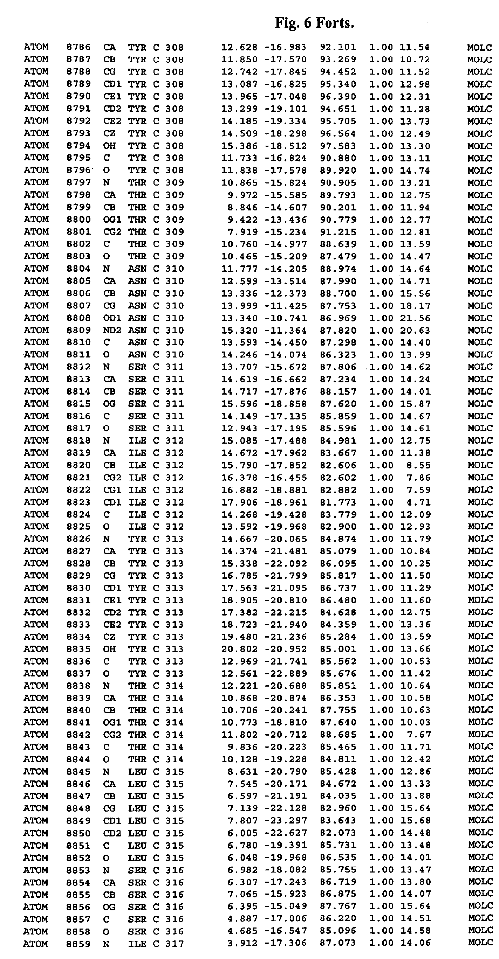

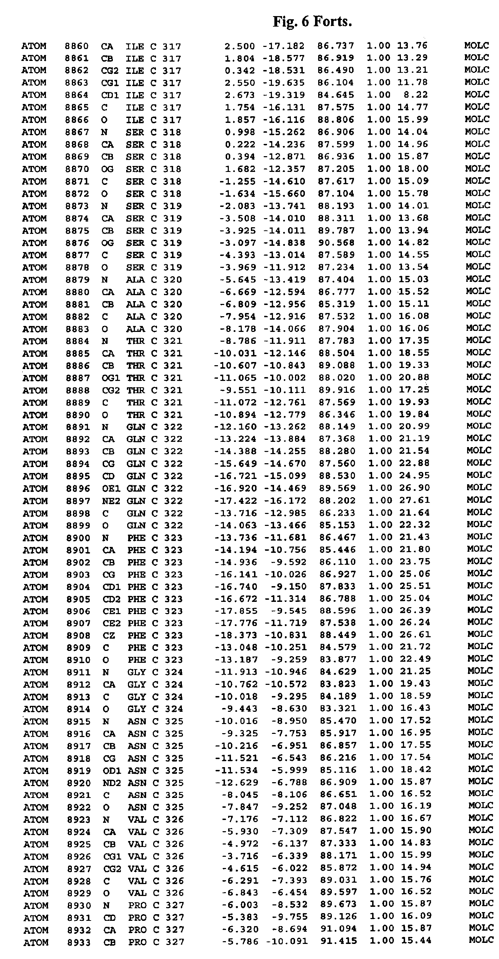

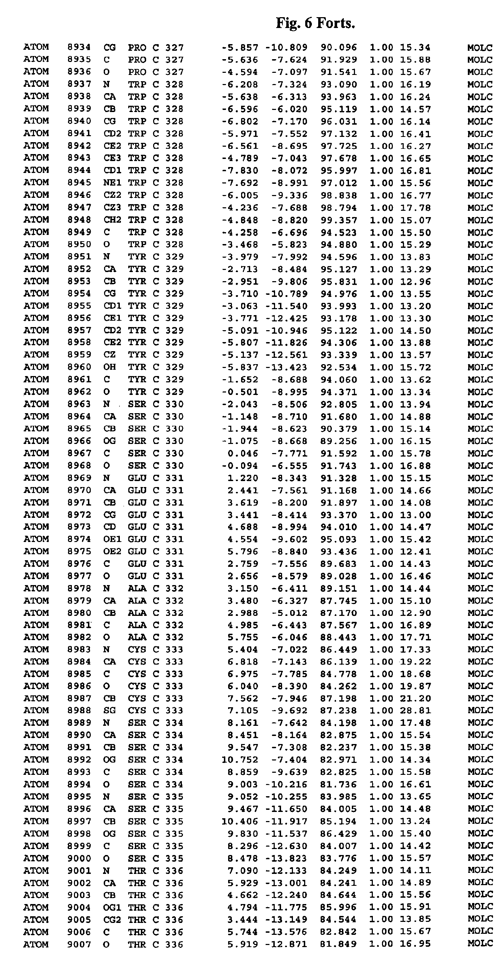

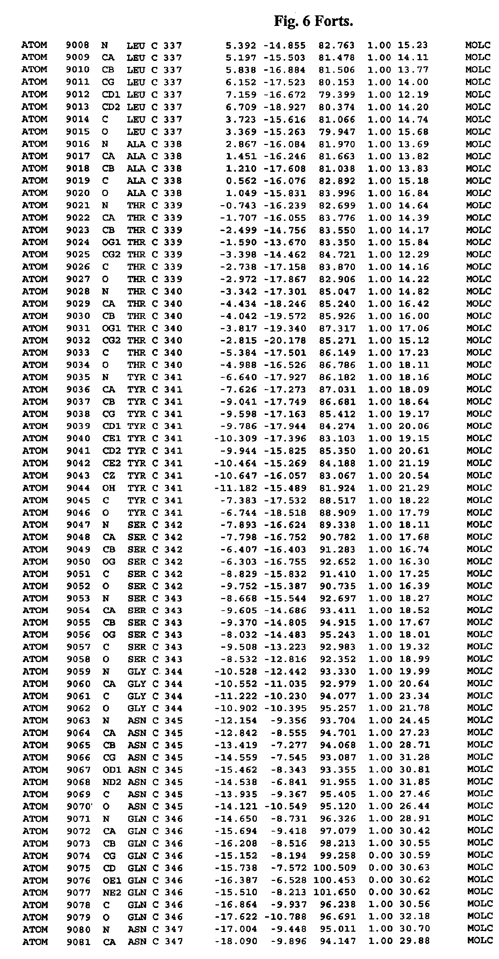

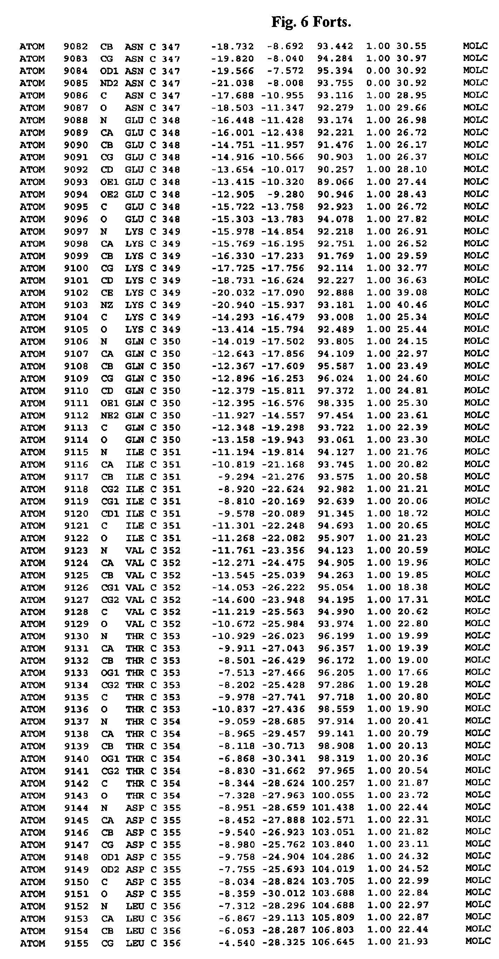

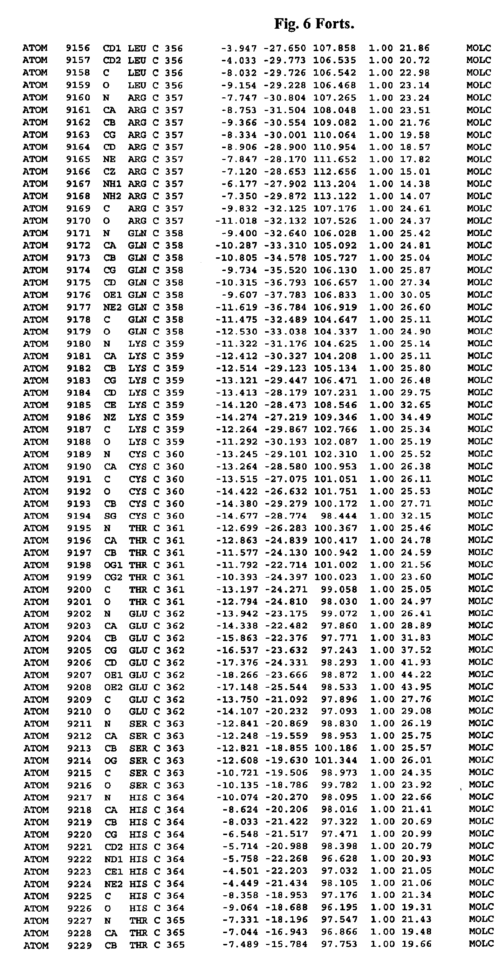

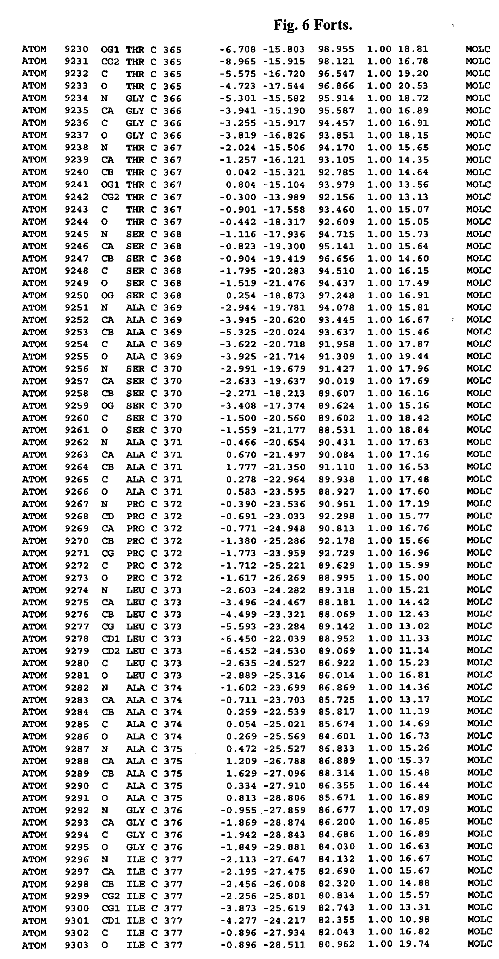

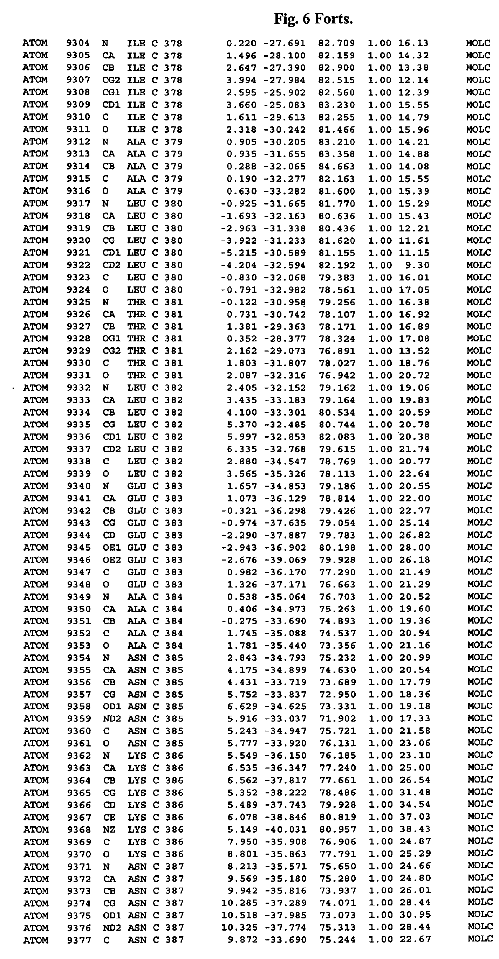

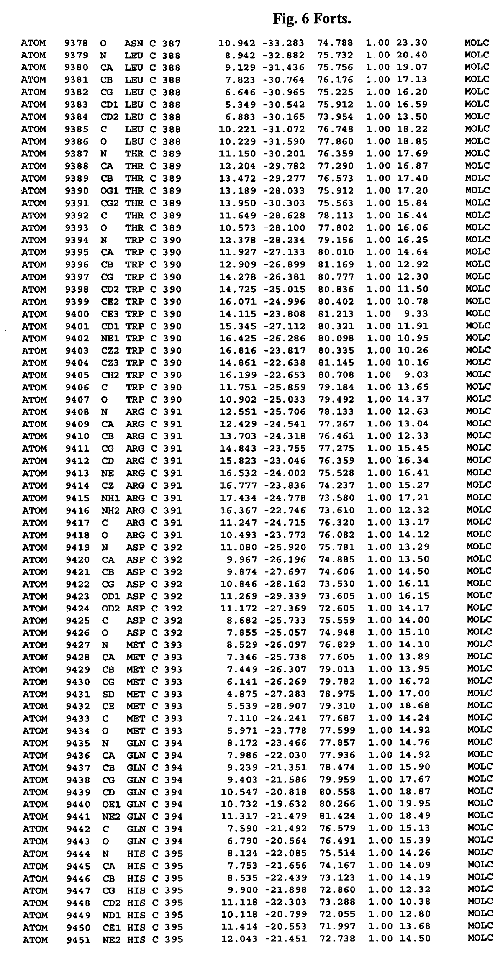

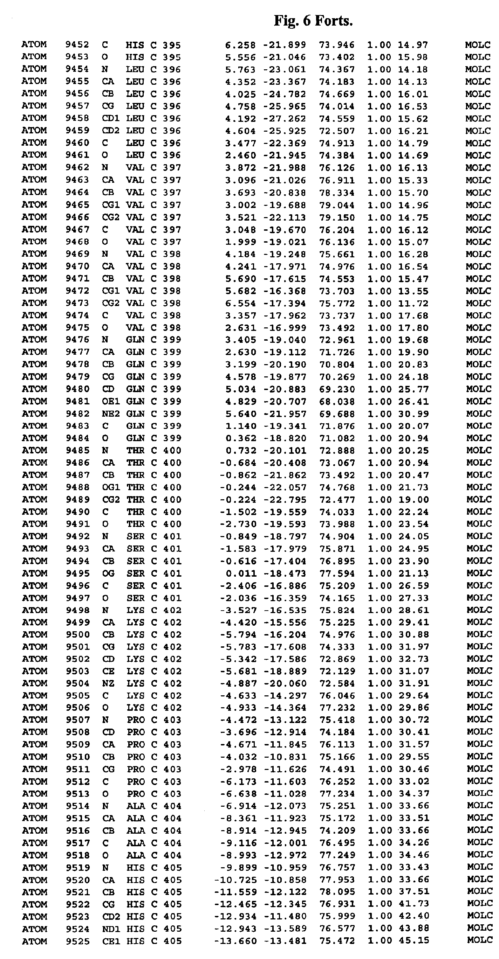

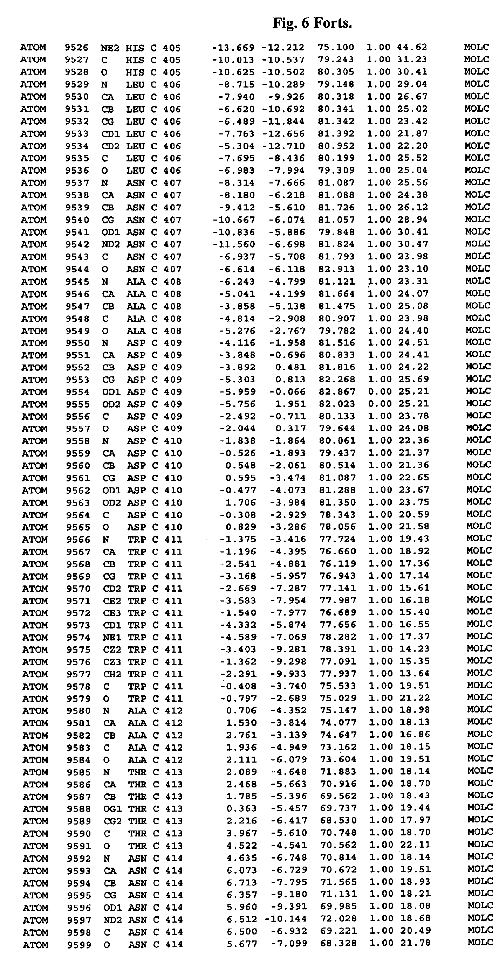

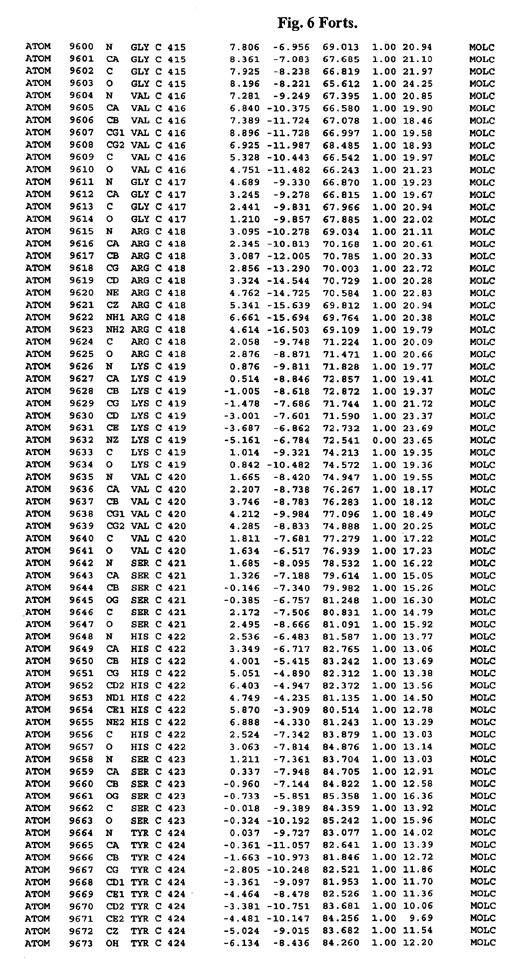

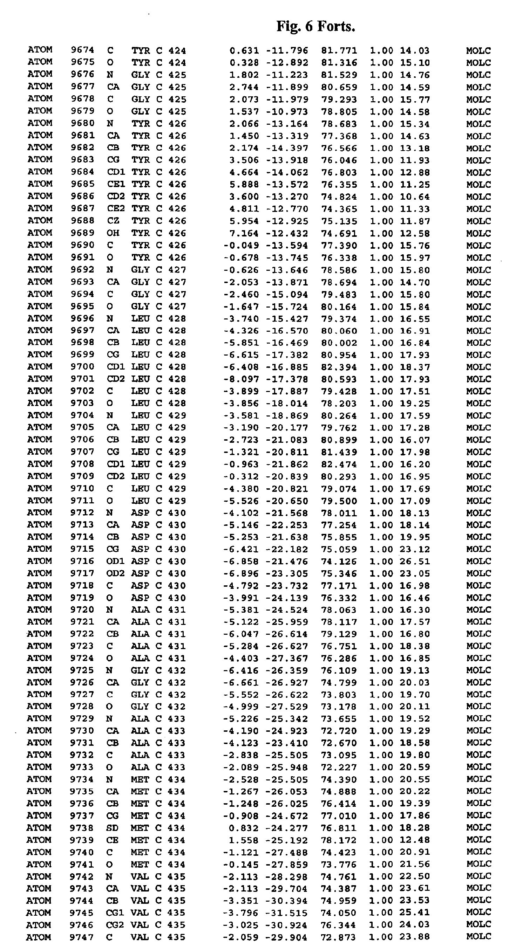

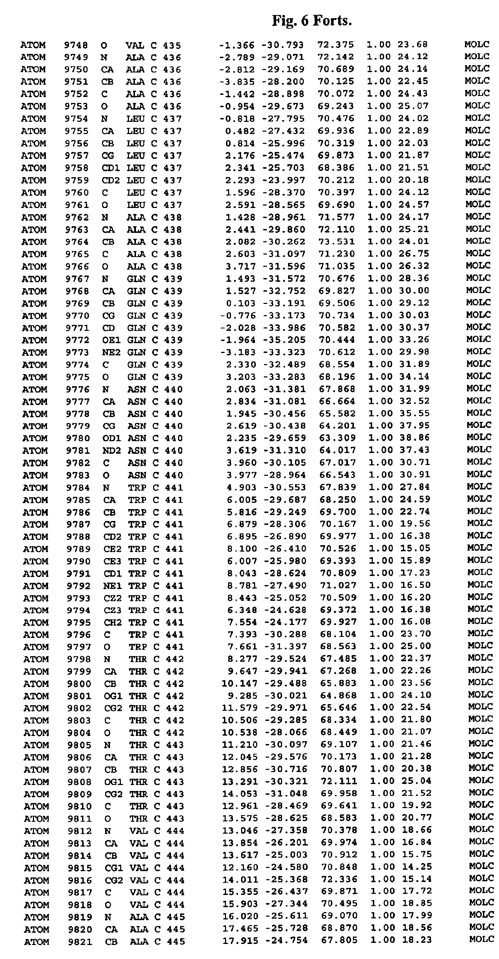

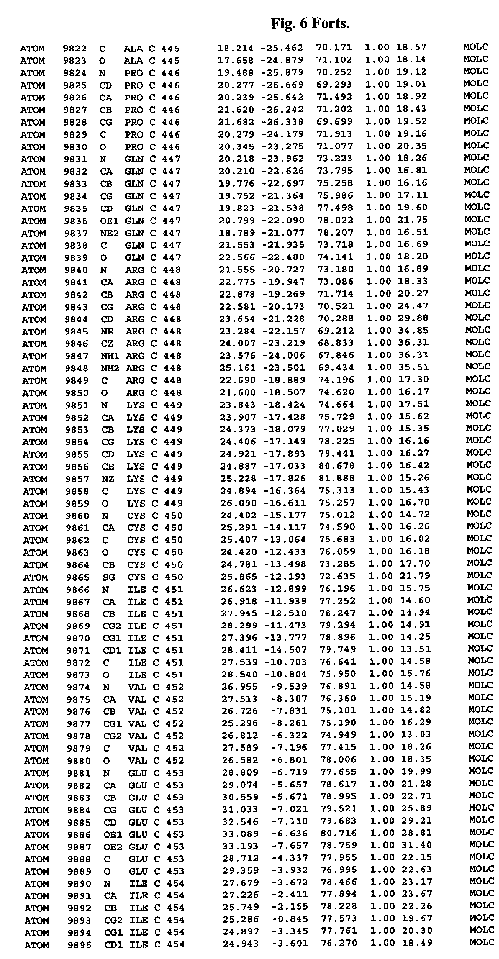

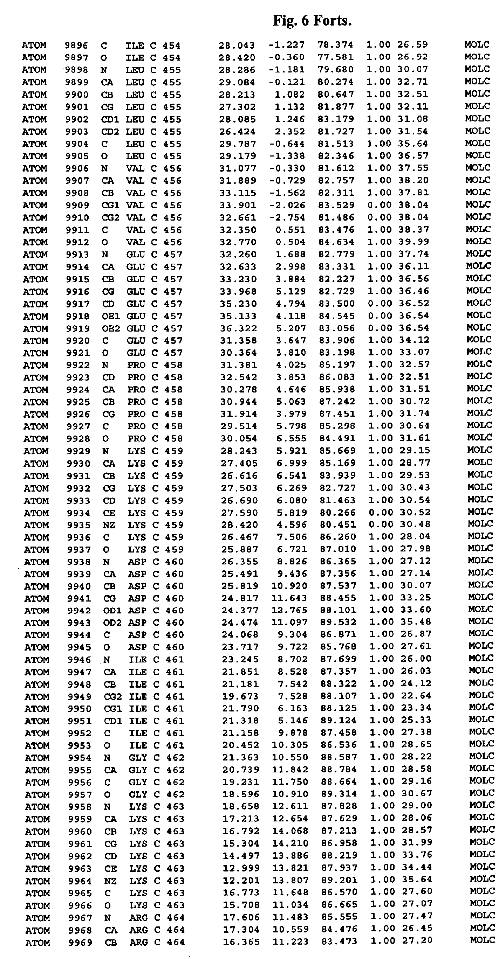

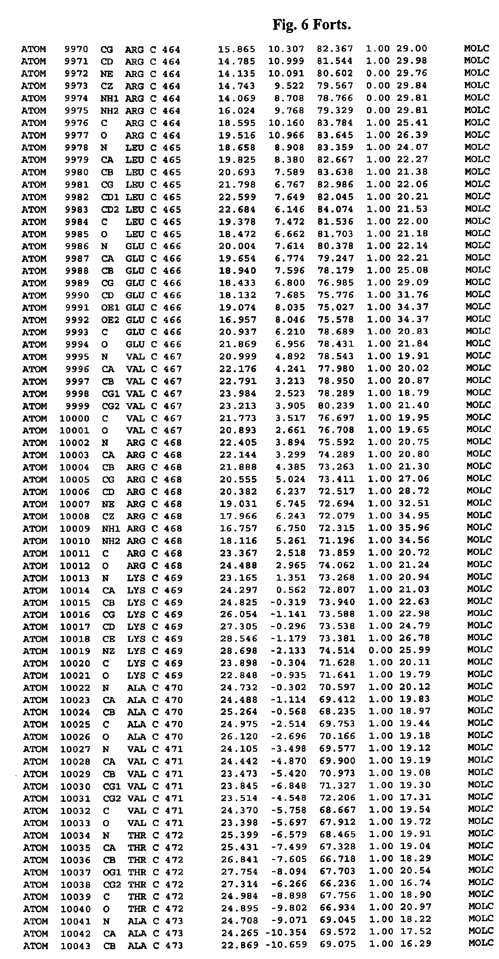

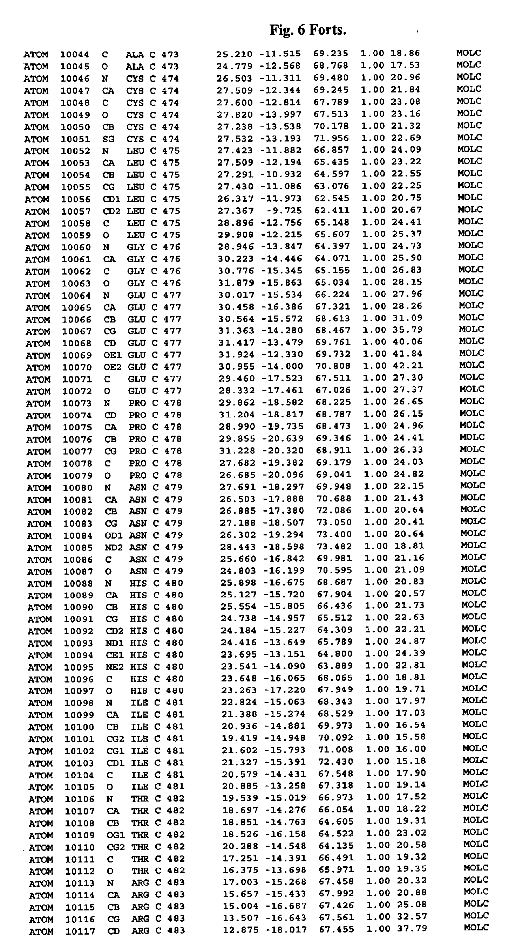

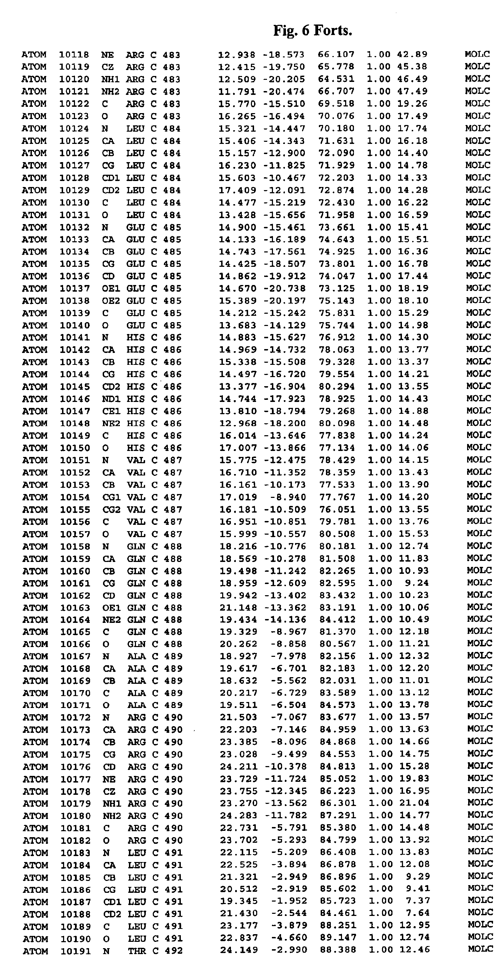

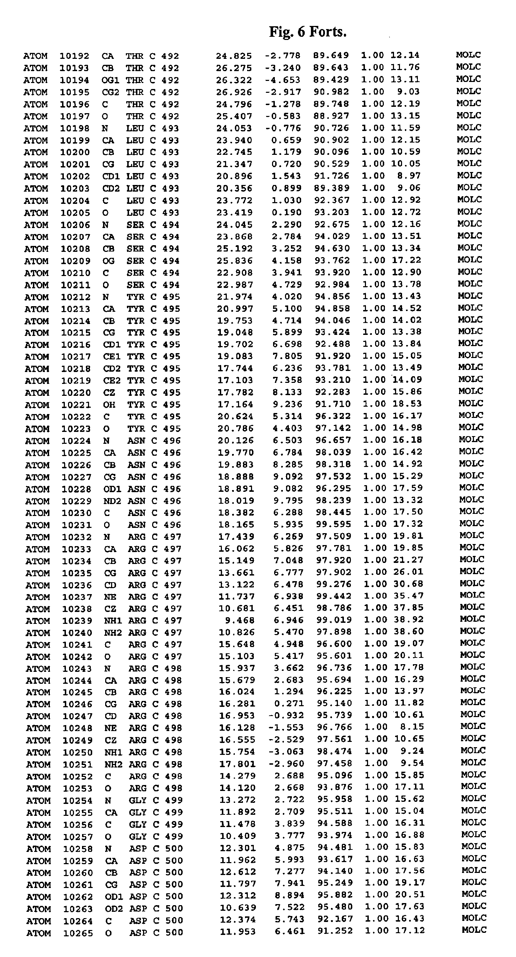

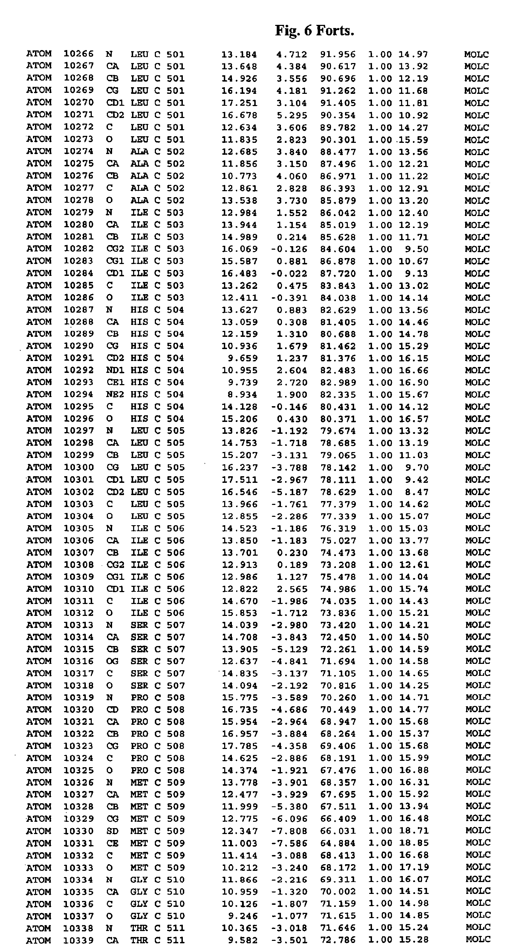

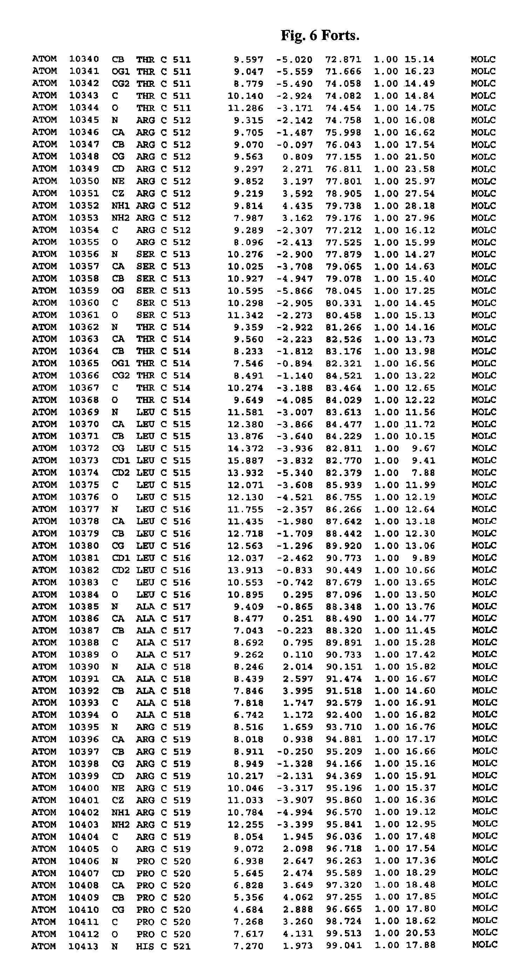

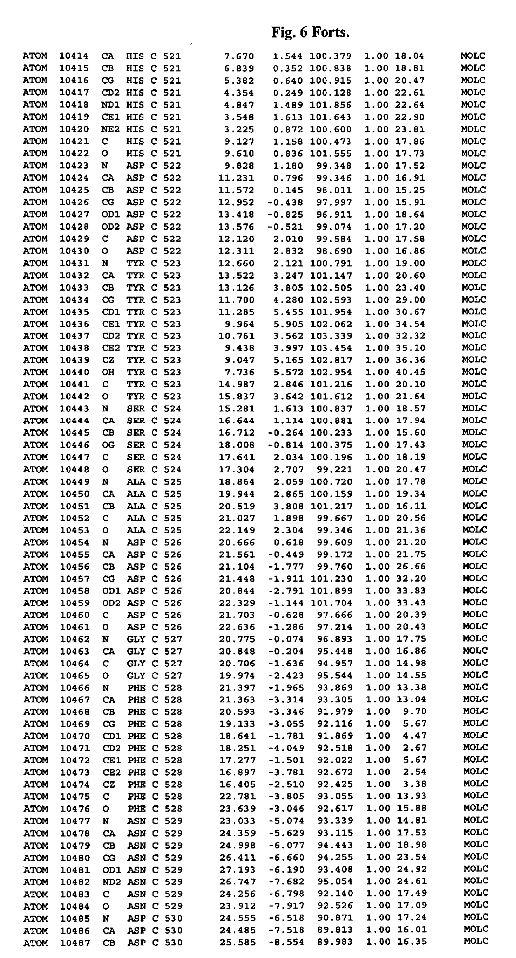

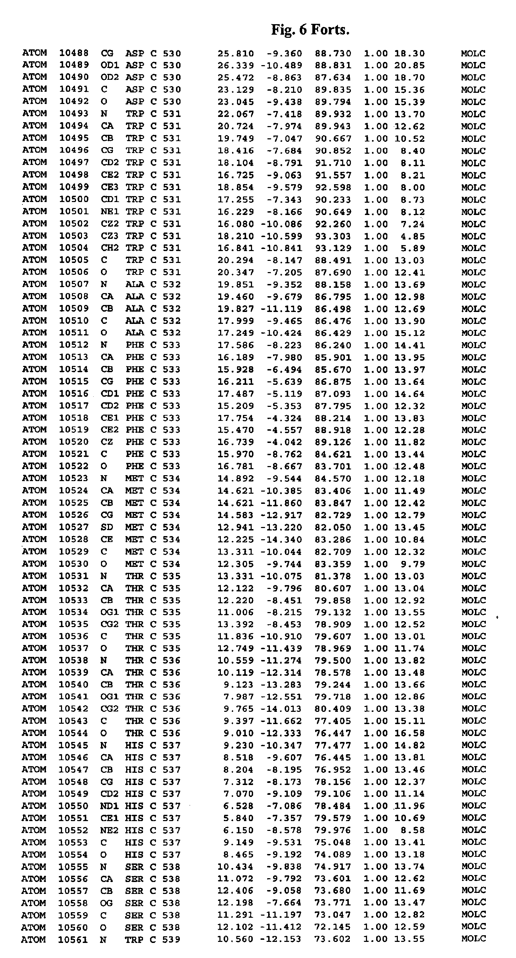

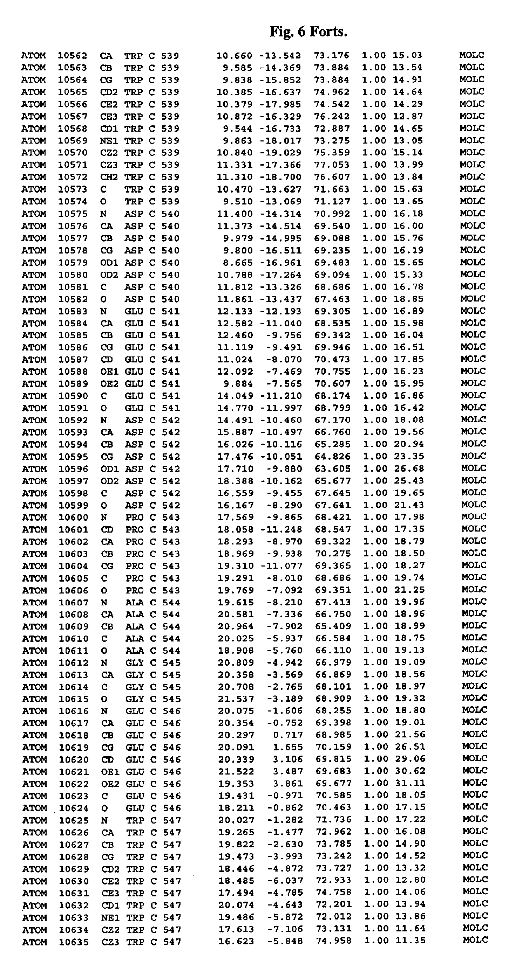

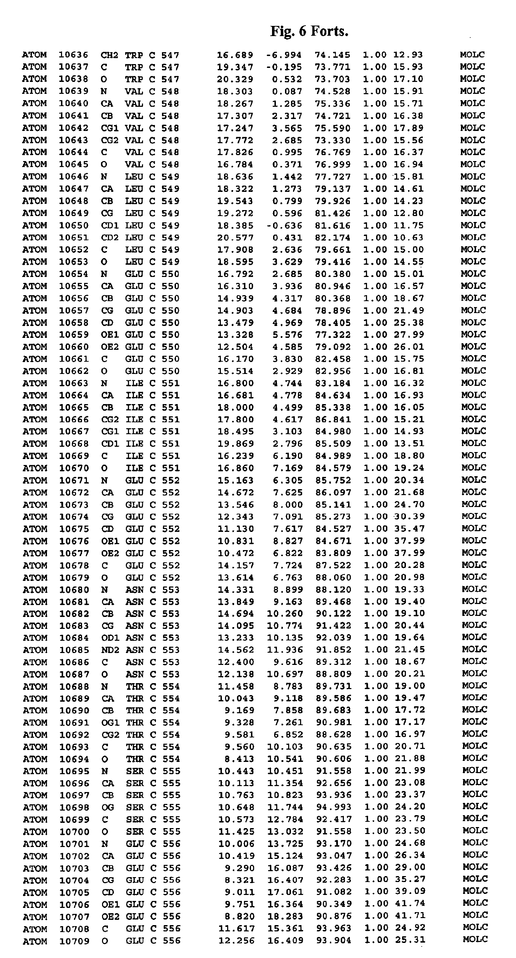

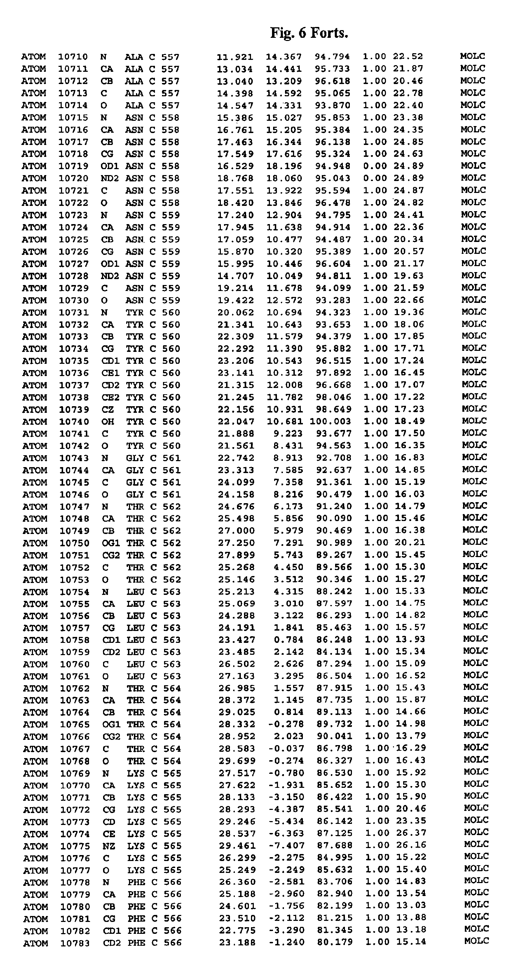

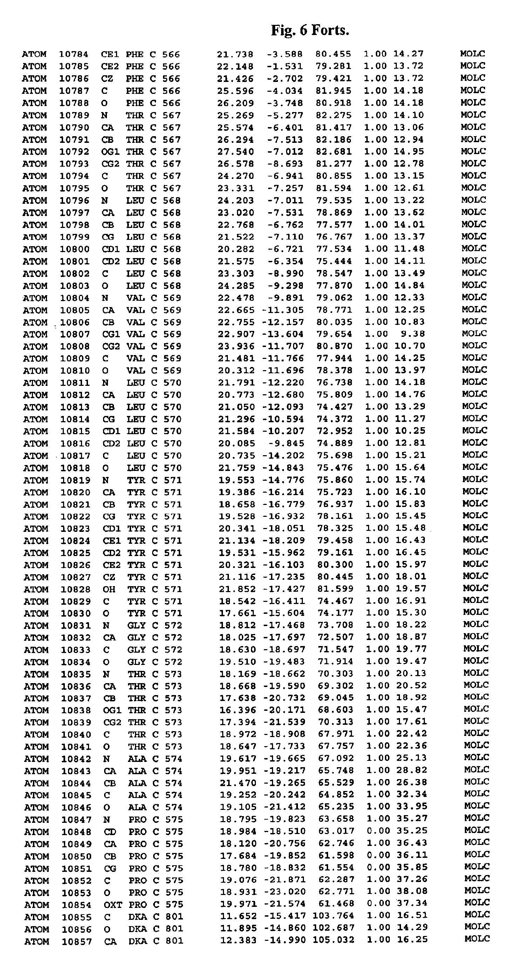

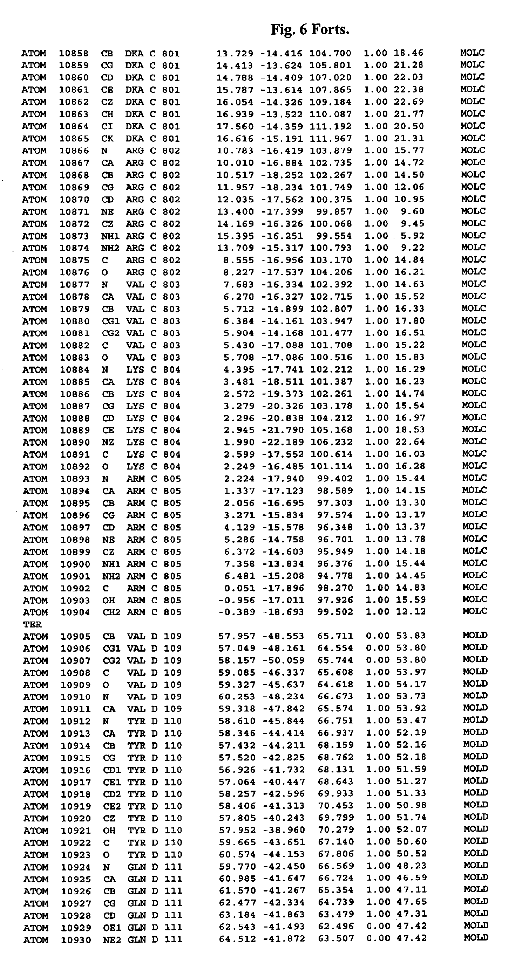

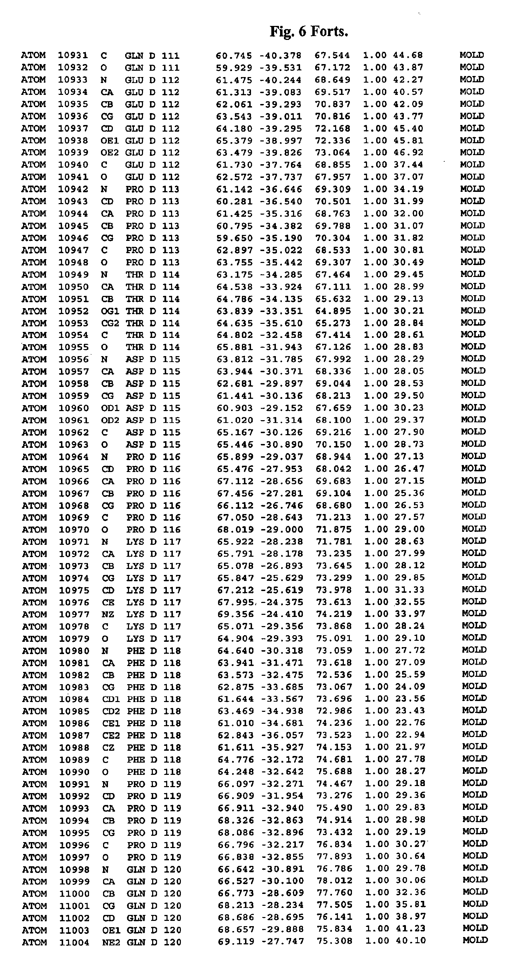

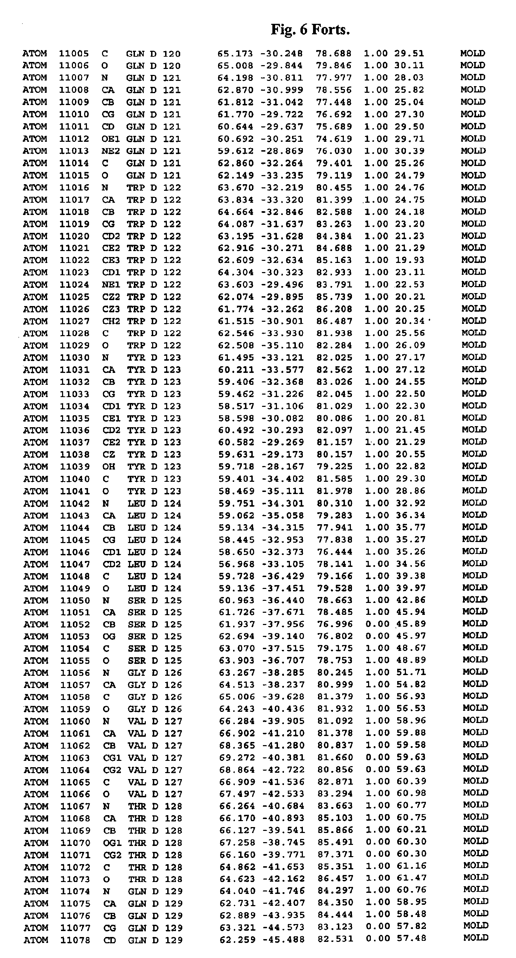

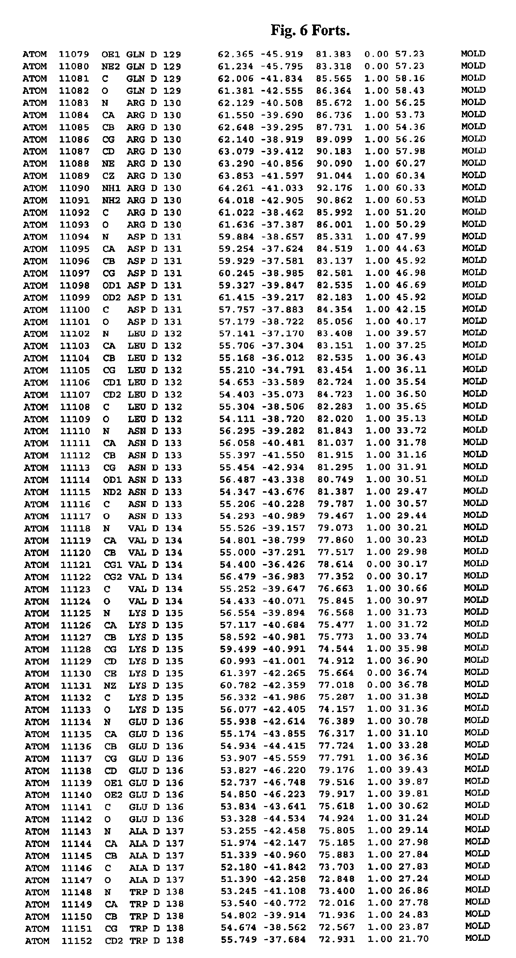

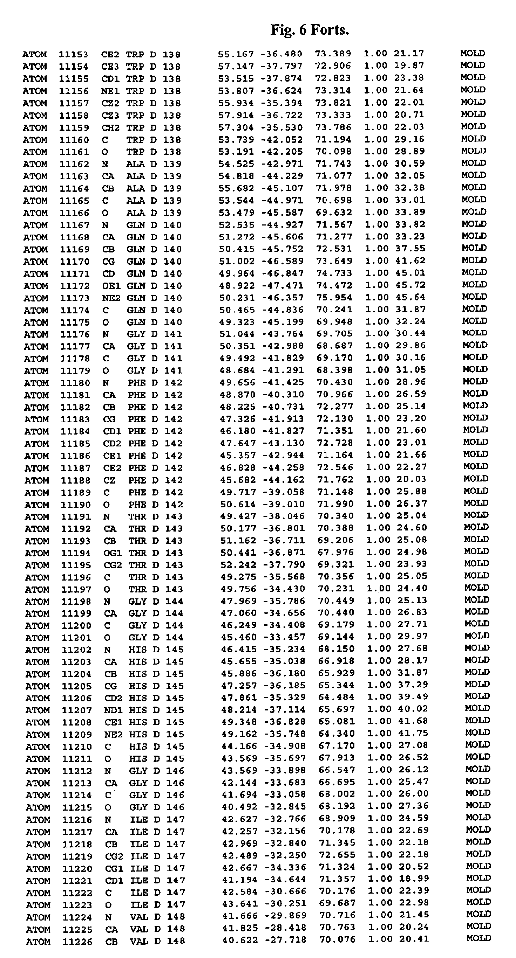

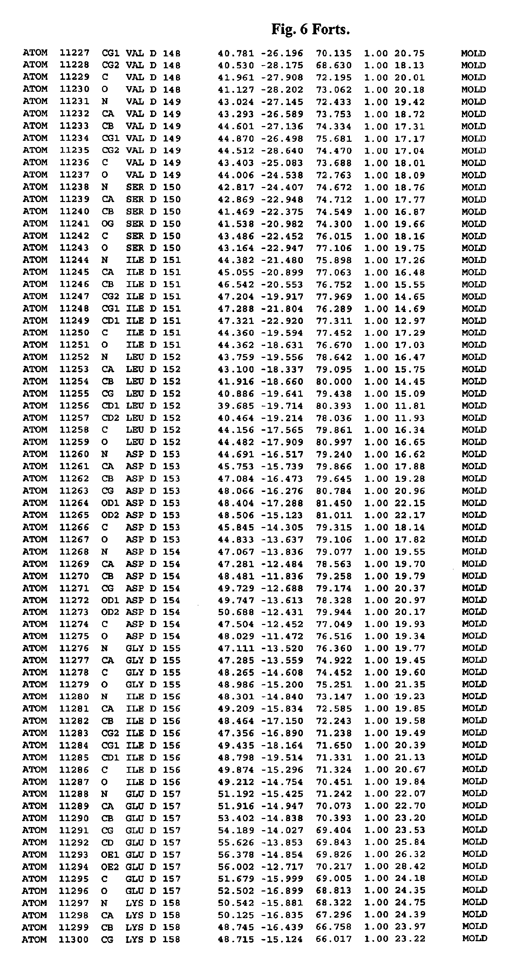

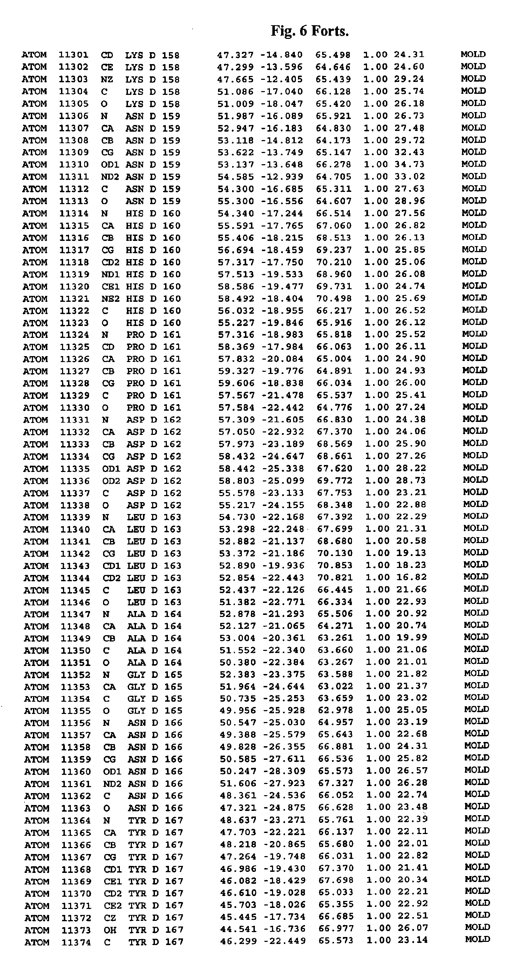

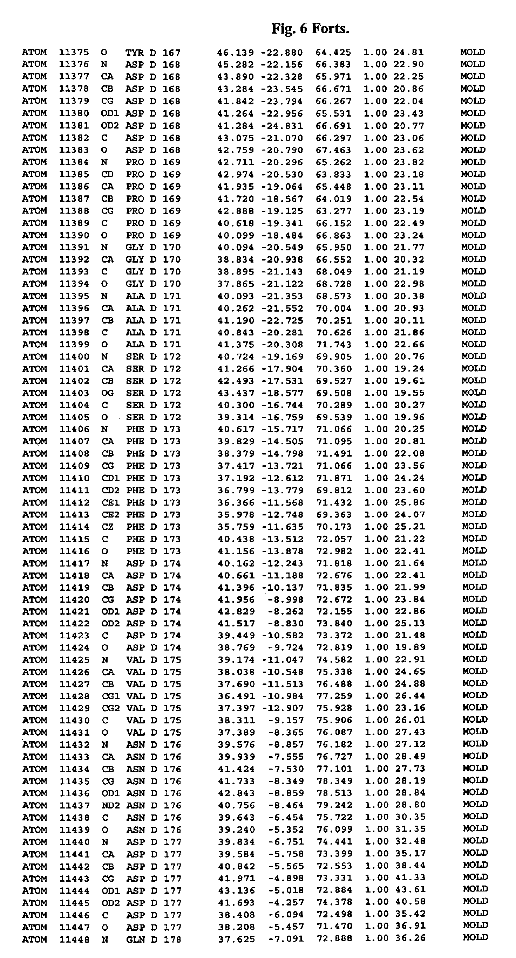

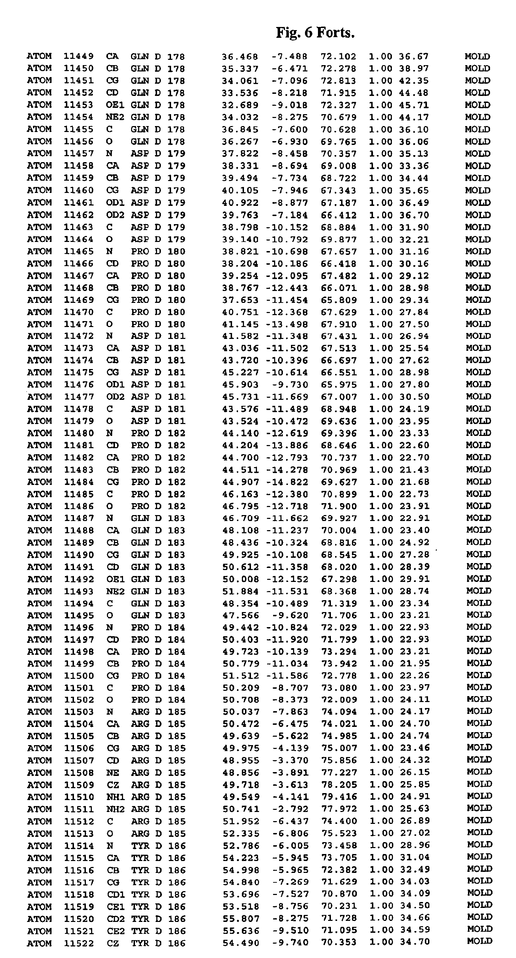

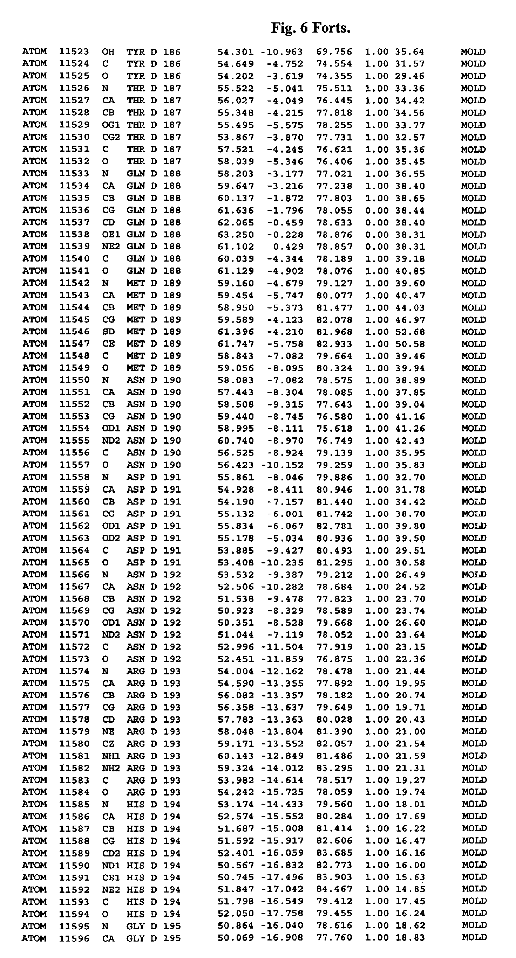

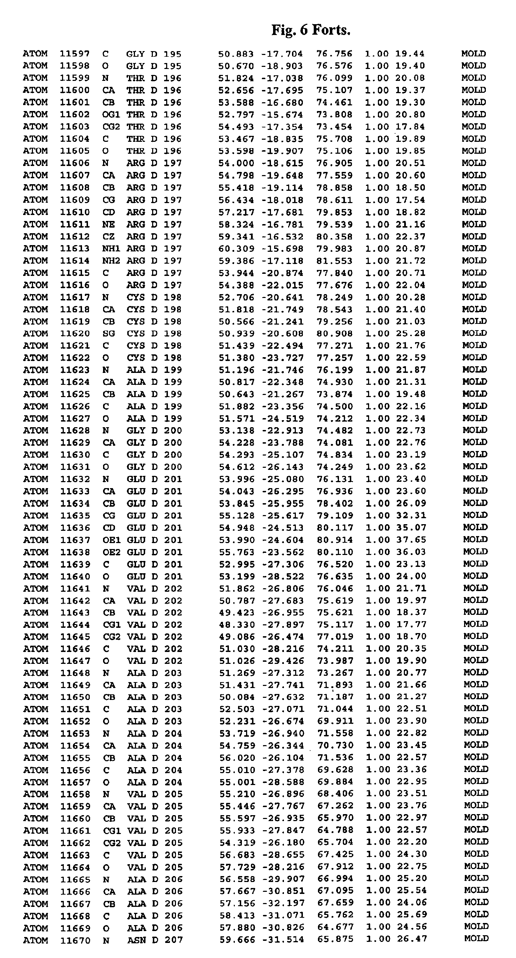

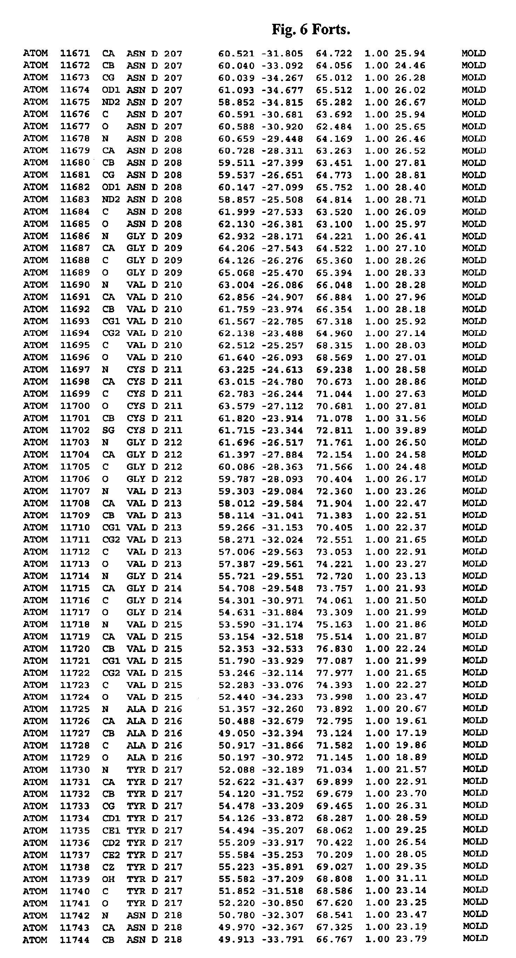

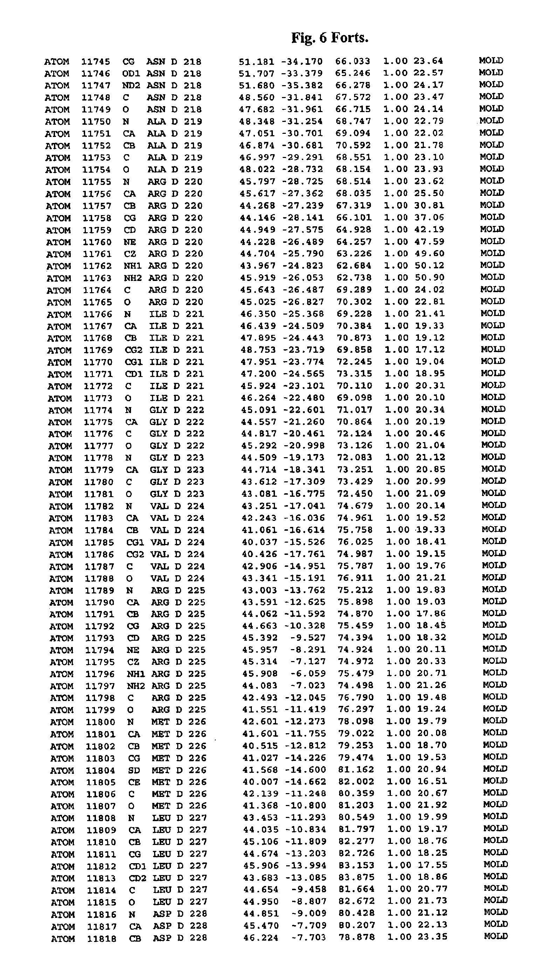

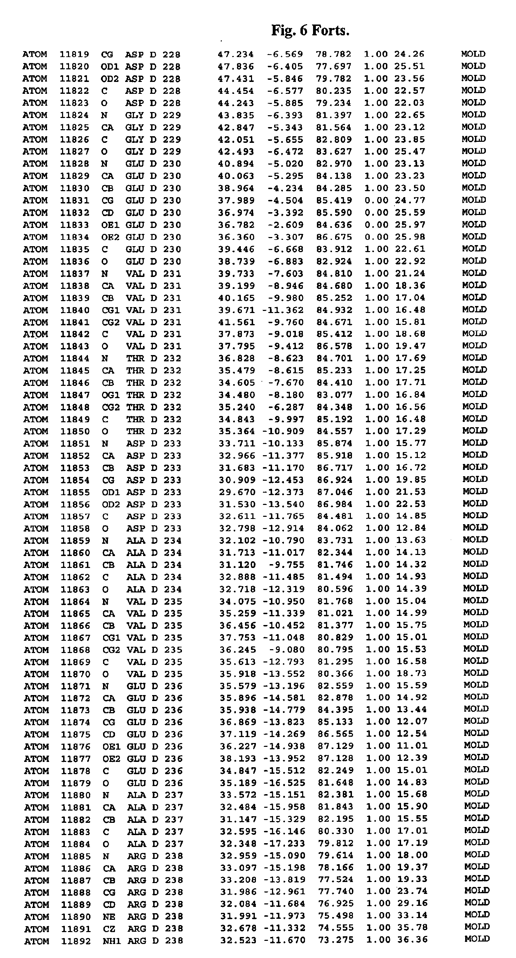

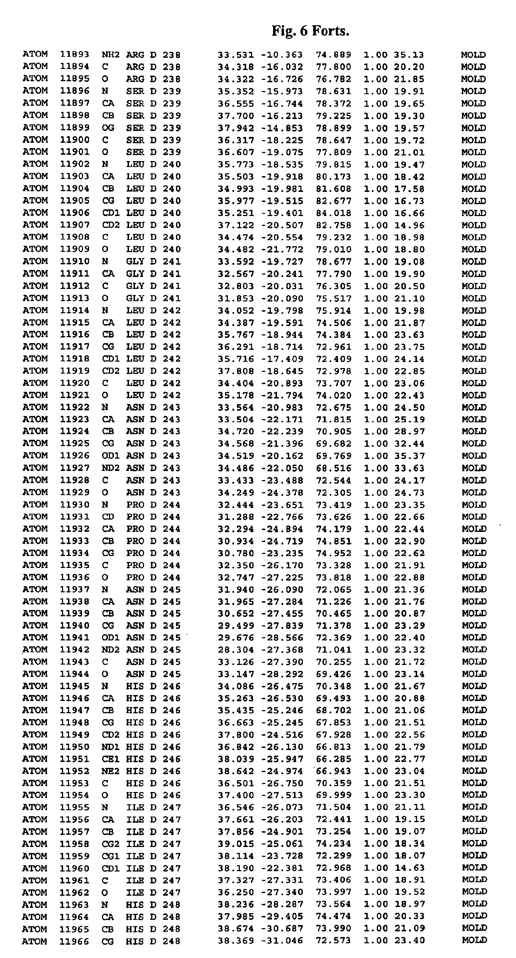

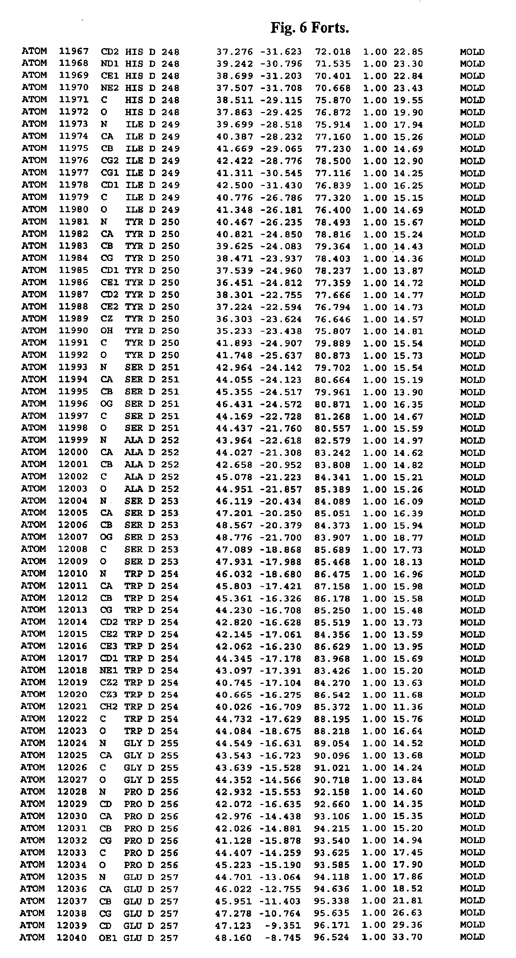

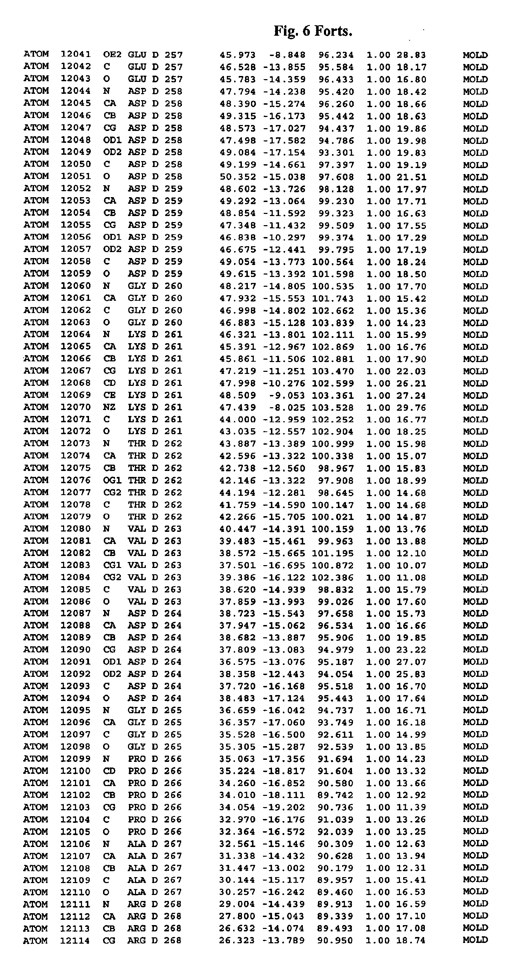

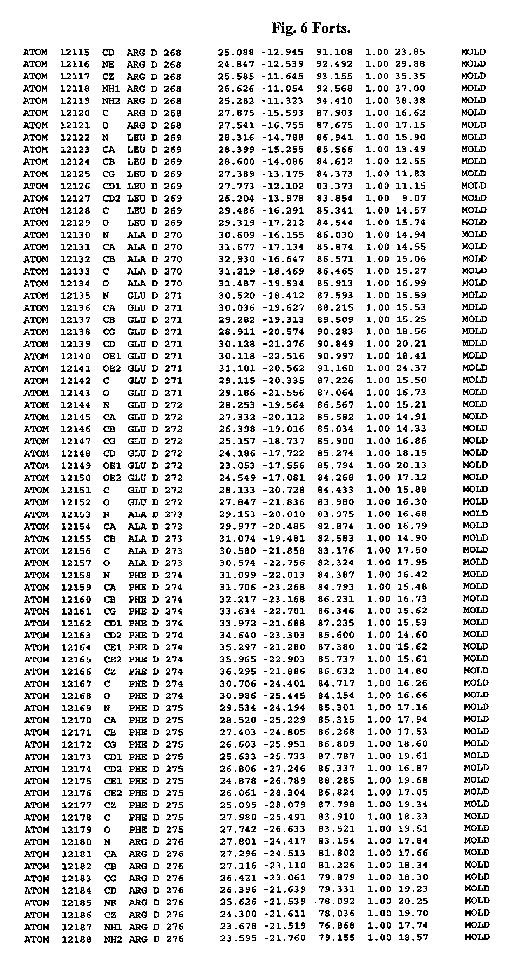

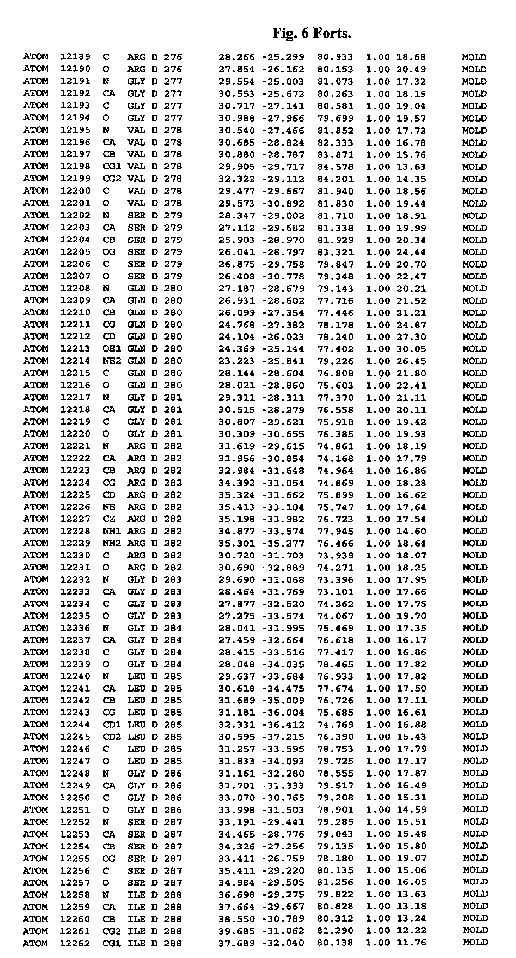

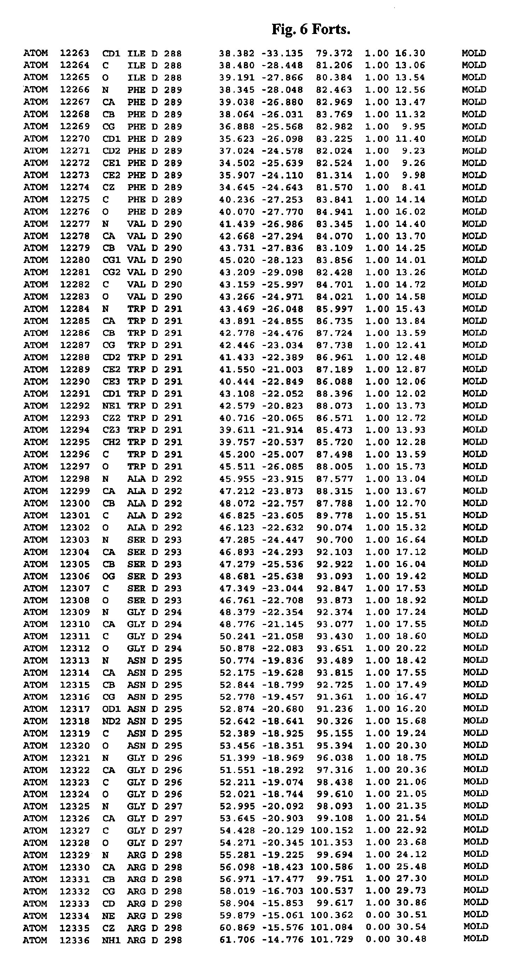

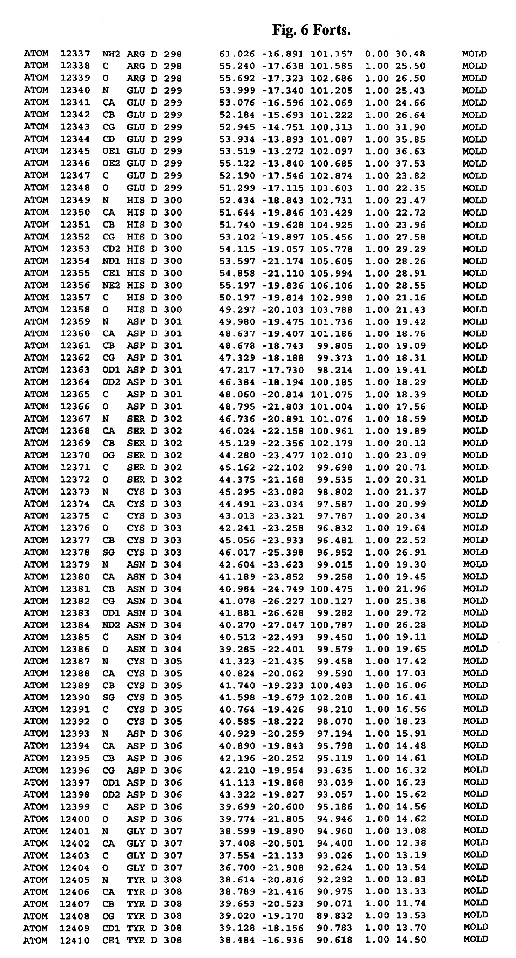

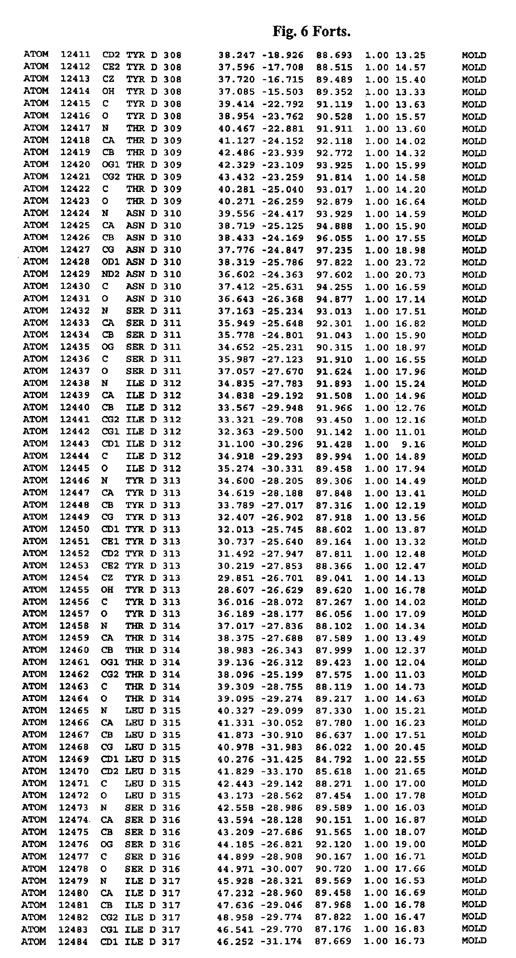

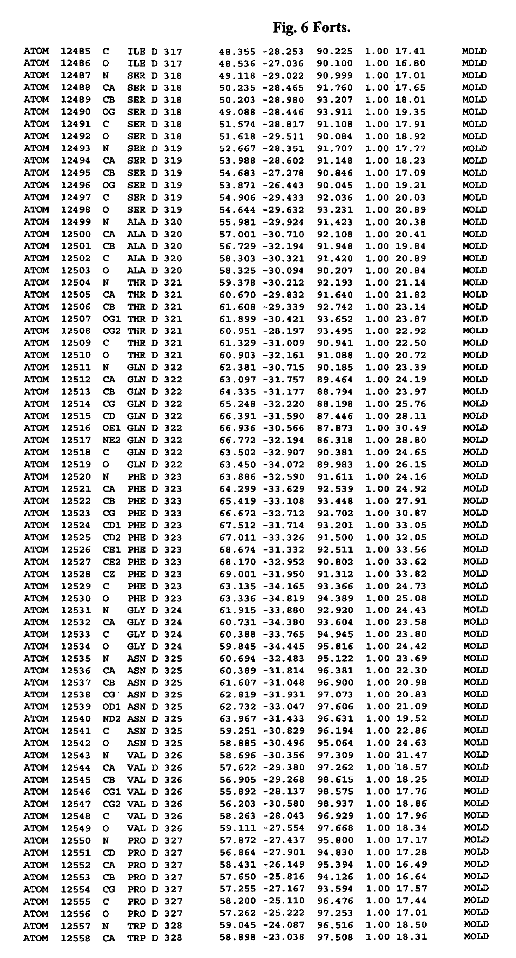

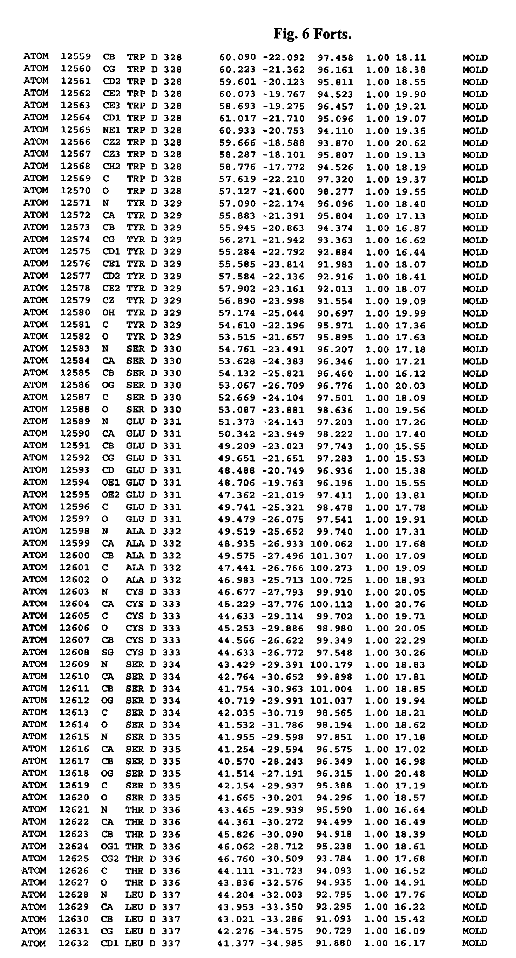

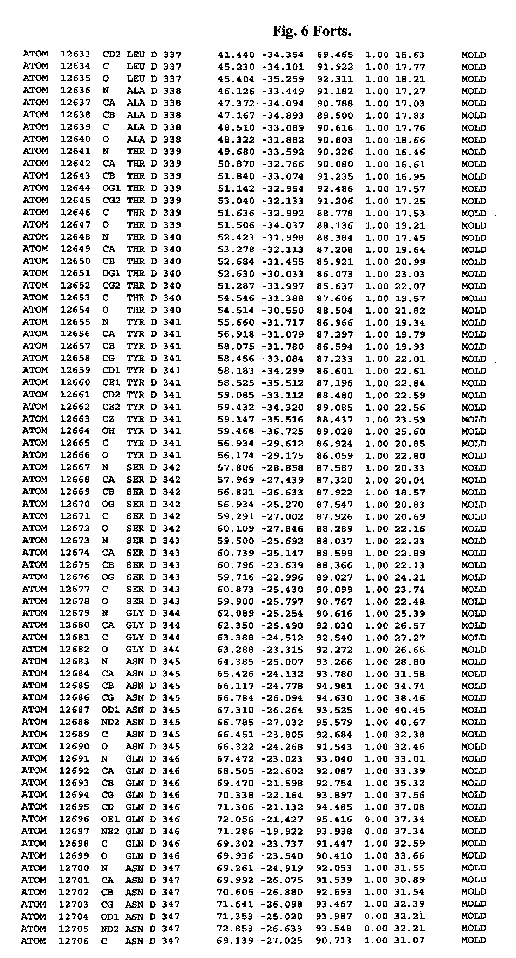

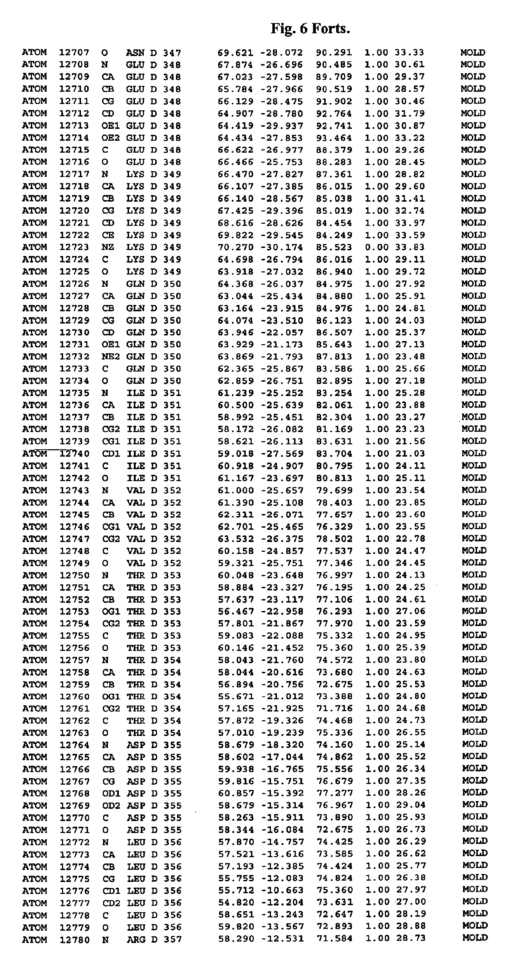

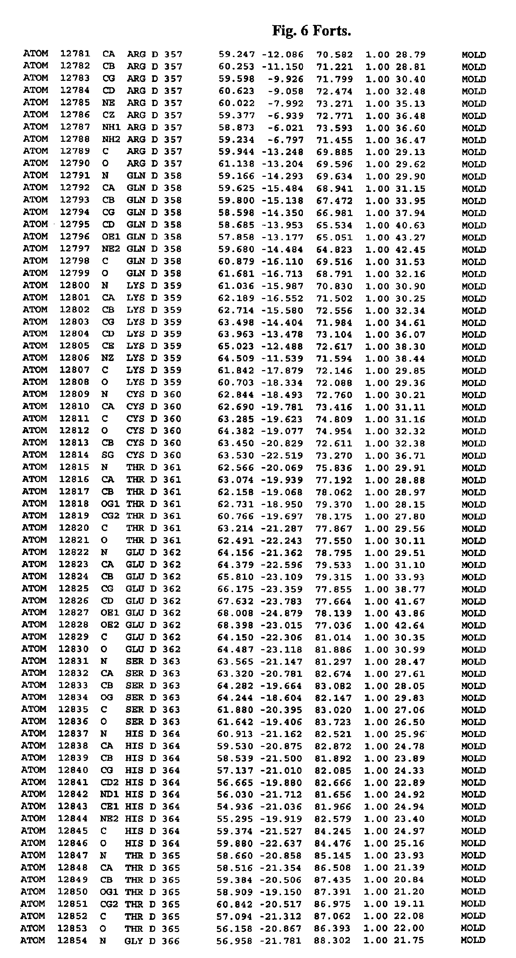

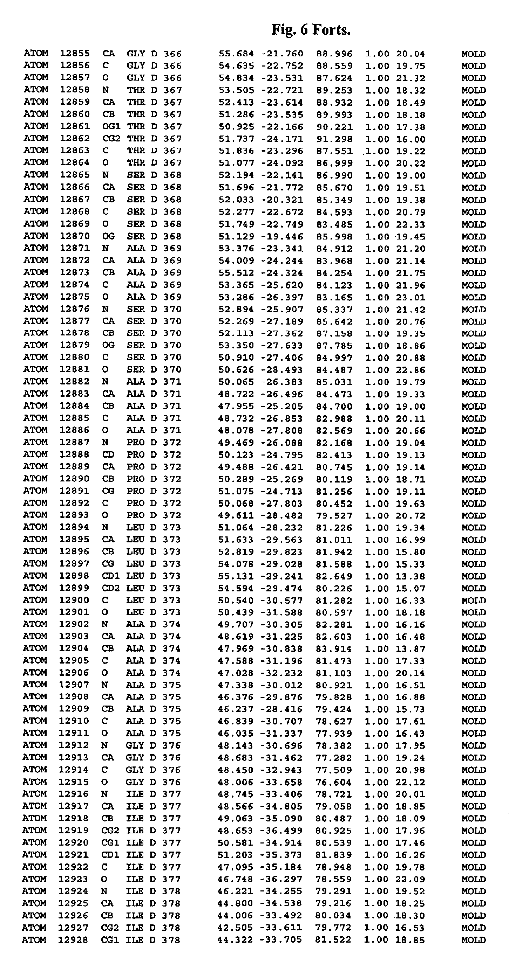

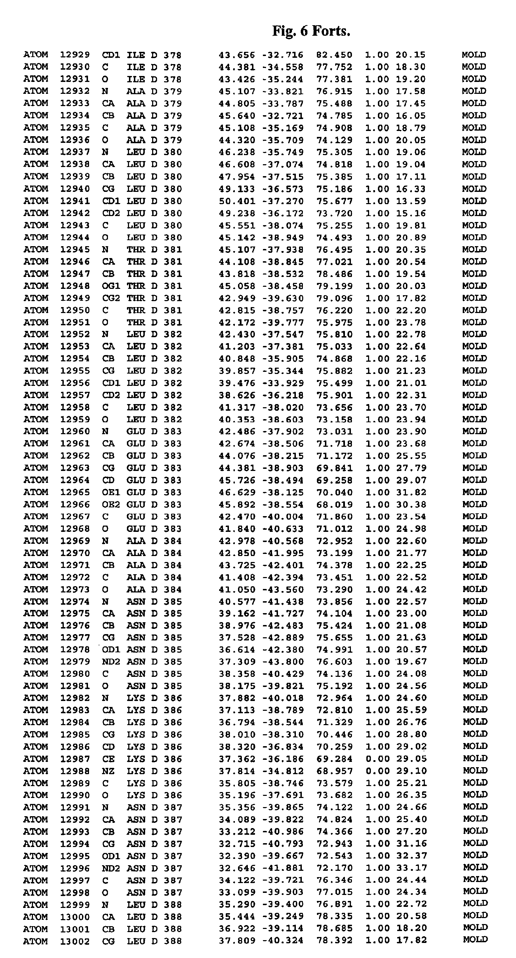

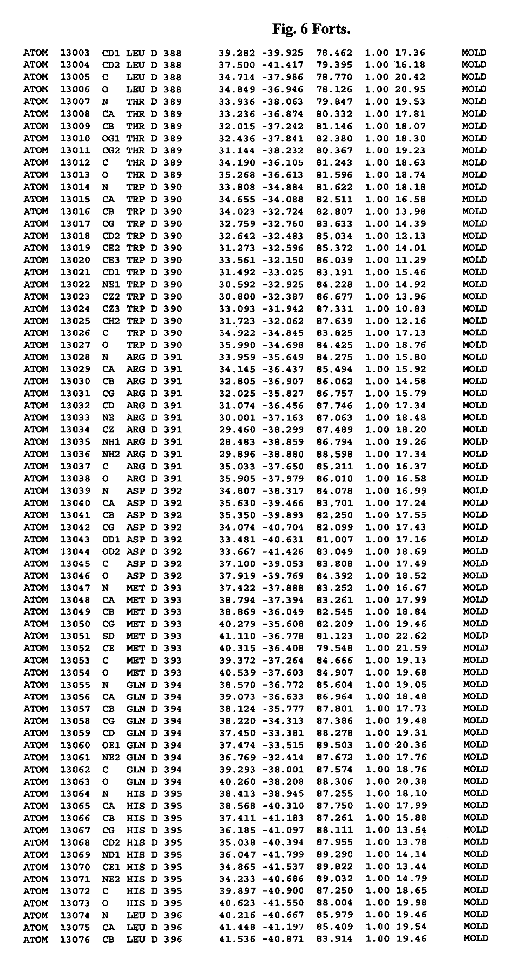

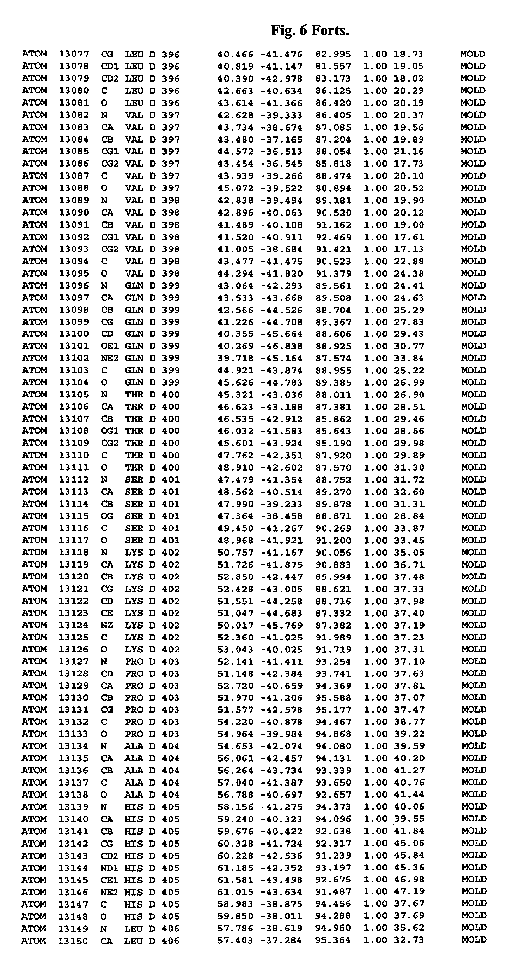

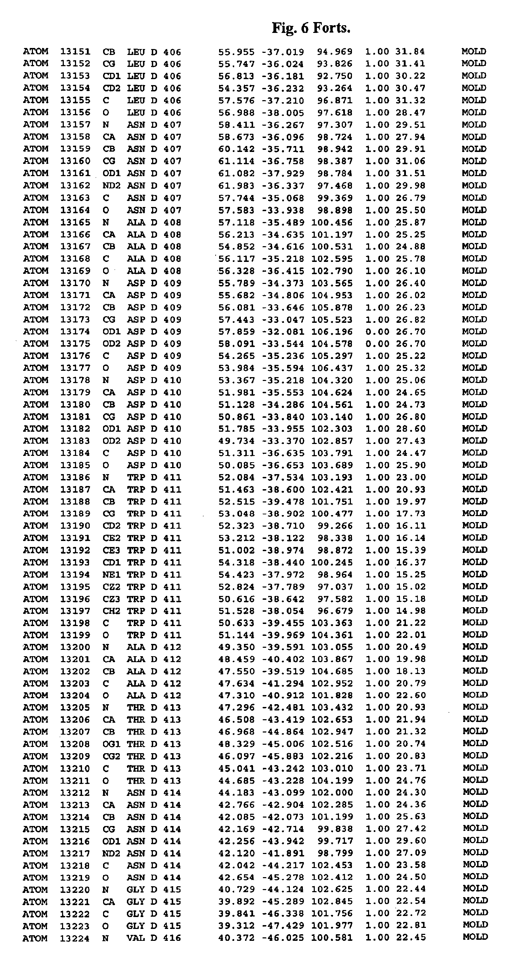

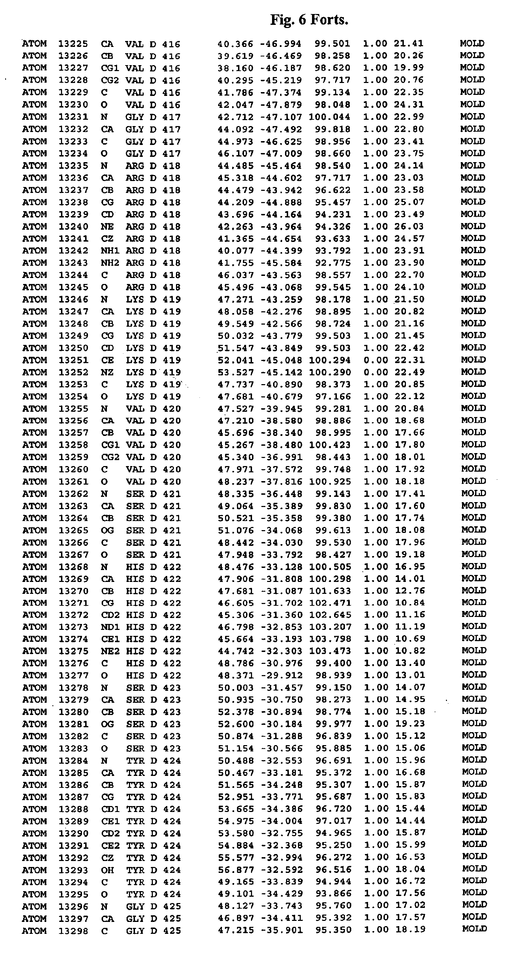

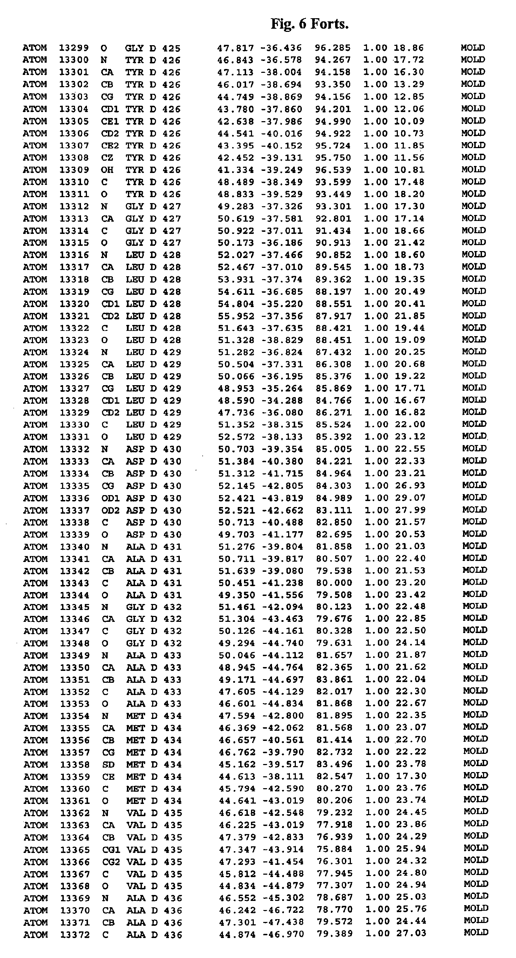

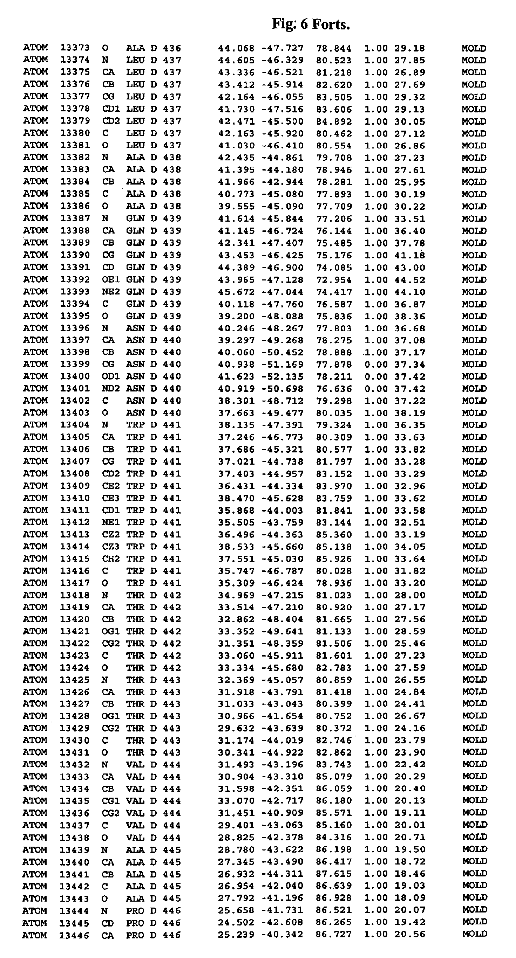

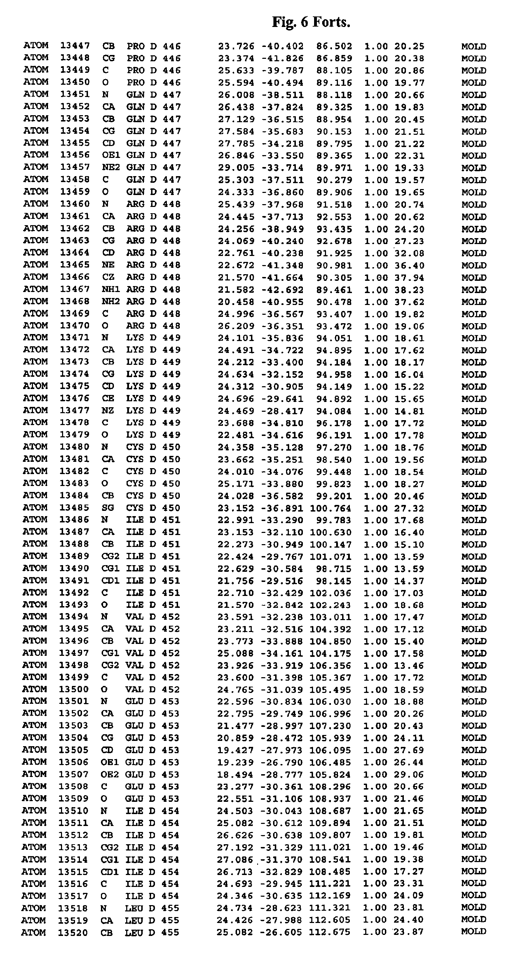

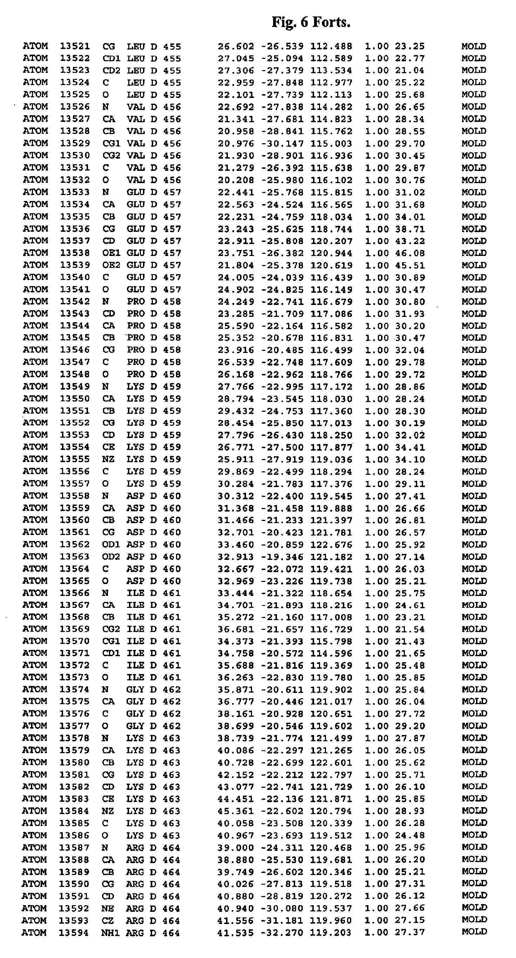

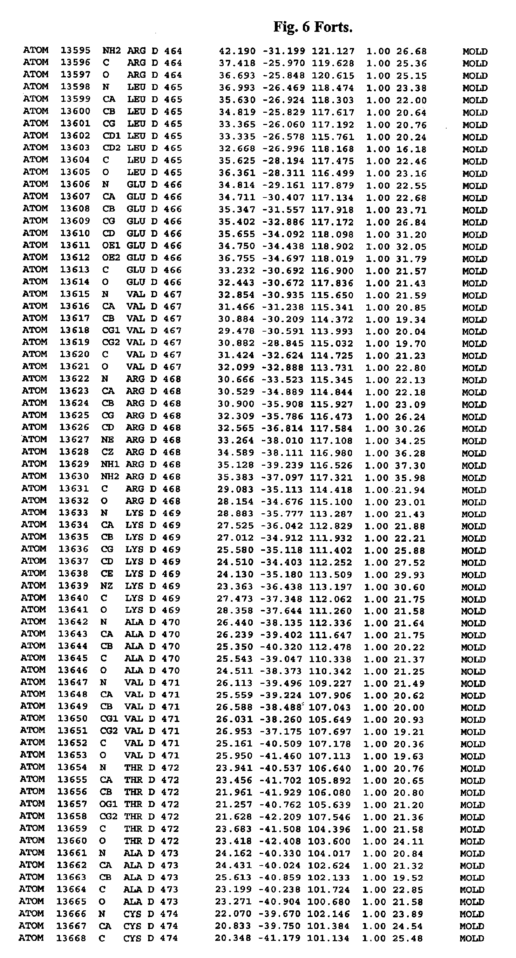

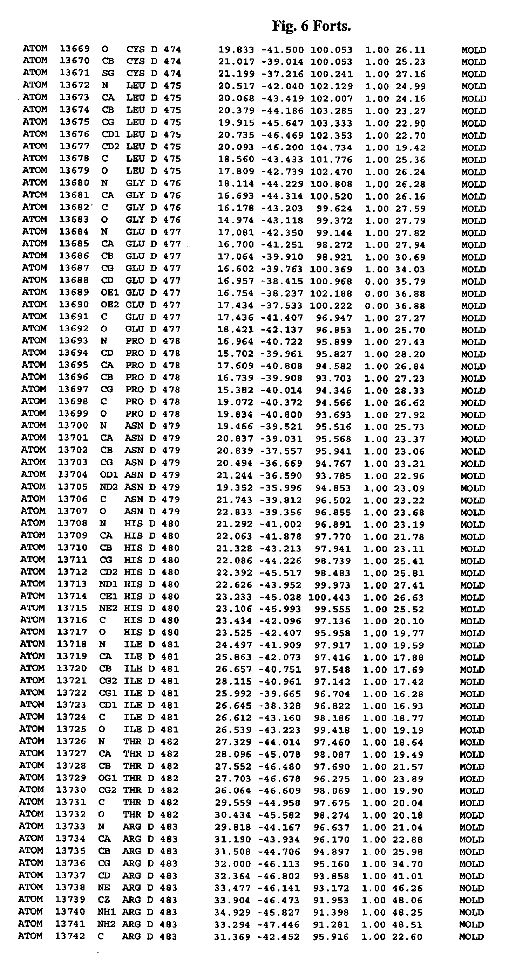

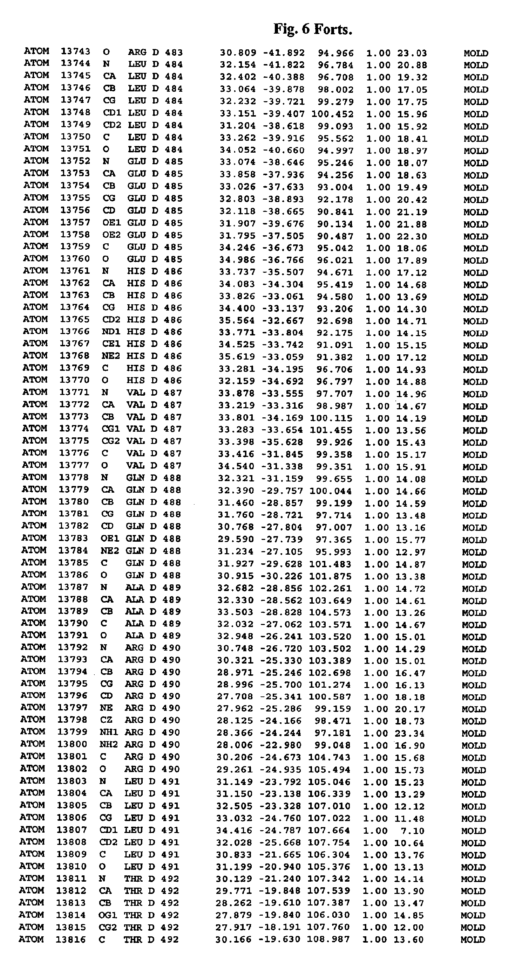

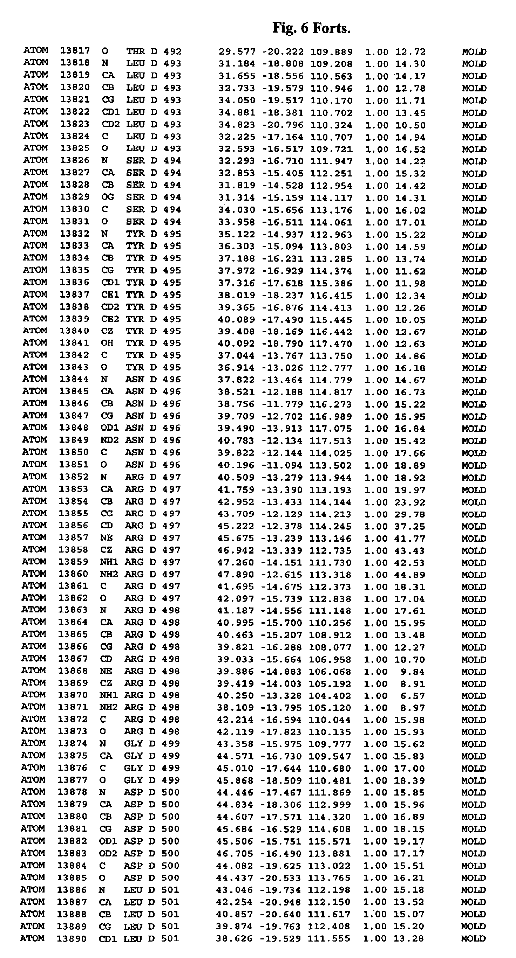

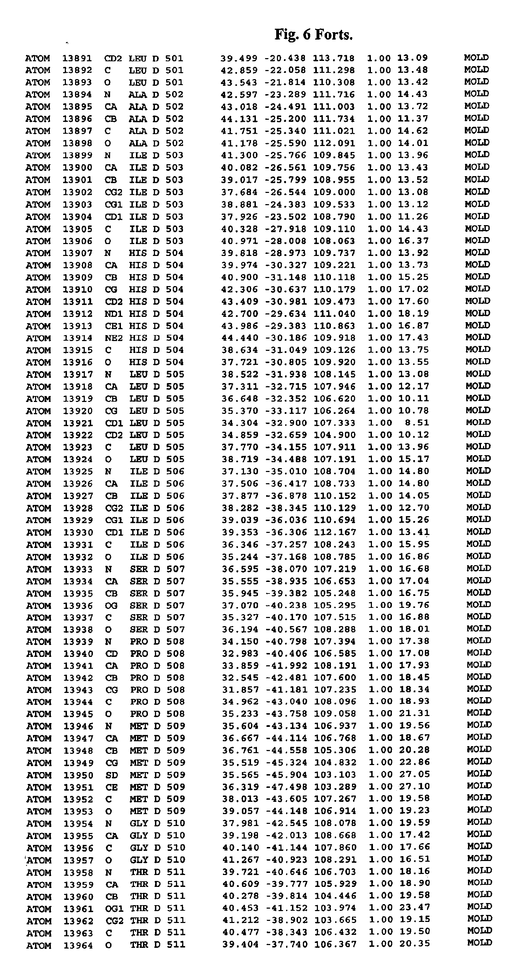

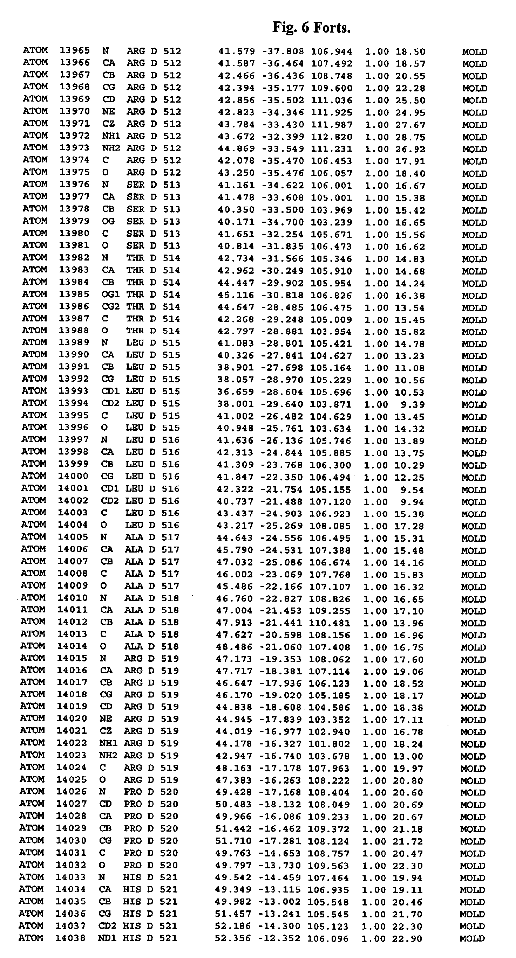

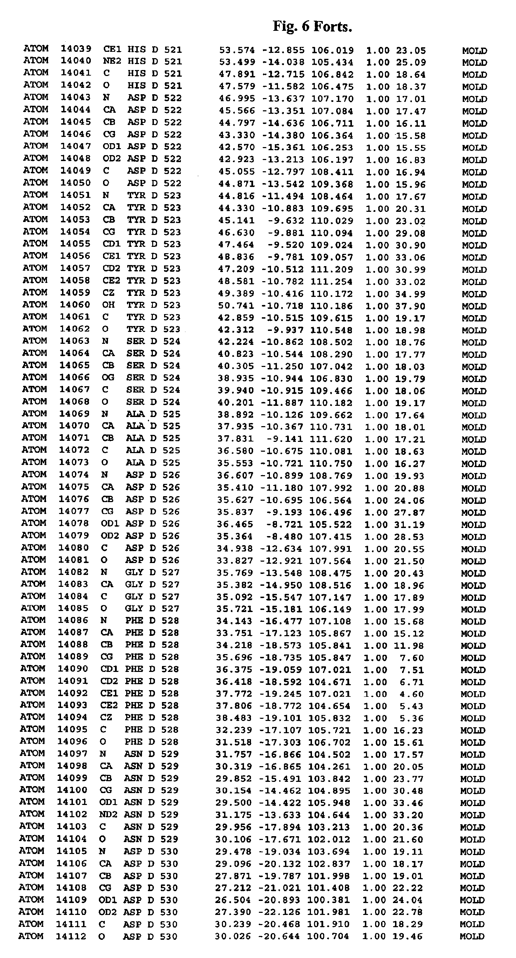

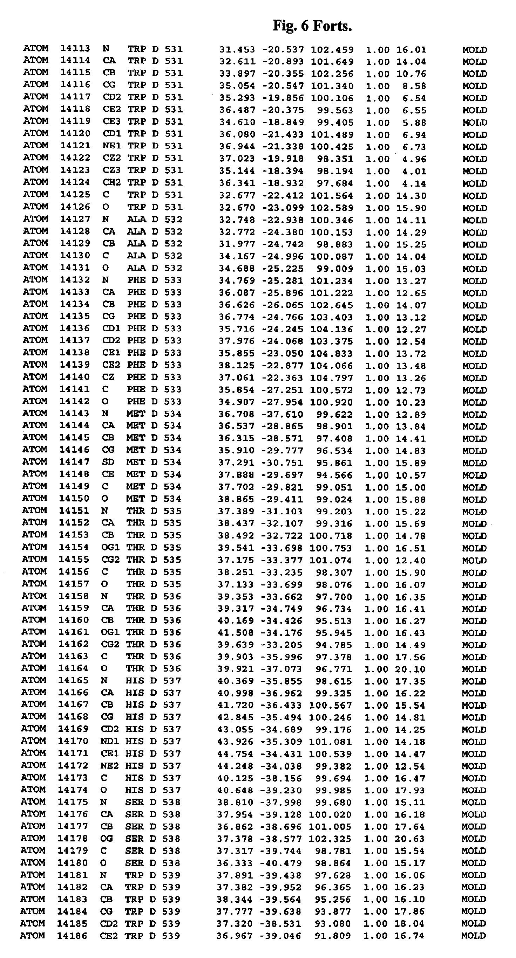

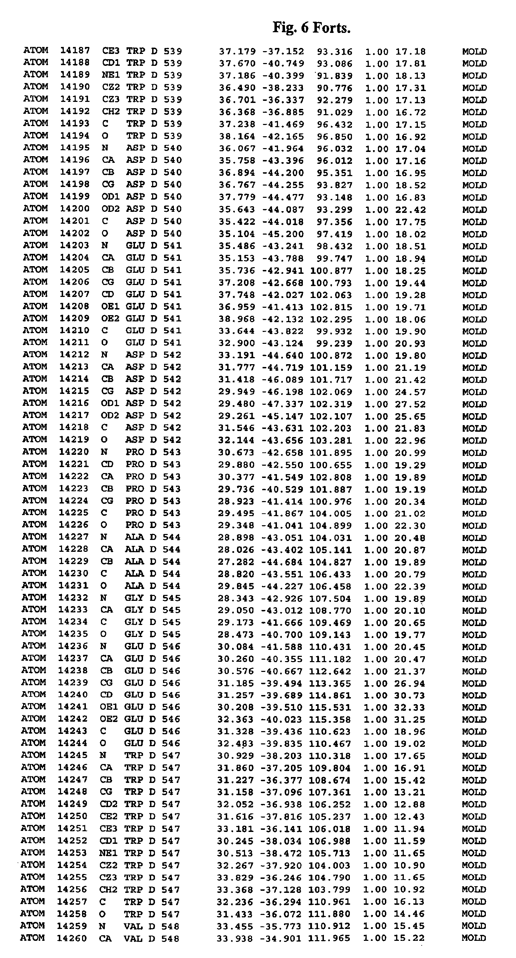

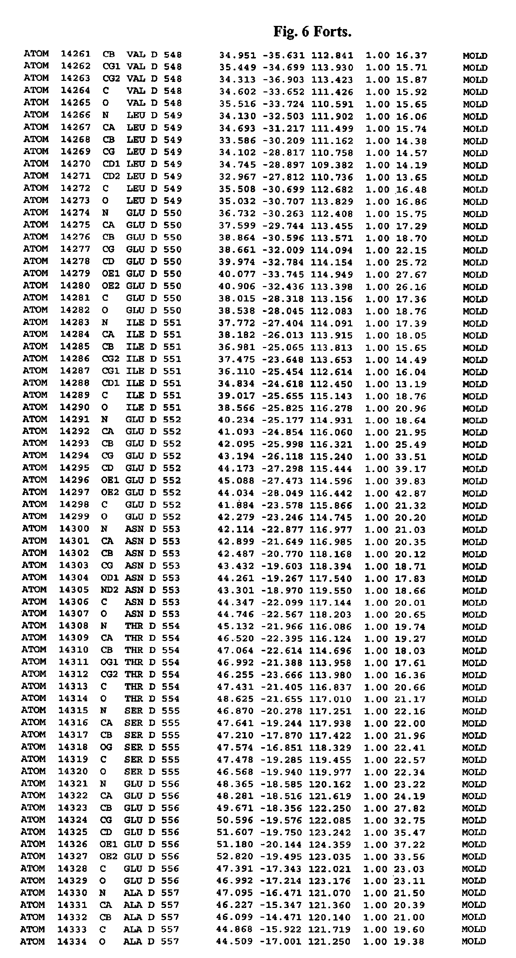

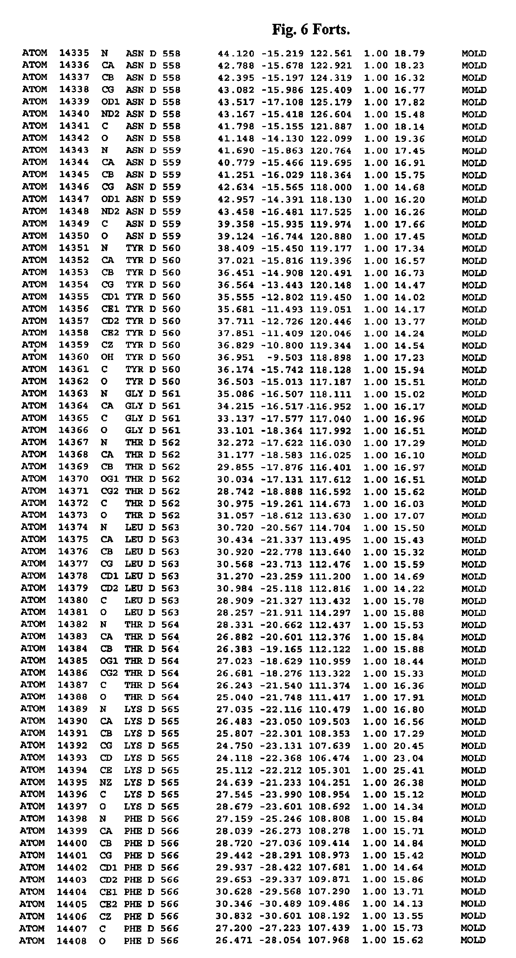

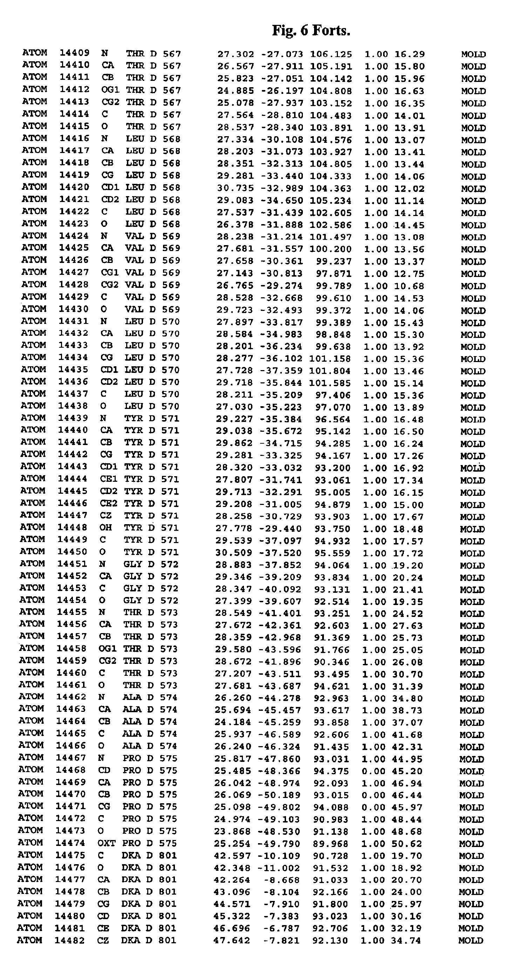

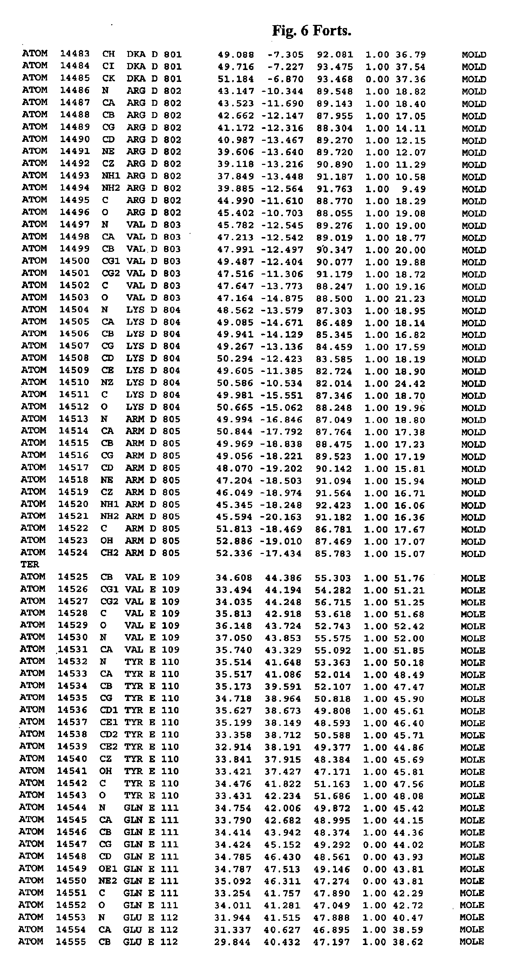

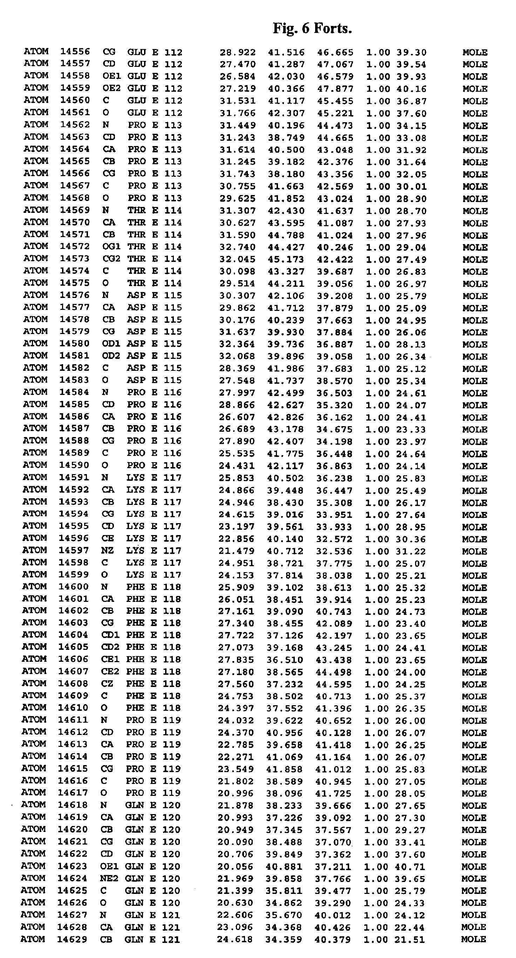

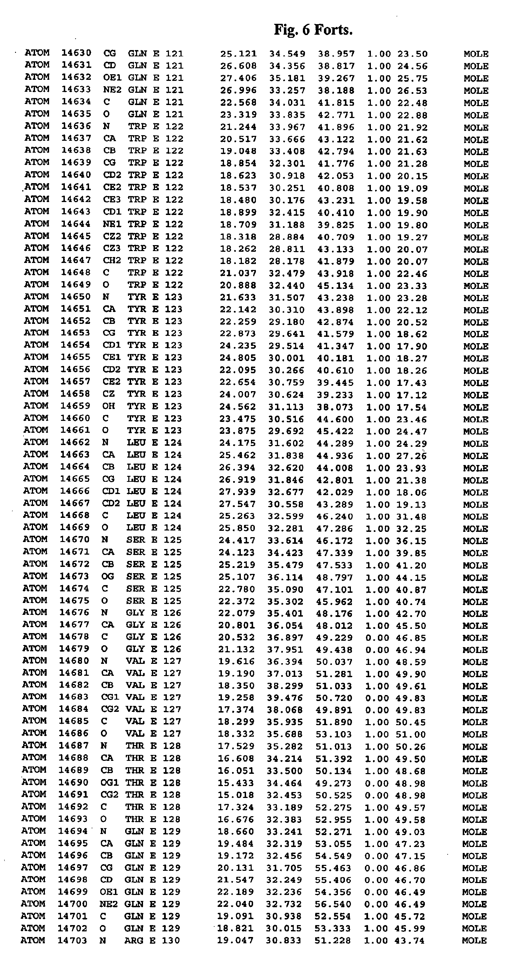

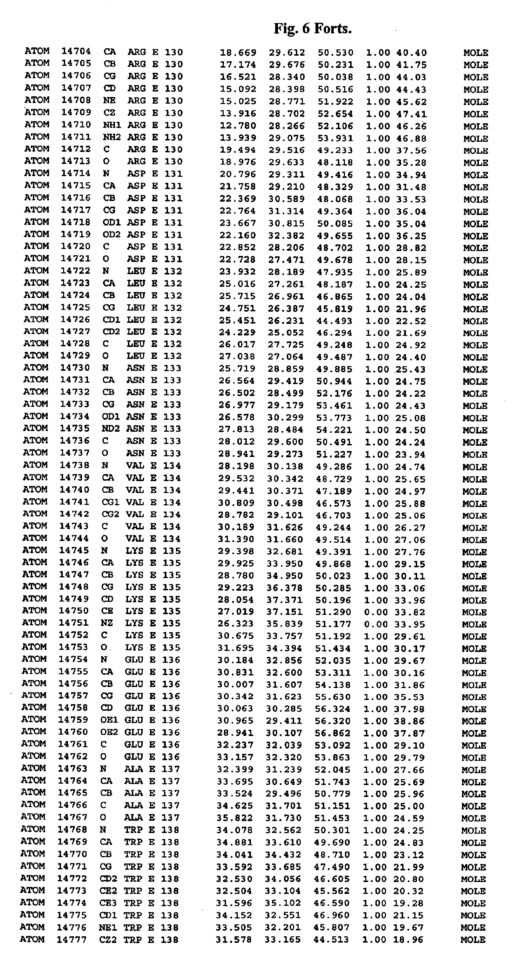

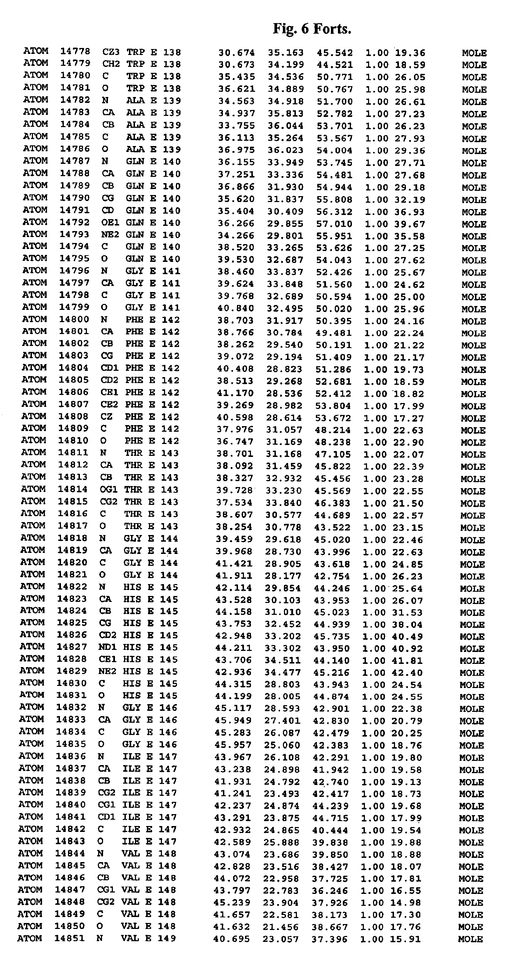

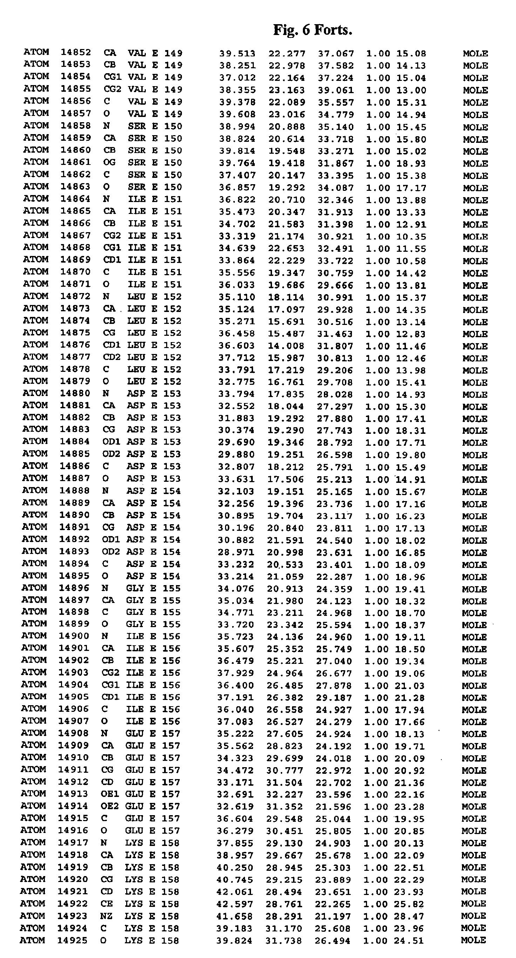

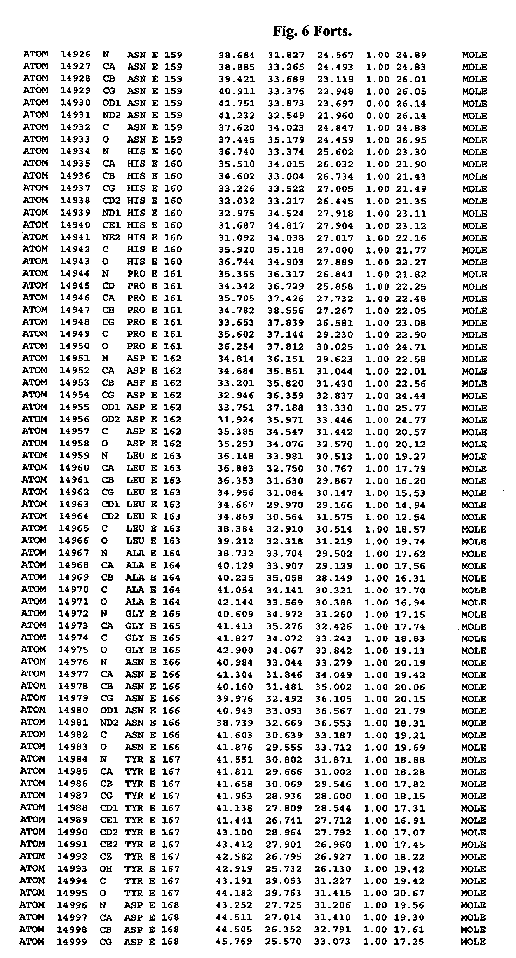

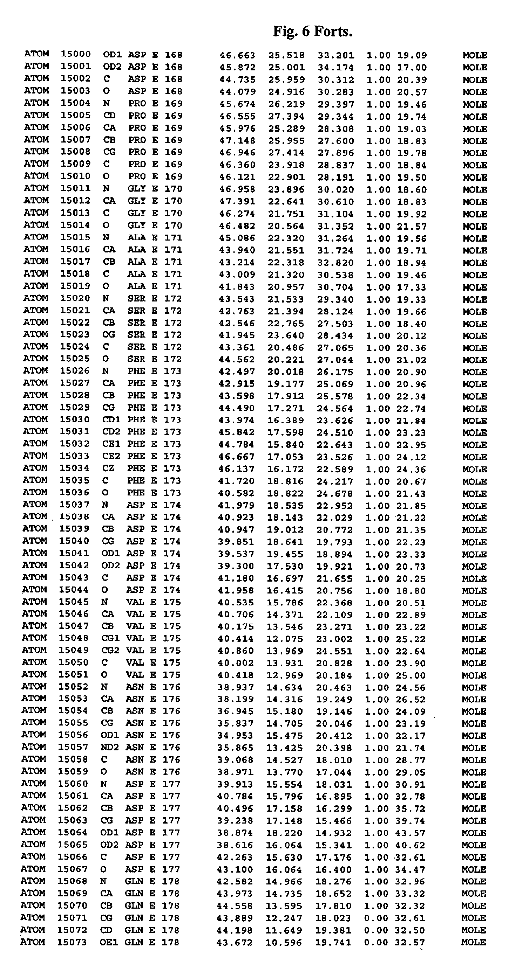

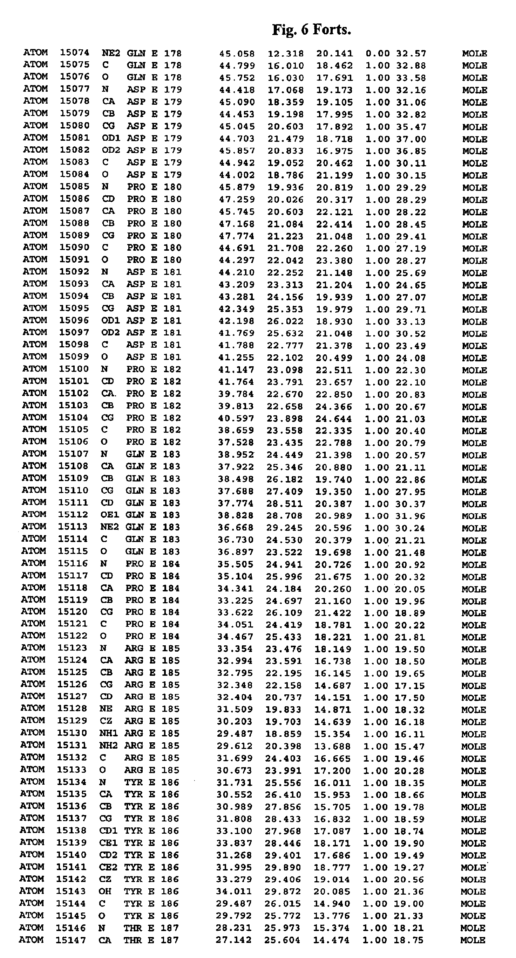

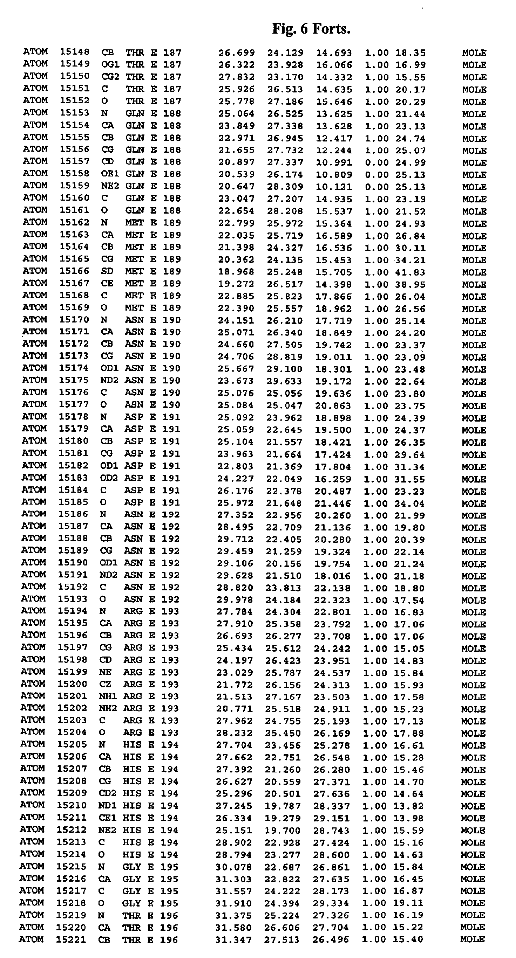

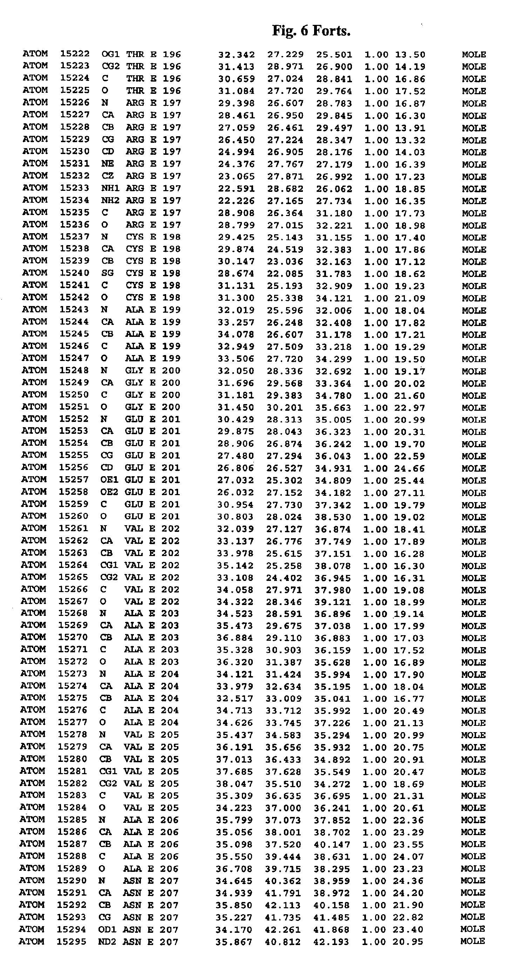

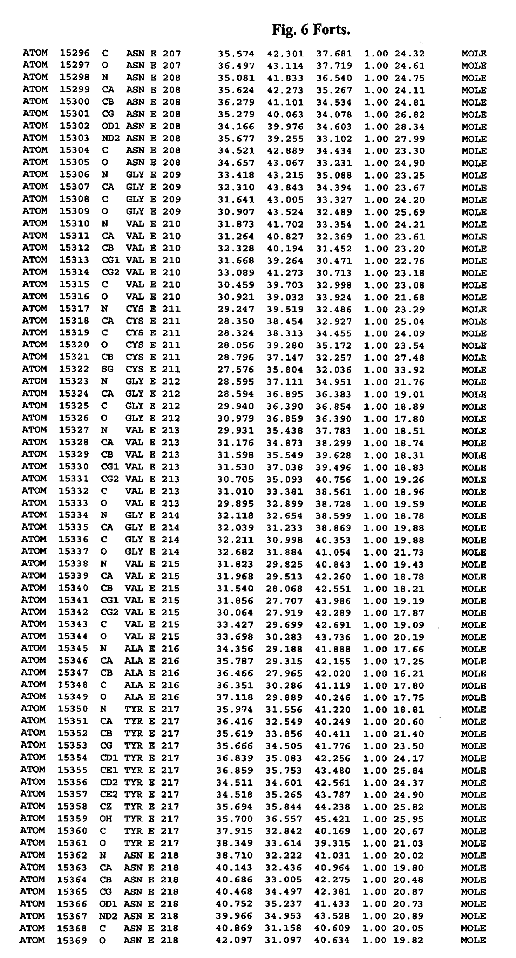

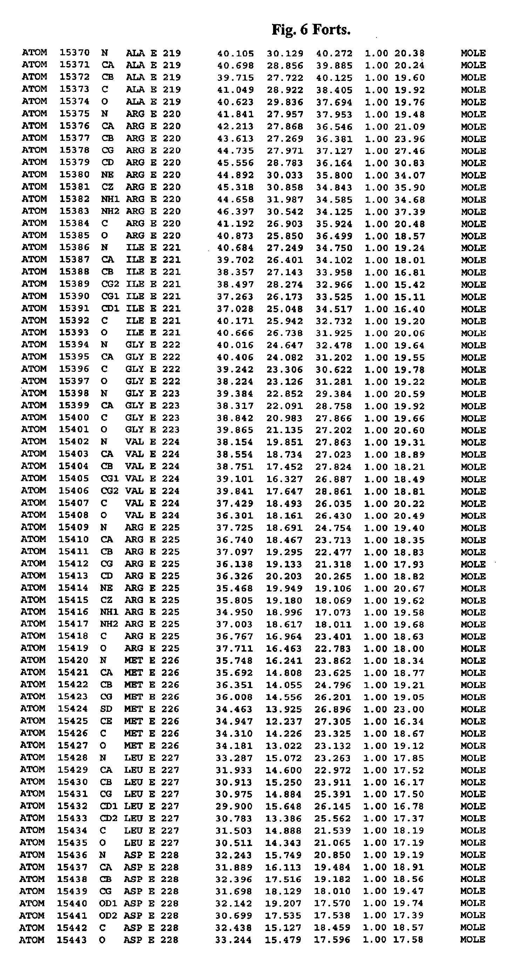

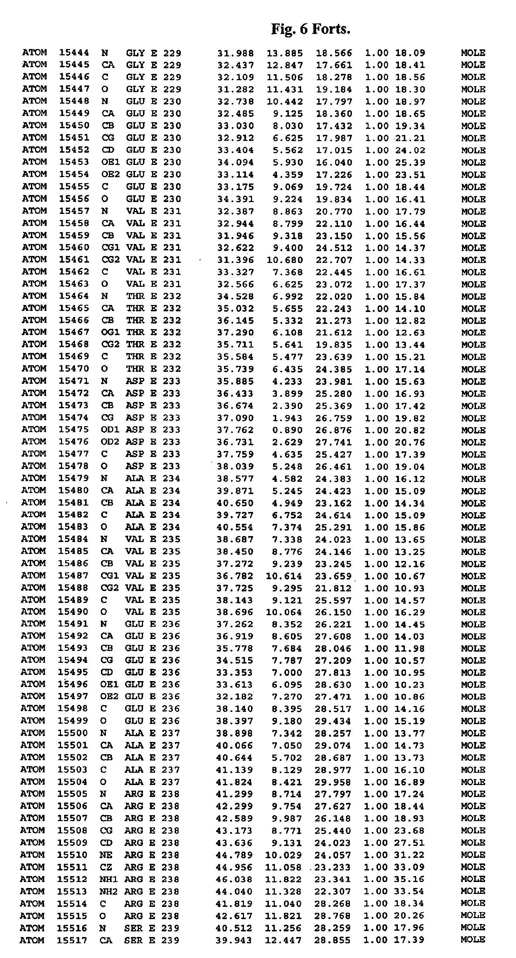

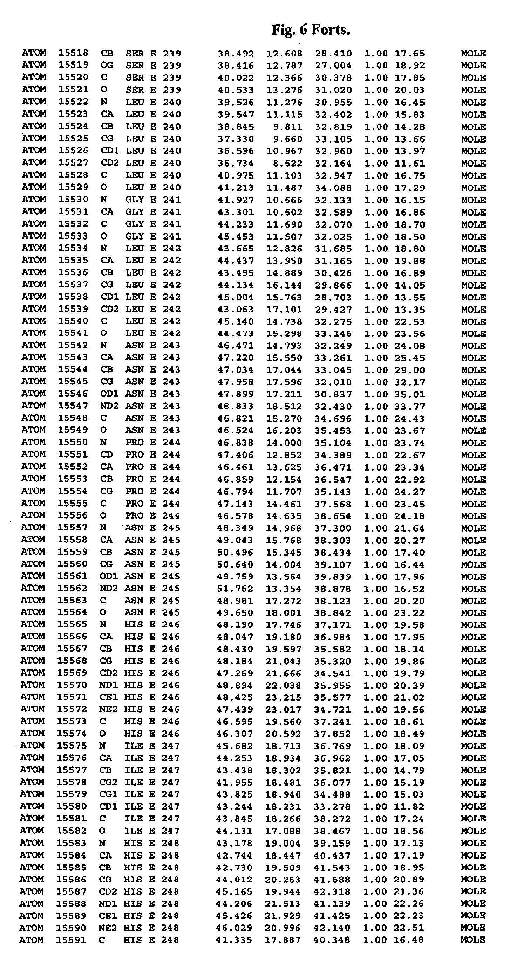

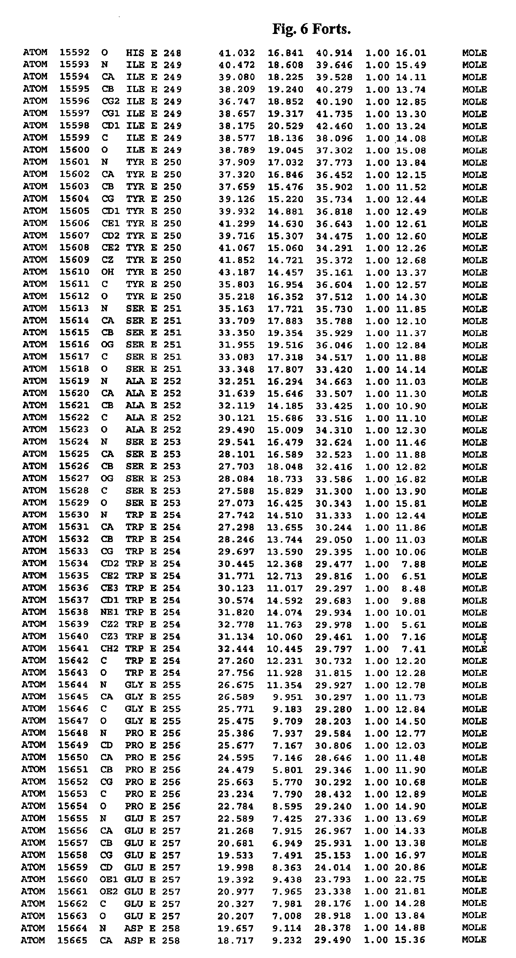

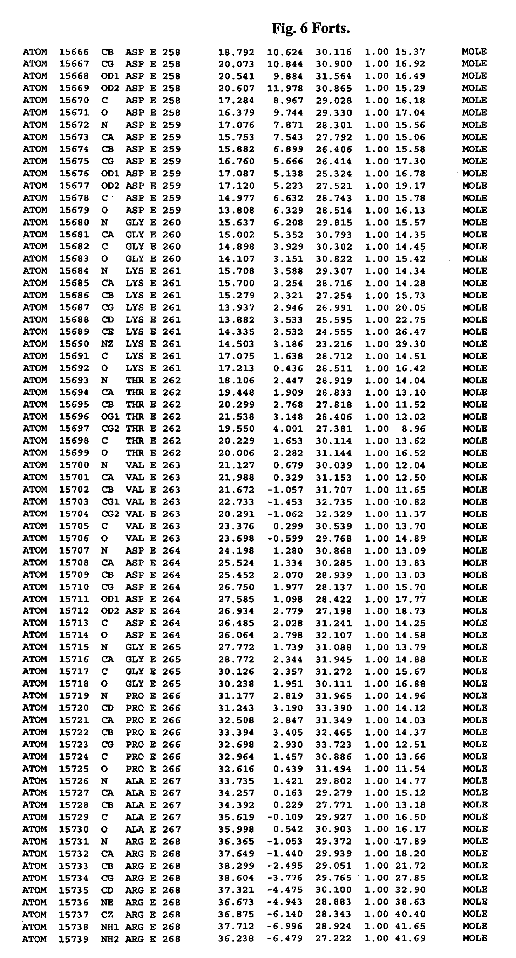

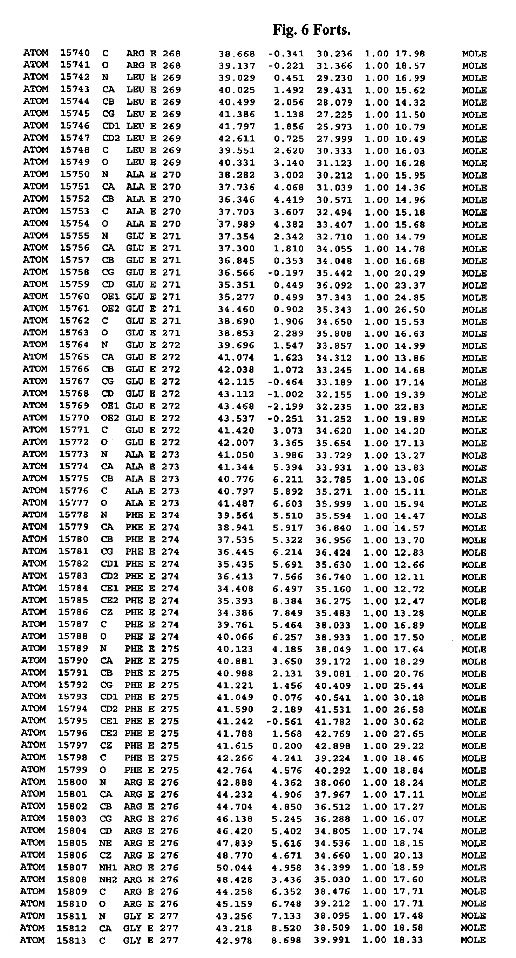

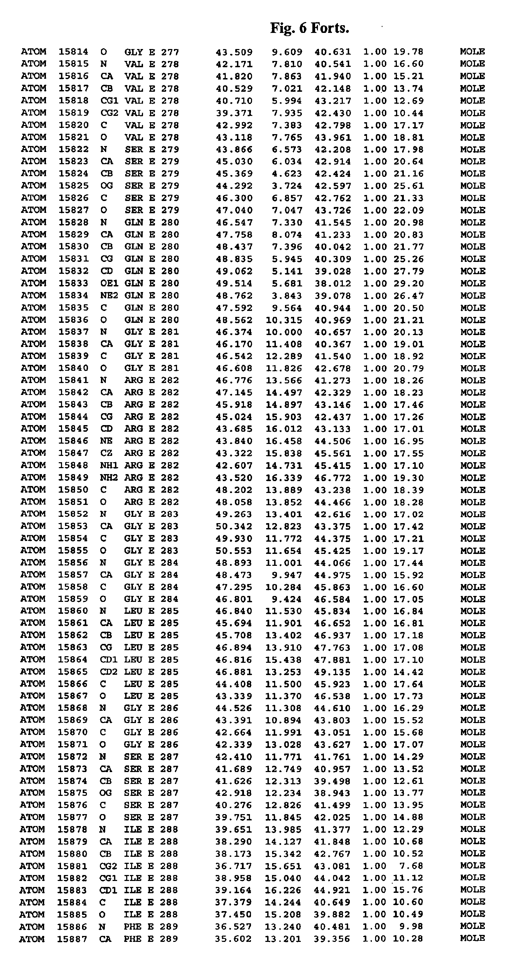

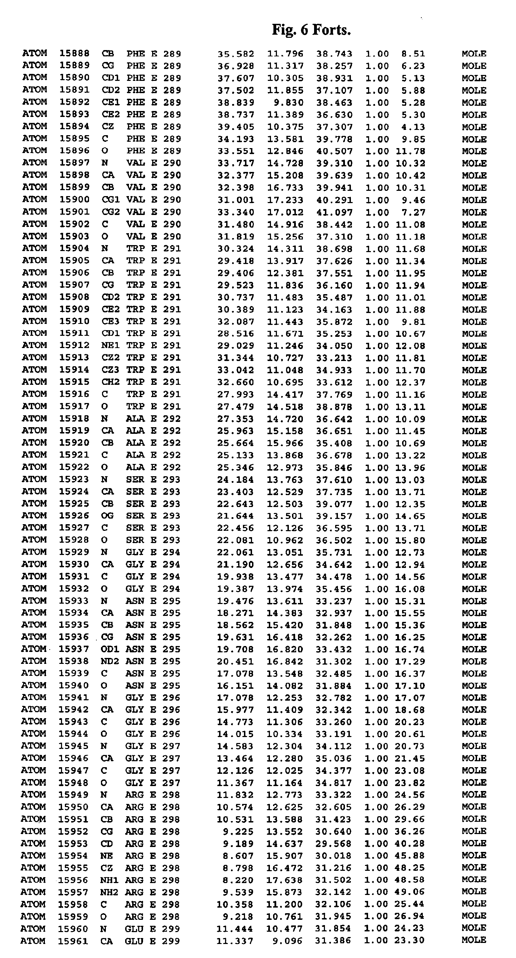

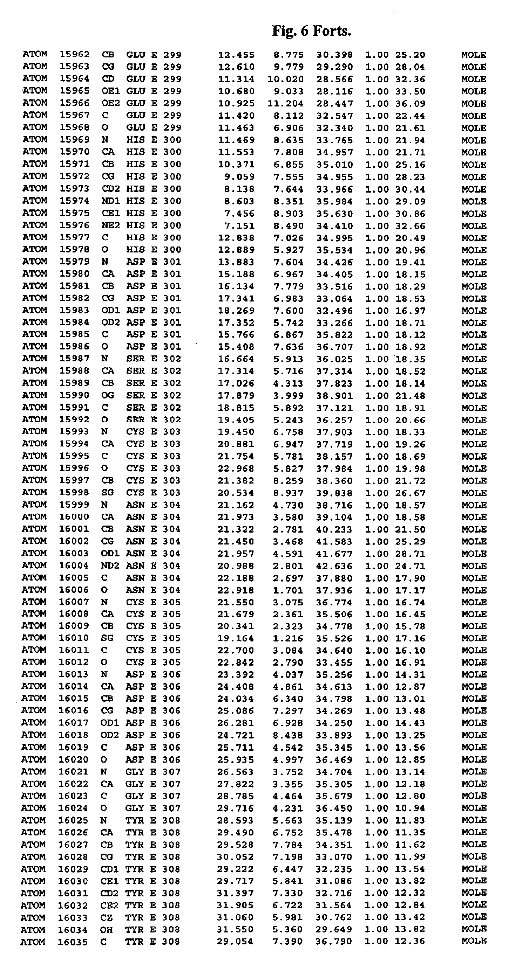

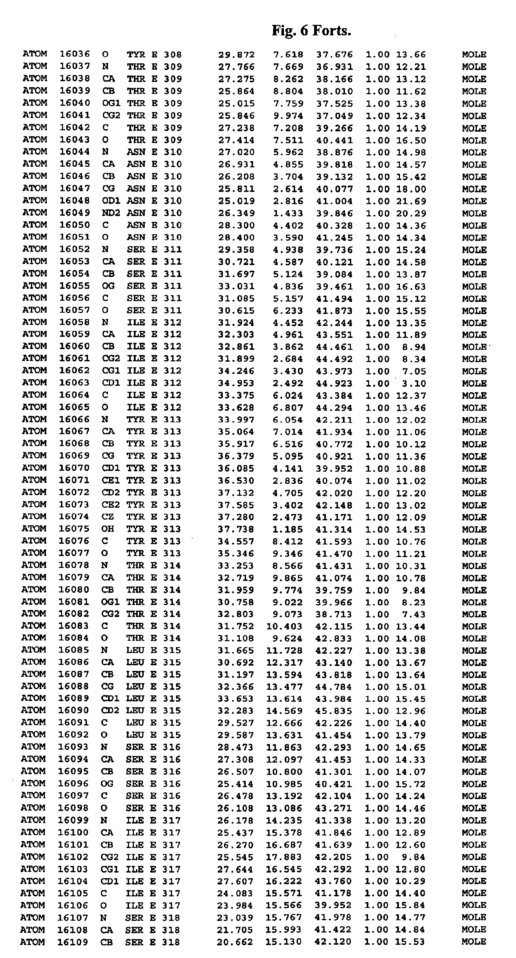

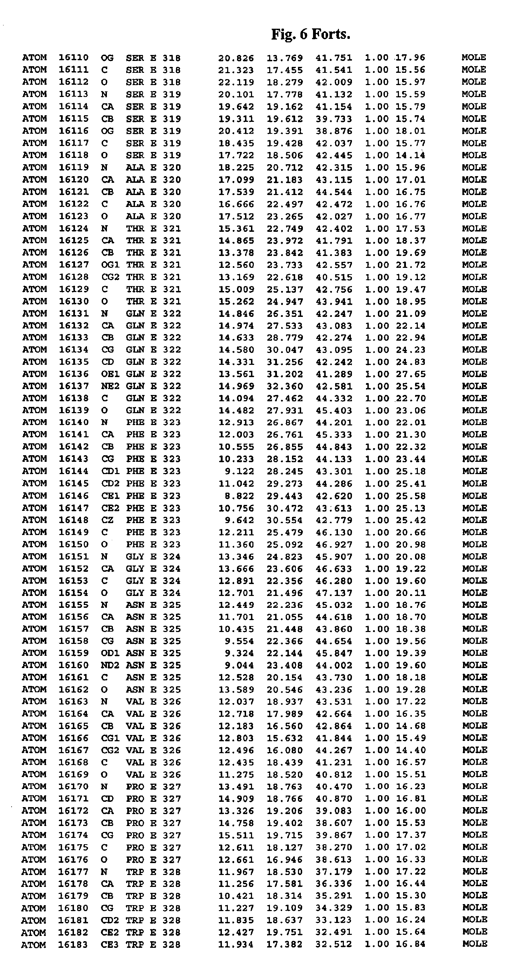

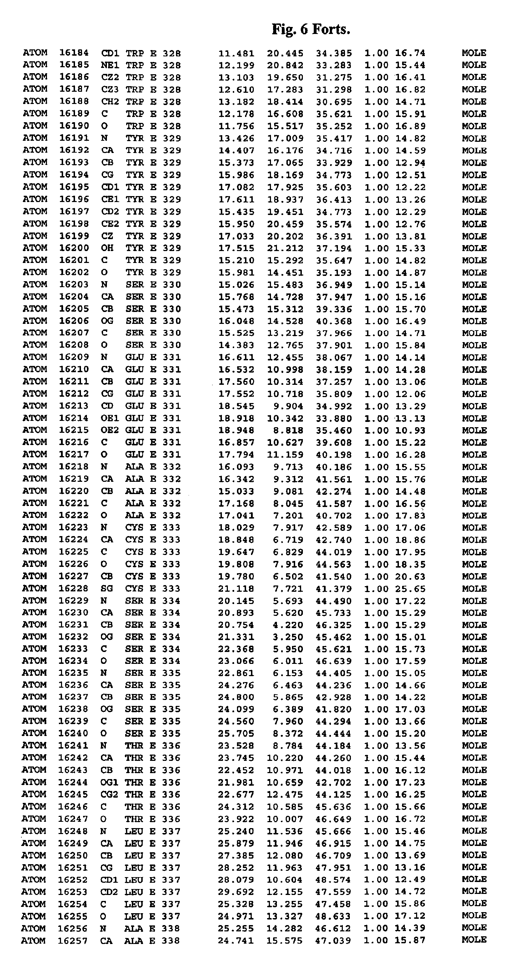

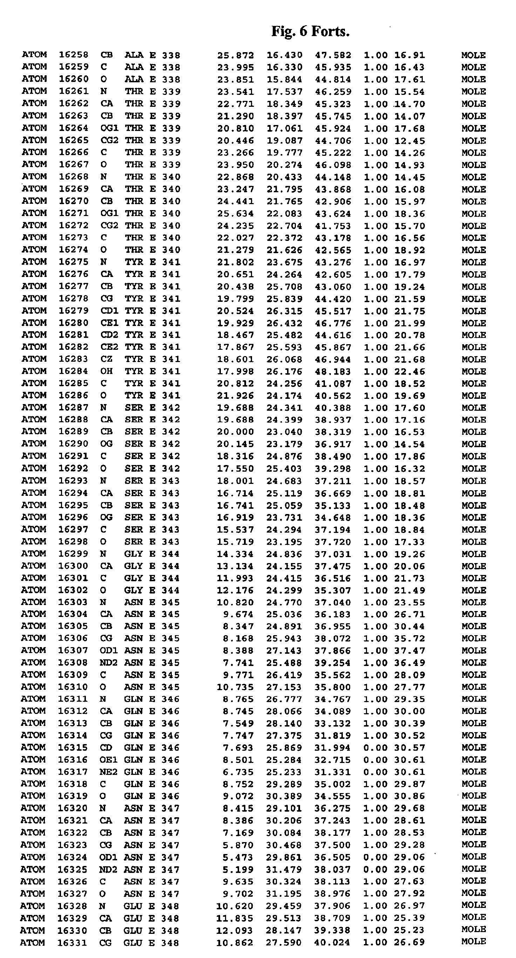

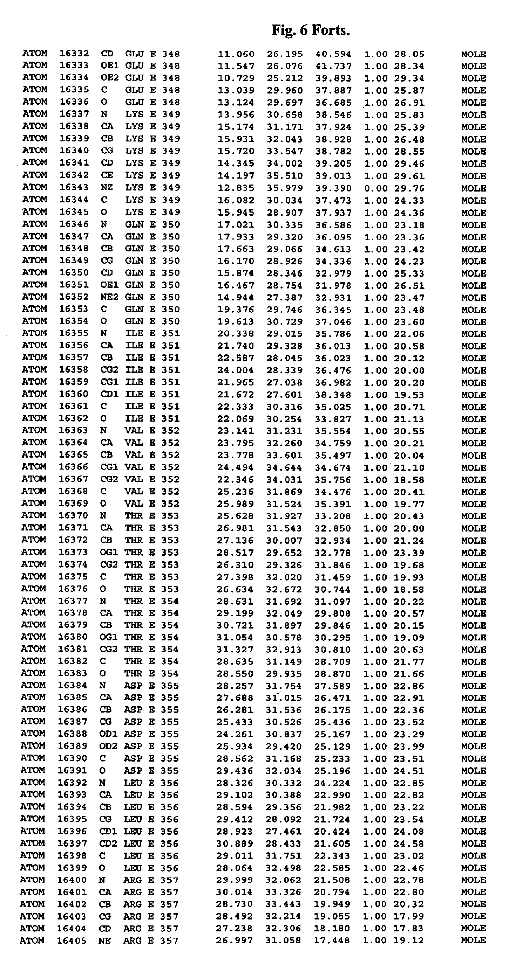

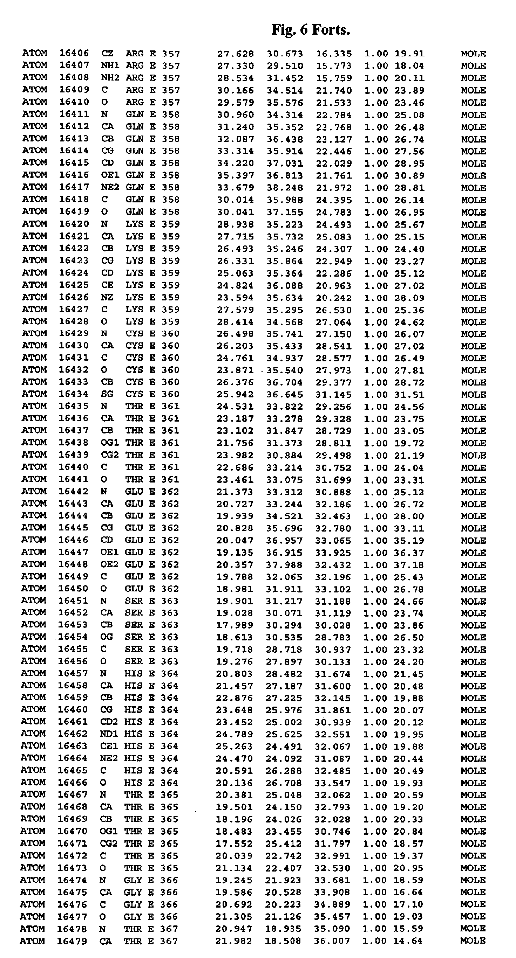

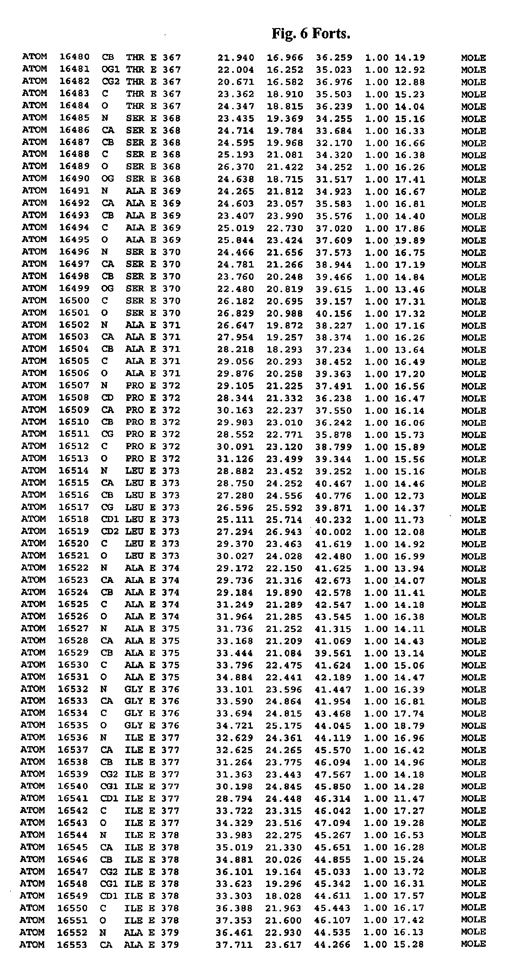

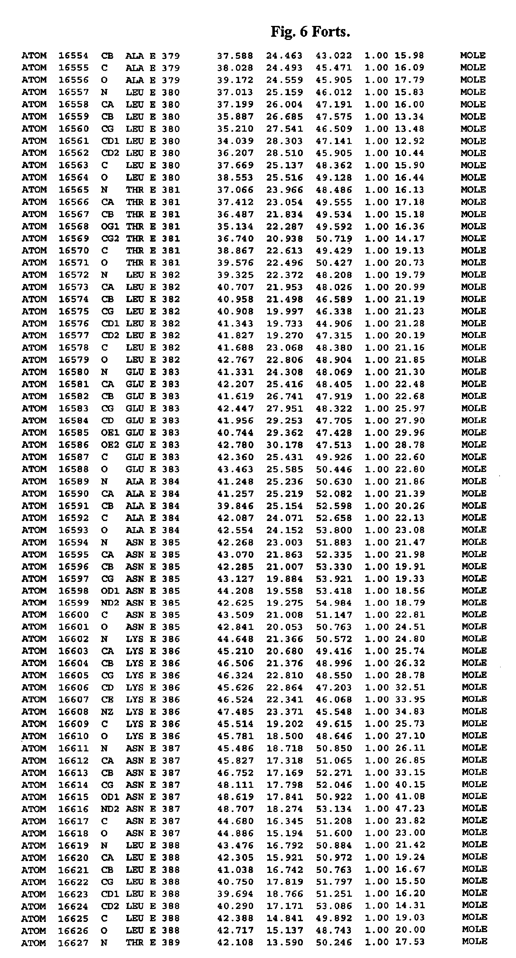

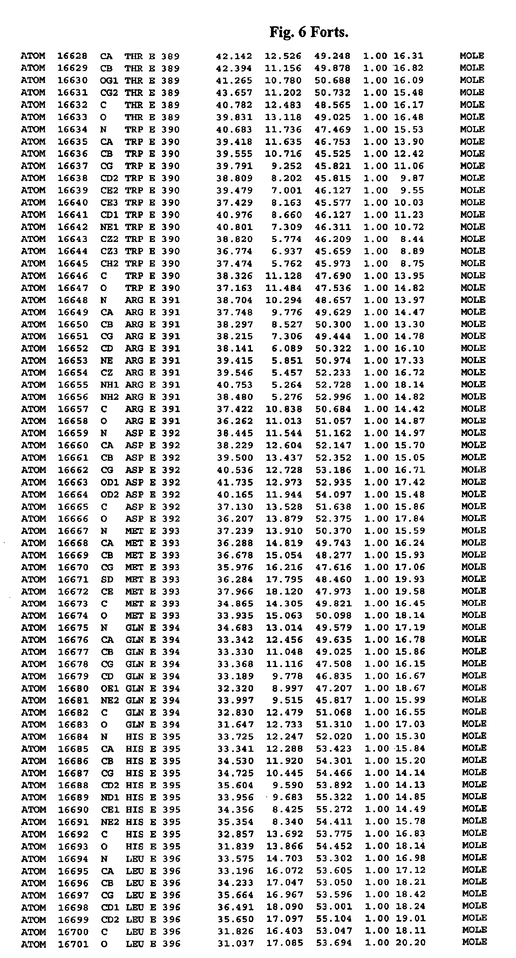

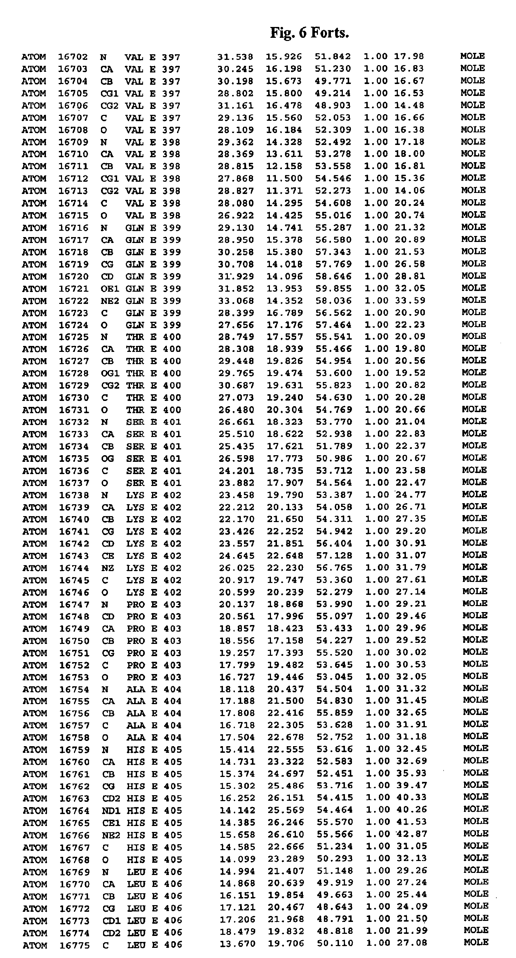

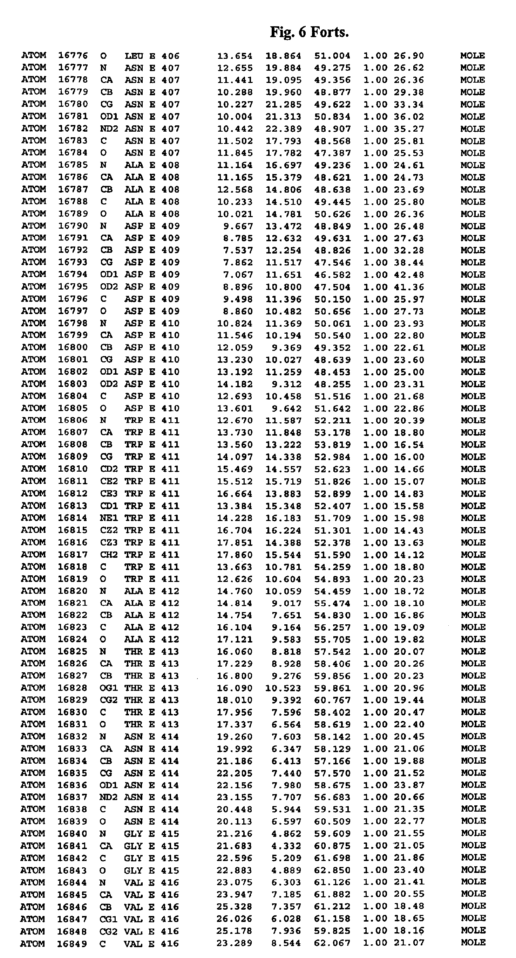

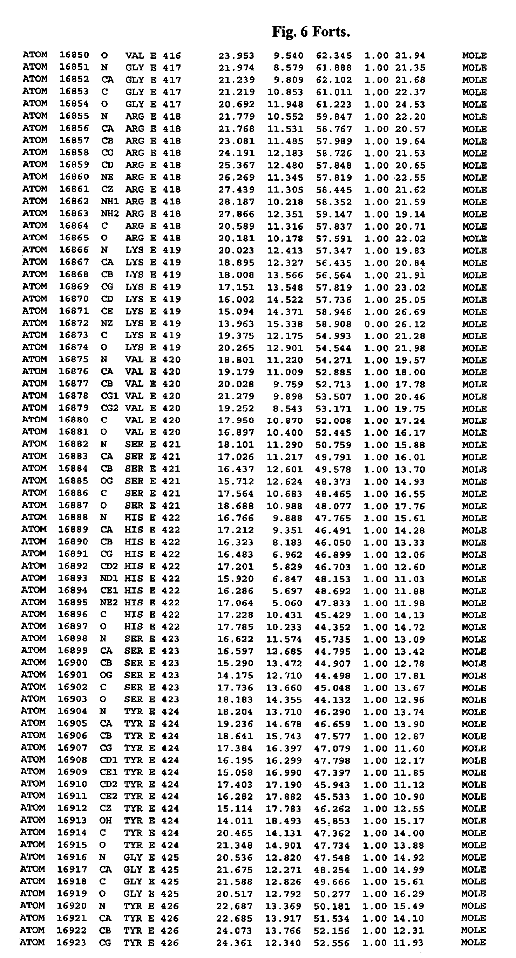

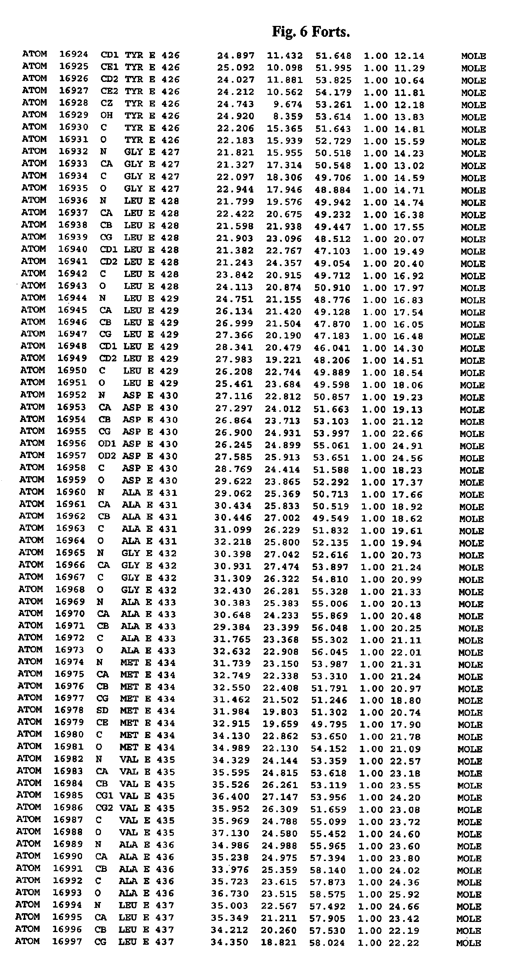

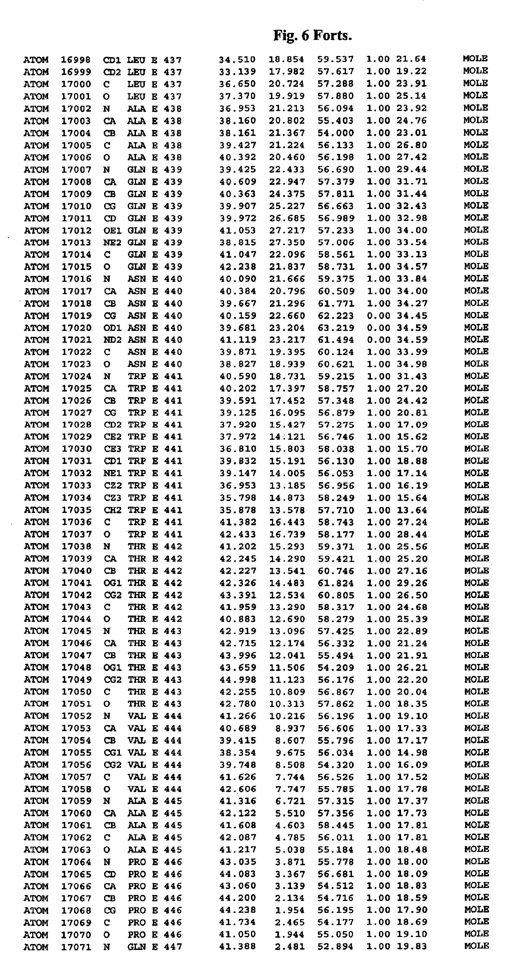

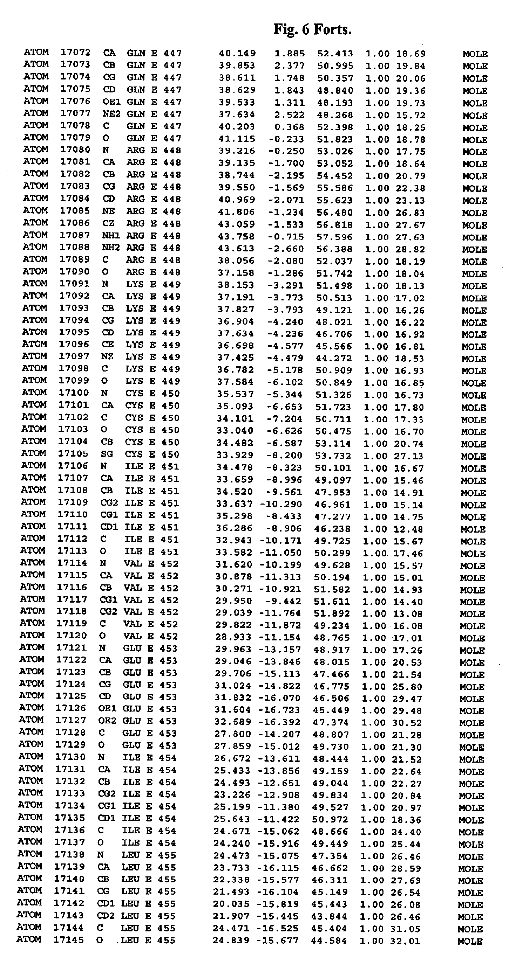

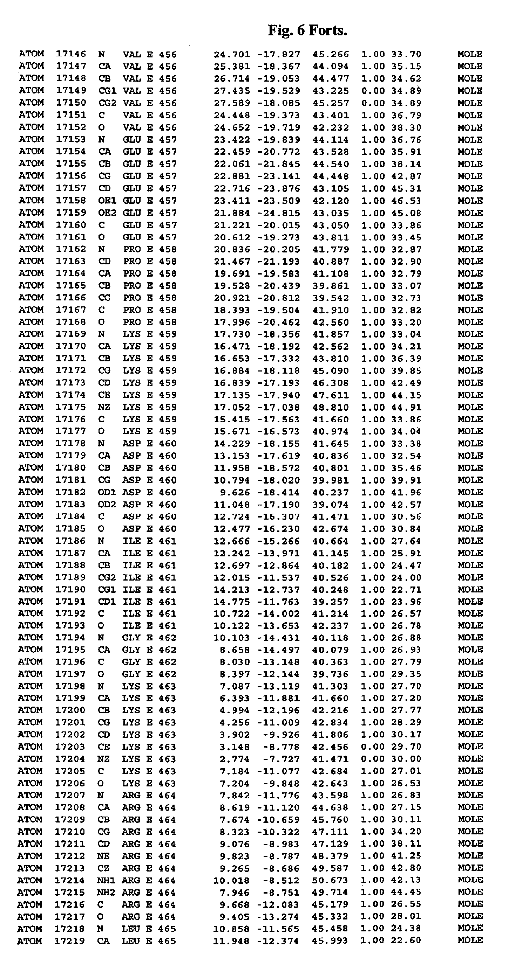

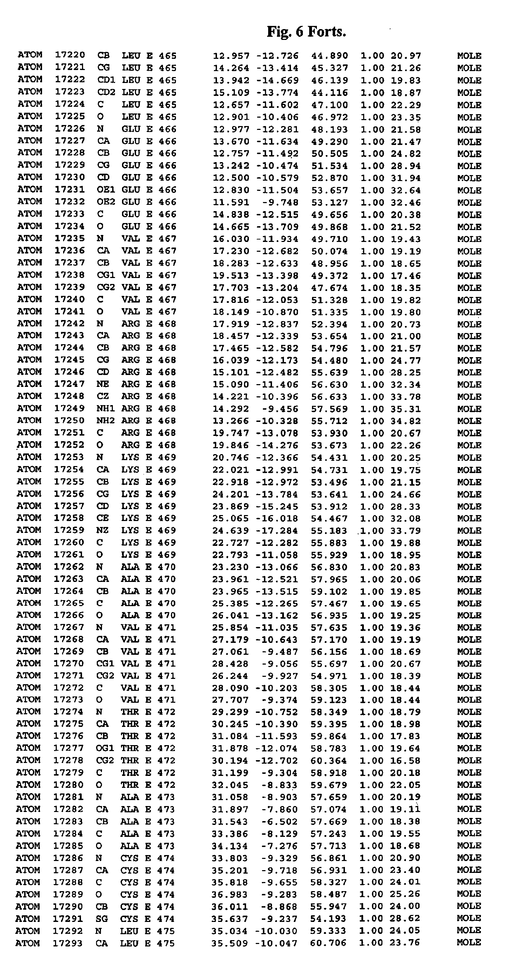

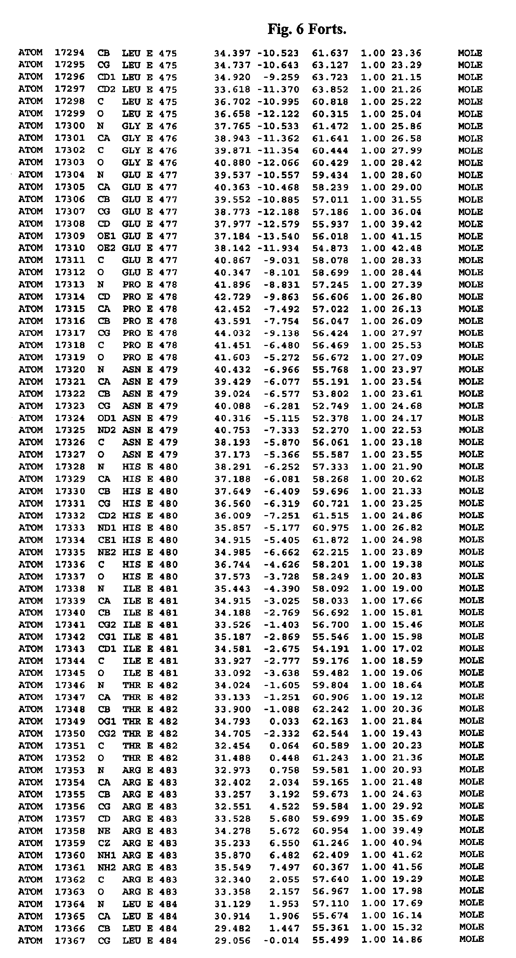

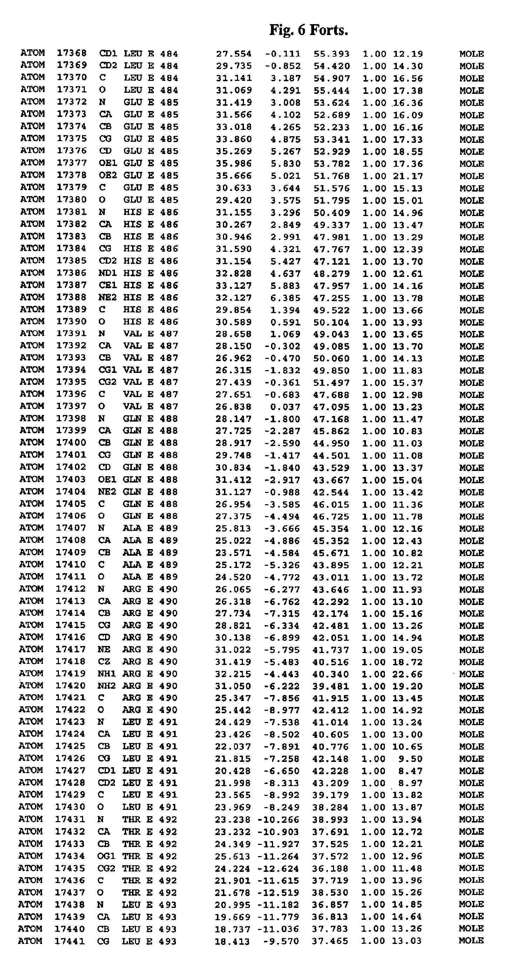

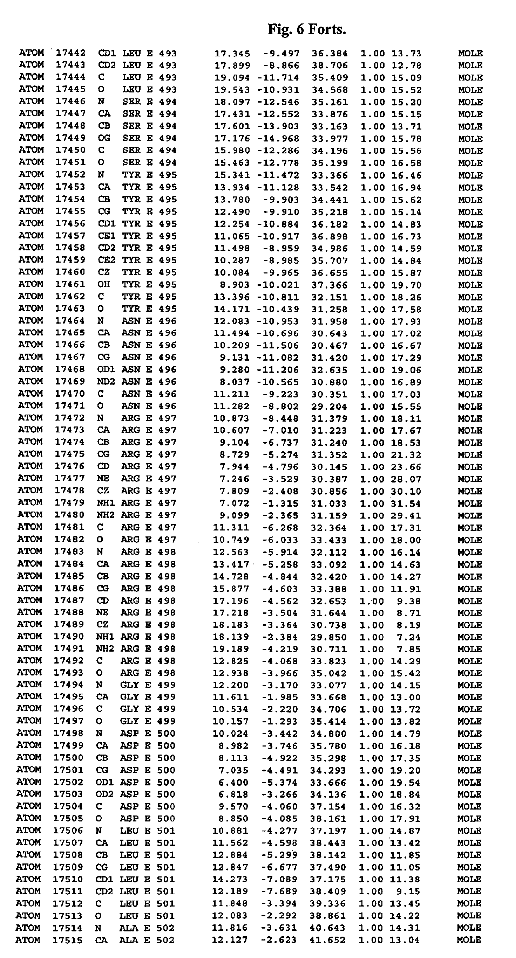

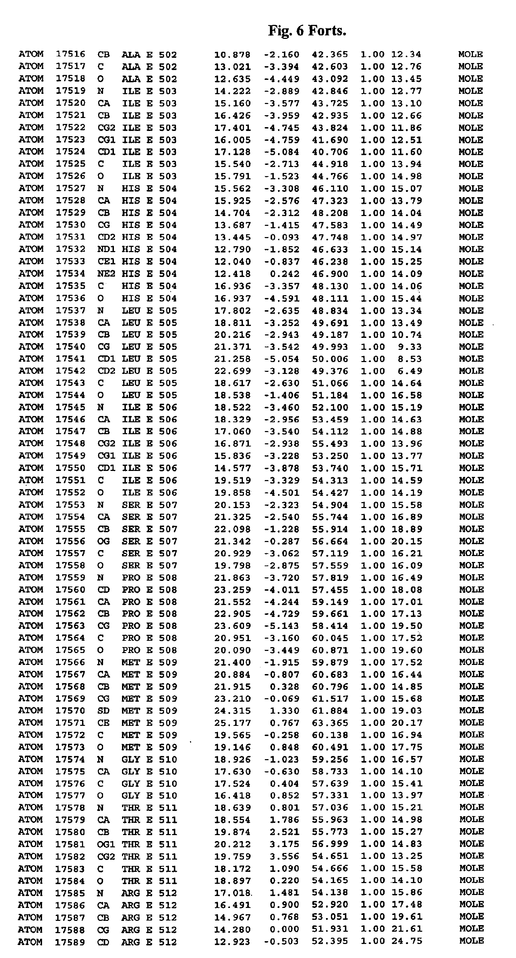

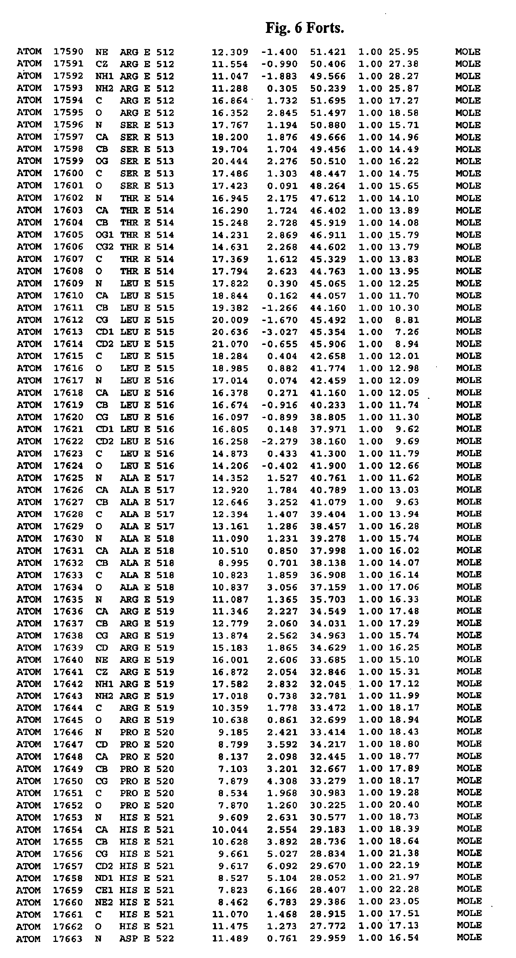

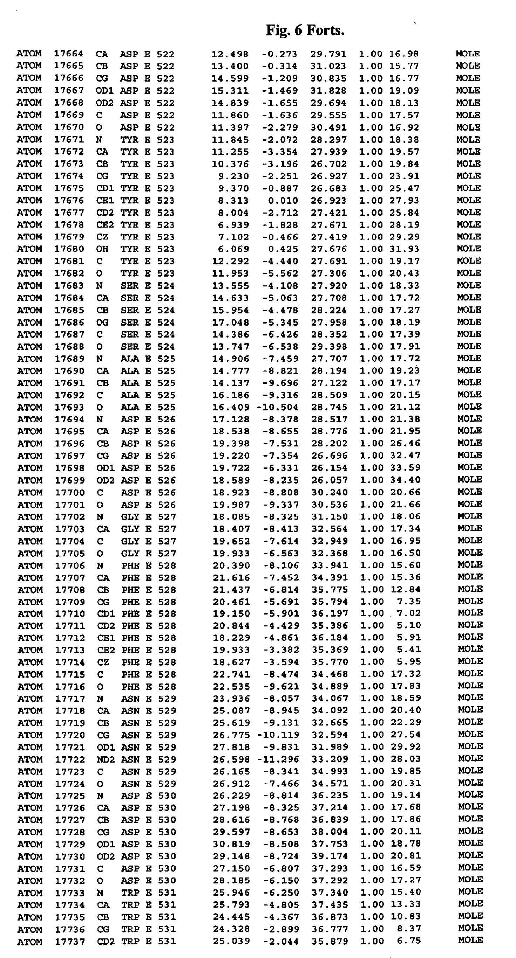

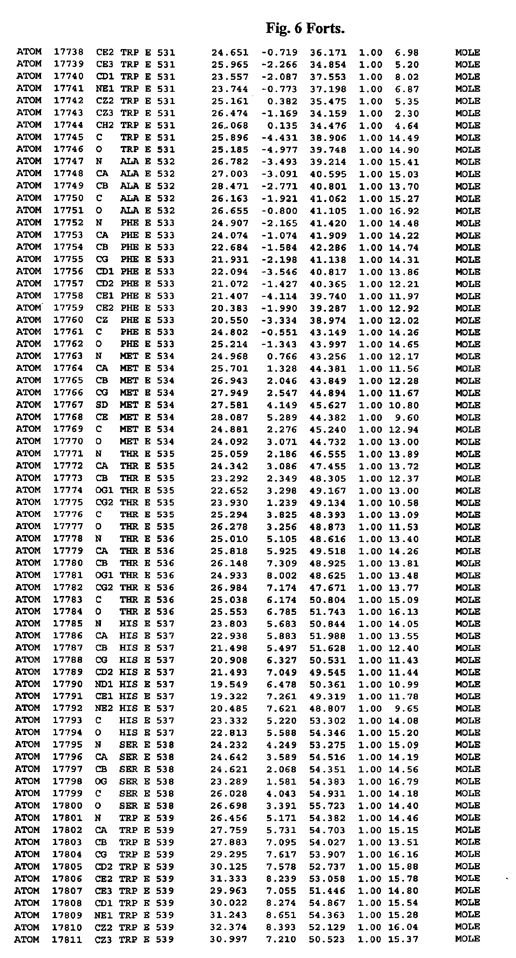

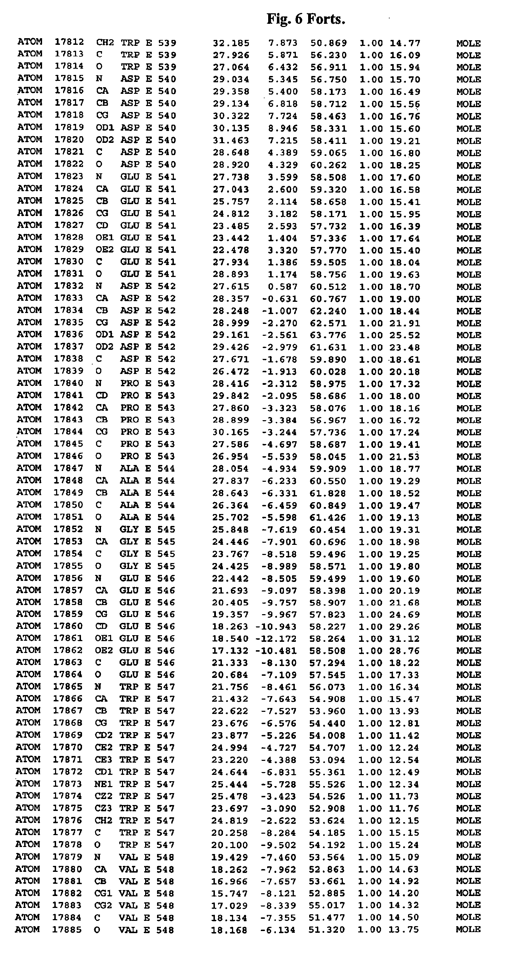

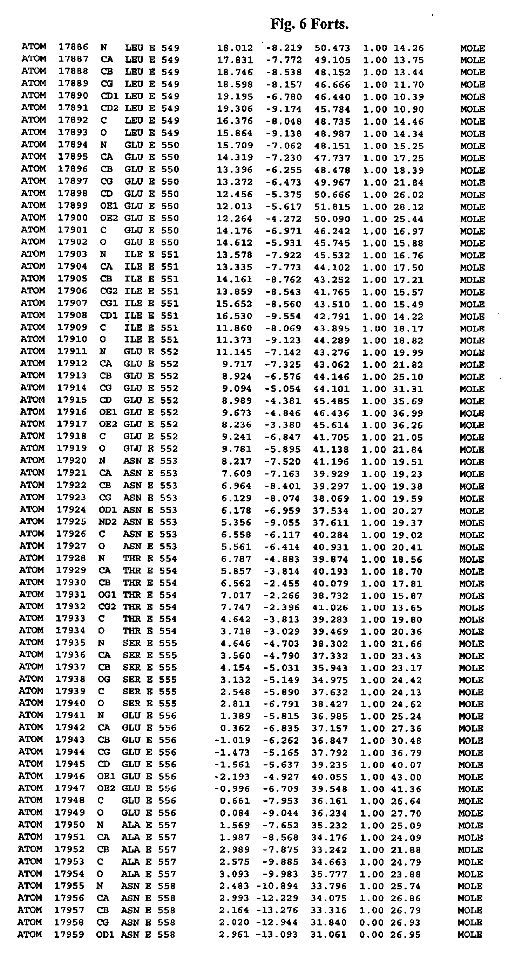

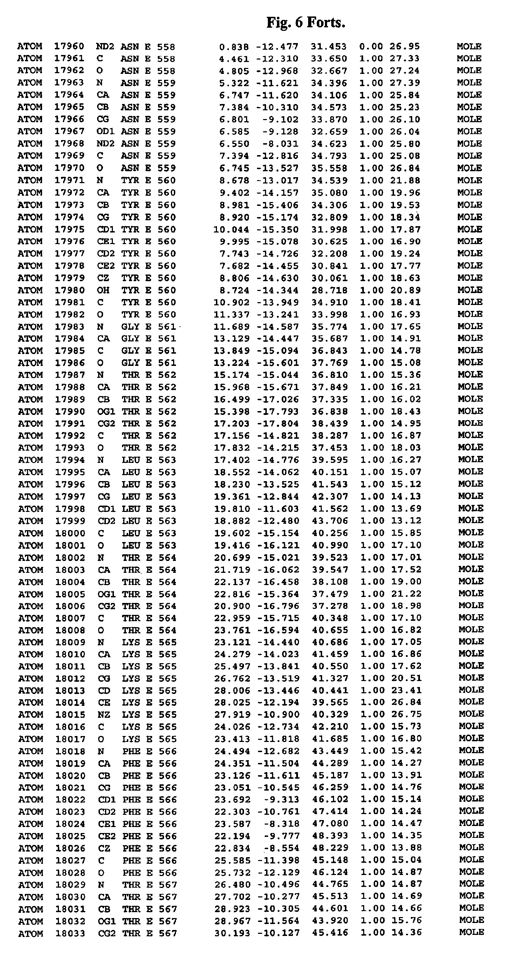

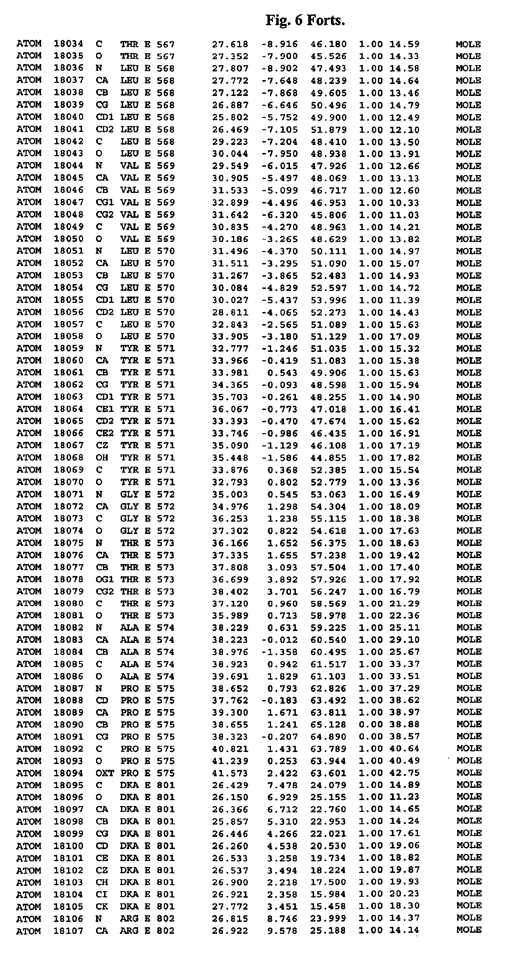

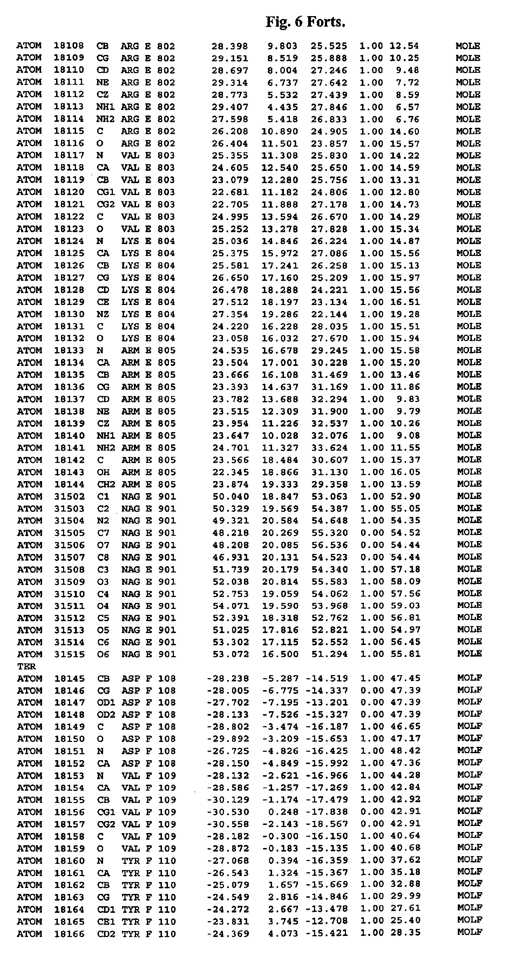

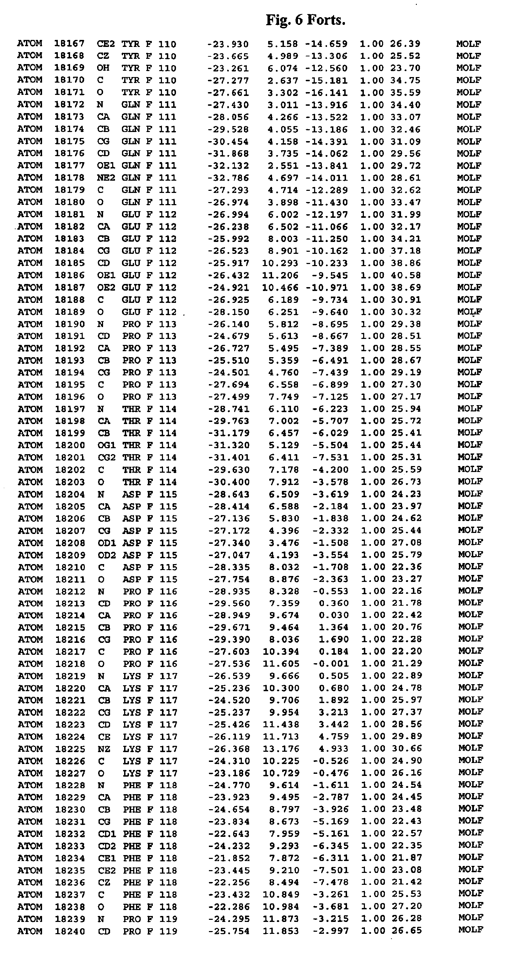

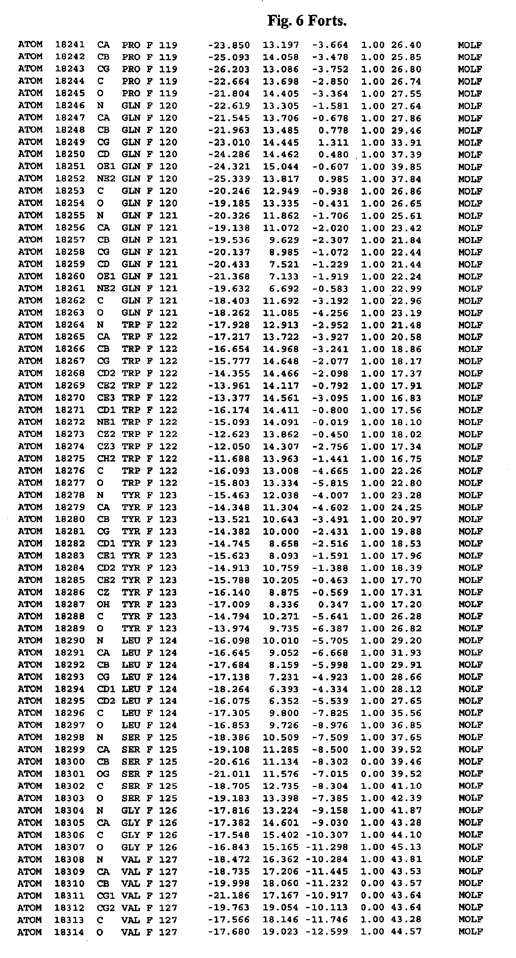

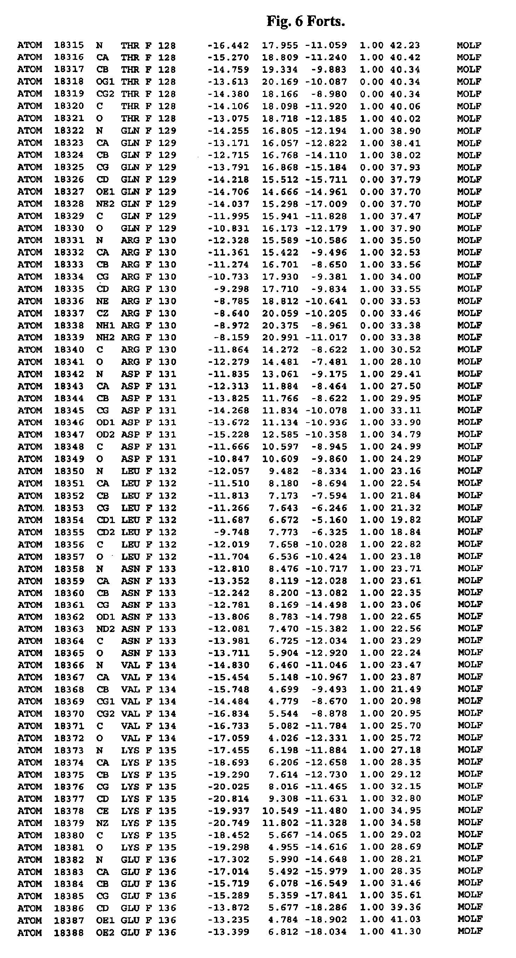

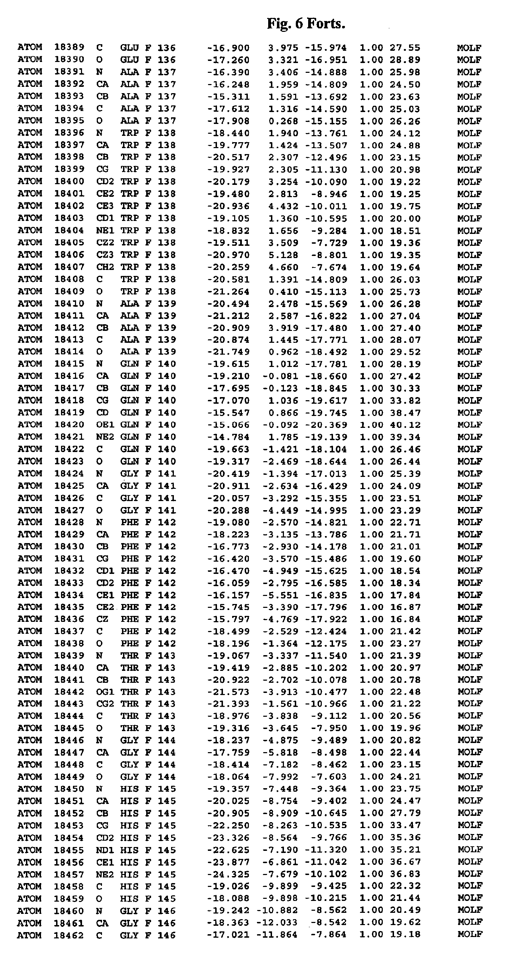

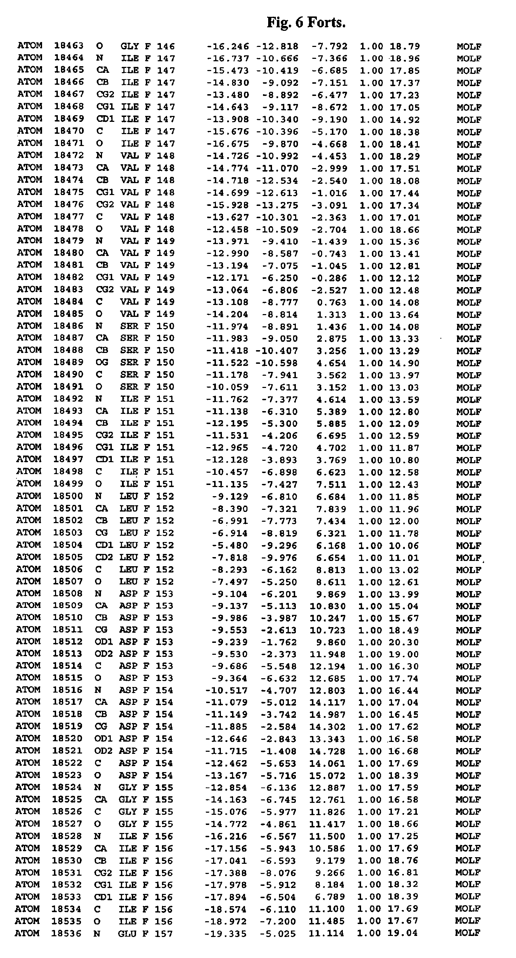

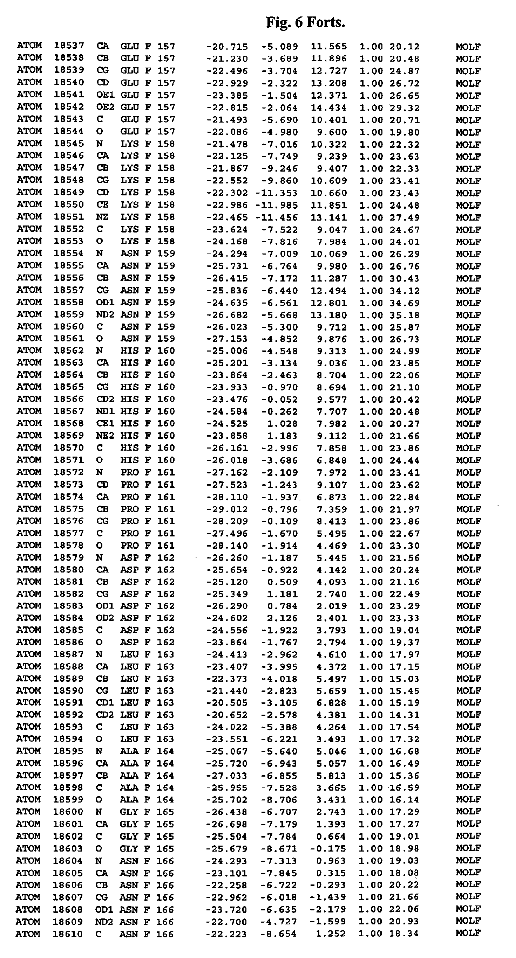

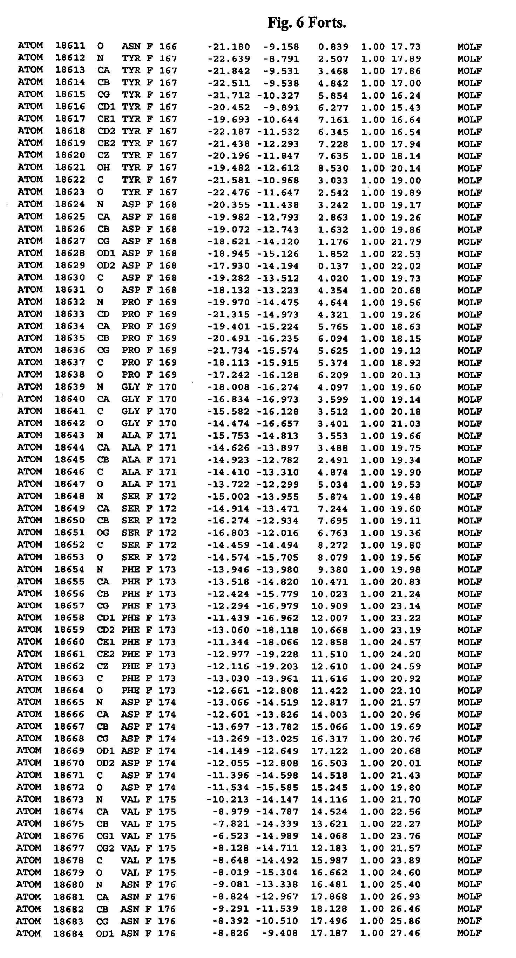

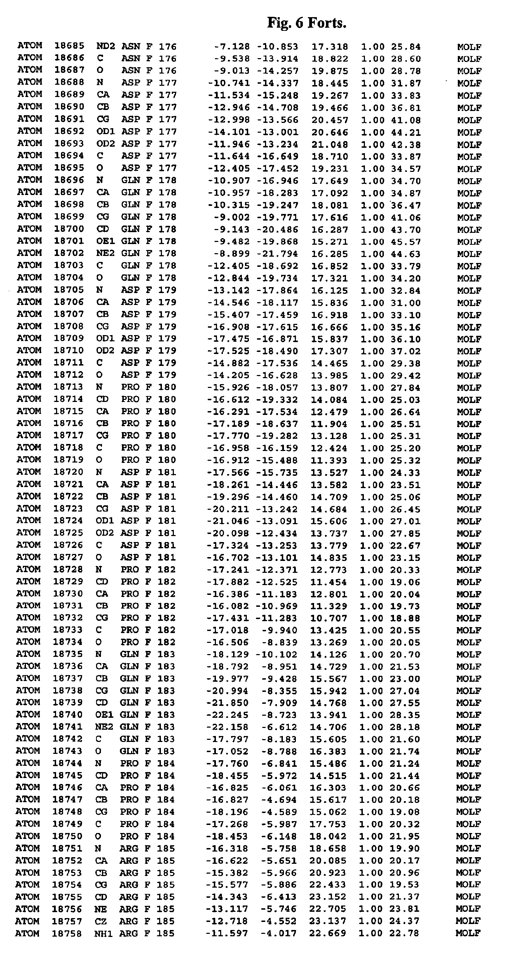

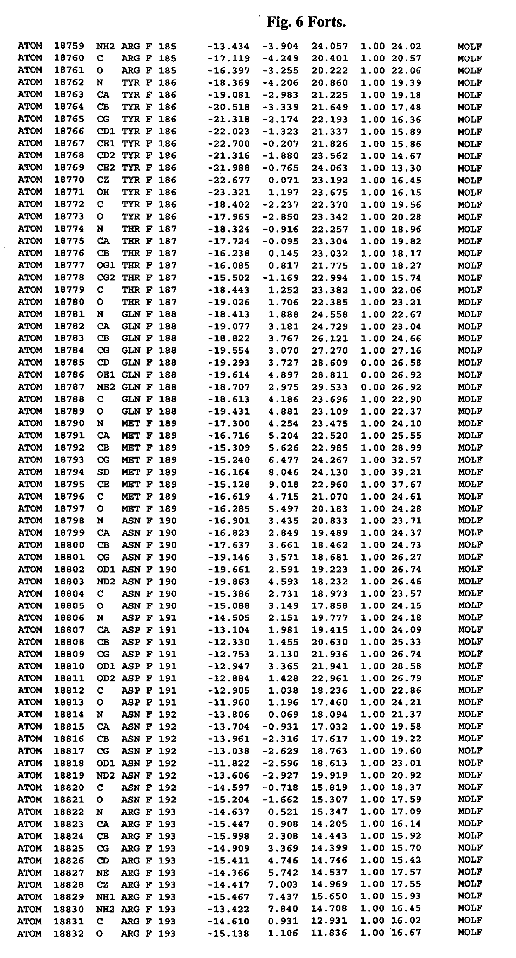

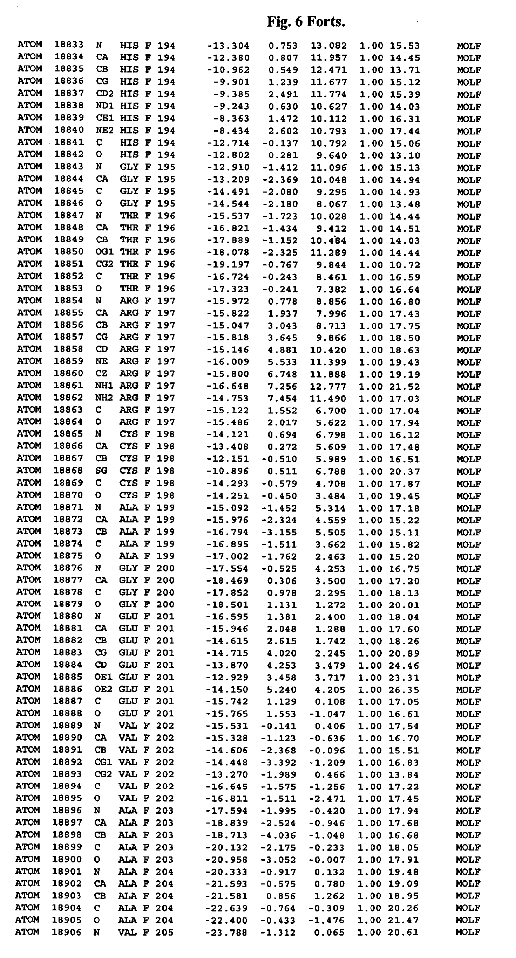

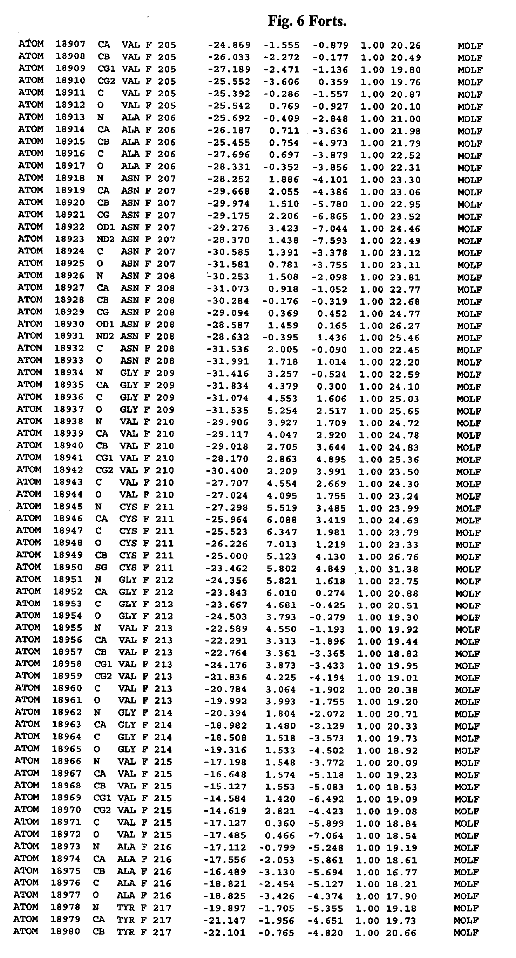

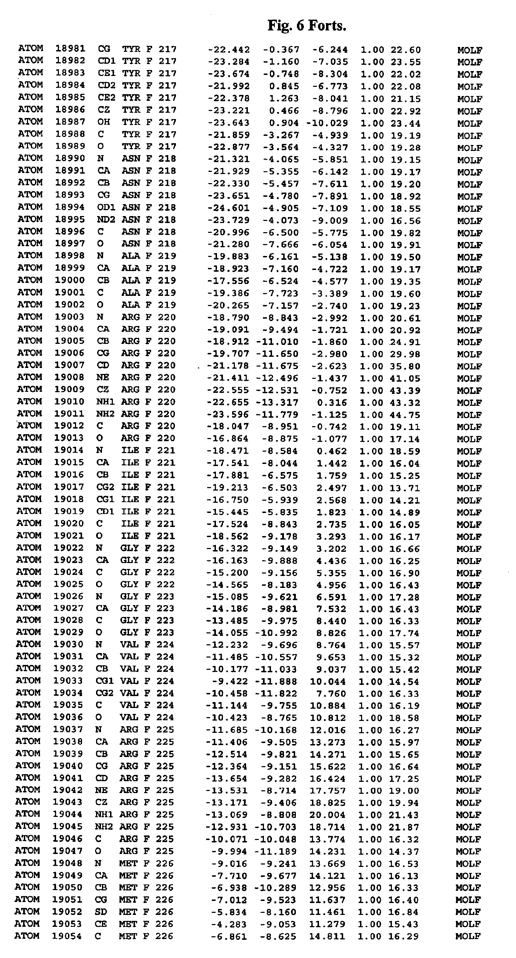

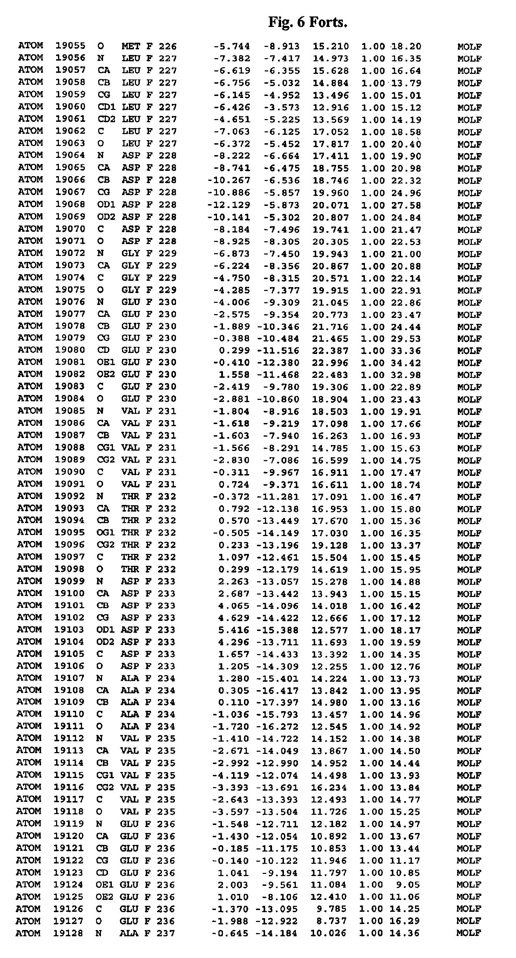

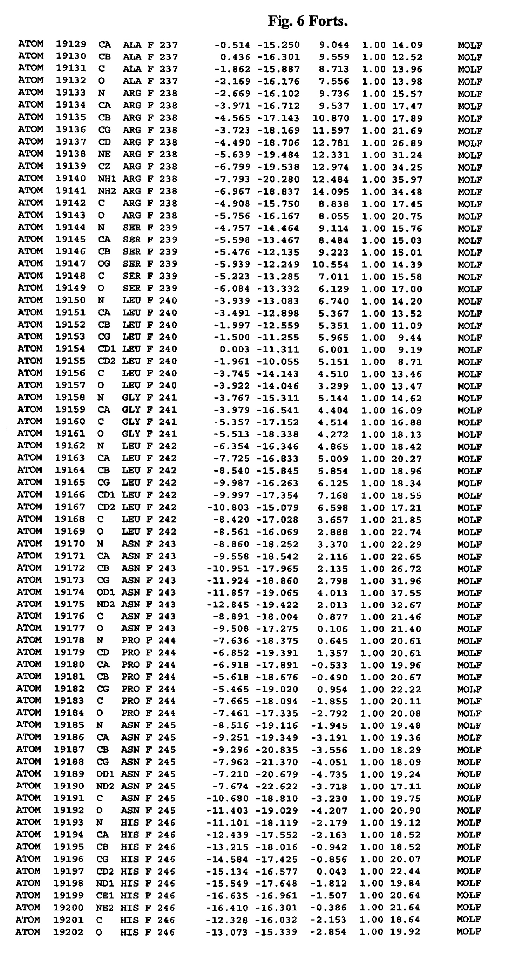

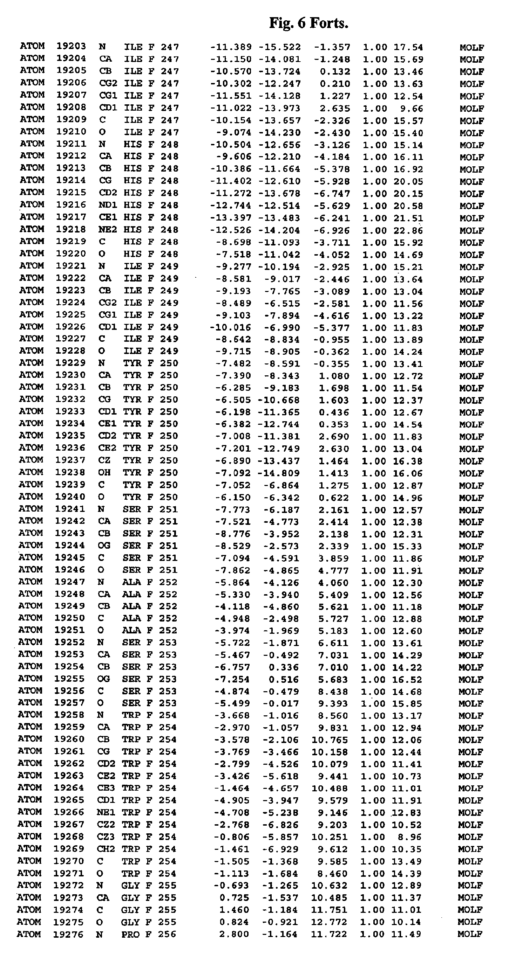

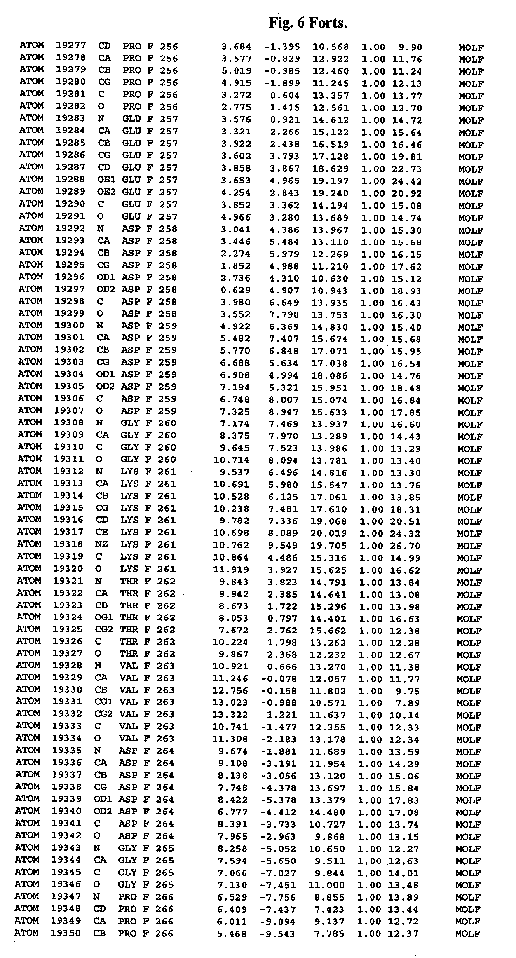

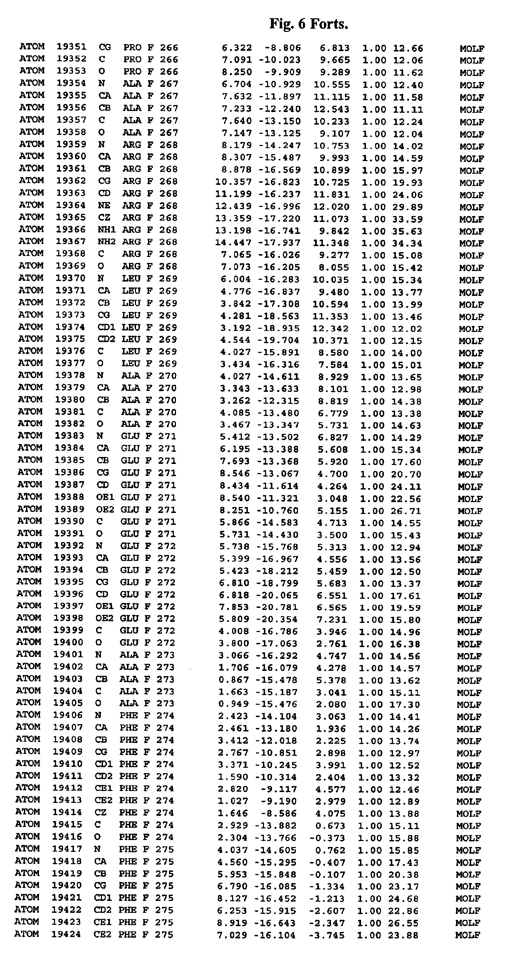

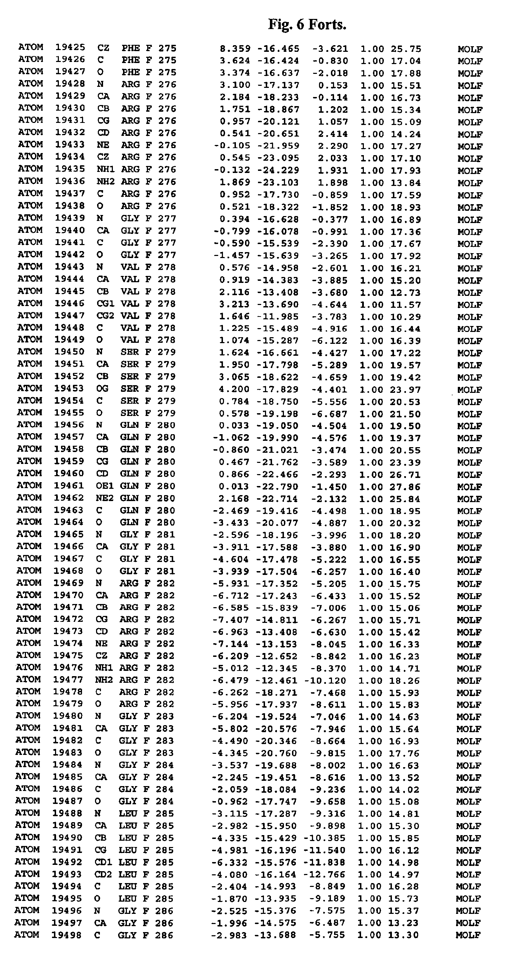

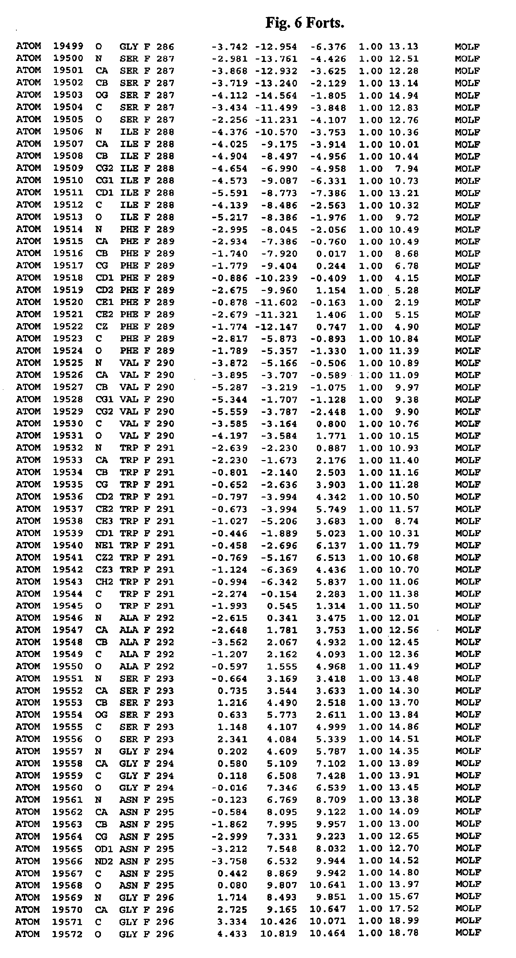

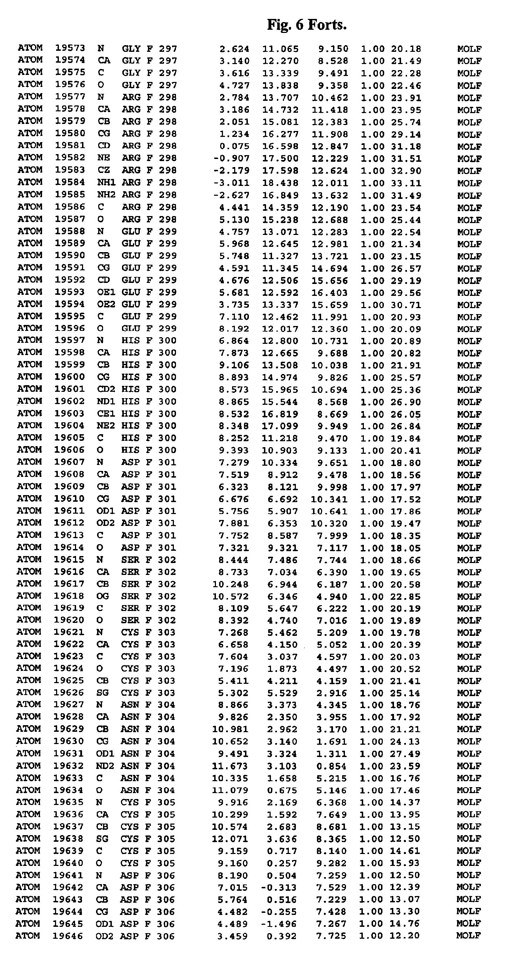

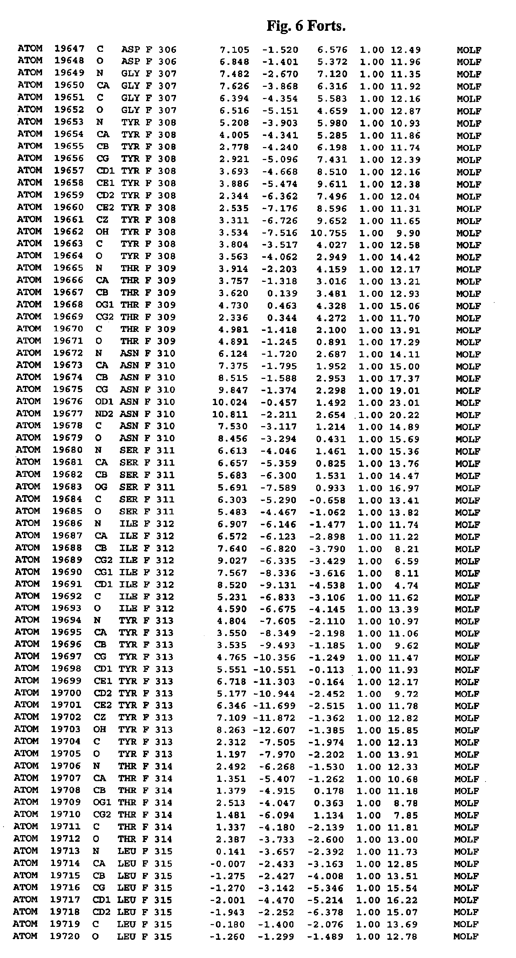

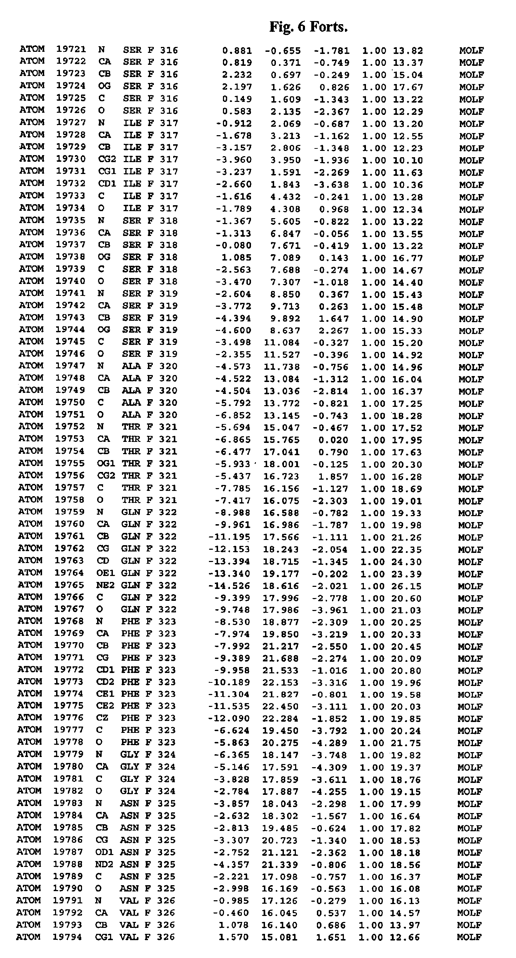

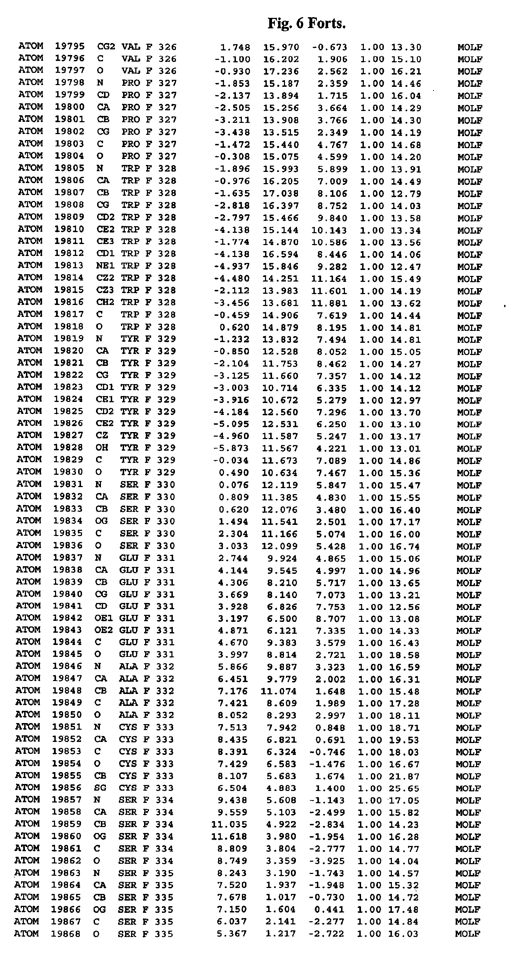

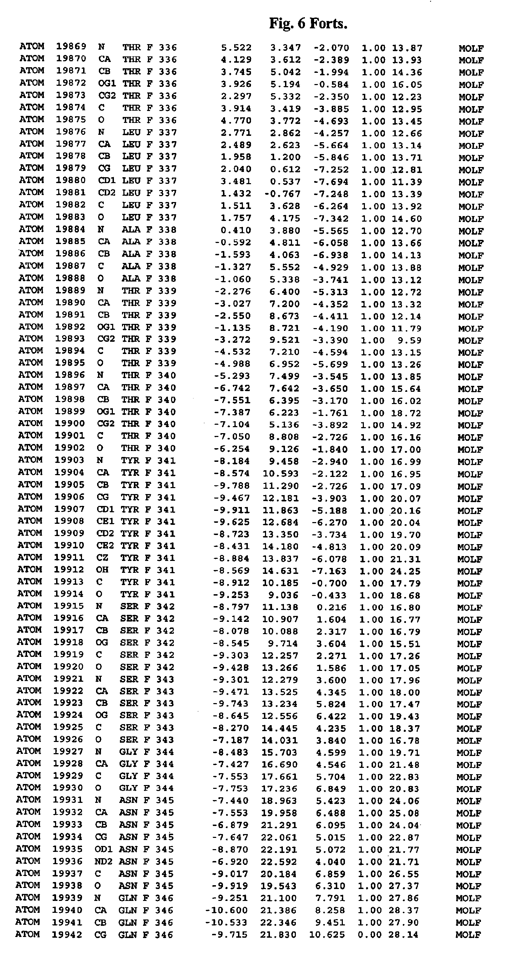

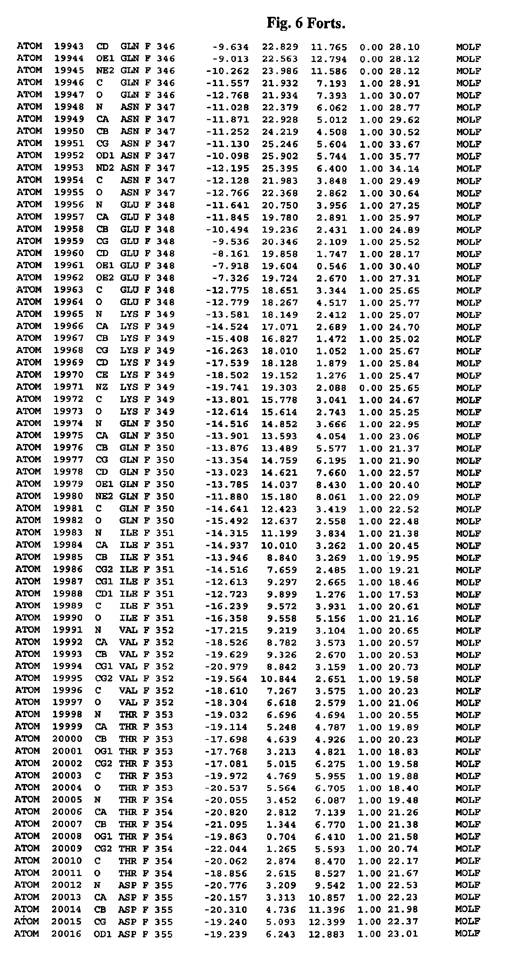

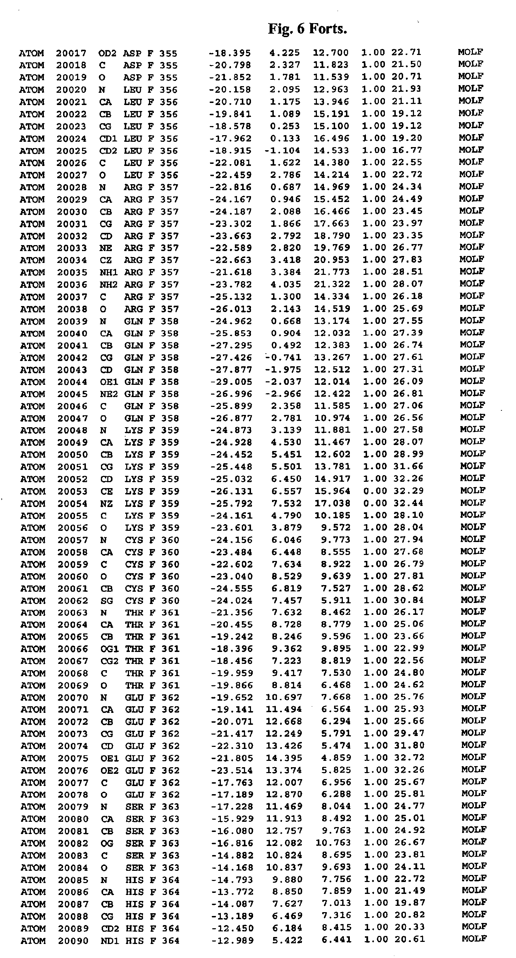

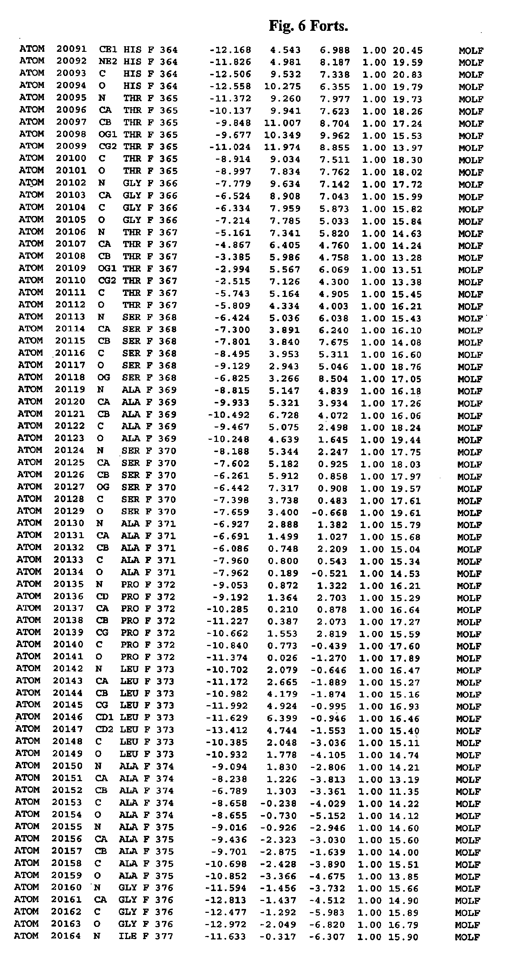

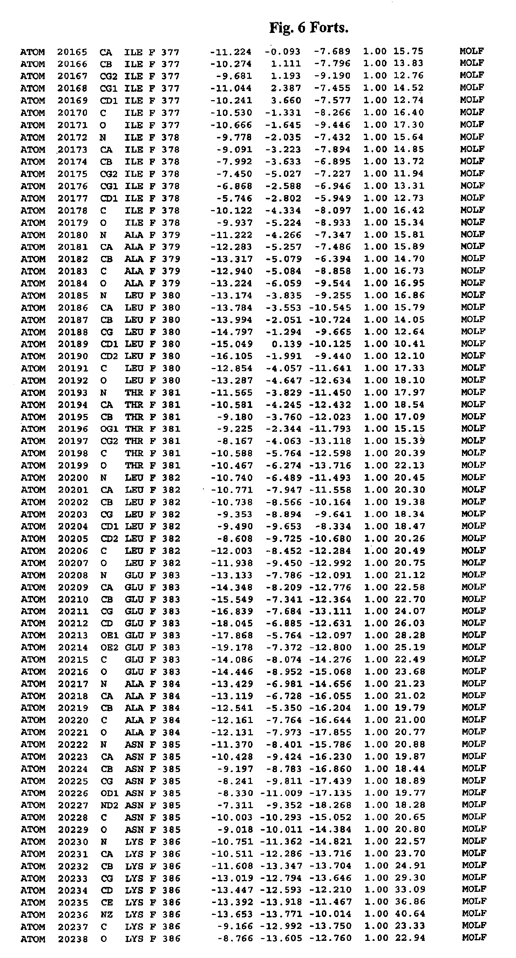

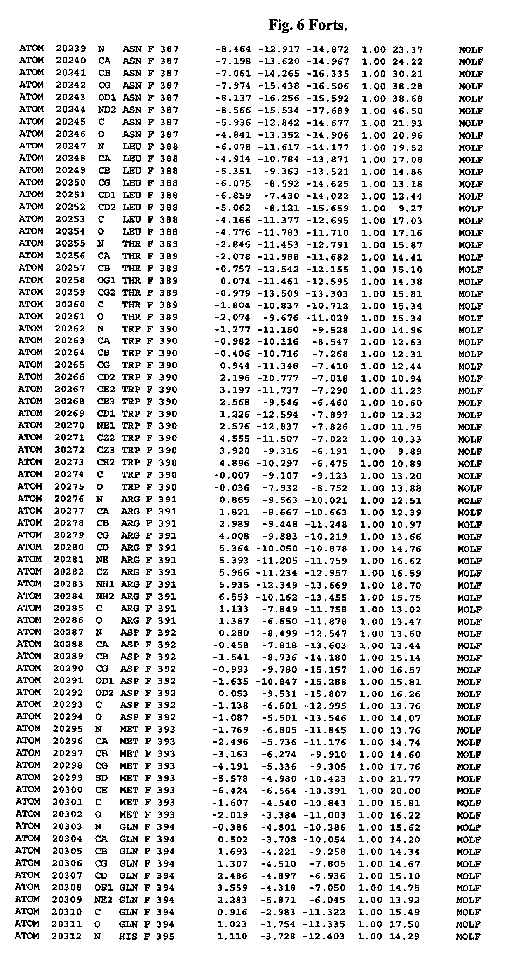

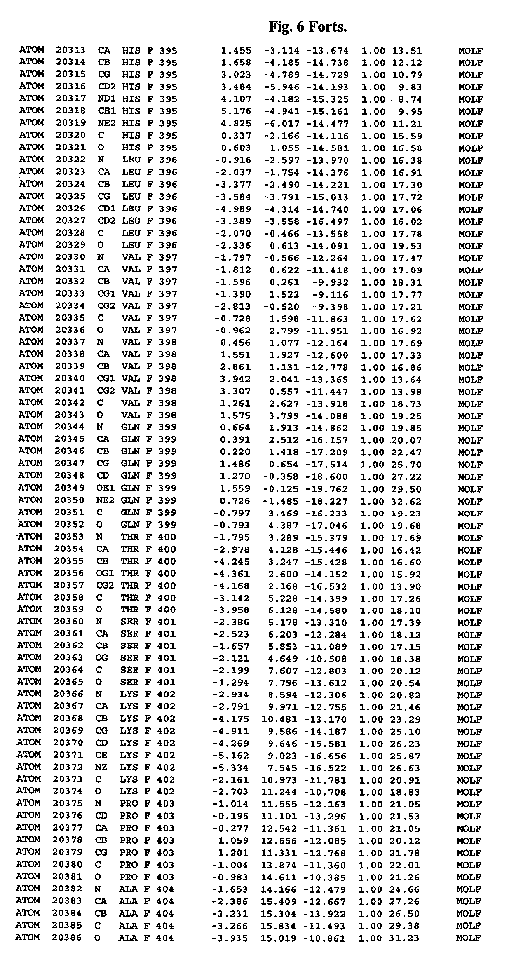

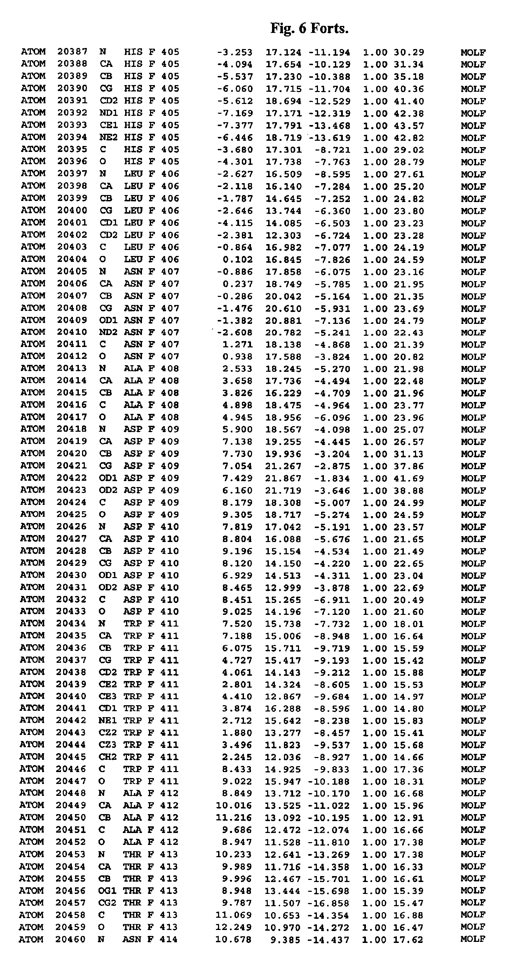

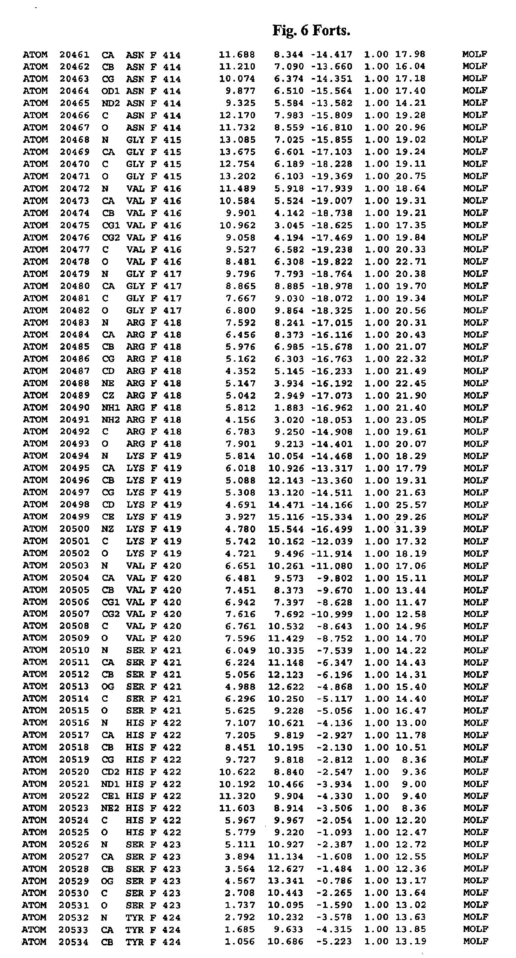

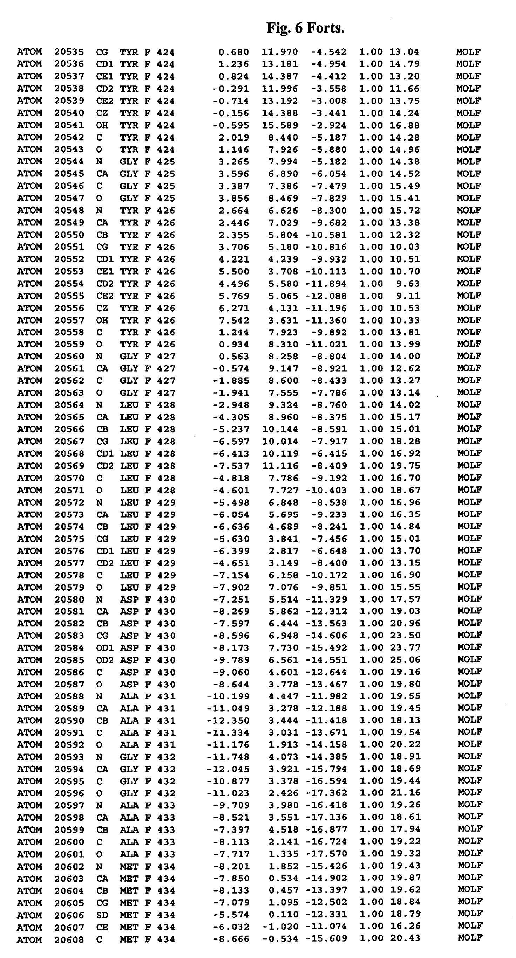

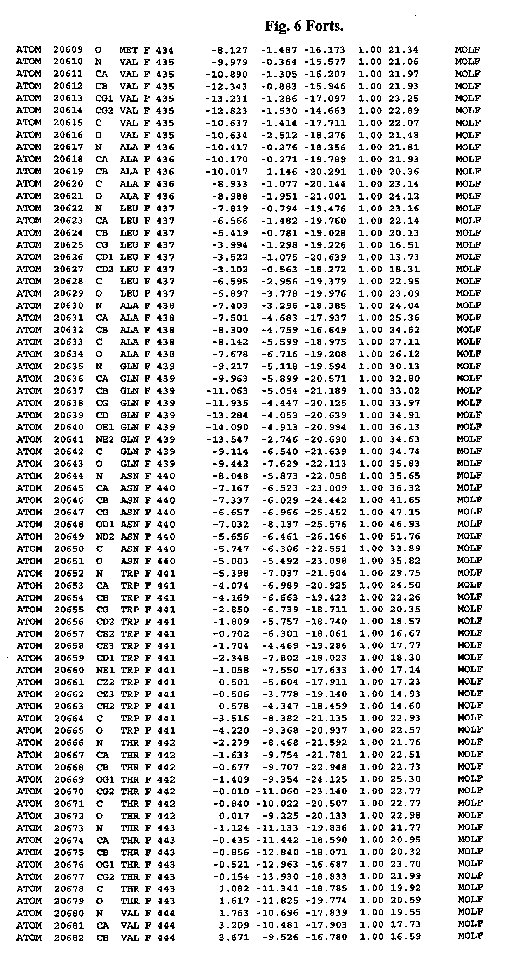

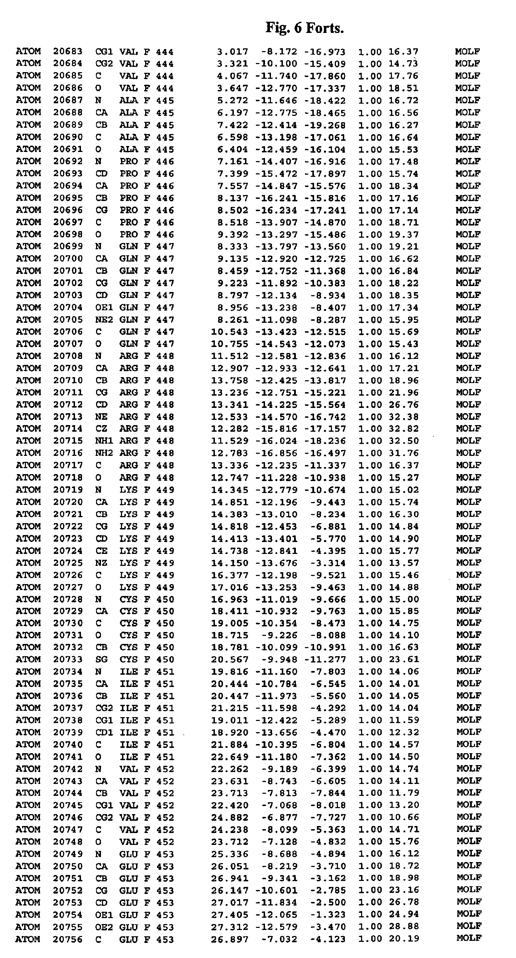

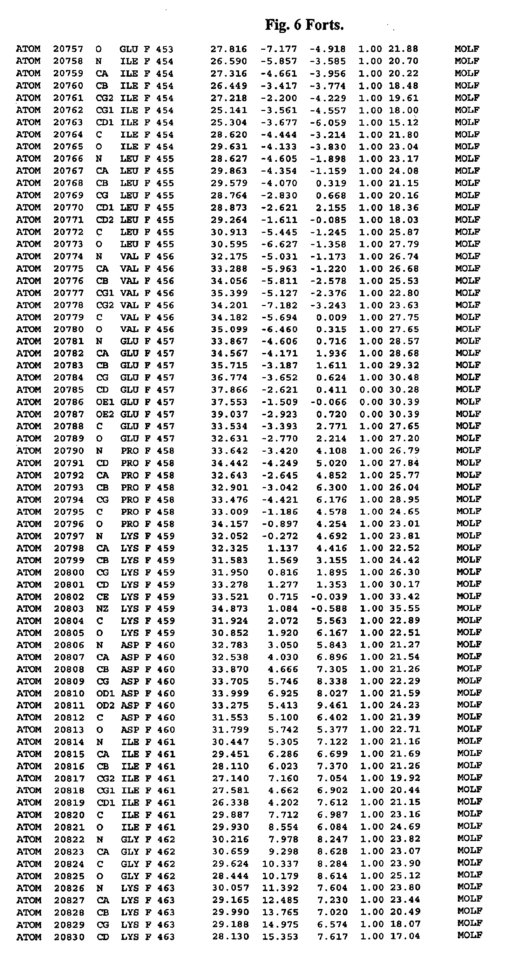

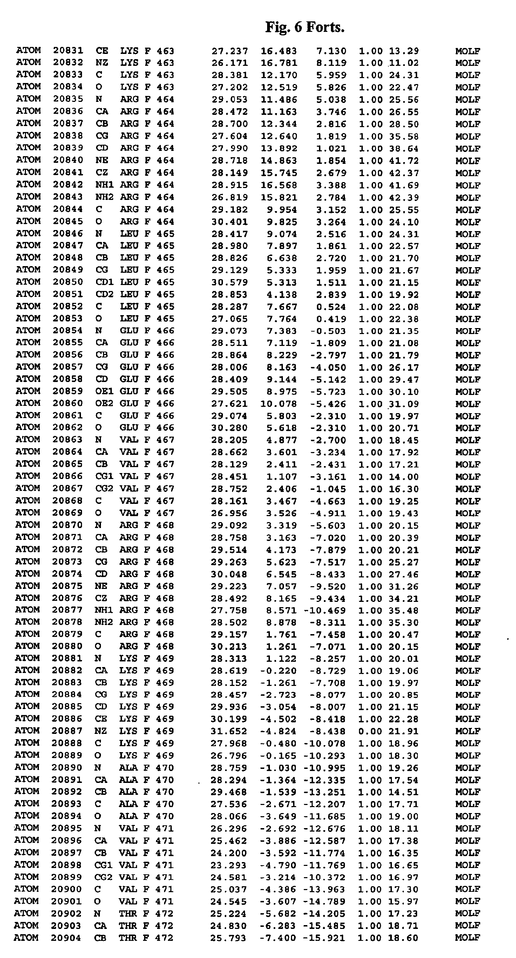

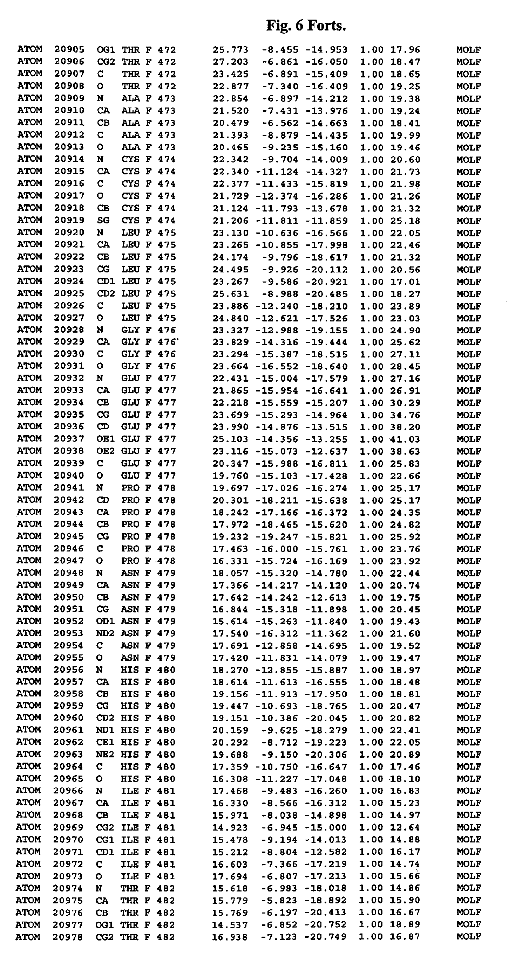

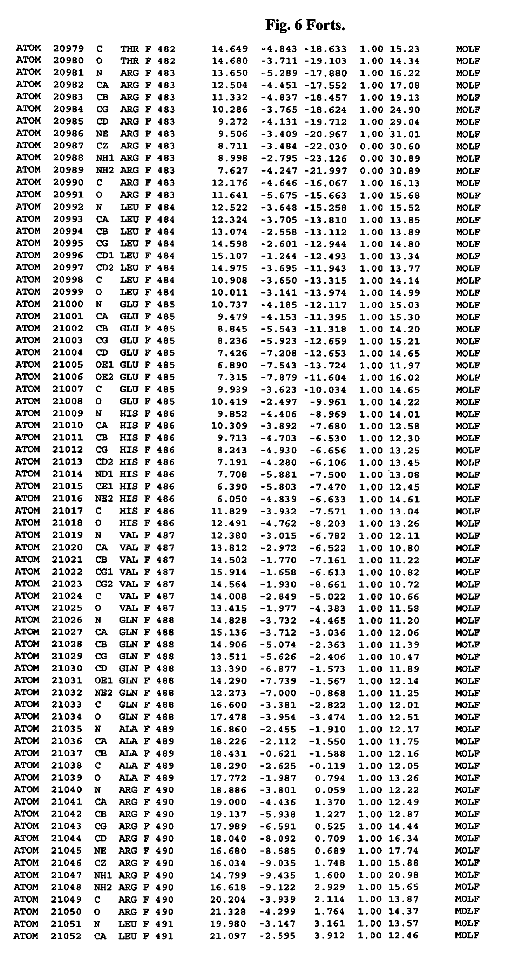

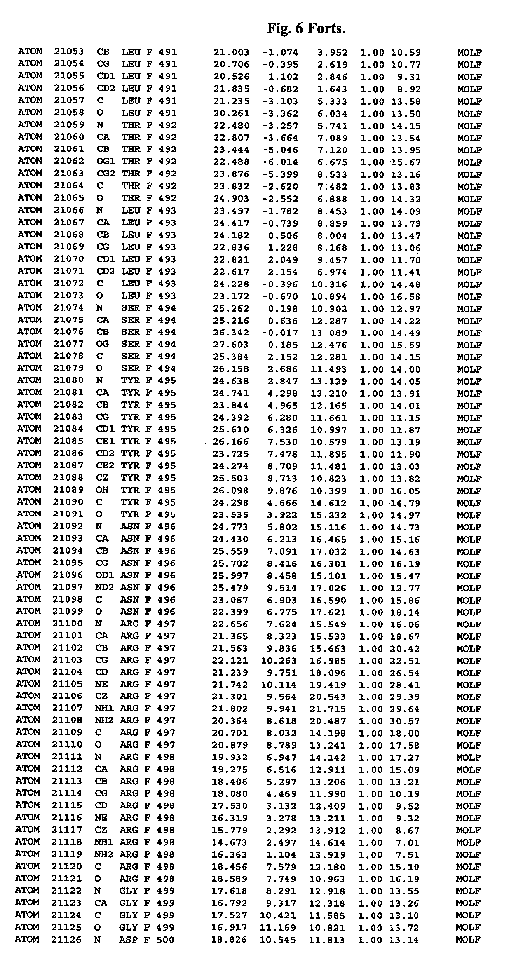

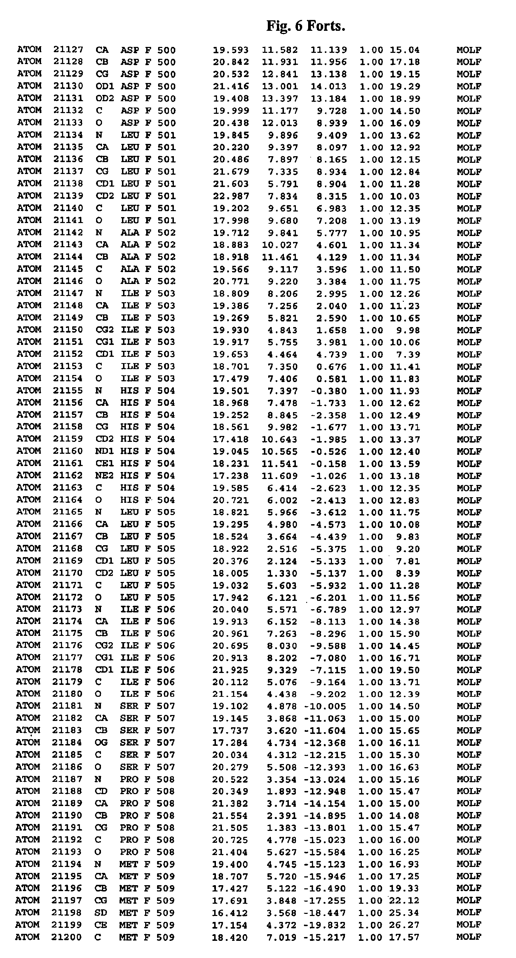

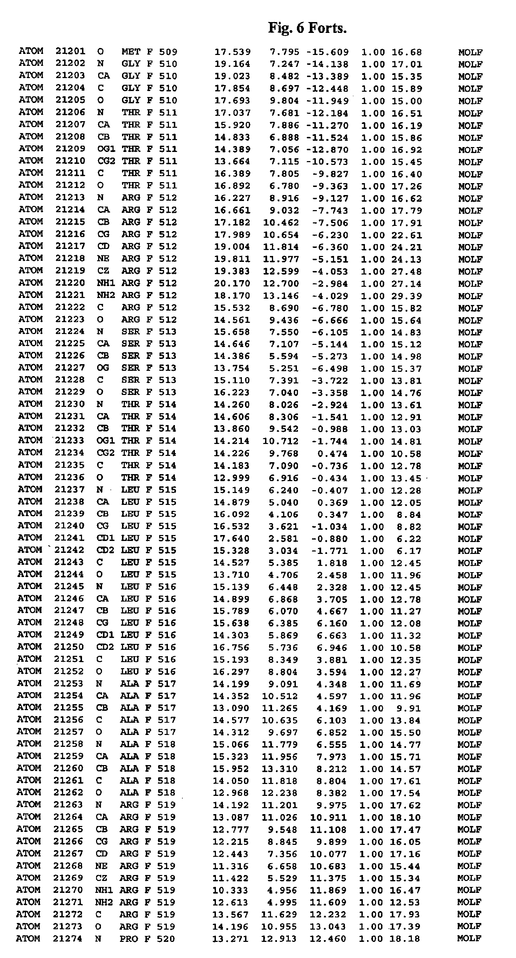

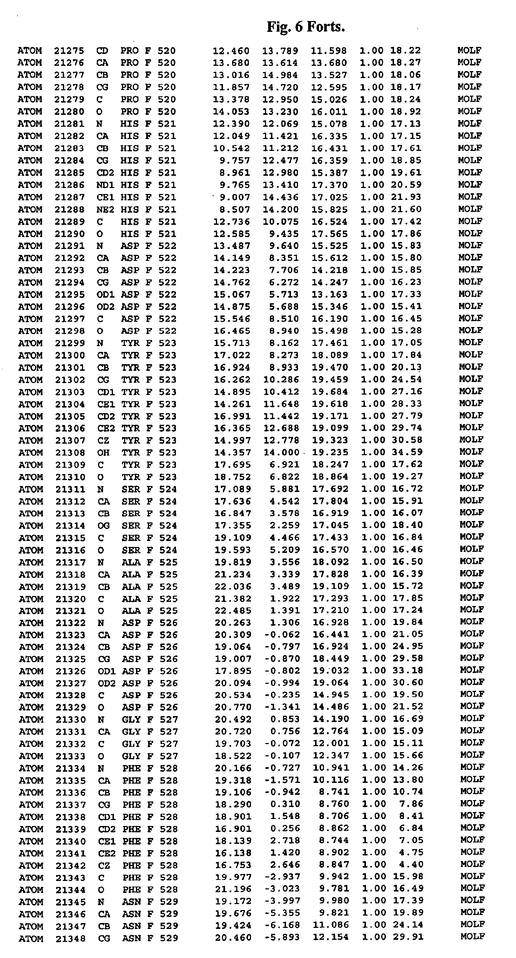

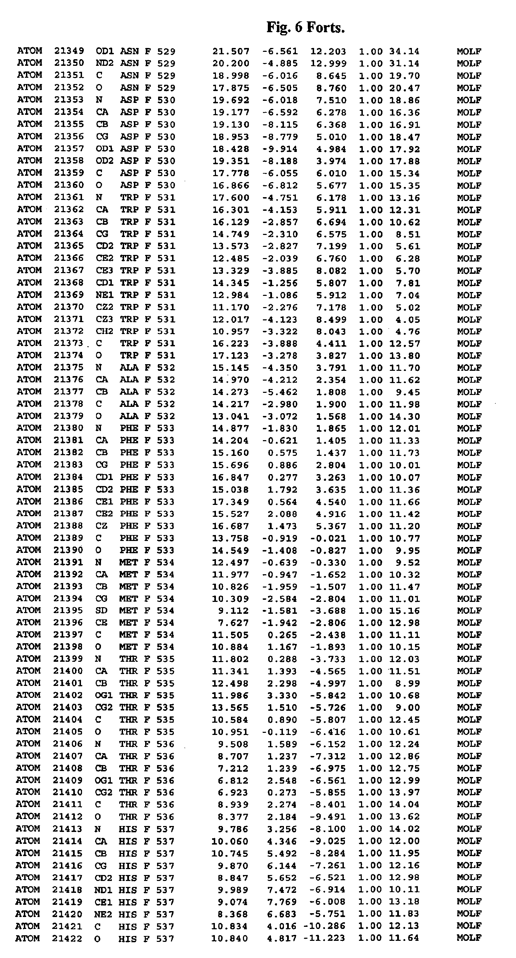

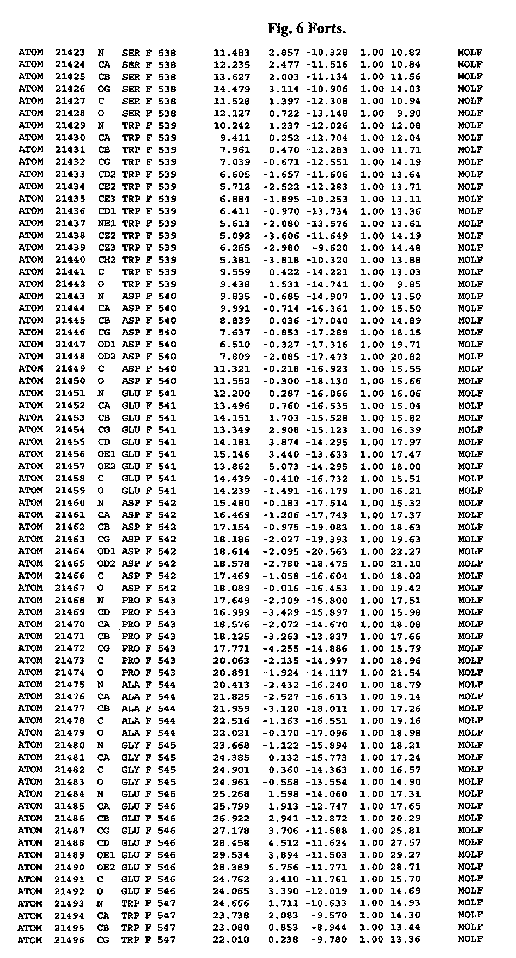

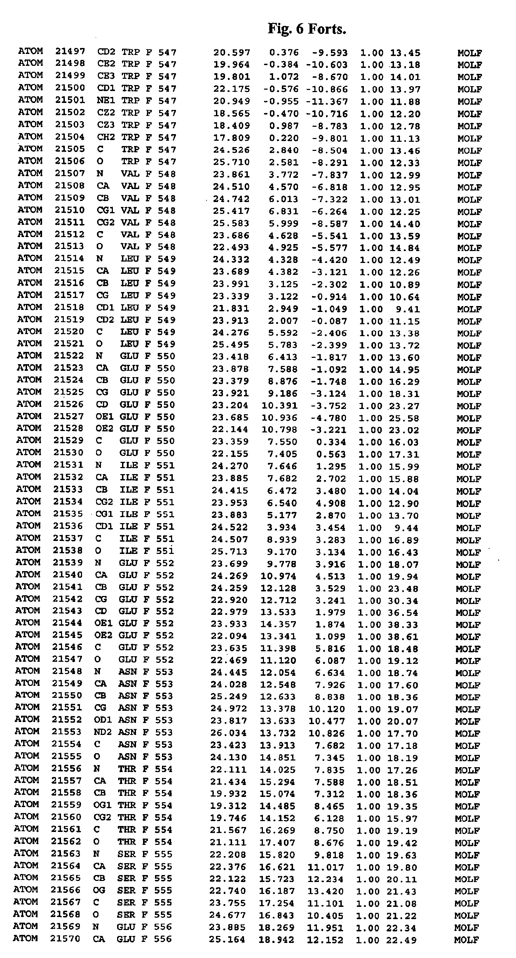

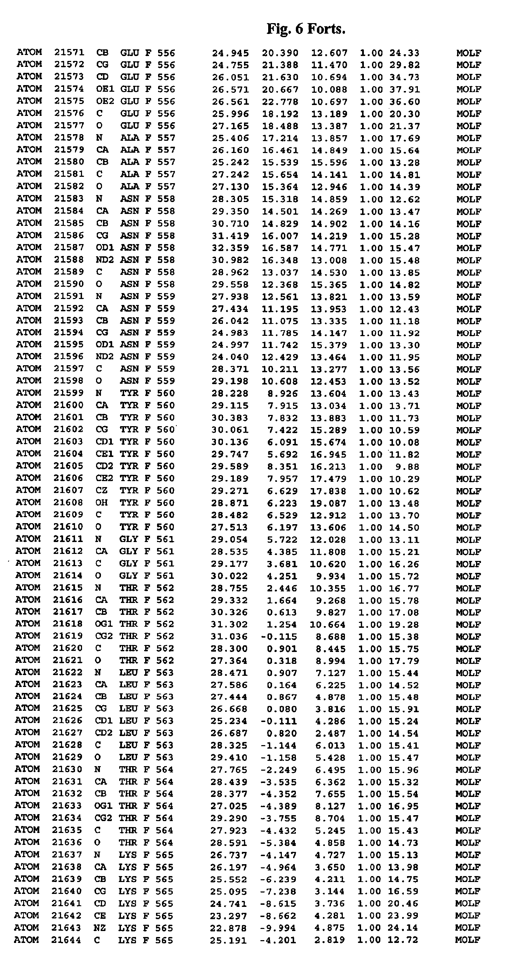

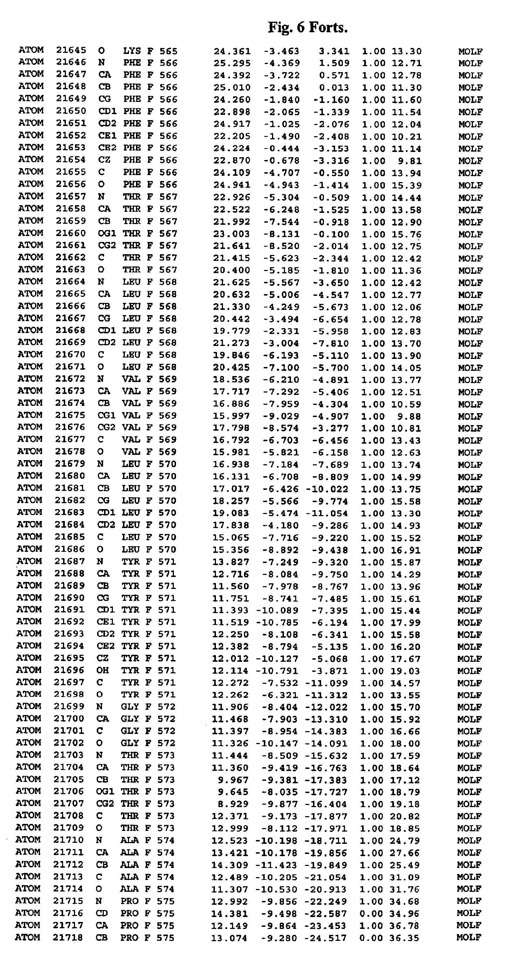

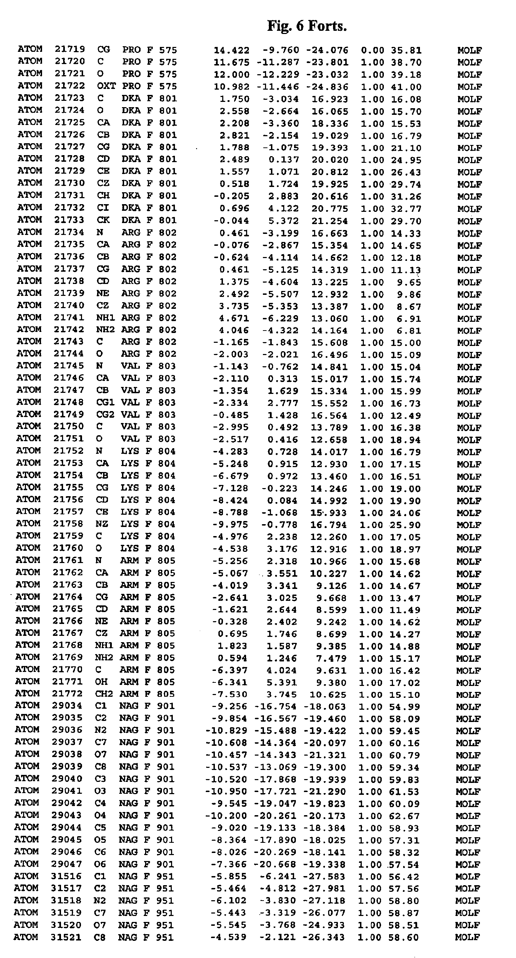

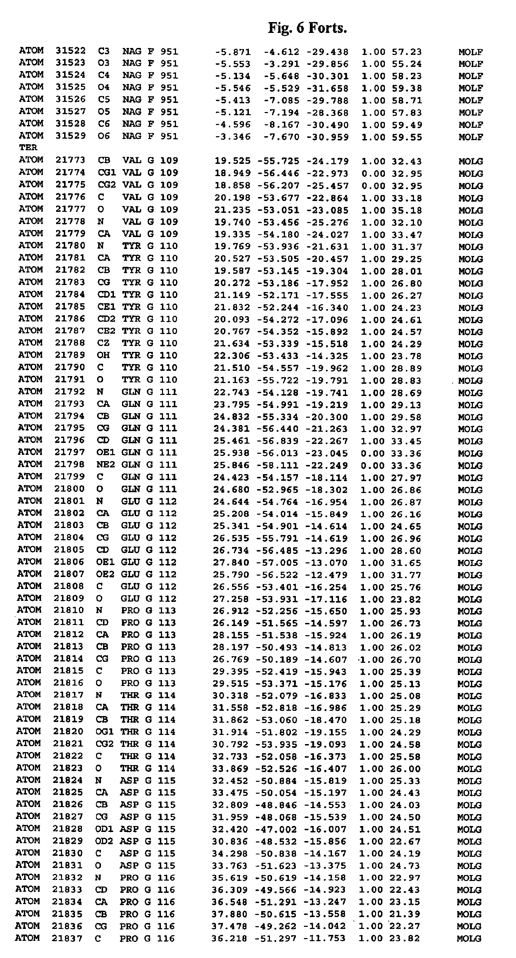

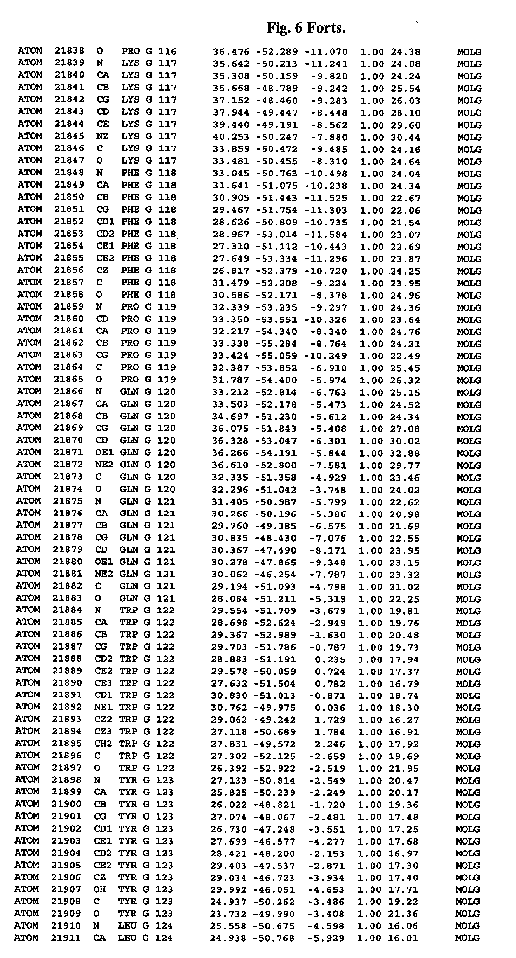

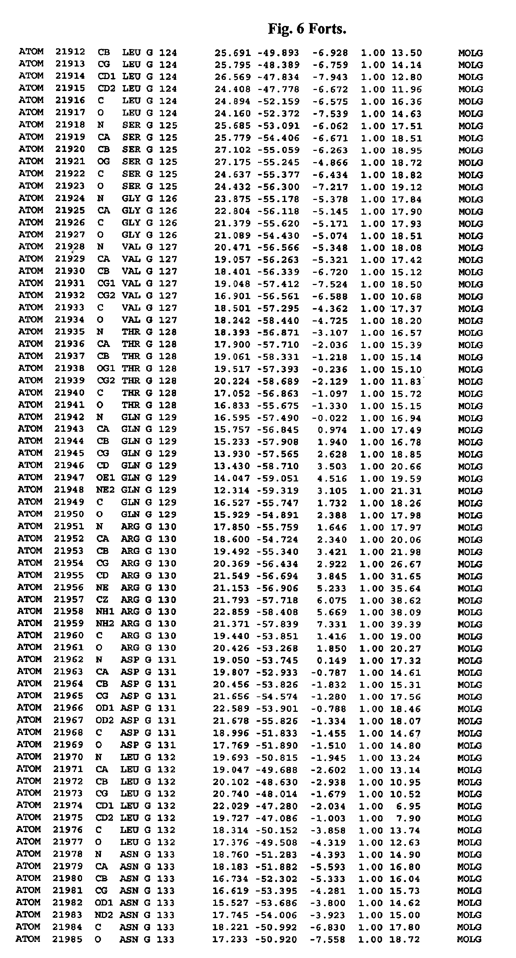

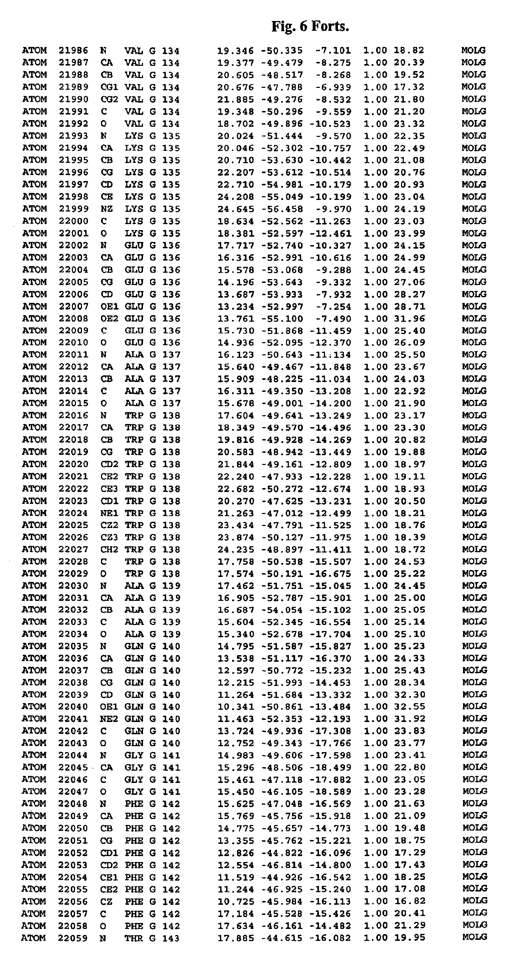

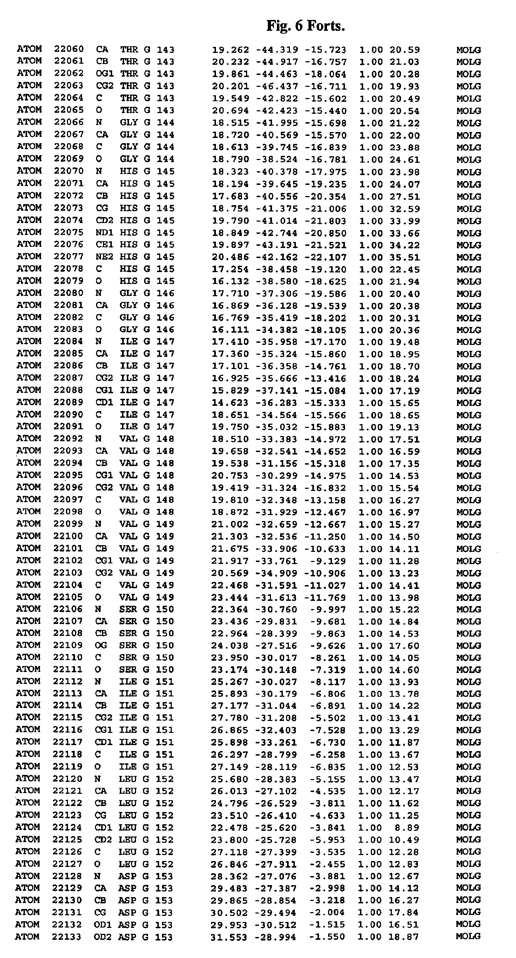

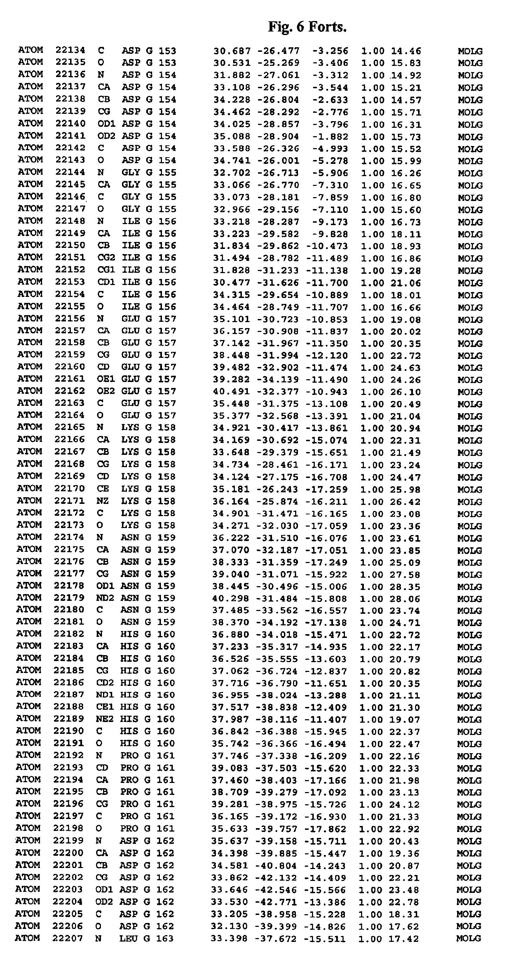

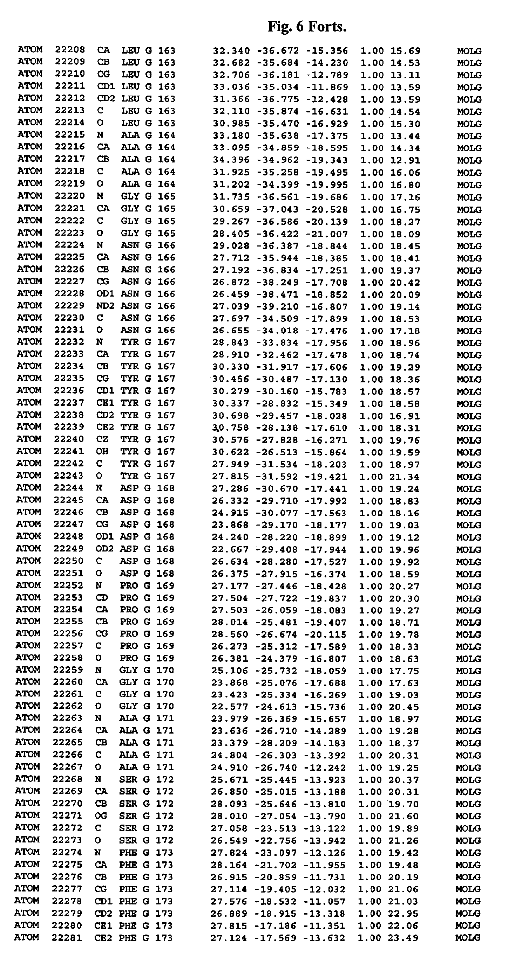

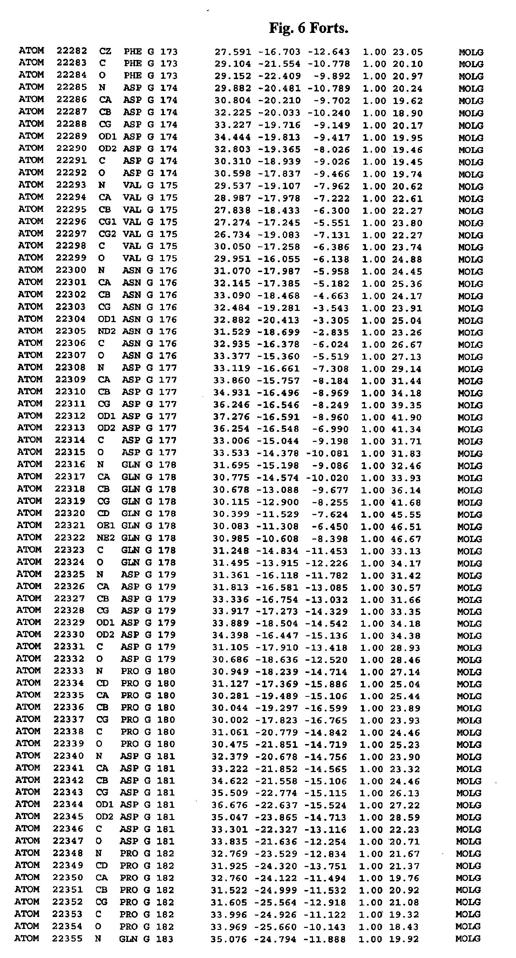

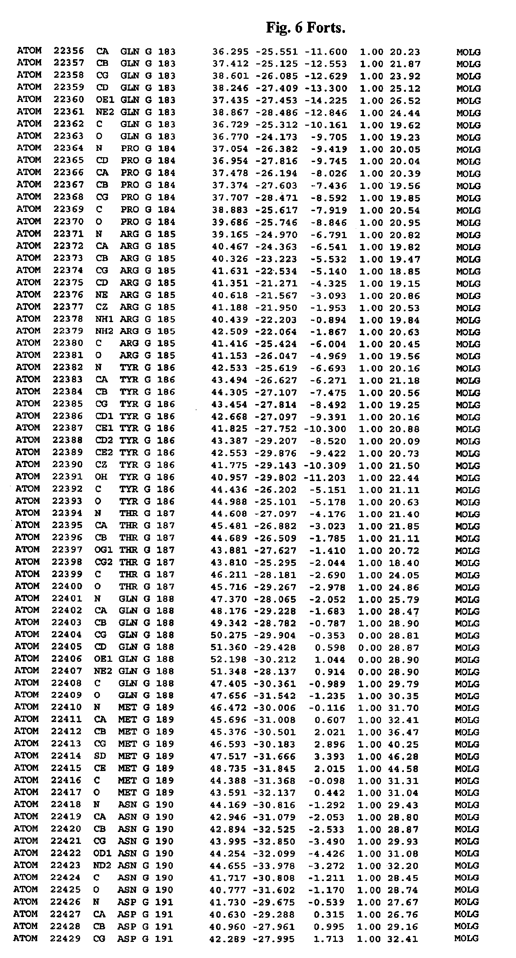

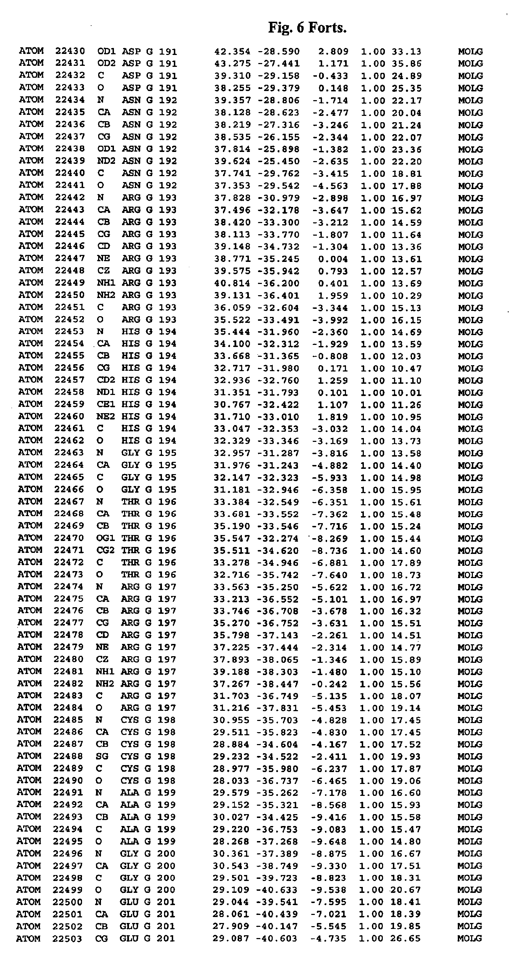

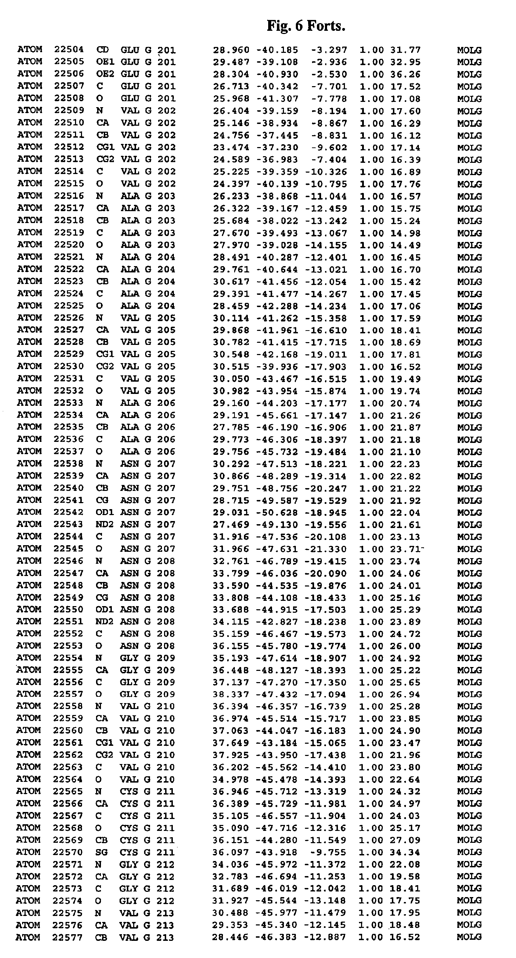

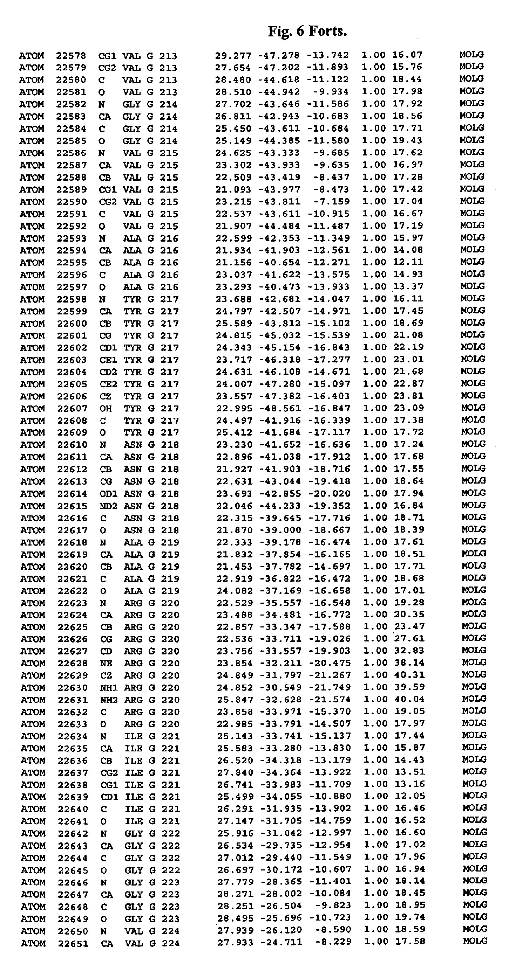

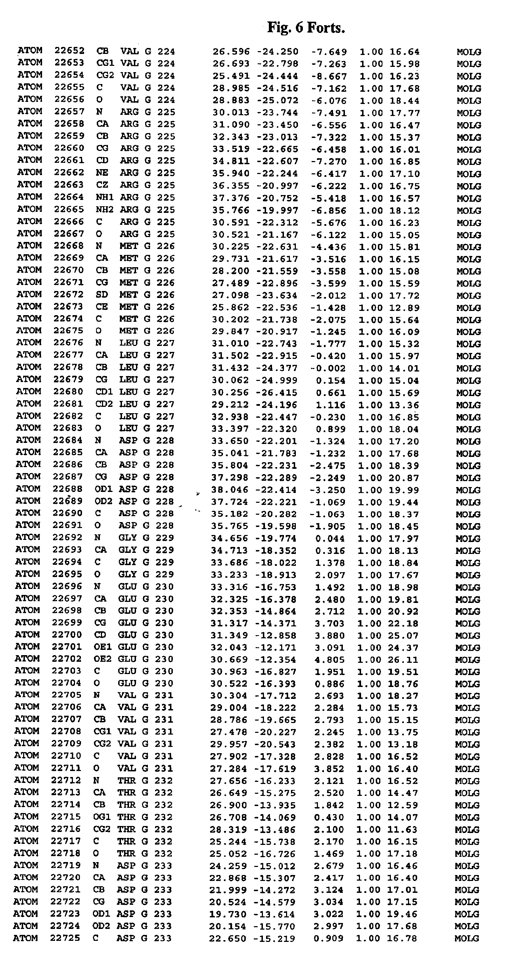

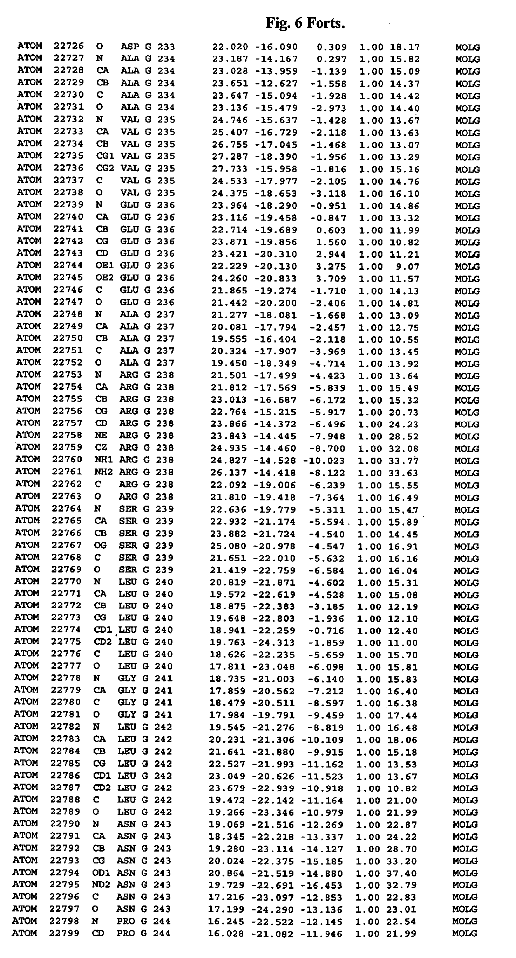

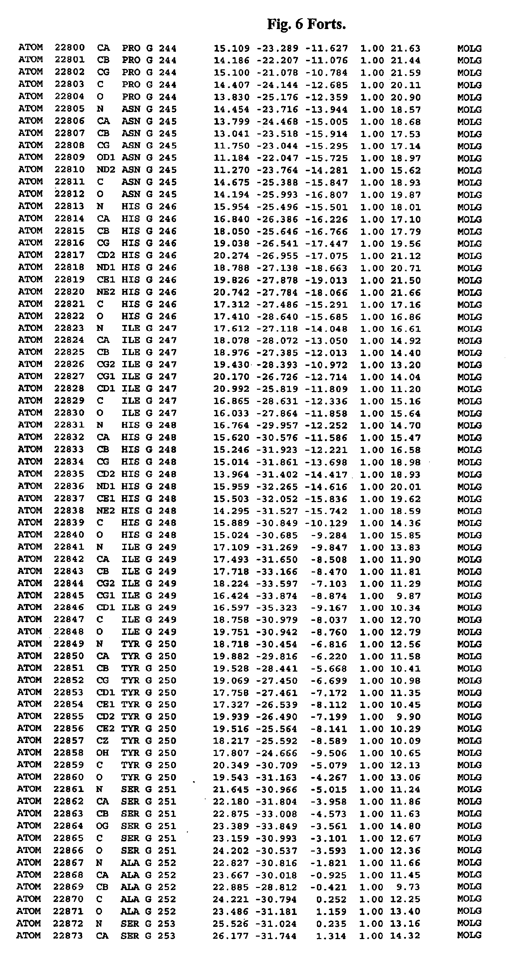

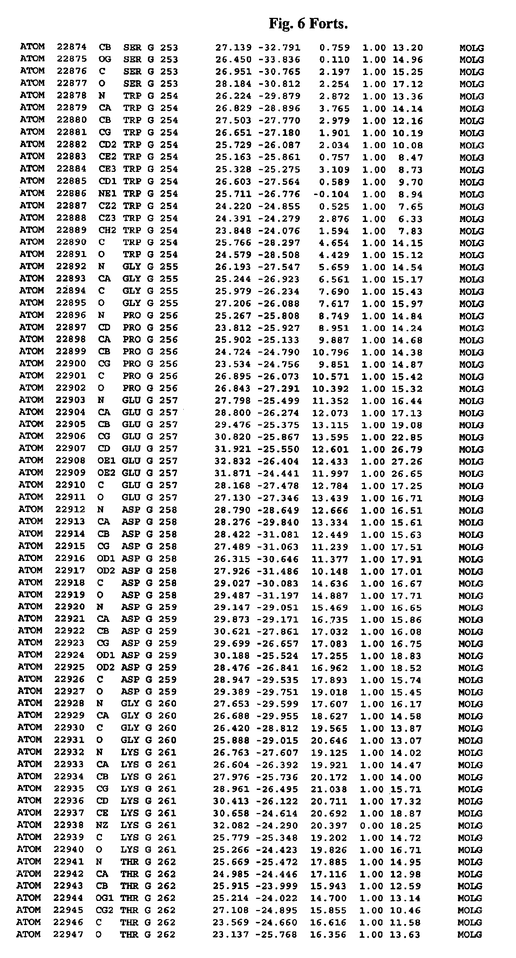

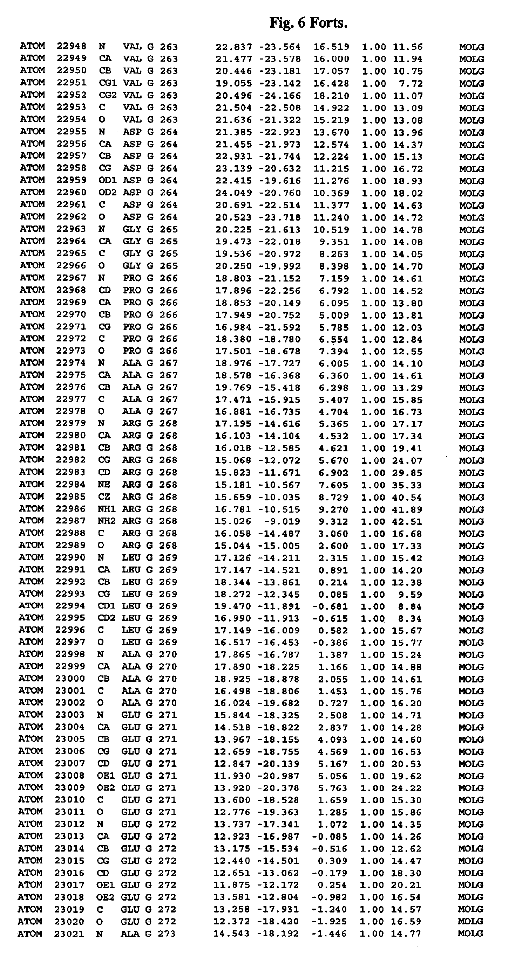

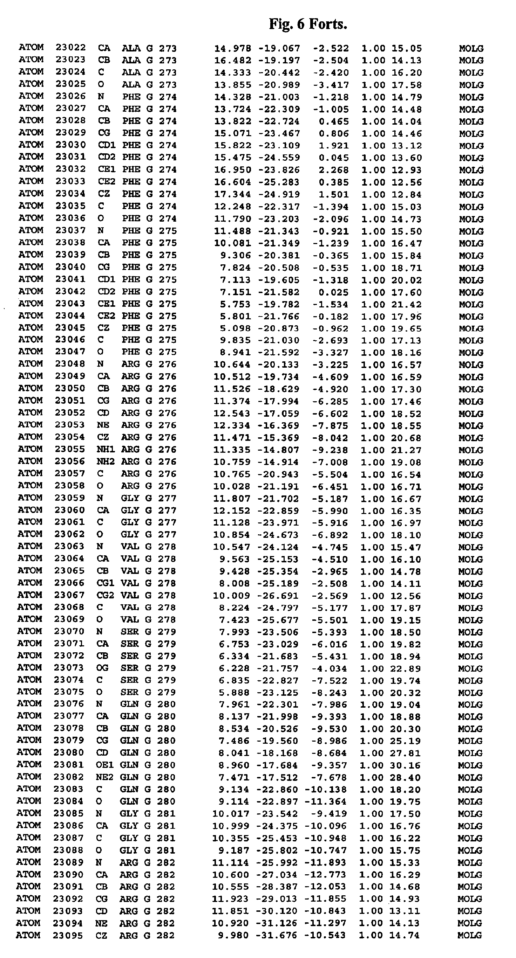

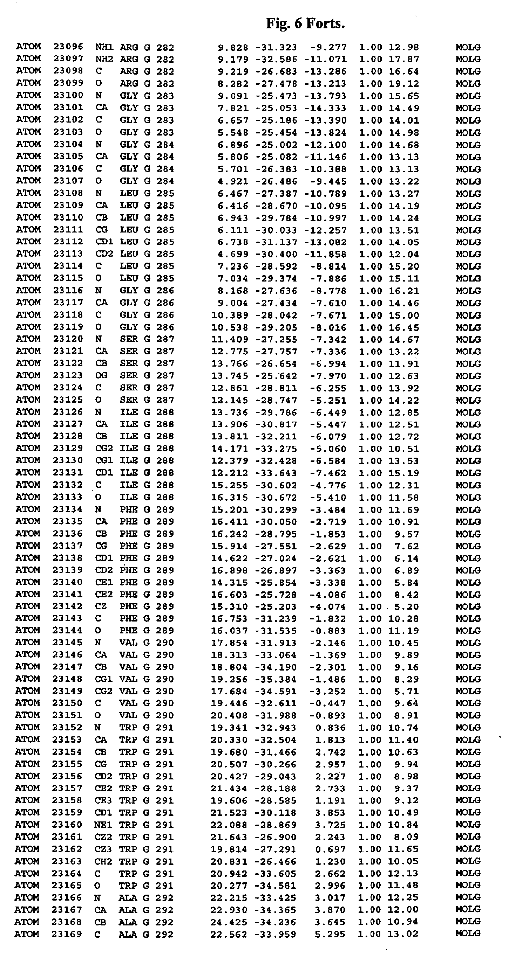

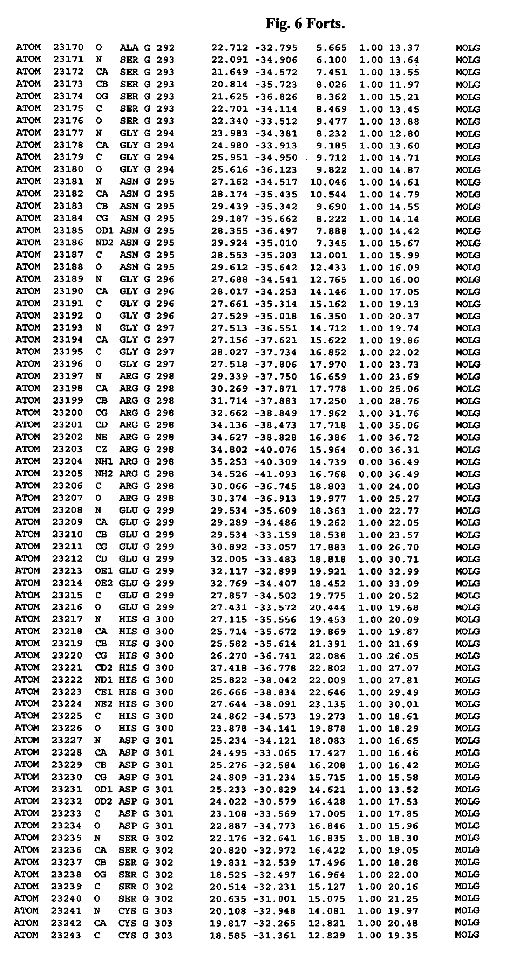

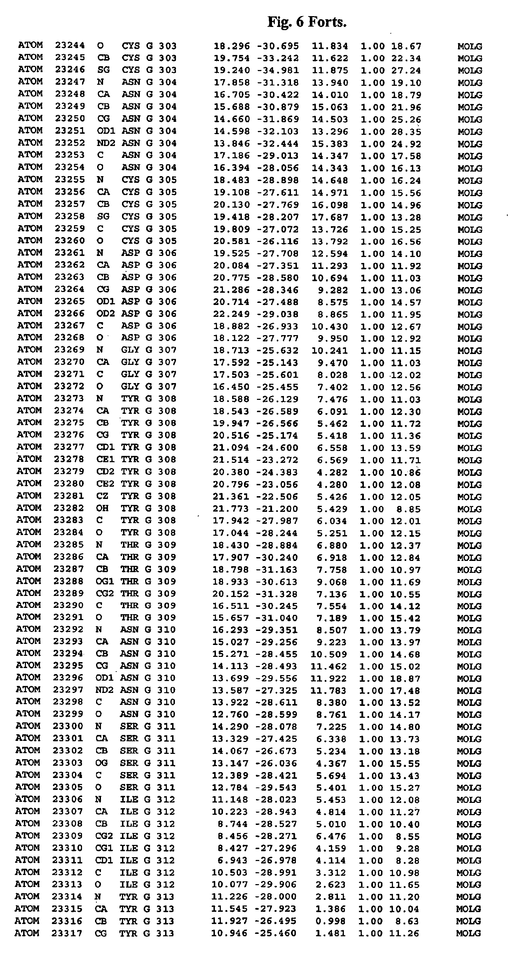

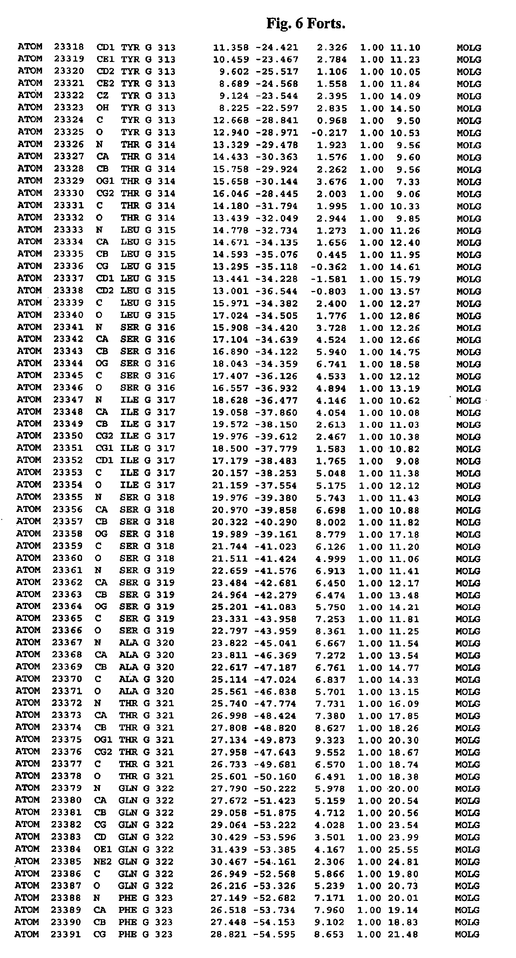

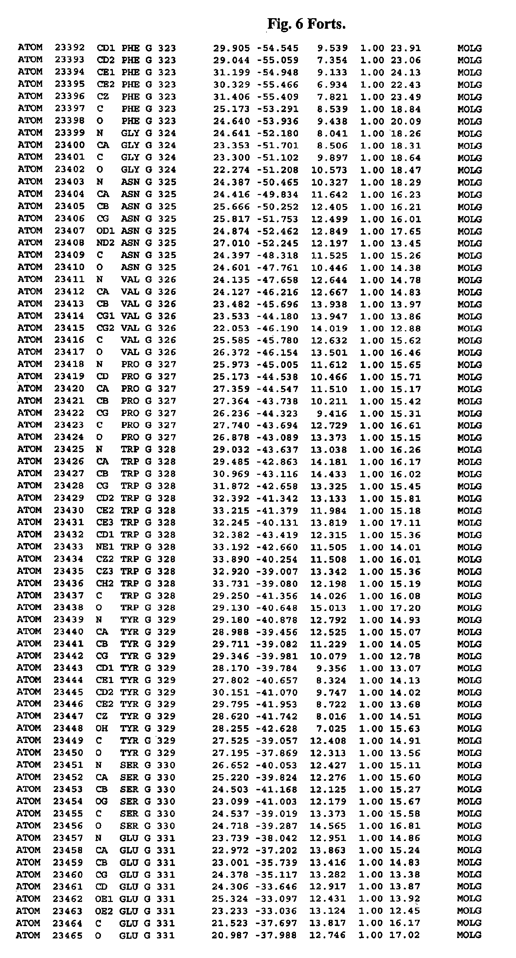

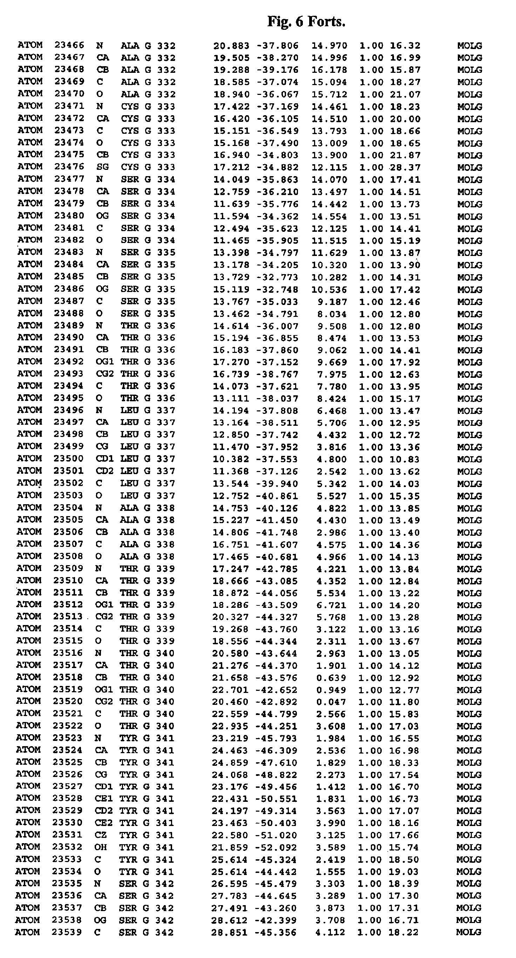

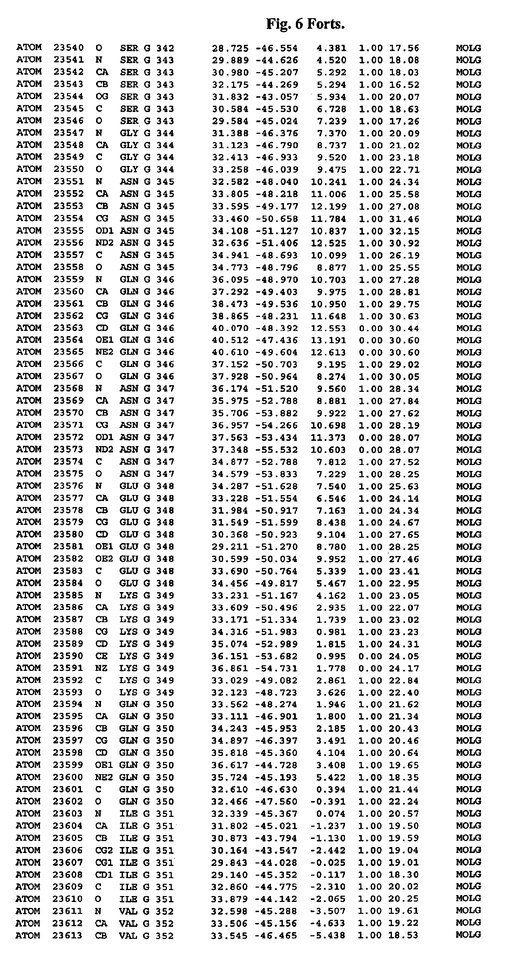

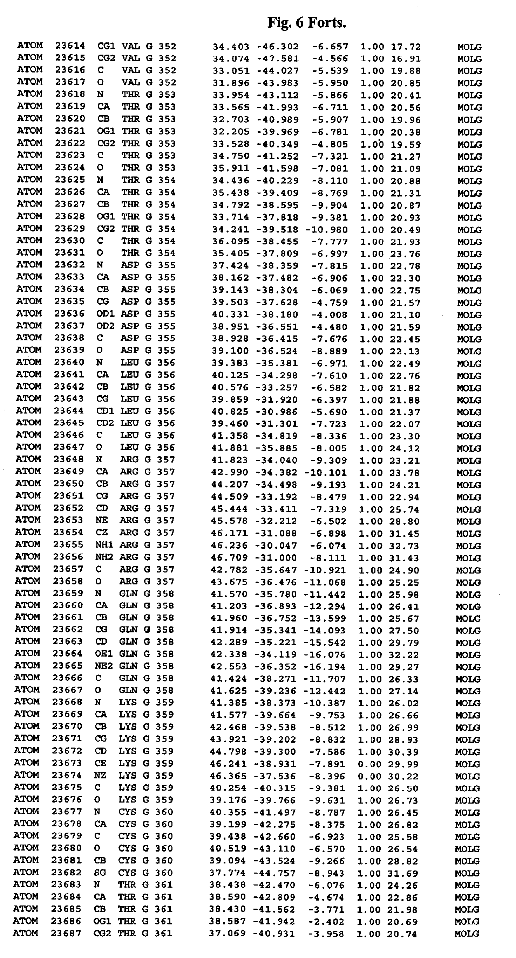

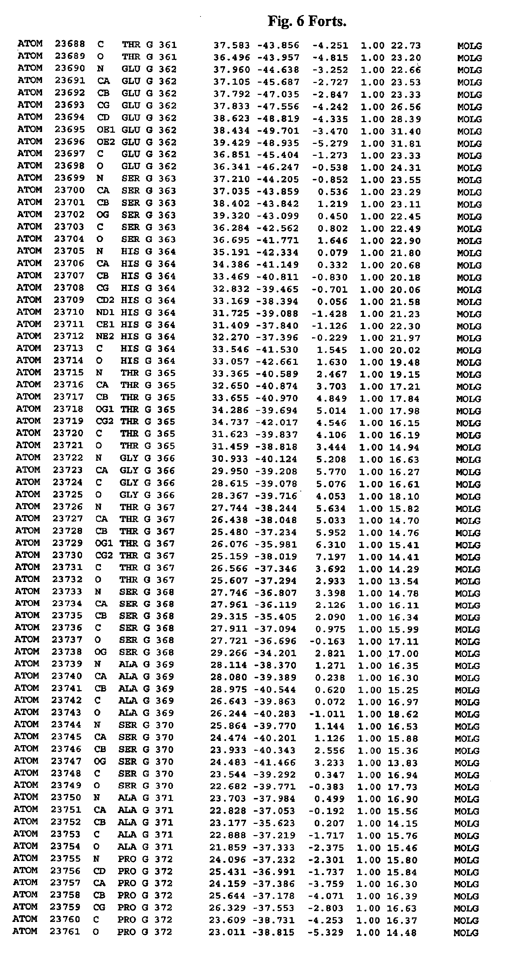

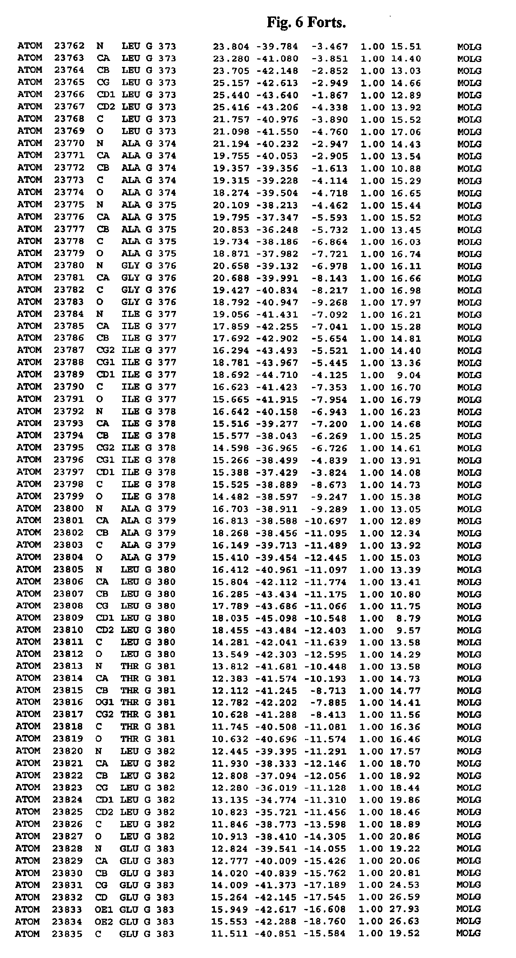

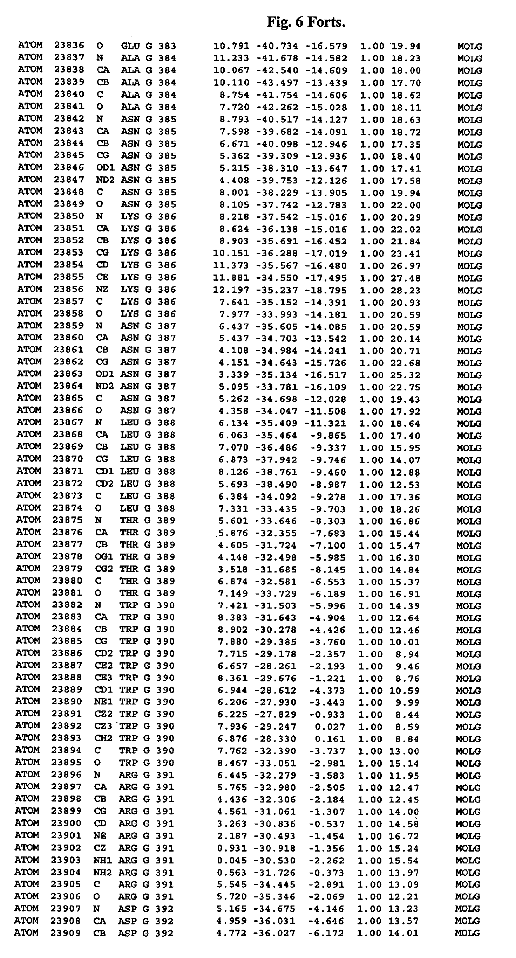

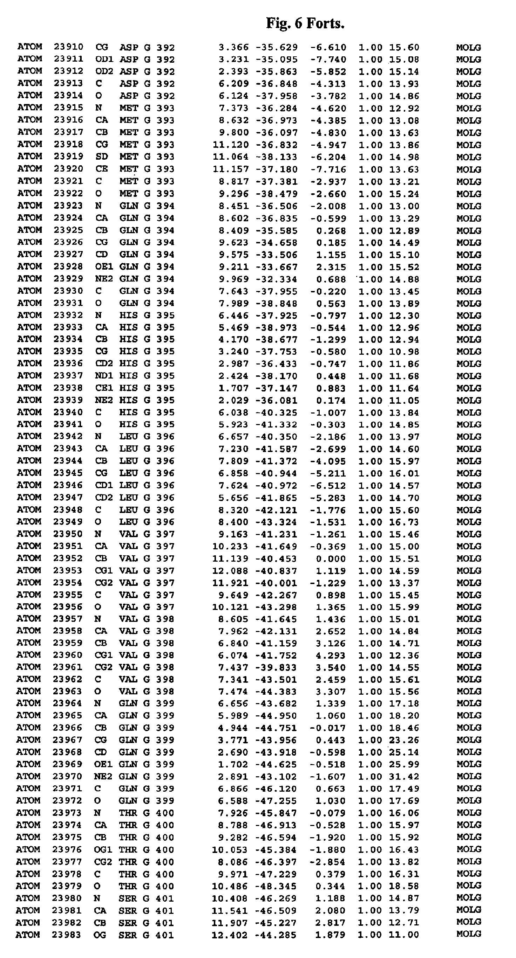

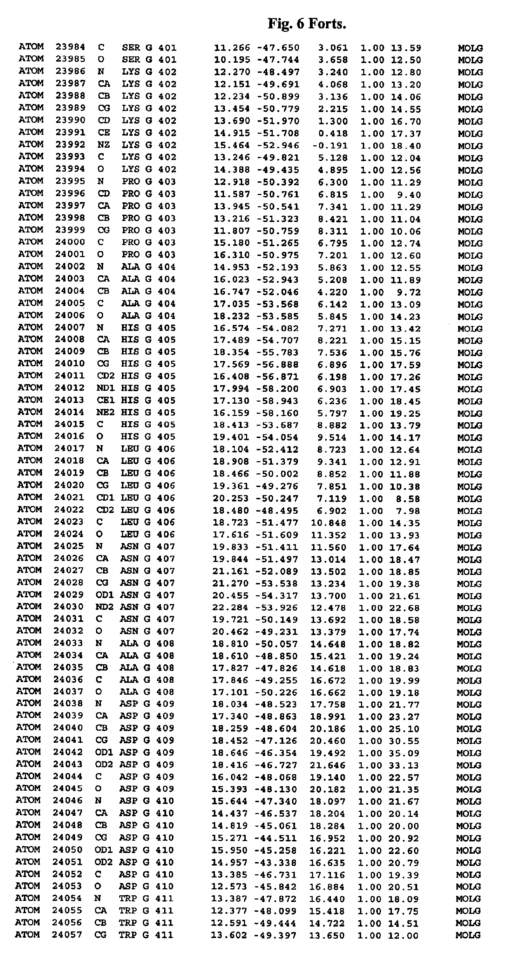

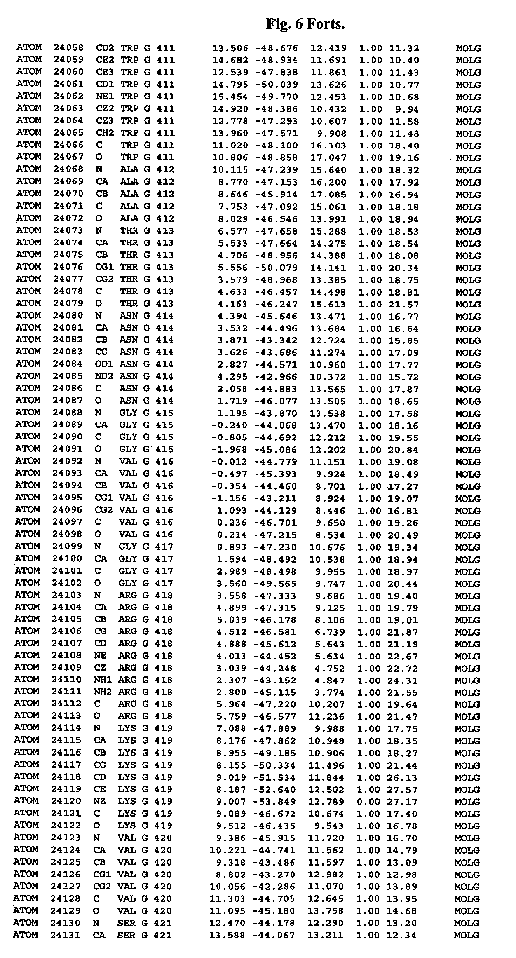

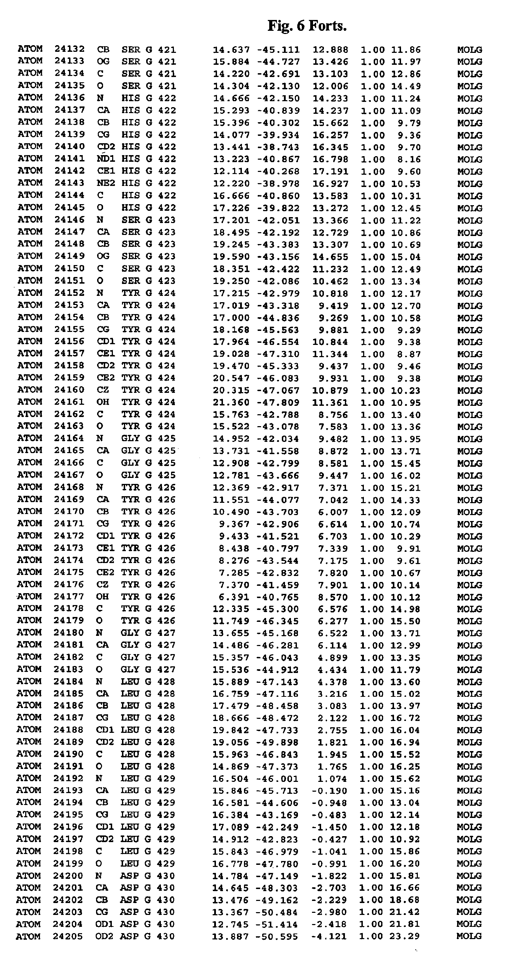

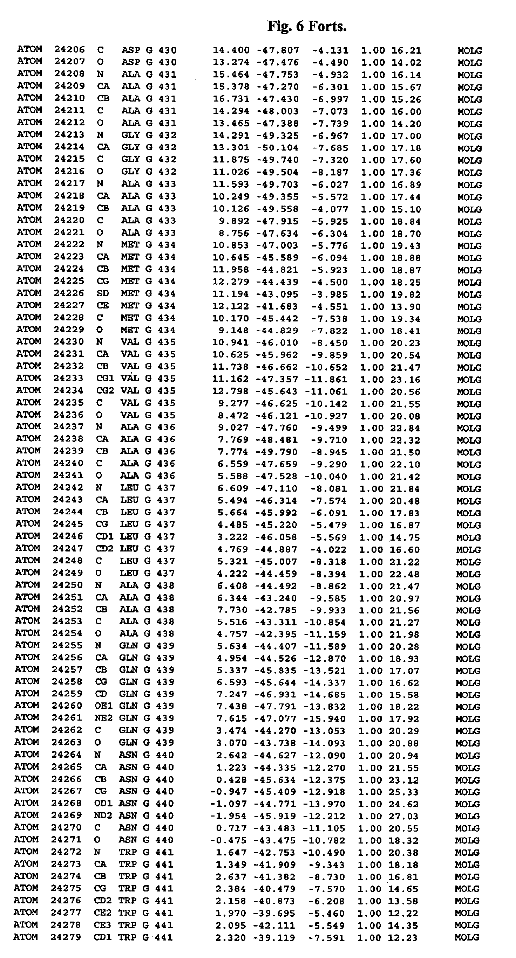

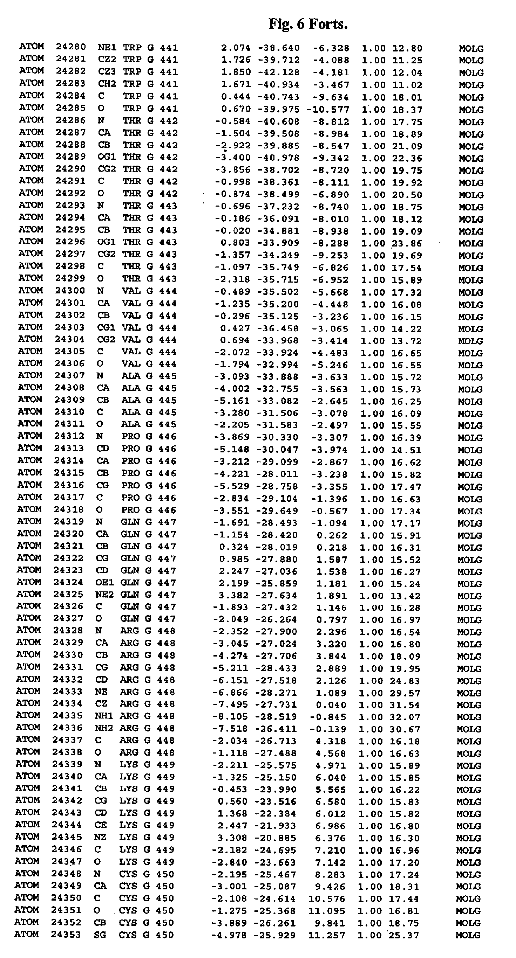

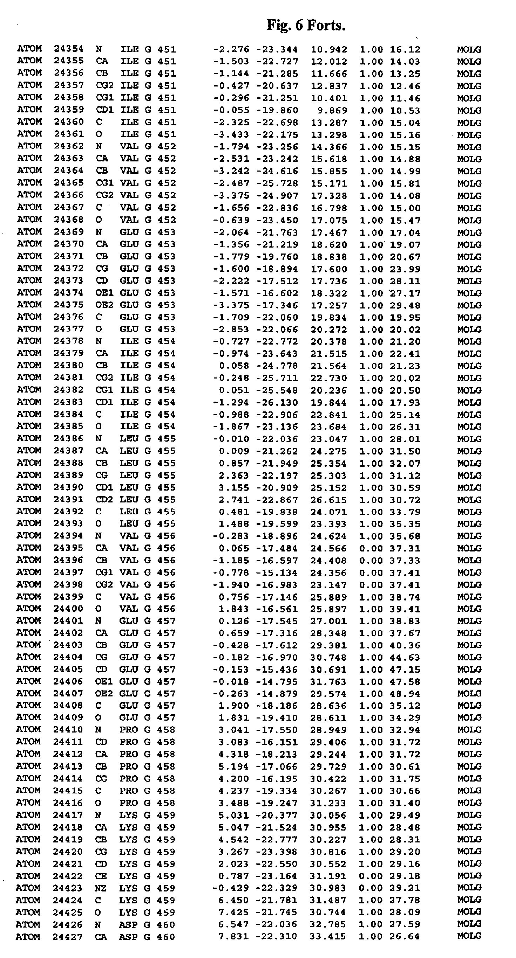

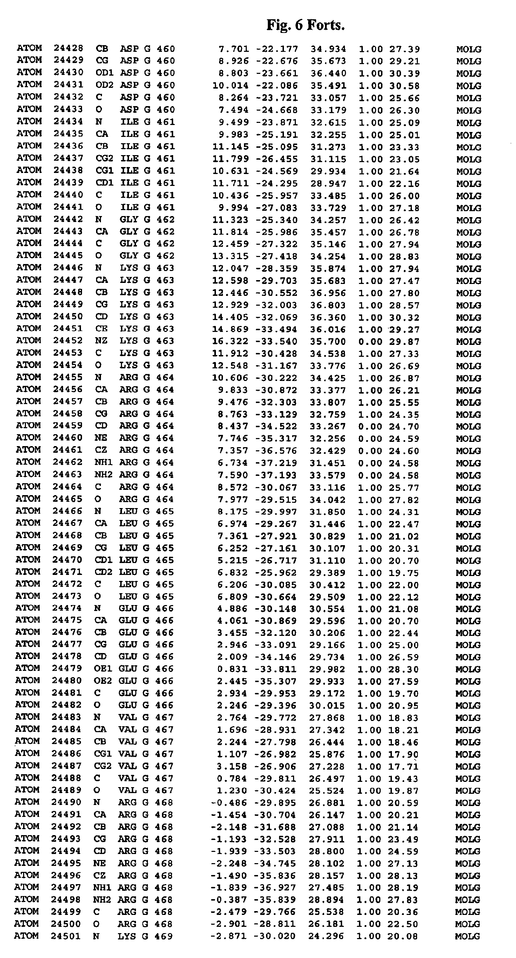

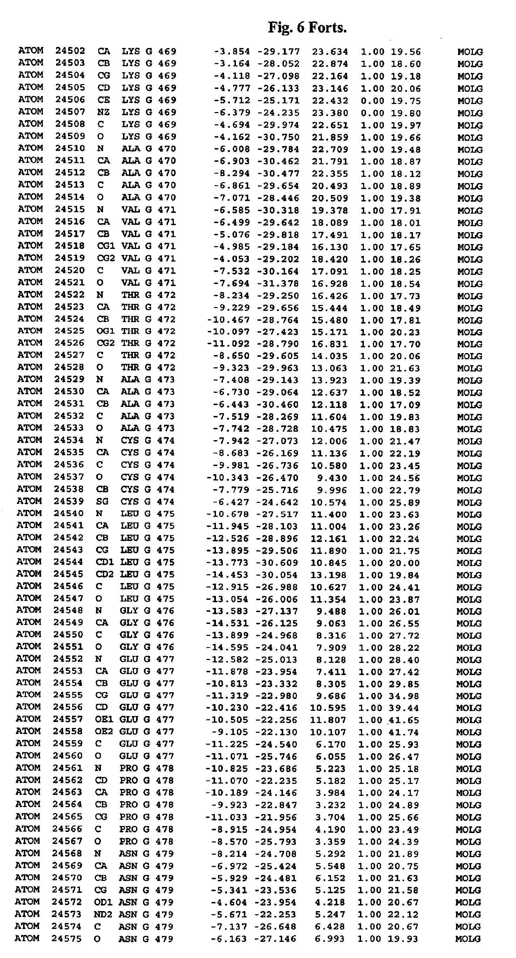

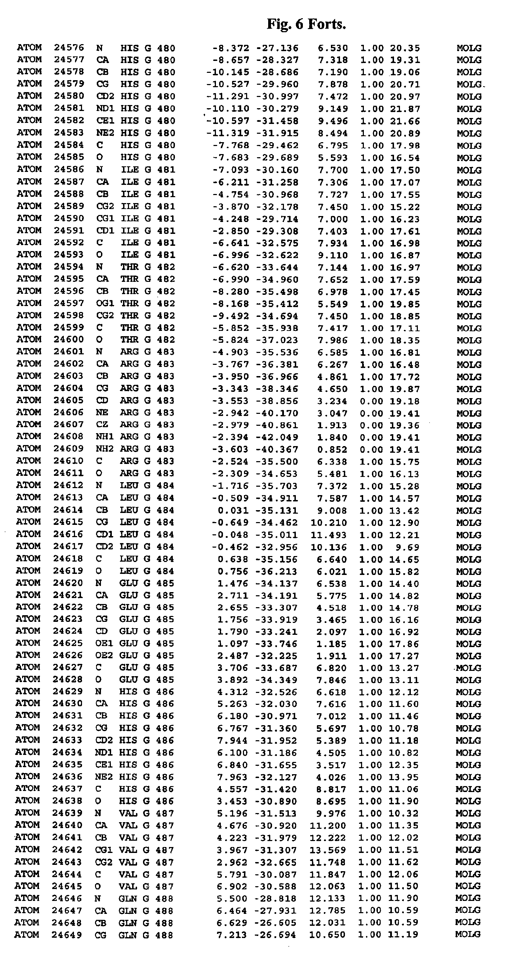

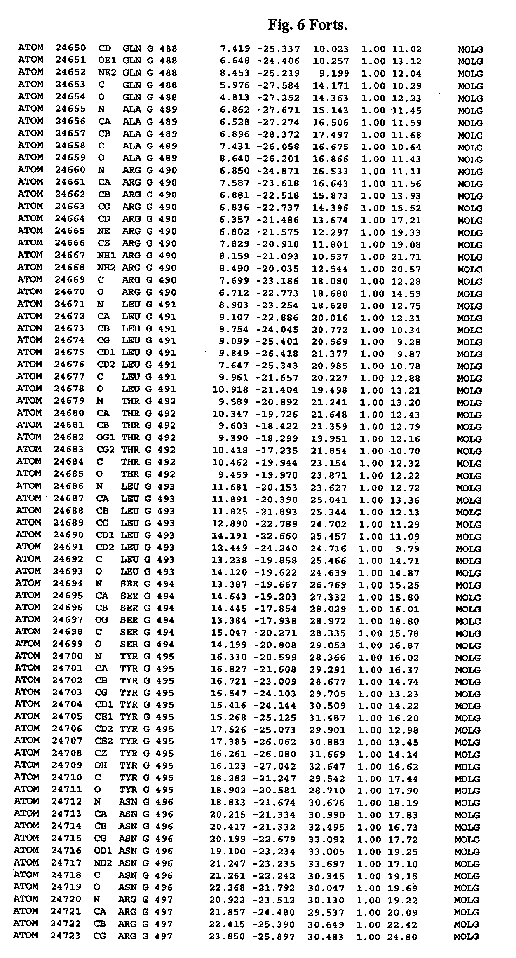

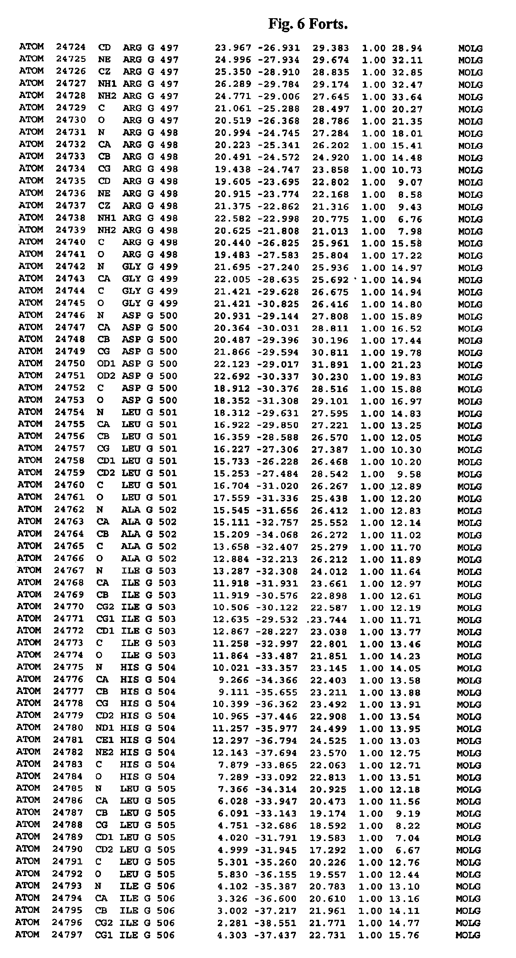

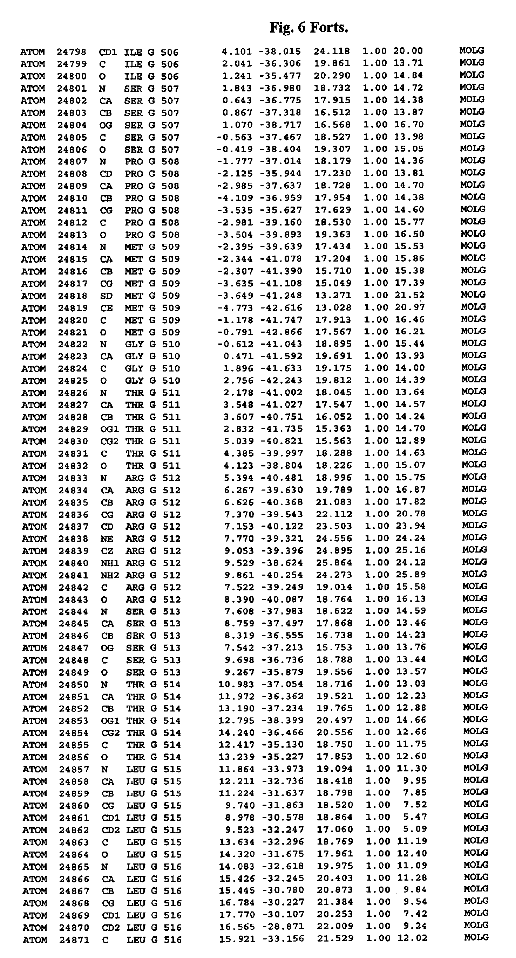

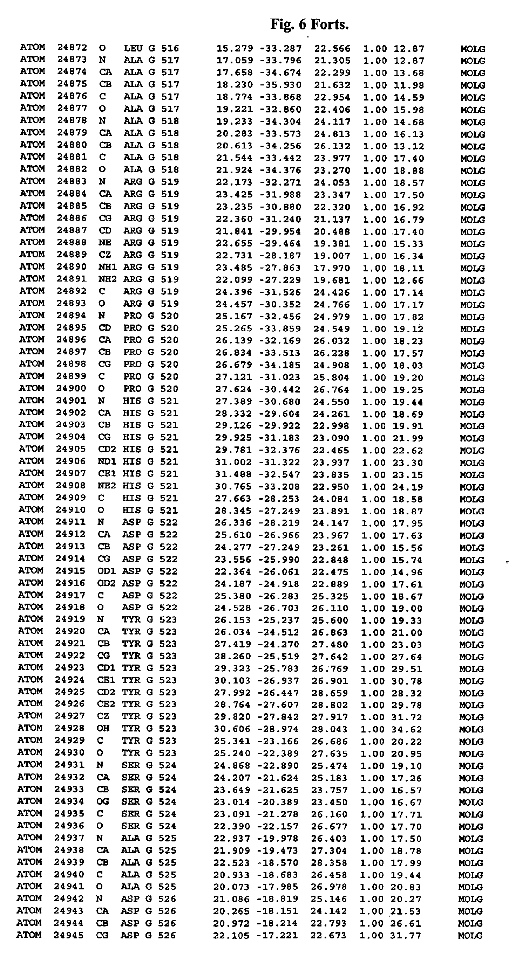

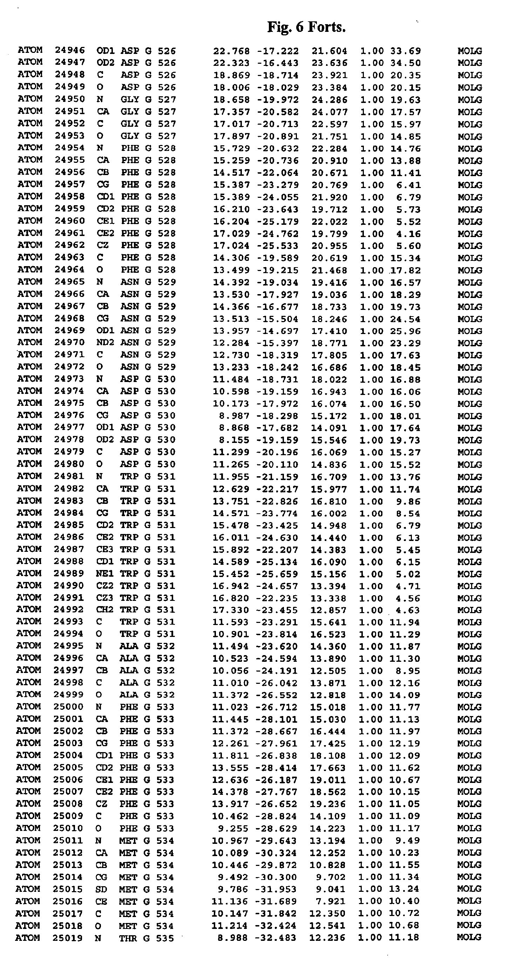

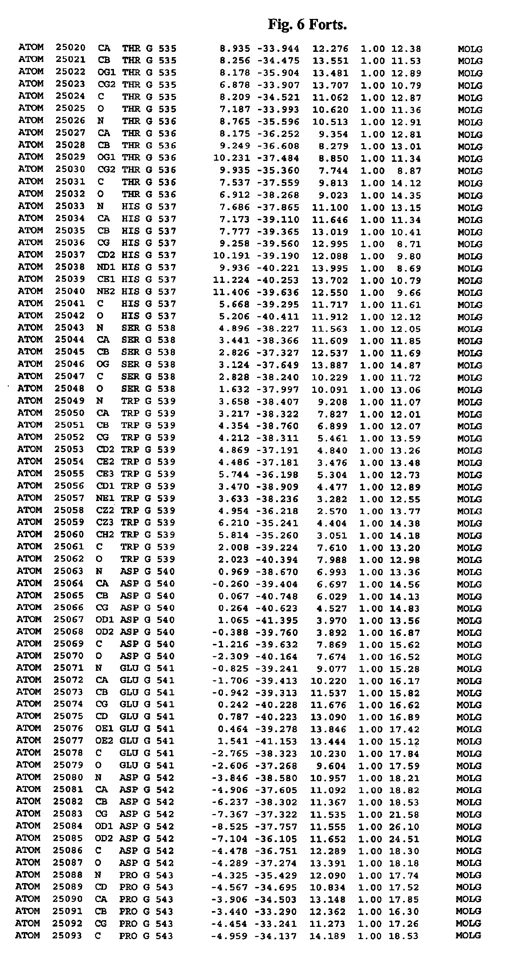

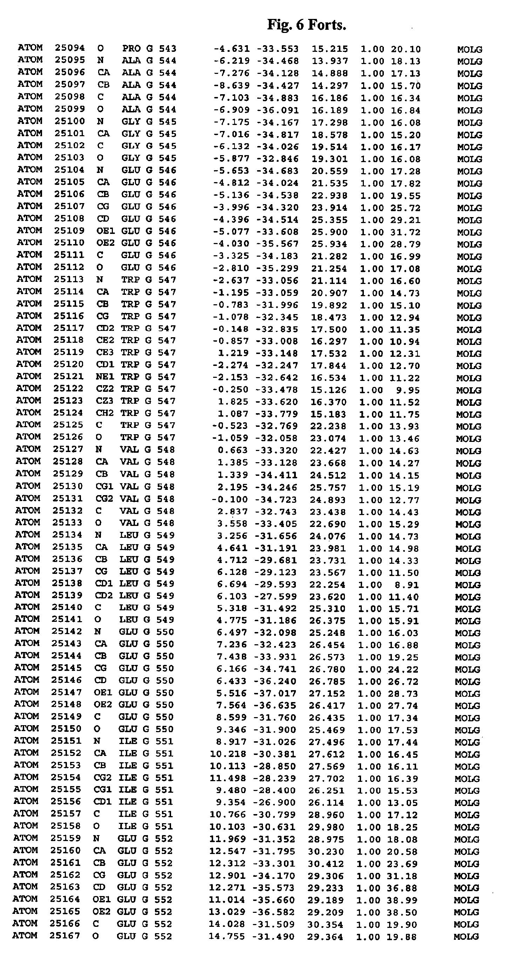

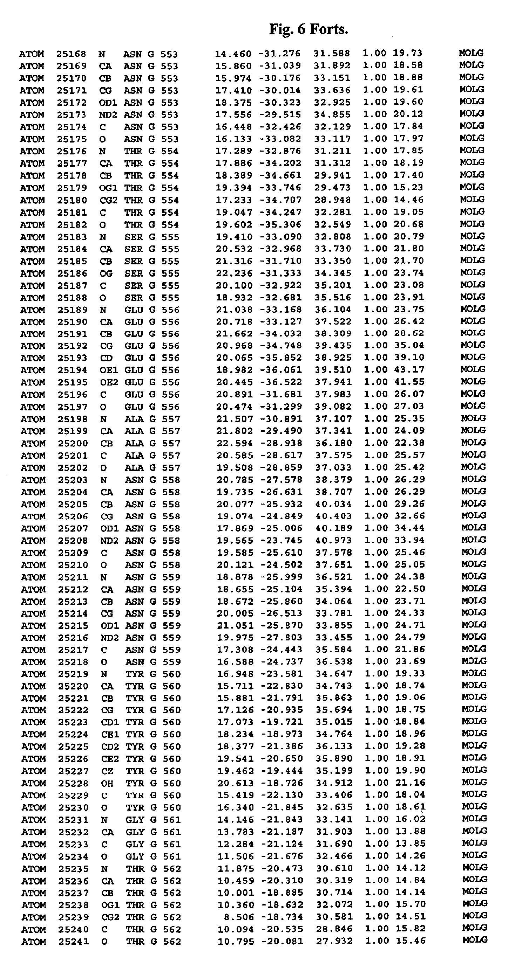

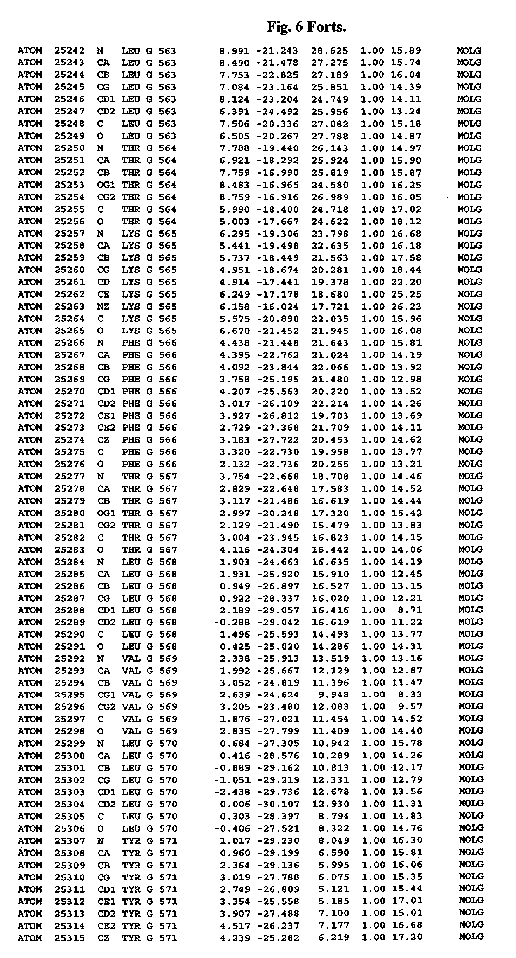

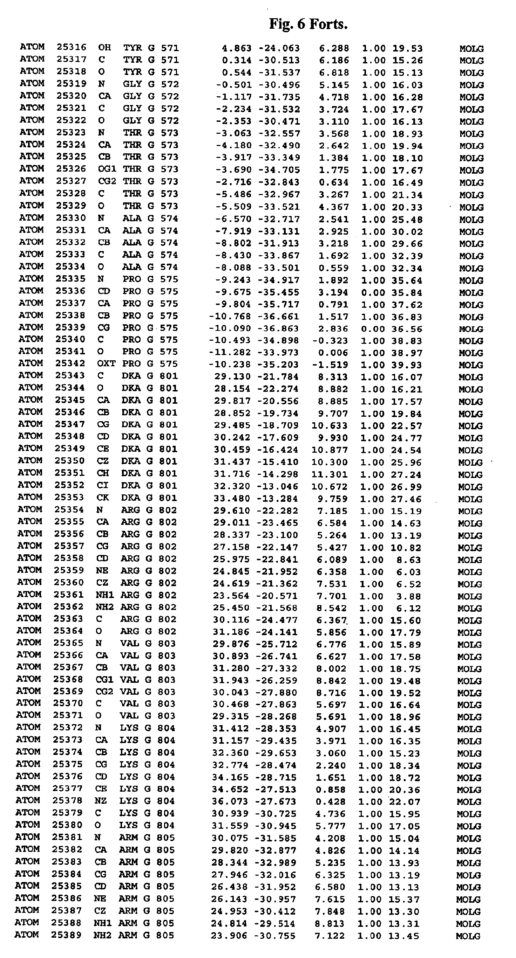

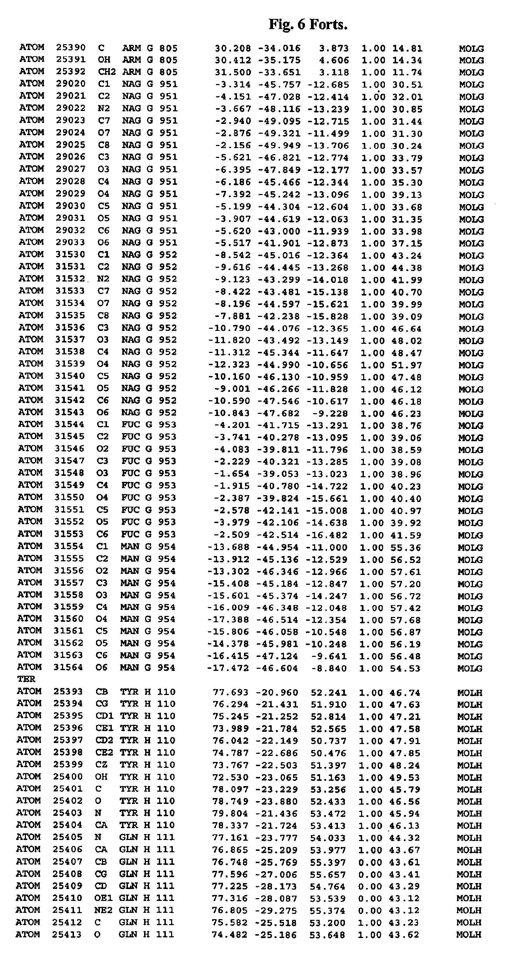

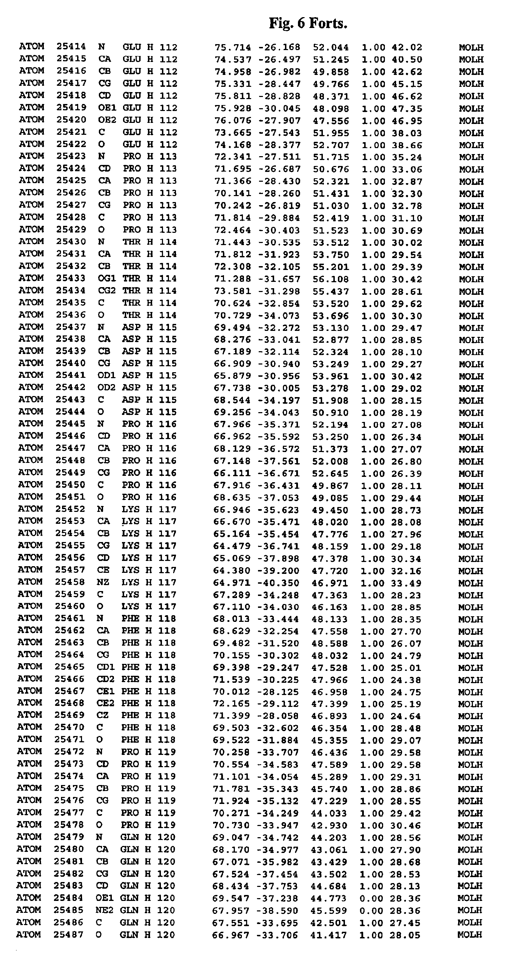

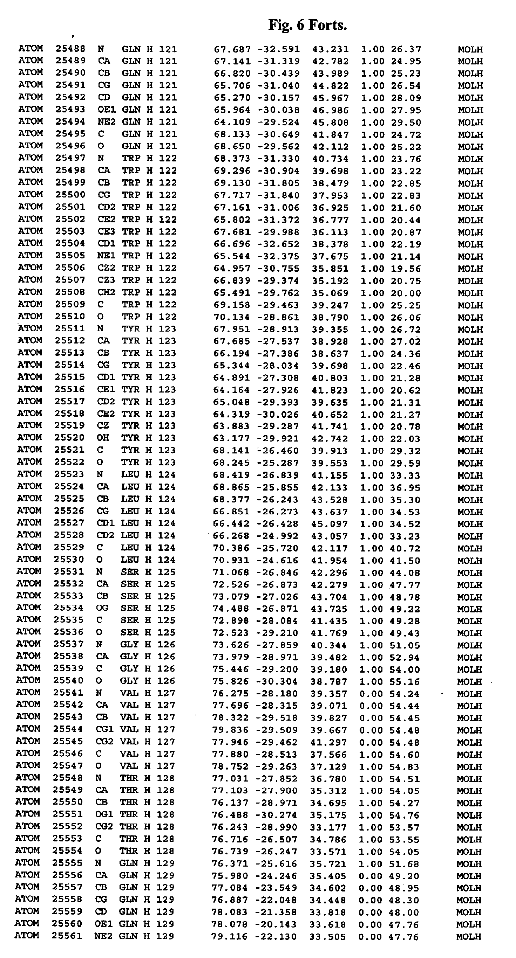

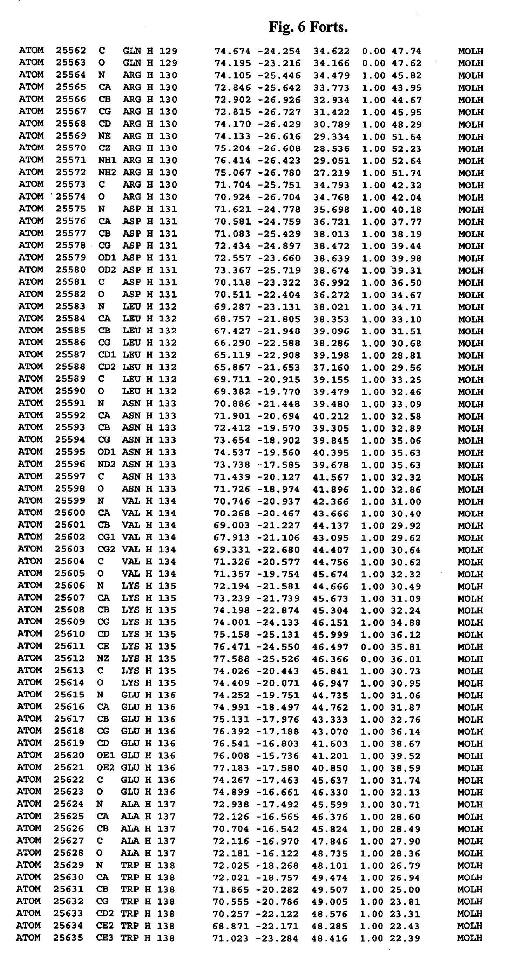

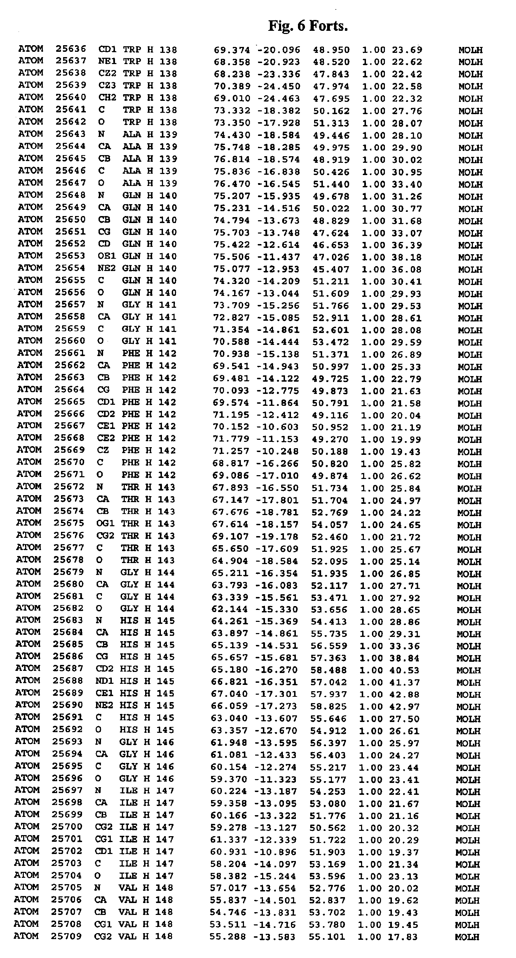

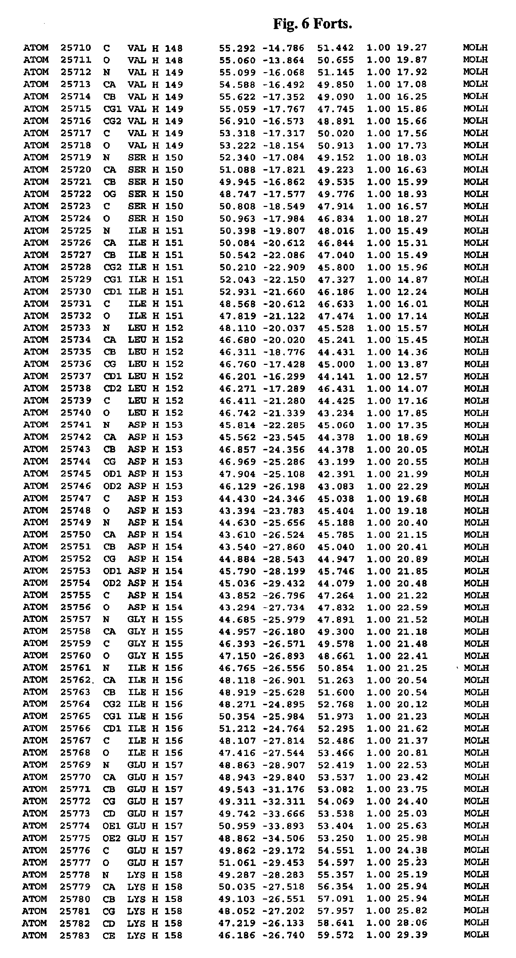

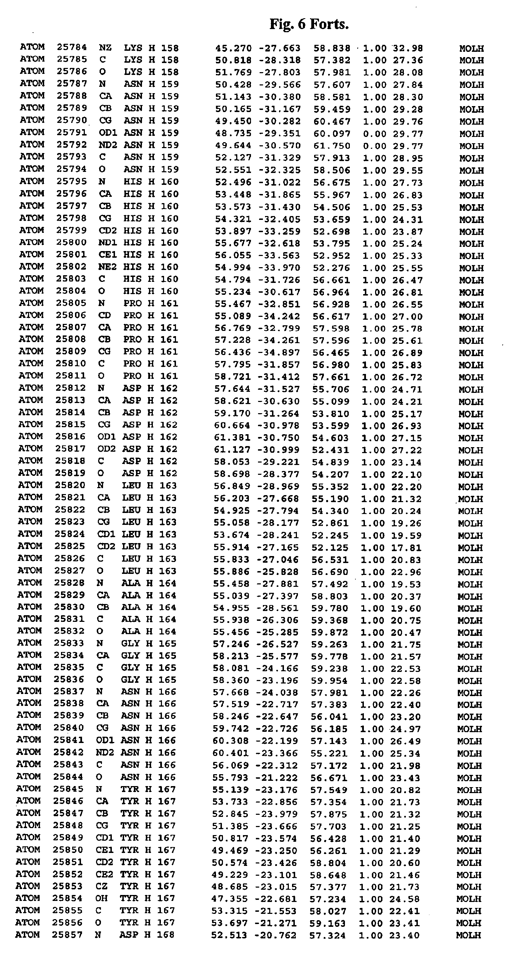

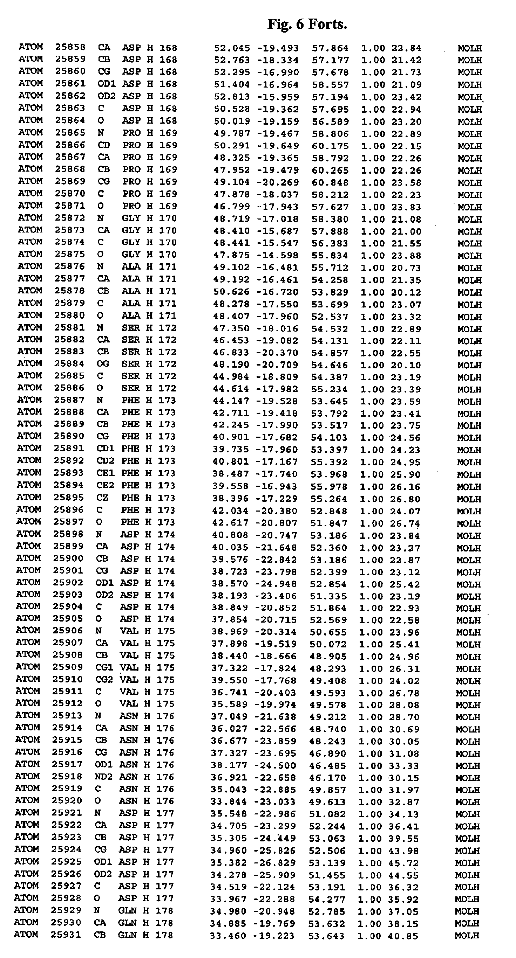

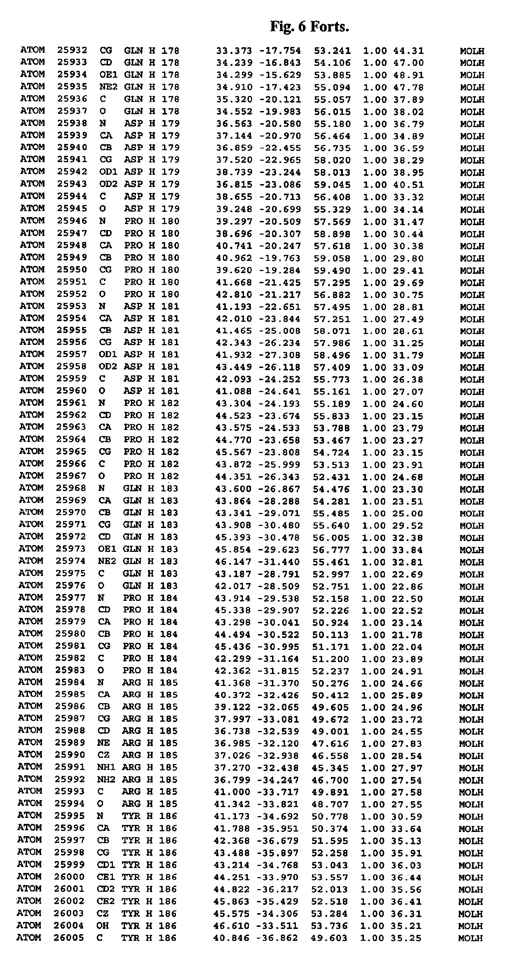

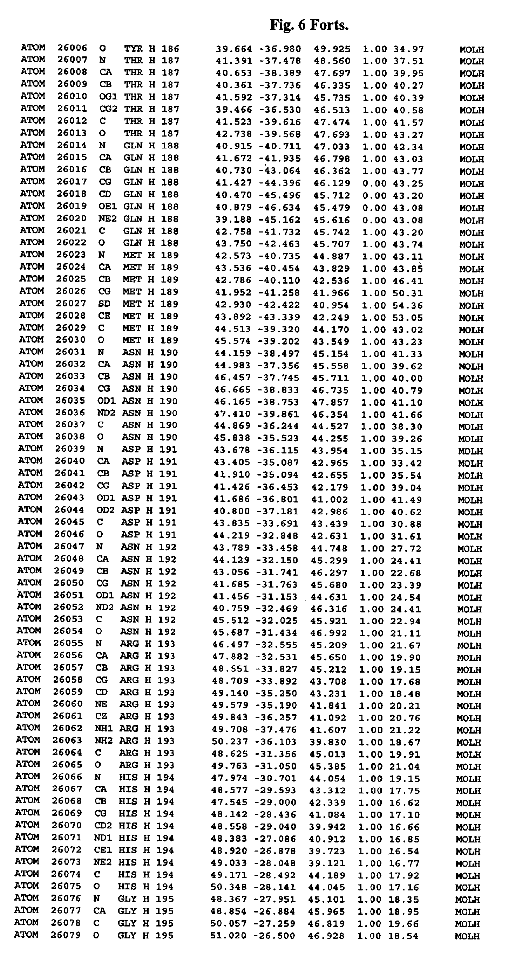

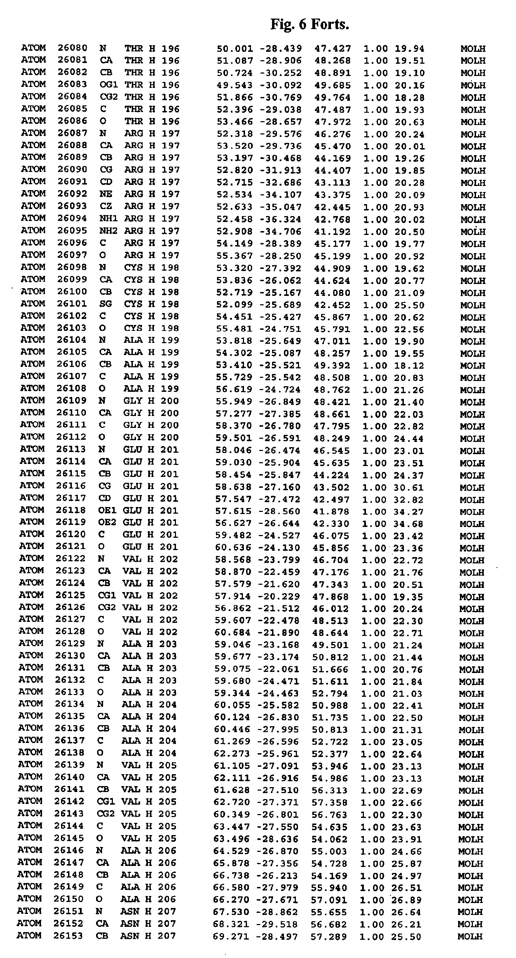

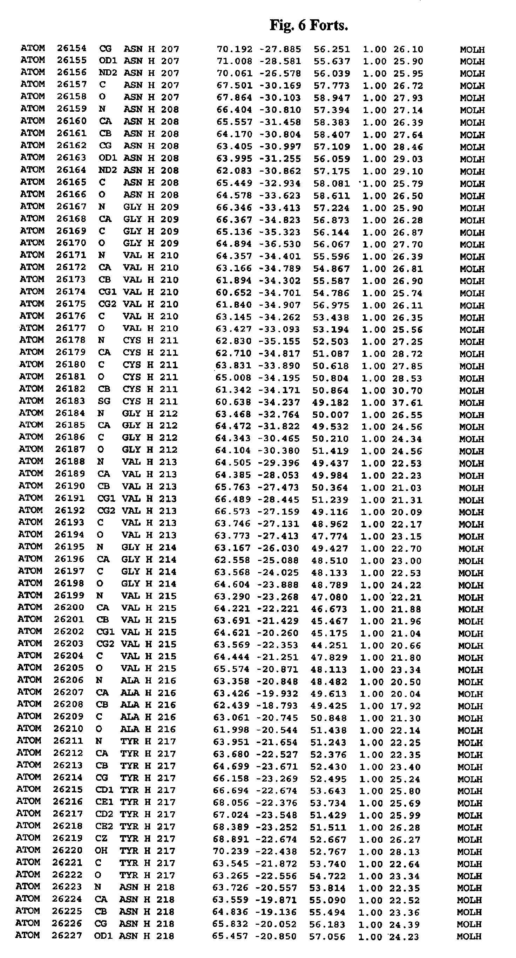

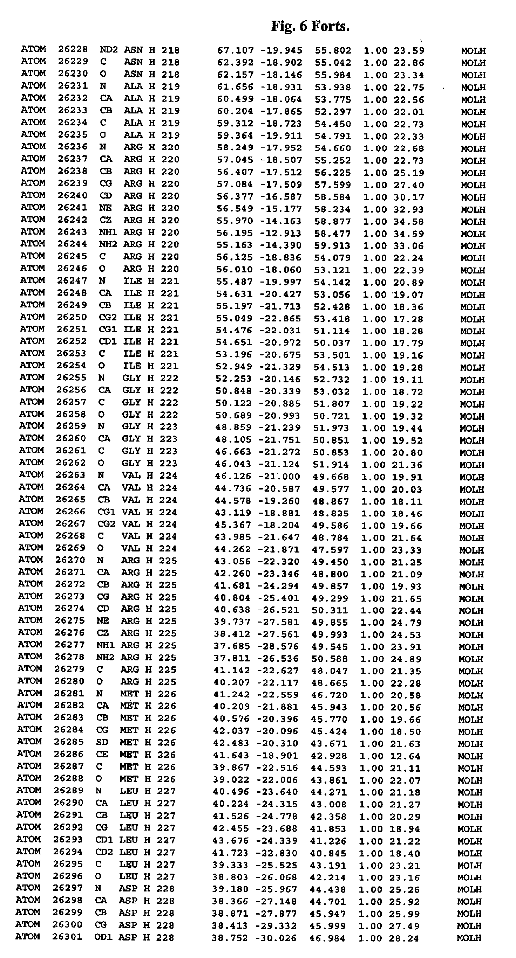

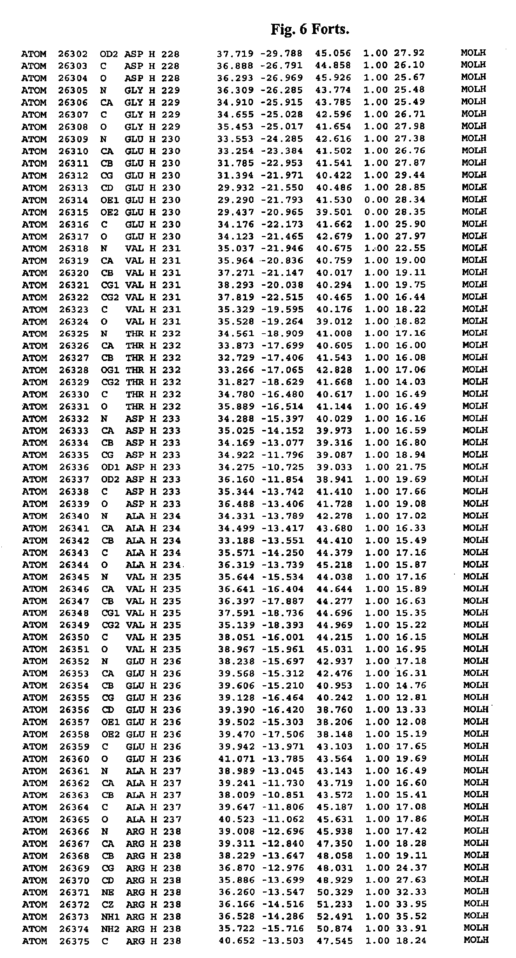

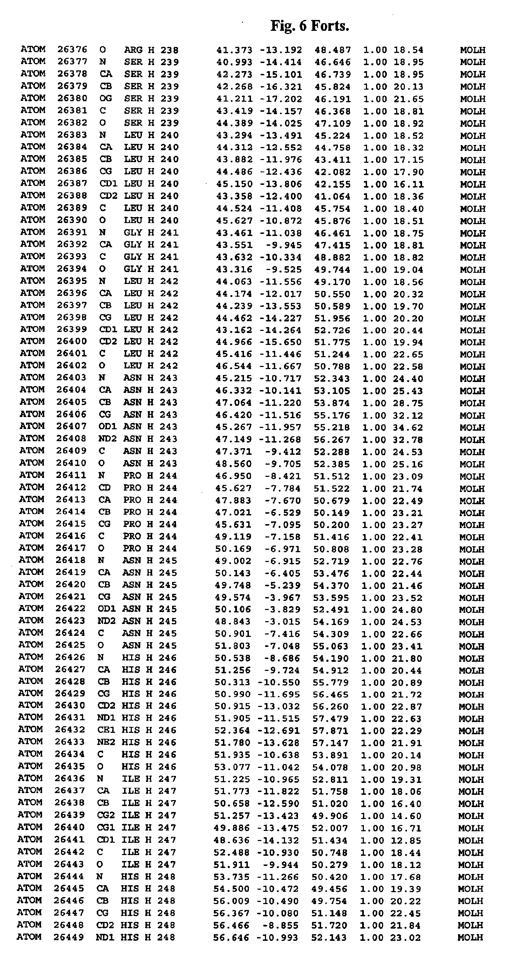

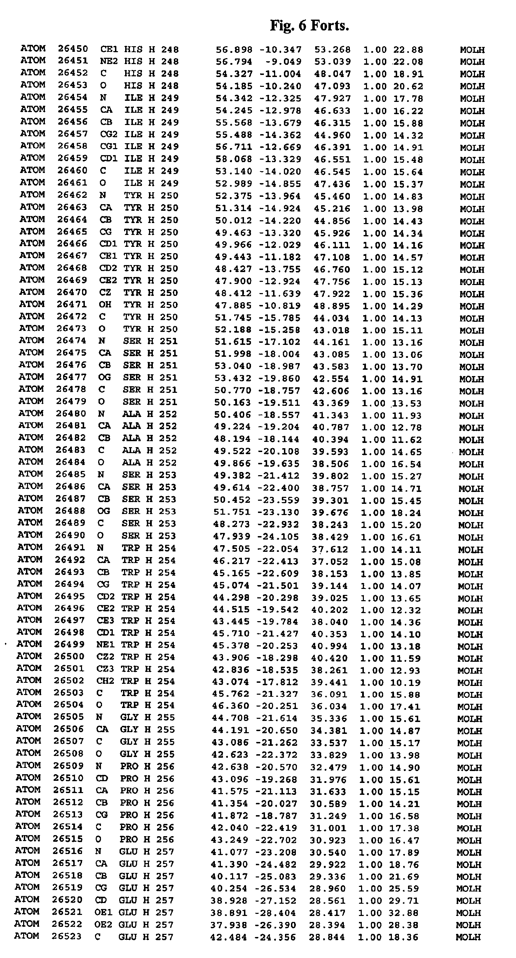

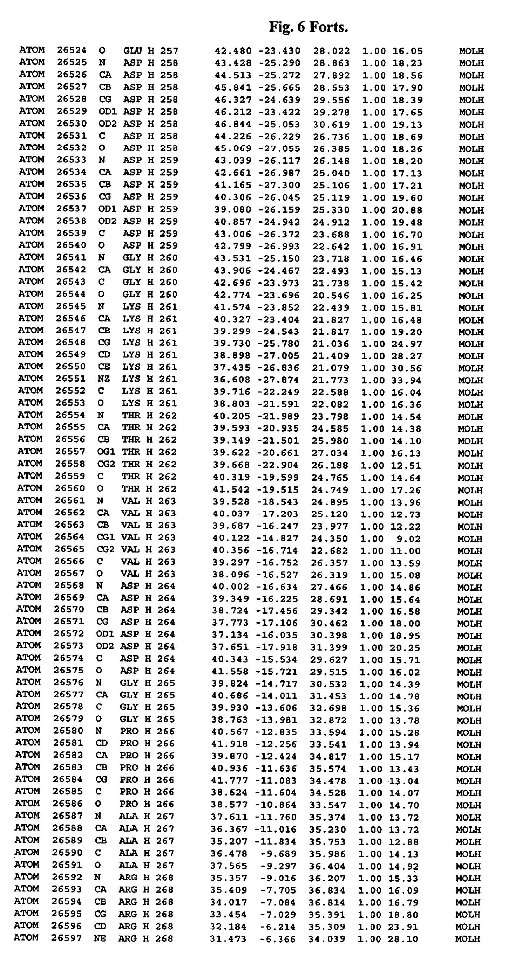

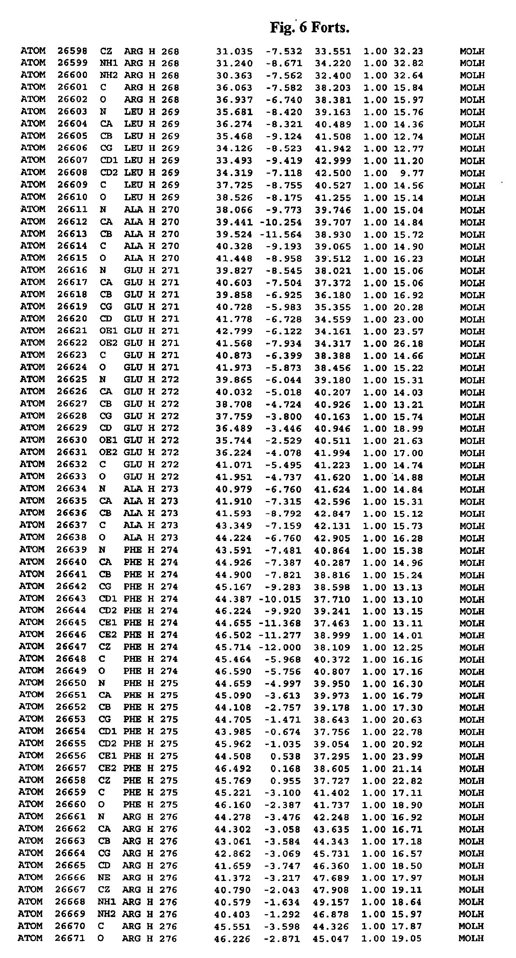

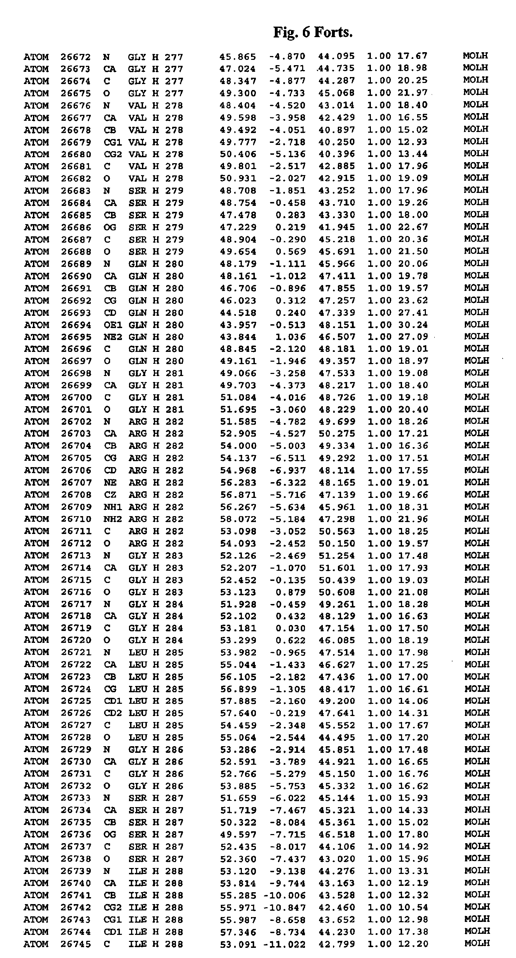

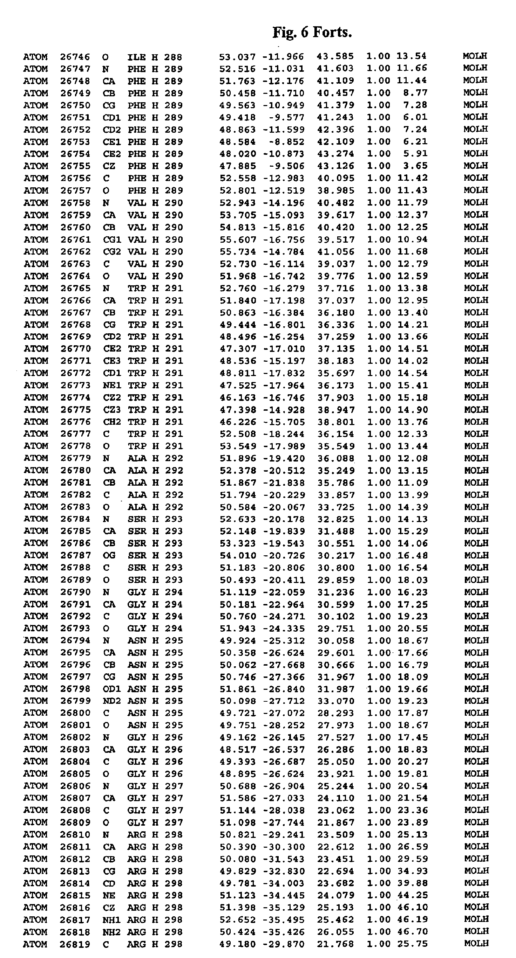

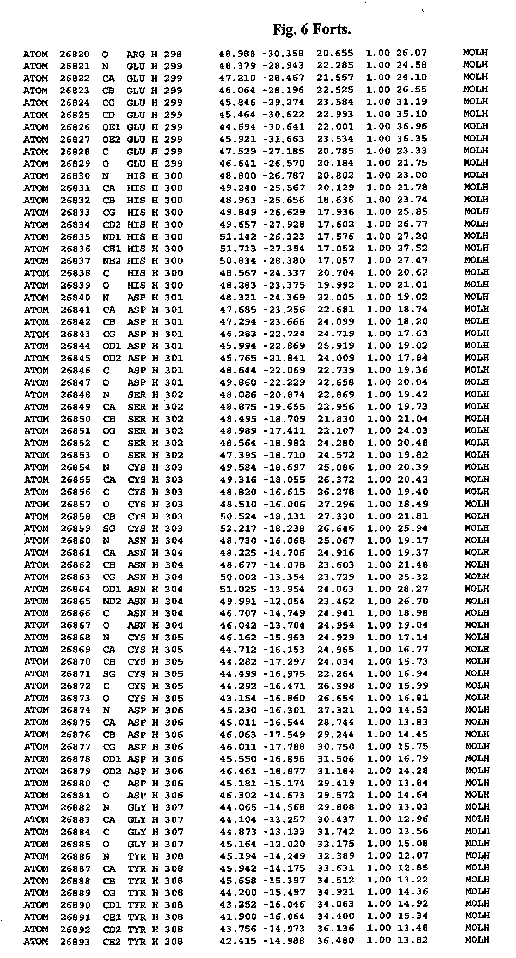

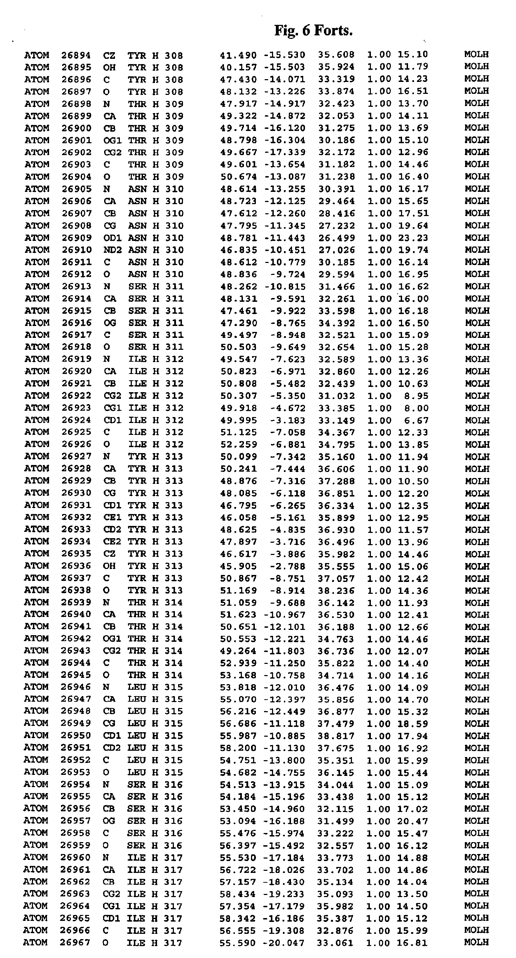

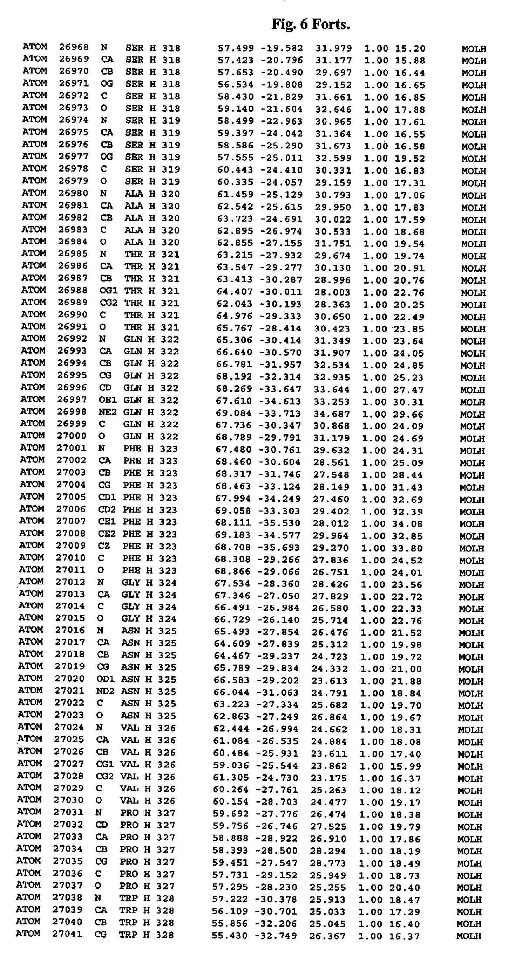

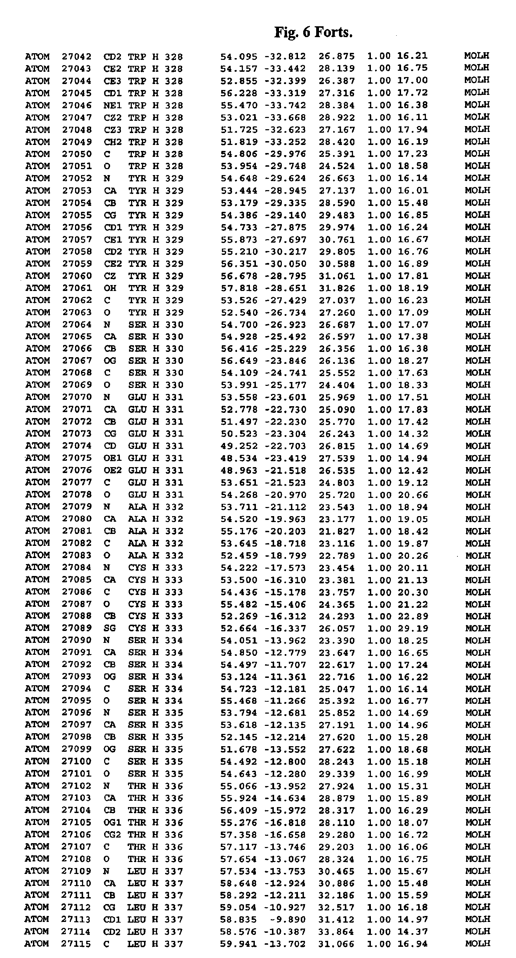

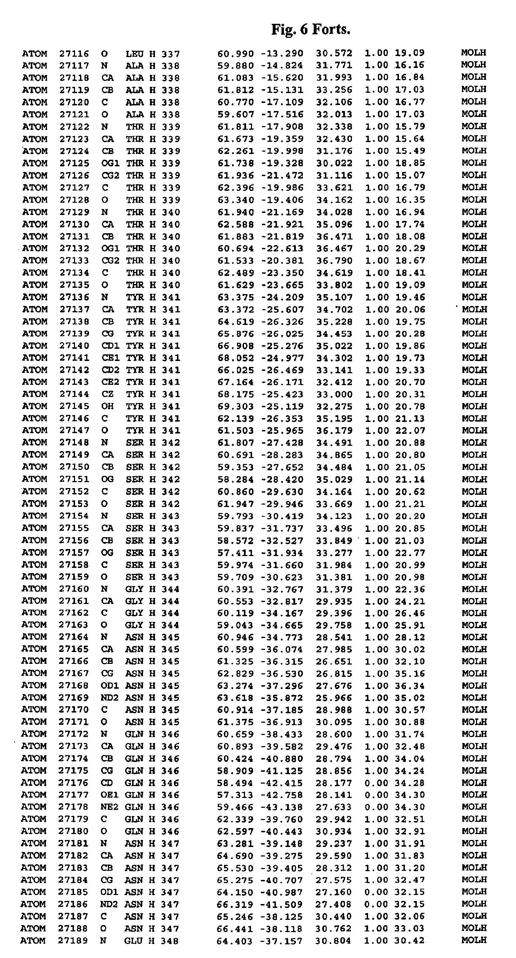

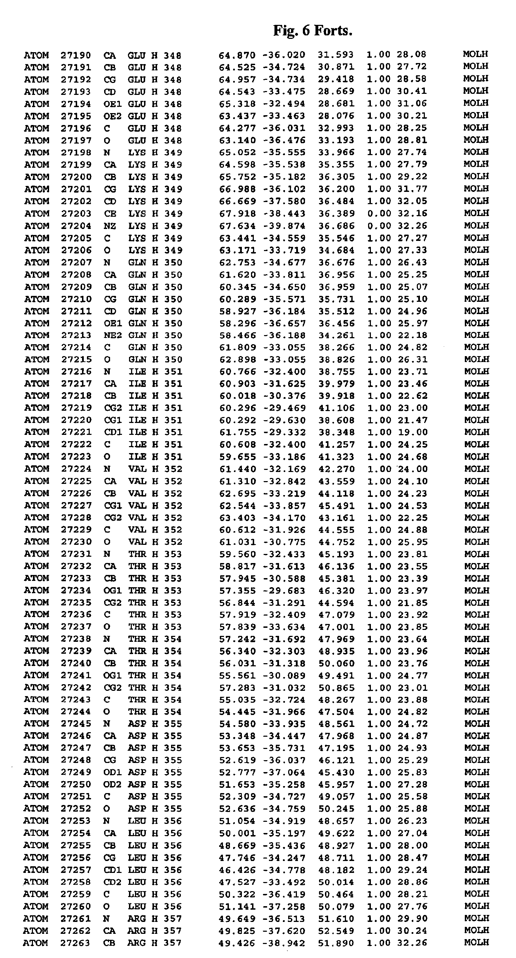

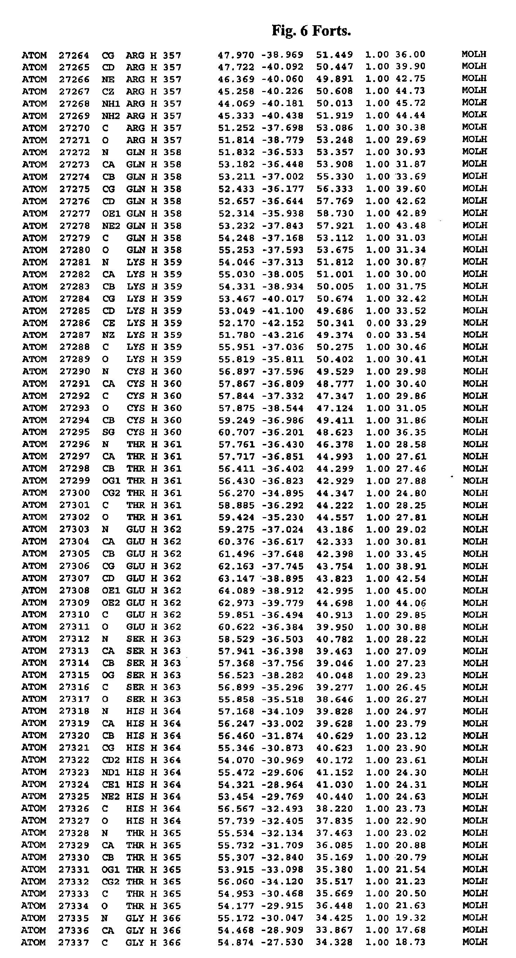

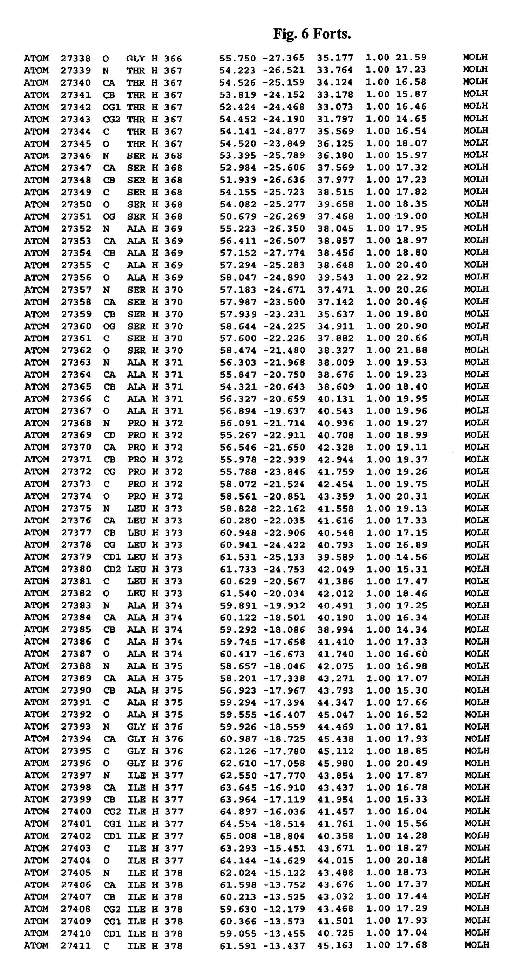

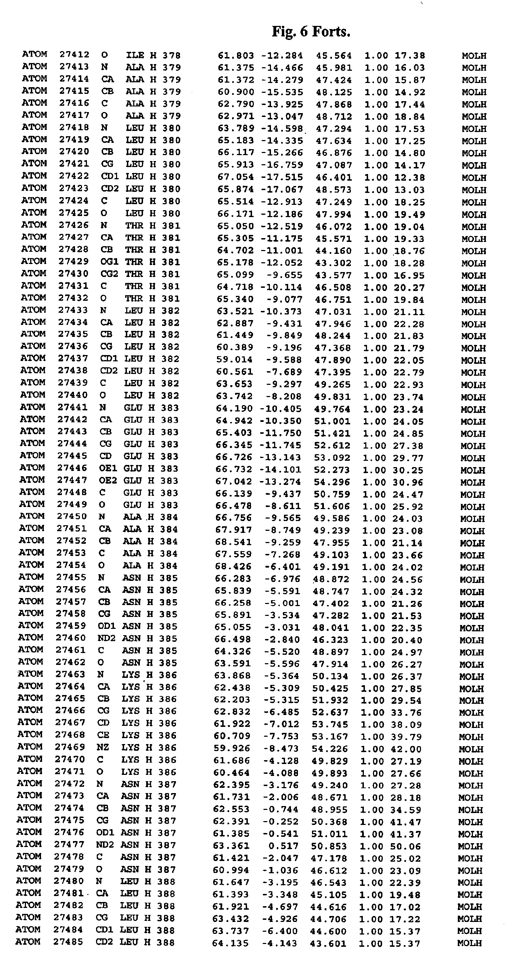

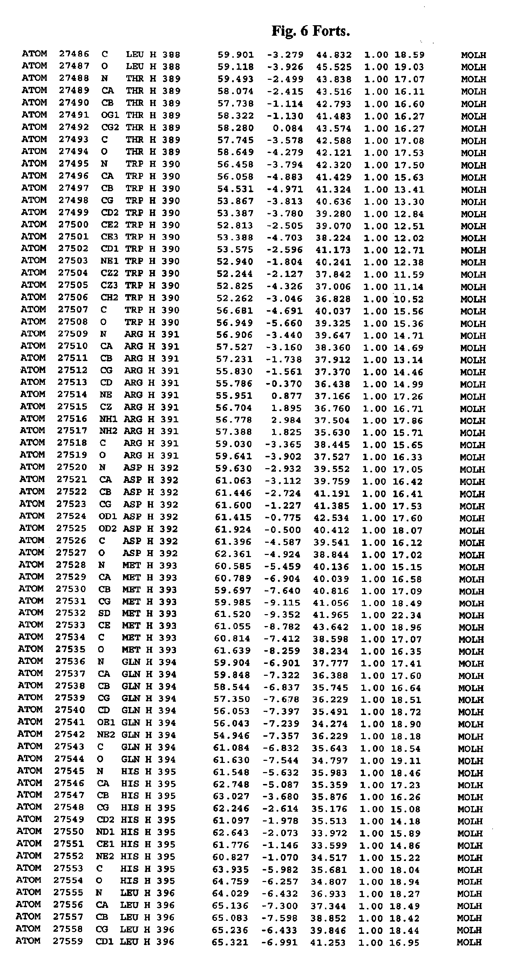

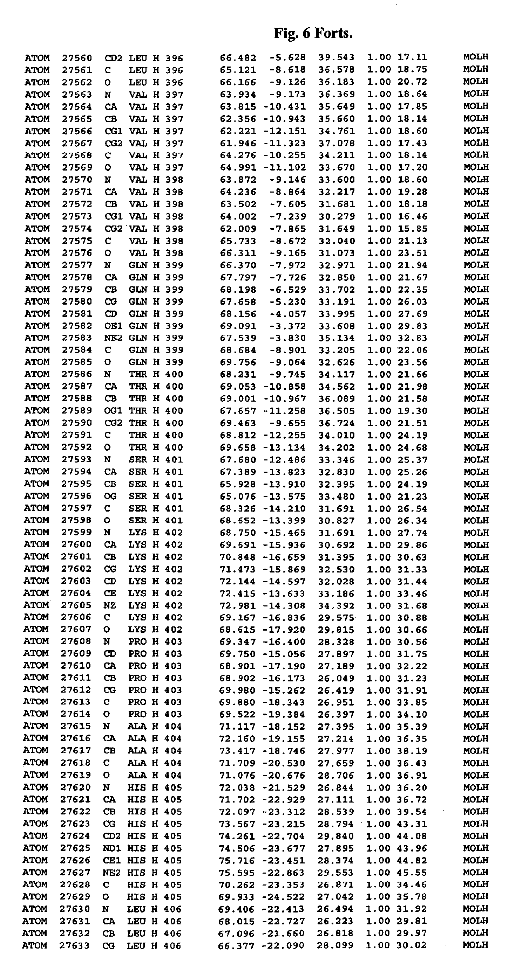

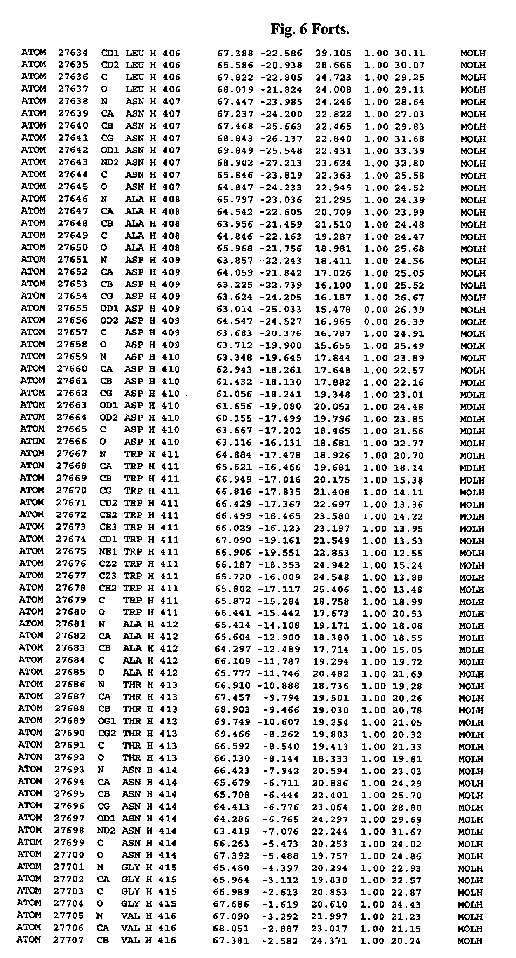

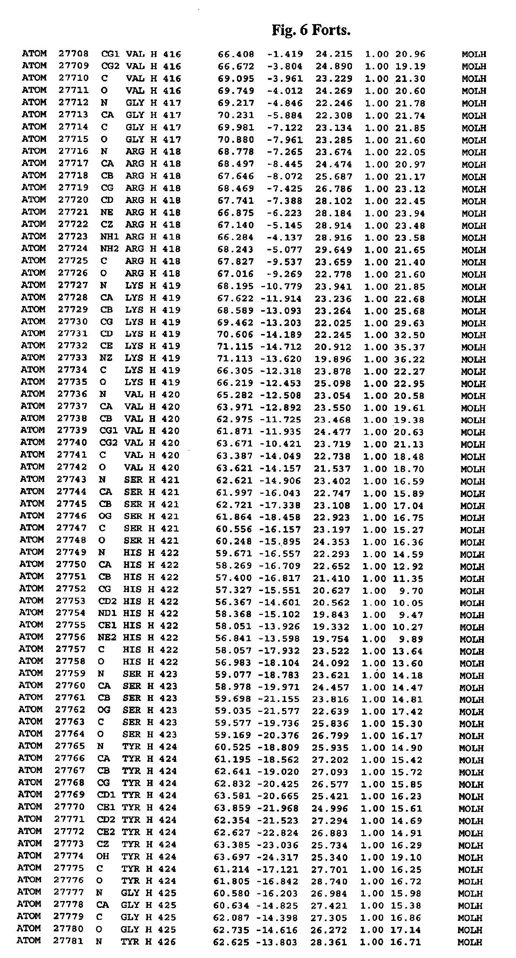

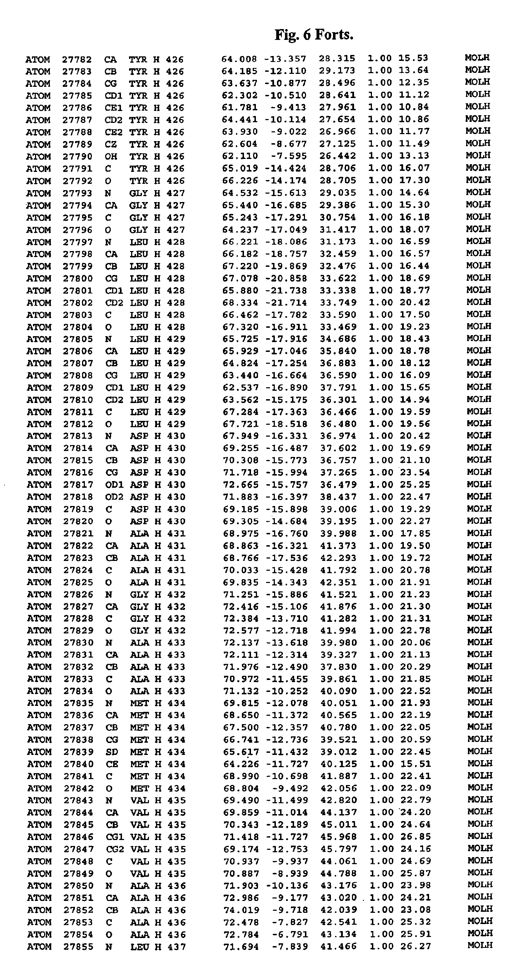

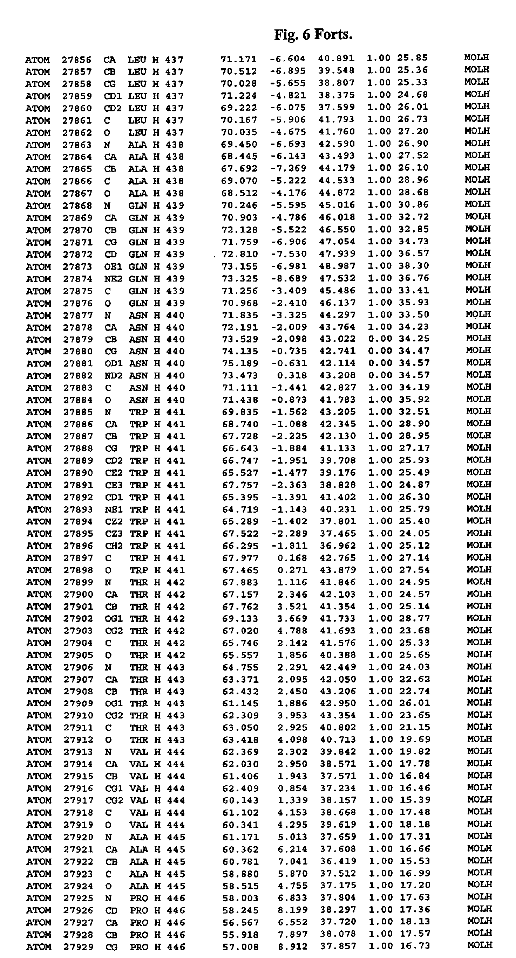

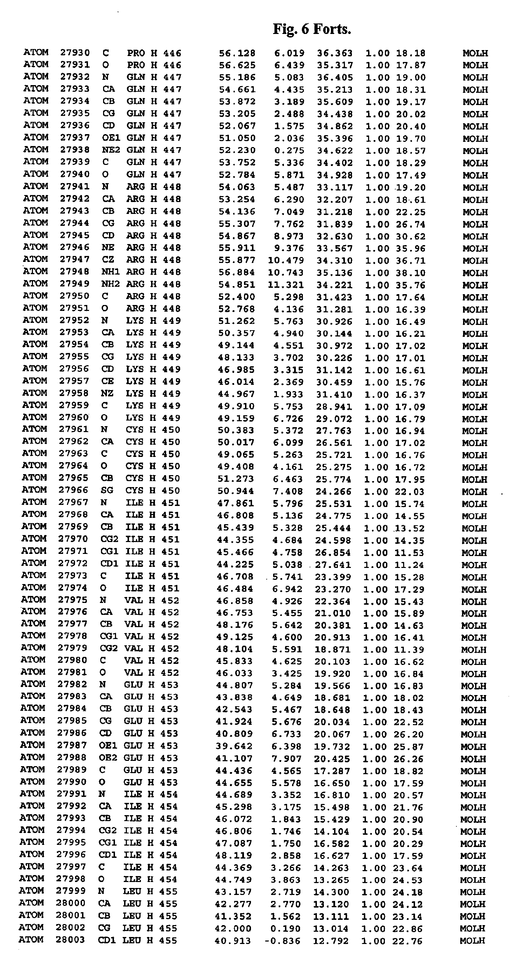

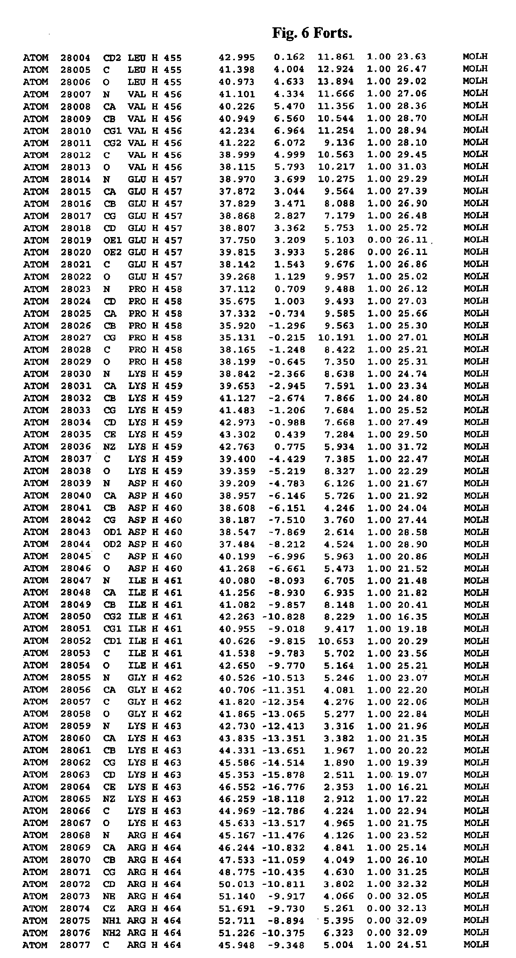

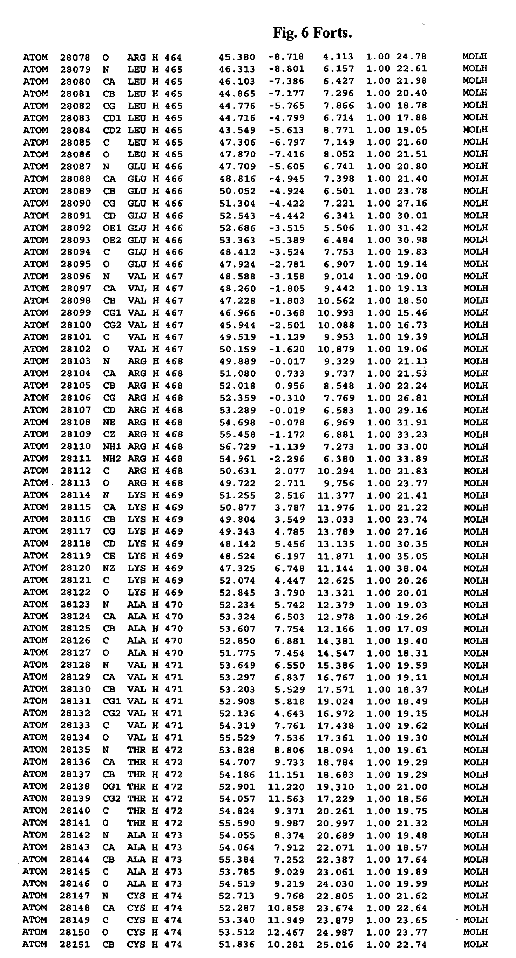

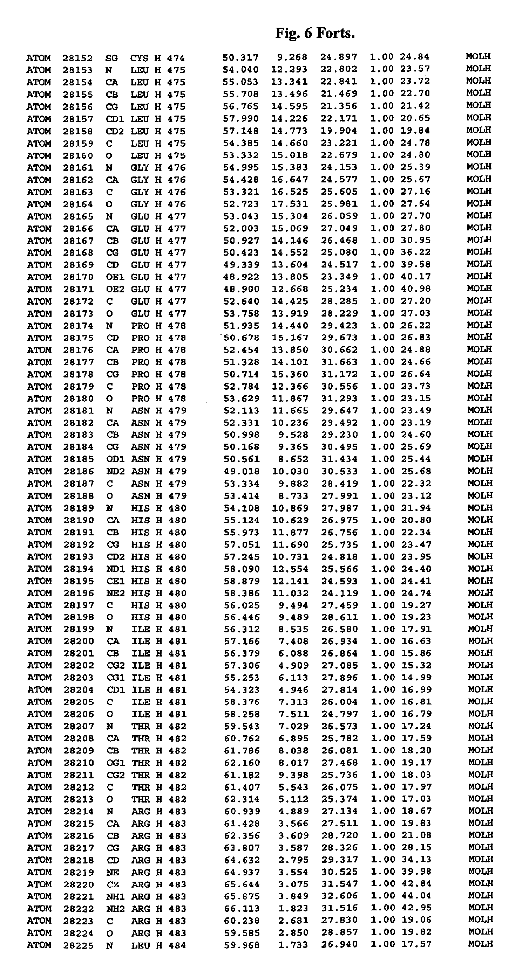

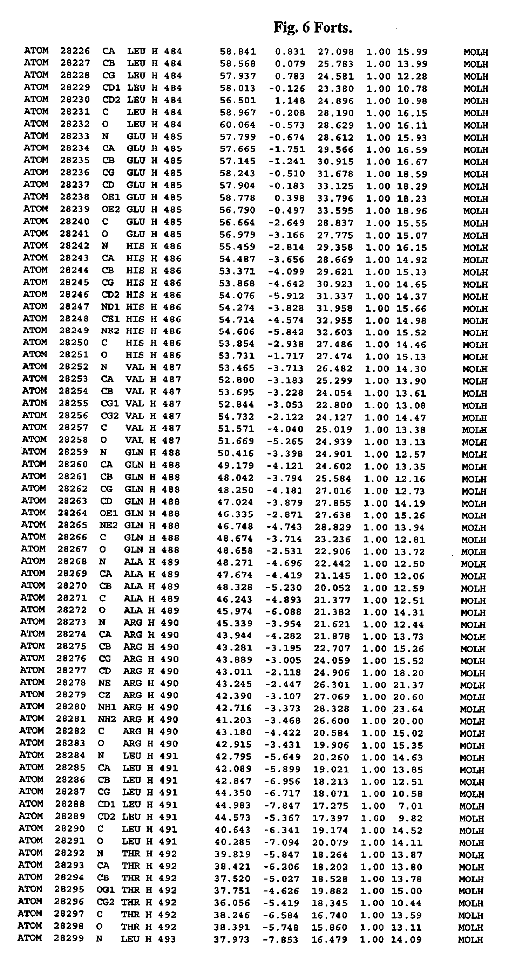

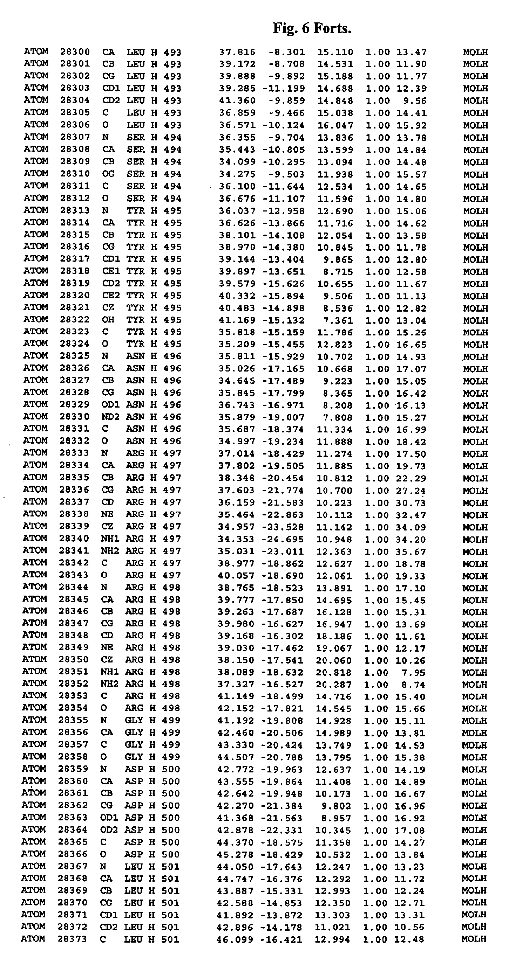

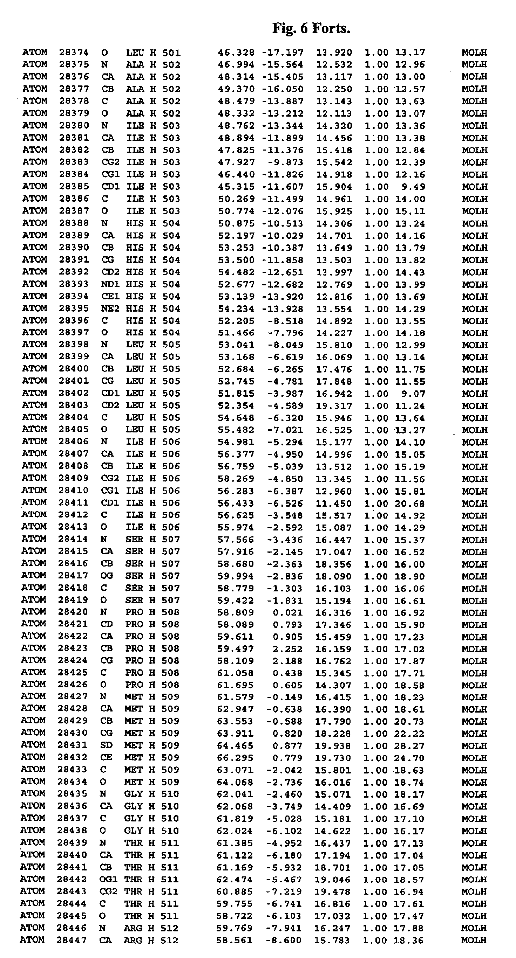

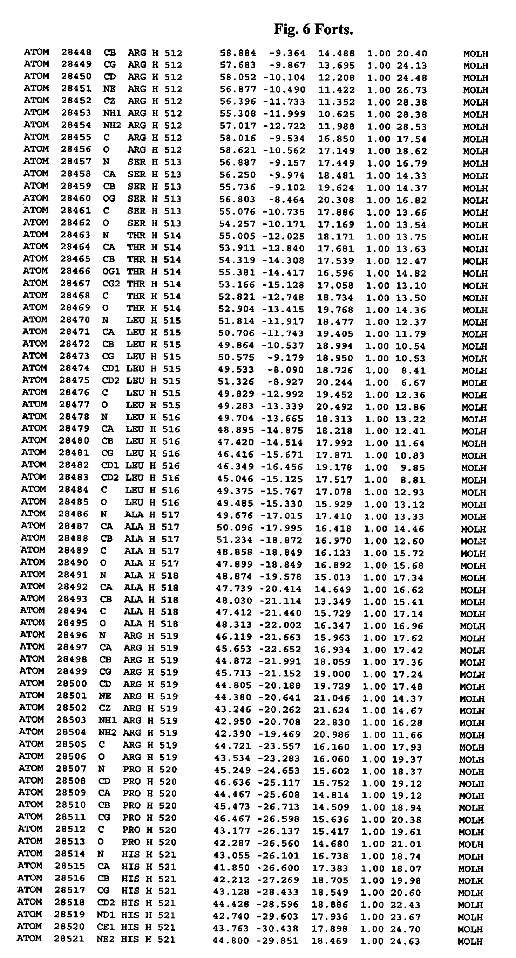

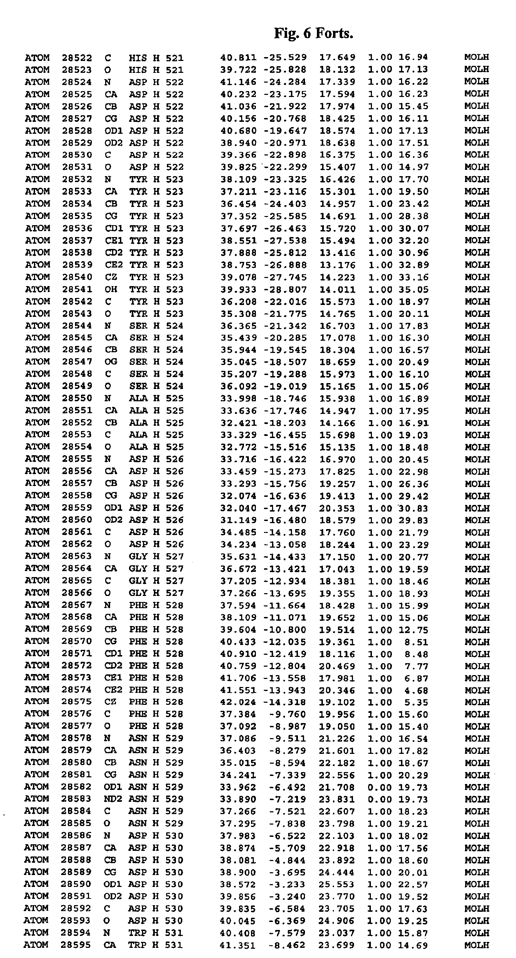

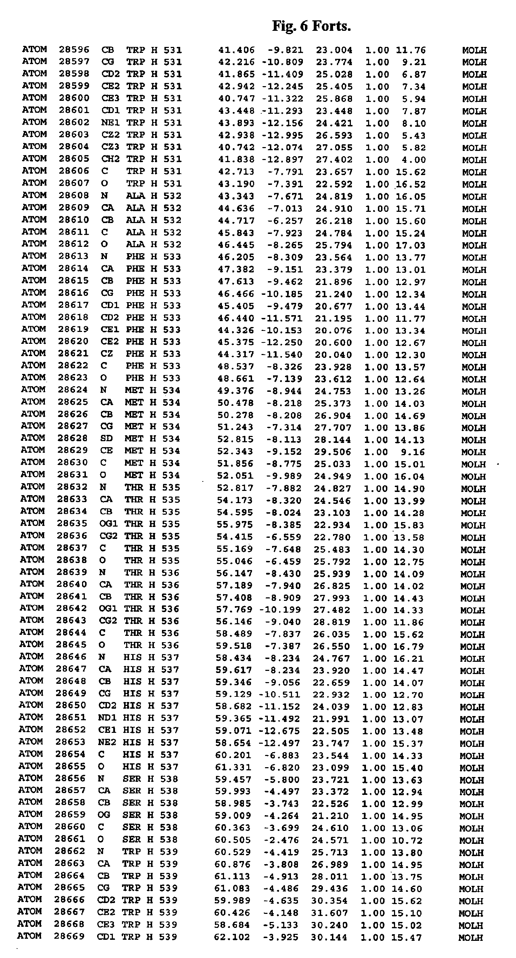

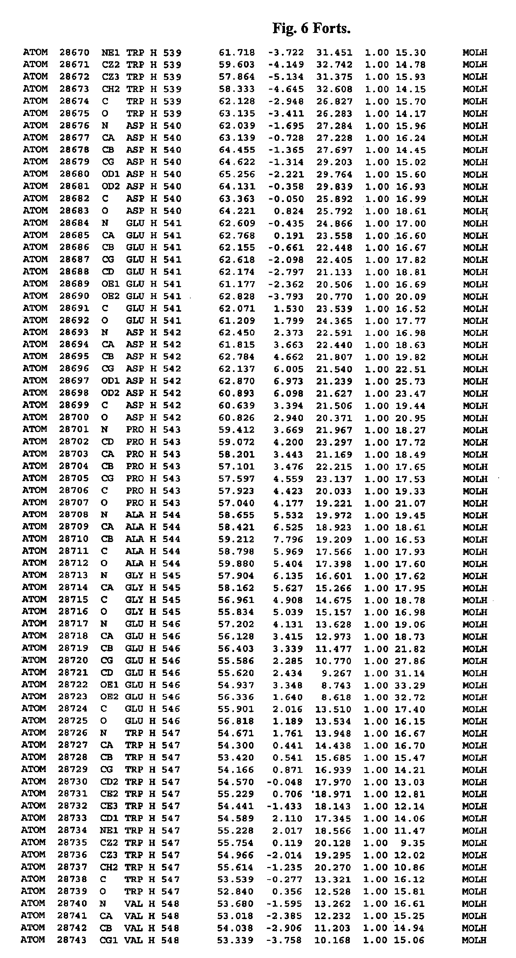

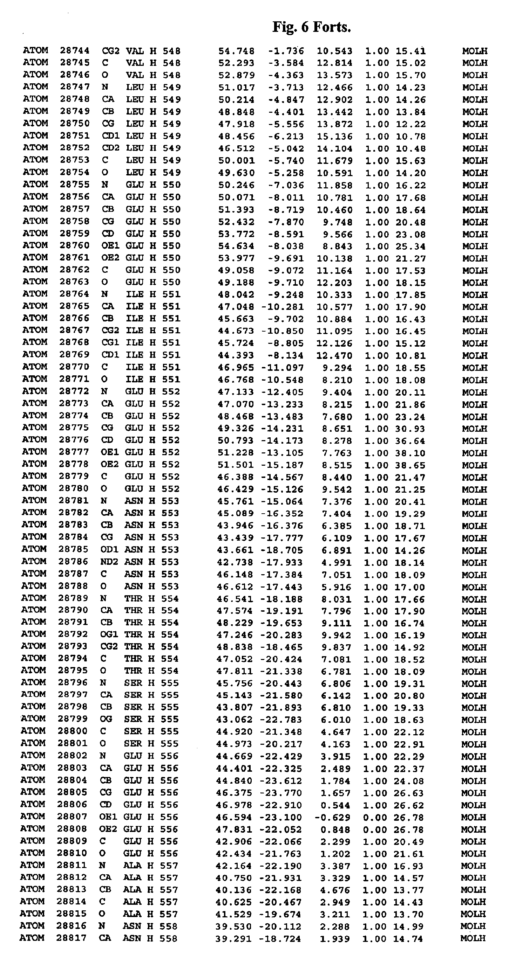

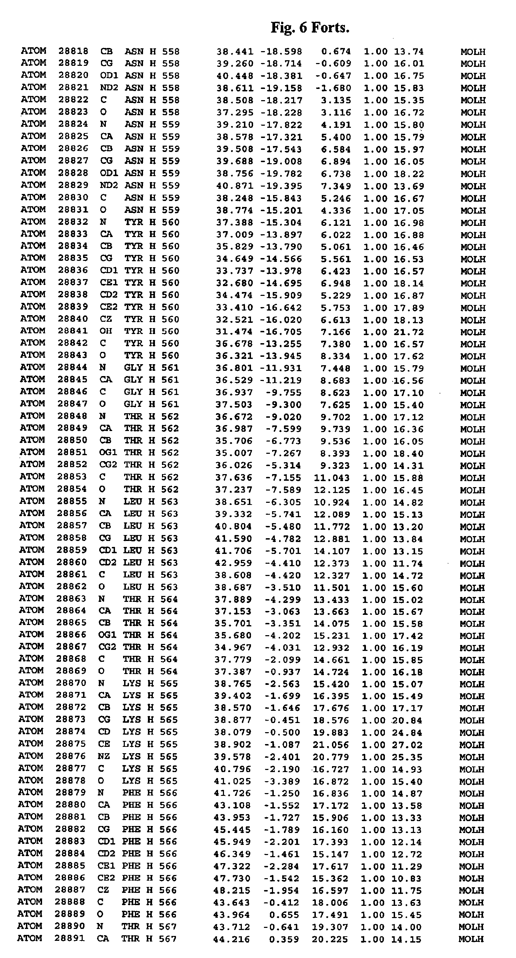

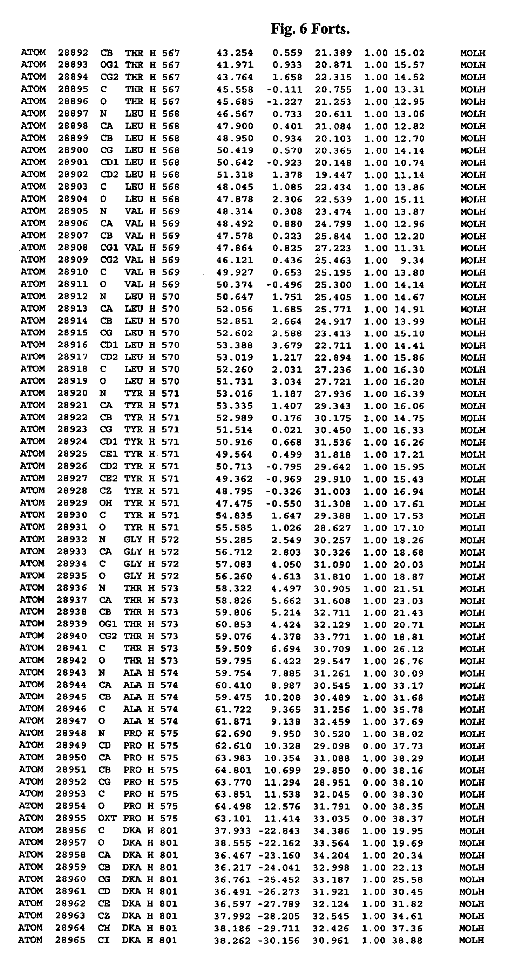

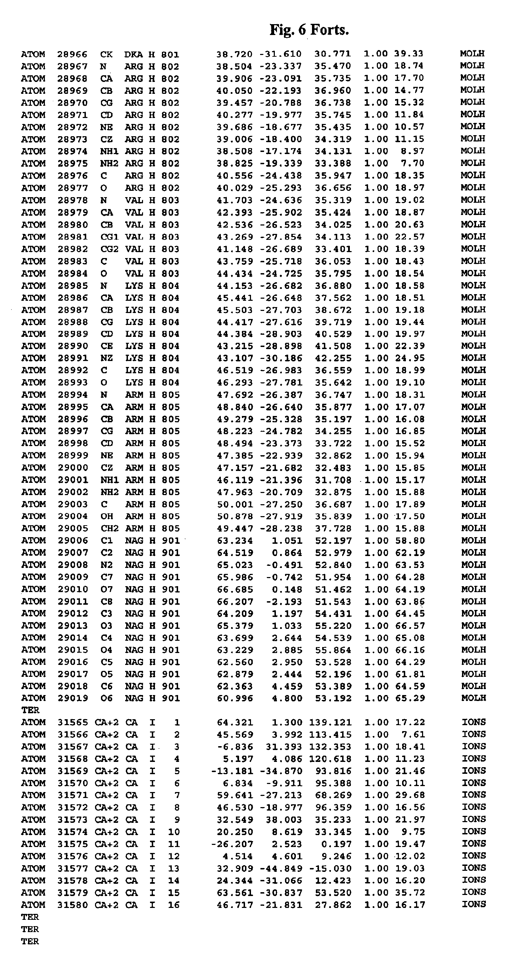

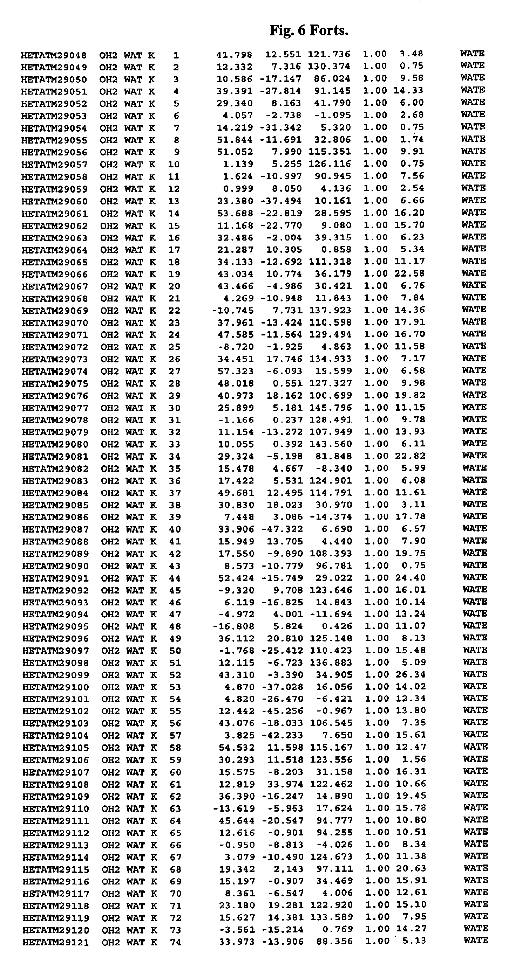

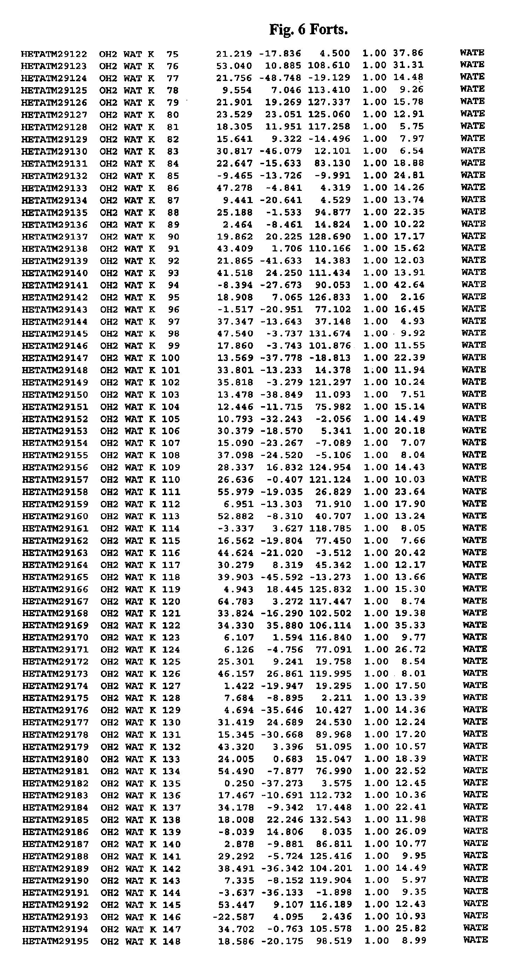

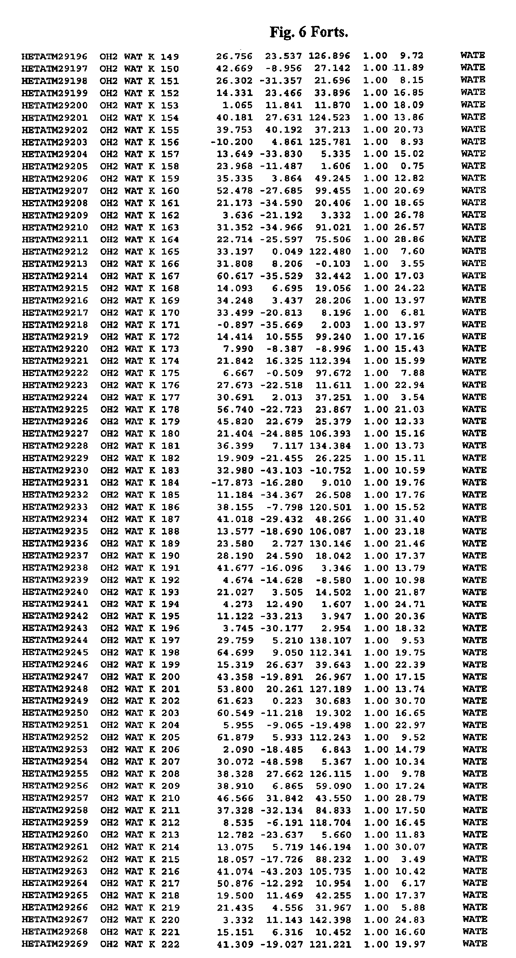

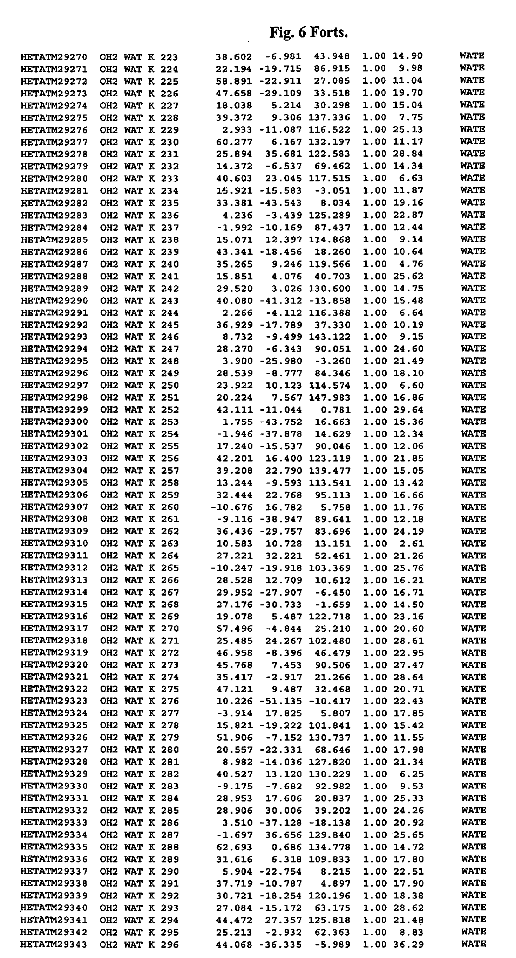

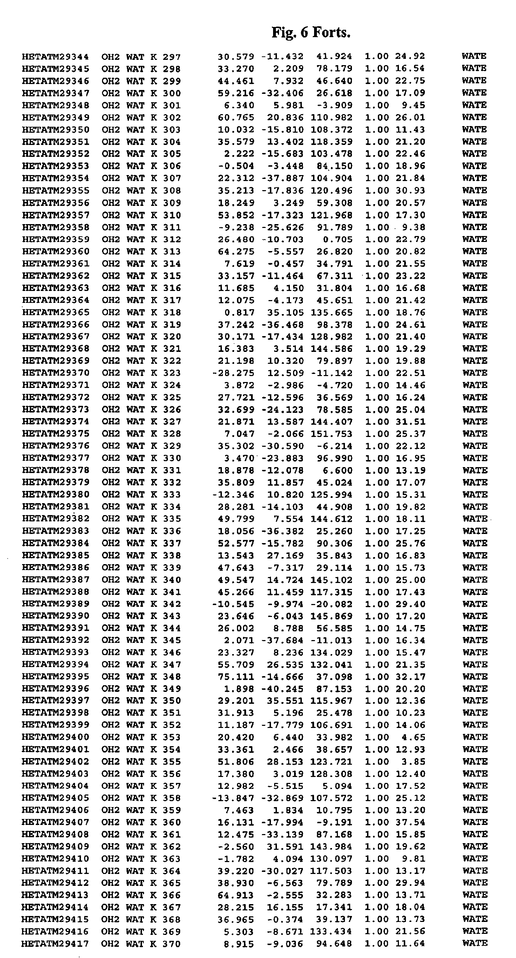

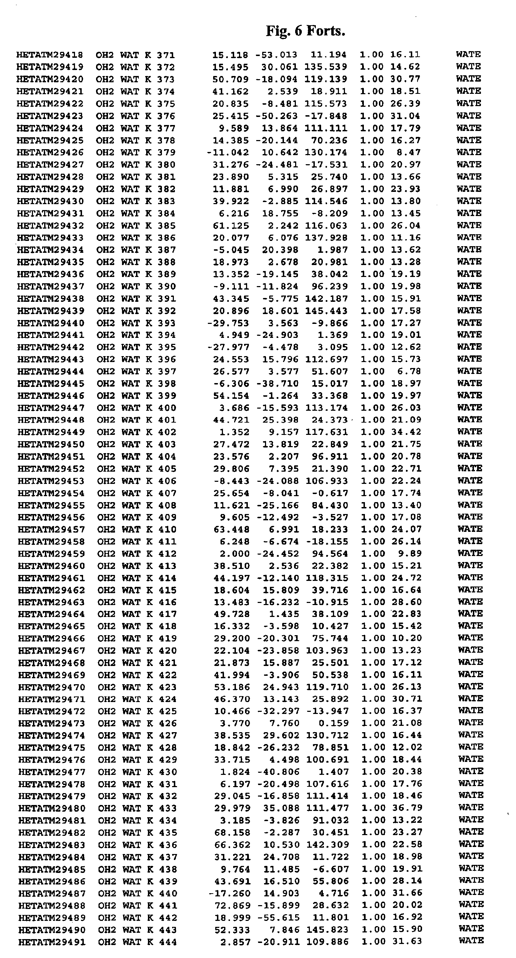

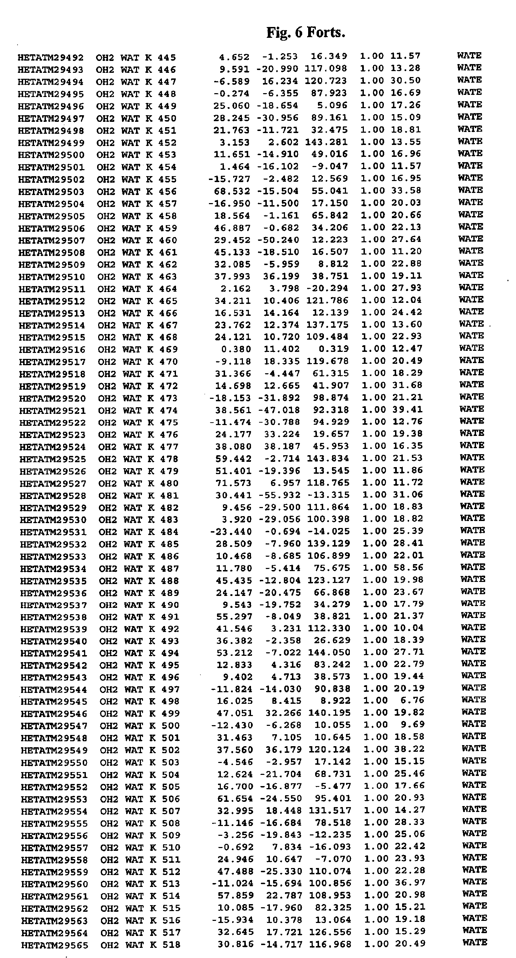

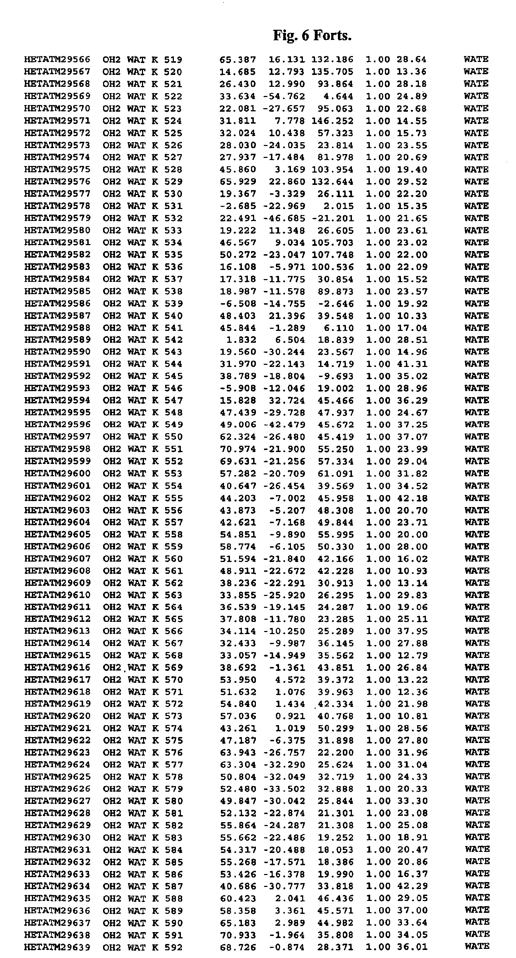

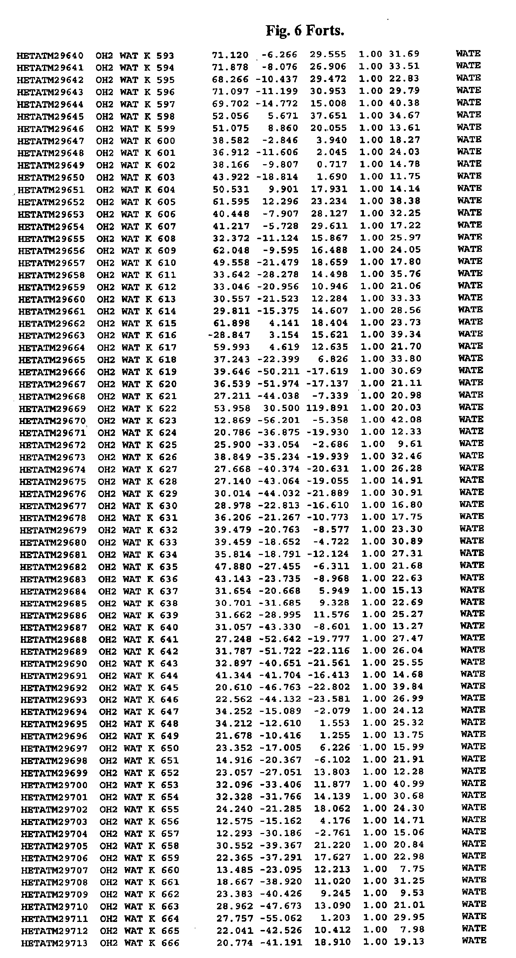

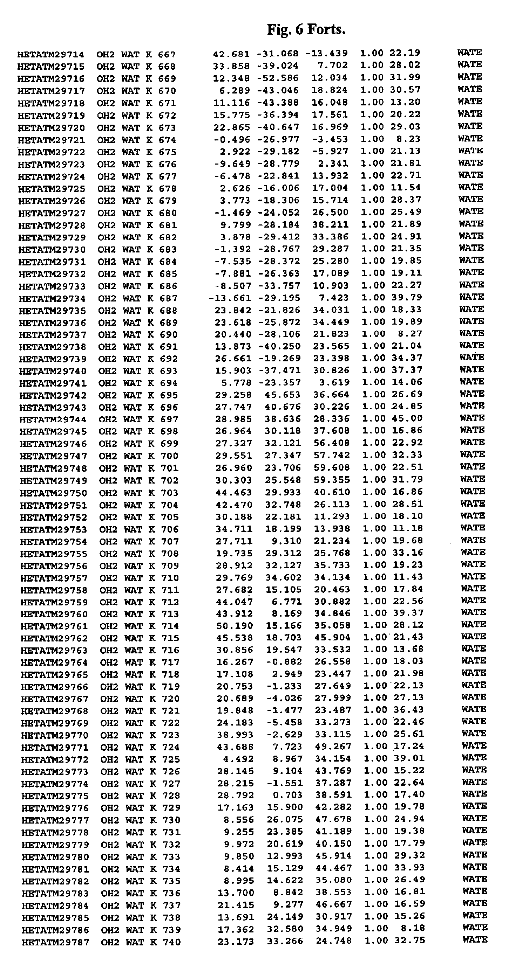

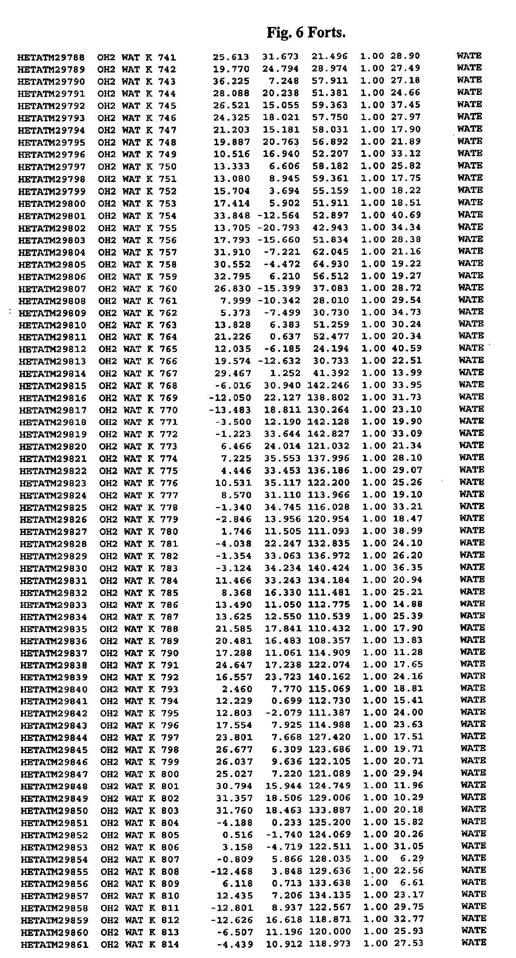

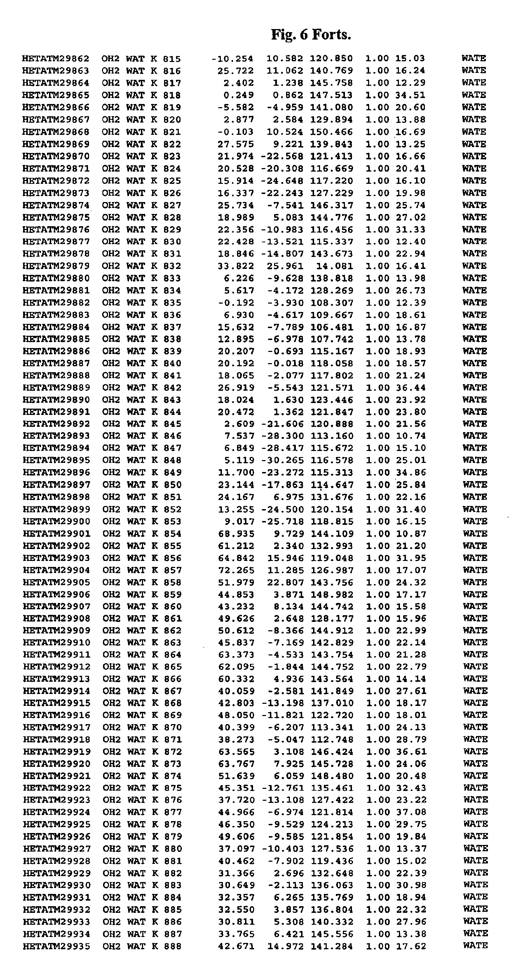

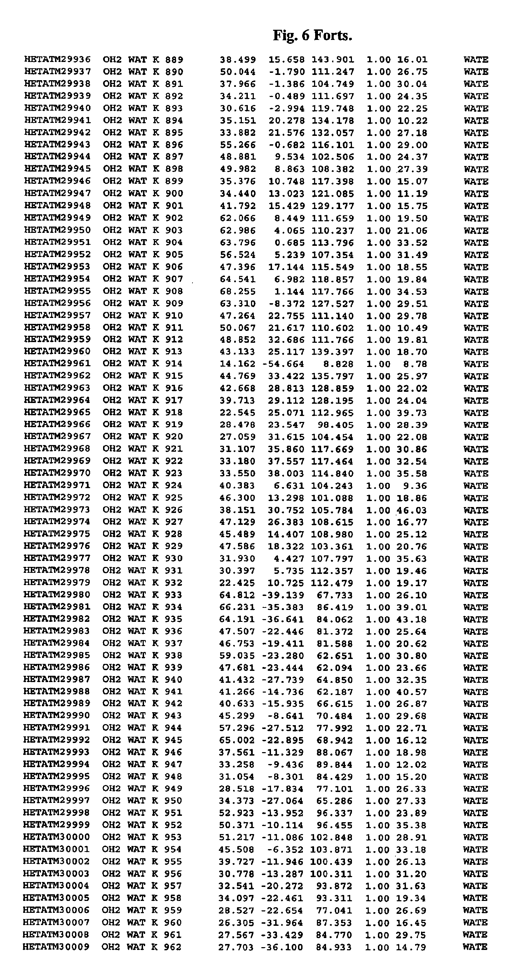

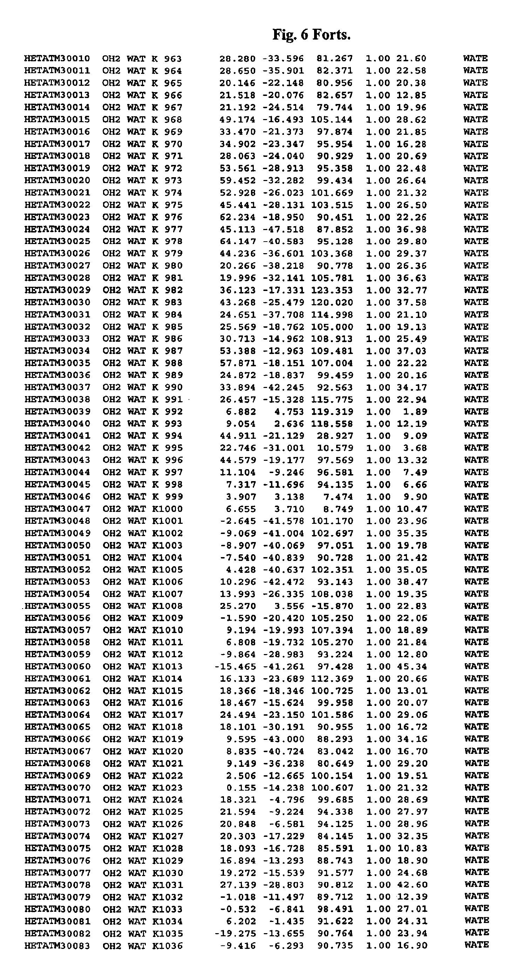

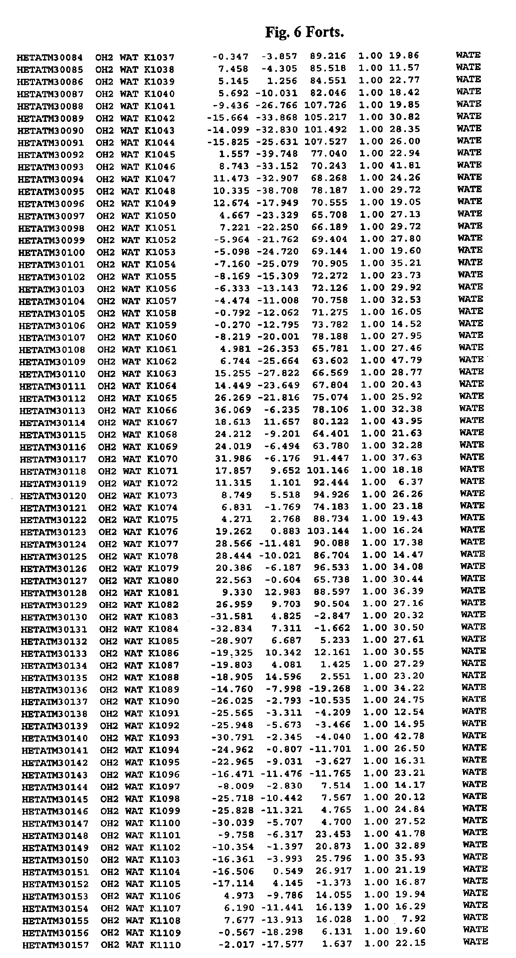

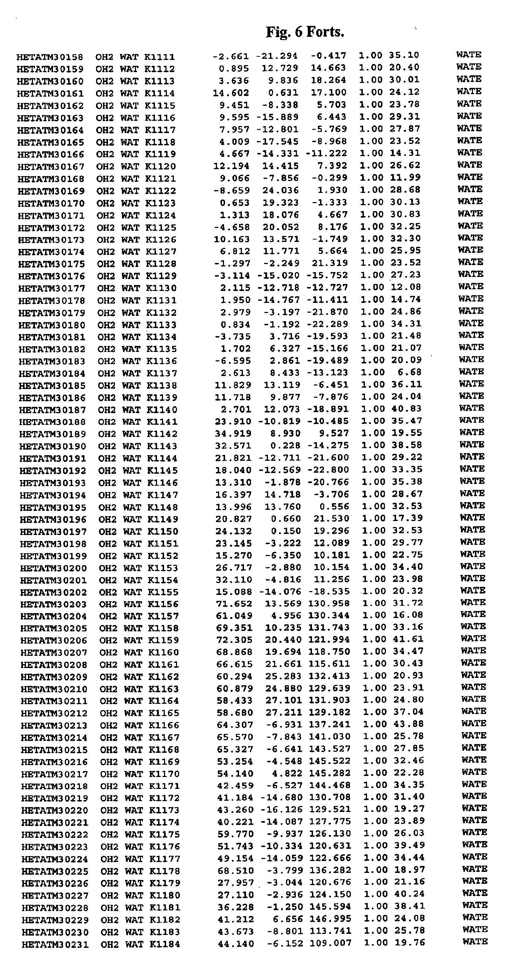

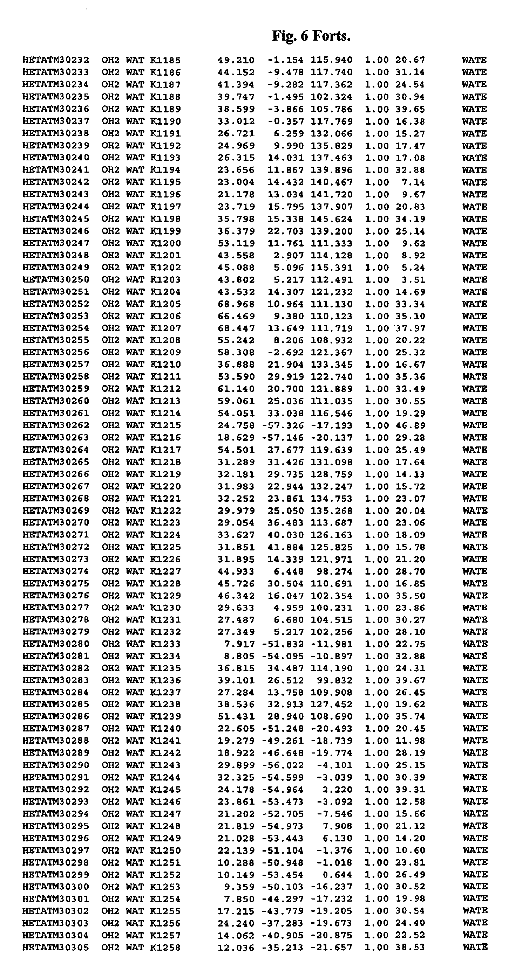

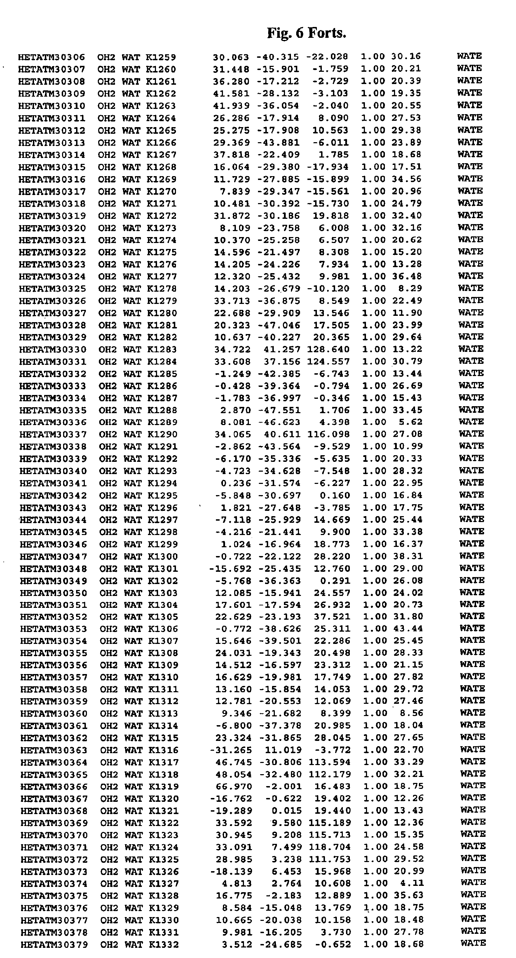

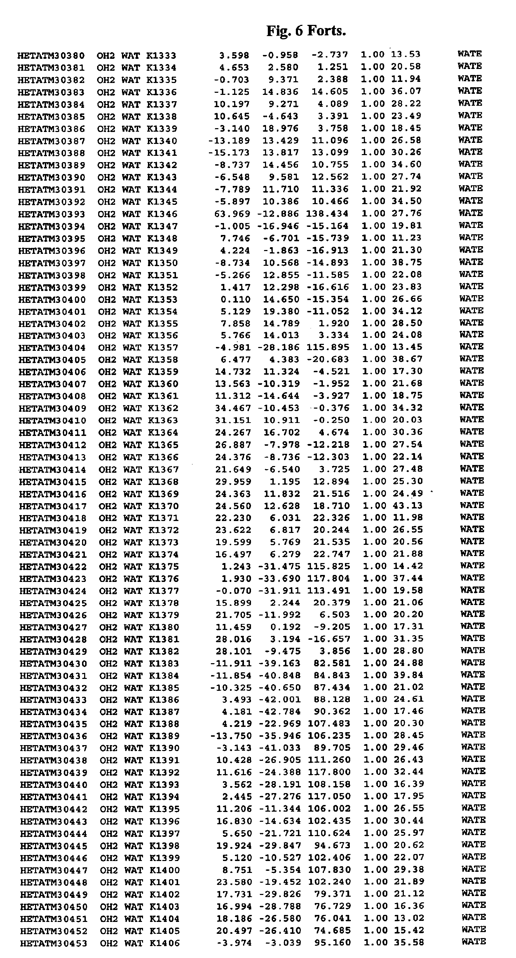

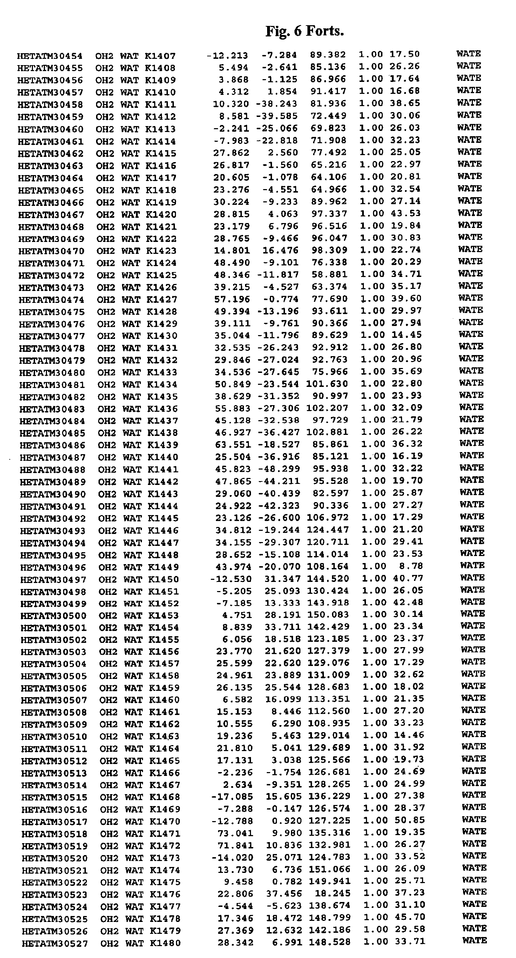

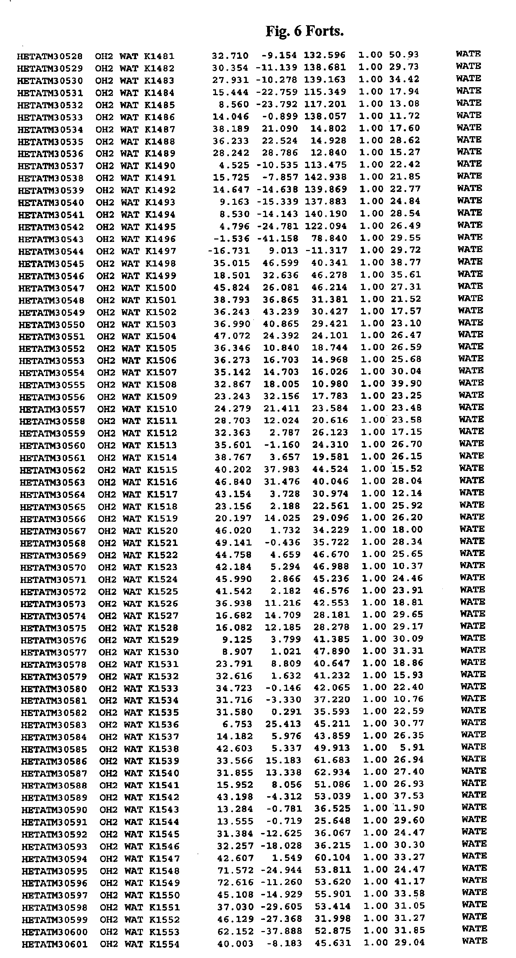

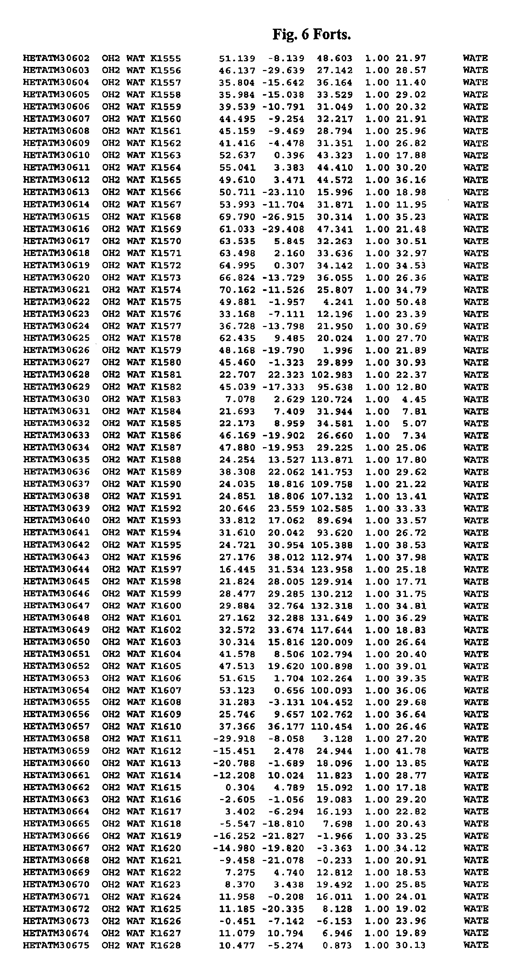

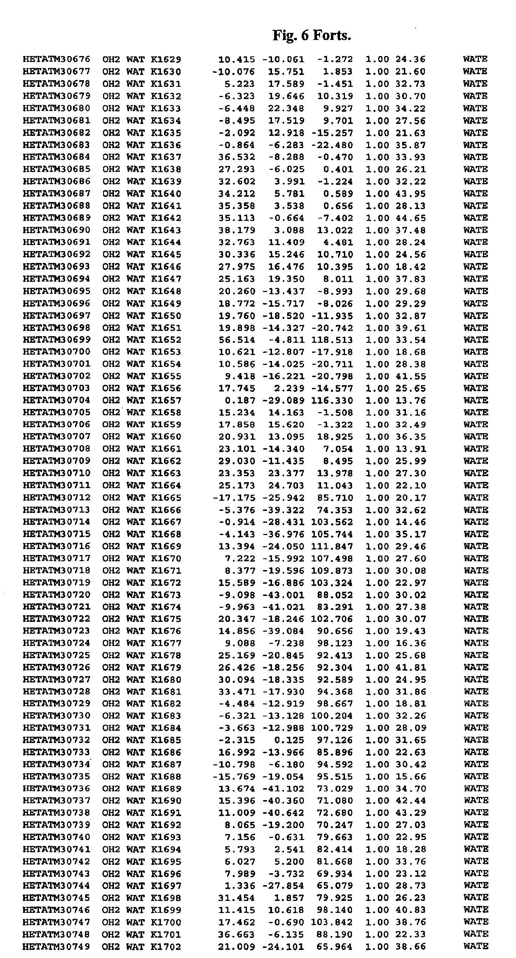

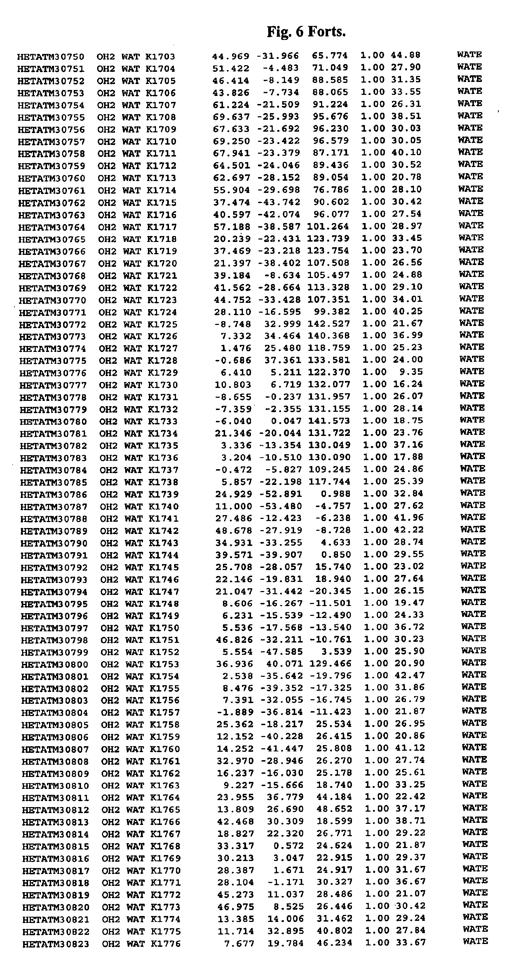

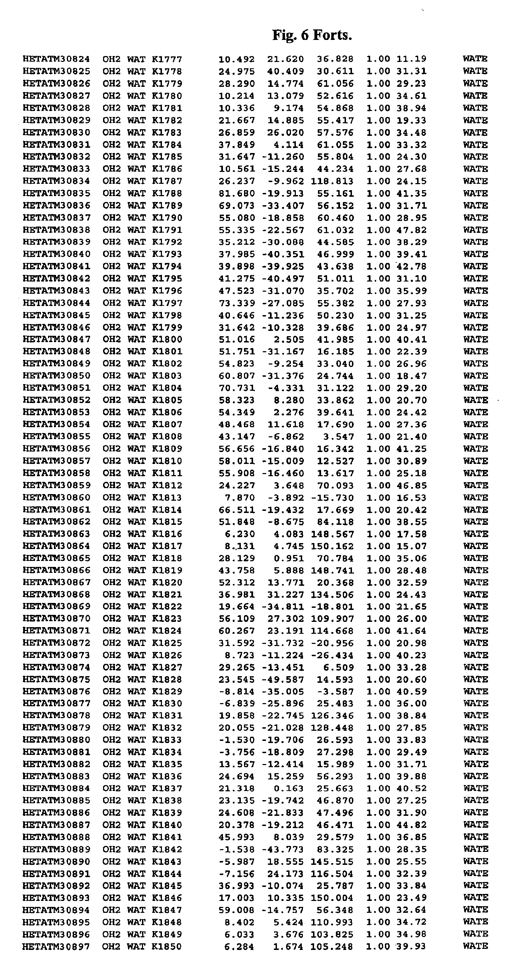

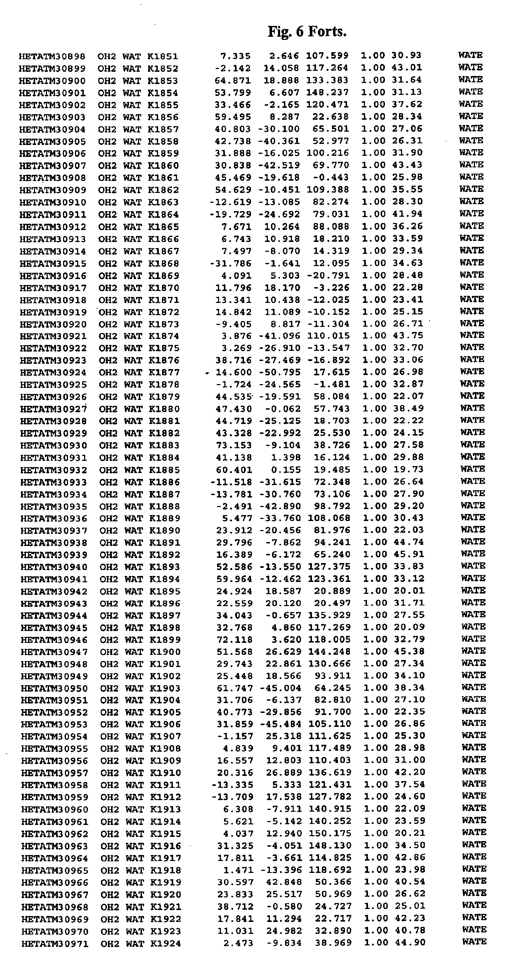

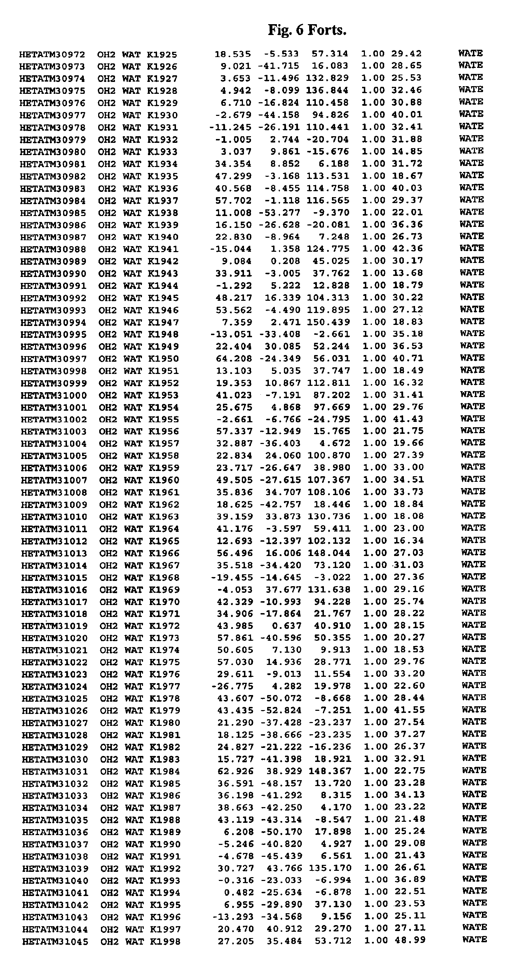

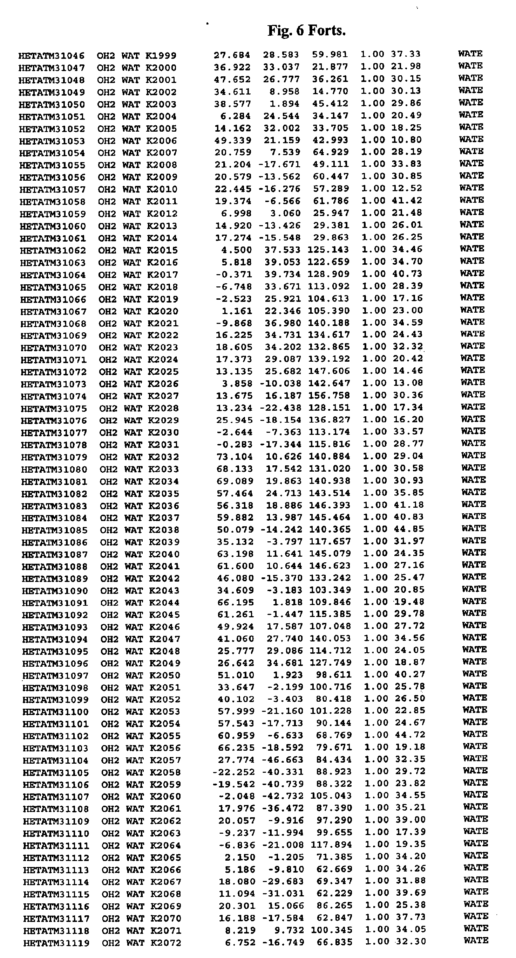

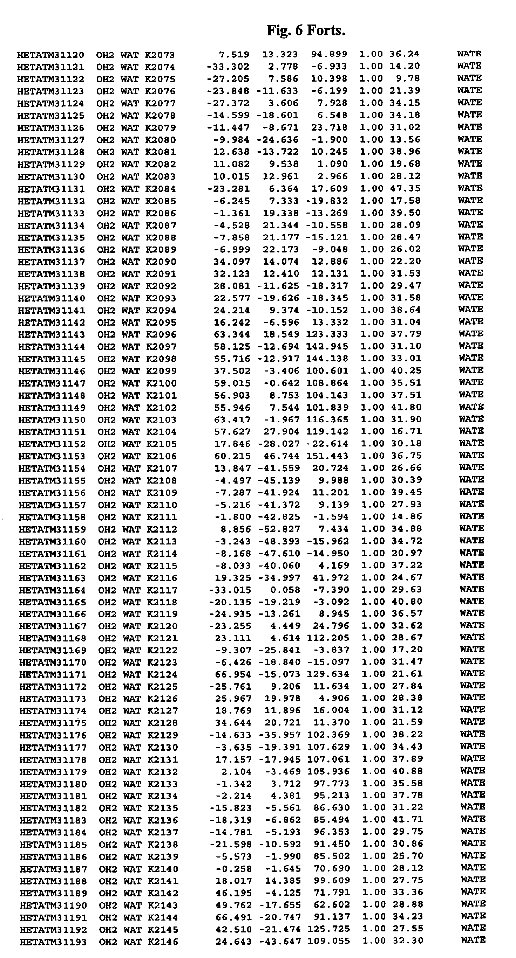

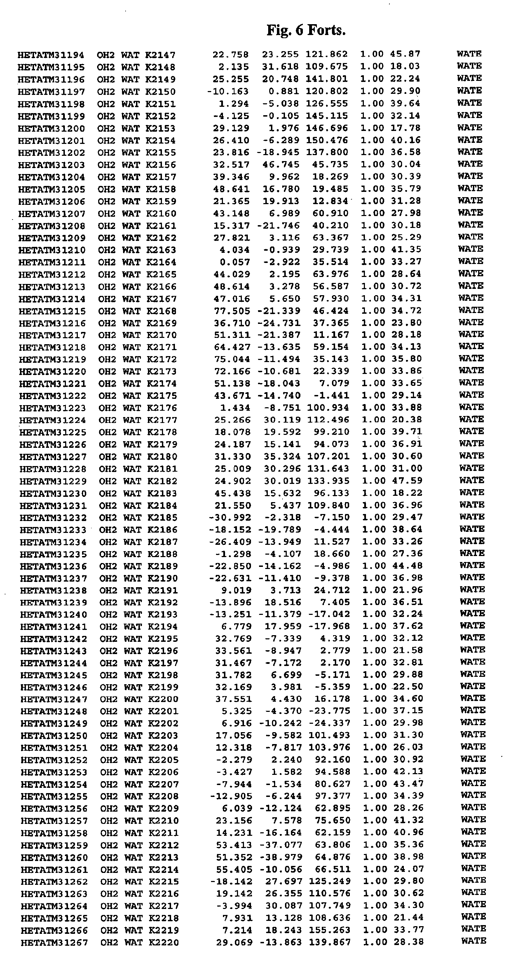

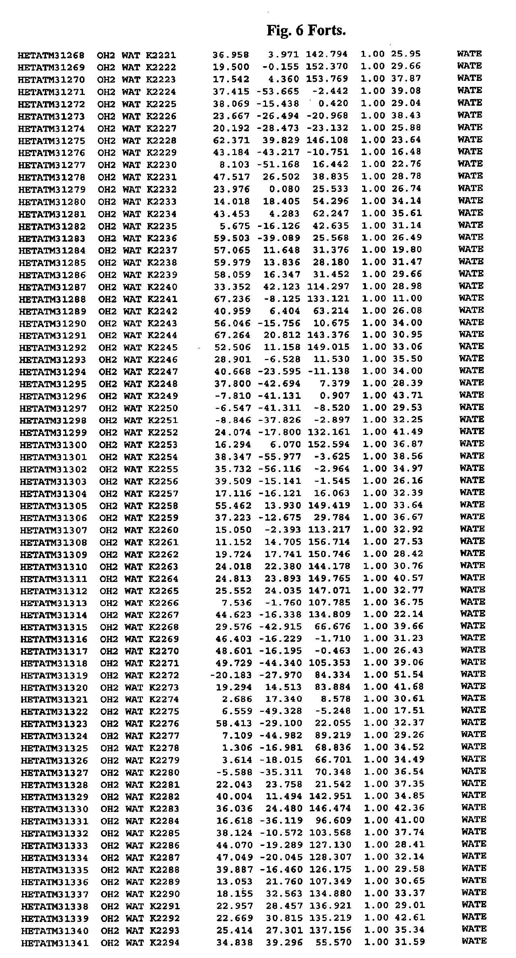

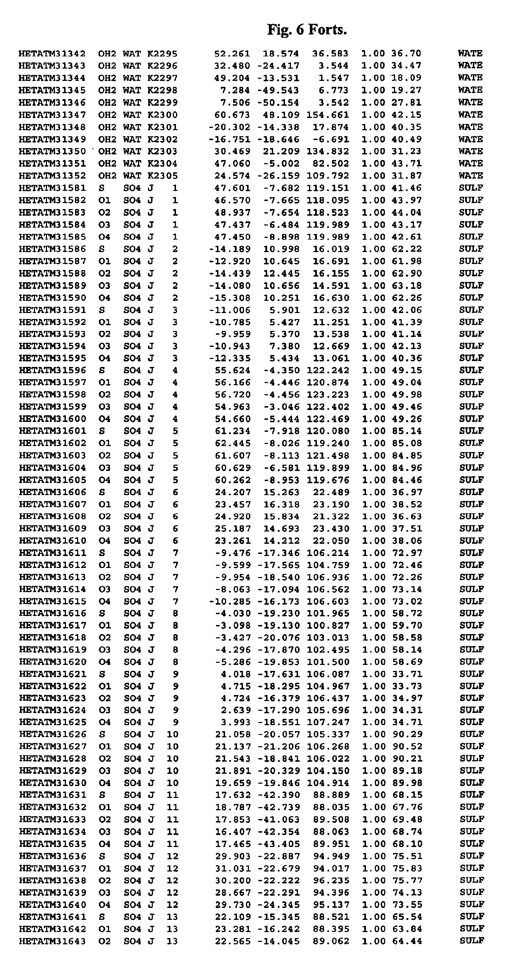

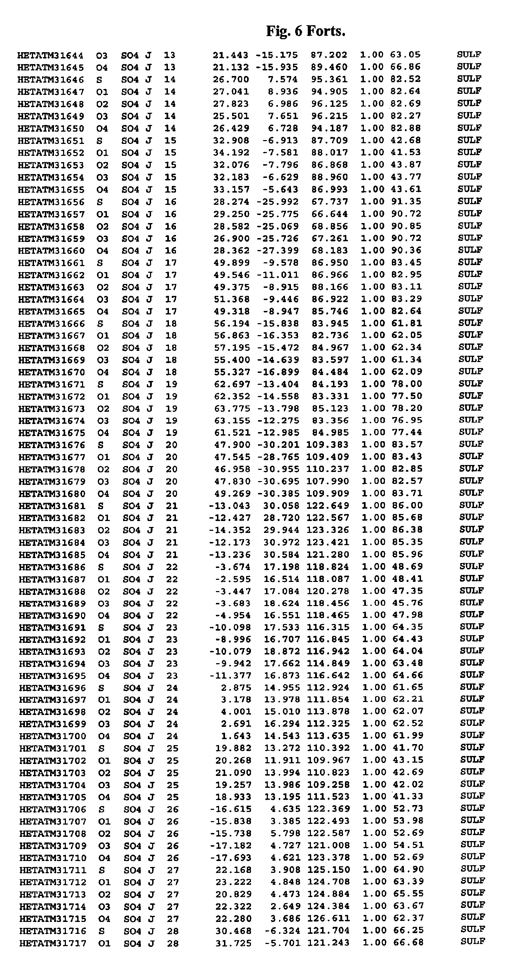

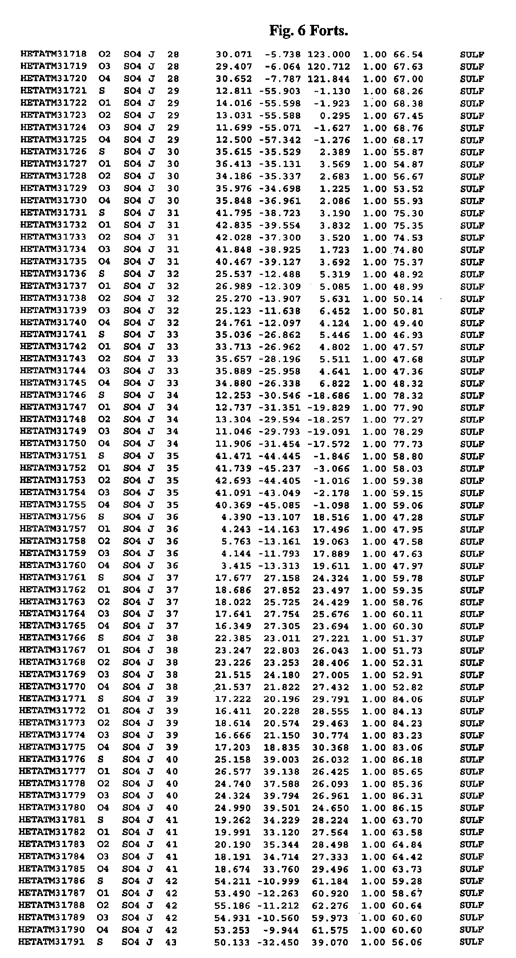

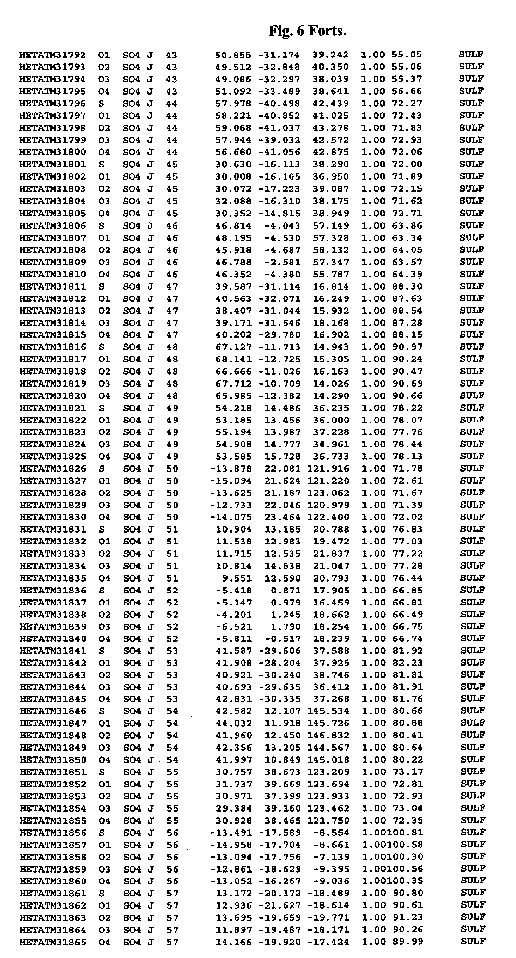

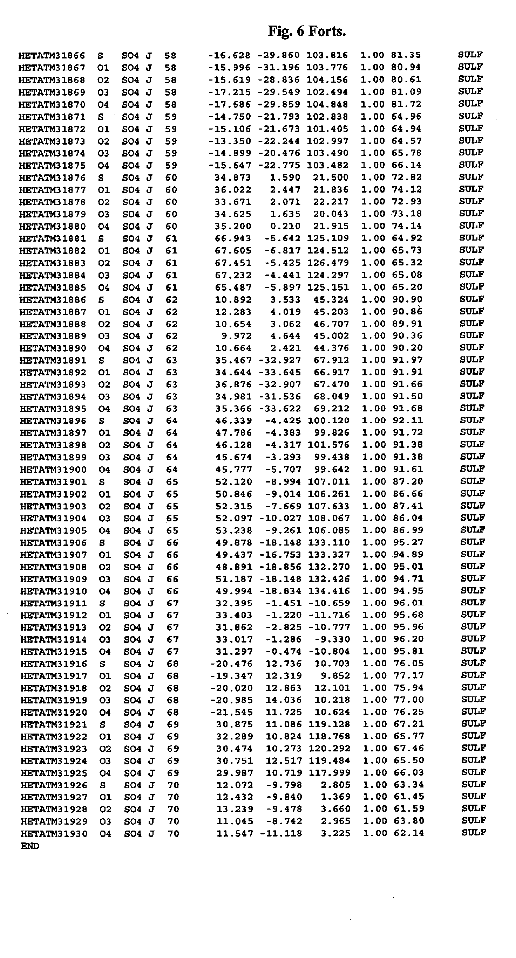

Die vorliegende Erfindung schließt dabei auch Raumformen von Derivaten der genannten Sequenzabschnitte ein. Unter dem Begriff Derivat eines wie vorstehend definierten Sequenzabschnitts von mindestens 50 oder - mehr bevorzugt - mehr AS eines Proteins der Familie der PCs, werden solche Primärsequenzen verstanden, die die Raumform, d.h. also die Tertiärstruktur, wie für den jeweiligen Abschnitt mit den Strukturkoordinaten gemäß Fig. 6 ermittelt, weitgehend aufrechterhält und nur lokale strukturelle Abweichungen erlaubt. Nach Überlagerung der Tertiärstruktur der Primärsequenz des Derivats mit einer der Ausgangsstrukturen, insbesondere gemäß Figur 6, ist eine mittlere Standardabweichung für die Rückgrat-Koordinaten (rmsd), also für die Atome des Protein-Rückgrats der zu vergleichenden Abschnitte, von weniger als 5 Å, insbesondere weniger als 3 und ganz besonders weniger als 2 Å bevorzugt.

Erfindungsgemäß sind bspw. alle Derivate derartiger Sequenzen mit eingeschlossenen, welche eine Identität von mindestens 50%, vorzugsweise 75%, mehr bevorzugt mindestens 80%, stärker bevorzugt 90% und noch stärker bevorzugt mindestens 95% mit den genannten Aminosäuresequenzen, insbesondere den Positionen 108 oder 110 bis 573, vorzugsweise den Positionen 108 oder 110 bis 445 oder den Positionen 446 bis 573 der in Fig. 1 (obere Sequenz) gezeigten Aminosäuresequenz aufweisen.

Insbesondere werden in diesem Zusammenhang solche Aminosäuresequenzen als Derivate bezeichnet, die nur konservative Substitutionen, d.h., den Austausch von bspw. polaren gegen polare Aminosäuren oder von hydrophoben gegen hydrophobe Aminosäuren (z.B. Leucin gegen Isoleucin oder Valin oder umgekehrt oder Serin gegen Threonin oder umgekehrt) haben, verstanden. Als Abschnitt oder Fragment eines Proteins aus der Familie der PCs werden solche Sequenzen verstanden, die im Unterschied zur physiologischen Aminosäuresequenz insbesondere am N- oder C-Terminus Deletionen aufweisen, ggf. aber auch intrasequentielle Deletionen in den vorgenannten und bspw. in der Figur 1 wiedergegebenen Sequenzen. Hierbei wird es sich typischerweise um mindestens eine Deletion handeln, wobei in jeder Deletion vorzugsweise weniger als zehn, insbesondere weniger als fünf, Aminosäuren, ganz besonders bevorzugt ein oder zwei Aminosäuren gegenüber der nativen Sequenz deletiert sein können.

In einer bevorzugten Ausführungsform der vorliegenden Erfindung wird eine Raumform eines Polypeptids beansprucht, das mindestens einen 50 AS, vorzugsweise einen 80 AS und stärker bevorzugt einen 100 und ganz besonders bevorzugt einen 120 AS umfassenden Abschnitt einer Sequenz eines Proteins aus der Familie der PCs, insbesondere von Furin, vorzugsweise eukaryotischen Ursprungs, stärker bevorzugt aus dem Säugetier, insbesondere human, enthält. Gegebenenfalls kann es sich auch um Derivate solcher Abschnitte von Proteinen, insbesondere auch der Volllängenproteine, handeln.

Weiterhin werden solche Raumformen beansprucht, die sowohl ein Polypeptid, enthaltend mindestens einen 50 AS, vorzugsweise einen 80 AS, mehr bevorzugt einen 100, stärker bevorzugt einen 120 und ganz besonders bevorzugt einen 150 AS umfassenden Abschnitt einer Sequenz eines Proteins aus der Familie der PCs, insbesondere von Furin, als auch mindestens eine weitere Verbindung aufweisen. Hierbei wird es sich typischerweise um Verbindungen handeln, die als Liganden an das Polypeptid binden können, so daß die Raumform einen Komplex aus Polypeptid und mindestens einer weiteren auf kovalente oder nicht-kovalente Art gebundenen Verbindung darstellt. Hierbei kann es sich bei der Verbindung bzw. den Liganden um ein physiologisch auftretendes Molekül oder auch um ein nicht-physiologisch auftretendes Molekül handeln. Gegebenenfalls können, sofern mehr als ein Ligand in der Raumform auftritt, auch Kombinationen von physiologisch bzw. nicht-physiologisch auftretenden Molekülen in der Raumform enthalten sein. Bevorzugt sind dabei solche Liganden, die insbesondere unter physiologischen Bedingungen mit dem Polypeptid in Wechselwirkung treten und vorzugsweise hochaffines Bindungsverhalten zeigen (vorzugsweise K_{d}<30 µM).

Vorzugsweise wird erfindungsgemäß eine Raumform eines Polypeptids in Kombination mit Abschnitten von physiologischen Liganden offenbart. Dabei handelt es sich beispielsweise um die für die Bindung an Proteine aus der Familie der PCs relevanten Sequenzabschnitte bzw. Domänen oder Proteine, die nativ von PCs endoproteolytisch gespalten werden. Typischerweise wird der Ligand, vorzugsweise ein physiologischer Ligand, an die in einem erfindungsgemäßen Raumform-Polypeptid enthaltene Sequenz eines Proteins aus der Familie der PCs binden. Der Ligand kann selbst ein Polypeptid, ein Oligopeptid, ein Dipeptid oder ein synthetisch modifiziertes Derivat eines Poly-, Oligo- oder Dipeptids, insbesondere von physiologisch die Bindung an Proteine aus der Familie der PCs verursachenden Abschnitten nativer Substratsequenzen von Proteinen aus der Familie der PCs, aber auch ein Peptidomimetikum oder ein organisch-chemisches Molekül mit einem Molekulargewicht von typischerweise <5000 sein.

Insbesondere bevorzugt sind solche Oligo- oder Polypeptidsequenzen als Liganden, deren endoproteolytisch zu schneidende Amidbindung derart modifiziert ist, daß die Spaltung nicht stattfinden kann. Hier kommen alle einschlägigen Modifikationen von Amidbindungen in Betracht, die im Ergebnis nicht der Proteolyse unterliegen.

Ganz besonders bevorzugt sind Raumformen von Polypeptiden mit einem oder mehr Ligand/en, wobei der Ligand einen Abschnitt der als Zielsubstrate von PCs bekannten Proteine enthält oder ggf. aus diesem Abschnitt besteht. Zu nennen wären hier Proteine oder Vorläuferproteinen aus Klassen der Wachstumsfaktoren und Hormone wie Pro-β-NGF, Pro-BDNF, Pro-NT-3, Pro-TGF-β1,Pro-Müller-Inhibitor-Substanz, Pro-IGF-1, Pro-Endothelin-1, Parathormon-verwandtes Propeptid und Pro-Parathormon, Rezeptoren wie Insulin-Prorezeptor, Hepatocyten-Wachstumsfaktor-Prorezeptor, Pro-LRP, Integrin-α3-Kette, Integrin-α6-Kette, Plasmaproteine wie Proalbumin, Komplement-pro-C3, Profaktor IX, Profaktor X, Pro-von-Willebrand-Faktor, Proprotein C, Matrixmetalloproteasen (MMPs) wie Stromelysin 3 und MT-MMPI, viralen Hüllglycoproteine wie HIV gp160, (z.B. humanem) CMV Glycoprotein B, MMTV-7 Superantigen, Hühnerpest-Virus A Hämagglutinin, Masern-Virus F₀, NDV F₀, Sindbis-Virus gpE2, (z.B. humanem) Parainfluenza-Typ-3-Virus F₀, bakteriellen Toxine, insbesondere Exotoxine wie Anthrax-Toxin-protektives Antigen, Diphtherie-Toxin, *Pseudomonas*-Endotoxin A, Shiga-Toxin, und anderen wie (Pro-)Furin, Proendopeptidase 3.4.24.18 und Pro-7B2. Es sind sind dabei Zielmoleküle aus Vertebraten wie Vögeln und Säugern, vor allem Nagern, wie Maus, Ratte oder Meerschweinchen, Schwein und Mensch bzw. Zielmoleküle aus Erregern, welche die genannten Spezies infizieren können, bevorzugt. Der Ligand wird dann, wenn er nicht die gesamte native Sequenz aufweist, typischerweise die 5 bis 50, vorzugsweise 5 bis 25, ganz besonders bevorzugt 5 bis 12, der die zu spaltende Amidbindung benachbarten Aminosäurereste eines solchen Zielsubstrats umfassen. Ganz besonders bevorzugt sind Raumformen, die insbesondere an die katalytische Domäne, vorzugsweise an das aktive Zentrum gebundene Liganden aufweisen, wobei die Liganden vorzugsweise eine Bindungsaffinität von K_{d}<50 µM haben und typischerweise dadurch zumindest in vitro, vorzugsweise auch in vivo, die physiologische Funktion, insbesondere die Endoprotease-Funktion, von Proteinen aus der Familie der PCs blockieren. Dabei wird sich der inhibitorische Charakter des Liganden typischerweise dadurch funktionell ergeben, daß die Bindungsstelle(n) der katalytischen Domäne(n) von Proteinen aus der Familie von PCs für Substratsequenzen mit dem Liganden besetzt sind, so daß das PC-Protein seine physiologische Funktion vor allem in Hinblick auf seine physiologische proteolytische Funktion verliert.

Erfindungsgemäße Beispiele solcher inhibitorischer Liganden sind acylierte Arg-Xaa-Lys/Arg-Arg-Chlormethane (z.B. Chlormethylketone (cmk), insbesondere Decanoyl-Arg-Xaa-Lys/Arg-Arg-Chlormethylketone, z.B. der in den Beispielen gezeigte Ligand; Xaa = irgendeine natürlich vorkommende AS), α₁-PIT (vgl. Brennan et aL (1988) FEBS Lett. 229: 167-170), α₁-PDX (vgl. Anderson et aL (1993) J. Biol. Chem 268: 24887-24891), Varianten der dritten Domäne von Ovomucoid, bei welcher die Sequenz des reaktiven Zentrums Ala-Cys-Thr-Leu durch Arg-Cys-Lys-Arg ersetzt ist (vgl. Lu et aL (1993) J. BioL Chem. 268: 14583-14585), von Propeptid von Proteinen der Familie der PCs abgeleitete Oligopeptide, insbesondere das Propeptid von Furin (z.B. murin oder human), bspw. Positionen 1 bis 75 oder 107 der in Fig. 1 (obere Sequenz) gezeigten Sequenz, insbesondere Oligopeptide, welche die Sequenz QQVAKRRTKR oder EQRLLKRAKR oder Derivate dieser Sequenzen enthalten, und Poly-L- oder Poly-D-Arginine, besonders bevorzugt nicht-acetylierte Poly-D-Arginine. Insbesondere bevorzugte inhibitorische Liganden von Proteinen aus der Familie der PCs sind die folgenden (Einbuchstabencode: Großbuchstaben = L-AS, Kleinbuchstaben = D-Spezies, Ac = Acetyl, -NH₂ = nicht-amidiert): Ac-HHKRRR-NH₂, Ac-MHKRRR-NH₂, Ac-KHKRRR-NH₂, Ac-RHKRRR-NH₂, Ac-HRKRRR-NH₂, Ac-MRKRRR-NH₂, Ac-KRKRRR-NH₂, Ac-RRKRRR-NH₂, Ac-wrrril-NH₂, Ac-wrrrir-NH₂, Ac-wrrrrl-NH₂, Acwrrrrr-NH₂, LLRVKR-NH₂, Ac-LLRVKR, Ac-LLRVKR-NH₂, LLRVKR, Tetra-L-Arg, Penta-L-Arg, Hexa-L-Arg, Hepta-L-Arg, Octa-L-Arg, Nona-L-Arg und Hexa-D-Arg.

Typischerweise PC-Protein-Inhibitoren enthalten multibasische Peptidylgruppen und sind häufig an Verbindungen gekoppelt, die an das Katalytische Zentrum binden, bspw. Chloromethylketone, Ketomethylen oder Phosphonatgruppen.

Bei der erfindungsgemäßen Raumform eines Polypeptids der zuvor offenbarten Art, ggf. in Kombination mit einem oder mehreren Ligand/en, wird es sich um eine durch NMR-Strukturanalyse gewonnene Raumform (Wüthrich, NMR-Spectroscopy, 1986) oder aber um eine Kristallform handeln. Die Kristallform wird als Ergebnis nach Kristallisierung des Polypeptids und ggf. weiteren Komponenten, bspw. mindestens einem Liganden, mit nachfolgender röntgenkristallographischer Strukturaufklärung erhalten (Stout und Jensen, X-RAY Structure Determination, Wiley, 1989; oder Blundell; die Offenbarung aus Stout und Jensen oder Blundell wird vollumfänglich in die vorliegende Offenbarung in Hinblick auf die Durchführung röntgenkristallographischer Experimente einbezogen).

Erfindungsgemäße Kristailformen zeichnen sich auch dadurch aus, daß sie als dreidimensionale Struktur, charakterisiert durch Strukturkoordinaten für jedes einzelne, die Struktur aufbauende Atom (außer Wasserstoffatomen), Bestandteil einer symmetrischen Anordnung in einem Kristall sind. Dabei ist es bevorzugt, daß eine erfindungsgemäße Raumoder Kristallform, die mindestens ein Polypeptid mit mindestens einem mindestens 50 AS oder - wie oben offenbart - längeren Abschnitt eines oder mehrerer Protine aus der Familie der PCs enthält, nach Überlagerung mit den in der Fig. 6 aufgelisteten Strukturkoordinaten für diesen mindestens einen Abschnitt eine mittlere Standardabweichung (rmsd) von weniger als 2,5 Å, vorzugsweise von weniger als 2 Å, aufweist, wobei sich die mittlere Standardabweichung entweder auf die Koordinaten der sogenannten Rückgratatome oder auf die Koordinaten aller Atome der Struktur (im Falle einer Kristallform aller Atome außer Wasserstoffatomen) handeln kann.

Damit sind auch Raumformen bzw. Kristallforrnen mit einem Strukturkoordinatensatz für den mindestens einen mindestens 50 AS oder - wie oben offenbart - mehr umfassenden Abschnitt eines Proteins aus der Familie der PCs mit der vorgenannten mittleren Standardabweichung Bestandteil der vorliegenden Offenbarung, ebenso wie der gemäß Fig. 6 dargelegte Strukturkoordinatensatz, gleichfalls auch unter Berücksichtigung dieser Standardabweichungen.

Liegt die Raumform als Kristallform vor, so wird die Kristallform typischerweise neben den Atomen des Polypeptids bzw. ggf. des/der Ligand/en (außer Wasserstoffatomen) weitere Metallionen enthalten, beispielsweise Erdalkali- oder Alkalimetallionen, insbesondere Calciumionen, aber auch vor allem zur Phasenermittlung geeignete Schwermetallionen, wie z.B. Gold-, Selen-, Nickel- oder Quecksilberionen.

Die vorgenannten mindestens 50 AS oder - wie oben dargelegt - mehr umfassenden Abschnitte eines Proteins aus der Familie der PCs können zunächst durch "Alignment" den entsprechenden Abschnitten humanen oder insbesondere murinen Furins zugeordnet werden. Dann können aus der Fig. 6 die Strukturkoordinaten bspw. für murines oder humanes Furin oder entsprechend für andere PCs, insbesondere gemäß obiger Auflistung, ermittelt werden. Hierzu sind die Strukturkoordinaten der in den vorgenannten Sequenzen auftretenden Atome der Fig. 6 zu entnehmen. Die Auflistung der Atome in der Fig. 6 erfolgt jeweils sequentiell Aminosäure für Aminosäure vom N- zum C-Terminus.

Ganz besonders bevorzugt sind schließlich Raumformen eines Polypeptids als Kristallform, wenn das Polypeptid in der Kristallform Furin, insbesondere eine Säugetier-Furinsequenz, vor allem die humane Sequenz ist. Die vorstehenden Ausführungen zu Abschnitten oder Derivaten gelten für die Furinsequenzen entsprechend.

Ein weiterer Gegenstand der vorliegenden Erfindung sind Kristalle, die Raum- bzw. Kristallformen, wie erfindungsgemäß, insbesondere durch die Ansprüche 1 bis 22, beansprucht, nach symmetrischen Gesetzmäßigkeiten angeordnet, aufweisen. Hierunter fallen Kristalle all jener Kristallformen, die gemäß vorliegender Erfindung offenbart werden. Gemäß der vorliegenden Erfindung werden nämlich Kristalle beansprucht, die aus Einheitszellen aufgebaut sind, wobei die asymmetrische Einheit in der Einheitszelle des Kristalls mindestens einen mindestens 50, vorzugsweise einen mindestens 80, stärker bevorzugt einen mindestens 100 und am stärksten bevorzugt einen mindestens 120 AS umfassenden Abschnitt eines Polypeptids aus der Familie der PCs und ggf. mindestens eine weitere Verbindung aufweist und wobei weiterhin das Polypeptid im Kristall eine Raumform als Kristallform einnimmt, wie vorhergehend offenbart. Besonders bevorzugt sind Kristalle eines Polypeptids, das mindestens eine Domäne (P-Domäne oder katalytische Domäne eines Proteins der Klasse der PCs enthält).

Vorzugsweise wird der Kristall eine Raumgruppe aufweisen, die monoklin, tetragonal, orthorhombisch, kubisch, triklin, hexagonal oder trigonal/rhombohedr-al ist. Hierbei kann es sich um native Kristalle, Derivatkristalle oder auch Cokristalle handeln, ggf. auch ein "gesoakter" Kristall, also nach Einführung eines Liganden in den Kristall durch entsprechende Lösungszugabe. Typischerweise wird dabei die Raumgruppe des eine erfindungsgemäße Raumform als Kristallform aufweisenden Kristalls die Raumgruppe P1 oder P6₅ sein. Grundsätzlich können aber erfindungsgemäße Raumformen (Kristallformen) in allen "proteinkristallographisch möglichen Raumgruppen auftreten. Ganz besonders bevorzugt sind solche erfindungsgemäßen Kristalle, deren Einheitszelle Zellkonstanten von ungefähr a = 93,3 Å, b = 135,4 Å, c = 137,8 Å und α = 103,6° β = 99,0° und γ = 107,1° oder von ungefähr a = 135,5 Å, b = 135,5 Å, c = 472,0 Å und α = 90° β = 90° und γ = 120°aufweist.

Ein weiterer Gegenstand der vorliegenden Erfindung sind Verfahren zur Herstellung eines Kristalls mit Einheitszellen, enthaltend in der asymmetrischen Einheit mindestens eine Raumform der erfindungsgemäßen Art, also eines Polypeptids und ggf. mindestens einer weitere Verbindung, wobei (a) das Polypeptid in einem Expressionssystem überexprimiert wird, (b) das überexprimierte Polypeptid gereinigt und aufkonzentriert wird, (c) das gemäß (b) erhaltene Polypetidkonzentrat in einem geeigneten Puffersystem, ggf. unter Hinzufügung mindestens einer weiteren Verbindung, gelöst wird und (d) die Kristallisierung beispielsweise durch (Dampf)diffusionsverfahren oder andere Verfahren eingeleitet wird. Auch in Hinblick auf die Verfahrensmöglichkeiten bei der Kristallisierung werden die Angaben bei Stout und Jensen (s.o.) vollumfänglich in die vorliegende Offenbarung einbezogen.

Als weiterer Erfindungsgegenstand werden Verbindungen offenbart, die als Liganden an Polypeptide aus der Klasse der PCs bzw. an Domänen der Polypeptide aus der Klasse der PCs (wobei die Domänen Bestandteil anderer Proteine als jener aus der Klasse der PCs sein können) binden können. Erfindungsgemäße Verbindungen bilden nicht-kovalente Wechselwirkungen mit der Hauptkette und oder den Seitenketten von Aminosäuren, die Bestandteil eines funktionellen Bereichs der Domänen von PCs (katalytische oder P-Domäne), vorzugsweise an das katalytische Zentrum oder regulatorische Zentren. Durch diese Bindung des Liganden wird vorzugsweise die physiologische Funktion einer oder mehrer Endoproteinasen blockiert. Daher bindet in einer bevorzugten Ausführungsform die erfindungsgemäße Verbindung die erfindungsgemäße Raumform eines Polypeptids, insbesondere wie in einem der Ansprüche 1 bis 22 definiert, oder die Raumform eines mindestens irgendeinen Strukturabschnitt, aufweisend mindestens 50 AS, vorzugsweise mindestens 80 AS und stärker bevorzugt mindestens 100 AS Länge, enthaltenden Polypeptids aus der Familie der PCs so, daß die physiologische Aktivität diese Polypeptids moduliert, vorzugsweise blockiert, wird

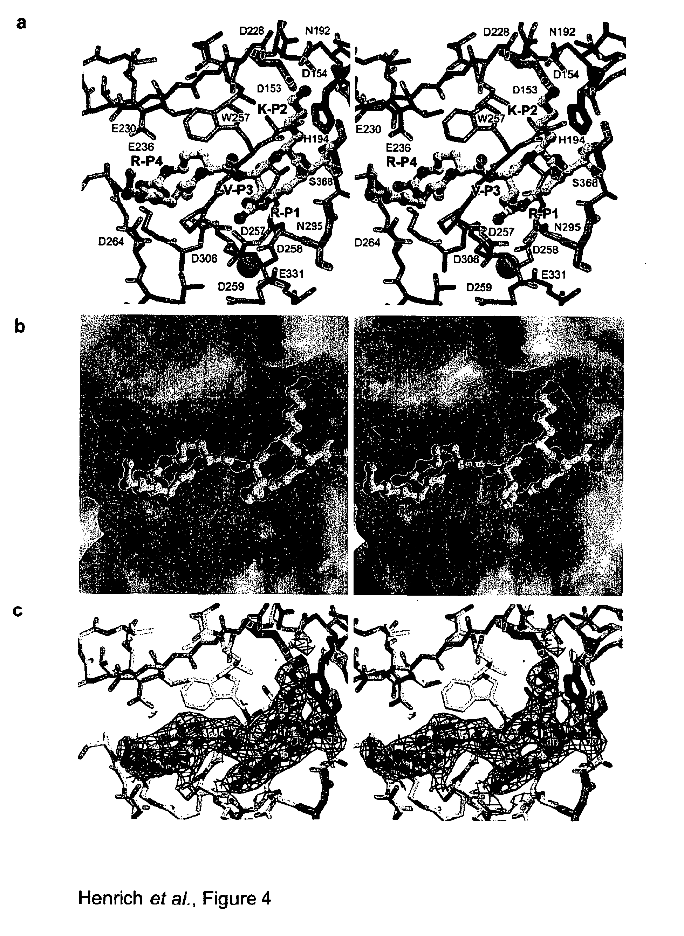

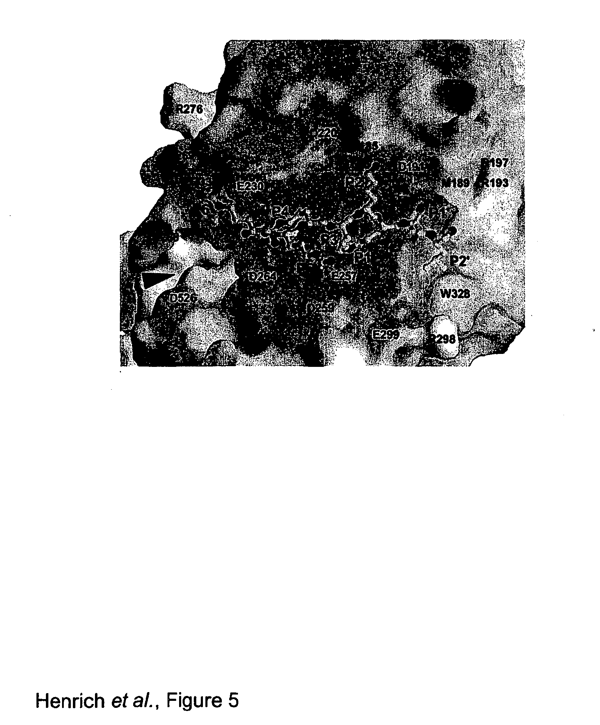

Die Liganden werden dabei vorzugsweise mit mindestens einer der Aminosäuren Ser368, His194, Asp153, Asn295, Ser253 bis Asp258, Ser293, Asp306, Ala292, Pro256 Asp154, Asp191, Glu236, Asp264, Trp254 und Tyr308, besonders bevorzugt mit mindestens einer der Aminosäuren Ser368, His194 und Asp153, von Furin (gemäß der Zählweise in Fig. 1) oder der an den gemäß Figur 6 entsprechenden Positionen befindlichen Aminosäuren aus katalytischen Domänen von mit Furin verwandten Proteinen (bspw. andere Proteine aus der Familie der PCs oder Furine unterschiedlichen Ursprungs) in Wechselwirkung treten. Ganz besonders bevorzugt werden die Liganden mit der katalytischen Domäne einen Teil oder alle Wechselwirkungen ausbilden, die in den Figuren 4 und 5 schematisch für den Liganden Decanoyl-RVKR-Chlormetlrylketon dargestellt sind. Insbesondere werden die Liganden jene Wasserstoffbrückenbindungen, hydrophoben Kontakte, van-der-Waals-Wechselwirkungen oder elektrostatischen Wechselwirkungen eingehen, die auch die nativen oder nicht-nativen, insbesondere synthetischen Substrate oder Derivate oder Analoge davon mit den für deren Bindung und gegebenenfalls Spaltung maßgeblich verantwortlichen Aminosäureresten der Proteine der Familie der PCs eingehen. D.h. ein erfindungsgemäßer Ligand wird vorzugsweise an äquivalenten sterischen Positionen mit den gebundenen Spezies äquivalente funktionelle Gruppen aufweisen. Insbesondere wird ein erfindungsgemäßer Ligand eine Peptid-Konsensussequenz RXXR, insbesondere RX(K/R)R (X steht für eine beliebige natürlich vorkommende AS), oder eine dazu äquivalente oder ähnliche Struktur, insbesondere mit basischem Charakter aufweisen, um an ein Protein aus der Familie der PCs zu binden. Im Zusammenhang mit den potentiellen Wechselwirkungen des Liganden eines Proteins aus der Familie der PCs wird auf die Beschreibung der Figuren 4 und 5 und auf die Darstellung der Ergebnisse bei der Beschreibung der Ergebnisse der Ausführungsbeispiele verwiesen. Ein erfindungsgemäßer Ligand wird mindestens einige, ggf. alle der dort beschriebenen Wechselwirkungen mit einem Protein der Familie der PCs, insbesondere Furin, vorzugsweise der katalytischen Domäne davon, ausbilden, so daß eine Bindungsaffinität Kd von weniger als 100 µM, vorzugsweise weniger als 50 µM, ganz besonders bevorzugt weniger 20 µM sichergestellt ist.

Insgesamt wird ein erfindungsgemäßer Ligand also eine strukturelle und sterische Ausgestaltung haben, die den jeweiligen Bindungsbereichen im Protein aus der Familie der PCs, insbesondere Furin, vor allem den strukturellen Vorgaben aus der Fig. 6, bspw. der P-Domäne oder der katalyischen Domäne, insbesondere im aktiven Zentrum, an der Schnittstelle der genannten Domänen bzw. regulatorischen Bereichen, komplementär ist. Bei diesen Liganden mit vorzugsweise Inhibitorfunktion für eine Funktion eines Proteins aus der Familie der PCs, insbesondere der proteolytischen Funktion, kann es sich um modifizierte oder unmodifizierte Di-, Oligo- oder Polypeptide handeln. Auch ein Peptidomimetikum eines Di- oder Oligopeptids ist denkbar. Bei den Peptidomirnet&a wird es sich vorzugsweise um solche Verbindungen handeln, deren Rückgrat keine amidartigen Bindungen, sondern andere chemische Brücken aufweisen, um die proteolytische Spaltung zu vermeiden.

Eine erfindungsgemäße Inhibitorverbindung kann bspw. eine native Zielsequenz eines Proteins aus der Familie der PCs sein. Bevorzugte Verbindungen, insbesondere Oligopeptide, enthalten bspw. ein oder mehrere Peptidmotive der Form (K/R)RX(K/R)R oder einem dazu ähnlichen Motiv, wobei X für eine beliebige, natürlich auftretende Aminosäure steht.

Besonders bevorzugte Peptidmotive, vor allem bei Oligopeptiden, die in erfindungsgemäßen Verbindungen enthalten sein können, sind bspw. WQREKR, THNRTKR, IENRKRR, SKKREKR, SSRRHKR, GGRRQRR, SSGRSKR, TDPRTKR , SNSRKKR, AGNRVRR, TRHRQPR, HASRVAR oder AKRRTKR sowie sämtliche in Nakayama (1997) Biochem. J. 327: 625-635, beschriebenen Motive natürlich vorkommender Substratsequenzen, wobei auf den diesbezüglichen Offenbarungsgehalt dieser Druckschrift explizit Bezug genommen wird und somit vollumfänglich in die vorliegende Beschreibung aufgenommen ist.

Insbesondere umfaßt die vorliegende Erfindung solche Liganden, die an eine Raum- oder Kristallform, die durch die Strukturkoordinaten nach Figur 6 dargelegt ist, binden können.

Ein weiterer Gegenstand der vorliegenden Erfindung sind Verfahren zur Identifizierung einer Verbindung, die die Eigenschaft besitzt, als Inhibitor der proteolytischen Wirkung eines Proteins aus der Familie (Klasse) der PCs, insbesondere Furin, vorzugsweise Furin aus einem Säuger, wie bspw. beim Menschen, zu wirken. Insbesondere bevorzugt ist ein solches Verfahren dann, wenn die Verbindung mit Ligandenfunktion an einen Bereich der katalytischen Domäne oder der Schnittstelle der katalytischen und der P-Domäne eines Proteins aus der Familie der PCs, insbesondere im Bereich des aktiven Zentrums, bindet. Ein solches Verfahren ist dadurch gekennzeichnet, daß (a) eine erfindungsgemäße Raum oder Kristallform, insbesondere wie in den Ansprüchen 1 bis 22 definiert, erhalten wird, wobei die Kristallform in Form ihrer Strukturkoordinaten vorliegt, (b) die Strukturkoordinaten der Kristallform in drei Dimensionen dargestellt werden, und (c) die sterischen Eigenschaften und/oder funktionellen Gruppen einer Verbindung mit Ligandenfunktion so gewählt werden, daß Wechselwirkungen zwischen der Verbindung und den Haupt- und/oder Seitenketten des Polypeptids, bspw. im aktiven Zentrum, möglich werden. Nach Maßgabe dieser Wechselwirkungen werden erfindungsgemäß geeignete Liganden, insbesondere geeignete inhibitorische Liganden, die die proteolytische oder andere Funktion eines Proteins aus der Familie der PCs, insbesondere Furin, blockieren, ermittelt.

Die Darstellung der Strukturkoordinaten einer erfindungsgemäßen Kristallform erfolgt vorzugsweise durch graphische Darstellung mit Hilfe entsprechender Computerprogramme auf einem Computerbildschirm. Anhand der, bezogen auf potentielle Liganden, komplementären Anordnung der Haupt- und Seitenketten der Kristallform, beispielsweise im aktiven Zentrum eines Proteins aus der Klasse der PCs, beispielsweise von Furin oder eines strukturell verwandten Proteins, können nicht-automatisiert nach Erfahrung des Operators geeignete Liganden mit entsprechenden chemischen und/oder sterischen Eigenschaften identifiziert, am Bildschirm konstruiert und schließlich deren Bindungsverhalten simuliert werden.

Vorzugsweise aber erfolgt die Auswahl geeigneter Liganden jedoch automatisiert dadurch, daß Computerdatenbanken, die eine Vielzahl von Verbindungen enthalten, durchsucht werden. Die Suche wird auf die zuvor erfolgende Charakterisierung von geometrischen, chemischen und/oder physikalischen Eigenschaften für die gewünschten Liganden, beispielsweise Verbindungen mit struktureller und/oder funktioneller Ähnlichkeit zu den bspw. in den Ansprüchen 28 bis 37 beanspruchten Verbindungen, gestützt. Zu durchmusternde Datenbanken enthalten natürlich auftretende wie auch synthetische Verbindungen. Beispielsweise können die in der CCDC (Cambridge Crystal Data Center, 12 Union Road, Cambridge, GB) gespeicherten Verbindungen für eine derartige Suche herangezogen werden. Aber auch die bei Tripos (s. Zitat a.a.O.) erhältlichen Datenbanken, nämlich Aldrich, Maybridge, Derwent World Drug Index, NCI und/oder Chapman & Hall können durchsucht werden. Die folgenden Computerprogramme können für eine derartige Durchmusterung eingesetzt werden: insbesondere das Programm "Unity", "FLEX-X" (Rarey et al. J. MoL BioL 261, 470-489, 1996), "Cscore" (Jones et aL, J. Mol. Biol. 245, 43, 1995) aus der Sybyl Base-Umgebung des Tripos-Programmpakets.

Im folgenden wird die Durchführung eines erfindungsgemäßen Verfahrens zur Computergestützten Identifizierung potentieller Liganden näher beschrieben. Zunächst muß der gewünschte Bindungsbereich eines Liganden in einer erfindungsgemäßen Kristallform definiert werden. Bei dem Liganden wird es sich typischerweise um einen solchen mit inhibitorischen Eigenschaften handeln, aber auch Aktivatoren sind denkbar. Der Bindungsbereich wird durch entsprechende Parameter, beispielsweise Atomabstände, Wasserstoffbrücken-Bindungspotentiale, hydrophobe Bereiche und/oder Ladungen, charakterisiert und auf dieser Basis Randbedingungen für die chemischen, physikalischen und/oder geometrischen Eigenschaften des Liganden definiert. Ganz besonders bevorzugt sind an der Bindung mindestens eine der bereits zuvor spezifizierten Aminosäuren (siehe Anspruch 31), insbesondere mit den vorgenannten Seitenketten derselben, beteiligt. Daher wird auch für ein vorliegendes erfindungsgemäßes Verfahren zur Identifizierung von Verbindungen auf die vorangegangene Offenbarung zum Erfindungsgegenstand "Verbindung" gemäß Ansprüchen 28 bis 37 vollinhaltlich Bezug genommen. Computerprogramme identifizieren in entsprechenden Datenbanken dann solche Verbindungen, die die zuvor eingeführten Bedingungen erfüllen. Hierbei ist es besonders bevorzugt, das Programmpaket Sybil Base (Tripos, 1699 South Hanley Road, St. Louis, Missouri, USA) zu verwenden. Besonders bevorzugt ist es dabei, daß die zu durchsuchende Datenbank Verbindungen unter Angabe ihrer jeweiligen dreidimensionalen Strukturen zur Verfügung stellt. Sollte dies nicht gegeben sein, wird für ein erfindungsgemäßes Verfahren vorzugsweise in einem Verfahrensschritt (d) ein Computerprogramm eingesetzt werden, das vor der Prüfung, ob die vorgegebenen Randbedingungen von einem Liganden erfüllt sind, zunächst deren dreidimensionale Struktur berechnet (z.B. das Programm "CONCORD" aus der Sybyl-Umgebung von Tripos Inc.).

Typischerweise wird in einem Verfahrensschritt (e) das Wechselwirkungspotential zwischen einer identifizierten Verbindung, beispielsweise im Rahmen einer automatisierten Suche einer Verbindung aus einer Computerdatenbank, und dem gewünschten Bindungsbereich in einer Kristallform ermittelt. Ganz besonders bevorzugt ist ein erfindungsgemäßes Verfahren dann, wenn es zur Identifizierung von Verbindungen, die an eine Kristallform mit den Strukturkoordinaten der Fig. 6 andocken sollen, dient. Die Stärke der gemäß Verfahrensschritt (e) ermittelten Wechselwirkung zwischen einer Verbindung aus einer Computerdatenbank und einer erfindungsgemäßen Kristallform geben Anhaltspunkte für deren Eignung, als Liganden eingesetzt werden zu können.

Nicht automatisiert gestaltet sich ein Verfahren zur Identifizierung geeigneter Verbindungen mit Ligandencharakter wie folgt. Eine Gerüstverbindung als Ausgangspunkt der Identifizierung wird in den durch die zu identifizierende Verbindung auszufüllenden Raum im Innern oder an der Oberfläche der Kristallform, z. B. in das katalytische Zentrum in dem kristallisierten Polypeptid, manuell eingefügt. Für den nach Einfügung der Gerüststruktur noch verbliebenen Raum wird nach Fragmenten gesucht, die in Wechselwirkung mit der umgebenden Kristallform treten können und sich an die Gerüststruktur anlagern lassen. Diese Suche nach geeigneten Fragmenten erfolgt also nach Maßgabe der geometrischen und/oder physikochemischen Gegebenheiten der dreidimensionalen Struktur. Die Suche nach geeigneten Fragmenten kann beispielsweise als automatisierte Computersuche unter Vorgabe entsprechender Randbedingungen geführt werden. Etwaige durch den Operator und/oder durch die Computersuche ermittelten Fragmente werden nach Maßgabe chemischer Gesetzmäßigkeiten an die Ausgangsgerüststruktur des Startmodells graphisch angelagert und nach jedem derartigen Schritt das Wechselwirkungspotential mit dem Zielstrukturbereich in der Kristallform errechnet. Die Vorgehensweise erfolgt so lange, bis das Wechselwirkungspotential zwischen der zu identifizierenden Verbindung und dem Zielstrukturbereich optimiert ist.

Die Vorgehensweise der Schritte (c), (d) und (e) kann zyklisch so lange wiederholt werden, bis eine Verbindung oder eine Verbindungsklasse in bezug auf ihr Bindungsverhalten, berechnet nach einem Wechselwirkungspotential, das dem jeweiligen Computerprogramm als Algorithmus zugrunde liegt, optimiert ist. Die durch eine relativ grobe Charakterisierung des Bindungsbereichs der Kristallform zunächst erhaltene große Zahl an potentiell bindungsfähigen Verbindungen kann durch weitergehende Vorgaben physikalisch-chemischer oder sterischer Charakteristika an die gewünschte Zielverbindung zunehmend reduziert werden.

Insbesondere bietet sich hierfür auch eine sinnfällige Kombination der nicht-automatisierten und der automatisierten Suchverfahren nach geeigneten Verbindungen an. So etwa könnte eine zunächst durch automatisierte Computersuche aus Computerdatenbanken identifizierte Verbindung nicht-automatisiert durch Anlagerung von Fragmenten mit geeigneten funktionellen Gruppen verbessert werden.

Schließlich ist es im Rahmen der vorliegenden Erfindung bevorzugt, die durch derartige erfindungsgemäße Verfahren per automatisierter Computersuche erhaltenen Verbindungen zu synthetisieren oder, falls bereits synthetisiert und zugänglich, einer chemischen Bibliothek zu entnehmen und in einem geeigneten biologischen Testsystem in einem Verfahrensschritt (f) auf ihre biologische Wirksamkeit hin zu untersuchen. Abhängig vom Ergebnis des biologischen Testsystems, bei dem es sich z. B. um einen Ligandenbindungsassay handeln kann, können dann weitere chemische Modifikationen der zuvor ermittelten Verbindung oder der Verbindungsklasse vorgenommen werden. Hierbei kann sich dann insbesondere die Anwendung von Programmpaketen zur Identifizierung geeigneter Fragmente, die gegen vorhandene Fragmente an der zuvor identifizierten Verbindung ausgetauscht oder an dieselbe zusätzlich angelagert werden könnten, als sinnvoll erweisen.

Ein weiterer Gegenstand der vorliegenden Erfindung sind gleichfalls Verfahren zur Identifizierung einer Verbindung mit der Eigenschaft, als Ligand, typischerweise als Inhibitor, der physiologischen Aktivität von Proteinen der Klasse der PCs wirken zu können, wobei in einem solchen erfindungsgemäßen Verfahren in einem Verfahrensschritt (a) ein biologisches Testsystem vorangestellt wird, anhand dessen ein sog. "Screening" nach geeigneten Zielverbindungen durchgeführt wird. Auch hier können bevorzugt ein Bindungsassay als biologisches Testsystem dienen. In den weiteren Verfahrensschritten werden zunächst gemäß (b) solche Verbindungen (beispielsweise aus einer Bibliothek chemischer Verbindung) identifiziert, die ein positives Ergebnis im biologischen Test gezeigt haben. Diese Verbindungen, beispielsweise inhibierend oder gegebenenfalls auch aktivierend, werden in bezug auf ihre beispielsweise geometrischen und/oder chemischen Eigenschaften, insbesondere in bezug auf ihre dreidimensionale Struktur, charakterisiert (Verfahrensschritt (c)). Sofern die dreidimensionale Struktur der als Treffer im biologischen Test ermittelten Verbindungen nicht a priori bekannt ist, kann dieselbe durch Methoden der Strukturaufklärung, nämlich Röntgenkristallographie und/oder NMR-Spektroskopie, oder auch durch Modellierungen oder z.B. semi-quantenchemische Berechnungen ermittelt werden. In die gemäß Verfahrensschritt (d) als dreidimensionale Struktur dargestellten atomaren Strukturkoordinaten einer erfindungsgemäßen Kristallform werden dann gemäß (e) die im Rahmen der Verfahrensschritte (b) und (c) erhaltenen Verbindungen eingefügt. Hierbei kann es sich um Verbindungen handeln, die an einem für die physiologische Bindungsstelle relevanten Abschnitt oder aber auch an die Oberfläche des Polypeptids in der Kristallform binden. Das Einfügen der Verbindung in die Kristallform kann manuell nach der Erfahrung des Operators erfolgen oder aber auch automatisiert, indem mit Hilfe entsprechender Computerprogramme ("Dock" Kuntz et al., 1982, J. Mol. Biol. 161, 269-288, Sibyl/Base "FLEX-X", s. Zitat a.a.O.) eine Positionierung des Liganden mit der stärkstmöglichen Wechselwirkung zwischen Ligand und dem Zielstrukturbereich ermittelt wird (Verfahrensschritt (e)).

Indem eine derart erhaltene Verbindung graphisch in Kombination mit der in der Kristallform vorliegenden Struktur dargestellt wird, können weitere Verfahrensschritte erfolgen, die die Wirksamkeit der Zielverbindung verbessern. Insbesondere kann eine derart, bereits als geeignet identifizierte Verbindung als Basis ("template") für noch wirksamere Verbindungen, z. B. Verbindungen mit noch höherer Bindungskonstante, dienen. In diesem Zusammenhang können die bereits gemäß Ansprüchen 41 bis 46 beschriebenen Verfahren und Ansätze zum Einsatz kommen. Bevorzugt ist eine Vorgehensweise, die insoweit zyklisch ist, also nach dem "Screening" im biologischen Testsystem eine strukturelle Darstellung erfolgt und mit Hilfe von Computermethoden auf der Basis der im biologischen Testsystem erhaltenen Ergebnisse wirksamere Verbindungen ermittelt werden, die schließlich wiederum als Ausgangspunkt für den nächsten Zyklus, an dessen Beginn ein biologisches Testsystem steht, dienen. Biologische Testsysteme (in vitro oder in vivo) können Aussagen über die Qualität der Verbindung, z.B. als Inhibitor der biologischen Reaktion, also des Bindungsereignisses, oder über die Bindungskonstante, die Toxizität oder die Metabolisierungseigenschaften oder gegebenenfalls über das Membrandurchtrittsvermögen der Verbindung etc., gemacht werden.

Schließlich werden im Rahmen der vorliegenden Erfindung alle solchen Verbindungen beansprucht, die als Ergebnis eines Verfahrens nach einem der Ansprüche 41 bis 48 erhalten werden oder erhältlich sind.

Ein weiterer Gegenstand der vorliegenden Erfindung sind Verfahren zur Herstellung eines Kristalls mit einer Raum- oder Kristallform mit mindestens einem Polypeptid nach einem der Ansprüche 1 bis 22, wobei in einem Verfahrensschritt (a) zunächst das Polypeptid in einem Expressionssystem überexprimiert, synthetisiert oder isoliert wird, (b) das gemäß (a) erhaltene Polypeptid in einem geeigneten Puffersystem gelöst wird und (c) die Kristallisierung, beispielsweise durch (Dampf)diffusionsverfahren oder andere Verfahren, bspw. Äquilibrierung, eingeleitet wird Typischerweise wird gemäß Verfahrensschritt (b) eine konzentrierte oder hochkonzentrierte Lösung des (der) Polypeptids/e vorliegen, wobei bspw. für die Kristallisation die Luftfeuchtigkeit oder die Umgebungstemperatur frei gewählt werden können. Erfolgt die Kristallisierung des mindestens einen Polypeptids zu erfindungsgemäßen Kristallen, die erfindungsgemäße Kristallformen aufweisen, mit dem Ziel, die Kristalle nachfolgend zur Röntgenstruckturanalyse einzusetzen, so folgt nach der Kristallisierung die Sammlung von Röntgendiffraktionsdaten, die Bestimmung der Einheitszellkonstanten und der Symmetrie sowie die Berechnung der Elektronendichtekarten, in die das (die) Polypetid/e hineinmodelliert werden. Offenbart werden also auch Verfahren zur Aufklärung einer Raum- oder Kristallform, enthaltend mindestens ein Polypeptid nach einem der Ansprüche 1 bis 22, oder zur Ermittlung von Strukturkoordinaten eines Polypeptids in einer Raum- oder Kristallform nach einem der Ansprüche 1 bis 22, wobei im Falle eines Verfahrens auf der Basis einer Kristallform zunächst die vorgenannten Verfahrensschritte (a) bis (c) durchgeführt werden und dann in weiteren Verfahrensschritten (d) und (e) die Röntgendiffraktionsdaten gesammelt, eine Elektronendichtekarte unter Verwendung von Phaseninformation (bspw. durch isomorphes Ersetzen oder "anomolous. scattering" Methoden oder durch Schwermetallatom-Derivate) berechnet und schließlich die Primärsequenz entsprechend den Vorgaben der Elektronendichtekarte modelliert wird.

Ein weiterer Gegenstand der vorliegenden Erfindung sind Verfahren zur dreidimensionalen Darstellung einer Kristallform eines Polypeptids unbekannter Struktur, das mindestens einen mindestens 50 AS, vorzugsweise mindestens 80 AS, mehr bevorzugt mindestens 100 AS, stärker bevorzugt mindestens 120 AS, noch stärker bevorzugt mindestens 150 AS umfassenden Abschnitt eines Proteins aus der Familie der PCs, insbesondere Furin, bspw. von Furin aus einem Säuger wie humanes Furin, enthält. Ein solches Verfahren ist dadurch gekennzeichnet, daß die Kristallform mit unbekannter Struktur auf der Basis einer erfindungsgemäßen Kristallform mit bekannter Struktur, beispielsweise auf Basis der in Fig. 6 festgehaltenen Strukturkoordinaten, ermittelt wird. Hierbei gibt es verschiedene Möglichkeiten, bekannte Strukturkoordinaten erfindungsgemäßer Kristallforrnen zur Strukturaufklärung von Polypeptiden oder Polypeptidkomplexen mit bislang unbekannten 3D-Strukturen (Zielstruktur), die jedoch mit der bekannten erfindungsgemäßen Kristallform gewisse Homologien in der Primärsequenz zeigen, einzusetzen.

Eine Möglichkeit ist in diesem Zusammenhang die Verwendung von Phaseninformation, die aus bekannten Ausgangsstrukturkoordinaten, beispielsweise den Strukturkoordinaten oder Teilen dieser Strukturkoordinaten gemäß Fig. 6, entnommen werden können. Hierzu wird die Phaseninformation, die im Falle einer bekannten 3D-Struktur einer erfindungsgemäßen Kristallform vorliegt bzw. berechnet werden kann, eingesetzt, um eine unbekannte Struktur, die sich vorzugsweise gegenüber der bekannten Struktur nur durch nicht wesentliche konformationelle Abweichungen unterscheidet (als Beispiele zu nennen wären Zielstrukturen, an die erstmals ein Ligand oder ein anderer Ligand als in der Ausgangsstruktur gebunden ist, oder Derivate, z.B. Zielstrukturen, die Mutanten der Ausgangsstruktur sind) zu lösen. Hierzu wird die Phaseninformation der gesamten oder eines Teils der bekannten Struktur mit den für die Kristallform unbekannter Struktur gesammelten Intensitäten der Beugungsreflexe kombiniert und aus dieser Kombination eine Elektronendichtekarte für die Kristallform unbekannter Struktur berechnet. Diese Methode wird als "molekulares Ersetzen" ("molecular replacement") bezeichnet. Vorzugsweise wird das molekulare Ersetzen mit dem Programmpaket X-PLORE (Brünger, Nature 355, 472-475, 1992) oder anderen kommerziell erhältlichen Programmpaketen durchgeführt.

Eine weitere Möglichkeit, vorliegende erfindungsgemäße Kristallformen zur Strukturaufklärung von strukturell verwandten Sequenzen bzw. beim Vergleich der Primärstrukturen mit zumindest teilweise homologen Polypeptidketten einzusetzen, besteht erfindungsgemäß darin, daß (a) die Primärsequenz eines Polypeptids unbekannter 3D-Struktur mit einer Primärsequenz eines Polypeptids, das mindestens einen Teilbereich, bspw. mindestens einen strukturell homogenen Bereich eines Proteins der Familie der PCs, bspw. eine Domäne (P-Domäne oder katalytische Domäne), aufweist, verglichen wird und im Rahmen dieses Vergleichs homologe Abschnitte zwischen dem Polypeptid unbekannter Struktur und der Primärsequenz eines Polypeptids oder Abschnitts eines Polypeptids aus der Familie der PCs, dessen Raum- oder vorzugsweise Kristallform bekannt ist, identifiziert werden, (b) die homologen Abschnitte in Anlehnung an die bekannte 3D-Struktur modelliert wird und schließlich gemäß Verfahrensschritt (c) mit Hilfe geeigneter Computerprogramme die modellierte 3D-Struktur des Polypeptids in Hinblick auf ihre sterischen Verhältnisse optimiert wird.

Das sog. "Alignment" der Primärsequenzen von zu vergleichenden Polypeptiden unbekannter bzw. bekannter 3D-Struktur gemäß (a) stellt eine zentrale Aufgabe für das Homologie-Modelling dar. Hierbei werden die ausgerichteten korrespondierenden Aminosäuren verschiedenen Kategorien zugeordnet, nämlich Positionen mit identischen, ähnlichen, entfernt ähnlichen oder unähnlichen Aminosäuren. Besondere Berücksichtigung müssen beim "Alignment" gegebenenfalls Insertionen oder Deletionen zwischen den zu vergleichenden Primärsequenzen finden. Die gemäß Verfahrensschritt (c) erfolgende Optimierung der auf der Grundlage der bekannten 3D-Struktur modellierten Zielstruktur kann durch die Methoden der Molekulardynamik-Simulation "molecular dynamics" oder durch Energieminimierung (z.B. Sybil Base von Tripos, s. Zitat a.a.O.) erfolgen.

Besonders bevorzugt ist ein erfindungsgemäßes Verfahren zur Aufklärung von Kristallformen unbekannter Struktur dann, wenn es sich bei der Kristallform bekannter Struktur um ein Protein aus der Klasse der PCs oder ein hiermit eng verwandtes Protein, insbesondere aber auch um einen Teilabschnitt derselben (ganz besonders um einen domänenspezifischen Abschnitt) handelt und die Kristallform eines in seiner Struktur unbekannten Isoproteins oder eines anderen verwandten Proteins unbekannter Struktur z.B. durch "Molekulares Ersetzen" oder durch "Homologie-Modelling" aufgeklärt werden soll. Gleichfalls kann auf der Basis einer erfindungsgemäßen bekannten Raum- oder Kristallform mit bekannten Strukturkoordinaten, die für einen Organismus aufgeklärt wurden, eine Kristallform für ein analoges Protein, gegebenenfalls mit einem Liganden komplexiert, eines anderen Wirtsorganismus ermittelt werden.

Damit also können Strukturkoordinaten von erfindungsgemäßen Kristallformen durch Homologie-Modelling als Strukturmodelle für sequentiell homologe Polypeptide unbekannter 3D-Strukturen dienen. Im Rahmen des Homologie-Modellings kommen Programmpakete zum Einsatz, insbesondere kann mit dem Insight II Modellierungspaket (Molecular Simulations Inc.) ein derartiges Modelling durchgeführt werden. Die Beschreibung des Programmpakets Insight II wird vollumfänglich in die vorliegende Offenbarung, insbesondere in Hinblick auf das Homologie-Modelling, einbezogen.

Ein weiterer Gegenstand der vorliegenden Erfindung sind Verfahren zur Identifizierung von Verbindungen, die die physiologische Funktion, insbesondere die proteolytische (Endoprotease-) Funktion, eines Proteins aus der Familie der PCs, enthaltend mindestens einen mindestens 50 AS oder - wie vorstehend offenbart - mehr umfassenden Teilabschnitt eines Proteins aus der Familie der PCs, vorzugsweise des Furins, insbesondere aus einem Säugetier oder einem Menschen, weiter bevorzugt enthaltend die katalytische Domäne, oder eines Derivats einer solchen Aminosäuresequenz moduliert, insbesondere die proteolytische Wirkung inhibiert, wobei hierfür (a) die unbekannte 3D-Struktur eines derartigen Polypeptids, das einem Polypeptid in einer erfindungsgemäßen Raumform entspricht, nach einem in den Ansprüchen 50 bis 52 beschriebenen Verfahren ermittelt wird und dann (b) mit Hilfe eines der in Zusammenhang mit den Ansprüchen 41 bis 48 beschriebenen Verfahren eine Verbindung mit der Fähigkeit, als Inhibitor der physiologischen Funktionm insbesondere der Proteolyse- (Endoprotease-)Funktion eines Proteins aus der Familie der PCs zu wirken, bestimmt wird.

Schließlich wird im Rahmen der vorliegenden Erfindung auch die Verwendung von Inhibitoren und/oder gegebenenfalls Aktivatoren der physiologischen Aktivität von PCs (insbesondere Furin, PC2, PC1/PC3, PACE4A, PC4, PC5/PC6A, PC5/PC6B, LPC/PC7/PC8/SPC7 aus Säugetieren, insbesondere humanen Ursprungs) oder Abschnitten derselben nach einem der Ansprüche 28 bis 37 bzw. erhalten oder erhältlich aus einem Verfahren nach einem der Ansprüche 41 bis 48 zur Herstellung eines Arzneimittels, zur Verwendung als Arzneimittel oder als Wirkstoff, der in einer pharmazeutischen Zusammensetzung enthalten ist, offenbart. In einer pharmazeutischen Zusammensetzung erfindungsgemäßer Art kann ein erfindungsgemäßer Inhibitor oder gegebenenfalls Aktivator mit mindestens einem weiteren Wirkstoff kombiniert sein und/oder die pharmazeutische Zusammensetzung bzw. der Inhibitor als Wirkstoff wird als Arzneimittel in eine dem Fachmann geläufigen Formulierung eingearbeitet sein, insbesondere unter Berücksichtigung geeigneter pharmazeutischer Hilfs-, Zusatz- oder Ergänzungsstoffe. Die Formulierung wird dabei insbesondere vom Verabreichungsweg abhängen. Dieser kann oral, rektal, intranasal oder parenteral, insbesondere subkutan, intravenös, oder intramuskulär, sein. Pharmazeutische Zusammensetzung, die einen derartigen Inhibitor und/oder Aktivator enthalten, können die Verabreichungsform eines Puders, einer Suspension, einer Lösung, eines Sprays, einer Emulsion, einer Salbe, eines Aerosols oder einer Creme haben.

Ein erfindungsgemäßer Inhibitor oder gegebenenfalls Aktivator kann mit einem pharmazeutisch akzeptablen Trägermaterial mit neutralem Charakter (wie z. B. wäßrigen oder nicht-wäßrigen Lösungsmitteln, Stabilisatoren, Emulgatoren, Detergentien und/oder Additiven und gegebenenfalls weiteren Farb- oder Geschmacksmitteln kombiniert sein. Die Konzentration eines erfindungsgemäßen Inhibitors und/oder Aktivators in einer pharmazeutischen Zusammensetzung kann zwischen 0,1% und 100% variieren, abhängig insbesondere von dem Verabreichungsweg. Eine pharmazeutische Zusammensetzung oder ein Arzneimittel, enthaltend einen erfindungsgemäßen Inhibitor oder gegebenenfalls Aktivator der physiologischen (insbesondere proteolytischen) Wirkung von PCs kann insbesondere zur Behandlung bzw. zur Herstellung eines Arzneimittels zur Behandlung von Tumorerkrankungen (bspw. Carcinome, Glioblastome), Autoimmunerkrankungen, Immunsuppression, Behandlung von Entzündungserkrankungen, GVHD oder zur Behandlung bzw. zur Herstellung eines Arzneimittels zur Behandlung von Virus- oder Bakterieninfektionen (HIV-Infektionen, Influenzainfektionen mit lokalem oder systemischen Infektionspotential, Ebola-Infektionen, Milzbrand sowie weiterer Infektionen mit Erregern, welche die vorstehend genannten natürlichen Substrate des Furins, einschließlich aller Analogen oder Derivate dieser Substrate, aufweisen), Fertilitätsproblemen, amyloide Demenzen (z.B. Morbus Alzheimer), Morbus Parkinson, neurodegenerative Erkrankungen dienen. Auch sind Inhibitoren von bspw. Furin geeignet, den Zelltod, der von Pseudomonas, Anthrax-Toxin oder Cytomegaloviren ausgelöst wird, zu blockieren. Entsprechende Verwendungen zur herstellung eines Arzneimittels ergeben sich.

Ein weiterer Gegenstand der vorliegenden Erfindung ist ein Satz von Strukturkoordinaten eines Proteins aus der Gruppe der PC-Proteine (bzw. eines Fragments eines solchen Proteins von typischerweise mindestens 50 AS Länge) oder eines Polypeptids, enthaltend mindestens einen Abschnitt von mindestens 50 (oder stärker bevorzugt, wie zuvor offenbart, von mehr AS Länge) eines Proteins aus der Gruppe der PC-Proteine, wie er bspw. in einer Raum- oder Kristallform auftritt, bspw. den Suukturkoordinatensatz von Furin, insbesondere gemäß Figur 6. Die erfindungsgemäßen Strukturkoordinaten, insbesondere ein Satz von Strukturkoordinaten eines PC-Enzyms bzw. eines PC-Substrat-Komplexes oder ein Teil davon, insbesondere die vorstehend definierten Domänen, bspw. eines Furins, ganz besonders bevorzugt den Strukturkoordinatensatz gemäß Figur 6, stellen einen relativen Satz von Koordinatenpunkten dar, die eine Struktur dreidimensional, bezogen auf einen (variablen) Bezugspunkt (Ursprung des Koordinatensystems), definieren. Daher können auch im wesentlichen vollständig von spezifischen Koordinatensätzen verschiedene (ebenfalls spezifische) Datensätze gemäß der vorliegenden Erfindung als Erfindungsgegenstände ebenfalls offenbart sein, welche aber dennoch eine ähnliche oder identische Struktur definieren. Darüber hinaus haben im allgemeinen geringe Variabilitäten bezüglich der individuellen Koordinaten nur eine geringe Auswirkung auf die Gesamtstruktur.

Die vorstehenden Koordinatenvariabilitäten, welche insbesondere nur geringe Auswirkungen auf die Gesamtstruktur haben, können durch mathematische Operationen ausgehend von den vorliegend offenbarten spezifischen Strukturkoordinaten erzeugt werden. Bspw. können die vorliegend offenbarten Strukturkoordinaten von Furin mittels kristallographischer Permutationen von Strukturdaten, deren Fraktionalisierung, Additionen oder Subtraktionen von ganzen Zahlen zu Strukturkoordinatensätzen, Inversion der Strukturkoordinaten oder irgendeiner Kombination davon manipuliert werden.

In einer anderen Ausführungsform können Modifikationen der Struktur aufgrund von Mutationen, Additionen, Substitutionen und/oder Deletionen von Aminosäuren oder anderen Veränderungen irgendeiner den Kristall bildenden Komponenten zu Veränderlichkeiten der Strukturkoordinaten führen. Falls derartige Variationen innerhalb einer annehmbaren Standardabweichung im Vergleich zu den ursprünglichen Koordinaten liegen, wird die resultierende dreidimensionale Struktur im wesentlichen die gleiche sein.

Somit sind im allgemeinen Rechner-gestützte Analysen erforderlich, um zu bestimmen, ob ein Molekül oder ein entsprechender Komplex oder ein Teil davon der erfindungsgemäßen PC-Struktur bzw. einzelnen Domänen davon ausreichend ähnlich ist, um nach den vorstehend dargelegten Merkmalen als "gleich" anzusehen ist. Derartige Analysen können mit Hilfe von bekannten Software-Programmen, wie die Anwendung "Molecular Simularity" des Programms QUANTA (molecular simulations Inc., San Diego, CA, USA), bspw. Version 4.1, nach Maßgabe der dem Software-Paket beigefügten Anleitung durchgeführt werden. Hierdurch können rechnerisch mittlere Standardabweichungen vom Bezugskoordinatensatz definiert werden.

Die Anwendung "Molecular Simularity" erlaubt Vergleiche zwischen verschiedenen Strukturen, verschiedenen Konfirmationen der gleichen Struktur und unterschiedlichen Teilen der gleichen Struktur. Der in Molecular Simularity zum Strukturvergleich verwendete Algorithmus gliedert sich in vier Schritte: (1) Laden der zu vergleichenden Strukturen; (2) Definieren der äquivalenten Atome in diesen Strukturen; (3) Durchführung einer Anpassungsoperation ("Fitting"); und (4) Analysieren der Ergebnisse.

Jede Struktur wird dabei durch einen Namen identifiziert. Eine Struktur wird als Zielstruktur ("Target") (d.h. die fixe Struktur), alle verbleibenden Strukturen sind Arbeitsstrukturen (d.h. veränderliche Strukturen). Da die äquivalenten Atome in QUANTA durch den Anwender definiert werden, werden erfindungsgemäß äquivalente Atome als die Peptidrückgrat-Atome (N, Cₐₗₚₕₐ C und O) aller zwischen den beiden verglichenen Strukturen konservierten Reste definiert. Es werden auch ausschließlich starre Anpassungsoperationen berücksichtigt.

Wenn ein starres Verfahren verwendet wird, wird die Arbeitsstruktur translatorisch und rotatorisch verändert, um eine optimale Übereinstimmung mit der Zielstruktur zu erhalten. Die Anpassungsoperation verwendet einen Algorithmus, der das Optimum der auf die Arbeitsstruktur anzuwendenden Translations- und Rotationsbewegungen derart berechnet, daß die Standardabweichungsdifferenz der Anpassung über die spezifischen Paare äquivalenter Atome ein absolutes Minimum darstellt. Diese Zahl (in Å) wird von QUANTA ausgegeben.

Erfindungsgemäß wird jedes Molekül oder jeder molekulare Komplex, der eine Standardabweichung bezüglich der Peptidrückgrat-Atome (N, Cₐₗₚₕₐ, C, O) aller konservierten Reste von weniger als 2 Å aufweist, wenn sie über die jeweils relevanten Rückgratatome, die durch die vorstehend definierten Strukturdaten der vorliegenden Erfindung definiert werden, übereinander gelegt werden, als identisch im Sinne der vorliegenden Erfindung betrachtet.

Vorzugsweise beträgt die Standardabweichung weniger als 1,5, mehr bevorzugt weniger als 1 Å.

Der Ausdruck "Standardabweichung" bedeutet die Quadratwurzel des arithmetischen Mittels der Quadrate der Abweichungen vom Mittelwert. Erfindungsgemäß definiert die "Standardabweichung" die Abweichung des Peptid-Rückgrats eines Proteins oder Proteinkomplexes von dem jeweils betrachteten Teil des Rückgrats der erfindungsgemäßen Polypeptide oder Komplexe davon, die durch die vorliegend offenbarten Strukturkoordinaten definiert werden.

Die Bereitstellung der erfindungsgemäßen Strukturdaten der PC-Proteine eignet sich auch zur Anwendung bei der Lösung von Strukturen anderer Proteinkristalle.

Vorteilhafterweise werden in einer weiteren Ausführungsform der vorliegenden Erfindung die erfindungsgemäßen Strukturdaten oder Teile davon auf einem Maschinen (Computer)-lesbaren Speichermedium gespeichert. Derartige Daten können dann für verschiedene, Zwecke, wie oben offenbart, bspw. für die Identifizierung von Arzneistoffen oder röntgenkristallographische Analysen verwendet werden.

Dementsprechend wird erfindungsgemäß ein maschinenlesbares Speichermedium, umfassend ein Datenspeichermaterial, das die erfindungsgemäß offenbarten Strukturkoordinaten codiert, bereitgestellt.

Erfindungsgemäß kann dieser Gegenstand der vorliegenden Erfindung bspw. ein System umfassen, das einen Computer, umfassend eine zentrale Rechnereinheit ("CPU"), einen Arbeitsspeicher, der bspw. ein RAM ("Random-Access-Memory") oder ein "Kern"-Speicher sein kann, einen Massenspeicher wie ein oder mehrere Diskettenlaufwerke oder CD-ROM-Laufwerke, ein oder mehrere Anzeigegeräte (bspw. Monitore oder TFT-Bildschirme), eine oder mehrere Tastaturen, eine oder mehrere Eingabedatenleitungen und ein oder mehrere Ausgabedatenleitungen einschließt, wobei sämtliche Komponenten durch einen üblichen bidirektionalen Systembus miteinander verbunden sind.

Es können verschiedene Eingabe-Einrichtungen, die mit dem Computer durch Eingabedatenleitungen verbunden sind, in verschiedener Weise in das System eingebunden werden. Die Computerlesbaren Daten der vorliegenden Erfindung können durch Verwendung ein oder mehrerer Modems, das/die durch ein Telefonkabel oder eine Datenleitung mit dem System verbunden ist/sind, eingegeben werden. Alternativ oder zusätzlich kann die Eingabe-Einrichtung CD-ROM-Laufwerke oder Diskettenlaufwerke einschließen. Vorzugsweise in Verbindung mit einer Anzeigevorrichtung kann auch eine Tastatur als Eingabe-Vorrichtung verwendet werden.

Des weiteren wird allgemein eine Ausgabe-Einrichtung vorgesehen, die mit dem Computer durch Ausgabedatenleitungen verbunden ist. Bspw. kann die Ausgabe-Vorrichtung einen Monitor oder ein TFT-Display zum Anzeigen einer graphischen Darstellung einer erfindungsgemäßen Struktur, bspw. unter Verwendung eines Programms wie das vorstehend genannte Software-Paket QUANTA einschließen. Eine Ausgabe-Einrichtung kann auch einen Drucker, bspw. zur Ausgabe einer Hard-Copy, oder ein Diskettenlaufwerk einschließen, um die vom System ausgegebenen Daten zur späteren Verwendung zu speichern.

Im Betrieb koordiniert die CPU die Verwendung der verschiedenen Eingabe- und Ausgabe-Vorrichtungen, den Datenzugriff auf den Massenspeicher und den Zugriff auf den Arbeitsspeicher sowie die Reihenfolge der Datenverarbeitungsschritte. Es steht eine Anzahl von Programmen zur Verfügung, welche der Verarbeitung der maschinenlesbaren Daten der vorliegenden Erfindung verwendet werden können. Derartige Programme sind einem Fachmann bekannt.

Ein maschinenlesbares Speichermedium der vorliegenden Erfindung wird bspw. durch ein magnetisches Speichermedium veranschaulicht, auf welchem die maschinenlesbaren Daten codiert (d.h. abgelegt) sind und das bspw. durch ein vorstehend beschriebenes System erzeugt bzw. gelesen werden kann. Das Medium kann bspw. eine übliche Diskette oder eine Festplatte sein mit einem geeigneten Substrat und einer geeigneten Beschichtung auf einer oder beiden Seiten, welche magnetische Domänen (nicht sichtbar) enthält, deren Polarität oder Orientierung magnetisch veränderlich ist. Das Medium kann auch eine Öffnung zur Aufnahme des Stifts eines Diskettenlaufwerks oder einer anderen Datenspeichervorrichtung aufweisen.

Die magnetischen Domänen der Beschichtung des Mediums sind derart polarisiert oder orientiert, daß sie die in der vorliegenden Anmeldung beschriebenen maschinenlesbaren Daten zum Einlesen, bspw. in das vorstehend beschriebene System, codieren.

Das maschinenlesbare Speichermedium der vorliegenden Erfindung kann auch ein optisches Datenspeichermedium sein. Das Medium kann bspw. eine übliche CD-ROM oder ein wiederbeschreibbares Medium, wie ein magneto-optisches Speichermedium sein, auch bspw. ein DVD-System, die optisch lesbar und magneto-optisch beschreibbar ist. Das Medium weist vorzugsweise ein geeignetes Substrat und eine geeignete Beschichtung auf, die üblicherweise auf einer Seite des Substrats vorgesehen ist.

Im Fall einer CD-ROM reflektiert die Beschichtung Licht, und es ist eine Vielzahl von Kerben in der Beschichtung vorhanden, welche die maschinenlesbaren Daten codieren. Die Anordnung der Kerben wird durch die Reflektion von Laserlicht von der Oberfläche der Beschichtung ausgelesen. Es wird weiterhin eine Schutzschicht vorgesehen, die vorzugsweise im wesentlichen transparent ist, und sich auf der Beschichtung befindet.

Im Fall eines magneto-optischen Speichermediums weist die Beschichtung keine Kerben auf, sondern eine Vielzahl magnetischer Domänen, deren Polarität oder Orientierung magnetisch veränderlich ist, wenn sie auf eine Temperatur oberhalb eines bestimmten Grenzwerts, bspw. durch einen Laser, erhitzt werden. Die Orientierung der Domänen kann durch Messung der Polarisation von Laserlicht, das von der Beschichtung reflektiert wird, ausgelesen werden. Die Anordnung der Domänen codiert die erfindungsgemäßen, maschinenlesbaren Strukturdaten.

Die erfindungsgemäßen Strukturdaten bezüglich der Familie der Proprotein/Prohormon-Convertasen, insbesondere Furin, ermöglicht erstmals die Verwendung von Strukturbasierenden oder sog. "Rational Drug Design"-Techniken der Auswahl, Gestaltung und Synthese chemischer Moleküle oder Gruppen, einschließlich inhibitorisch wirkender Verbindungen, die zur Bindung an ein Protein der PC-Familie, insbesondere Furin, oder einen Teil davon befähigt sind.

Eine besonders geeignete Drug-Design-Technik, die erfindungsgemäß einsetzbar ist, ist, wie bereits vorstehend offenbart, das sog. iterative Drug Design. Das iterative Drug-Design ist ein Verfahren zur Optimierung von Wechselwirkungen zwischen einem Protein und einer Verbindung durch Bestimmung und Evaluierung der dreidimensionalen Strukturen sukzessiver Sätze von Komplexen zwischen Protein und jeweils untersuchter Verbindung.

Es ist im vorliegenden Fachgebiet selbstverständlich bekannt, daß die Assoziierung natürlicher Liganden oder Substrate mit den Bindungstaschen entsprechender Rezeptoren oder Enzyme die Basis jeder biologischer Mechanismen bildet. Der Begriff "Bindungstasche" betrifft erfindungsgemäß einen Bereich eines Moleküls oder eines Molekülkomplexes, der sich aufgrund seiner Struktur bevorzugt mit einer anderen chemischen Einheit oder Verbindung assoziiert. In ähnlicher Weise üben viele Arzneistoffe ihre biologischen Wirkung aufgrund der Assoziation mit der Bindungstasche eines Rezeptors oder Enzyms aus. Derartige Assoziationen können sich auf alle oder irgendeinen Teil der Bindungstasche beziehen. Das Verständnis derartiger Assoziationen bzw. Wechselwirkungen ermöglicht bzw. eröffnet das Design von Arzneistoffen, welche eine verbesserte Wechselwirkung bzw. eine verbesserte Assoziation mit dem Zielrezeptor oder Zielenzym aufweisen und daher verbesserte biologische Wirkungen hervorrufen. Daher sind die erfindungsgemäßen Strukturdaten sehr wertvoll für die Gestaltung potentieller Liganden oder Inhibitoren von Proteinen der PC-Familie, wie Furin, oder Domänen davon.

Der Ausdruck "assoziieren mit bzw. wechselwirken mit" betrifft einen Zustand einer bestimmten Nähe oder Aneinanderlagerung zwischen chemischen Einheiten oder Verbindungen oder Teilen davon. Die Assoziierung kann nicht-kovalent - wobei die Aneinanderlagerung energetisch durch Wasserstoffbrücken oder Van der Wals- oder elektrostatische Wechselwirkungen bevorzugt ist - oder kovalent sein.

Beim iterativen Drug-Design werden Kristalle einer Serie von Komplexen aus Zielproteinen und potenziellem Ligand erhalten und dann dreidimensionale Strukturen des Komplexes gelöst. Ein derartiger Ansatz resultiert in detailierten Informationen über die die Assoziierung zwischen den Proteinen und potenziellen Liganden jedes Komplexes. Es werden Verbindungen mit inhibitorischer Aktivität ausgewählt und Kristalle dieser neuen Protein/Ligand-Komplexe erhalten, deren dreidimensionale Struktur gelöst wird, und es werden die Wechselwirkungen bzw. Assoziationen in den neuen Komplexen und den zuvor gelösten Komplexen verglichen. Indem die Auswirkungen der Veränderungen in der potenziellen Ligandenverbindung auf die Protein/Ligand-Wechselwirkungen verfolgt werden, können diese Wechselwirkungen optimiert werden.

In einigen Fällen wird das iterative Drug-Design derart durchgeführt, daß sukzessive Komplexe aus Protein und potenziellen Liganden gebildet werden und dann jeder neue Komplex kristallisiert wird. In einer anderen Ausführungsform wird ein zuvor hergestellter Proteinkristall in Gegenwart eines Inhibitors "gesoakt", wobei sich ein Protein/Inhibitor-Komplex bildet. Dies vermeidet das Erfordernis, jeden einzelnen Protein/Ligand-Komplex zu kristallisieren. Erfindungsgemäß bezieht sich der Ausdruck "gesoakt" auf ein Verfahren, bei dem der Kristall in eine Lösung überführt wird, welche die interessierende Verbindung enthält.

Die vorliegende Erfindung wird durch die nachfolgenden Figuren näher erläutert.

Figur 1 zeigt die Primärstruktur von murinem Furin (obere Sequenz) und Subtilisin Carlsberg (untere Sequenz). Beim Subtilisin sind alle topologisch nicht-äquivalenten Reste kursiv dargestellt. Identische und ähnliche Reste sind rot bzw. gelb hervorgehoben. Cys-Reste, die Reste des aktiven Zentrums und die zwei glycosylierten Asn-Reste in blau, grün bzw. pink dargestellt. Die "non-prime"-Reste der primären und sekundären Aktivierungsstelle (numerierte Pfeilspitzen) sind mit einem orangefarbenen Hintergrund hinterlegt, und die Signalpeptid-Spaltstelle ist durch die S-markierte Pfeilspitze angedeutet. Die Numerierung der Positionen bezieht sich auf vollständig translatiertes Furin der Maus, wobei die wenigen Substitutionen in der humanen Variante oberhalb der Maus-Sequenz angegeben sind. Unterhalb der Sequenz sind die β-Faltblätter und Helices von Furin durch schwarze Pfeile und Zylinder angezeigt, wie sie erfindungsgemäß beispielhaft für die katalytische und die P-Domäne ermittelt wurden. Grau hinterlegte Pfeile und Zylinder zeigen die entsprechenden vorhergesagten Sekundärstrukturelemente für den C-terminalen Bereich an. Reste des Vollängen-Furins, die in der Elektronendichtekarte nicht sichtbar waren, sind grau hinterlegt.

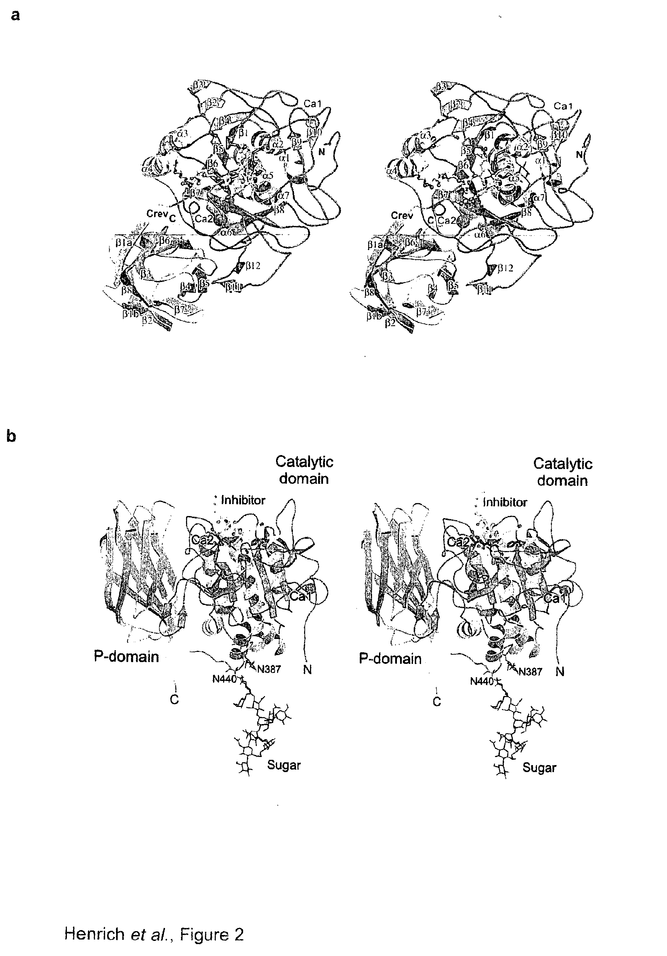

Figur 2 (a/b) ist eine "Ribbon"-Darstellung von löslichem Furin, wobei Helices , β-Stränge und ungeordnete Strukturbereiche der katalytischen Domäne als gelbe Helices, rote Pfeile bzw. dunkelblaue Seilstrukturen dargestellt sind. Die P-Domäne ist in hellblau dargestellt. Die Residuen des aktiven Zentrums (dunkelgrau) und die decanoyl-Arg-Val-Lys-Arg-cmk-Inhibitorreste (hellgrün als Stabmodell) sind mit allen Nicht-Wasserstoffatomen abgebildet. Zwei Calciumatome sind als lila Kugeln dargestellt. Die tiefe Spalte zwischen den Domänen ist als Crev abgekürzt. In Figur 2b ist eine Orientierung der Struktur gezeigt, wobei die N-gebundenen Oligosaccharide eingezeichnet sind. Das Volllängenprotein Furin ist mittels eines Linkers (im unteren Bereich der Darstellung gemäß Figur 2b) an die Membran gebunden.

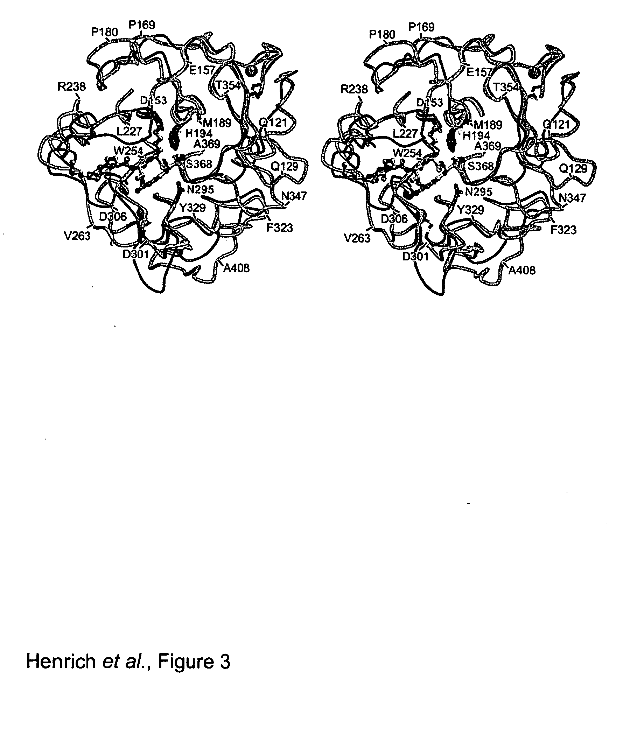

Figur 3 ist eine Stereoansicht und stellt die Faltungsunterschiede zwischen Subtilisin und der katalytischen Domäne von Furin dar. Die Überlagerung der beiden Strukturen (katalytische Domäne von Furin als rotes Seil; Subtilisin Carlsberg als blaues Seil) enthält auch den decanoyl-Arg-Val-Lys-Arg-cmk-Inhibitor, die gebundenen Calcium-Ionen (blau :Subtilisin, lila: Furin) und die Disulfidbrücken der katalytischen Domäne von Furin. Die Orientierung entspricht Fig. 1a.

Figur 4 zeigt die Wechselwirkungen zwischen dem Inhibitor und der Spalte am aktiven Zentrum Die Stereodarstellung enthält die Bindungssituation von Furin mit dem decanoyl-Arg-Val-Lys-Arg-cmk-Inhibitor. Orientierung wie in Figur 1a. Gemäß Figur 4a wird ein Stabmodell des Inhibitors mit den umgebenden AS-Resten von Furin gezeigt (dunkelgrau: C, blau: N, rot: O). Figur 4b verzeichnet den Inhibitor in Anbetracht des elektrostatischen Oberflächenpotentials der katalytischen Domäne. Rote Färbung entspricht negativem (-27 e/kT), blaue Färbung positivem Potential (27 e/kT). Figur 4c entspricht Figur 4a weist aber zudem die Elektronendichtekarte des Inhibitors bei einer Kontourierung von 1σ auf (2_{fobs}f_{calc}).

Figur 5 zeigt die potentielle Interaktion von Furin mit dem Substrat Arg-Arg-Arg-Val-Lys-Arg- -Ser-Leu, das in die Struktur hineinmodelliert wurde (Positionen S6 bis S2'). Die oberfläche ist farblich entsprechend dem Oberflächenpotential der katalytischen Domäne eingefärbt. Die Orientierung ist wie in den anderen Figuren.

Figur 6 enthält den Strukturkoordinatensatz von Furin der Maus.

Die best definierte Kette eines Furinmonomers nach beigefügter Struktur reicht von Asp108 bis Leu578. Im wesentlichen besteht dieses Monomer aus zwei separaten aber angrenzenden Domänen, die sphärische katalytische Domäne (Tyr110 bis Ala445) und die "Barrel"-ähnliche P-Domäne (Pro446 bis Thr573), die miteinander über eine 1100 Å² Schnittstelle interagieren (siehe Fig. 2a). Der Kern der katalytischen Domäne besteht, ähnlich wie bei Subtilisin, aus einem in hohem Maße verdrehten ("Twisted") β-Faltblatt ("β sheet"), das aus sieben parallelen und einem antiparallelen β-Faltblatt-Strang (Cβ1 - Cβ8, Fig. 2) zusammengesetzt ist. Flankiert wird dieses β-Faltblatt von fünf benachbarten (Cα2 bis Cα6) und zwei peripheren Helices (Cα1 und Cα7) und durch zwei β-Haarschleifen ("β-hairpin loop"). Eine Anzahl von ausgedehnten Oberflächenschleifen, von denen mehrere die Tasche des aktiven Zentrums umgeben bzw. charakteristisch formen oder die Wechselwirkung mit der P-Domäne vermitteln, haben durchaus unterschiedliche Längen und Konfirmationen als jene, die bei bakteriellen Subtilisen auftreten (Fig. 3). Die katalytische Domäne von Furin ist kreuzvemetzt durch zwei Disulfidbrücken zwischen Cys211 und Cys360 bzw. Cys303 und Cys333 (Fig. 3). An zwei Stellen enthält diese gebundene Metallionen, bei denen es sich nach Maßgabe der Elektronendichte der Gegenladungen, der Sauerstoffkoordinierung und der Distanzkriterien um Calciumionen handelt (Figuren 2, 3). Calcium 1, umgeben von dem Schleifenabschnitt Ala204 bis Gly212 ist siebenfach koordiniert durch drei Carbonylsauerstoffe und die Carboxylatsauerstoffe von Asp115, Asp162 (2x) und Asn208. Das zweite Calciumion Calcium 2, angeordnet unter der S1-Tasche und koordiniert von den Carboxylatsauerstoffen von Glu331 (2x) Asp258 und Asp301 und drei eingeschlossenen Lösungsmolekülen, existiert bei entfernt strukturähnlichen Proteinen nicht und wurde daher durch Modelling nicht vorhergesagt. Das Calciumion scheint die S1-Tasche zu stabilisieren. Von den drei potentiellen N-gebundenen Oligosaccharidplätzen wurden bis zu elf Zucker durch Elektronendichte an Asn440 und ein Zucker an Asn387 identifiziert (Fig. 2b).

Die benachbarte P-Domäne ist als separater, achtsträngiger ß-Sandwich organisiert. Die Stränge sind vergleichbar den in "Jelly-Roll-ß-Barrels" organisiert, nämlich mit ß-Strängen Pβ1a/b···Pβ8···Pβ3···Pβ6 gegenüber den β-Strängen Pβ2···Pβ7···Pβ4···Pβ5 (Fig. 2). Die geladenen Arg- und Asp-Seitenketten des Arg498-Gly-Asp-Segments, angeordnet an der Pβ3-Pβ4-Schleife (Loop), ragen aus verschiedenen Oberflächenbereichen heraus. Ein hydrophober Kern im Innern ist umgeben von polaren Resten auf der Oberfläche. Das geschlossene P-Barrel zeigt an, daß kürzere Ketten die P-Domäne destabilisieren würden, was der Erkenntnis entspricht, daß Thr573 wesentlich für die Aktivität der P-Domäne ist. Die Außenstränge ("edge strands") Pβ5 und Pβ6 der P-Domäne und die großen katalytischen Domänenschleifen Cβ5-Cα4, Cβ6-Cβ7-Cβ8, Cβ11-Cβ12 ("finger hair pin") sind an den Kontakten zwischen den Domänen beteiligt, was durch den im wesentlichen hydrophoben Kern (Phe528...Val263, Met534...I312, Trp539...Val398) ermöglicht wird, allerdings auch durch eine Vielzahl von polaren Wechselwirkungen bzw. Salzbrücken. Das vermeintlich kompakte, aber unvollständig gefaltete Propeptid im Profurin dürfte an die Spalte zwischen der katalytischen und der P-Domäne binden, so daß es als intramolekularer "Chaperon" wirkt, wobei das Arg-Ala-Lys-Arg107-↓-Asp108 Spaltungssegment (Fig. 1) durch das aktive Zentrum produktiv gebunden wird. So eine Wechselwirkung würde eine teilweise Entfaltung der folgenden zehn N-terminalen Furinreste erforderlich machen, was die erste Calcium-Bindungsstelle zerstören würde.

Die Spalte des aktiven Zentrums von Furin unterscheidet sich erheblich von der von bakteriellen Subtilisinen hinsichtlich deren Tiefe, Form und der Ladungen (Fig. 3). Beim Furin handelt es sich um eine Canyon-ähnliche Spalte, die durch die exponierte Cβ2-Cα2-Schleife und das Cβ4-Cα3-Oberflächensegment auf der einen Seite und durch die Cβ5-Cα4, Cβ6-Cβ7 und Cβ7-Cβ8 Schleifen auf der anderen Seite gebildet wird (Fig. 4), wobei Ser368···His194···Asp153 als Triade des aktiven Zentrums in der Mitte angeordnet sind. Die P1-Arg-Carbonylgruppe des gebundene Decanoyl-Arg-Val-Lys-Arg-Chlormethylketons, die in das Oxyanion-Loch, das durch die Carboxamidstickstoffe von Asn295 und Ser368N gebildet wird, wird kovalent durch das Oγ und die Methylengruppe der tetrahedralen hemiketalen Gruppe (die ein Übergangszustandsintermediat nachahmt) mit Ser368 und His194 (mit P1, P2 ... und P1', P2'... werden die Substratreste N-/C-terminal der zu schneidenden Peptidbindung bezeichnet, gegenüber den Enzymstellen S1, S2 .... und S1', S2'...). Die Peptidylgruppe des Inhibitors liegt neben dem ausgedehnten Ser253-Trp-Gly-Pro-Glu-Abschnitt, der ein verdrehtes antiparalleles β-Faltblatt formt.

Die P1-Arg-Seitenkette reicht in die S1-Tasche durch die Ser253-Asp258 "Eingangsumrahmung" ("entrance frame") (Fig. 4A), die eher so wie Trypsin als wie Subtilisin geknickt ist. Diese Seitenkette ist eingeschlossen zwischen den Abschnitten Ser253-Gly255 und Ser293-Asn295, wobei dessen Guanidylgruppe perfekt zwischen die hydrophoben Gruppen in eine flache Furche, umgeben von den Carboxylaten von Asp258 und Asp306 und den Carbonylen von Ala292 und Pro256, gepackt ist. Jede andere Seitenkette, einschließlich Lys-Seitenkette, würde weniger gut hineinpassen, was im Ergebnis die unbedingte Vorgabe eines Furinsubstrats für P1-Arg erklärt. Die P2-Lysinseitenkette reicht in eine Oberflächenspalte hinein, wobei deren α-Ammoniumgruppe von Sauerstoffen des Asp154-Carboxylats, des Asn192-Carboxamids und des Asp191-Carbonyls für eine gute Wasserstoffbrückenbindung umgeben ist (Fig. 4). Derart sind die Geometrie und die Ladung der S2-Stelle vorteilhaft für die Aufnahme von Lysin, schließen aber auch eine P2-Arg-Seitenkette nicht aus. Die P3-Val-Seitenkette reicht in das "Bulk"-Lösungsmittel hinein, was der Sequenzbeliebigkeit an dieser Position entspricht (Fig. 4). Die Seitenketten eines alkalischen P3-Rests könnten gute Kontakte mit dem an der Oberfläche angeordneten G1u257-Carboxylats machen, wodurch lange basische P3-Seitenketten bevorzugt sein könnten - in Übereinstimmung mit der regelmäßigen Präsenz von alkalischen P3-Resten bei In-vivo-Substraten von Furin. Die geknickte P4-Arg-Seitenkette reicht in einen Spalt hinein, wobei deren Guanidylgruppe vorteilhafterweise durch die Carboxylatgruppen von Glu236 und Asp264 eingerahmt wird und gegen Trp254 und Tyr 308 gepackt ist (Fig. 4, 5). Diese komplementäre Packungsweise bevorzugt die Aufnahme von P4-Arg Seitenketten, in Übereinstimmung mit der starken Präferenz des Furins für Arg und schwächer Lys-P4-Resten, erlaubt jedoch auch die Besetzung mit anderen Seitenketten.

Aufgrund von Modellberechnungen erscheint eine ausgedehnte Konfirmation für P5-P6-Reste wahrscheinlich (Fig. 5). Entsprechend würden die P5-Seitenketten entlang der Cβ5-Cα4-Schleife über Glu257 laufen, während die P6-Seitenketten entweder zwischen den an der Oberfläche angeordneten Resten Glu230 und Asp233 interkalieren oder im Falle von kurzen P5-Resten sich in die S4-Tasche gegenüber den P4-Seitenketten hineinwinden könnten. Diese Vorstellung ist mit der Beobachtung konsistent, daß die Mehrheit der Furinsubstrate alkalische Reste entweder bei P4 oder P6 aufweisen. Die positiv geladene S1'-Stelle scheint dazu geeignet, vorzugsweise polare/negativ geladene P1'-Seitenketten und die S2'-Stelle hydrophobe mittelgroße Reste aufzunehmen (Fig. 5), in Übereinstimmung mit der geforderten Abwesenheit von Lysin-Resten an P1' und P2'.

Derart wird die Substratbindung stark von ausgedehnten elektrostatischen Wechselwirkungen durch die 18 vereinten, negativ geladenen Reste um die "Non-Prime" Bindungsregion von Furin dominiert. Während die S1 plus S2 plus S4 Stellen so gestaltet sind, daß sie Arg- und Lys-Reste aufnehmen können, sind P3-, P5- und P6- (und sogar P7- und P8-) Reste auf ein negatives Oberflächenpotenzial ausgerichtet, in Übereinstimmung mit der Präferenz von Furin für polybasische Peptidsegmente (Fig. 5). Weniger positiv geladene Peptidsubstrate dürften, entsprechend nicht ausreichender Ladungskompensation, in eine weniger geeignete Positionierung für die Spaltung gezwungen werden, was eine ungünstige Präsentation der P1-P1' Peptidbindung an die katalytisch wirkende Gruppe Ser368Oγ hervorruft. Die polare Ladungsverteilung über das aktive Zentrum sollte dazu beitragen, die anstehenden Peptidsegmente vorab zu orientieren und in die richtige Position zu bringen.

Die vorliegende Erfindung wird durch die nachfolgenden Ausführungsbeispiele näher erläutert.

### 1. Protein- und Peptidpräparation

### Herstellung rekombinanter Convertase

Der murine Furin-Clon wurde von K. Nakayama (Fukuoka University School of Medicine, Fukuoka, Yapan) erhalten. Die murine Furin-cDNA wurde N-terminal zur Transmembrandomäne ab His711 mit Hilfe der Polymerase-Kettenreaktion (PCR) gekürzt. Dieses PCR-Produkt wurde dann in pcDNA3.1(-)-Myc-His (Invitrogen) über die *Nbe*I- und *Xba*I-Restriktionsschnittstellen subcloniert. Dihydrofolatreduktase-negative DG44 CHO-Zellen (erhalten von L. Chasin, Columbia University, New York, NY, USA) wurden unter Verwendung von Lipofectin (Life Technologies Inc.) transfiziert, und die Kolonien wurden bei 37°C und 5% CO₂ in alpha-minimal essentiellem Medium (ohne Nukleoside), enthaltend 10% ausgiebig dialysiertes fötales Kälberserum (Life Technologies, Inc.), selektioniert. Konditioniertes Koloniemedium wurde unter Verwendung eines enzymatischen Tests (nachstehend beschrieben) gescreent und Klone mit hoher Expression wurden ausgewählt. Eine Überexpression von Furin wurde durch Erhöhen der Methotrexat-Konzentration von 5 nM auf 50 µM in Fünfer- bis Zehnerschritten, wie bei Lindberg et al. (1995) Methods Neurosci. 23: 94 - 108, beschrieben, erhalten. Amplifizierte Zelllinien wurden hinsichtlich einer ansteigenden Furin-Expression durch den Enzymtest untersucht. Nachdem die Methotrexat-Konzentration von 50 µM erreicht war, wurden die Zellen zweimal in der Woche in Verhältnissen von 1:6 passagiert. 100 ml konditioniertes Medium (Opti-MEM (Life Technologies, Inc.)), enthaltend 100 µg/ml Aprotinin (Miles Laboratories, Kankakee, IL, USA)) wurden konfluenten Zellkultur-Rollflaschen alle 24 h entnommen. Das Medium wurde bei geringer Geschwindigkeit zum Entfernen von Zellen zentrifugiert, und der Überstand wurde bei -80°C zur weiteren Verwendung gelagert.

Der Enzymtest bezüglich der Furin-Aktivität wurde im wesentlichen wie in Abletalina et aL (1998) J. Biol. Chem. 273: 26589 - 26595, beschrieben, unter Verwendung des Substrats pERTKR-MCA bei pH 7,0 in 100 mM HEPES, 5 mM CaCl₂ und 0,1% Brij 35 durchgeführt.
Alle Tests wurden bei 37°C in einem 96-Well-Fluorimeter (Lab Systems Inc.) bei einer Anregungswellenlänge von 380 nm und einer Emissionswellenlänge von 460 nm durchgeführt. Das Gesamtvolumen betrug 50 µl. Die Endkonzentration des Substrats betrug bei den Test im allgemeinen 200 µM Alle Tests wurden als Doppel- oder Dreifachbestimmungen durchgeführt.

### Aufreinigung von Furin

Konditioniertes Medium wurde aufgetaut, gesammelt und 1:3,5 mit Puffer A (20 mM HEPS, 0,1 % Brij 35 und 5 mM CaCl₂, pH 7,4) verdünnt und bei 40 ml/min durch eine D 100 Anionenaustauschermembran (Sartorius, Göttingen, Deutschland) gepumpt. Die Membran wurde mit 40 ml Puffer A, gefolgt von 40 ml Puffer A, enthaltend 50 mM NaCl, und schließlich mit 50 ml Puffer A, enthaltend 200 mM NaCl, gewaschen. Die mit 200 mM NaCl eluierende Fraktion wurde 1:4 mit Puffer A verdünnt und auf eine 1 ml Mono Q HR5/5 Anionenaustauschersäule (Amersham Pharmacia Biotech) bei einer Flußrate von 1 ml/min aufgetragen. Nach dem Waschen mit 10 ml Puffer A wurde Furin durch einen linearen Gradienten von 0 bis 500 mM NaCl in Puffer A über 30 ml eluiert, wobei Fraktionen von 2 ml gesammelt wurden. Die Peak-Fraktionen mit erhöhter Furin-Aktivität wurden gesammelt und Aliquots von 200 µl wurden einer Gelpermeationschromatographie unter Verwendung einer Superose-12-Säule (Amersham Pharmada Biotech) unterworfen, wobei Puffer A, enthaltend 200 mM NaCl, bei einer Fließgeschwindigkeit von 0,5 ml/min verwendet wurde. Gesammelte Fraktionen wurden hinsichtlich der Furin-Aktivität, wie vorstehend beschrieben, getestet. Der Gesamtproteingehalt wurde mit Hilfe der Bradford-Methode bestimmt. Alle Reinigungsschritte wurden bei 4°C durchgeführt.

Alternativ wurde die enzymhaltige Fraktion, welche von der Ionenaustauschmembran mit 200 mM NaCl eluierte, auf eine 1 ml Ni²⁺-Nitrilotriessigsäure-Superflow-Säule (QIAGEN Inc.) bei 0,3 ml/min aufgetragen, wonach mit 10 ml Puffer A gewaschen und dann mit einem zweistufigen Gradienten von 0 bis 200 mM Imidazol in Puffer A über 20 ml, gefolgt von einem linearen Gradienten von 20 bis 200 mM Imidazol in Puffer A, eluiert wurde. Die Peak-Fraktionen mit erhöhter Enzymaktivität wurden gesammelt und der Ionenaustauschchromatographie mit einer Mono-Q-Säule, wie vorstehend beschrieben, unterworfen.

### 2. Kristallisierung

Die Kristalle des Polypeptids (C-terminal verkürztes, lösliches Furin der Maus), erhalten gemäß Ausführungsbeispiel 1 wurden der Kristallisierung unterzogen, wobei zur Inaktivierung nach Aufreinigung Chloromethylketon-Inhibitor (decanoyl-Arg-Val-Lys-Arg-CMK) hinzugegeben wurde. Es wurden gleiche Volumina von 10 mg/ml Proteinlösung und des Präzipitierungspuffers (1,0 M (NH₄)₂SO₄, 0,4 M Li₂SO₄, 0,1 M Na₃Citrat, pH 6 mit/ohne 1,5-Pentandiol) gemischt und bei Raumtemperatur gegenüber dem Präzipitierungspuffer äquilibriert. Hierdurch wurden trikline und hexagonale Kristalle erhalten. Die triklinen Kristalle der Raumgruppe P1 weisen die Zellkonstanten a=93,3 Å, b= 135,4 Å, c= 137,8 Å; α = 103,6°, β=99,0°, γ = 107,1° auf.

Nach Zugabe von 1,5-Pentandiol zum Prazipitierungspuffer wurde eine zweite (hexagonale) Raumform erhalten (P6₅), die große Zellkonstanten aufwies: a=b=135,5 Å, c=472,0 Å, α = β = 90°, γ = 120° und hohe Mosaizität zeigte.

Um die hohe Mosaizität der hexagonalen Kristalle zu reduzieren und damit das schlechte Diffraktionsmuster (insbesondere nach Gefrierschock in flüssigem Stickstoff zu verbessern, wurden die Kristalle in Mineralöl überführt und in einen Kryo-"Loop" eines "free-mounting-Systems" gesetzt (s. insbesondere Cameron et al. J. Biol. Chem. 275 (47), 36741 oder Kiefersauer et aL J. Appl. Crystallography 33 (part 5) 1223). Luftstrom definierter Feuchtigkeit wurde dem Kristall zugeführt und Diffraktionsbilder wurden aufgenommen. Die Einstellung der Luftfeuchtigkeit auf 81% verbesserte das Diffraktionsmuster erheblich. Dieser Transformationsprozeß wurde durch Einfrieren des Kristalls in einem Kryo-Stickstoffstrom beendet.

### 3. Datensammlung, Strukturbestimmung und Strukturverfeinerung

2,6/2,7 Å kristallographische Daten wurden direkt (für die triklinen Kristalle, die in einem Kryo-Loop montiert wurden und bei 100 K in einem Kryostrom schockgeroren wurden) gesammelt oder nach Transformation bei 81% relativer Feuchtigkeit (im Falle der hexagonalen Kristalle, siehe Kiefersauer et aL: AppL Kristallogr. 33, 1223 - 1230, 2000), aus schockgefrorenen Kristallen bei 100 K bei BW6/DORIS/Hamburg, unter Verwendung eines MarCCD-Detektors und Mar345-Bildplattendetektoren erhalten. Im Falle der triklinen Kristalle wurde ein Datensatz unter Verwendung einer 165 mm Mar CCD-Detektors (Mar-USA, Evanston, IL) gesammelt.

Alle Daten wurden prozessiert und skaliert unter Verwendung des HKL Pakets (Otwinowski & Minor, Methods EnzymoL 276, 307 - 326, 1997)und MOSFLM/SCALA. Experimentelle 4,0 Ä Phaseninformationen wurden für die hexagonalen Kristalle aus MAD-Experimenten (multiple anisomorphe Diffraktionsdaten) (drei Wellenlängen) unter Verwendung von neun Ta₆Br₁₂²⁺ Cluster-Plätzen (in derivatisierten hexagonalen Kristallen), bei Verwendung von MLPHARE und DM (CCP4, Acta Cristallographica D50, 760 - 763, 1994) erhalten. Nach der Phasenerweiterung bis 2,7 Å durch 8-faches zyklisches "density averaging"/"solvent flattening" (CCP4/DM, Acta Cristallographica D50, 760 - 763, 1994) wurde ein erstes Furin-Modell mit MAIN (Turk, Weiterentwicklung eines Programms für Molekülgraphik und Elektrondichte Manipulation und seine Anwendung auf verschiedene Protein-Strukturaufklärungen, Dissertation, Technische Universität München, 1992) gebaut und mit CNSv1.1 (Brünger et aL, Acta Crystallographica, D54, 1905 - 1921, 1998) unter Verwendung starker NCS-Randbedingungen verfeinert. Dieses Modell wurde verwendet, um mit AMORE (CCP4, siehe oben) die Orientierung in den trikllnen Kristallen zu bestimmen. Die trikline Struktur wurde verfeinert bei 2,6 Å bis zu einem R-Faktor/R_{free} von 18,8/21,9% für das Endmodell. Weitere Einzelheiten der Struktur sind den beigefügten Tabellen 1 (Statistik der Datenaufnahmen) und 2 (Statistik der Verfeinerung der triklinen Kristalle) zu entnehmen.

Zur Messung der langen Achse (c-Achse) der hexagonalen Kristalle wurden entsprechende Vorkehrungen getroffen. Daher wurde die c-Achse parallel zur Spindelachse ausgerichtet, wofür ein besonderer Goniometerkopf eingesetzt wurde. Die Beugungsreflexe nahe des Strahlzentrums (niedrige Auflösung) wurde mit einem CCD-Detektor im Abstand von 285 mm gemessen. Die Hochauflösungsreflexe im Außenbereich wurde bei einem Abstand von 800 mm gesammelt. Um alle Reflexe bis zu einer Auflösung von 2,7 Å sammeln zu können, wurden die "image plate" (Mar345-Bildplattendetektoren) aus dem Strahl in drei verschiedenen Positionen gerückt. Die drei Datensätze wurden dann mit dem Datensatz des CCD-Detektors (der auf Grund seiner Größe die Auflösung bei 3,9 Å begrenzte) zusammengeführt.

Der gesamte Offenbarungsgehalt sämtlicher in der vorliegenden Anmeldung zitierten Veröffentlichungen, Patente, Patentanmeldungen und anderweitigen Dokumente ist durch Bezugnahme ausdrücklich in die Offenbarung der vorliegenden Erfindung in gleicher Weise aufgenommen, als wenn jedes einzelne Dokumente durch Bezugnahme aufgenommen wäre.

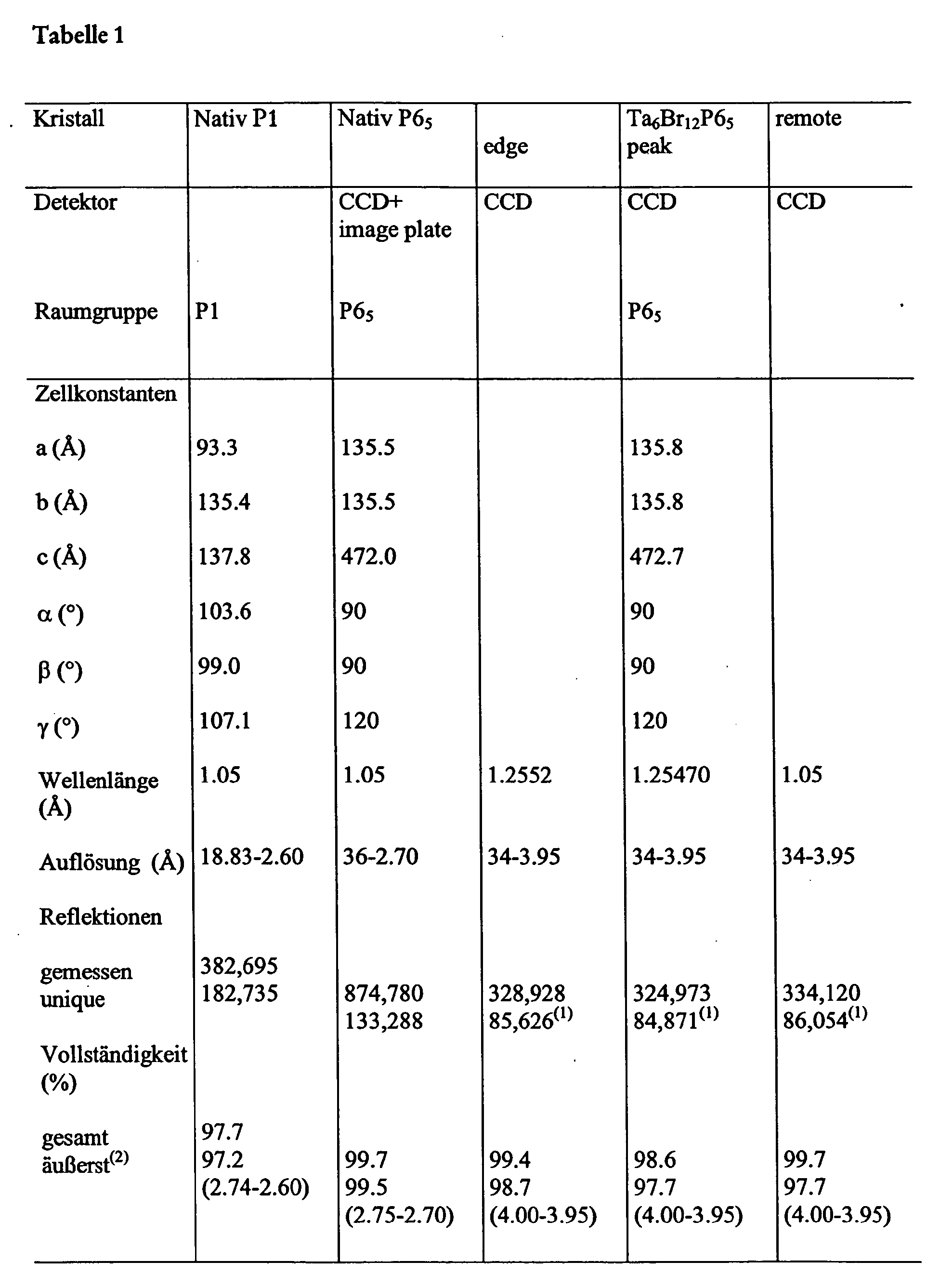

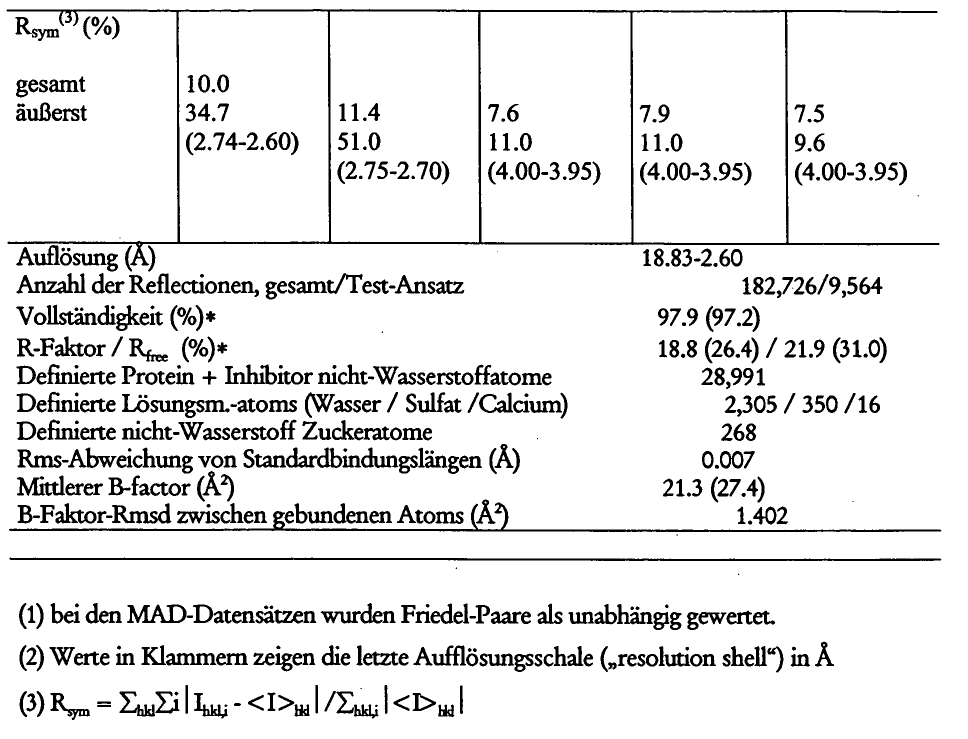

**Tabelle 2**

| | |
|---|---|
| Auflösung (Å) | 18.83-2.60 |
| Anzahl der Reflectionen, gesamt/Test-Ansatz | 182,726/9,564 |
| Vollständigkeit (%)* | 97.9 (97.2) |
| R-Faktor / R_{free} (%)* | 18.8 (26.4) / 21.9 (31.0) |
| Definierte Protein + Inhibitor nicht-Wasserstoffatome | 28,991 |
| Definierte Lösungsm.-atoms (Wasser / Sulfat /Calcium) | 2,305 / 350 /16 |
| Definierte nicht-Wasserstoff Zuckeratome | 268 |
| Rms-Abweichung von Standardbindungslängen (Å) | 0.007 |
| Mittlerer B-factor (Å²) | 21.3 (27.4) |
| B-Faktor-Rmsd zwischen gebundenen Atoms (Å²) | 1.402 |

| | |
|---|---|
| * Werte der letzten Auflösungschale ("resolution shell") (2.60-2.76 Å) sind in Klammern. Berechnet anhand von 5.2 % der Reflektionen, ausgewählt in "thin shells" und bei der Verfeinerung weggelassen. Wilson-Plot-B-Faktor in Klammern. | |

## Patentansprüche

1. Raumform eines Polypeptids, **dadurch gekennzeichnet, daß** das Polypeptid in der Raumform mindestens eine mindestens 50 Aminosäuren umfassende Aminosäuresequenz eines Proteins (Teilsequenz) aus der Familie der PCs oder ein Derivat einer solchen Aminosäuresequenz enthält.

2. Raumform eines Polypeptids nach Anspruch 1, **dadurch gekennzeichnet, daß** das Polypeptid in der Raumform mindestens eine mindestens 50 Aminosäuren umfassende Sequenz eines Proteins, ausgewählt aus der Gruppe, bestehend aus PC1/PC3, PC2, PACE4, PC4, PC5/PC6, PC7/PC8/LPC/SPC7 und Furin, enthält.

3. Raumform eines Polypeptids nach Anspruch 1 oder 2, **dadurch gekennzeichnet, daß** das Polypeptid in der Raumform mindestens eine mindestens 80, vorzugsweise mindestens 100, stärker bevorzugt mindestens 120, noch stärker bevorzugt mindestens 150 Aminosäuren unfassende Aminosäuresequenz eines Proteins oder Derivats/e aus der Familie der PCs enthält.

4. Raumform eines Polypeptids nach einem der vorgenannten Ansprüche 1 bis 3, **dadurch gekennzeichnet, daß** das Polypeptid in der Raumform mindestens eine Aminosäuresequenz einer katalytischen Domäne eines Proteins aus der Familie der PCs enthält, insbesondere den Teilabschnitt von AS 110 (Tyr) bis AS 445 (Ala) im Falle murinen Furins oder entsprechend analoge Abschnitte bei Isoformen von Furin anderer Organismen, insbesondere der humanen Isoform.

5. Raumform eines Polypeptids nach einem der vorgenannten Ansprüche 1 bis 4, **dadurch gekennzeichnet, daß** die in dem Polypeptid enthaltene mindestens eine Aminosäuresequenz eines Proteins aus der Familie der PCs eukaryotischen Ursprungs ist.

6. Raumform eines Polypeptids nach einem der vorgenannten Ansprüche 1 bis 5, **dadurch gekennzeichnet, daß** die in dem Polypeptid enthaltene mindestens eine Aminosäuresequenz eines Proteins aus der Familie der PCs humanen Ursprungs ist.

7. Raumform eines Polypeptids nach einem der vorgenannten Ansprüche 1 bis 6, **dadurch gekennzeichnet, daß** das Polypeptid die Aminosäuresequenz eines eukaryotischen Furins oder eines Abschnitts oder Derivats derselben aufweist.

8. Raumform eines Polypeptids nach einem der vorgenannten Ansprüche 1 bis 7, **dadurch gekennzeichnet, daß** die Raumform eine weitere Verbindung aufweist.

9. Raumform eines Polypeptids nach Anspruch 8, **dadurch gekennzeichnet, daß** die Raumform eine weitere Verbindung aufweist, die als Ligand an das Polypeptid, so wie in einem der Ansprüche 1 bis 7 gekennzeichnet, bindet.

10. Raumform eine Polypeptids nach Anspruch 8 oder 9, **dadurch gekennzeichnet, daß** die Verbindung oder der Ligand ein nicht physiologisch auftretendes Molekül ist.

11. Raumform eines Polypeptids nach einem der vorgenannten Ansprüche 1 bis 10, **dadurch gekennzeichnet, daß** die Raumform ein Polypeptid, wie in den Ansprüchen 1 bis 7 gekennzeichnet, und mindestens einen physiologischen Liganden oder mindestens einen Abschnitt eines physiologischen Liganden für das Polypeptid aufweist.

12. Raumform eines Polypeptids nach einem der vorgenannten Ansprüche 1 bis 11, **dadurch gekennzeichnet, daß** die Raumform ein Polypeptid und mindestens einen Liganden oder einen Abschnitt eines Liganden für das Polypeptid aufweist, wobei der Ligand an die katalytische Domäne oder an die P-Domäne oder an die Schnittstelle der beiden Domänen des Polypeptids bindet.

13. Raumform eines Polypeptids nach einem der vorgenannten Ansprüche 8 bis 12, **dadurch gekennzeichnet, daß** der Ligand ein Polypeptid, Oligopeptid, Dipeptid oder ein synthetisch modifiziertes Derivat eines Poly-, Oligo- oder Dipeptids ist, insbesondere ein bezüglich der Amidbindung modifiziertes Poly-, Oligo- oder Dipeptid ist, vorzugsweise mindestens an der zu spaltenden Amidbindung chemisch modifiziert ist, insbesondere so modifiziert ist, dass keine endoproteolytische Spaltung der Peptidbindung möglich ist.

14. Raumform eines Polypeptids nach einem der vorgenannten Ansprüche 8 bis 13, **dadurch gekennzeichnet, daß** der Ligand eine Aminosäuresequenz oder einen Abschnitt einer Aminosäuresequenz eines Proteins aus der Klasse der endoproteolytisch von den Proteinen der Familie der PCs gespaltenen Zielsubstrate oder ein Derivat eines/r solchen Aminosäuresequenz oder eines solchen Abschnitts enthält.

15. Raumform eines Polypeptids nach einem der vorgenannten Ansprüche 8 bis 14, **dadurch gekennzeichnet, daß** der Ligand ausgewählt ist aus Vollängensequenzen oder Teilsequenzen, ausgewählt. aus den folgenden physiologischen Zielsubstraten, bestehend aus der Gruppe: Pro-β-NGF, Pro-BDNF, Pro-NT-3, Pro-TGF-β1,Pro-Müller-Inhibitor-Substanz, Pro-IGF-1, Pro-Endothelin-1, Parathormon-verwandtes Propeptid, Pro-Parathormon, Insulin-Prorezeptor, Hepatocyten-Wachstumsfaktor-Prorezeptor, Pro-LRP, Integrin-α3-Kette, Integrin-α6-Kette, Proalbumin, Komplement-pro-C3, Profaktor IX, Profaktor X, Pro-von-Willebrand-Faktor, Proprotein C, Stromelysin 3, MT-MMPI, HIV gp160, CMV Glycoprotein B, MMTV-7 Superantigen, Hühnerpest-Virus A Hämagglutinin, Masern-Virus F₀, NDV F₀, Sindbis-Virus gpE2, Parainfluenza-Typ-3-Virus F₀, Anthrax-Toxin-protektives Antigen, Diphtherie-Toxin, *Pseudomonas*-Endotoxin A, Shiga-Toxin, Pro-Furin, Proendopeptidase 3.4.24.18 und Pro-7B2.

16. Raumform eines Polypeptids nach einem der vorgenannten Ansprüche 8 bis 15, **dadurch gekennzeichnet, daß** der Ligand ausgewählt ist aus der Gruppe, bestehend aus: acyliertem Arg-Xaa-Lys/Arg-Arg-Chlormethan, α₁-PIT, α₁-PDX, einer Variante der dritten Domäne von Ovomucoid, bei welcher die Sequenz des reaktiven Zentrums Ala-Cys-Thr-Leu durch Arg-Cys-Lys-Arg ersetzt ist, nicht-acetylierten Poly-D-Argininen und einer vom Propeptid von Furin abgeleiteten Sequenz, insbesondere mit dem Motiv QQVAKRRTKR.

17. Raumform eines Polypeptids nach einem der vorgenannten Ansprüche 8 bis 16, **dadurch gekennzeichnet, daß** der Ligand ein Inhibitor der physiologischen Funktion, insbesondere der proteolytischen Funktion, eines Proteins der Familie der PCs ist.

18. Raumform eines Polypeptids nach einem der vorgenannten Ansprüche 8 bis 17, **dadurch gekennzeichnet, daß** der Ligand ein Inhibitor des Typs Decanoyl-Arg-Xaa-Lys/Arg-Arg-Chlormethylketon ist.

19. Raumform eines Polypeptids nach einem der vorgenannten Ansprüche 1 bis 18, **dadurch gekennzeichnet, daß** die Raumform des Polypeptids und ggf. mindestens einer weiteren Verbindung eine Kristallform ist.

20. Raumform eines Polypeptids als Kristallform nach Anspruch 19, **dadurch gekennzeichnet, daß** die Kristallform Metall-Ionen, insbesondere Schwermetall-Ionen, enthält.

21. Raumform eines Polypeptids als Kristallform nach Anspruch 19 oder 20, **dadurch gekennzeichnet, daß** die Kristallform des Polypeptids einen mindestens 50 Aminosäuren umfassenden Sequenzabschnitt des Proteins Furin gemäß Fig. 6 mit den dort wiedergegebenen Strukturkoordinaten der entsprechenden Atome dieses Sequenzabschnitts enthält.

22. Raumform eines Polypeptids als Kristallform nach einem der Ansprüche 19 bis 21, **dadurch gekennzeichnet, daß** die Kristallform des Polypeptids einen mindestens 50, bevorzugt mindestens 80, mehr bevorzugt mindestens 100, stärker bevorzugt mindestens 120, noch stärker bevorzugt mindestens 150 Aminosäuren umfassenden Sequenzabschnitt aus der katalytischen Domäne eines Proteins aus der Familie der PCs, insbesondere mit den Strukturkoordinaten für Furin, wie in Fig. 6 wiedergegeben, enthält.

23. Kristall, enthaltend pro asymmetrischer Einheit in der Einheitszelle des Kristalls mindestens ein Polypeptid und ggf. mindestens eine weitere Verbindung, **dadurch gekennzeichnet, daß** die Polypeptide im Kristall eine Raumform nach einem der Ansprüche 19 bis 22 einnehmen.

24. Kristall nach Anspruch 23, **dadurch gekennzeichnet, daß** die Raumgruppe des die Kristallform aufweisenden Kristalls monoklin, tetragonal, orthorhombisch, kubisch, triklin, hexagonal oder trigonal/rhombohedral ist.

25. Kristall nach Anspruch 23 oder 24, **dadurch gekennzeichnet, daß** die Raumgruppe des eine Kristallform nach einem der Ansprüche 19 bis 22 aufweisenden Kristalls P1 ist.

26. Kristall nach einem der Ansprüche 23 bis 25, **dadurch gekennzeichnet, daß** die Einheitszelle des die Kristallform enthaltenden Kristalls Zellkonstanten von ungefähr a = 93,3 Å, b = 135,4 Å, c = 137,8 Å und α = 103,6°, β = 99,0° und γ = 107,1° aufweist.

27. Verfahren zur Herstellung eines Kristalls mit Einheitszellen, enthaltend in der asymmetrischen Einheit mindestens eine Raumform eines Polypeptids und ggf. mindestens einer weiteren Verbindung, nach einem der Ansprüche 24 bis 27, **dadurch gekennzeichnet, daß**
(a) das Polypeptid in einem Expressionssystem überexprimiert wird,
(b) das überexprimierte Polypeptid gereinigt und aufkonzentriert wird,
(c) das gemäß (b) erhaltene Polypeptidkonzentrat in einem geeigneten Puffersystem, ggf. unter Hinzufügen mindestens einer weiteren Verbindung, gelöst wird und
(d) die Kristallisierung, bspw. durch (Dampf-)Diffusionsverfahren, eingeleitet wird.

28. Verbindung mit der Eigenschaft, als Ligand an Strukturbereiche eines Proteins aus der Familie der PCs oder strukturell mit einem Protein aus der Familie der PCs verwandter Proteine, insbesondere PC1/PC3, PC2, PACE4, PC4, PC5/PC6, PC7/PC8/LPC/SPC7 oder Furin, zu binden, **dadurch gekennzeichnet, daß** die Verbindung nicht-kovalente Wechselwirkung mit der Hauptkette und/oder den Seitenketten von Aminosäuren, die Bestandteil einer der katalytischen Domäne eines Proteins aus der Familie der PCs oder eines strukturell mit einem Protein aus der Familie der PCs verwandten Proteins sind, eingeht.

29. Verbindung nach Anspruch 28, **dadurch gekennzeichnet, daß** die Verbindung als Inhibitor der physiologischen Funktion, insbesondere der Endoprotease-Funktion, eines Proteins aus der Familie der PCs, insbesondere PC1/PC3, PC2, PACE4, PC4, PC5/PC6, PC7/PC8/LPC/SPC7 und/oder Furin, wirkt.

30. Verbindung nach Anspruch 28 oder 29, **dadurch gekennzeichnet, daß** die Verbindung mit Aminosäuren der katalytischen Domäne eines Proteins aus der Familie der PCs, insbesondere PC1/PC3, PC2, PACE4, PC4, PC5/PC6, PC7/PC8/LPC/SPC7 und/oder Furin, in Wechselwirkung tritt.

31. Verbindung nach einem der Ansprüche 28 bis 30, **dadurch gekennzeichnet, daß** die Verbindung mit mindestens einer der Aminosäuren Ser368, His194, Asp153, Asn295, Ser253 bis Asp258, Ser293, Asp306, Ala292, Pro256 Asp154, Asp191, Glu236, Asp264, Trp254 und Tyr308 von Furin oder der an den gemäß Figur 6 entsprechenden Positionen befindlichen Aminosäuren aus katalytischen Domänen von mit Furin verwandten Proteinen (bspw. andere Proteine aus der Familie der PCs oder Furine unterschiedlichen Ursprungs) in Wechselwirkung tritt.

32. Verbindung nach Anspruch 31, **dadurch gekennzeichnet, daß** die Verbindung mit mindestens einer der Aminosäuren Ser368, His194 und Asp153 von Furin oder der an den gemäß Figur 6 entsprechenden Positionen befindlichen Aminosäuren aus katalytischen Domänen von mit Furin verwandten Proteinen (bspw. andere Proteine aus der Familie der PCs oder Furine unterschiedlichen Ursprungs) in Wechselwirkung tritt.

33. Verbindung nach einem der Ansprüche 28 bis 32, **dadurch gekennzeichnet, daß** die Verbindung ein modifiziertes oder unmodifiziertes Di- oder Oligopeptid oder ein Peptidomimetikum eines solchen Di- oder Oligopeptids ist.

34. Verbindung nach einem der Ansprüche 28 bis 33, **dadurch gekennzeichnet, daß** die Verbindung ein Oligopeptid ist, das die native Aminosäuresequenz eines Zielsubstrats von Proteinen aus der Familie der PCs, insbesondere von PC1/PC3, PC2 oder Furin, enthält.

35. Verbindung nach einem der Ansprüche 28 bis 34, **dadurch gekennzeichnet, daß** die Verbindung ein Oligopeptid ist, das die Aminosäuresequenz mit dem Motiv RXXR, insbesondere RX(K/R)R, oder einem dazu ähnlichen Motiv enthält, wobei X für eine beliebige, natürlich auftretende Aminosäure steht.

36. Verbindung nach einem der Ansprüche 28 bis 35, **dadurch gekennzeichnet, daß** die Verbindung ein Oligopeptid ist, das die Aminosäuresequenz mit dem Motiv (K/R)RX(K/R)R oder einem dazu ähnlichen Motiv enthält, wobei X für eine beliebige, natürlich auftretende Aminosäure steht.

37. Verbindung nach einem der Ansprüche 34 bis 35, **dadurch gekennzeichnet, daß** die Verbindung ein Oligopeptid ist, das die Aminosäuresequenz VVQREKR, THNRTKR, IENRKRR, SKKREKR, SSRRHKR, GGRRQRR, SSGRSKR, oder TDPRTKR enthält.

38. Verbindung nach einem der Ansprüche 28 bis 35, **dadurch gekennzeichnet, daß** die Verbindung ein Oligopeptid ist, das die Aminosäuresequenz SNSRKKR, AGNRVRR, TRHRQPR, HASRVAR oder AKRRTKR enthält.

39. Verwendung einer Verbindung oder eines Polypeptids nach einem der Ansprüche 28 bis 38 zur Herstellung eines Arzneimittels.

40. Verwendung einer Verbindung nach Anspruch 39 zur Herstellung eines Arzneimittels zur Behandlung von Tumorerkrankungen, zur Immunsuppression, insbesondere von Autoimmunerkrankungen oder anderen Entzündungserkrankungen, GvHD oder zur Behandlung von bakteriellen Infektionserkrankungen oder Virusinfektionen, insbesondere AIDS oder Influenza.

41. Verfahren zur Identifizierung einer Verbindung mit der Eigenschaft, als Inhibitor der physiologischen Funktion, insbesondere der Endoprotease-Funktion, eines Proteins aus der Familie der PCs, insbesondere Furin, zu wirken, **dadurch gekennzeichnet, daß**
(a) eine Raumform nach einem der Ansprüche 1 bis 22 durch Strukturaufklärung erhalten wird,
(b) die Struktur der Raumform mit Hilfe der Strukturkoordinaten dreidimensional dargestellt wird,
(c) sterische Eigenschaften und/oder funktionelle Gruppen einer Verbindung so gewählt werden, daß Wechselwirkungen zwischen der Verbindung und der Haupt- und/oder den Seitenketten des Polypeptids in dem Bindungsbereich ausgebildet oder ggf. optimiert werden.

42. Verfahren nach Anspruch 41, **dadurch gekennzeichnet, daß** in einem Verfahrensschritt (d) die dreidimensionale Struktur der gemäß (c) erhaltenen Verbindung ermittelt und in die Polypeptidstruktur modelliert wird.

43. Verfahren nach Anspruch 41 oder 42, **dadurch gekennzeichnet, daß** in einem Verfahrensschritt (e) die Stärke der Wechselwirkung zwischen der Verbindung und dem aufgefalteten Polypeptid gemäß einer Raumform nach einem der Ansprüche 1 bis 22 bestimmt wird.

44. Verfahren nach einem der Ansprüche 41 bis 43, **dadurch gekennzeichnet, daß** gemäß Verfahrensschritt (b) alle oder ein Teil der Strukturkoordinaten aus Fig. 6 dreidimensional dargestellt werden.

45. Verfahren nach einem der Ansprüche 41 bis 44, **dadurch gekennzeichnet, daß** die Verfahrensschritte (c), (d) und (e) zyklisch so lange wiederholt werden, bis die gemäß
(e) erhaltene Stärke der Wechselwirkung zwischen der Verbindung und der Polypeptidstruktur in einer Raumform mit einer Struktur gemäß einem der Ansprüche 1 bis 22 optimiert wird.

46. Verfahren nach einem der Ansprüche 41 bis 45, **dadurch gekennzeichnet, daß** in einem Verfahrensschritt (f) die Eigenschaften, insbesondere die inhibitorischen Eigenschaften, der modellierten Verbindung in einem biologischen Testsystem ermittelt werden.

47. Verfahren zur Identifizierung einer Verbindung, mit der Eigenschaft, als Inhibitor der physiologischen Funktion, insbesondere der Endoprotease-Funkdon, eines Proteins aus der Familie der PCs, insbesondere Furin, zu wirken, **dadurch gekennzeichnet, daß**
(a) ein biologische Testsystem für derartige potentielle Inhibitor-Verbindungen etabliert wird,
(b) als Inhibitoren wirkende Verbindungen durch ein biologisches Testsystem gemäß (a) ermittelt werden,
(c) die Konformation der Verbindung bestimmt wird,
(d) die Struktur eines Polypeptids aus einer Raumform, wie nach einem der Ansprüche 1 bis 22 gegeben, durch deren Strukturkoordinaten 3-dimensional graphisch dargestellt wird und
(e) die gemäß (b) und (c) erhaltene Struktur der Verbindung in die gemäß (d) erhaltene Struktur des Polypeptids eingefügt wird.

48. Verfahren nach Anspruch 47, **dadurch gekennzeichnet, daß** in einem Verfahrensschritt (f) Art und/oder Stärke der Wechselwirkung zwischen der Verbindung und der Raumform eines Polypeptids bestimmt wird/werden.

49. Verbindung als Inhibitor der physiologischen Funktion, insbesondere der Endoprotease-Funktion, eines Proteins aus der Familie der PCs, insbesondere Furin, **dadurch gekennzeichnet, daß** sie aus einem Verfahren nach einem der Ansprüche 41 bis 48 erhalten wird bzw. erhältlich ist.

50. Verfahren zur Darstellung einer 3-dimensionalen Struktur eines Polypeptids unbekannter Struktur, enthaltend mindestens eine Aminosäuresequenz oder ein Derivat einer solchen Aminosäuresequenz eines Proteins aus der Familie der PCs, insbesondere Furin, oder eines Komplexes unbekannter Struktur, enthaltend ein derartiges Polypeptid, **dadurch gekennzeichnet, daß** die unbekannte Struktur des Polypeptids oder des Komplexes auf der Basis einer bekannten Raumform nach einem der Ansprüche 1 bis 22 ermittelt wird.

51. Verfahren nach Anspruch 50, **dadurch gekennzeichnet, daß** zur Aufklärung der unbekannten Struktur Strukturkoordinaten einer Raumform nach einem der Ansprüche 1 bis 22, insbesondere Strukturkoordinaten wie in Figur 6 dargestellt, eingesetzt werden.

52. Verfahren nach Anspruch 50 oder 51, **dadurch gekennzeichnet, daß**
(a) die Primärsequenz eines Polypeptids unbekannter 3D-Struktur mit der Primärsequenz eines Polypeptids bekannter Struktur verglichen wird,
(b) für gemäß (a) erhaltene homologe Abschnitte die 3D-Struktur des Polypeptids unbekannter Struktur in Anlehnung an die Struktur aus einer Raumform nach einem der Ansprüche 1 bis 22 modelliert wird, und
(c) mit Hilfe entsprechender Computer-Programme energetische Optimierungen der gemäß (b) modellierten Struktur oder Strukturbereiche durchgeführt werden.

53. Verfahren zur Identifizierung einer Verbindung, die die physiologische Wirkung eines Polypeptids unbekannter Struktur, enthaltend mindestens einen mindestens 50 Aminosäuren umfassenden Sequenzabschnitt eines Proteins aus der Familie der PCs oder ein Derivat eines solchen Sequenzabschnitts, insbesondere eines mindestens 50 Aminosäuren umfassenden Sequenzabschnitts eines Furins, in Hinblick auf dessen Fähigkeit zur endoproteolytischen Spaltung einer Zielsequenz inhibiert, **dadurch gekennzeichnet, daß**
(a) die unbekannte 3D-Struktur des Polypeptids unbekannter Struktur nach einem Verfahren nach einem der Ansprüche 50 bis 52 ermittelt wird und
(b) mit Hilfe eines Verfahrens nach einem der Ansprüche 41 bis 48 eine Verbindung, mit der Eigenschaft als Inhibitor der physiologischen Funktion, insbesondere der Endoprotease-Funktion, eines Proteins aus der Familie der PCs, insbesondere Furin, zu wirken, ermittelt wird.

54. Arzneimittel, enthaltend einen Inhibitor, erhältlich aus einem Verfahren nach einem der Ansprüche 41 bis 48 oder Anspruch 53.

55. Verwendung eines Inhibitors, erhältlich aus einem Verfahren nach einem der Ansprüche 41 bis 48 oder Anspruch 53, zur Herstellung eines Arzneimittels zur Behandlung bzw. zur Behandlung von Tumorerkrankungen, zur Immunsuppression, insbesondere zur Behandlung von Autoimmunerkrankungen oder zur Behandlung von anderen Entzündungserkrankungen, GvHD oder zur Behandlung von bakteriellen Infektionserkrankungen oder Virusinfektionen, insbesondere AIDS oder Influenza.

56. Maschinenlesbares Datenspeichermedium, umfassend ein Datenspeichermaterial, auf dem maschinenlesbare Daten kodiert sind, wobei die Daten durch die Strukturkoordinaten eines Proteins aus der Familie der PCs, insbesondere Furin, oder eines Homologen dieses Komplexes definiert sind, wobei das Homologe Rückgratatome umfaßt, bei denen die Wurzel aus dem Quadrat der mittleren Abweichung von den Rückgratatomen des Komplexes nicht mehr als 2 Å beträgt.

57. Maschinenlesbares Datenspeichermedium nach Anspruch 56, wobei das Molekül oder der molekulare Komplex durch den Satz von Strukturkoordinaten gemäß Fig. 6 definiert ist, oder ein Homologes dieses Moleküls oder molekularen Komplexes, wobei die Wurzel aus dem Quadrat der mittleren Abweichung des Homologen von den Rückgratatomen der Aminosäuren nicht mehr als 2 Å beträgt.

58. Maschinenlesbares Datenspeichermedium, umfassend ein Datenspeichermaterial, auf dem ein erster Satz maschinenlesbarer Daten kodiert vorliegt, umfassend ein Fourier-Transformat von zumindest einem Teil der Strukturkoordinaten eines Proteins oder Abschnitts eines Proteins aus der Familie der PC-Proteine oder gemäß Fig. 6, das, wenn es mit einem zweiten Satz maschinenlesbarer Daten, umfassend ein Röntgenbeugungsmuster eines Moleküls oder molekularen Komplexes unbekannter Struktur, unter Verwendung einer Maschine, programmiert mit Befehlen zum Gebrauch des ersten Datensatzes und des zweiten Datensatzes, kombiniert wird, zumindest einen Teil der Strukturkoordinaten, die dem zweiten Satz maschinenlesbarer Daten entsprechen, bestimmen kann.

59. Verfahren zum Erhalt struktureller Informationen über ein Molekül oder einen molekularen Komplex unbekannter Struktur durch Gebrauch der Strukturkoordinaten eines Proteins aus der Familie der PCs, insbesondere dargelegt in Fig. 6, umfassend die Schritte:
(a) Erzeugen von Röntgenbeugungsdaten von dem kristallisierten Molekül oder molekularen Komplex;
(b) Anwenden zumindest eines Teils der Strukturkoordinaten, dargelegt in Fig. 6, auf das Röntgenbeugungsmuster, um eine dreidimensionale Elektronendichtekarte von zumindest einem Teil des Moleküls oder des molekularen Komplexes zu erzeugen.

60. Verfahren nach Anspruch 59, wobei das Molekül oder der molekulare Komplex unbekannter Struktur ein Polypeptid, ausgewählt aus Proteinen der Familie der PCs oder ähnlichen Polypeptiden, umfasst.

61. Satz von Strukturkoordinaten eines Proteins oder Abschnitts eines Proteins aus der Familie der PCs, insbesondere eines Furins, ganz besonders gemäß Figur 6 oder eines Teilstrukturkoordinatensatzes gemäß Figur 6.


SEQUENCE LISTING

<110> Max-Planck-Gesell. z. Förd. d. Wissenschaften e.V.
      Proteros Biostructures GmbH

<120> Struktur einer Prohormon/Proprotein-Proteinase

<130> MP01P006EP

<140>
<141>

<160> 55

<170> PatentIn Ver. 2.1

<210> 1
<211> 753
<212> PRT
<213> Homo sapiens

<220>
<223> human PC1/PC3

<220>
<223> Komplette Sequenz vom humanen PC1

<400> 1

<210> 2
<211> 638
<212> PRT
<213> Homo sapiens

<220>
<223> Komplette Sequenz vom humanen PC2

<400> 2
Met Lys Gly Gly Cys Val Ser Gln Trp Lys Ala Ala Ala Gly Phe Leu
1               5                   10                  15

Phe Cys Val Met Val Phe Ala Ser Ala Glu Arg Pro Val Phe Thr Asn
            20                  25                  30

His Phe Leu Val Glu Leu His Lys Gly Gly Glu Asp Lys Ala Arg Gln
        35                  40                  45

Val Ala Ala Glu His Gly Phe Gly Val Arg Lys Leu Pro Phe Ala Glu

50                  55                  60

Gly Leu Tyr His Phe Tyr His Asn Gly Leu Ala Lys Ala Lys Arg Arg
65                  70                  75                  80

Arg Ser Leu His His Lys Gln Gln Leu Glu Arg Asp Pro Arg Val Lys
                85                  90                  95

Met Ala Leu Gln Gln Glu Gly Phe Asp Arg Lys Lys Arg Gly Tyr Arg
            100                 105                 110

Asp Ile Asn Glu Ile Asp Ile Asn Met Asn Asp Pro Leu Phe Thr Lys
        115                 120                 125

Gln Trp Tyr Leu Ile Asn Thr Gly Gln Ala Asp Gly Thr Pro Gly Leu
    130                 135                 140

Asp Leu Asn Val Ala Glu Ala Trp Glu Leu Gly Tyr Thr Gly Lys Gly
145                 150                 155                 160

Val Thr Ile Gly Ile Met Asp Asp Gly Ile Asp Tyr Leu His Pro Asp
                165                 170                 175

Leu Ala Ser Asn Tyr Asn Ala Glu Ala Ser Tyr Asp Phe Ser Ser Asn
            180                 185                 190

Asp Pro Tyr Pro Tyr Pro Arg Tyr Thr Asp Asp Trp Phe Asn Ser His
        195                 200                 205

Gly Thr Arg Cys Ala Gly Glu Val Ser Ala Ala Ala Asn Asn Asn Ile
    210                 215                 220

Cys Gly Val Gly Val Ala Tyr Asn Ser Lys Val Ala Gly Ile Arg Met
225                 230                 235                 240

Leu Asp Gln Pro Phe Met Thr Asp Ile Ile Glu Ala Ser Ser Ile Ser
                245                 250                 255

His Met Pro Gln Leu Ile Asp Ile Tyr Ser Ala Ser Trp Gly Pro Thr
            260                 265                 270

Asp Asn Gly Lys Thr Val Asp Gly Pro Arg Glu Leu Thr Leu Gln Ala
        275                 280                 285

Met Ala Asp Gly Val Asn Lys Gly Arg Gly Gly Lys Gly Ser Ile Tyr
    290                 295                 300

Val Trp Ala Ser Gly Asp Gly Gly Ser Tyr Asp Asp Cys Asn Cys Asp

305                 310                 315                 320

Gly Tyr Ala Ser Ser Met Trp Thr Ile Ser Ile Asn Ser Ala Ile Asn
                325                 330                 335

Asp Gly Arg Thr Ala Leu Tyr Asp Glu Ser Cys Ser Ser Thr Leu Ala
            340                 345                 350

Ser Thr Phe Ser Asn Gly Arg Lys Arg Asn Pro Glu Ala Gly Val Ala
        355                 360                 365

Thr Thr Asp Leu Tyr Gly Asn Cys Thr Leu Arg His Ser Gly Thr Ser
    370                 375                 380

Ala Ala Ala Pro Glu Ala Ala Gly Val Phe Ala Leu Ala Leu Glu Ala
385                 390                 395                 400

Asn Leu Gly Leu Thr Trp Arg Asp Met Gln His Leu Thr Val Leu Thr
                405                 410                 415

Ser Lys Arg Asn Gln Leu His Asp Glu Val His Gln Trp Arg Arg Asn
            420                 425                 430

Gly Val Gly Leu Glu Phe Asn His Leu Phe Gly Tyr Gly Val Leu Asp
        435                 440                 445

Ala Gly Ala Met Val Lys Met Ala Lys Asp Trp Lys Thr Val Pro Glu
    450                 455                 460

Arg Phe His Cys Val Gly Gly Ser Val Gln Asp Pro Glu Lys Ile Pro
465                 470                 475                 480

Ser Thr Gly Lys Leu Val Leu Thr Leu Thr Thr Asp Ala Cys Glu Gly
                485                 490                 495

Lys Glu Asn Phe Val Arg Tyr Leu Glu His Val Gln Ala Val Ile Thr
            500                 505                 510

Val Asn Ala Thr Arg Arg Gly Asp Leu Asn Ile Asn Met Thr Ser Pro
        515                 520                 525

Met Gly Thr Lys Ser Ile Leu Leu Ser Arg Arg Pro Arg Asp Asp Asp
    530                 535                 540

Ser Lys Val Gly Phe Asp Lys Trp Pro Phe Met Thr Thr His Thr Trp
545                 550                 555                 560

Gly Glu Asp Ala Arg Gly Thr Trp Thr Leu Glu Leu Gly Phe Val Gly

                565                 570                 575

Ser Ala Pro Gln Lys Gly Val Leu Lys Glu Trp Thr Leu Met Leu His
            580                 585                 590

Gly Thr Gln Ser Ala Pro Tyr Ile Asp Gln Val Val Arg Asp Tyr Gln
        595                 600                 605

Ser Lys Leu Ala Met Ser Lys Lys Glu Glu Leu Glu Glu Glu Leu Asp
    610                 615                 620

Glu Ala Val Glu Arg Ser Leu Lys Ser Ile Leu Asn Lys Asn
625                 630                 635

<210> 3
<211> 969
<212> PRT
<213> Homo sapiens

<220>
<223> Komplette Sequenz vom humanen PACE4

<400> 3
Met Pro Pro Arg Ala Pro Pro Ala Pro Gly Pro Arg Pro Pro Pro Arg
1               5                   10                  15

Ala Ala Ala Ala Thr Asp Thr Ala Ala Gly Ala Gly Gly Ala Gly Gly
            20                  25                  30

Ala Gly Gly Ala Gly Gly Pro Gly Phe Arg Pro Leu Ala Pro Arg Pro
        35                  40                  45

Trp Arg Trp Leu Leu Leu Leu Ala Leu Pro Ala Ala Cys Ser Ala Pro
    50                  55                  60

Pro Pro Arg Pro Val Tyr Thr Asn His Trp Ala Val Gln Val Leu Gly
65                  70                  75                  80

Gly Pro Ala Glu Ala Asp Arg Val Ala Ala Ala His Gly Tyr Leu Asn
                85                  90                  95

Leu Gly Gln Ile Gly Asn Leu Glu Asp Tyr Tyr His Phe Tyr His Ser
            100                 105                 110

Lys Thr Phe Lys Arg Ser Thr Leu Ser Ser Arg Gly Pro His Thr Phe
        115                 120                 125

Leu Arg Met Asp Pro Gln Val Lys Trp Leu Gln Gln Gln Glu Val Lys
    130                 135                 140

Arg Arg Val Lys Arg Gln Val Arg Ser Asp Pro Gln Ala Leu Tyr Phe
145                 150                 155                 160

Asn Asp Pro Ile Trp Ser Asn Met Trp Tyr Leu His Cys Gly Asp Lys
                165                 170                 175

Asn Ser Arg Cys Arg Ser Glu Met Asn Val Gln Ala Ala Trp Lys Arg
            180                 185                 190

Gly Tyr Thr Gly Lys Asn Val Val Val Thr Ile Leu Asp Asp Gly Ile
        195                 200                 205

Glu Arg Asn His Pro Asp Leu Ala Pro Asn Tyr Asp Ser Tyr Ala Ser
    210                 215                 220

Tyr Asp Val Asn Gly Asn Asp Tyr Asp Pro Ser Pro Arg Tyr Asp Ala
225                 230                 235                 240

Ser Asn Glu Asn Lys His Gly Thr Arg Cys Ala Gly Glu Val Ala Ala
                245                 250                 255

Ser Ala Asn Asn Ser Tyr Cys Ile Val Gly Ile Ala Tyr Asn Ala Lys
            260                 265                 270

Ile Gly Gly Ile Arg Met Leu Asp Gly Asp Val Thr Asp Val Val Glu
        275                 280                 285

Ala Lys Ser Leu Gly Ile Arg Pro Asn Tyr Ile Asp Ile Tyr Ser Ala
    290                 295                 300

Ser Trp Gly Pro Asp Asp Asp Gly Lys Thr Val Asp Gly Pro Gly Arg
305                 310                 315                 320

Leu Ala Lys Gln Ala Phe Glu Tyr Gly Ile Lys Lys Gly Arg Gln Gly
                325                 330                 335

Leu Gly Ser Ile Phe Val Trp Ala Ser Gly Asn Gly Gly Arg Glu Gly
            340                 345                 350

Asp Tyr Cys Ser Cys Asp Gly Tyr Thr Asn Ser Ile Tyr Thr Ile Ser
        355                 360                 365

Val Ser Ser Ala Thr Glu Asn Gly Tyr Lys Pro Trp Tyr Leu Glu Glu
    370                 375                 380

Cys Ala Ser Thr Leu Ala Thr Thr Tyr Ser Ser Gly Ala Phe Tyr Glu
385                 390                 395                 400

Arg Lys Ile Val Thr Thr Asp Leu Arg Gln Arg Cys Thr Asp Gly His
                405                 410                 415

Thr Gly Thr Ser Val Ser Ala Pro Met Val Ala Gly Ile Ile Ala Leu
            420                 425                 430

Ala Leu Glu Ala Asn Ser Gln Leu Thr Trp Arg Asp Val Gln His Leu
        435                 440                 445

Leu Val Lys Thr Ser Arg Pro Ala His Leu Lys Ala Ser Asp Trp Lys
    450                 455                 460

Val Asn Gly Ala Gly His Lys Val Ser His Phe Tyr Gly Phe Gly Leu
465                 470                 475                 480

Val Asp Ala Glu Ala Leu Val Val Glu Ala Lys Lys Trp Thr Ala Val
                485                 490                 495

Pro Ser Gln His Met Cys Val Ala Ala Ser Asp Lys Arg Pro Arg Ser
            500                 505                 510

Ile Pro Leu Val Gln Val Leu Arg Thr Thr Ala Leu Thr Ser Ala Cys
        515                 520                 525

Ala Glu His Ser Asp Gln Arg Val Val Tyr Leu Glu His Val Val Val
    530                 535                 540

Arg Thr Ser Ile Ser His Pro Arg Arg Gly Asp Leu Gln Ile Tyr Leu
545                 550                 555                 560

Val Ser Pro Ser Gly Thr Lys Ser Gln Leu Leu Ala Lys Arg Leu Leu
                565                 570                 575

Asp Leu Ser Asn Glu Gly Phe Thr Asn Trp Glu Phe Met Thr Val His
            580                 585                 590

Cys Trp Gly Glu Lys Ala Glu Gly Gln Trp Thr Leu Glu Ile Gln Asp
        595                 600                 605

Leu Pro Ser Gln Val Arg Asn Pro Glu Lys Gln Gly Lys Leu Lys Glu
    610                 615                 620

Trp Ser Leu Ile Leu Tyr Gly Thr Ala Glu His Pro Tyr His Thr Phe
625                 630                 635                 640

Ser Ala His Gln Ser Arg Ser Arg Met Leu Glu Leu Ser Ala Pro Glu
                645                 650                 655

Leu Glu Pro Pro Lys Ala Ala Leu Ser Pro Ser Gln Val Glu Val Pro
            660                 665                 670

Glu Asp Glu Glu Asp Tyr Thr Ala Gln Ser Thr Pro Gly Ser Ala Asn
        675                 680                 685

Ile Leu Gln Thr Ser Val Cys His Pro Glu Cys Gly Asp Lys Gly Cys
    690                 695                 700

Asp Gly Pro Asn Ala Asp Gln Cys Leu Asn Cys Val His Phe Ser Leu
705                 710                 715                 720

Gly Ser Val Lys Thr Ser Arg Lys Cys Val Ser Val Cys Pro Leu Gly
                725                 730                 735

Tyr Phe Gly Asp Thr Ala Ala Arg Arg Cys Arg Arg Cys His Lys Gly
            740                 745                 750

Cys Glu Thr Cys Ser Ser Arg Ala Ala Thr Gln Cys Leu Ser Cys Arg
        755                 760                 765

Arg Gly Phe Tyr His His Gln Glu Met Asn Thr Cys Val Thr Leu Cys
    770                 775                 780

Pro Ala Gly Phe Tyr Ala Asp Glu Ser Gln Lys Asn Cys Leu Lys Cys
785                 790                 795                 800

His Pro Ser Cys Lys Lys Cys Val Asp Glu Pro Glu Lys Cys Thr Val
                805                 810                 815

Cys Lys Glu Gly Phe Ser Leu Ala Arg Gly Ser Cys Ile Pro Asp Cys
            820                 825                 830

Glu Pro Gly Thr Tyr Phe Asp Ser Glu Leu Ile Arg Cys Gly Glu Cys
        835                 840                 845

His His Thr Cys Gly Thr Cys Val Gly Pro Gly Arg Glu Glu Cys Ile
    850                 855                 860

His Cys Ala Lys Asn Phe His Phe His Asp Trp Lys Cys Val Pro Ala
865                 870                 875                 880

Cys Gly Glu Gly Phe Tyr Pro Glu Glu Met Pro Gly Leu Pro His Lys
                885                 890                 895

Val Cys Arg Arg Cys Asp Glu Asn Cys Leu Ser Cys Ala Gly Ser Ser
            900                 905                 910

Arg Asn Cys Ser Arg Cys Lys Thr Gly Phe Thr Gln Leu Gly Thr Ser
        915                 920                 925

Cys Ile Thr Asn His Thr Cys Ser Asn Ala Asp Glu Thr Phe Cys Glu
    930                 935                 940

Met Val Lys Ser Asn Arg Leu Cys Glu Arg Lys Leu Phe Ile Gln Phe
945                 950                 955                 960

Cys Cys Arg Thr Cys Leu Leu Ala Gly
                965

<210> 4
<211> 655
<212> PRT
<213> Mus musculus

<220>
<223> Komplette Sequenz vom mouse PC4

<400> 4
Met Arg Pro Ser Gln Thr Glu Leu Trp Leu Gly Leu Thr Leu Thr Leu
  1               5                  10                  15

Ala Leu Leu Ala Val Arg Trp Ala Ser Ala Gln Ala Pro Ile Tyr Val
             20                  25                  30

Ser Ser Trp Ala Val Arg Val Thr Lys Gly Tyr Gln Glu Ala Glu Arg
         35                  40                  45

Leu Ala Arg Lys Phe Gly Phe Val Asn Leu Gly Gln Ile Phe Pro Asp
     50                  55                  60

Asp Gln Tyr Phe His Leu Arg His Arg Gly Val Ala Gln Gln Ser Leu
 65                  70                  75                  80

Thr Pro His Trp Gly His Arg Leu Arg Leu Lys Lys Asp Pro Lys Val
                 85                  90                  95

Arg Trp Phe Glu Gln Gln Thr Leu Arg Arg Arg Val Lys Arg Ser Leu
            100                 105                 110

Val Val Pro Thr Asp Pro Trp Phe Ser Lys Gln Trp Tyr Met Asn Lys
        115                 120                 125

Glu Ile Gln Gln Asp Leu Asn Ile Leu Lys Ala Trp Asn Gln Gly Leu
    130                 135                 140

Thr Gly Arg Gly Val Val Ile Ser Ile Leu Asp Asp Gly Ile Glu Lys
145                 150                 155                 160

Asp His Pro Asp Leu Trp Ala Asn Tyr Asp Pro Leu Ala Ser Tyr Asp
                165                 170                 175

Phe Asn Asp Tyr Asp Pro Asp Pro Gln Pro Arg Tyr Thr Pro Asn Asp
            180                 185                 190

Glu Asn Arg His Gly Thr Arg Cys Ala Gly Glu Val Ser Ala Thr Ala
        195                 200                 205

Asn Asn Gly Phe Cys Gly Ala Gly Val Ala Phe Asn Ala Arg Ile Gly
    210                 215                 220

Gly Val Arg Met Leu Asp Gly Ala Ile Thr Asp Ile Val Glu Ala Gln
225                 230                 235                 240

Ser Leu Ser Leu Gln Pro Gln His Ile His Ile Tyr Ser Ala Ser Trp
                245                 250                 255

Gly Pro Glu Asp Asp Gly Arg Thr Val Asp Gly Pro Gly Leu Leu Thr
            260                 265                 270

Gln Glu Ala Phe Arg Arg Gly Val Thr Lys Gly Arg Gln Gly Leu Gly
        275                 280                 285

Thr Leu Phe Ile Trp Ala Ser Gly Asn Gly Gly Leu His Tyr Asp Asn
    290                 295                 300

Cys Asn Cys Asp Gly Tyr Thr Asn Ser Ile His Thr Leu Ser Val Gly
305                 310                 315                 320

Ser Thr Thr Arg Gln Gly Arg Val Pro Trp Tyr Ser Glu Ala Cys Ala
                325                 330                 335

Ser Thr Phe Thr Thr Thr Phe Ser Ser Gly Val Val Thr Asp Pro Gln
            340                 345                 350

Ile Val Thr Thr Asp Leu His His Gln Cys Thr Asp Lys His Thr Gly
        355                 360                 365

Thr Ser Ala Ser Ala Pro Leu Ala Ala Gly Met Ile Ala Leu Ala Leu
    370                 375                 380

Glu Ala Asn Pro Leu Leu Thr Trp Arg Asp Leu Gln His Leu Val Val
385                 390                 395                 400

Arg Ala Ser Arg Pro Ala Gln Leu Gln Ala Glu Asp Trp Arg Ile Asn
                405                 410                 415

Gly Val Gly Arg Gln Val Ser His His Tyr Gly Tyr Gly Leu Leu Asp
            420                 425                 430

Ala Gly Leu Leu Val Asp Leu Ala Arg Val Trp Leu Pro Thr Lys Pro
        435                 440                 445

Gln Lys Lys Cys Ala Ile Arg Val Val His Thr Pro Thr Pro Ile Leu
    450                 455                 460

Pro Arg Met Leu Val Pro Lys Asn Val Thr Ala Cys Ser Asp Gly Ser
465                 470                 475                 480

Arg Arg Arg Leu Ile Arg Ser Leu Glu His Val Gln Val Gln Leu Ser
                485                 490                 495

Leu Ser Tyr Ser Arg Arg Gly Asp Leu Glu Ile Phe Leu Thr Ser Pro
            500                 505                 510

Met Gly Thr Arg Ser Thr Leu Val Ala Ile Arg Pro Leu Asp Ile Ser
        515                 520                 525

Gly Gln Gly Tyr Asn Asn Trp Ile Phe Met Ser Thr His Tyr Trp Asp
    530                 535                 540

Glu Asp Pro Gln Gly Leu Trp Thr Leu Gly Leu Glu Asn Lys Gly Tyr
545                 550                 555                 560

Tyr Phe Asn Thr Gly Thr Leu Tyr Tyr Tyr Thr Leu Leu Leu Tyr Gly
                565                 570                 575

Thr Ala Glu Asp Met Thr Ala Arg Pro Gln Ala Pro Gln Val Thr Ser
            580                 585                 590

Arg Ala Arg Ala Cys Val Gln Arg Asp Thr Glu Gly Leu Cys Gln Glu
        595                 600                 605

Ser His Ser Pro Leu Ser Ile Leu Ala Gly Leu Cys Leu Ile Ser Ser
    610                 615                 620

Gln Gln Trp Trp Trp Leu Tyr Ser His Pro Gln Gln Pro Val Thr Glu
625                 630                 635                 640

Gly Gln Ala Ser Cys His Pro Pro Val Thr Pro Ala Ala Ala Ala
                645                 650                 655

<210> 5
<211> 913
<212> PRT
<213> Homo sapiens

<220>
<223> Komplette Sequenz vom human PC5/PC6

<400> 5
   Met Asp Trp Glu Ser Arg Cys Cys Cys Pro Gly Arg Leu Asp Leu Leu
   1 5 10 15
   Cys Val Leu Ala Leu Leu Gly Gly Cys Leu Leu Pro Val Cys Arg Thr
   20 25 30
   Arg Val Tyr Thr Asn His Trp Ala Val Lys Ile Ala Gly Gly Phe Pro
   35 40 45
   Glu Ala Asn Arg Ile Ala Ser Lys Tyr Gly Phe Ile Asn Ile Gly Gln
   50 55 60
   Ile Gly Ala Leu Lys Asp Tyr Tyr His Phe Tyr His Ser Arg Thr Ile
   65 70 75 80
   Lys Arg Ser Val Ile Ser Ser Arg Gly Thr His Ser Phe Ile Ser Met
   85 90 95
   Glu Pro Lys Val Glu Trp Ile Gln Gln Gln Val Val Lys Lys Arg Thr
   100 105 110
   Lys Arg Asp Tyr Asp Ser Ser Arg Val Gln Ser Thr Tyr Phe Asn Asp
   115 120 125
   Pro Lys Trp Pro Ser Met Trp Tyr Met His Cys Ser Asp Asn Thr His
   130 135 140
   Pro Cys Gln Ser Asp Met Asn Ile Glu Gly Ala Trp Lys Arg Gly Tyr
   145 150 155 160
   Thr Gly Lys Asn Ile Val Val Thr Ile Leu Asp Asp Gly Ile Glu Arg
   165 170 175
   Thr His Pro Asp Leu Met Gln Asn Tyr Asp Ala Leu Ala Ser Cys Asp
   180 185 190
   Val Asn Gly Asn Asp Leu Asp Pro Met Pro Arg Tyr Asp Ala Ser Asn
   195 200 205
   Glu Asn Lys His Gly Thr Arg Cys Ala Gly Glu Val Ala Ala Ala Ala
   210 215 220
   Asn Asn Ser His Cys Thr Val Gly Ile Ala Phe Asn Ala Lys Ile Gly
   225 230 235 240
   Gly Val Arg Met Leu Asp Gly Asp Val Thr Asp Met Val Glu Ala Lys
   245 250 255
   Ser Val Ser Phe Asn Pro Gln His Val His Ile Tyr Ser Ala Ser Trp
   260 265 270
   Gly Pro Asp Asp Asp Gly Lys Thr Val Asp Gly Pro Ala Pro Leu Thr
   275 280 285
   Arg Gln Ala Phe Glu Asn Gly Val Arg Met Gly Arg Arg Gly Leu Gly
   290 295 300
   Ser Val Phe Val Trp Ala Ser Gly Asn Gly Gly Arg Ser Lys Asp His
   305 310 315 320
   Cys Ser Cys Asp Gly Tyr Thr Asn Ser Ile Tyr Thr Ile Ser Ile Ser
   325 330 335
   Ser Thr Ala Glu Ser Gly Lys Lys Pro Trp Tyr Leu Glu Glu Cys Ser
   340 345 350
   Ser Thr Leu Ala Thr Thr Tyr Ser Ser Gly Glu Ser Tyr Asp Lys Lys
   355 360 365
   Ile Ile Thr Thr Asp Leu Arg Gln Arg Cys Thr Asp Asn His Thr Gly
   370 375 380
   Thr Ser Ala Ser Ala Pro Met Ala Ala Gly Ile Ile Ala Leu Ala Leu
   385 390 395 400
   Glu Ala Asn Pro Phe Leu Thr Trp Arg Asp Val Gln His Val Ile Val
   405 410 415
   Arg Thr Ser Arg Ala Gly His Leu Asn Ala Asn Asp Trp Lys Thr Asn
   420 425 430
   Ala Ala Gly Phe Lys Val Ser His Leu Tyr Gly Phe Gly Leu Met Asp
   435 440 445
   Ala Glu Ala Met Val Met Glu Ala Glu Lys Trp Thr Thr Val Pro Arg
   450 455 460
   Gln His Val Cys Val Glu Ser Thr Asp Arg Gln Ile Lys Thr Ile Arg
   465 470 475 480
   Pro Asn Ser Ala Val Arg Ser Ile Tyr Lys Ala Ser Gly Cys Ser Asp
   485 490 495
   Asn Pro Asn Arg His Val Asn Tyr Leu Glu His Val Val Val Arg Ile
   500 505 510
   Thr Ile Thr His Pro Arg Arg Gly Asp Leu Ala Ile Tyr Leu Thr Ser
   515 520 525
   Pro Ser Gly Thr Arg Ser Gln Leu Leu Ala Asn Arg Leu Phe Asp His
   530 535 540
   Ser Met Glu Gly Phe Lys Asn Trp Glu Phe Met Thr Ile His Cys Trp
   545 550 555 560
   Gly Glu Arg Ala Ala Gly Asp Trp Val Leu Glu Val Tyr Asp Thr Pro
   565 570 575
   Ser Gln Leu Arg Asn Phe Lys Thr Pro Gly Lys Leu Lys Glu Trp Ser
   580 585 590
   Leu Val Leu Tyr Gly Thr Ser Val Arg Pro Tyr Ser Pro Thr Asn Glu
   595 600 605
   Phe Pro Lys Val Glu Arg Phe Arg Tyr Ser Arg Val Glu Asp Pro Thr
   610 615 620
   Asp Asp Tyr Gly Thr Glu Asp Tyr Ala Gly Pro Cys Asp Pro Glu Cys
   625 630 635 640
   Ser Glu Val Gly Cys Asp Gly Pro Gly Pro Asp His Cys Asn Asp Cys
   645 650 655
   Leu His Tyr Tyr Tyr Lys Leu Lys Asn Asn Thr Arg Ile Cys Val Ser
   660 665 670
   Ser Cys Pro Pro Gly His Tyr His Ala Asp Lys Lys Arg Cys Arg Lys
   675 680 685
   Cys Ala Pro Asn Cys Glu Ser Cys Phe Gly Ser His Gly Asp Gln Cys
   690                 695                 700
   Met Ser Cys Lys Tyr Gly Tyr Phe Leu Asn Glu Glu Thr Asn Ser Cys
   705                 710                 715                 720
   Val Thr His Cys Pro Asp Gly Ser Tyr Gln Asp Thr Lys Lys Asn Leu
                   725                 730                 735
   Cys Arg Lys Cys Ser Glu Asn Cys Lys Thr Cys Thr Glu Phe His Asn
               740                 745                 750
   Cys Thr Glu Cys Arg Asp Gly Leu Ser Leu Gln Gly Ser Arg Cys Ser
           755                 760                 765
   Val Ser Cys Glu Asp Gly Arg Tyr Phe Asn Gly Gln Asp Cys Gln Pro
       770                 775                 780
   Cys His Arg Phe Cys Ala Thr Cys Ala Gly Ala Gly Ala Asp Gly Cys
   785                 790                 795                 800
   Ile Asn Cys Thr Glu Gly Tyr Phe Met Glu Asp Gly Arg Cys Val Gln
                   805                 810                 815
   Ser Cys Ser Ile Ser Tyr Tyr Phe Asp His Ser Ser Glu Asn Gly Tyr
               820                 825                 830
   Lys Ser Cys Lys Lys Cys Asp Ile Ser Cys Leu Thr Cys Asn Gly Pro
           835                 840                 845
   Gly Phe Lys Asn Cys Thr Ser Cys Pro Ser Gly Tyr Leu Leu Asp Leu
       850                 855                 860
   Gly Met Cys Gln Met Gly Ala Ile Cys Lys Asp Ala Thr Glu Glu Ser
   865                 870                 875                 880
   Trp Ala Glu Gly Gly Phe Cys Met Leu Val Lys Lys Asn Asn Leu Cys
                   885                 890                 895
   Gln Arg Lys Val Leu Gln Gln Leu Cys Cys Lys Thr Cys Thr Phe Gln
               900                 905                 910
   Gly

<210> 6
<211> 785
<212> PRT

<213> Homo sapiens

<220>
<223> Komplette Sequenz vom human PC7/PC8/LPC/SPC7

<400> 6
Met Pro Lys Gly Arg Gln Lys Val Pro His Leu Asp Ala Pro Leu Gly
1               5                   10                  15

Leu Pro Thr Cys Leu Trp Leu Glu Leu Ala Gly Leu Phe Leu Leu Val
            20                  25                  30

Pro Trp Val Met Gly Leu Ala Gly Thr Gly Gly Pro Asp Gly Gln Gly
        35                  40                  45

Thr Gly Gly Ala Ser Trp Ala Val His Leu Glu Ser Leu Glu Gly Asp
    50                  55                  60

Gly Glu Glu Glu Thr Leu Glu Gln Gln Ala Asp Ala Leu Ala Gln Ala
65                  70                  75                  80

Ala Gly Leu Val Asn Ala Gly Arg Ile Gly Glu Leu Gln Gly His Tyr
                85                  90                  95

Leu Phe Val Gln Pro Ala Gly His Arg Pro Ala Leu Glu Val Glu Pro
            100                 105                 110

Ile Arg Gln Gln Val Glu Ala Val Leu Ala Gly His Glu Ala Val Arg
        115                 120                 125

Trp His Ser Glu Gln Arg Leu Leu Arg Arg Ala Lys Arg Ser Val His
    130                 135                 140

Phe Asn Asp Pro Lys Tyr Pro Gln Gln Trp His Leu Asn Asn Arg Arg
145                 150                 155                 160

Ser Pro Gly Arg Asp Ile Asn Val Thr Gly Val Trp Glu Arg Asn Val
                165                 170                 175

Thr Gly Arg Gly Val Thr Val Val Val Val Asp Asp Gly Val Glu His
            180                 185                 190

Thr Ile Gln Asp Ile Ala Pro Asn Tyr Ser Pro Glu Gly Ser Tyr Asp
        195                 200                 205

Leu Asn Ser Asn Asp Pro Asp Pro Met Pro His Pro Asp Val Glu Asn
    210                 215                 220

Gly Asn His His Gly Thr Arg Cys Ala Gly Glu Ile Ala Ala Val Pro
225                 230                 235                 240

Asn Asn Ser Phe Cys Ala Val Gly Val Ala Tyr Gly Ser Arg Ile Ala
                245                 250                 255

Gly Ile Arg Val Leu Asp Gly Pro Leu Thr Asp Ser Met Glu Ala Val
            260                 265                 270

Ala Phe Asn Lys His Tyr Gln Ile Asn Asp Ile Tyr Ser Cys Ser Trp
        275                 280                 285

Gly Pro Asp Asp Asp Gly Lys Thr Val Asp Gly Pro His Gln Leu Gly
    290                 295                 300

Lys Ala Ala Leu Gln His Gly Val Ile Ala Gly Arg Gln Gly Phe Gly
305                 310                 315                 320

Ser Ile Phe Val Val Ala Ser Gly Asn Gly Gly Gln His Asn Asp Asn
                325                 330                 335

Cys Asn Tyr Asp Gly Tyr Ala Asn Ser Ile Tyr Thr Val Thr Ile Gly
            340                 345                 350

Ala Val Asp Glu Glu Gly Arg Met Pro Phe Tyr Ala Glu Glu Cys Ala
        355                 360                 365

Ser Met Leu Ala Val Thr Phe Ser Gly Gly Asp Lys Met Leu Arg Ser
    370                 375                 380

Ile Val Thr Thr Asp Trp Asp Leu Gln Lys Gly Thr Gly Cys Thr Glu
385                 390                 395                 400

Gly His Thr Gly Thr Ser Ala Ala Ala Pro Leu Ala Ala Gly Met Ile
                405                 410                 415

Ala Leu Met Leu Gln Val Arg Pro Cys Leu Thr Trp Arg Asp Val Gln
            420                 425                 430

His Ile Ile Val Phe Thr Ala Thr Arg Tyr Glu Asp Arg Arg Ala Glu
        435                 440                 445

Trp Val Thr Asn Glu Ala Gly Phe Ser His Ser His Gln His Gly Phe
    450                 455                 460

Gly Leu Leu Asn Ala Trp Arg Leu Val Asn Ala Ala Lys Ile Trp Thr
465                 470                 475                 480

Ser Val Pro Tyr Leu Ala Ser Tyr Val Ser Pro Val Leu Lys Glu Asn
                485                 490                 495

Lys Ala Ile Pro Gln Ser Pro Arg Ser Leu Glu Val Leu Trp Asn Val
            500                 505                 510

Ser Arg Met Asp Leu Glu Met Ser Gly Leu Lys Thr Leu Glu His Val
        515                 520                 525

Ala Val Thr Val Ser Ile Thr His Pro Arg Arg Gly Ser Leu Glu Leu
    530                 535                 540

Lys Leu Phe Cys Pro Ser Gly Met Met Ser Leu Ile Gly Ala Pro Arg
545                 550                 555                 560

Ser Met Asp Ser Asp Pro Asn Gly Phe Asn Asp Trp Thr Phe Ser Thr
                565                 570                 575

Val Arg Cys Trp Gly Glu Arg Ala Arg Gly Thr Tyr Arg Leu Val Ile
            580                 585                 590

Arg Asp Val Gly Asp Glu Ser Phe Gln Val Gly Ile Leu Arg Gln Trp
        595                 600                 605

Gln Leu Thr Leu Tyr Gly Ser Val Trp Ser Ala Val Asp Ile Arg Asp
    610                 615                 620

Arg Gln Arg Leu Leu Glu Ser Ala Met Ser Gly Lys Tyr Leu His Asp
625                 630                 635                 640

Asp Phe Ala Leu Pro Cys Pro Pro Gly Leu Lys Ile Pro Glu Glu Asp
                645                 650                 655

Gly Tyr Thr Ile Thr Pro Asn Thr Leu Lys Thr Leu Val Leu Val Gly
            660                 665                 670

Cys Phe Thr Val Phe Trp Thr Val Tyr Tyr Met Leu Glu Val Tyr Leu
        675                 680                 685

Ser Gln Arg Asn Val Ala Ser Asn Gln Val Cys Arg Ser Gly Pro Cys
    690                 695                 700

His Trp Pro His Arg Ser Arg Lys Ala Lys Glu Glu Gly Thr Glu Leu
705                 710                 715                 720

Glu Ser Val Pro Leu Cys Ser Ser Lys Asp Pro Asp Glu Val Glu Thr
                725                 730                 735

Glu Ser Arg Gly Pro Pro Thr Thr Ser Asp Leu Leu Ala Pro Asp Leu
            740                 745                 750

Leu Glu Gln Gly Asp Trp Ser Leu Ser Gln Asn Lys Ser Ala Leu Asp
        755                 760                 765

Cys Pro His Gln His Leu Asp Val Pro His Gly Lys Glu Glu Gln Ile
    770                 775                 780

Cys
785

<210> 7
<211> 379
<212> PRT
<213> Bacillus licheniformis

<220>
<223> Komplette Sequenz vom Subtilisin Carlsberg

<400> 7
Met Met Arg Lys Lys Ser Phe Trp Leu Gly Met Leu Thr Ala Phe Met
  1               5                  10                  15

Leu Val Phe Thr Met Ala Phe Ser Asp Ser Ala Ser Ala Ala Gln Pro
             20                  25                  30

Ala Lys Asn Val Glu Lys Asp Tyr Ile Val Gly Phe Lys Ser Gly Val
         35                  40                  45

Lys Thr Ala Ser Val Lys Lys Asp Ile Ile Lys Glu Ser Gly Gly Lys
     50                  55                  60

Val Asp Lys Gln Phe Arg Ile Ile Asn Ala Ala Lys Ala Lys Leu Asp
 65                  70                  75                  80

Lys Glu Ala Leu Lys Glu Val Lys Asn Asp Pro Asp Val Ala Tyr Val
                 85                  90                  95

Glu Glu Asp His Val Ala His Ala Leu Ala Gln Thr Val Pro Tyr Gly
            100                 105                 110

Ile Pro Leu Ile Lys Ala Asp Lys Val Gln Ala Gln Gly Phe Lys Gly
        115                 120                 125

Ala Asn Val Lys Val Ala Val Leu Asp Thr Gly Ile Gln Ala Ser His
    130                 135                 140

Pro Asp Leu Asn Val Val Gly Gly Ala Ser Phe Val Ala Gly Glu Ala
145                 150                 155                 160

Tyr Asn Thr Asp Gly Asn Gly His Gly Thr His Val Ala Gly Thr Val
                165                 170                 175

Ala Ala Leu Asp Asn Thr Thr Gly Val Leu Gly Val Ala Pro Ser Val
            180                 185                 190

Ser Leu Tyr Ala Val Lys Val Leu Asn Ser Ser Gly Ser Gly Thr Tyr
        195                 200                 205

Ser Gly Ile Val Ser Gly Ile Glu Trp Ala Thr Thr Asn Gly Met Asp
    210                 215                 220

Val Ile Asn Met Ser Leu Gly Gly Pro Ser Gly Ser Thr Ala Met Lys
225                 230                 235                 240

Gln Ala Val Asp Asn Ala Tyr Ala Arg Gly Val Val Val Val Ala Ala
                245                 250                 255

Ala Gly Asn Ser Gly Ser Ser Gly Asn Thr Asn Thr Ile Gly Tyr Pro
            260                 265                 270

Ala Lys Tyr Asp Ser Val Ile Ala Val Gly Ala Val Asp Ser Asn Ser
        275                 280                 285

Asn Arg Ala Ser Phe Ser Ser Val Gly Ala Glu Leu Glu Val Met Ala
    290                 295                 300

Pro Gly Ala Gly Val Tyr Ser Thr Tyr Pro Thr Ser Thr Tyr Ala Thr
305                 310                 315                 320

Leu Asn Gly Thr Ser Met Ala Ser Pro His Val Ala Gly Ala Ala Ala
                325                 330                 335

Leu Ile Leu Ser Lys His Pro Asn Leu Ser Ala Ser Gln Val Arg Asn
            340                 345                 350

Arg Leu Ser Ser Thr Ala Thr Tyr Leu Gly Ser Ser Phe Tyr Tyr Gly
        355                 360                 365

Lys Gly Leu Ile Asn Val Glu Ala Ala Ala Gln
    370                 375

<210> 8

<211> 814
<212> PRT
<213> Saccharomyces cerevisiae

<220>
<223> Komplette Sequenz vom yeast Kexin

<400> 8
Met Lys Val Arg Lys Tyr Ile Thr Leu Cys Phe Trp Trp Ala Phe Ser
1               5                   10                  15

Thr Ser Ala Leu Val Ser Ser Gln Gln Ile Pro Leu Lys Asp His Thr
            20                  25                  30

Ser Arg Gln Tyr Phe Ala Val Glu Ser Asn Glu Thr Leu Ser Arg Leu
        35                  40                  45

Glu Glu Met His Pro Asn Trp Lys Tyr Glu His Asp Val Arg Gly Leu
    50                  55                  60

Pro Asn His Tyr Val Phe Ser Lys Glu Leu Leu Lys Leu Gly Lys Arg
65                  70                  75                  80

Ser Ser Leu Glu Glu Leu Gln Gly Asp Asn Asn Asp His Ile Leu Ser
                85                  90                  95

Val His Asp Leu Phe Pro Arg Asn Asp Leu Phe Lys Arg Leu Pro Val
            100                 105                 110

Pro Ala Pro Pro Met Asp Ser Ser Leu Leu Pro Val Lys Glu Ala Glu
        115                 120                 125

Asp Lys Leu Ser Ile Asn Asp Pro Leu Phe Glu Arg Gln Trp His Leu
    130                 135                 140

Val Asn Pro Ser Phe Pro Gly Ser Asp Ile Asn Val Leu Asp Leu Trp
145                 150                 155                 160

Tyr Asn Asn Ile Thr Gly Ala Gly Val Val Ala Ala Ile Val Asp Asp
                165                 170                 175

Gly Leu Asp Tyr Glu Asn Glu Asp Leu Lys Asp Asn Phe Cys Ala Glu
            180                 185                 190

Gly Ser Trp Asp Phe Asn Asp Asn Thr Asn Leu Pro Lys Pro Arg Leu
        195                 200                 205

Ser Asp Asp Tyr His Gly Thr Arg Cys Ala Gly Glu Ile Ala Ala Lys

    210                 215                 220

Lys Gly Asn Asn Phe Cys Gly Val Gly Val Gly Tyr Asn Ala Lys Ile
225                 230                 235                 240

Ser Gly Ile Arg Ile Leu Ser Gly Asp Ile Thr Thr Glu Asp Glu Ala
                245                 250                 255

Ala Ser Leu Ile Tyr Gly Leu Asp Val Asn Asp Ile Tyr Ser Cys Ser
            260                 265                 270

Trp Gly Pro Ala Asp Asp Gly Arg His Leu Gln Gly Pro Ser Asp Leu
        275                 280                 285

Val Lys Lys Ala Leu Val Lys Gly Val Thr Glu Gly Arg Asp Ser Lys
    290                 295                 300

Gly Ala Ile Tyr Val Phe Ala Ser Gly Asn Gly Gly Thr Arg Gly Asp
305                 310                 315                 320

Asn Cys Asn Tyr Asp Gly Tyr Thr Asn Ser Ile Tyr Ser Ile Thr Ile
                325                 330                 335

Gly Ala Ile Asp His Lys Asp Leu His Pro Pro Tyr Ser Glu Gly Cys
            340                 345                 350

Ser Ala Val Met Ala Val Thr Tyr Ser Ser Gly Ser Gly Glu Tyr Ile
        355                 360                 365

His Ser Ser Asp Ile Asn Gly Arg Cys Ser Asn Ser His Gly Gly Thr
    370                 375                 380

Ser Ala Ala Ala Pro Leu Ala Ala Gly Val Tyr Thr Leu Leu Leu Glu
385                 390                 395                 400

Ala Asn Pro Asn Leu Thr Trp Arg Asp Val Gln Tyr Leu Ser Ile Leu
                405                 410                 415

Ser Ala Val Gly Leu Glu Lys Asn Ala Asp Gly Asp Trp Arg Asp Ser
            420                 425                 430

Ala Met Gly Lys Lys Tyr Ser His Arg Tyr Gly Phe Gly Lys Ile Asp
        435                 440                 445

Ala His Lys Leu Ile Glu Met Ser Lys Thr Trp Glu Asn Val Asn Ala
    450                 455                 460

Gln Thr Trp Phe Tyr Leu Pro Thr Leu Tyr Val Ser Gln Ser Thr Asn

465                 470                 475                 480

Ser Thr Glu Glu Thr Leu Glu Ser Val Ile Thr Ile Ser Glu Lys Ser
                485                 490                 495

Leu Gln Asp Ala Asn Phe Lys Arg Ile Glu His Val Thr Val Thr Val
            500                 505                 510

Asp Ile Asp Thr Glu Ile Arg Gly Thr Thr Thr Val Asp Leu Ile Ser
        515                 520                 525

Pro Ala Gly Ile Ile Ser Asn Leu Gly Val Val Arg Pro Arg Asp Val
    530                 535                 540

Ser Ser Glu Gly Phe Lys Asp Trp Thr Phe Met Ser Val Ala His Trp
545                 550                 555                 560

Gly Glu Asn Gly Val Gly Asp Trp Lys Ile Lys Val Lys Thr Thr Glu
                565                 570                 575

Asn Gly His Arg Ile Asp Phe His Ser Trp Arg Leu Lys Leu Phe Gly
            580                 585                 590

Glu Ser Ile Asp Ser Ser Lys Thr Glu Thr Phe Val Phe Gly Asn Asp
        595                 600                 605

Lys Glu Glu Val Glu Pro Ala Ala Thr Glu Ser Thr Val Ser Gln Tyr
    610                 615                 620

Ser Ala Ser Ser Thr Ser Ile Ser Ile Ser Ala Thr Ser Thr Ser Ser
625                 630                 635                 640

Ile Ser Ile Gly Val Glu Thr Ser Ala Ile Pro Gln Thr Thr Thr Ala
                645                 650                 655

Ser Thr Asp Pro Asp Ser Asp Pro Asn Thr Pro Lys Lys Leu Ser Ser
            660                 665                 670

Pro Arg Gln Ala Met His Tyr Phe Leu Thr Ile Phe Leu Ile Gly Ala
        675                 680                 685

Thr Phe Leu Val Leu Tyr Phe Met Phe Phe Met Lys Ser Arg Arg Arg
    690                 695                 700

Ile Arg Arg Ser Arg Ala Glu Thr Tyr Glu Phe Asp Ile Ile Asp Thr
705                 710                 715                 720

Asp Ser Glu Tyr Asp Ser Thr Leu Asp Asn Gly Thr Ser Gly Ile Thr

725                 730                 735

Glu Pro Glu Glu Val Glu Asp Phe Asp Phe Asp Leu Ser Asp Glu Asp
            740                 745                 750

His Leu Ala Ser Leu Ser Ser Ser Glu Asn Gly Asp Ala Glu His Thr
        755                 760                 765

Ile Asp Ser Val Leu Thr Asn Glu Asn Pro Phe Ser Asp Pro Ile Lys
    770                 775                 780

Gln Lys Phe Pro Asn Asp Ala Asn Ala Glu Ser Ala Ser Asn Lys Leu
785                 790                 795                 800

Gln Glu Leu Gln Pro Asp Val Pro Pro Ser Ser Gly Arg Ser
                805                 810

<210> 9
<211> 794
<212> PRT
<213> Homo sapiens

<220>
<223> Komplette Sequenz vom human Furin

<400> 9
Met Glu Leu Arg Pro Trp Leu Leu Trp Val Val Ala Ala Thr Gly Thr
1               5                   10                  15

Leu Val Leu Leu Ala Ala Asp Ala Gln Gly Gln Lys Val Phe Thr Asn
            20                  25                  30

Thr Trp Ala Val Arg Ile Pro Gly Gly Pro Ala Val Ala Asn Ser Val
        35                  40                  45

Ala Arg Lys His Gly Phe Leu Asn Leu Gly Gln Ile Phe Gly Asp Tyr
    50                  55                  60

Tyr His Phe Trp His Arg Gly Val Thr Lys Arg Ser Leu Ser Pro His
65                  70                  75                  80

Arg Pro Arg His Ser Arg Leu Gln Arg Glu Pro Gln Val Gln Trp Leu
                85                  90                  95

Glu Gln Gln Val Ala Lys Arg Arg Thr Lys Arg Asp Val Tyr Gln Glu
            100                 105                 110

Pro Thr Asp Pro Lys Phe Pro Gln Gln Trp Tyr Leu Ser Gly Val Thr
        115                 120                 125

Gln Arg Asp Leu Asn Val Lys Ala Ala Trp Ala Gln Gly Tyr Thr Gly
    130                 135                 140

His Gly Ile Val Val Ser Ile Leu Asp Asp Gly Ile Glu Lys Asn His
145                 150                 155                 160

Pro Asp Leu Ala Gly Asn Tyr Asp Pro Gly Ala Ser Phe Asp Val Asn
                165                 170                 175

Asp Gln Asp Pro Asp Pro Gln Pro Arg Tyr Thr Gln Met Asn Asp Asn
            180                 185                 190

Arg His Gly Thr Arg Cys Ala Gly Glu Val Ala Ala Val Ala Asn Asn
        195                 200                 205

Gly Val Cys Gly Val Gly Val Ala Tyr Asn Ala Arg Ile Gly Gly Val
    210                 215                 220

Arg Met Leu Asp Gly Glu Val Thr Asp Ala Val Glu Ala Arg Ser Leu
225                 230                 235                 240

Gly Leu Asn Pro Asn His Ile His Ile Tyr Ser Ala Ser Trp Gly Pro
                245                 250                 255

Glu Asp Asp Gly Lys Thr Val Asp Gly Pro Ala Arg Leu Ala Glu Glu
            260                 265                 270

Ala Phe Phe Arg Gly Val Ser Gln Gly Arg Gly Gly Leu Gly Ser Ile
        275                 280                 285

Phe Val Trp Ala Ser Gly Asn Gly Gly Arg Glu His Asp Ser Cys Asn
    290                 295                 300

Cys Asp Gly Tyr Thr Asn Ser Ile Tyr Thr Leu Ser Ile Ser Ser Ala
305                 310                 315                 320

Thr Gln Phe Gly Asn Val Pro Trp Tyr Ser Glu Ala Cys Ser Ser Thr
                325                 330                 335

Leu Ala Thr Thr Tyr Ser Ser Gly Asn Gln Asn Glu Lys Gln Ile Val
            340                 345                 350

Thr Thr Asp Leu Arg Gln Lys Cys Thr Glu Ser His Thr Gly Thr Ser
        355                 360                 365

Ala Ser Ala Pro Leu Ala Ala Gly Ile Ile Ala Leu Thr Leu Glu Ala
    370                 375                 380

Asn Lys Asn Leu Thr Trp Arg Asp Met Gln His Leu Val Val Gln Thr
385                 390                 395                 400

Ser Lys Pro Ala His Leu Asn Ala Asn Asp Trp Ala Thr Asn Gly Val
                405                 410                 415

Gly Arg Lys Val Ser His Ser Tyr Gly Tyr Gly Leu Leu Asp Ala Gly
            420                 425                 430

Ala Met Val Ala Leu Ala Gln Asn Trp Thr Thr Val Ala Pro Gln Arg
        435                 440                 445

Lys Cys Ile Ile Asp Ile Leu Thr Glu Pro Lys Asp Ile Gly Lys Arg
    450                 455                 460

Leu Glu Val Arg Lys Thr Val Thr Ala Cys Leu Gly Glu Pro Asn His
465                 470                 475                 480

Ile Thr Arg Leu Glu His Ala Gln Ala Arg Leu Thr Leu Ser Tyr Asn
                485                 490                 495

Arg Arg Gly Asp Leu Ala Ile His Leu Val Ser Pro Met Gly Thr Arg
            500                 505                 510

Ser Thr Leu Leu Ala Ala Arg Pro His Asp Tyr Ser Ala Asp Gly Phe
        515                 520                 525

Asn Asp Trp Ala Phe Met Thr Thr His Ser Trp Asp Glu Asp Pro Ser
    530                 535                 540

Gly Glu Trp Val Leu Glu Ile Glu Asn Thr Ser Glu Ala Asn Asn Tyr
545                 550                 555                 560

Gly Thr Leu Thr Lys Phe Thr Leu Val Leu Tyr Gly Thr Ala Pro Glu
                565                 570                 575

Gly Leu Pro Val Pro Pro Glu Ser Ser Gly Cys Lys Thr Leu Thr Ser
            580                 585                 590

Ser Gln Ala Cys Val Val Cys Glu Glu Gly Phe Ser Leu His Gln Lys
        595                 600                 605

Ser Cys Val Gln His Cys Pro Pro Gly Phe Ala Pro Gln Val Leu Asp
    610                 615                 620

Thr His Tyr Ser Thr Glu Asn Asp Val Glu Thr Ile Arg Ala Ser Val
625                 630                 635                 640

Cys Ala Pro Cys His Ala Ser Cys Ala Thr Cys Gln Gly Pro Ala Leu
                645                 650                 655

Thr Asp Cys Leu Ser Cys Pro Ser His Ala Ser Leu Asp Pro Val Glu
            660                 665                 670

Gln Thr Cys Ser Arg Gln Ser Gln Ser Ser Arg Glu Ser Pro Pro Gln
        675                 680                 685

Gln Gln Pro Pro Arg Leu Pro Pro Glu Val Glu Ala Gly Gln Arg Leu
    690                 695                 700

Arg Ala Gly Leu Leu Pro Ser His Leu Pro Glu Val Val Ala Gly Leu
705                 710                 715                 720

Ser Cys Ala Phe Ile Val Leu Val Phe Val Thr Val Phe Leu Val Leu
                725                 730                 735

Gln Leu Arg Ser Gly Phe Ser Phe Arg Gly Val Lys Val Tyr Thr Met
            740                 745                 750

Asp Arg Gly Leu Ile Ser Tyr Lys Gly Leu Pro Pro Glu Ala Trp Gln
        755                 760                 765

Glu Glu Cys Pro Ser Asp Ser Glu Glu Asp Glu Gly Arg Gly Glu Arg
    770                 775                 780

Thr Ala Phe Ile Lys Asp Gln Ser Ala Leu
785                 790


<210> 10
<211> 793
<212> PRT
<213> Mus musculus

<220>
<223> Komplette Proteinsequenz vom Maus-Furin

<400> 10
Met Glu Leu Arg Ser Trp Leu Leu Trp Val Val Ala Ala Ala Gly Ala
  1               5                  10                  15

Val Val Leu Leu Ala Ala Asp Ala Gln Gly Gln Lys Ile Phe Thr Asn
             20                  25                  30

Thr Trp Ala Val His Ile Pro Gly Gly Pro Ala Val Ala Asp Arg Val
        35                  40                  45

Ala Gln Lys His Gly Phe His Asn Leu Gly Gln Ile Phe Gly Asp Tyr
    50                  55                  60

Tyr His Phe Trp His Arg Ala Val Thr Lys Arg Ser Leu Ser Pro His
65                  70                  75                  80

Arg Pro Arg His Ser Arg Leu Gln Arg Glu Pro Gln Val Lys Trp Leu
                85                  90                  95

Glu Gln Gln Val Ala Lys Arg Arg Ala Lys Arg Asp Val Tyr Gln Glu
            100                 105                 110

Pro Thr Asp Pro Lys Phe Pro Gln Gln Trp Tyr Leu Ser Gly Val Thr
        115                 120                 125

Gln Arg Asp Leu Asn Val Lys Glu Ala Trp Ala Gln Gly Phe Thr Gly
    130                 135                 140

His Gly Ile Val Val Ser Ile Leu Asp Asp Gly Ile Glu Lys Asn His
145                 150                 155                 160

Pro Asp Leu Ala Gly Asn Tyr Asp Pro Gly Ala Ser Phe Asp Val Asn
                165                 170                 175

Asp Gln Asp Pro Asp Pro Gln Pro Arg Tyr Thr Gln Met Asn Asp Asn
            180                 185                 190

Arg His Gly Thr Arg Cys Ala Gly Glu Val Ala Ala Val Ala Asn Asn
        195                 200                 205

Gly Val Cys Gly Val Gly Val Ala Tyr Asn Ala Arg Ile Gly Gly Val
    210                 215                 220

Arg Met Leu Asp Gly Glu Val Thr Asp Ala Val Glu Ala Arg Ser Leu
225                 230                 235                 240

Gly Leu Asn Pro Asn His Ile His Ile Tyr Ser Ala Ser Trp Gly Pro
                245                 250                 255

Glu Asp Asp Gly Lys Thr Val Asp Gly Pro Ala Arg Leu Ala Glu Glu
            260                 265                 270

Ala Phe Phe Arg Gly Val Ser Gln Gly Arg Gly Gly Leu Gly Ser Ile
        275                 280                 285

Phe Val Trp Ala Ser Gly Asn Gly Gly Arg Glu His Asp Ser Cys Asn
    290                 295                 300

Cys Asp Gly Tyr Thr Asn Ser Ile Tyr Thr Leu Ser Ile Ser Ser Ala
305                 310                 315                 320

Thr Gln Phe Gly Asn Val Pro Trp Tyr Ser Glu Ala Cys Ser Ser Thr
                325                 330                 335

Leu Ala Thr Thr Tyr Ser Ser Gly Asn Gln Asn Glu Lys Gln Ile Val
            340                 345                 350

Thr Thr Asp Leu Arg Gln Lys Cys Thr Glu Ser His Thr Gly Thr Ser
        355                 360                 365

Ala Ser Ala Pro Leu Ala Ala Gly Ile Ile Ala Leu Thr Leu Glu Ala
    370                 375                 380

Asn Lys Asn Leu Thr Trp Arg Asp Met Gln His Leu Val Val Gln Thr
385                 390                 395                 400

Ser Lys Pro Ala His Leu Asn Ala Asp Asp Trp Ala Thr Asn Gly Val
                405                 410                 415

Gly Arg Lys Val Ser His Ser Tyr Gly Tyr Gly Leu Leu Asp Ala Gly
            420                 425                 430

Ala Met Val Ala Leu Ala Gln Asn Trp Thr Thr Val Ala Pro Gln Arg
        435                 440                 445

Lys Cys Ile Val Glu Ile Leu Val Glu Pro Lys Asp Ile Gly Lys Arg
    450                 455                 460

Leu Glu Val Arg Lys Ala Val Thr Ala Cys Leu Gly Glu Pro Asn His
465                 470                 475                 480

Ile Thr Arg Leu Glu His Val Gln Ala Arg Leu Thr Leu Ser Tyr Asn
                485                 490                 495

Arg Arg Gly Asp Leu Ala Ile His Leu Ile Ser Pro Met Gly Thr Arg
            500                 505                 510

Ser Thr Leu Leu Ala Ala Arg Pro His Asp Tyr Ser Ala Asp Gly Phe
        515                 520                 525

Asn Asp Trp Ala Phe Met Thr Thr His Ser Trp Asp Glu Asp Pro Ala
    530                 535                 540

Gly Glu Trp Val Leu Glu Ile Glu Asn Thr Ser Glu Ala Asn Asn Tyr
545                 550                 555                 560

Gly Thr Leu Thr Lys Phe Thr Leu Val Leu Tyr Gly Thr Ala Pro Glu
                565                 570                 575

Gly Leu Ser Thr Pro Pro Glu Ser Ser Gly Cys Lys Thr Leu Thr Ser
            580                 585                 590

Ser Gln Ala Cys Val Val Cys Glu Glu Gly Tyr Ser Leu His Gln Lys
        595                 600                 605

Ser Cys Val Gln His Cys Pro Pro Gly Phe Ile Pro Gln Val Leu Asp
    610                 615                 620

Thr His Tyr Ser Thr Glu Asn Asp Val Glu Ile Ile Arg Ala Ser Val
625                 630                 635                 640

Cys Thr Pro Cys His Ala Ser Cys Ala Thr Cys Gln Gly Pro Ala Pro
                645                 650                 655

Thr Asp Cys Leu Ser Cys Pro Ser His Ala Ser Leu Asp Pro Val Glu
            660                 665                 670

Gln Thr Cys Ser Arg Gln Ser Gln Ser Ser Arg Glu Ser Arg Pro Gln
        675                 680                 685

Gln Gln Pro Pro Ala Leu Arg Pro Glu Val Glu Met Glu Pro Arg Leu
    690                 695                 700

Gln Ala Gly Leu Ala Ser His Leu Pro Glu Val Leu Ala Gly Leu Ser
705                 710                 715                 720

Cys Leu Ile Ile Val Leu Ile Phe Gly Ile Val Phe Leu Phe Leu His
                725                 730                 735

Arg Cys Ser Gly Phe Ser Phe Arg Gly Met Lys Val Tyr Thr Met Asp
            740                 745                 750

Arg Gly Leu Ile Ser Tyr Lys Gly Leu Pro Pro Glu Ala Trp Gln Glu
        755                 760                 765

Glu Cys Pro Ser Asp Ser Glu Glu Asp Glu Gly Arg Gly Glu Arg Thr
    770                 775                 780

Ala Phe Ile Lys Asp Gln Ser Ala Leu
785                 790

<210> 11
<211> 470
<212> PRT
<213> Mus musculus

<220>
<223> mouse Furin: In der Dichte erkennbare Sequenz.
      Position 1 entspricht der Position 109 in der
      kompletten Proteinsequenz vom Maus-Furin

<400> 11
Val Tyr Gln Glu Pro Thr Asp Pro Lys Phe Pro Gln Gln Trp Tyr Leu
1               5                   10                  15

Ser Gly Val Thr Gln Arg Asp Leu Asn Val Lys Glu Ala Trp Ala Gln
            20                  25                  30

Gly Phe Thr Gly His Gly Ile Val Val Ser Ile Leu Asp Asp Gly Ile
        35                  40                  45

Glu Lys Asn His Pro Asp Leu Ala Gly Asn Tyr Asp Pro Gly Ala Ser
    50                  55                  60

Phe Asp Val Asn Asp Gln Asp Pro Asp Pro Gln Pro Arg Tyr Thr Gln
65                  70                  75                  80

Met Asn Asp Asn Arg His Gly Thr Arg Cys Ala Gly Glu Val Ala Ala
                85                  90                  95

Val Ala Asn Asn Gly Val Cys Gly Val Gly Val Ala Tyr Asn Ala Arg
            100                 105                 110

Ile Gly Gly Val Arg Met Leu Asp Gly Glu Val Thr Asp Ala Val Glu
        115                 120                 125

Ala Arg Ser Leu Gly Leu Asn Pro Asn His Ile His Ile Tyr Ser Ala
    130                 135                 140

Ser Trp Gly Pro Glu Asp Asp Gly Lys Thr Val Asp Gly Pro Ala Arg
145                 150                 155                 160

Leu Ala Glu Glu Ala Phe Phe Arg Gly Val Ser Gln Gly Arg Gly Gly
                165                 170                 175

Leu Gly Ser Ile Phe Val Trp Ala Ser Gly Asn Gly Gly Arg Glu His
            180                 185                 190

Asp Ser Cys Asn Cys Asp Gly Tyr Thr Asn Ser Ile Tyr Thr Leu Ser
        195                 200                 205

Ile Ser Ser Ala Thr Gln Phe Gly Asn Val Pro Trp Tyr Ser Glu Ala
    210                 215                 220

Cys Ser Ser Thr Leu Ala Thr Thr Tyr Ser Ser Gly Asn Gln Asn Glu
225                 230                 235                 240

Lys Gln Ile Val Thr Thr Asp Leu Arg Gln Lys Cys Thr Glu Ser His
                245                 250                 255

Thr Gly Thr Ser Ala Ser Ala Pro Leu Ala Ala Gly Ile Ile Ala Leu
            260                 265                 270

Thr Leu Glu Ala Asn Lys Asn Leu Thr Trp Arg Asp Met Gln His Leu
        275                 280                 285

Val Val Gln Thr Ser Lys Pro Ala His Leu Asn Ala Asp Asp Trp Ala
    290                 295                 300

Thr Asn Gly Val Gly Arg Lys Val Ser His Ser Tyr Gly Tyr Gly Leu
305                 310                 315                 320

Leu Asp Ala Gly Ala Met Val Ala Leu Ala Gln Asn Trp Thr Thr Val
                325                 330                 335

Ala Pro Gln Arg Lys Cys Ile Val Glu Ile Leu Val Glu Pro Lys Asp
            340                 345                 350

Ile Gly Lys Arg Leu Glu Val Arg Lys Ala Val Thr Ala Cys Leu Gly
        355                 360                 365

Glu Pro Asn His Ile Thr Arg Leu Glu His Val Gln Ala Arg Leu Thr
    370                 375                 380

Leu Ser Tyr Asn Arg Arg Gly Asp Leu Ala Ile His Leu Ile Ser Pro
385                 390                 395                 400

Met Gly Thr Arg Ser Thr Leu Leu Ala Ala Arg Pro His Asp Tyr Ser
                405                 410                 415

Ala Asp Gly Phe Asn Asp Trp Ala Phe Met Thr Thr His Ser Trp Asp
            420                 425                 430

Glu Asp Pro Ala Gly Glu Trp Val Leu Glu Ile Glu Asn Thr Ser Glu
        435                 440                 445

Ala Asn Asn Tyr Gly Thr Leu Thr Lys Phe Thr Leu Val Leu Tyr Gly
450                 455                 460

Thr Ala Pro Glu Gly Leu
465                 470

<210> 12
<211> 604
<212> PRT
<213> Mus musculus

<220>
<223> mouse Furin: Der in den CHO-Zellen klonierte
      Bereich der Ektodomäne. Position 1 entspricht der
      Position 108 in der kompletten Proteinsequenz vom
      Maus-Furin

<400> 12
Asp Val Tyr Gln Glu Pro Thr Asp Pro Lys Phe Pro Gln Gln Trp Tyr
1               5                   10                  15

Leu Ser Gly Val Thr Gln Arg Asp Leu Asn Val Lys Glu Ala Trp Ala
            20                  25                  30

Gln Gly Phe Thr Gly His Gly Ile Val Val Ser Ile Leu Asp Asp Gly
        35                  40                  45

Ile Glu Lys Asn His Pro Asp Leu Ala Gly Asn Tyr Asp Pro Gly Ala
    50                  55                  60

Ser Phe Asp Val Asn Asp Gln Asp Pro Asp Pro Gln Pro Arg Tyr Thr
65                  70                  75                  80

Gln Met Asn Asp Asn Arg His Gly Thr Arg Cys Ala Gly Glu Val Ala
                85                  90                  95

Ala Val Ala Asn Asn Gly Val Cys Gly Val Gly Val Ala Tyr Asn Ala
            100                 105                 110

Arg Ile Gly Gly Val Arg Met Leu Asp Gly Glu Val Thr Asp Ala Val
        115                 120                 125

Glu Ala Arg Ser Leu Gly Leu Asn Pro Asn His Ile His Ile Tyr Ser
    130                 135                 140

Ala Ser Trp Gly Pro Glu Asp Asp Gly Lys Thr Val Asp Gly Pro Ala

145                 150                 155                 160

Arg Leu Ala Glu Glu Ala Phe Phe Arg Gly Val Ser Gln Gly Arg Gly
                165                 170                 175

Gly Leu Gly Ser Ile Phe Val Trp Ala Ser Gly Asn Gly Gly Arg Glu
            180                 185                 190

His Asp Ser Cys Asn Cys Asp Gly Tyr Thr Asn Ser Ile Tyr Thr Leu
        195                 200                 205

Ser Ile Ser Ser Ala Thr Gln Phe Gly Asn Val Pro Trp Tyr Ser Glu
    210                 215                 220

Ala Cys Ser Ser Thr Leu Ala Thr Thr Tyr Ser Ser Gly Asn Gln Asn
225                 230                 235                 240

Glu Lys Gln Ile Val Thr Thr Asp Leu Arg Gln Lys Cys Thr Glu Ser
                245                 250                 255

His Thr Gly Thr Ser Ala Ser Ala Pro Leu Ala Ala Gly Ile Ile Ala
            260                 265                 270

Leu Thr Leu Glu Ala Asn Lys Asn Leu Thr Trp Arg Asp Met Gln His
        275                 280                 285

Leu Val Val Gln Thr Ser Lys Pro Ala His Leu Asn Ala Asp Asp Trp
    290                 295                 300

Ala Thr Asn Gly Val Gly Arg Lys Val Ser His Ser Tyr Gly Tyr Gly
305                 310                 315                 320

Leu Leu Asp Ala Gly Ala Met Val Ala Leu Ala Gln Asn Trp Thr Thr
                325                 330                 335

Val Ala Pro Gln Arg Lys Cys Ile Val Glu Ile Leu Val Glu Pro Lys
            340                 345                 350

Asp Ile Gly Lys Arg Leu Glu Val Arg Lys Ala Val Thr Ala Cys Leu
        355                 360                 365

Gly Glu Pro Asn His Ile Thr Arg Leu Glu His Val Gln Ala Arg Leu
    370                 375                 380

Thr Leu Ser Tyr Asn Arg Arg Gly Asp Leu Ala Ile His Leu Ile Ser
385                 390                 395                 400

Pro Met Gly Thr Arg Ser Thr Leu Leu Ala Ala Arg Pro His Asp Tyr

405                 410                 415

Ser Ala Asp Gly Phe Asn Asp Trp Ala Phe Met Thr Thr His Ser Trp
            420                 425                 430

Asp Glu Asp Pro Ala Gly Glu Trp Val Leu Glu Ile Glu Asn Thr Ser
        435                 440                 445

Glu Ala Asn Asn Tyr Gly Thr Leu Thr Lys Phe Thr Leu Val Leu Tyr
    450                 455                 460

Gly Thr Ala Pro Glu Gly Leu Ser Thr Pro Pro Glu Ser Ser Gly Cys
465                 470                 475                 480

Lys Thr Leu Thr Ser Ser Gln Ala Cys Val Val Cys Glu Glu Gly Tyr
                485                 490                 495

Ser Leu His Gln Lys Ser Cys Val Gln His Cys Pro Pro Gly Phe Ile
            500                 505                 510

Pro Gln Val Leu Asp Thr His Tyr Ser Thr Glu Asn Asp Val Glu Ile
        515                 520                 525

Ile Arg Ala Ser Val Cys Thr Pro Cys His Ala Ser Cys Ala Thr Cys
    530                 535                 540

Gln Gly Pro Ala Pro Thr Asp Cys Leu Ser Cys Pro Ser His Ala Ser
545                 550                 555                 560

Leu Asp Pro Val Glu Gln Thr Cys Ser Arg Gln Ser Gln Ser Ser Arg
                565                 570                 575

Glu Ser Arg Pro Gln Gln Gln Pro Pro Ala Leu Arg Pro Glu Val Glu
            580                 585                 590

Met Glu Pro Arg Leu Gln Ala Gly Leu Ala Ser His
        595                 600

<210> 13
<211> 4
<212> PRT
<213> Künstliche Sequenz

<220>
<223> Beschreibung der künstlichen Sequenz: Ligand für
      eine erfindungsgemäße Raumform eines Polypeptids
      (Anspruch 16).

<220>
<221> MOD_RES
<222> (1)
<223> Acyl-Gruppe

<220>
<221> MOD_RES
<222> (4)
<223> Chlormethan

<400> 13
Arg Xaa Lys Arg
  1


<210> 14
<211> 4
<212> PRT
<213> Künstliche Sequenz

<220>
<223> Beschreibung der künstlichen Sequenz: Ligand für
      eine erfindungsgemäße Raumform eines Polypeptids
      (Anspruch 16).

<220>
<221> MOD_RES
<222> (1)
<223> Acyl-Gruppe

<220>
<221> MOD_RES
<222> (4)
<223> Chlormethan

<400> 14
Arg Xaa Arg Arg
  1


<210> 15
<211> 4
<212> PRT
<213> Unbekannt

<220>
<223> Beschreibung des unbekannten Organismus: Er weist

im reaktiven Zentrum der dritten Domäne von
Ovomucoid die angegebene Sequenz auf (S. 7, Z. 26
der Beschreibung).

<400> 15
Ala Cys Thr Leu
1

<210> 16
<211> 4
<212> PRT
<213> Künstliche Sequenz

<220>
<223> Beschreibung der künstlichen Sequenz: Sequenz,
durch die die vorstehende Sequenz d. reaktiven
Zentrums der 3. Domäne von Ovomucoid ersetzt wird,
um einen inhibitorischen Liganden zu bilden
(Anspr. 16)

<400> 16
Arg Cys Lys Arg
1

<210> 17
<211> 10
<212> PRT
<213> Künstliche Sequenz

<220>
<223> Beschreibung der künstlichen Sequenz: Motiv einer
vom Propeptid von Furin abgeleitete Sequenz (kann
auch natürlichen Ursprungs sein) (Anspruch 16).

<400> 17
Gln Gln Val Ala Lys Arg Arg Thr Lys Arg
1               5                   10

<210> 18
<211> 4
<212> PRT
<213> Künstliche Sequenz

<220>
<223> Beschreibung der künstlichen Sequenz: Ligand für

eine erfindungsgemäße Raumform eines Peptids;
Inhibitor der physiologischen Funktion eines
Proteins der Familie der PCs (Anspruch 18).

<220>
<221> MOD_RES
<222> (1)
<223> Decanoyl-Gruppe

<220>
<221> MOD_RES
<222> (4)
<223> Chlormethylketon

<400> 18
Arg Xaa Lys Arg
1

<210> 19
<211> 4
<212> PRT
<213> Künstliche Sequenz

<220>
<223> Beschreibung der künstlichen Sequenz: Ligand für
eine erfindungsgemäße Raumform eines Peptids;
Inhibitor der physiologischen Funktion eines
Proteins der Familie der PCs (Anspruch 18)

<220>
<221> MOD_RES
<222> (1)
<223> Decanoyl-Gruppe

<220>
<221> MOD_RES
<222> (4)
<223> Chlormethylketon

<400> 19
Arg Xaa Arg Arg
1

<210> 20
<211> 4
<212> PRT

<213> Künstliche Sequenz

<220>
<223> Beschreibung der künstlichen Sequenz: AS-Motiv
      eines Oligopeptids nach Anspruch 35 (kann auch
      natürlichen Ursprungs sein).

<400> 20
Arg Xaa Xaa Arg
  1


<210> 21
<211> 4
<212> PRT
<213> Künstliche Sequenz

<220>
<223> Beschreibung der künstlichen Sequenz: AS-Motiv
      eines Oligopeptids nach Anspruch 35 (kann auch
      natürlichen Ursprungs sein).

<400> 21
Arg Xaa Lys Arg
  1


<210> 22
<211> 4
<212> PRT
<213> Künstliche Sequenz

<220>
<223> Beschreibung der künstlichen Sequenz: AS-Motiv
      eines Oligopeptids nach Anspruch 35 (kann auch
      natürlichen Ursprungs sein).

<400> 22
Arg Xaa Arg Arg
  1


<210> 23
<211> 5
<212> PRT
<213> Künstliche Sequenz

<220>

<223> Beschreibung der künstlichen Sequenz: AS-Motiv
      eines Oligopeptids nach Anspruch 36 (kann auch
      natürlichen Ursprungs sein).

<400> 23
Lys Arg Xaa Lys Arg
  1               5


<210> 24
<211> 5
<212> PRT
<213> Künstliche Sequenz

<220>
<223> Beschreibung der künstlichen Sequenz: AS-Motiv
      eines Oligopeptids nach Anspruch 36 (kann auch
      natürlichen Ursprungs sein).

<400> 24
Arg Arg Xaa Lys Arg
  1               5


<210> 25
<211> 5
<212> PRT
<213> Künstliche Sequenz

<220>
<223> Beschreibung der künstlichen Sequenz: AS-Motiv
      eines Oligopeptids nach Anspruch 36 (kann auch
      natürlichen Ursprungs sein).

<400> 25
Lys Arg Xaa Arg Arg
  1               5


<210> 26
<211> 5
<212> PRT
<213> Künstliche Sequenz

<220>
<223> Beschreibung der künstlichen Sequenz: AS-Motiv
      eines Oligopeptids nach Anspruch 36 (kann auch
      natürlichen Ursprungs sein).

<400> 26
Arg Arg Xaa Arg Arg
  1               5


<210> 27
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 37 enthaltende Aminosäuresequenz.

<400> 27
Val Val Gln Arg Glu Lys Arg
  1               5


<210> 28
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 37 enthaltende Aminosäuresequenz.

<400> 28
Thr His Asn Arg Thr Lys Arg
  1               5


<210> 29
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 37 enthaltende Aminosäuresequenz.

<400> 29
Ile Glu Asn Arg Lys Arg Arg

1                 5


<210> 30
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 37 enthaltende Aminosäuresequenz.

<400> 30
Ser Lys Lys Arg Glu Lys Arg
  1               5


<210> 31
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 37 enthaltende Aminosäuresequenz.

<400> 31
Ser Ser Arg Arg His Lys Arg
  1               5


<210> 32
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 37 enthaltende Aminosäuresequenz.

<400> 32
Gly Gly Arg Arg Gln Arg Arg
  1               5

<210> 33
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 37 enthaltende Aminosäuresequenz.

<400> 33
Ser Ser Gly Arg Ser Lys Arg
  1               5


<210> 34
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 37 enthaltende Aminosäuresequenz.

<400> 34
Thr Asp Pro Arg Thr Lys Arg
  1               5


<210> 35
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 38 enthaltende Aminosäuresequenz.

<400> 35
Ser Asn Ser Arg Lys Lys Arg
  1               5


<210> 36
<211> 7
<212> PRT

<213> Künstliche Sequenz

<220>
<223> Beschreibung der künstlichen Sequenz: In dem
      Oligopeptid (kann auch natürlichen Ursprungs sein)
      nach Anspruch 38 enthaltende Aminosäuresequenz.

<400> 36
Ala Gly Asn Arg Val Arg Arg
  1               5


<210> 37
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 38 enthaltende Aminosäuresequenz.

<400> 37
Thr Arg His Arg Gln Pro Arg
  1               5


<210> 38
<211> 7
<212> PRT
<213> Künstliche Sequenz

<220>
<223> Beschreibung der künstlichen Sequenz: In dem
      Oligopeptid (kann auch natürlichen Ursprungs sein)
      nach Anspruch 38 enthaltende Aminosäuresequenz.

<400> 38
His Ala Ser Arg Val Ala Arg
  1               5


<210> 39
<211> 7
<212> PRT
<213> Künstliche Sequenz

<220>

<223> Beschreibung der künstlichen Sequenz: In dem
      Oligopeptid (kann auch natürlichen Ursprungs sein)
      nach Anspruch 38 enthaltende Aminosäuresequenz.

<400> 39
Ala Lys Arg Arg Thr Lys Arg
  1               5

<210> 40
<211> 10
<212> PRT
<213> Künstliche Sequenz

<220>
<223> Beschreibung der künstlichen Sequenz: In einem als
      inhibitorischer Ligand fungierenden Oligopeptid
      (kann auch natürlichen Ursprungs sein) enthaltenes
      AS-Motiv (S. 7, Z. 31 der Beschreibung).

<400> 40
Glu Gln Arg Leu Leu Lys Arg Ala Lys Arg
  1               5                   10

<210> 41
<211> 6
<212> PRT
<213> Künstliche Sequenz

<220>
<223> Beschreibung der künstlichen Sequenz:
      Inhibitorischer Ligand eines Proteins aus der
      Familie der PCs (S.8, Z3 der Beschreibung).

<220>
<221> MOD_RES
<222> (1)
<223> ACETYLATION

<400> 41
His His Lys Arg Arg Arg
  1               5

<210> 42
<211> 6
<212> PRT

<213> Künstliche Sequenz

<220>
<223> Beschreibung der künstlichen Sequenz:
      Inhibitorischer Ligand eines Proteins aus der
      Familie der PCs (S.8, Z. 3).

<220>
<221> MOD_RES
<222> (1)
<223> ACETYLATION

<400> 42
Met His Lys Arg Arg Arg
  1               5

<210> 43
<211> 6
<212> PRT
<213> Künstliche Sequenz

<220>
<223> Beschreibung der künstlichen Sequenz:
      Inhibitorischer Ligand eines Proteins aus der
      Familie der PCs (S. 8, Z. 4).

<220>
<221> MOD_RES
<222> (1)
<223> ACETYLATION

<400> 43
Lys His Lys Arg Arg Arg
  1               5

<210> 44
<211> 6
<212> PRT
<213> Künstliche Sequenz

<220>
<223> Beschreibung der künstlichen Sequenz:
      Inhibitorischer Ligand eines Proteins aus der
      Familie der PCs (S. 8, Z. 4).

<220>

<221> MOD_RES
<222> (1)
<223> ACETYLATION

<400> 44
Arg His Lys Arg Arg Arg
  1               5


<210> 45
<211> 6
<212> PRT
<213> Künstliche Sequenz

<220>
<223> Beschreibung der künstlichen Sequenz:
      Inhibitorischer Ligand eines Proteins aus der
      Familie der PCs (S. 8, Z.4 der Beschreibung).

<220>
<221> MOD_RES
<222> (1)
<223> ACETYLATION

<400> 45
His Arg Lys Arg Arg Arg
  1               5


<210> 46
<211> 6
<212> PRT
<213> Künstliche Sequenz

<220>
<223> Beschreibung der künstlichen Sequenz:
      Inhibitorischer Ligand eines Proteins aus der
      Familie der PCs (S. 8, Z. 4 der Beschreibung).

<220>
<221> MOD_RES
<222> (1)
<223> ACETYLATION

<400> 46
Met Arg Lys Arg Arg Arg
  1               5

<210> 47
<211> 6
<212> PRT
<213> Künstliche Sequenz

<220>
<223> Beschreibung der künstlichen Sequenz:
      Inhibitorischer Ligand eines Proteins aus der
      Familie der PCs (S. 8, Z. 4 der Beschreibung).

<220>
<221> MOD_RES
<222> (1)
<223> ACETYLATION

<400> 47
Lys Arg Lys Arg Arg Arg
  1               5


<210> 48
<211> 6
<212> PRT
<213> Künstliche Sequenz

<220>
<223> Beschreibung der künstlichen Sequenz:
      Inhibitorischer Ligand eines Proteins aus der
      Familie der PCs (S. 8, Z. 5 der Beschreibung).

<220>
<221> MOD_RES
<222> (1)
<223> ACETYLATION

<400> 48
Arg Arg Lys Arg Arg Arg
  1               5


<210> 49
<211> 6
<212> PRT
<213> Künstliche Sequenz

<220>
<223> Beschreibung der künstlichen Sequenz:

Inhibitorischer Ligand (kann auch natürlichen
      Ursprungs sein) eines Proteins aus der Familie der
      PCs (S. 8, Z. 6 der Beschreibung).

<400> 49
Leu Leu Arg Val Lys Arg
  1               5



<210> 50
<211> 6
<212> PRT
<213> Künstliche Sequenz

<220>
<223> Beschreibung der künstlichen Sequenz:
      Inhibitorischer Ligand eines Proteins aus der
      Familie der PCs (S. 8, Z. 6 der Beschreibung).

<220>
<221> MOD_RES
<222> (1)
<223> ACETYLATION

<400> 50
Leu Leu Arg Val Lys Arg
  1               5



<210> 51
<211> 4
<212> PRT
<213> Künstliche Sequenz

<220>
<223> Beschreibung der künstlichen Sequenz:
      Poly-L-Arginine (Tetra-L-Arg bis Nona-L-Arg). Sie
      können auch natürlichen Ursprungs sein.

<220>
<221> REPEAT
<222> (4)
<223> Diese AS tritt 1 bis 6 mal auf.

<220>
<223> Inhibitorischer Ligand von Proteinen aus der
      Familie der PCs (S. 8, Z. 6 der Beschreibung).

<400> 51
Arg Arg Arg Arg
  1

<210> 52
<211> 4
<212> PRT
<213> Künstliche Sequenz

<220>
<223> Beschreibung der künstlichen Sequenz:
      Inhibitorischer Ligand von Furin (S. 31, Z. 17 der
      Beschreibung).

<220>
<221> MOD_RES
<222> (1)
<223> Decanoyl-Gruppe

<220>
<221> MOD_RES
<222> (4)
<223> Chlormethylketon

<400> 52
Arg Val Lys Arg
  1

<210> 53
<211> 8
<212> PRT
<213> Künstliche Sequenz

<220>
<223> Beschreibung der künstlichen Sequenz: Mit dem
      Furin interagierendes Substrat der Figur 5.

<400> 53
Arg Arg Arg Val Lys Arg Ser Leu
  1               5

<210> 54
<211> 5
<212> PRT
<213> Mus musculus

<220>
<223> Spaltungssegment (Arg107 / Asp108) im M.m. furin
      (Figur 1) (S.33, Z. 16 der Beschreibung).

<400> 54
Arg Ala Lys Arg Asp
  1               5


<210> 55
<211> 5
<212> PRT
<213> Mus musculus

<220>
<223> Kennzeichnet den Abschnitt des M.m.-Furins, der
      ein verdrehtes antiparalleles Faltblatt formt und
      neben dem die Peptidylgruppe des Inhibitors liegt
      (S. 34, Z. 2 der Beschreibung).

<400> 55
Ser Trp Gly Pro Glu
  1               5